# EUROPEAN PATENT APPLICATION

(11) **EP 4 825 842 A1**
(43) Date of publication of application: **21.10.2026**
(21) Application number: 24903803.5
(22) Date of filing: 13.12.2024
(51) Int. Cl.: C07D 215/46, A61K 31/496, A61K 31/4985, A61K 31/4995, A61K 31/551, A61K 39/395, A61K 47/68, A61P 35/00, A61P 35/02, C07D 401/12, C07D 401/14, C07D 487/04, C07D 487/08, C07K 16/28, C08G 65/333, C12N 15/13

(54) **DIVALENT BET DECOMPOSITION AGENT AND TARGETING MOLECULE-DRUG COMPLEX**

(30) Priority: 15.12.2023 JP 2023212506
(71) Applicant: Kyowa Kirin Co., Ltd., Tokyo 100-0004 (JP)
(72) Inventor: YOSHIDA, Kei, Tokyo 100-0004 (JP); NAKAJIMA, Takahiro, Tokyo 100-0004 (JP); ISHIHARA, Shuji, Tokyo 100-0004 (JP); TANIGAWA, Mari, Tokyo 100-0004 (JP); ESAKI, Masahiro, Tokyo 100-0004 (JP)
(74) Representative: J A Kemp LLP
(86) International application number: PCT/JP2024/044309
(87) International publication number: WO 2025/127150

(57) **Abstract**

Provided are a divalent BET degrader and a targeting molecule-drug conjugate containing the divalent BET degrader as a drug. A compound represented by the following formula (I) or a pharmaceutically acceptable salt thereof is used as the divalent BET degrader: 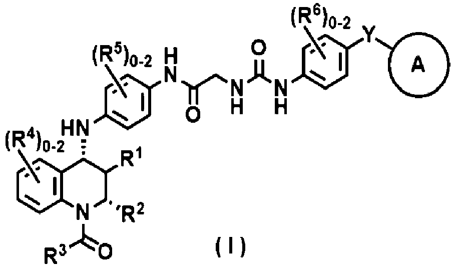 (wherein,
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-, and
ring A represents a group of (i), (ii), or (iii)).

## Description

### [Cross-Reference to Related Application]

The present patent application claims priority to Japanese Patent Application No. 2023-212506 filed on December 15, 2023, and the entire disclosure of which is incorporated herein by reference.

### [Technical Field]

The present invention relates to a divalent BET degrader having degrading action on BET proteins and a targeting molecule-drug conjugate containing the divalent BET degrader as a drug.

### [Background Art]

In humans, 46 bromodomain proteins that recognize the acetylated lysine in histone proteins are known. The BET (bromodomain and extra-terminal domain) family has been reported as one of the families, and recognizes acetyllysine in histones H3 and H4. The BET family is known to include BRD (bromodomain containing protein) 2, BRD3, BRD4 and BRDT (bromodomain testis specific protein). BET family proteins have two bromo domains (BD1, BD2) at the N-terminus, and the sequences are strongly conserved between the families. Further, it has been reported that the BET protein is involved in cancer growth [see non-patent documents 1 and 2] and progression of inflammation [see non-patent document 3].

BRD4 enhances expression of genes that promote growth by recruiting P-TEBb on mitotic chromosomes. In NUT-midline carcinoma (NMC), increased expression of c-MYC protein has been confirmed by the BRD4-NUT fusion protein [see non-patent document 4]. It has also been reported that the degree of decrease in expression of the MYC gene is the most significant in the human multiple myeloma-derived MM1.S cells, among the genes whose expression is decreased by the BET inhibitor JQ-1 treatment [See non-patent document 5].

RVX-208/Apabetalone [see non-patent document 6], I-BET762/GSK-525762A [see non-patent document 7], OTX-015/MK8628 [see non-patent document 8], CPI-0610 [see non-patent document 9], TEN-010 [see non-patent document 10], ABBV-075 [see non-patent document 11], and the like have been reported as typical BET inhibitors, none of which have yet received drug's approval.

In addition, in recent years, a compound, as a bivalent BET inhibitor, having a stronger BET inhibitory activity by simultaneously inhibiting the BD1 and BD2 domains has also been reported [see non-patent document 12]. As the divalent BET inhibitor described above, for example, compounds represented by the following formulas (P1) to (P6) are known [see non-patent documents 12 and 17 and patent document s 1, 2, and 3].

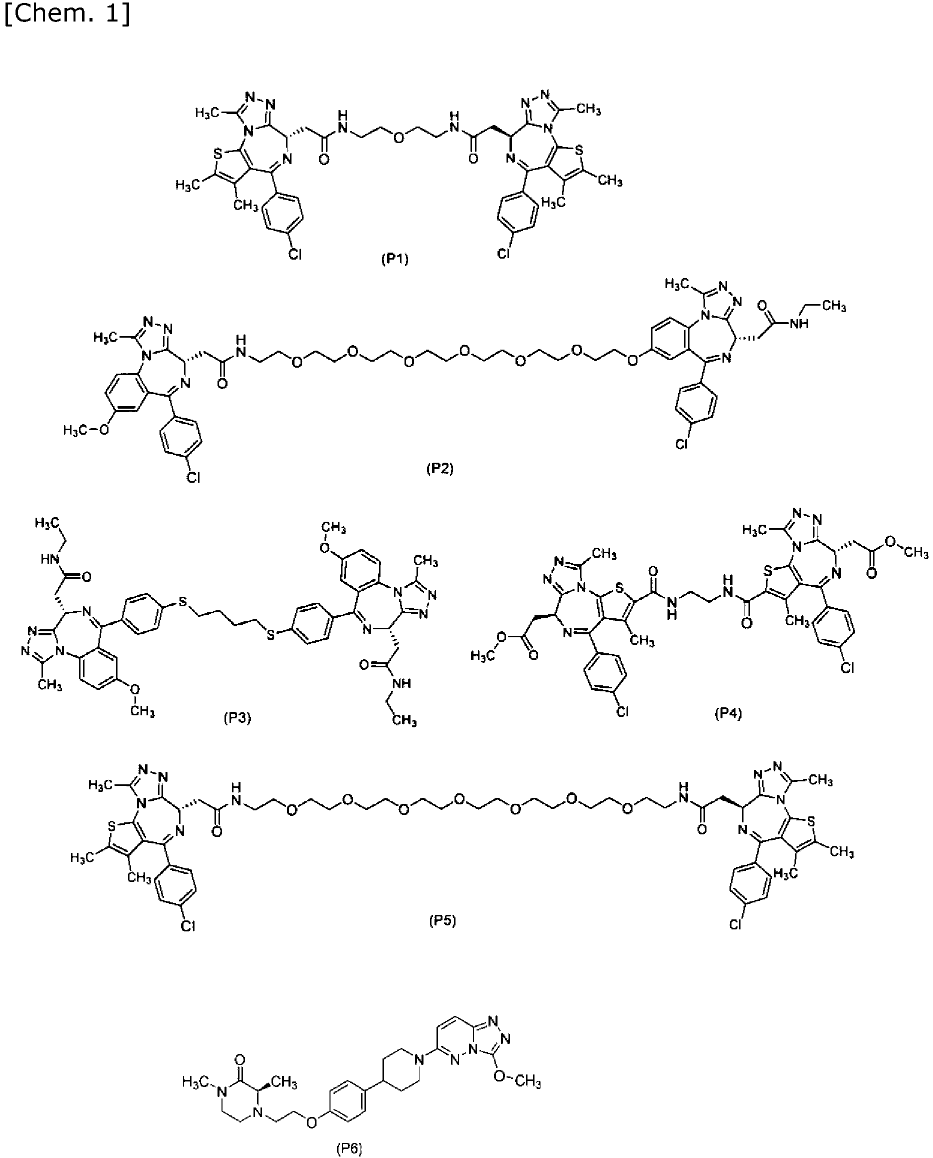

Further, a compound that degrades BET proteins in the ubiquitin-proteasome system has been reported as a new BET function regulator. It is known that the compound more efficiently induces the apoptosis of cancer cells by the degradation of BET proteins than BET inhibitors [see non-patent document 13]. Therefore, the BET degrader can be expected to be capable of serving as an anticancer drug superior to BET inhibitors.

It is known that, as for the divalent forms mentioned above, two small-molecule ligands against BET proteins can be connected via a spacer having a specific structure and thereby used as a BET degrader. More specifically, for example, N-(4-(((2S^{*},4R^{*})-2-methyl-1-propionyl-1,2,3,4-tetrahydroquinolin-4-yl)amino)phenyl)-2-(3-(4-(((2S^{*},4R^{*})-2-methyl-1-propionyl-1,2,3,4-tetrahydroquinolin-4-yl)amino)phenyl)ureido)acetamide (hereinafter, referred to as "g" in the present specification) is known as compound 6j [see patent document 4].

An antibody-drug conjugate (ADC) is generally constituted by a monoclonal antibody covalently bound with a drug via a chemically synthesized linker. ADC is capable of accurately and efficiently removing target cells by combining both the advantages of highly specific targeting ability of the antibody and a strong killing effect of the drug. Cytotoxic payloads currently approved for their use in ADC by FDA are typically strong tubulin inhibitors, DNA damaging agents, and topoisomerase I inhibitors [see non-patent document 14].

ADC has been reported in which a BET protein degrading drug represented by the formula (P7) given below (GNE-987) [see non-patent document 15] or a BET protein degrading drug represented by (P8) (EBET-1593) [see non-patent document 16] is conjugated with an antibody via a linker. However, no clinical trial has been conducted thereon, and it is therefore uncertain whether these BET degraders effectively function as payloads of antibody-drug conjugates.

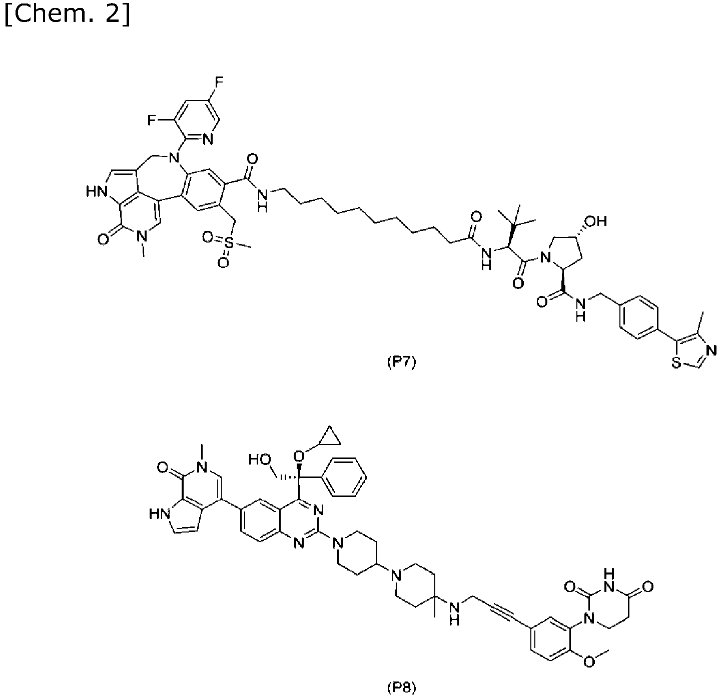

### [PRIOR ART DOCUMENTS]

### [Patent Literature]

Patent Literature 1: WO 2013/033268
Patent Literature 2: WO 2015/081284
Patent Literature 3: WO 2017/091673
Patent Literature 4: WO 2021/065980

### [Non Patent Literature]

Non Patent Literature 1: Nature, 2011, vol. 478, p. 524-528
Non Patent Literature 2: Cell, 2011, vol. 146, p. 904-917
Non Patent Literature 3: Nature, 2010, vol. 468, p. 1119-1123
Non Patent Literature 4: Cancer Res., 2003, vol. 63, p. 304-307
Non Patent Literature 5: Cell, 2013, vol. 153, p. 320-334
Non Patent Literature 6: Proc. Natl. Acad. Sci., 2013, vol. 110, p. 19754-19759
Non Patent Literature 7: J. Med. Chem., 2013, vol. 56, p. 7501-7515
Non Patent Literature 8: Oncotarget, 2015, vol. 6, p. 17698-17712.
Non Patent Literature 9: Blood, 2015, vol. 126, p. 4255-4255
Non Patent Literature 10: Mol. Cancer Ther., 2015, vol. 14, A49
Non Patent Literature 11: Cancer Res., 2016, vol. 76, p. 4718-4718.
Non Patent Literature 12: Nat. Chem. Bio., 2016, vol. 112, p. 1089-1096
Non Patent Literature 13: Science, 2015, vol. 348, p. 1376-1381
Non Patent Literature 14: Sig Transduct Target Ther, 2022, vol. 1, p. 1-25
Non Patent Literature 15: J. Med. Chem., 2021, vol. 64, p. 2576-2607
Non Patent Literature 16: Nat. Commun., 2024, vol. 15, p. 2192-2208
Non Patent Literature 17: Mol. Cancer Ther., 2016, vol. 15, p. 2563-2574

### [Summary of Invention]

The present inventors have pursued drug discovery research on divalent BET degraders and consequently found that the existing divalent BET degraders have peripheral neurotoxicity, a safety hazard that must be avoided, though exhibiting high antitumor activity. The present inventors have conducted various studies to solve the problem and found a divalent BET degrader that avoids the safety hazard, i.e., peripheral neurotoxicity, while maintaining high antitumor activity. The present inventors have also found that the divalent BET degrader found is also useful as a payload (drug) in a targeting molecule-drug conjugate such as an antibody-drug conjugate (ADC).

The present invention encompasses the following [1] to [162].
[1] A compound represented by the following formula (I) or a pharmaceutically acceptable salt thereof: 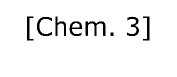 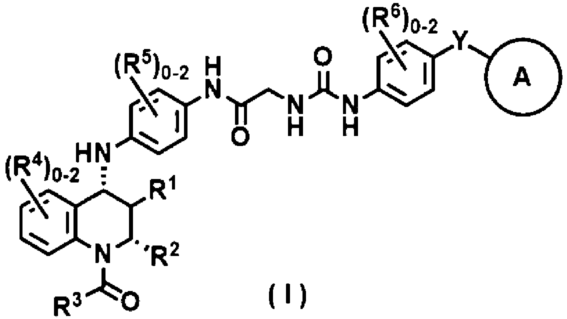 (wherein,
   R¹ and R² are the same or different and each represents a hydrogen atom or lower alkyl,
   R³ represents lower alkyl,
   R⁴ represents halogen,
   R⁵ represents halogen,
   R⁶ represents halogen or lower alkoxy,
   Y represents -O-CH₂-CH₂-, -CH₂-CH₂-CH₂-, -CH=CH-CH₂-, or - NH-C(=O)-CH₂-, and
   ring A represents
      (i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
      (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
      (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
[2] The compound or the pharmaceutically acceptable salt thereof according to [1], wherein (i) the aliphatic heterocyclic group in ring A is a nitrogen-containing aliphatic heterocyclic group.
[3] The compound or the pharmaceutically acceptable salt thereof according to [2], wherein ring A represented by (i) the nitrogen-containing aliphatic heterocyclic group is the following ring A': 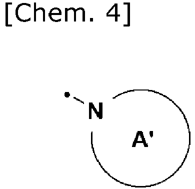 (wherein, when ring A' has a substituent, the substituent is as defined for the substituent of ring A).
[4] The compound or the pharmaceutically acceptable salt thereof according to any of [1] to [3], wherein the compound represented by the formula (I) is a compound represented by the following formula (I'): 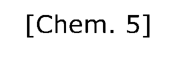 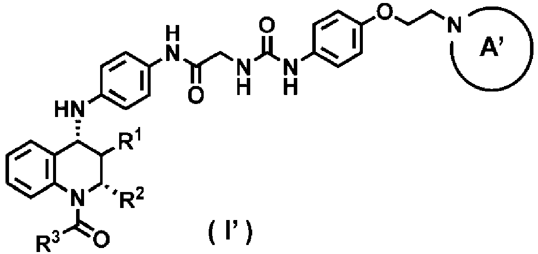 (wherein,
   R¹, R², and R³ are as defined in the formula (I), and
   ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
[5] The compound or the pharmaceutically acceptable salt thereof according to any of [1] to [4], wherein ring A is a nitrogen-containing aliphatic heterocyclic group represented by the following formula (AA), (AB), (AC), or (AD), an aromatic heterocyclic group represented by the following formula (AE) or (AF), or aryl represented by the following formula (AG): 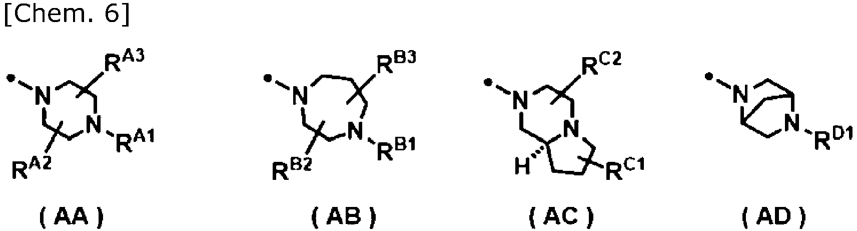 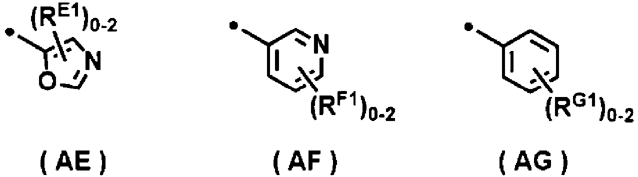 (wherein,
   R^{A1}, R^{B1}, R^{D1}, R^{E1}, R^{F1}, and R^{G1} are the same or different and each represents lower alkyl, hydroxy-lower alkyl, lower alkylcarbonyl, lower alkoxycarbonyl, cycloalkylcarbonyl, or lower alkylcarbamoyl, and
   R^{A2}, R^{A3}, R^{B2}, R^{B3}, R^{C1}, and R^{C2} are the same or different and each represents a hydrogen atom, oxo, lower alkyl, or hydroxy-lower alkyl).
[6] The compound or the pharmaceutically acceptable salt thereof according to any of [1] to [5], wherein ring A is a nitrogen-containing aliphatic heterocyclic group represented by the following formula (AA): 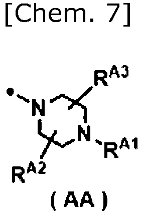 (wherein,
   R^{A1} represents lower alkyl, lower alkylcarbonyl, or cycloalkylcarbonyl, and
   R^{A2} and R^{A3} are the same or different and each represents a hydrogen atom, oxo, lower alkyl, or hydroxy-lower alkyl).
[7] The compound or the pharmaceutically acceptable salt thereof according to [6], wherein R^{A1} is lower alkyl or lower alkylcarbonyl.
[8] The compound or the pharmaceutically acceptable salt thereof according to [6] or [7], wherein R^{A2} and R^{A3} are the same or different and are each a hydrogen atom, oxo, or lower alkyl.
[9] The compound or the pharmaceutically acceptable salt thereof according to any of [6] to [8], wherein R^{A1} is lower alkyl, and R^{A2} and R^{A3} are the same or different and are each oxo or lower alkyl.
[10] The compound or the pharmaceutically acceptable salt thereof according to any of [1] to [5], wherein ring A is a nitrogen-containing aliphatic heterocyclic group represented by the following formula (AB): 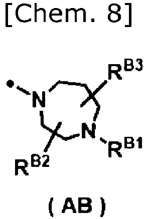 (wherein,
   R^{B1} represents lower alkyl or lower alkylcarbonyl, and
   R^{B2} and R^{B3} are the same or different and each represents a hydrogen atom or oxo).
[11] The compound or the pharmaceutically acceptable salt thereof according to [10], wherein R^{B1} is lower alkyl.
[12] The compound or the pharmaceutically acceptable salt thereof according to any of [1] to [5], wherein ring A is a nitrogen-containing aliphatic heterocyclic group represented by the following formula (AC): 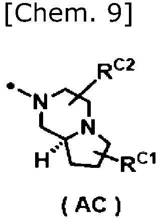 (wherein R^{C1} and R^{C2} are the same or different and each represents a hydrogen atom or oxo).
[13] The compound or the pharmaceutically acceptable salt thereof according to any of [1] to [5], wherein ring A is a nitrogen-containing aliphatic heterocyclic group represented by the following formula (AD): 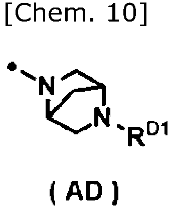 (wherein R^{D1} represents lower alkylcarbonyl).
[14] The compound or the pharmaceutically acceptable salt thereof according to [1], wherein ring A is an aromatic heterocyclic group represented by the following formula (AE): 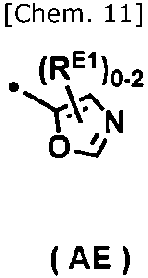 (wherein R^{E1} represents lower alkyl).
[15] The compound or the pharmaceutically acceptable salt thereof according to [1], wherein ring A is an aromatic heterocyclic group represented by the following formula (AF): 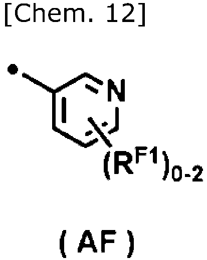 (wherein R^{F1} represents lower alkyl).
[16] The compound or the pharmaceutically acceptable salt thereof according to [1], wherein ring A is aryl represented by the following formula (AG): 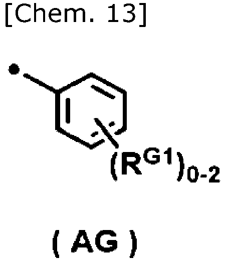 (wherein R^{G1} represents lower alkoxycarbonyl or lower alkylcarbamoyl).
[17] The compound or the pharmaceutically acceptable salt thereof according to any of [1] to [16], wherein Y is -O-CH₂-CH₂-.
[18] The compound or the pharmaceutically acceptable salt thereof according to any of [1] to [17], wherein R¹ is a hydrogen atom, R² is methyl, and R³ is ethyl.
[19] A compound represented by the following formula or a pharmaceutically acceptable salt thereof: 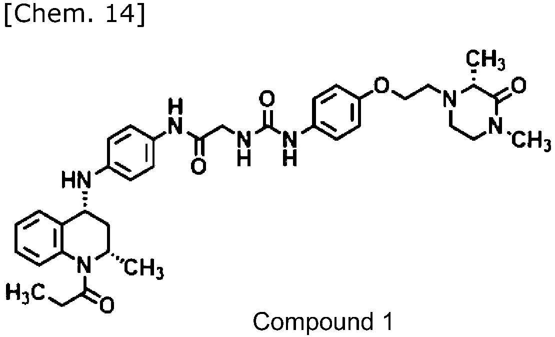
[20] A compound represented by the following formula or a pharmaceutically acceptable salt thereof: 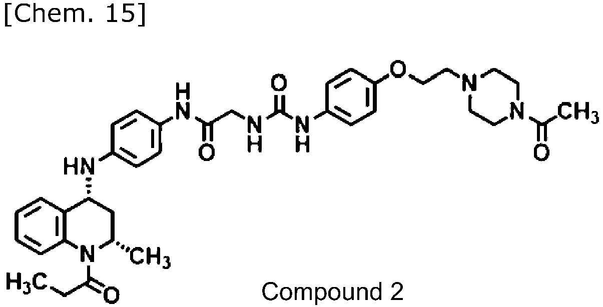
[21] A compound represented by the following formula or a pharmaceutically acceptable salt thereof: 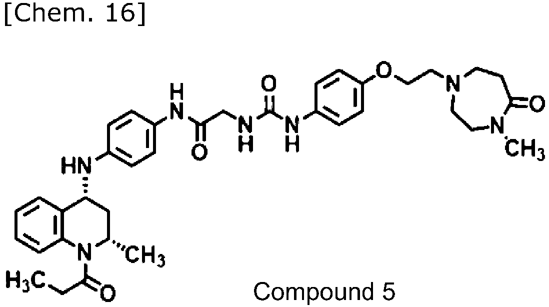
[22] A compound represented by the following formula or a pharmaceutically acceptable salt thereof: 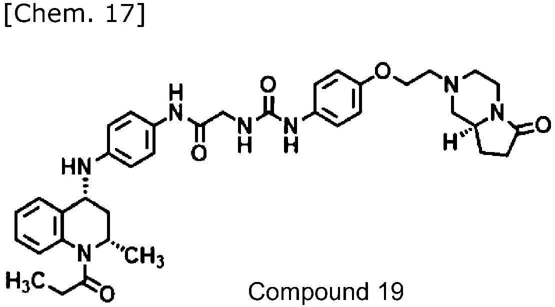
[23] A compound represented by the following formula or a pharmaceutically acceptable salt thereof: 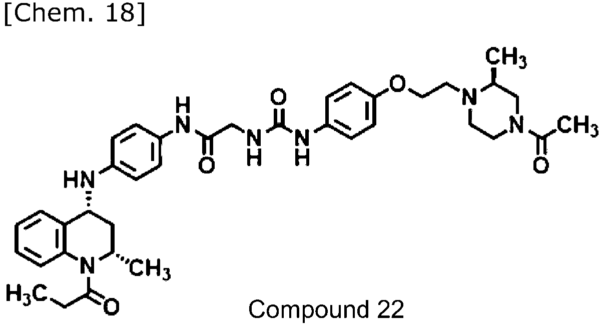
[24] A therapeutic agent for cancer, containing the compound or the pharmaceutically acceptable salt thereof according to any of [1] to [23] as an active ingredient.
[25] The agent according to [24], wherein the cancer is B cell lymphoma.
[26] A method for treating cancer, including administering the compound or the pharmaceutically acceptable salt thereof according to any of [1] to [23] to a subject.
[27] The method according to [26], wherein the cancer is B cell lymphoma.
[28] The compound or the pharmaceutically acceptable salt thereof according to any of [1] to [23] for use in the treatment of cancer.
[29] The compound or the pharmaceutically acceptable salt thereof according to [28], wherein the cancer is B cell lymphoma.
[30] Use of the compound or the pharmaceutically acceptable salt thereof according to any of [1] to [23] for the production of a drug for the treatment of cancer.
[31] The use according to [30], wherein the cancer is B cell lymphoma.
[32] A targeting molecule-drug conjugate containing a targeting molecule and a drug, wherein the drug is the compound or the pharmaceutically acceptable salt thereof according to any of [1] to [23].
[33] The targeting molecule-drug conjugate according to [32], wherein the drug is linked to the targeting molecule via a linker.
[34] The targeting molecule-drug conjugate according to [32] or [33], wherein the targeting molecule-drug conjugate is represented by the following formula (II): 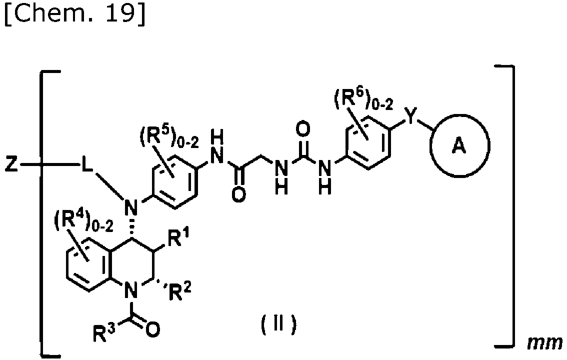 (wherein,
   R¹ and R² are the same or different and each represents a hydrogen atom or lower alkyl,
   R³ represents lower alkyl,
   R⁴ represents halogen,
   R⁵ represents halogen,
   R⁶ represents halogen or lower alkoxy,
   Y represents -O-CH₂-CH₂-, -CH₂-CH₂-CH₂-, -CH=CH-CH₂-, or - NH-C(=O)-CH₂-,
   ring A represents
      (i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
      (ii) an aromatic heterocyclic group optionally having 1 to 3 substituents selected from the group consisting of lower alkyl, hydroxy-lower alkyl, lower alkylcarbonyl, lower alkoxycarbonyl, hydroxy-lower alkylcarbonyl, cycloalkylcarbonyl, and lower alkylcarbamoyl, or
      (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
   L represents a linker,
   mm represents an integer of 1 to 8, and
   Z represents a targeting molecule).
[35] The compound or the pharmaceutically acceptable salt thereof according to [34], wherein (i) the aliphatic heterocyclic group in ring A is a nitrogen-containing aliphatic heterocyclic group.
[36] The targeting molecule-drug conjugate according to [34] or [35], wherein ring A represented by (i) the aliphatic heterocyclic group is the following ring A': 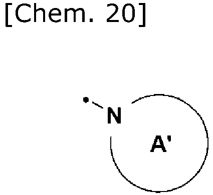 (wherein, when ring A' has a substituent, the substituent of ring A' is as defined for the substituent of ring A).
[37] The targeting molecule-drug conjugate according to any of [33] to [36], wherein the targeting molecule-drug conjugate is represented by the following formula (II'): 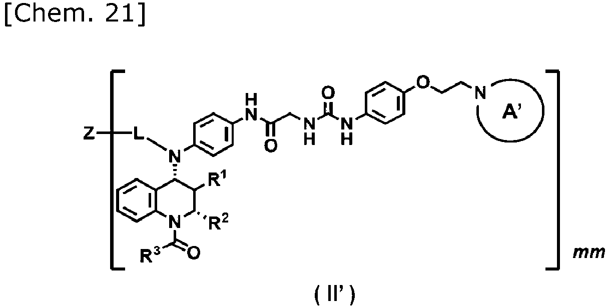 (wherein,
   R¹, R², R³, L, mm, and Z are as defined in the formula (II), and
   ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
[38] The targeting molecule-drug conjugate according to any of [34] to [36], wherein L is represented by the following formula (III): 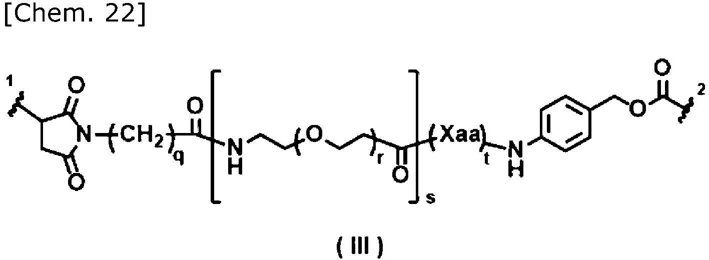 (wherein,
   wavy line 1 represents a bonding site to Z,
   wavy line 2 represents a bonding site to the N atom, and
   each Xaa independently represents
      (a) a residue of at least one amino acid selected from the group consisting of valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents
      (b) a peptide mimetic represented by the following formula (IV): 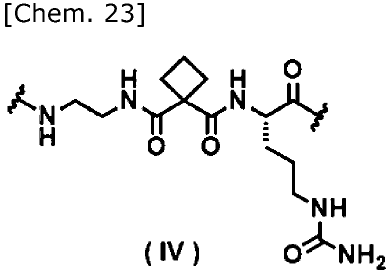 wherein,
         q represents an integer of 1 to 5,
         r represents an integer of 2 to 24,
         s represents 0 or 1, and
         t represents 1, 2, 3, or 4).
[39] The targeting molecule-drug conjugate according to [38], wherein each Xaa is independently a residue of at least one amino acid selected from the group consisting of valine, alanine, citrulline, glycine, proline, phenylalanine, and glutamic acid, or is a peptide mimetic represented by the following formula (IV): 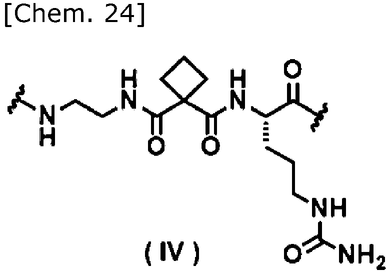
[40] The targeting molecule-drug conjugate according to [38], wherein each Xaa is independently a residue of an amino acid selected from valine and alanine.
[41] The targeting molecule-drug conjugate according to any of [38] to [40], wherein t is 2.
[42] The targeting molecule-drug conjugate according to any of [38] to [41], wherein r is 6 or 12.
[43] The targeting molecule-drug conjugate according to any of [38] to [41], wherein r is 12.
[44] The targeting molecule-drug conjugate according to any of [38] to [43], wherein q is 2 or 5.
[45] The targeting molecule-drug conjugate according to any of [38] to [43], wherein q is 2.
[46] The targeting molecule-drug conjugate according to any of [38] to [45], wherein s is 1.
[47] The targeting molecule-drug conjugate according to any of [32] to [46], wherein the targeting molecule is an antibody or an antigen binding fragment thereof, a small-molecule compound, or a peptide.
[48] The targeting molecule-drug conjugate according to any of [32] to [46], wherein the targeting molecule is an antibody or an antigen binding fragment thereof.
[49] The targeting molecule-drug conjugate according to [48], wherein the antibody is an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-Nectin4, or an anti-TROP2 antibody.
[50] The targeting molecule-drug conjugate according to [48], wherein the antibody is an anti-CD79b antibody.
[51] The targeting molecule-drug conjugate according to [50], wherein the anti-CD79b antibody contains a heavy chain variable region (hereinafter, also referred to as "VH") containing CDR1 consisting of the amino acid sequence set forth in SEQ ID NO: 11, CDR2 consisting of the amino acid sequence set forth in SEQ ID NO: 12, and CDR3 consisting of the amino acid sequence set forth in SEQ ID NO: 13, and a light chain variable region (hereinafter, also referred to as "VL") containing CDR1 consisting of the amino acid sequence set forth in SEQ ID NO: 14, CDR2 consisting of the amino acid sequence set forth in SEQ ID NO: 15, and CDR3 consisting of the amino acid sequence set forth in SEQ ID NO: 16.
[52] The targeting molecule-drug conjugate according to [50] or [51], wherein the amino acid sequence of VH of the anti-CD79b antibody contains the amino acid sequence set forth in SEQ ID NO: 5, and the amino acid sequence of VL of the anti-CD79b antibody contains the amino acid sequence set forth in SEQ ID NO: 6.
[53] The targeting molecule-drug conjugate according to any of [50] to [52], wherein the amino acid sequence of VH of the anti-CD79b antibody contains an amino acid sequence having 90% or higher sequence identity to the amino acid sequence set forth in SEQ ID NO: 5, and the amino acid sequence of VL of the anti-CD79b antibody contains an amino acid sequence having 90% or higher sequence identity to the amino acid sequence set forth in SEQ ID NO: 6.
[54] The targeting molecule-drug conjugate according to [48], wherein the antibody is an anti-CD19 antibody.
[55] The targeting molecule-drug conjugate according to [54], wherein the anti-CD19 antibody contains VH containing CDR1 consisting of the amino acid sequence set forth in SEQ ID NO: 17, CDR2 consisting of the amino acid sequence set forth in SEQ ID NO: 18, and CDR3 consisting of the amino acid sequence set forth in SEQ ID NO: 19, and VL containing CDR1 consisting of the amino acid sequence set forth in SEQ ID NO: 20, CDR2 consisting of the amino acid sequence set forth in SEQ ID NO: 21, and CDR3 consisting of the amino acid sequence set forth in SEQ ID NO: 22.
[56] The targeting molecule-drug conjugate according to [54] or [55], wherein the amino acid sequence of VH of the anti-CD19 antibody contains the amino acid sequence set forth in SEQ ID NO: 7, and the amino acid sequence of VL of the anti-CD19 antibody contains the amino acid sequence set forth in SEQ ID NO: 8.
[57] The targeting molecule-drug conjugate according to any of [54] to [56], wherein the amino acid sequence of VH of the anti-CD19 antibody contains an amino acid sequence having 90% or higher sequence identity to the amino acid sequence set forth in SEQ ID NO: 7, and the amino acid sequence of VL of the anti-CD19 antibody contains an amino acid sequence having 90% or higher sequence identity to the amino acid sequence set forth in SEQ ID NO: 8.
[58] The targeting molecule-drug conjugate according to [48], wherein the antibody is an anti-CD22 antibody.
[59] The targeting molecule-drug conjugate according to [58], wherein the anti-CD22 antibody contains VH containing CDR1 consisting of the amino acid sequence set forth in SEQ ID NO: 23, CDR2 consisting of the amino acid sequence set forth in SEQ ID NO: 24, and CDR3 consisting of the amino acid sequence set forth in SEQ ID NO: 25, and VL containing CDR1 consisting of the amino acid sequence set forth in SEQ ID NO: 26, CDR2 consisting of the amino acid sequence set forth in SEQ ID NO: 27, and CDR3 consisting of the amino acid sequence set forth in SEQ ID NO: 28.
[60] The targeting molecule-drug conjugate according to [58] or [59], wherein the amino acid sequence of VH of the anti-CD22 antibody contains the amino acid sequence set forth in SEQ ID NO: 9, and the amino acid sequence of VL of the anti-CD22 antibody contains the amino acid sequence set forth in SEQ ID NO: 10.
[61] The targeting molecule-drug conjugate according to any of [58] to [60], wherein the amino acid sequence of VH of the anti-CD22 antibody contains an amino acid sequence having 90% or higher sequence identity to the amino acid sequence set forth in SEQ ID NO: 9, and the amino acid sequence of VL of the anti-CD22 antibody contains an amino acid sequence having 90% or higher sequence identity to the amino acid sequence set forth in SEQ ID NO: 10.
[62] The targeting molecule-drug conjugate according to [48], wherein the antibody is an anti-HER2 antibody.
[63] The targeting molecule-drug conjugate according to [62], wherein the anti-HER2 antibody contains VH containing CDR1 consisting of the amino acid sequence set forth in SEQ ID NO: 67, CDR2 consisting of the amino acid sequence set forth in SEQ ID NO: 68, and CDR3 consisting of the amino acid sequence set forth in SEQ ID NO: 69, and VL containing CDR1 consisting of the amino acid sequence set forth in SEQ ID NO: 70, CDR2 consisting of the amino acid sequence set forth in SEQ ID NO: 71, and CDR3 consisting of the amino acid sequence set forth in SEQ ID NO: 72.
[64] The targeting molecule-drug conjugate according to [62] or [63], wherein the amino acid sequence of VH of the anti-HER2 antibody contains the amino acid sequence set forth in SEQ ID NO: 29, the amino acid sequence of VL of the anti-HER2 antibody contains the amino acid sequence set forth in SEQ ID NO: 30.
[65] The targeting molecule-drug conjugate according to any of [62] to [64], wherein the amino acid sequence of VH of the anti-HER2 antibody contains an amino acid sequence having 90% or higher sequence identity to the amino acid sequence set forth in SEQ ID NO: 29, and the amino acid sequence of VL of the anti-HER2 antibody contains an amino acid sequence having 90% or higher sequence identity to the amino acid sequence set forth in SEQ ID NO: 30.
[66] The targeting molecule-drug conjugate according to [48], wherein the antibody is an anti-DLL3 antibody.
[67] The targeting molecule-drug conjugate according to [66], wherein the anti-DLL3 antibody contains VH containing CDR1 consisting of the amino acid sequence set forth in SEQ ID NO: 73, CDR2 consisting of the amino acid sequence set forth in SEQ ID NO: 74, and CDR3 consisting of the amino acid sequence set forth in SEQ ID NO: 75, and VL containing CDR1 consisting of the amino acid sequence set forth in SEQ ID NO: 76, CDR2 consisting of the amino acid sequence set forth in SEQ ID NO: 77, and CDR3 consisting of the amino acid sequence set forth in SEQ ID NO: 78.
[68] The targeting molecule-drug conjugate according to [66] or [67], wherein the amino acid sequence of VH of the anti-DLL3 antibody contains the amino acid sequence set forth in SEQ ID NO: 31, and the amino acid sequence of VL of the anti-DLL3 antibody contains the amino acid sequence set forth in SEQ ID NO: 32.
[69] The targeting molecule-drug conjugate according to any of [66] to [68], wherein the amino acid sequence of VH of the anti-DLL3 antibody contains an amino acid sequence having 90% or higher sequence identity to the amino acid sequence set forth in SEQ ID NO: 31, and the amino acid sequence of VL of the anti-DLL3 antibody contains an amino acid sequence having 90% or higher sequence identity to the amino acid sequence set forth in SEQ ID NO: 32.
[70] The targeting molecule-drug conjugate according to [48], wherein the antibody is an anti-GM2 antibody.
[71] The targeting molecule-drug conjugate according to [70], wherein the anti-GM2 antibody contains VH containing CDR1 consisting of the amino acid sequence set forth in SEQ ID NO: 79, CDR2 consisting of the amino acid sequence set forth in SEQ ID NO: 80, and CDR3 consisting of the amino acid sequence set forth in SEQ ID NO: 81, and VL containing CDR1 consisting of the amino acid sequence set forth in SEQ ID NO: 82, CDR2 consisting of the amino acid sequence set forth in SEQ ID NO: 83, and CDR3 consisting of the amino acid sequence set forth in SEQ ID NO: 84.
[72] The targeting molecule-drug conjugate according to [70] or [71], wherein the amino acid sequence of VH of the anti-GM2 antibody contains the amino acid sequence set forth in SEQ ID NO: 33, and the amino acid sequence of VL of the anti-GM2 antibody contains the amino acid sequence set forth in SEQ ID NO: 34.
[73] The targeting molecule-drug conjugate according to any of [70] to [72], wherein the amino acid sequence of VH of the anti-GM2 antibody contains an amino acid sequence having 90% or higher sequence identity to the amino acid sequence set forth in SEQ ID NO: 33, and the amino acid sequence of VL of the anti-GM2 antibody contains an amino acid sequence having 90% or higher sequence identity to the amino acid sequence set forth in SEQ ID NO: 34.
[74] The targeting molecule-drug conjugate according to [48], wherein the antibody is an anti-EphA2 antibody.
[75] The targeting molecule-drug conjugate according to [74], wherein the anti-EphA2 antibody contains VH containing CDR1 consisting of the amino acid sequence set forth in SEQ ID NO: 85, CDR2 consisting of the amino acid sequence set forth in SEQ ID NO: 86, and CDR3 consisting of the amino acid sequence set forth in SEQ ID NO: 87, and VL containing CDR1 consisting of the amino acid sequence set forth in SEQ ID NO: 88, CDR2 consisting of the amino acid sequence set forth in SEQ ID NO: 89, and CDR3 consisting of the amino acid sequence set forth in SEQ ID NO: 90.
[76] The targeting molecule-drug conjugate according to [74] or [75], wherein the amino acid sequence of VH of the anti-EphA2 antibody contains the amino acid sequence set forth in SEQ ID NO: 35, and the amino acid sequence of VL of the anti-EphA2 antibody contains the amino acid sequence set forth in SEQ ID NO: 36.
[77] The targeting molecule-drug conjugate according to any of [74] to [76], wherein the amino acid sequence of VH of the anti-EphA2 antibody contains an amino acid sequence having 90% or higher sequence identity to the amino acid sequence set forth in SEQ ID NO: 35, and the amino acid sequence of VL of the anti-EphA2 antibody contains an amino acid sequence having 90% or higher sequence identity to the amino acid sequence set forth in SEQ ID NO: 36.
[78] The targeting molecule-drug conjugate according to [48], wherein the antibody is an anti-FAP antibody.
[79] The targeting molecule-drug conjugate according to [78], wherein the anti-FAP antibody contains VH containing CDR1 consisting of the amino acid sequence set forth in SEQ ID NO: 91, CDR2 consisting of the amino acid sequence set forth in SEQ ID NO: 92, and CDR3 consisting of the amino acid sequence set forth in SEQ ID NO: 93, and VL containing CDR1 consisting of the amino acid sequence set forth in SEQ ID NO: 94, CDR2 consisting of the amino acid sequence set forth in SEQ ID NO: 95, and CDR3 consisting of the amino acid sequence set forth in SEQ ID NO: 96.
[80] The targeting molecule-drug conjugate according to [78] or [79], wherein the amino acid sequence of VH of the anti-FAP antibody contains the amino acid sequence set forth in SEQ ID NO: 37, and the amino acid sequence of VL of the anti-FAP antibody contains the amino acid sequence set forth in SEQ ID NO: 38.
[81] The targeting molecule-drug conjugate according to any of [78] to [80], wherein the amino acid sequence of VH of the anti-FAP antibody contains an amino acid sequence having 90% or higher sequence identity to the amino acid sequence set forth in SEQ ID NO: 37, and the amino acid sequence of VL of the anti-FAP antibody contains an amino acid sequence having 90% or higher sequence identity to the amino acid sequence set forth in SEQ ID NO: 38.
[82] The targeting molecule-drug conjugate according to [48], wherein the antibody is an anti-SSTR2 antibody.
[83] The targeting molecule-drug conjugate according to [82], wherein the anti-SSTR2 antibody contains VH containing CDR1 consisting of the amino acid sequence set forth in SEQ ID NO: 97, CDR2 consisting of the amino acid sequence set forth in SEQ ID NO: 98, and CDR3 consisting of the amino acid sequence set forth in SEQ ID NO: 99, and VL containing CDR1 consisting of the amino acid sequence set forth in SEQ ID NO: 100, CDR2 consisting of the amino acid sequence set forth in SEQ ID NO: 101, and CDR3 consisting of the amino acid sequence set forth in SEQ ID NO: 102.
[84] The targeting molecule-drug conjugate according to [82] or [83], wherein the amino acid sequence of VH of the anti-SSTR2 antibody contains the amino acid sequence set forth in SEQ ID NO: 39, and the amino acid sequence of VL of the anti-SSTR2 antibody contains the amino acid sequence set forth in SEQ ID NO: 40.
[85] The targeting molecule-drug conjugate according to any of [82] to [84], wherein the amino acid sequence of VH of the anti-SSTR2 antibody contains an amino acid sequence having 90% or higher sequence identity to the amino acid sequence set forth in SEQ ID NO: 39, and the amino acid sequence of VL of the anti-SSTR2 antibody contains an amino acid sequence having 90% or higher sequence identity to the amino acid sequence set forth in SEQ ID NO: 40.
[86] The targeting molecule-drug conjugate according to [48], wherein the antibody is an anti-CD30 antibody.
[87] The targeting molecule-drug conjugate according to [86], wherein the anti-CD30 antibody contains VH containing CDR1 consisting of the amino acid sequence set forth in SEQ ID NO: 103, CDR2 consisting of the amino acid sequence set forth in SEQ ID NO: 104, and CDR3 consisting of the amino acid sequence set forth in SEQ ID NO: 105, and VL containing CDR1 consisting of the amino acid sequence set forth in SEQ ID NO: 106, CDR2 consisting of the amino acid sequence set forth in SEQ ID NO: 107, and CDR3 consisting of the amino acid sequence set forth in SEQ ID NO: 108.
[88] The targeting molecule-drug conjugate according to [86] or [87], wherein the amino acid sequence of VH of the anti-CD30 antibody contains the amino acid sequence set forth in SEQ ID NO: 41, and the amino acid sequence of VL of the anti-CD30 antibody contains the amino acid sequence set forth in SEQ ID NO: 42.
[89] The targeting molecule-drug conjugate according to any of [86] to [88], wherein the amino acid sequence of VH of the anti-CD30 antibody contains an amino acid sequence having 90% or higher sequence identity to the amino acid sequence set forth in SEQ ID NO: 41, and the amino acid sequence of VL of the anti-CD30 antibody contains an amino acid sequence having 90% or higher sequence identity to the amino acid sequence set forth in SEQ ID NO: 42.
[90] The targeting molecule-drug conjugate according to [48], wherein the antibody is an anti-BCMA antibody.
[91] The targeting molecule-drug conjugate according to [90], wherein the anti-BCMA antibody contains VH containing CDR1 consisting of the amino acid sequence set forth in SEQ ID NO: 109, CDR2 consisting of the amino acid sequence set forth in SEQ ID NO: 110, and CDR3 consisting of the amino acid sequence set forth in SEQ ID NO: 111, and VL containing CDR1 consisting of the amino acid sequence set forth in SEQ ID NO: 112, CDR2 consisting of the amino acid sequence set forth in SEQ ID NO: 113, and CDR3 consisting of the amino acid sequence set forth in SEQ ID NO: 114.
[92] The targeting molecule-drug conjugate according to [90] or [91], wherein the amino acid sequence of VH of the anti-BCMA antibody contains the amino acid sequence set forth in SEQ ID NO: 43, and the amino acid sequence of VL of the anti-BCMA antibody contains the amino acid sequence set forth in SEQ ID NO: 44.
[93] The targeting molecule-drug conjugate according to any of [90] to [92], wherein the amino acid sequence of VH of the anti-BCMA antibody contains an amino acid sequence having 90% or higher sequence identity to the amino acid sequence set forth in SEQ ID NO: 43, and the amino acid sequence of VL of the anti-BCMA antibody contains an amino acid sequence having 90% or higher sequence identity to the amino acid sequence set forth in SEQ ID NO: 44.
[94] The targeting molecule-drug conjugate according to [48], wherein the antibody is an anti-CD33 antibody.
[95] The targeting molecule-drug conjugate according to [94], wherein the anti-CD33 antibody contains VH containing CDR1 consisting of the amino acid sequence set forth in SEQ ID NO: 115, CDR2 consisting of the amino acid sequence set forth in SEQ ID NO: 116, and CDR3 consisting of the amino acid sequence set forth in SEQ ID NO: 117, and VL containing CDR1 consisting of the amino acid sequence set forth in SEQ ID NO: 118, CDR2 consisting of the amino acid sequence set forth in SEQ ID NO: 119, and CDR3 consisting of the amino acid sequence set forth in SEQ ID NO: 120.
[96] The targeting molecule-drug conjugate according to [94] or [95], wherein the amino acid sequence of VH of the anti-CD33 antibody contains the amino acid sequence set forth in SEQ ID NO: 45, and the amino acid sequence of VL of the anti-CD33 antibody contains the amino acid sequence set forth in SEQ ID NO: 46.
[97] The targeting molecule-drug conjugate according to any of [94] to [96], wherein the amino acid sequence of VH of the anti-CD33 antibody contains an amino acid sequence having 90% or higher sequence identity to the amino acid sequence set forth in SEQ ID NO: 45, and the amino acid sequence of VL of the anti-CD33 antibody contains an amino acid sequence having 90% or higher sequence identity to the amino acid sequence set forth in SEQ ID NO: 46.
[98] The targeting molecule-drug conjugate according to [48], wherein the antibody is an anti-FoLRα antibody.
[99] The targeting molecule-drug conjugate according to [98], wherein the anti-FoLRα antibody contains VH containing CDR1 consisting of the amino acid sequence set forth in SEQ ID NO: 121, CDR2 consisting of the amino acid sequence set forth in SEQ ID NO: 122, and CDR3 consisting of the amino acid sequence set forth in SEQ ID NO: 123, and VL containing CDR1 consisting of the amino acid sequence set forth in SEQ ID NO: 124, CDR2 consisting of the amino acid sequence set forth in SEQ ID NO: 125, and CDR3 consisting of the amino acid sequence set forth in SEQ ID NO: 126.
[100] The targeting molecule-drug conjugate according to [98] or [99], wherein the amino acid sequence of VH of the anti-FoLRα antibody contains the amino acid sequence set forth in SEQ ID NO: 47, and the amino acid sequence of VL of the anti-FoLRα antibody contains the amino acid sequence set forth in SEQ ID NO: 48.
[101] The targeting molecule-drug conjugate according to any of [98] to [100], wherein the amino acid sequence of VH of the anti-FoLRα antibody contains an amino acid sequence having 90% or higher sequence identity to the amino acid sequence set forth in SEQ ID NO: 47, and the amino acid sequence of VL of the anti-FoLRα antibody contains an amino acid sequence having 90% or higher sequence identity to the amino acid sequence set forth in SEQ ID NO: 48.
[102] The targeting molecule-drug conjugate according to [48], wherein the antibody is an anti-CD25 antibody.
[103] The targeting molecule-drug conjugate according to [102], wherein the anti-CD25 antibody contains VH containing CDR1 consisting of the amino acid sequence set forth in SEQ ID NO: 127, CDR2 consisting of the amino acid sequence set forth in SEQ ID NO: 128, and CDR3 consisting of the amino acid sequence set forth in SEQ ID NO: 129, and VL containing CDR1 consisting of the amino acid sequence set forth in SEQ ID NO: 130, CDR2 consisting of the amino acid sequence set forth in SEQ ID NO: 131, and CDR3 consisting of the amino acid sequence set forth in SEQ ID NO: 132.
[104] The targeting molecule-drug conjugate according to [102] or [103], wherein the amino acid sequence of VH of the anti-CD25 antibody contains the amino acid sequence set forth in SEQ ID NO: 49, and the amino acid sequence of VL of the anti-CD25 antibody contains the amino acid sequence set forth in SEQ ID NO: 50.
[105] The targeting molecule-drug conjugate according to any of [102] to [104], wherein the amino acid sequence of VH of the anti-CD25 antibody contains an amino acid sequence having 90% or higher sequence identity to the amino acid sequence set forth in SEQ ID NO: 49, and the amino acid sequence of VL of the anti-CD25 antibody contains an amino acid sequence having 90% or higher sequence identity to the amino acid sequence set forth in SEQ ID NO: 50.
[106] The targeting molecule-drug conjugate according to [48], wherein the antibody is an anti-CADM1 antibody.
[107] The targeting molecule-drug conjugate according to [106], wherein the anti-CADM1 antibody contains VH containing CDR1 consisting of the amino acid sequence set forth in SEQ ID NO: 133, CDR2 consisting of the amino acid sequence set forth in SEQ ID NO: 134, and CDR3 consisting of the amino acid sequence set forth in SEQ ID NO: 135, and VL containing CDR1 consisting of the amino acid sequence set forth in SEQ ID NO: 136, CDR2 consisting of the amino acid sequence set forth in SEQ ID NO: 137, and CDR3 consisting of the amino acid sequence set forth in SEQ ID NO: 138.
[108] The targeting molecule-drug conjugate according to [106] or [107], wherein the amino acid sequence of VH of the anti-CADM1 antibody contains the amino acid sequence set forth in SEQ ID NO: 51, and the amino acid sequence of VL of the anti-CADM1 antibody contains the amino acid sequence set forth in SEQ ID NO: 52.
[109] The targeting molecule-drug conjugate according to any of [106] to [108], wherein the amino acid sequence of VH of the anti-CADM1 antibody contains an amino acid sequence having 90% or higher sequence identity to the amino acid sequence set forth in SEQ ID NO: 51, and the amino acid sequence of VL of the anti-CADM1 antibody contains an amino acid sequence having 90% or higher sequence identity to the amino acid sequence set forth in SEQ ID NO: 52.
[110] The targeting molecule-drug conjugate according to [48], wherein the antibody is an anti-CLL1 antibody.
[111] The targeting molecule-drug conjugate according to [110], wherein the anti-CLL1 antibody contains VH containing CDR1 consisting of the amino acid sequence set forth in SEQ ID NO: 139, CDR2 consisting of the amino acid sequence set forth in SEQ ID NO: 140, and CDR3 consisting of the amino acid sequence set forth in SEQ ID NO: 141, and VL containing CDR1 consisting of the amino acid sequence set forth in SEQ ID NO: 142, CDR2 consisting of the amino acid sequence set forth in SEQ ID NO: 143, and CDR3 consisting of the amino acid sequence set forth in SEQ ID NO: 144.
[112] The targeting molecule-drug conjugate according to [110] or [111], wherein the amino acid sequence of VH of the anti-CLL1 antibody contains the amino acid sequence set forth in SEQ ID NO: 53, and the amino acid sequence of VL of the anti-CLL1 antibody contains the amino acid sequence set forth in SEQ ID NO: 54.
[113] The targeting molecule-drug conjugate according to any of [110] to [112], wherein the amino acid sequence of VH of the anti-CLL1 antibody contains an amino acid sequence having 90% or higher sequence identity to the amino acid sequence set forth in SEQ ID NO: 53, and the amino acid sequence of VL of the anti-CLL1 antibody contains an amino acid sequence having 90% or higher sequence identity to the amino acid sequence set forth in SEQ ID NO: 54.
[114] The targeting molecule-drug conjugate according to [48], wherein the antibody is an anti-CD38 antibody.
[115] The targeting molecule-drug conjugate according to [114], wherein the anti-CD38 antibody contains VH containing CDR1 consisting of the amino acid sequence set forth in SEQ ID NO: 145, CDR2 consisting of the amino acid sequence set forth in SEQ ID NO: 146, and CDR3 consisting of the amino acid sequence set forth in SEQ ID NO: 147, and VL containing CDR1 consisting of the amino acid sequence set forth in SEQ ID NO: 148, CDR2 consisting of the amino acid sequence set forth in SEQ ID NO: 149, and CDR3 consisting of the amino acid sequence set forth in SEQ ID NO: 150.
[116] The targeting molecule-drug conjugate according to [114] or [115], wherein the amino acid sequence of VH of the anti-CD38 antibody contains the amino acid sequence set forth in SEQ ID NO: 55, and the amino acid sequence of VL of the anti-CD38 antibody contains the amino acid sequence set forth in SEQ ID NO: 56.
[117] The targeting molecule-drug conjugate according to any of [114] to [116], wherein the amino acid sequence of VH of the anti-CD38 antibody contains an amino acid sequence having 90% or higher sequence identity to the amino acid sequence set forth in SEQ ID NO: 55, and the amino acid sequence of VL of the anti-CD38 antibody contains an amino acid sequence having 90% or higher sequence identity to the amino acid sequence set forth in SEQ ID NO: 56.
[118] The targeting molecule-drug conjugate according to [48], wherein the antibody is an anti-CD74 antibody.
[119] The targeting molecule-drug conjugate according to [118], wherein the anti-CD74 antibody contains VH containing CDR1 consisting of the amino acid sequence set forth in SEQ ID NO: 151, CDR2 consisting of the amino acid sequence set forth in SEQ ID NO: 152, and CDR3 consisting of the amino acid sequence set forth in SEQ ID NO: 153, and VL containing CDR1 consisting of the amino acid sequence set forth in SEQ ID NO: 154, CDR2 consisting of the amino acid sequence set forth in SEQ ID NO: 155, and CDR3 consisting of the amino acid sequence set forth in SEQ ID NO: 156.
[120] The targeting molecule-drug conjugate according to [118] or [119], wherein the amino acid sequence of VH of the anti-CD74 antibody contains the amino acid sequence set forth in SEQ ID NO: 57, and the amino acid sequence of VL of the anti-CD74 antibody contains the amino acid sequence set forth in SEQ ID NO: 58.
[121] The targeting molecule-drug conjugate according to any of [118] to [120], wherein the amino acid sequence of VH of the anti-CD74 antibody contains an amino acid sequence having 90% or higher sequence identity to the amino acid sequence set forth in SEQ ID NO: 57, and the amino acid sequence of VL of the anti-CD74 antibody contains an amino acid sequence having 90% or higher sequence identity to the amino acid sequence set forth in SEQ ID NO: 58.
[122] The targeting molecule-drug conjugate according to [48], wherein the antibody is an anti-Nectin4 antibody.
[123] The targeting molecule-drug conjugate according to [122], wherein the anti-Nectin4 antibody contains VH containing CDR1 consisting of the amino acid sequence set forth in SEQ ID NO: 187, CDR2 consisting of the amino acid sequence set forth in SEQ ID NO: 188, and CDR3 consisting of the amino acid sequence set forth in SEQ ID NO: 189, and VL containing CDR1 consisting of the amino acid sequence set forth in SEQ ID NO: 190, CDR2 consisting of the amino acid sequence set forth in SEQ ID NO: 191, and CDR3 consisting of the amino acid sequence set forth in SEQ ID NO: 192.
[124] The targeting molecule-drug conjugate according to [122] or [123], wherein the amino acid sequence of VH of the anti-Nectin4 antibody contains the amino acid sequence set forth in SEQ ID NO: 183, and the amino acid sequence of VL of the anti-Nectin4 antibody contains the amino acid sequence set forth in SEQ ID NO: 184.
[125] The targeting molecule-drug conjugate according to any of [122] to [124], wherein the amino acid sequence of VH of the anti-Nectin4 antibody contains an amino acid sequence having 90% or higher sequence identity to the amino acid sequence set forth in SEQ ID NO: 183, and the amino acid sequence of VL of the anti-Nectin4 antibody contains an amino acid sequence having 90% or higher sequence identity to the amino acid sequence set forth in SEQ ID NO: 184.
[126] The targeting molecule-drug conjugate according to [48], wherein the antibody is an anti-TROP2 antibody.
[127] The targeting molecule-drug conjugate according to [126], wherein the anti-TROP2 antibody contains VH containing CDR1 consisting of the amino acid sequence set forth in SEQ ID NO: 193, CDR2 consisting of the amino acid sequence set forth in SEQ ID NO: 194, and CDR3 consisting of the amino acid sequence set forth in SEQ ID NO: 195, and VL containing CDR1 consisting of the amino acid sequence set forth in SEQ ID NO: 196, CDR2 consisting of the amino acid sequence set forth in SEQ ID NO: 197, and CDR3 consisting of the amino acid sequence set forth in SEQ ID NO: 198.
[128] The targeting molecule-drug conjugate according to [126] or [128], wherein the amino acid sequence of VH of the anti-TROP2 antibody contains the amino acid sequence set forth in SEQ ID NO: 185, and the amino acid sequence of VL of the anti-TROP2 antibody contains the amino acid sequence set forth in SEQ ID NO: 186.
[129] The targeting molecule-drug conjugate according to any of [126] to [128], wherein the amino acid sequence of VH of the anti-TROP2 antibody contains an amino acid sequence having 90% or higher sequence identity to the amino acid sequence set forth in SEQ ID NO: 185, and the amino acid sequence of VL of the anti-TROP2 antibody contains an amino acid sequence having 90% or higher sequence identity to the amino acid sequence set forth in SEQ ID NO: 186.
[130] The targeting molecule-drug conjugate according to any of [48] to [129], wherein an amino acid sequence of a heavy chain constant region (hereinafter, also referred to as "CH") of the antibody contains
   (a) the amino acid sequence set forth in SEQ ID NO: 1, or
   (b) an amino acid sequence having 80% or higher sequence identity to the amino acid sequence set forth in SEQ ID NO: 1.
[131] The targeting molecule-drug conjugate according to [130], wherein the amino acid sequence (b) is an amino acid sequence derived from the amino acid sequence set forth in SEQ ID NO: 1 by the substitution of at least one amino acid by cysteine.
[132] The targeting molecule-drug conjugate according to any of [48] to [129], wherein the amino acid sequence of CH of the antibody contains the amino acid sequence set forth in SEQ ID NO: 2.
[133] The targeting molecule-drug conjugate according to [132], wherein the antibody is connected to linker L via a sulfur atom of cysteine at position 239 of the heavy chain.
[134] The targeting molecule-drug conjugate according to any of [48] to [133], wherein an amino acid sequence of a light chain constant region (hereinafter, also referred to as "CL") of the antibody contains
   (e) the amino acid sequence set forth in SEQ ID NO: 3, or
   (f) an amino acid sequence having 80% or higher sequence identity to the amino acid sequence set forth in SEQ ID NO: 3.
[135] The targeting molecule-drug conjugate according to [134], wherein the amino acid sequence (f) is an amino acid sequence derived from the amino acid sequence set forth in SEQ ID NO: 3 by the substitution of at least one amino acid by cysteine.
[136] The targeting molecule-drug conjugate according to any of [48] to [135], wherein the amino acid sequence of CL of the antibody contains the amino acid sequence set forth in SEQ ID NO: 4.
[137] The targeting molecule-drug conjugate according to [136], wherein the antibody is connected to linker L via a sulfur atom of cysteine at position 124 of the light chain.
[138] A therapeutic agent for cancer, containing the targeting molecule-drug conjugate according to any of [32] to [137] as an active ingredient.
[139] The agent according to [138], wherein the cancer is B cell lymphoma.
[140] A method for treating cancer, including administering the targeting molecule-drug conjugate according to any of [32] to [137] to a subject.
[141] The method according to [140], wherein the cancer is B cell lymphoma.
[142] The targeting molecule-drug conjugate according to any of [32] to [137] for use in the treatment of cancer.
[143] The targeting molecule-drug conjugate according to [142], wherein the cancer is B cell lymphoma.
[144] Use of the targeting molecule-drug conjugate according to any of [32] to [137] for the production of a drug for the treatment of cancer.
[145] The use according to [144], wherein the cancer is B cell lymphoma.
[146] A drug-linker conjugate comprising a drug and a linker linked to the drug, wherein
   the drug is the compound or the pharmaceutically acceptable salt thereof according to any of [1] to [23].
[147] The drug-linker conjugate according to [146], wherein the drug-linker conjugate is represented by the following formula (V): 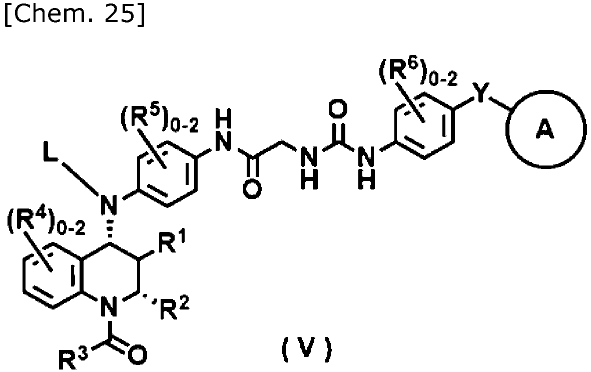 (wherein,
   R¹ and R² are the same or different and each represents a hydrogen atom or lower alkyl,
   R³ represents lower alkyl,
   R⁴ represents halogen,
   R⁵ represents halogen,
   R⁶ represents halogen or lower alkoxy,
   Y represents -O-CH₂-CH₂-, -CH₂-CH₂-CH₂-, -CH=CH-CH₂-, or - NH-C(=O)-CH₂-,
   ring A represents
      (i) a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
      (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
      (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, and
   L represents a linker).
[148] The compound or the pharmaceutically acceptable salt thereof according to [147], wherein (i) the aliphatic heterocyclic group in ring A is a nitrogen-containing aliphatic heterocyclic group.
[149] The drug-linker conjugate according to [147] or [148],
   wherein ring A represented by (i) the aliphatic heterocyclic group is the following ring A': 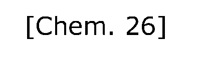 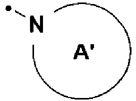
   (wherein, when ring A' has a substituent, the substituent of ring A' is as defined for the substituent of ring A).
[150] The drug-linker conjugate according to any of [146] to [149], wherein the drug-linker conjugate is represented by the following formula (V'): 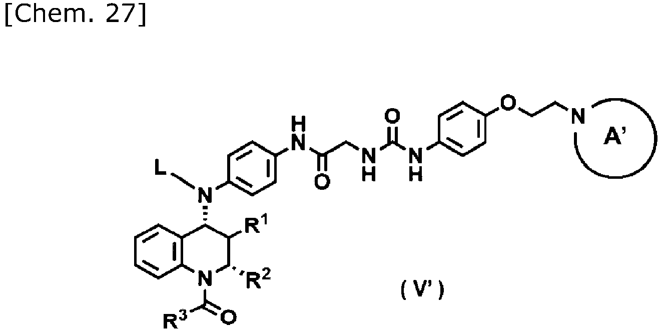 (wherein,
   R¹, R², R³, and L are as defined in the formula (V), and
   ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
[151] The drug-linker conjugate according to [147], wherein the drug-linker conjugate is represented by the following formula (Va): 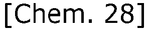 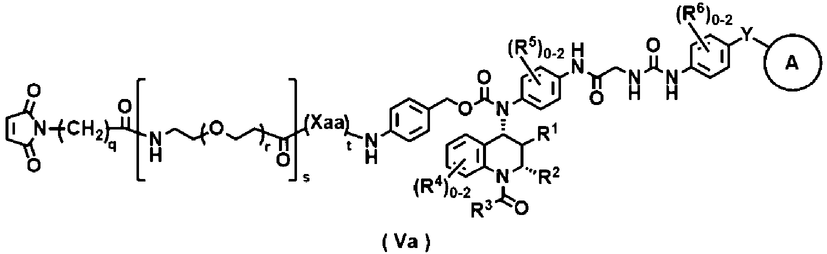 (wherein,
   R¹, R², R³, R⁴, R⁵, R⁶, Y, and ring A are as defined above, and
   each Xaa independently represents
      (a) a residue of at least one amino acid selected from the group consisting of valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents
      (b) a peptide mimetic represented by the following formula (IV): 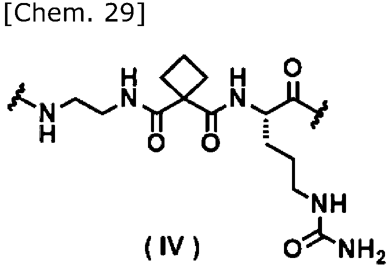 wherein,
         q represents an integer of 1 to 5,
         r represents an integer of 2 to 24,
         s represents 0 or 1, and
         t represents 1, 2, 3, or 4).
[152]The compound or the pharmaceutically acceptable salt thereof according to [151], wherein (i) the aliphatic heterocyclic group in ring A is a nitrogen-containing aliphatic heterocyclic group.
[153]The drug-linker conjugate according to [151] or [152], wherein ring A represented by (i) the aliphatic heterocyclic group is the following ring A': 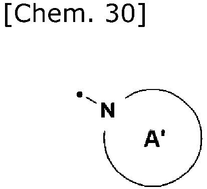 (wherein, when ring A' has a substituent, the substituent of ring A' is as defined for the substituent of ring A).
[154]The drug-linker conjugate according to any of [146] to [153], wherein the drug-linker conjugate is represented by the following formula (V'a): 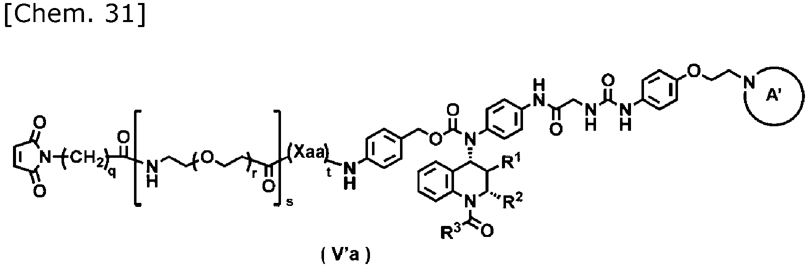 (wherein,
   R¹, R², R³, Xaa, q, r, s, and t are as defined in the formula (Va), and
   ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
[155] The drug-linker conjugate according to any of [151] to [154], wherein each Xaa is independently a residue of an amino acid selected from valine, alanine, citrulline, glycine, phenylalanine, and glutamic acid, or is a peptide mimetic represented by the following formula (IV): 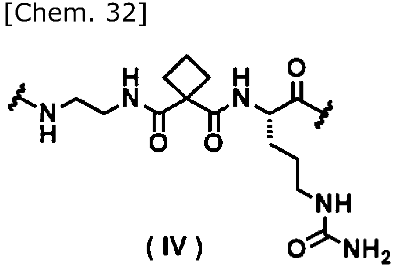
[156] The drug-linker conjugate according to any of [151] to [155], wherein each Xaa is independently a residue of an amino acid selected from valine and alanine.
[157] The drug-linker conjugate according to any of [151] to [156], wherein t is 2.
[158] The drug-linker conjugate according to any of [151] to [157], wherein r is 6 or 12.
[159] The drug-linker conjugate according to any of [151] to [158], wherein r is 12.
[160] The drug-linker conjugate according to any of [151] to [159], wherein q is 2 or 5.
[161] The drug-linker conjugate according to any of [151] to [160], wherein q is 2.
[162] The drug-linker conjugate according to any of [151] to [161], wherein s is 1.
   is provided.

The compound represented by the formula (I) or the pharmaceutically acceptable salt thereof is a divalent BET degrader and can avoid the safety hazard, i.e., peripheral neurotoxicity, while maintaining high antitumor activity. The compound represented by the formula (I) or the pharmaceutically acceptable salt thereof is also useful as a payload (drug) in a targeting molecule-drug conjugate such as an antibody-drug conjugate (ADC).

The targeting molecule-drug conjugate containing the compound represented by the formula (I) or the pharmaceutically acceptable salt thereof as a drug can avoid the safety hazard, i.e., peripheral neurotoxicity, while maintaining high antitumor activity.

### [Brief Description of Drawings]

[Figure 1] Figure 1 is a diagram showing results of Test Example 4 (*in vivo* drug efficacy evaluation of compound and ADC).
[Figure 2] Figure 2 is a diagram showing results of Test Example 5 (BET protein degradation evaluation by Western blotting (WB) using SU-DHL-4 cell line).

### [Mode for Carrying Out the Invention]

Hereinafter, the present invention will be described. As used herein, the phrase "value A to value B" means the value A or more and the value B or less. Two or more embodiments described in the present specification can be combined, and such a combination of two or more embodiments is also encompassed by the present invention.

### <<Description of term>>

Hereinafter, terms used in the present specification will be described. The following description is applied to the entire present specification unless otherwise specified.

### Oxo

The oxo is a group represented by the formula: =O.

### Lower alkyl

The lower alkyl is, for example, linear or branched alkyl having 1 to 10 carbon atoms. Examples of the lower alkyl include methyl, ethyl, n-propyl, isopropyl, n-butyl, isobutyl, sec-butyl, tert-butyl, n-pentyl, isopentyl, neopentyl, hexyl, heptyl, octyl, nonyl, and decyl.

### Hydroxy-lower alkyl

The hydroxy-lower alkyl is lower alkyl in which one or more hydrogen atoms are replaced by hydroxy. The definition of the lower alkyl is as described above. The position or the number of hydroxy in the hydroxy-lower alkyl is not particularly limited. The number of hydroxy is, for example, 1 to 4, preferably 1 to 3, more preferably 1 or 2, further preferably 1. Examples of the hydroxy-lower alkyl include hydroxymethyl, 1-hydroxyethyl, 2-hydroxyethyl, 1-hydroxypropyl, 2-hydroxypropyl, 1,2-dihydroxyethyl, and 1,2-dihydroxypropyl.

### Lower alkylcarbonyl

The lower alkylcarbonyl is a group represented by the formula: -CO-lower alkyl. The definition of the lower alkyl is as described above. Examples of the lower alkylcarbonyl include methylcarbonyl(acetyl), ethylcarbonyl, n-propylcarbonyl, isopropylcarbonyl, n-butylcarbonyl, isobutylcarbonyl, sec-butylcarbonyl, tert-butylcarbonyl, n-pentylcarbonyl, isopentylcarbonyl, neopentylcarbonyl, hexylcarbonyl, heptylcarbonyl, octylcarbonyl, nonylcarbonyl, and decylcarbonyl.

### Hydroxy-lower alkylcarbonyl

The hydroxy-lower alkylcarbonyl is a group represented by the formula: -CO-hydroxy-lower alkyl. The definition of the hydroxy-lower alkyl is as described above. Examples of the hydroxy-lower alkylcarbonyl include hydroxymethylcarbonyl, 1-hydroxyethylcarbonyl, 2-hydroxyethylcarbonyl, 1-hydroxypropylcarbonyl, 2-hydroxypropylcarbonyl, 1,2-dihydroxyethylcarbonyl, and 1,2-dihydroxypropylcarbonyl.

### Cycloalkylcarbonyl

The cycloalkylcarbonyl is a group represented by the formula: -CO-cycloalkyl. The number of carbon atoms in the cycloalkyl is, for example, 3 to 10. Examples of the cycloalkylcarbonyl include cyclopropylcarbonyl, cyclobutylcarbonyl, cyclopentylcarbonyl, cyclohexylcarbonyl, cycloheptylcarbonyl, cyclooctylcarbonyl, cyclononylcarbonyl, and cyclodecylcarbonyl.

### Halogen

The halogen is each of a fluorine atom, a chlorine atom, a bromine atom, and an iodine atom.

### Lower alkoxy

The lower alkoxy is, for example, linear or branched alkoxy having 1 to 10 carbon atoms, preferably alkoxy having 1 to 5 carbon atoms, more preferably alkoxy having 1 to 3 carbon atoms. Examples of the lower alkoxy include methoxy, ethoxy, propyloxy, i-propyloxy, n-butoxy, i-butoxy, s-butoxy, and t-butoxy, with methoxy being preferred.

### Lower alkoxycarbonyl

The lower alkoxycarbonyl is a group represented by the formula: -CO-lower alkoxy. The definition of the lower alkoxy is as described above. The lower alkoxycarbonyl is, for example, linear or branched alkoxycarbonyl having 1 to 11 carbon atoms, preferably alkoxycarbonyl having 1 to 5 carbon atoms, more preferably alkoxycarbonyl having 1 to 3 carbon atoms. Examples of the lower alkoxycarbonyl include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentoxycarbonyl, isopentoxycarbonyl, and neopentoxycarbonyl, with methoxycarbonyl and ethoxycarbonyl being preferred.

### Lower alkylcarbamoyl

The lower alkylcarbamoyl is a group represented by the formula: -CONH-lower alkyl. The definition of the lower alkyl is as described above. The lower alkylcarbamoyl is, for example, linear or branched alkylcarbamoyl having 1 to 11 carbon atoms, preferably alkylcarbamoyl having 1 to 5 carbon atoms, more preferably alkylcarbamoyl having 1 to 3 carbon atoms. Examples of the lower alkylcarbamoyl include methylcarbamoyl, ethylcarbamoyl, dimethylcarbamoyl, and diethylcarbamoyl, with methylcarbamoyl and dimethylcarbamoyl being preferred.

### Aliphatic heterocyclic group

The aliphatic heterocyclic group contains, as ring-constituting elements, a carbon atom as well as one or more non-carbon heteroatoms selected from the group consisting of a nitrogen atom, an oxygen atom, and a sulfur atom. The number of heteroatoms in the aliphatic heterocyclic group is, for example, 1 to 3, preferably 1 or 2, more preferably 2. The number of carbon atoms in the aliphatic heterocyclic group is appropriately determined depending on the number of heteroatoms and the number of members in the aliphatic heterocyclic group.

Two carbon atoms constituting a ring in the aliphatic heterocyclic group may be bridged by an alkylene group. The number of carbon atoms in the alkylene group that bridges two carbon atoms constituting a ring is, for example, 1 to 3, preferably 1 or 2, further preferably 1.

The aliphatic heterocyclic group may be constituted only by saturated bonds or may contain a double bond and is preferably constituted only by saturated bonds.

The aliphatic heterocyclic group may be monocyclic or may be fused polycyclic. The fused polycyclic group is, for example, a fused bicyclic or fused tricyclic group, preferably a fused bicyclic group.

The number of members in the monocyclic aliphatic heterocyclic group is, for example, 3 to 8, preferably 4 to 8, more preferably 5 to 7, further preferably 6 or 7.

Examples of the monocyclic aliphatic heterocyclic group include aliphatic heterocyclic groups containing only a nitrogen atom as a heteroatom, such as aziridinyl, azetidinyl, pyrrolidinyl, pyrrolinyl, imidazolidinyl, imidazolinyl, pyrazolidinyl, pyrazolinyl, piperidinyl, piperazinyl, tetrahydropyridinyl, dihydropyridinyl, dihydrothiopyranyl, tetrahydropyrimidinyl, tetrahydropyridazinyl, azepanyl, 1,4-diazepanyl, azocanyl, and diazocanyl. Examples of the aliphatic heterocyclic group containing a nitrogen atom and an oxygen atom as heteroatoms include oxazolidinyl, oxazolinyl, and morpholinyl. Examples of the aliphatic heterocyclic group containing a nitrogen atom and a sulfur atom as heteroatoms include thiazolidinyl, thiazolinyl, and thiomorpholinyl. Piperazinyl or 1,4-diazepanyl is preferred, with piperazinyl being more preferred.

Examples of the fused polycyclic aliphatic heterocyclic group include aliphatic heterocyclic groups containing only a nitrogen atom as a heteroatom, such as bicyclic or tricyclic nitrogen-containing aliphatic heterocyclic groups with 3- to 8-membered rings fused, specifically, hexahydropyrrolopyrazinyl, octahydropyridopyrazinyl, octahydropyrrolodiazepanyl, decahydropyridodiazepanyl, and decahydroazapinodiazepanyl.

### Nitrogen-containing aliphatic heterocyclic group

The nitrogen-containing aliphatic heterocyclic group is an aliphatic heterocyclic group containing a carbon atom as well as one or more nitrogen atoms as ring-constituting elements. The number of nitrogen atoms in the nitrogen-containing aliphatic heterocyclic group is, for example, 1 to 3, preferably 1 or 2, more preferably 2. The number of carbon atoms in the nitrogen-containing aliphatic heterocyclic group is appropriately determined depending on the number of heteroatoms and the number of members in the nitrogen-containing aliphatic heterocyclic group. The bonding hand of the nitrogen-containing aliphatic heterocyclic group is not particularly limited and, this bonding hand preferably has a nitrogen atom. The nitrogen-containing aliphatic heterocyclic group may contain one or more non-nitrogen heteroatoms selected from the group consisting of an oxygen atom and a sulfur atom. When the nitrogen-containing aliphatic heterocyclic group contains a non-nitrogen heteroatom, the number of non-nitrogen heteroatoms is, for example, 1 or 2, preferably 1.

Two carbon atoms constituting a ring in the nitrogen-containing aliphatic heterocyclic group may be bridged by an alkylene group. The number of carbon atoms in the alkylene group that bridges two carbon atoms constituting a ring is, for example, 1 to 3, preferably 1 or 2, further preferably 1.

The nitrogen-containing aliphatic heterocyclic group may be constituted only by saturated bonds or may contain a double bond and is preferably constituted only by saturated bonds.

The nitrogen-containing aliphatic heterocyclic group may be monocyclic or may be fused polycyclic. The fused polycyclic group is, for example, a fused bicyclic or fused tricyclic group, preferably a fused bicyclic group.

The number of members in the monocyclic nitrogen-containing aliphatic heterocyclic group is, for example, 3 to 8, preferably 4 to 8, more preferably 5 to 7, further preferably 6 or 7.

Examples of the monocyclic nitrogen-containing aliphatic heterocyclic group include aziridinyl, azetidinyl, pyrrolidinyl, pyrrolinyl, imidazolidinyl, imidazolinyl, oxazolidinyl, oxazolinyl, pyrazolidinyl, pyrazolinyl, thiazolidinyl, thiazolinyl, piperidinyl, piperazinyl, tetrahydropyridinyl, dihydropyridinyl, dihydrothiopyranyl, tetrahydropyrimidinyl, tetrahydropyridazinyl, morpholinyl, thiomorpholinyl, azepanyl, 1,4-diazepanyl, azocanyl, and diazocanyl. Piperazinyl or 1,4-diazepanyl is preferred, with piperazinyl being more preferred.

Examples of the fused polycyclic nitrogen-containing aliphatic heterocyclic group include bicyclic or tricyclic nitrogen-containing aliphatic heterocyclic groups with 3- to 8-membered rings fused, specifically, hexahydropyrrolopyrazinyl, octahydropyridopyrazinyl, octahydropyrrolodiazepanyl, decahydropyridodiazepanyl, and decahydroazapinodiazepanyl.

### Aromatic heterocyclic group

The aromatic heterocyclic group contains, as ring-constituting elements, a carbon atom as well as one or more non-carbon heteroatoms selected from the group consisting of a nitrogen atom, an oxygen atom, and a sulfur atom. The number of heteroatoms in the aromatic heterocyclic group is, for example, 1 to 3, preferably 1 or 2. The number of carbon atoms in the aromatic heterocyclic group is appropriately determined depending on the number of heteroatoms and the number of members in the aromatic heterocyclic group.

The aromatic heterocyclic group may be monocyclic or may be fused polycyclic. The fused polycyclic group is, for example, a fused bicyclic or fused tricyclic group, preferably a fused bicyclic group.

The number of members in the aromatic heterocyclic group is, for example, 5 to 10, preferably 5 to 9, more preferably 5 or 6.

Examples of the aromatic heterocyclic group include 5-membered aromatic heterocyclic groups such as pyrrolyl, furanyl, thienyl, pyrazolyl, imidazolyl, triazolyl, tetrazolyl, oxazolyl, isoxazolyl, thiazolyl, isothiazolyl, oxadiazolyl, and thiadiazolyl; 6-membered aromatic heterocyclic groups such as pyridyl, pyridazinyl, pyrimidinyl, pyrazinyl, triazinyl, and tetrazinyl; 9-membered aromatic heterocyclic groups such as indazolyl, indolizinyl, imidazopyridyl, and 1,3-benzodioxolyl; and 10-membered aromatic heterocyclic groups such as quinolyl, isoquinolyl, quinazolinyl, naphthyridinyl, benzopyranyl, and dihydrobenzopyranyl. Oxazolyl or pyridyl is preferred.

### Aryl

The aryl (also referred to as an aryl group) means a monovalent aromatic hydrocarbon group obtained by removing one hydrogen atom from an aromatic carbocyclic ring. The number of carbon atoms in the aryl is not particularly limited and is, for example, 6 to 18, preferably, 6 to 10.

The aryl may be monocyclic or may be fused polycyclic. The fused polycyclic group is, for example, a fused bicyclic or fused tricyclic group, preferably a fused bicyclic group.

Examples of the aryl include phenyl, 1-naphthyl, and 2-naphthyl. Phenyl is preferred.

### Antibody

The antibody (immunoglobulin) is a glycoprotein made up of two heavy chains (hereinafter, also referred to as "H chains") and two light chains (hereinafter, also referred to as "L chains"). The antibody has five classes IgG, IgM, IgA, IgD, and IgE. The basic structure of the antibody is common among the classes, and two heavy chains and two light chains are joined through disulfide bonds and noncovalent bonds to form a Y-shaped four-chain structure.

Domains positioned at the N termini of both the heavy chain and the light chain are called variable regions, which are involved in the binding specificity of the antibody for an antigen. The amino acid sequences of C-terminal domains downstream of the variable regions are called constant regions (hereinafter, also referred to as "C regions"). The heavy chain has a heavy chain variable region (VH) and a heavy chain constant region (CH) from the N terminus toward the C terminus. CH is further divided into three domains, a CH1 domain, a CH2 domain, and a CH3 domain, in order from the N terminus. The light chain has a light chain variable region (VL) and a light chain constant region (CL) from the N terminus toward the C terminus.

VH and VL each have three complementarity determining regions (CDRs), i.e., CDR1, CDR2, and CDR3. Since CDRs determine specificity for an antigen, these regions largely differ in amino acid sequence among antibodies and are also called hypervariable domains. Moieties other than CDRs in the variable region are called framework regions (FRs) and relatively low vary in amino acid sequence. CDR1, CDR2, and CDR3 residing in VH are also called CDRH1, CDRH2, and CDRH3, respectively, and CDR1, CDR2, and CDR3 residing in VL are also called CDRL1, CDRL2, and CDRL3, respectively.

In the present specification, amino acid positions assigned to CDRs and FRs are defined according to the Kabat numbering (see Sequences of Proteins of Immunological Interest (National Institute of Health, Bethesda, Md., (1987) and (1991)).

In the present specification, amino acid positions in the constant regions are defined according to the EU numbering of Kabat (see Sequences of proteins of immunological interest, NIH Publication No. 91-3242).

The antibody may be a polyclonal antibody or may be a monoclonal antibody and is preferably a monoclonal antibody.

Examples of the monoclonal antibody include antibodies produced by hybridomas, and gene recombinant antibodies produced by transformants obtained by transformation with expression vectors containing antibody genes.

The monoclonal antibody is an antibody secreted by a single clone of an antibody-producing cell and recognizes only one epitope (also called antigenic determinant). The amino acid sequence (primary structure) constituting the monoclonal antibody is homogeneous.

The hybridoma can be prepared, for example, by inducing an antibody-producing cell having antigen specificity using an animal immunized with an antigen, and fusing the antibody-producing cell with a myeloma cell. The monoclonal antibody can be obtained by culturing the hybridoma or administering the hybridoma cells to an animal to cause ascites cancer in the animal, and separating and purifying the monoclonal antibody from the culture solution or the ascitic fluid. Any animal can be used as the animal to be immunized with the antigen as long as the animal is capable of producing the hybridoma. Preferably, a mouse, a rat, a hamster, a chicken, a rabbit, or the like is used. The monoclonal antibody may be an antibody produced by a hybridoma produced by obtaining a cell having the ability to produce the antibody from the animal, and immunizing the cell *in vitro*, followed by fusion with a myeloma cell.

Examples of the gene recombinant antibody include antibodies produced by gene recombination, such as human-type chimeric antibodies, humanized antibodies, and human antibodies.

The human-type chimeric antibody is an antibody made up of VH and VL of a nonhuman animal antibody and CH and CL of a human antibody. The human-type chimeric antibody can be produced by obtaining DNAs encoding VH and VL of a nonhuman animal antibody, and respectively inserting the DNAs to expression vectors for animal cells having DNAs encoding CH and CL of a human antibody to construct a human-type chimeric antibody expression vector, which is then transferred to animal cells for expression.

CH of the human-type chimeric antibody may be any CH that belongs to a human immunoglobulin (hereinafter, also referred to as "hIg"). For example, CH of a hIgG class, specifically, CH of a hIgG subclass such as hIgG1, hIgG2, hIgG3, or hIgG4, or a variant thereof can be used. CL of the human-type chimeric antibody may be any CL that belongs to hIg. For example, CL of a human κ chain or a human λ chain can be used.

Examples of the humanized antibody include human-type CDR-grafted antibodies and humanized antibodies based on a resurfacing technique. The humanized antibody also includes an antibody produced by a combined method of methods for producing these humanized antibodies. The humanized antibody also includes an antibody that consists of an amino acid sequence derived by the deletion, substitution, insertion, or addition of one or more amino acids from the amino acid sequence of the humanized antibody designed by such a method, and binds (preferably, specifically binds) to the antigen of interest.

The human-type CDR-grafted antibody is an antibody wherein the amino acid sequences of nonhuman animal antibody VH and VL CDRs were grafted at proper positions of human antibody VH and VL. The human-type CDR-grafted antibody can be produced as follows: DNAs encoding V regions in which the amino acid sequences of VH and VL CDRs of a nonhuman animal monoclonal antibody that binds (preferably, specifically binds) to the antigen of interest are grafted in VH and VL framework regions (FRs) of a freely-selected human antibody, are constructed. Subsequently, the DNAs are respectively inserted to expression vectors for animal cells having DNAs encoding human antibody CH and CL to construct a human-type CDR-grafted antibody expression vector, which is then transferred to animal cells for expression.

The humanized antibody based on the resurfacing technique is an antibody in which, among the amino acids of nonhuman animal antibody variable regions, an amino acid residue of FR considered to have no influence on the binding activity of the antibody is substituted with an amino acid residue considered to reduce antigenicity by the resurfacing method (Proc. Natl. Acad. Sci. USA 1994,91 (3): 969-73 and Protein Engineering 1996, 10, 895-90). The humanized antibody based on the resurfacing technique can be produced as follows: DNAs encoding V regions in which a freely-selected amino acid residue in FRs of a nonhuman animal monoclonal antibody that binds (preferably, specifically binds) to the antigen of interest is substituted with another amino acid residue, are constructed. Subsequently, the DNAs are respectively inserted to expression vectors for animal cells having DNAs encoding human antibody CH and CL to construct a humanized antibody expression vector based on the resurfacing technique, which is then transferred to animal cells for expression.

CH of the humanized antibody may be any CH that belongs to a human immunoglobulin (hereinafter, also referred to as "hIg"). For example, CH of a hIgG class, specifically, CH of a hIgG subclass such as hIgG1, hIgG2, hIgG3, or hIgG4, or a variant thereof can be used. CL of the humanized antibody may be any CL that belongs to hIg. For example, CL of a human κ chain or a human λ chain can be used.

The human antibody originally means a naturally occurring antibody in the human body. The human antibody also includes, for example, antibodies obtained from a human antibody phage library and a human antibody-producing transgenic animal produced through recent advance in genetic engineering, cellular engineering, and developmental engineering techniques.

As for the naturally occurring antibody in the human body, for example, lymphocytes producing the antibody can be cultured by isolating human peripheral blood lymphocytes, immortalizing the lymphocytes by infection with EB virus or the like, and cloning the lymphocytes, and the antibody can be purified from the culture supernatant.

The human antibody phage library is a library allowed to express antibody fragments such as Fab or scFv on phage surface by inserting antibody genes prepared from human B cells or B cells of a human antibody-producing transgenic animal to phage genes. A phage expressing an antibody fragment having the desired antigen binding activity on the surface can be recovered from the library by using binding activity against an antigen-immobilized substrate as an index. The antibody fragment may be further converted to a human antibody molecule made up of two complete H chains and two complete L chains by a genetic engineering approach.

The human antibody-producing transgenic animal means an animal with a human antibody gene incorporated in cells. Specifically, for example, a human antibody-producing transgenic mouse can be produced by transferring a human antibody gene to mouse ES cells, and transplanting the ES cells to mouse early embryos, followed by development. The human antibody from the human antibody-producing transgenic animal can be produced by using a hybridoma production method that is usually used for nonhuman animals, obtaining human antibody-producing hybridomas, and culturing the hybridomas so that the human antibody is produced and accumulated in the culture supernatant. The human antibody gene can be isolated by isolating B cells of the human antibody-producing transgenic animal, and cloning the B cells.

### Antigen binding fragment of antibody

The antigen binding fragment of the antibody is a fragment of an antibody having binding activity against the antigen of interest. The antigen binding fragment of the antibody is also called functional fragment of the antibody. Examples of the antigen binding fragment of the antibody include Fab, F(ab')₂, Fab', a single-chain variable fragment (scFv), a dimerized V region (diabody), a disulfide-stabilized variable fragment (dsFv), and peptides containing CDRs.

Fab is a fragment obtained by the papain treatment of the antibody and is a fragment that has about the half on the N-terminal side of the heavy chain and the whole L chain joined through a disulfide bond with a molecular weight of approximately 50000 and has antigen binding activity. Fab can be obtained by the papain treatment of the antibody. Also, Fab can be produced by inserting DNA encoding Fab of the antibody to an expression vector for a prokaryote or an expression vector for a eukaryote, and transferring the vector to a prokaryote or a eukaryote for expression.

F(ab')₂ is obtained through pepsin which degrades the antibody at a site below the disulfide bonds in the hinge region, and is a fragment that is constituted by two Fab regions joined at the hinge moiety with a molecular weight of approximately 100000 and has antigen binding activity. F(ab')₂ can be obtained by the pepsin treatment of the antibody. Also, F(ab')₂ can be produced by joining Fab' units through thioether bonds or disulfide bonds.

Fab' is obtained by the cleavage of the disulfide bonds in the hinge region of F(ab')₂, and is a fragment with a molecular weight of approximately 50000 having antigen binding activity. Fab' can be obtained by the treatment of F(ab')₂ with a reducing agent such as dithiothreitol. Also, Fab' can be produced by inserting DNA encoding Fab' to an expression vector for a prokaryote or an expression vector for a eukaryote, and transferring the vector to a prokaryote or a eukaryote for expression.

scFv is a fragment that has one VH and one VL linked through an appropriate peptide linker (hereinafter, also referred to as "P") and has antigen binding activity, and is represented by VH-P-VL or VL-P-VH.

scFv can be produced by constructing DNA encoding scFv using DNAs encoding VH and VL, inserting the DNA to an expression vector for a prokaryote or an expression vector for a eukaryote, and transferring the expression vector to a prokaryote or a eukaryote for expression.

Diabody is a dimerized fragment of scFvs and has divalent antigen binding activity. The divalent antigen binding activity may be the same activity or different activities.

Diabody can be produced by constructing DNA encoding scFvs using DNAs encoding VH and VL so as to have an amino acid sequence length of 8 or less residues of a peptide linker, inserting the DNA to an expression vector for a prokaryote or an expression vector for a eukaryote, and transferring the expression vector to a prokaryote or a eukaryote for expression.

dsFv is a fragment in which polypeptides of VH and VL, one amino acid residue each of which is substituted with a cysteine residue, are joined via a disulfide bond between the cysteine residues. The amino acid residues to be substituted with a cysteine residue can be selected on the basis of the conformational prediction of the antibody according to a known method [Protein Engineering, 7, 697 (1994)].

dsFv can be produced by constructing DNA encoding dsFv using cDNAs encoding VH and VL, inserting the DNA to an expression vector for a prokaryote or an expression vector for a eukaryote, and transferring the expression vector to a prokaryote or a eukaryote for expression.

The peptide containing CDRs is constituted so as to contain at least one or more CDR regions of VH or VL. A plurality of peptides containing CDRs can be joined directly or via an appropriate peptide linker.

The peptide containing CDRs can be produced by constructing DNAs encoding VH and VL CDRs, inserting the DNAs to an expression vector for a prokaryote or an expression vector for a eukaryote, and transferring the expression vector to a prokaryote or a eukaryote for expression. Also, the peptide containing CDRs may be produced by a chemical synthesis technique such as an Fmoc technique or a Boc technique.

### Bispecific antibody and multispecific antibody

The antibody or the antigen binding fragment thereof may have binding specificity for one antigen or may have binding specificity for two or more different antigens. In the present specification, the antibody or the antigen binding fragment thereof having binding specificity for two or more different antigens is referred to as a multispecific antibody. The multispecific antibody is, for example, an antibody or an antigen binding fragment thereof having binding specificity for two different antigens (i.e., a bispecific antibody) and may be an antibody or an antigen binding fragment thereof having binding specificity for three or more different antigens.

Examples of the format of the multispecific antibody include formats described in WO 2009/131239, WO 2014/054804, WO 2001/077342, WO 2007/024715, Wu et al., Nature Biotechnology, 2007, 25 (11), p.1290-1297, Labrijn et al., PNAS 2013, vol.110, no.13, p.5145-5150, Jong et al., PLoS Biology 2016, vol. 14, no.1, e1002344, Kontermann et al., mAbs 2012, vol.4, issue 2, p.182-197, Spiess et al., Molecular Immunology 67 (2015) 95-106, Ridgway et al., Protein engineering, 1996 vol. 9 no.7 p.617-621, WO 2009/080251, WO 2010/151792, and WO 2014/033074. The multispecific antibody can be prepared by use of methods described in these documents.

### Deletion, substitution, insertion, or addition of amino acid

The deletion, substitution, insertion, or addition of an amino acid as to a predetermined amino acid sequence can be performed by a well-known technique such as site-directed mutagenesis [Molecular Cloning 2nd Edition, Cold Spring Harbor Laboratory Press (1989), Current Protocols inmolecular Biology, John Wiley & Sons (1987-1997), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985)].

The deletion, substitution, insertion, or addition of an amino acid as to a predetermined amino acid sequence encompasses the case where two or more events selected from deletion, substitution, insertion, and addition occur.

The amino acid residue to be substituted, inserted, or added may be a natural form or may be a non-natural form. Examples of the natural amino acid include L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, and L-cysteine.

Hereinafter, preferred examples of interchangeably substitutable amino acids will be shown. The amino acids included in the same group can be substituted with each other.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

### Sequence identity

The term "%" used for the sequence identity between two amino acid sequences means the percentage of the number of identical amino acids based on the total number of amino acids (including a gap) when two amino acid sequences are aligned so as to maximize the degree of match of amino acids.

### <<First aspect>>

A first aspect of the present invention provides a compound represented by the formula (I) given below or a pharmaceutically acceptable salt thereof. In the present specification, the compound represented by the formula (I) given below is also referred to as the "compound (I)". The compound (I) or the pharmaceutically acceptable salt thereof is a divalent BET degrader and can avoid the safety hazard, i.e., peripheral neurotoxicity, while maintaining high antitumor activity. The compound (I) or the pharmaceutically acceptable salt thereof is also useful as a payload (drug) in a targeting molecule-drug conjugate such as an antibody-drug conjugate (ADC).

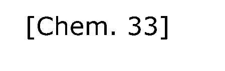

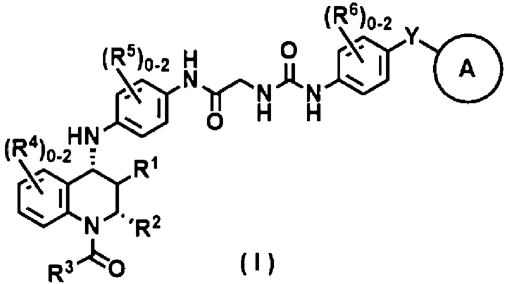

Examples of the pharmaceutically acceptable salt of the compound (I) include pharmaceutically acceptable acid addition salts, metal salts, ammonium salts, organic amine addition salts, and amino acid addition salts. Examples of the pharmaceutically acceptable acid addition salt of the compound (I) include inorganic acid salts such as hydrochloride, hydrobromide, nitrate, sulfate, and phosphate; and organic acid salts such as acetate, oxalate, maleate, fumarate, citrate, benzoate, and methanesulfonate. Examples of the pharmaceutically acceptable metal salt of the compound (I) include alkali metal salts such as sodium salt and potassium salt; alkaline earth metal salts such as magnesium salt and calcium salt; aluminum salt; and zinc salt. Examples of the pharmaceutically acceptable ammonium salt of the compound (I) include salts of ammonium, tetramethylammonium, and the like. Examples of the pharmaceutically acceptable organic amine addition salt of the compound (I) include addition salts of morpholine, piperidine, and the like. Examples of the pharmaceutically acceptable amino acid addition salt of the compound (I) include addition salts of lysine, glycine, phenylalanine, aspartic acid, glutamic acid, and the like.

The compound (I) or the pharmaceutically acceptable salt thereof may have stereoisomers such as geometrical isomers or optical isomers, tautomers, or the like. The present invention encompasses all possible isomers including these and mixtures thereof.

A part or all of atoms in the compound (I) or the pharmaceutically acceptable salt thereof may be replaced by corresponding isotope atoms. The present invention also encompasses the compounds substituted with these isotope atoms. For example, a part or all of hydrogen atoms in the compound (I) or the pharmaceutically acceptable salt thereof may be hydrogen atoms having an atomic weight of 2 (deuterium atoms).

A compound obtained by replacing a part or all of atoms in the compound (I) or the pharmaceutically acceptable salt thereof with the corresponding isotope atoms can be produced by a method similar to each of production methods mentioned later using commercially available building blocks. A compound obtained by replacing a part or all of hydrogen atoms in the compound (I) or the pharmaceutically acceptable salt thereof with deuterium atoms can also be synthesized, for example, by 1) a method of deuterating a carboxylic acid and the like by using deuterium peroxide under a basic condition (see U.S. Patent No. 3849458), 2) a method of deuterating an alcohol, a carboxylic acid, and the like by using an iridium complex as a catalyst and heavy water as a deuterium source [see J. Am. Chem. Soc., Vol. 124, No. 10, 2092 (2002)], 3) a method of deuterating a fatty acid by using palladium-carbon as a catalyst and only deuterium gas as a deuterium source [see LIPIDS, Vol. 9, No. 11, 913 (1974)], 4) a method of deuterating acrylic acid, methyl acrylate, methacrylic acid, methyl methacrylate, and the like by using a metal such as platinum, palladium, rhodium, ruthenium, or iridium as a catalyst and heavy water or heavy water and deuterium gas as a deuterium source (see Japanese Examined Patent Application Publication No. H5-19536, Japanese Unexamined Patent Application Publication No. S61-277648, and Japanese Unexamined Patent Application Publication No. S61-275241), or 5) a method of deuterating acrylic acid, methyl methacrylate, and the like by using a catalyst such as palladium, nickel, copper, or copper chromite, and using heavy water as a deuterium source (see Japanese Unexamined Patent Application Publication No. S63-198638).

When the compound (I) or the pharmaceutically acceptable salt thereof is desired to be obtained, the compound (I) obtained in the form of a salt can be purified as it is, or the compound (I) obtained in a free form can be dissolved or suspended in an appropriate solvent and isolated and purified after forming a salt by the addition of an acid or a base.

The compound (I) or the pharmaceutically acceptable salt thereof may be present in the form of an adduct with water or various solvents. These adducts are also encompassed by the present invention.

In the formula (I), R¹ and R² are the same or different and each represents a hydrogen atom or lower alkyl. The lower alkyls in R¹ and R² are the same or different and are each preferably alkyl having 1 to 5 carbon atoms, more preferably alkyl having 1 to 3 carbon atoms, further preferably methyl or ethyl, further preferably methyl.

In the formula (I), R³ represents lower alkyl. The lower alkyl in R³ is preferably alkyl having 1 to 5 carbon atoms, more preferably alkyl having 1 to 3 carbon atoms, further preferably methyl or ethyl, further preferably ethyl.

In the formula (I), R⁴ represents halogen. The halogen in R⁴ is, for example, a fluorine atom, a chlorine atom, a bromine atom, or an iodine atom, preferably a fluorine atom or a chlorine atom. The number of R⁴ is 0 to 2, preferably 0 or 1. When two R⁴ are present, R⁴ may be the same as or different from each other.

In the formula (I), R⁵ represents halogen. The halogen in R⁵ is, for example, a fluorine atom, a chlorine atom, a bromine atom, or an iodine atom, preferably a chlorine atom. The number of R⁵ is 0 to 2, preferably 0 or 1. When two R⁵ are present, R⁵ may be the same as or different from each other.

In the formula (I), R⁶ represents halogen or lower alkoxy. The halogen in R⁶ is, for example, a fluorine atom, a chlorine atom, a bromine atom, or an iodine atom, preferably a fluorine atom. The lower alkoxy in R⁶ is preferably alkoxy having 1 to 5 carbon atoms, more preferably alkoxy having 1 to 3 carbon atoms, further preferably methoxy or ethoxy, further preferably methoxy. The number of R⁶ is 0 to 2, preferably 0 or 1. When two R⁶ are present, R⁶ may be the same as or different from each other.

In the formula (I), Y represents, for example, -O-CH₂-CH₂-, - CH₂-CH₂-CH₂-, -CH=CH-CH₂-, or -NH-C(=O)-CH₂- and is preferably -O-CH₂-CH₂-.

The compound of the present invention having one or more asymmetric carbon atoms may be present as two or more stereoisomers. Use of a solid line that represents a bond to the asymmetric carbon atom is meant to represent that all possible stereoisomers (e.g., diastereomer mixtures, racemic mixtures, and specific enantiomers) for the carbon atom are included.

In a preferred embodiment, R¹ is a hydrogen atom, R² is lower alkyl, R³ is lower alkyl, R⁴ is absent (i.e., 0) or halogen, R⁵ is absent (i.e., 0) or halogen, R⁶ is absent (i.e., 0), halogen, or lower alkoxy, and Y is -O-CH₂-CH₂-, -CH₂-CH₂-CH₂-, -CH=CH-CH₂-, or - NH-C(=O)-CH₂-.

In a more preferred embodiment, R¹ is a hydrogen atom, R² is lower alkyl having 1 to 5 carbon atoms, R³ is lower alkyl having 1 to 5 carbon atoms, R⁴ is absent or a fluorine atom, R⁵ is absent or a chlorine atom, R⁶ is absent, a fluorine atom, or lower alkoxy having 1 to 5 carbon atoms, and Y is -O-CH₂-CH₂-.

In a further preferred embodiment, R¹ is a hydrogen atom, R² is lower alkyl having 1 to 3 carbon atoms, R³ is lower alkyl having 1 to 3 carbon atoms, R⁴ is absent or a fluorine atom, R⁵ is absent or a chlorine atom, R⁶ is absent, a fluorine atom, or lower alkoxy having 1 to 3 carbon atoms, and Y is -O-CH₂-CH₂-.

In a further preferred embodiment, R¹ is a hydrogen atom, R² is methyl, R³ is ethyl, R⁴ is absent, R⁵ is absent, R⁶ is absent, and Y is -O-CH₂-CH₂-, -CH₂-CH₂-CH₂-, -CH=CH-CH₂-, or -NH-C(=O)-CH₂-. In a further preferred alternative embodiment, R¹ is a hydrogen atom, R² is methyl, R³ is ethyl, R⁴ is a fluorine atom, R⁵ is absent, R⁶ is absent, and Y is -O-CH₂-CH₂-. In a further preferred alternative embodiment, R¹ is a hydrogen atom, R² is methyl, R³ is ethyl, R⁴ is absent, R⁵ is a chlorine atom, R⁶ is absent, and Y is -O-CH₂-CH₂-.

In a further preferred alternative embodiment, R¹ is a hydrogen atom, R² is methyl, R³ is ethyl, R⁴ is absent, R⁵ is absent, R⁶ is a fluorine atom, and Y is -O-CH₂-CH₂- or -CH₂-CH₂-CH₂-. In a further preferred alternative embodiment, R¹ is a hydrogen atom, R² is methyl, R³ is ethyl, R⁴ is absent, R⁵ is absent, R⁶ is methoxy, and Y is -O-CH₂-CH₂-.

In the formula (I), ring A represents (i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl.

The lower alkyl serving as a substituent for (i) the aliphatic heterocyclic group, (ii) the aromatic heterocyclic group, and (iii) the aryl is preferably alkyl having 1 to 5 carbon atoms, more preferably alkyl having 1 to 3 carbon atoms, further preferably methyl or ethyl, further preferably methyl.

The lower alkyl in the hydroxy-lower alkyl, the lower alkylcarbonyl, the hydroxy-lower alkylcarbonyl, and the lower alkylcarbamoyl serving as a substituent for (i) the aliphatic heterocyclic group, (ii) the aromatic heterocyclic group, and (iii) the aryl is preferably alkyl having 1 to 5 carbon atoms, more preferably alkyl having 1 to 3 carbon atoms, further preferably methyl or ethyl, further preferably methyl.

The position or the number of hydroxy in the hydroxy-lower alkyl and the hydroxy-lower alkylcarbonyl serving as a substituent for (i) the aliphatic heterocyclic group, (ii) the aromatic heterocyclic group, and (iii) the aryl is not particularly limited. The number of hydroxy is, for example, 1 to 4, preferably 1 to 3, more preferably 1 or 2, further preferably 1.

The cycloalkyl in the cycloalkylcarbonyl serving as a substituent for (i) the aliphatic heterocyclic group, (ii) the aromatic heterocyclic group, and (iii) the aryl is preferably cycloalkyl having 3 to 10 carbon atoms, more preferably cycloalkyl having 3 to 8 carbon atoms, further preferably cycloalkyl having 3 to 6 carbon atoms, further preferably cyclopropyl.

The lower alkoxy in the lower alkoxycarbonyl serving as a substituent for (i) the aliphatic heterocyclic group, (ii) the aromatic heterocyclic group, and (iii) the aryl is preferably alkoxy having 1 to 5 carbon atoms, more preferably alkoxy having 1 to 3 carbon atoms, further preferably methoxy or ethoxy, further preferably methoxy.

When ring A is (i) the aliphatic heterocyclic group, the 1 to 3 substituents in (i) the aliphatic heterocyclic group are each independently selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl. The 1 to 3 substituents in the aliphatic heterocyclic group are preferably selected from the group consisting of oxo, lower alkyl, and lower alkylcarbonyl.

In a preferred embodiment, (i) the aliphatic heterocyclic group in ring A is a nitrogen-containing aliphatic heterocyclic group.

In a more preferred embodiment, ring A represented by (i) the nitrogen-containing aliphatic heterocyclic group is the following ring A':

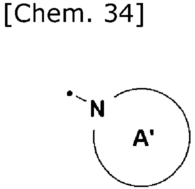

When ring A' has a substituent, the substituent of ring A' is as defined for the substituent of ring A which is (i) the aliphatic heterocyclic group. Specifically, ring A' may have 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, as in ring A which is (i) the aliphatic heterocyclic group. In this context, the dot (·) in the formula represents a bonding site to a linker.

In the nitrogen-containing aliphatic heterocyclic group (preferably ring A'), a nitrogen atom (except for a nitrogen atom having the bonding hand of the nitrogen-containing aliphatic heterocyclic group) may have a substituent, or a carbon atom may have a substituent. One carbon atom may have two substituents.

The nitrogen-containing aliphatic heterocyclic group (preferably ring A') is preferably an aliphatic heterocyclic group containing two nitrogen atoms, more preferably a monocyclic or bicyclic aliphatic heterocyclic group containing two nitrogen atoms, further preferably piperazinyl, 1,4-diazepanyl, or hexahydropyrrolopyrazinyl. The nitrogen-containing aliphatic heterocyclic group may be constituted only by saturated bonds or may contain a double bond and is preferably constituted only by saturated bonds. Two carbon atoms constituting a ring in the nitrogen-containing aliphatic heterocyclic group may be bridged by an alkylene group. The number of carbon atoms in the alkylene group that bridges two carbon atoms constituting a ring is, for example, 1 to 3, preferably 1 or 2, further preferably 1.

When the nitrogen-containing aliphatic heterocyclic group (preferably ring A') is a monocyclic aliphatic heterocyclic group containing two nitrogen atoms, one of the nitrogen atoms constitutes the bonding hand of the nitrogen-containing aliphatic heterocyclic group, and the other nitrogen atom preferably has a substituent selected from the group consisting of lower alkyl, hydroxy-lower alkyl, lower alkylcarbonyl, hydroxy-lower alkylcarbonyl, and cycloalkylcarbonyl. The substituent of the other nitrogen atom is more preferably selected from the group consisting of lower alkyl, lower alkylcarbonyl, and cycloalkylcarbonyl, and further preferably selected from the group consisting of lower alkyl and lower alkylcarbonyl. The monocyclic aliphatic heterocyclic group containing two nitrogen atoms may have 1 or 2 substituents other than the substituent of the other nitrogen atom. The 1 or 2 substituents other than the substituent of the other nitrogen atom are each independently preferably selected from the group consisting of oxo, lower alkyl, and hydroxy-lower alkyl, and more preferably selected from the group consisting of oxo and lower alkyl.

When the nitrogen-containing aliphatic heterocyclic group (preferably ring A') is a bicyclic aliphatic heterocyclic group containing two nitrogen atoms, one of the nitrogen atoms constitutes the bonding hand of the nitrogen-containing aliphatic heterocyclic group, and the other nitrogen atom is preferably shared by two rings (i.e., has no substituent). The bicyclic aliphatic heterocyclic group containing two nitrogen atoms preferably has oxo as a substituent. The bicyclic aliphatic heterocyclic group containing two nitrogen atoms may have 1 or 2 substituents selected from the group consisting of lower alkyl, hydroxy-lower alkyl, lower alkylcarbonyl, hydroxy-lower alkylcarbonyl, and cycloalkylcarbonyl, in addition to oxo.

In a preferred embodiment, when ring A is (i) the aliphatic heterocyclic group, the compound represented by the formula (I) is a compound represented by the following formula (I'): 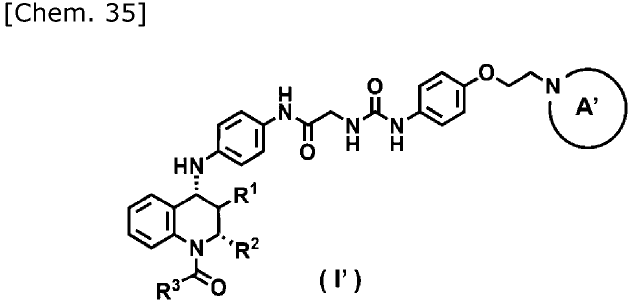 (wherein, R¹, R², and R³ are as defined in the formula (I), and ring A' is the nitrogen-containing aliphatic heterocyclic group described above).

In a preferred embodiment of the compound of the formula (I'), R¹ is a hydrogen atom, R² is lower alkyl, and R³ is lower alkyl.

In a more preferred embodiment of the compound of the formula (I'), R¹ is a hydrogen atom, R² is lower alkyl having 1 to 5 carbon atoms, and R³ is lower alkyl having 1 to 5 carbon atoms.

In a further preferred embodiment of the compound of the formula (I'), R¹ is a hydrogen atom, R² is lower alkyl having 1 to 3 carbon atoms, R³ is lower alkyl having 1 to 3 carbon atoms.

In a further preferred embodiment of the compound of the formula (I'), R¹ is a hydrogen atom, R² is methyl, and R³ is ethyl.

When ring A is (ii) the aromatic heterocyclic group, the 1 to 3 substituents in the aromatic heterocyclic group are each independently selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl. Each of the 1 to 3 substituents in the aromatic heterocyclic group is preferably lower alkyl.

In a preferred embodiment, (ii) the aromatic heterocyclic group in ring A is preferably a 5-membered aromatic heterocyclic group or a 6-membered aromatic heterocyclic group, more preferably a 5-membered aromatic heterocyclic group or a 6-membered aromatic heterocyclic group containing a nitrogen atom and/or an oxygen atom, further preferably oxazolyl or pyridyl.

When ring A is (iii) the aryl, the 1 to 3 substituents in the aryl are each independently selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl. The 1 to 3 substituents in the aryl are preferably selected from the group consisting of lower alkoxycarbonyl and lower alkylcarbamoyl.

In a preferred embodiment, (iii) the aryl in ring A is preferably phenyl.

In a preferred embodiment, ring A (including the case where ring A is ring A') is a nitrogen-containing aliphatic heterocyclic group represented by the following formula (AA), (AB), (AC), or (AD), an aromatic heterocyclic group represented by the following formula (AE) or (AF), or aryl represented by the following formula (AG):

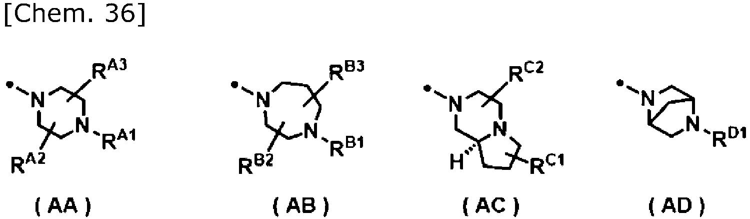

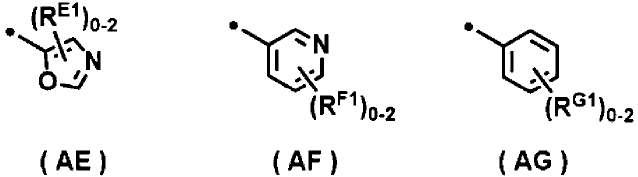

In the formulas (AA), (AB), (AC), (AD), (AE), (AF), and (AG), R^{A1}, R^{B1}, R^{D1}, R^{E1}, R^{F1}, and R^{G1} are the same or different and each represents lower alkyl, hydroxy-lower alkyl, lower alkylcarbonyl, hydroxy-lower alkylcarbonyl, lower alkoxycarbonyl, cycloalkylcarbonyl, or lower alkylcarbamoyl, and R^{A2}, R^{A3}, R^{B2}, R^{B3}, R^{C1}, and R^{C2} are the same or different and each represents a hydrogen atom, oxo, lower alkyl, or hydroxy-lower alkyl.

The lower alkyl in R^{A1}, R^{B1}, R^{D1}, R^{E1}, R^{F1}, and R^{G1} is preferably alkyl having 1 to 5 carbon atoms, more preferably alkyl having 1 to 3 carbon atoms, further preferably methyl or ethyl, further preferably methyl.

The lower alkyl in the hydroxy-lower alkyl, the lower alkylcarbonyl, the hydroxy-lower alkylcarbonyl, and the lower alkylcarbamoyl in R^{A1}, R^{B1}, R^{D1}, R^{E1}, R^{F1}, and R^{G1} is preferably alkyl having 1 to 5 carbon atoms, more preferably alkyl having 1 to 3 carbon atoms, further preferably methyl or ethyl, further preferably methyl. In this context, when the hydroxy-lower alkyl, the lower alkylcarbonyl, the hydroxy-lower alkylcarbonyl, or the lower alkylcarbamoyl has two or more lower alkyl, the lower alkyl may be the same as or different from each other.

The position or the number of hydroxy in the hydroxy-lower alkyl and the hydroxy-lower alkylcarbonyl in R^{A1}, R^{B1}, R^{D1}, R^{E1}, R^{F1}, and R^{G1} is not particularly limited. The number of hydroxy is, for example, 1 to 4, preferably 1 to 3, more preferably 1 or 2, further preferably 1.

The lower alkoxy in the lower alkoxycarbonyl in R^{A1}, R^{B1}, R^{D1}, R^{E1}, R^{F1}, and R^{G1} is preferably alkoxy having 1 to 5 carbon atoms, more preferably alkoxy having 1 to 3 carbon atoms, further preferably methoxy or ethoxy, further preferably methoxy.

The cycloalkyl in the cycloalkylcarbonyl in R^{A1}, R^{B1}, R^{D1}, R^{E1}, R^{F1}, and R^{G1} is preferably cycloalkyl having 3 to 10 carbon atoms, more preferably cycloalkyl having 3 to 8 carbon atoms, further preferably cycloalkyl having 3 to 6 carbon atoms, further preferably cyclopropyl.

The lower alkyl in R^{A2}, R^{A3}, R^{B2}, R^{B3}, R^{C1}, and R^{C2} is preferably alkyl having 1 to 5 carbon atoms, more preferably alkyl having 1 to 3 carbon atoms, further preferably methyl or ethyl, further preferably methyl.

The lower alkyl in the hydroxy-lower alkyl in R^{A2}, R^{A3}, R^{B2}, R^{B3}, R^{C1}, and R^{C2} is preferably alkyl having 1 to 5 carbon atoms, more preferably alkyl having 1 to 3 carbon atoms, further preferably methyl or ethyl, further preferably methyl.

The position or the number of hydroxy in the hydroxy-lower alkyl in R^{A2}, R^{A3}, R^{B2}, R^{B3}, R^{C1}, and R^{C2} is not particularly limited. The number of hydroxy is, for example, 1 to 4, preferably 1 to 3, more preferably 1 or 2, further preferably 1.

In a preferred embodiment, ring A or ring A' is a nitrogen-containing aliphatic heterocyclic group represented by the formula (AA), and in the formula (AA), R^{A1} represents lower alkyl, lower alkylcarbonyl, hydroxy-lower alkylcarbonyl, or cycloalkylcarbonyl, and R^{A2} and R^{A3} are the same or different and each represents a hydrogen atom, oxo, lower alkyl, or hydroxy-lower alkyl.

The lower alkyl in R^{A1} is preferably alkyl having 1 to 5 carbon atoms, more preferably alkyl having 1 to 3 carbon atoms, further preferably methyl or ethyl, further preferably methyl.

The lower alkyl in the lower alkylcarbonyl and the hydroxy-lower alkylcarbonyl in R^{A1} is preferably alkyl having 1 to 5 carbon atoms, more preferably alkyl having 1 to 3 carbon atoms, further preferably methyl or ethyl, further preferably methyl.

The position or the number of hydroxy in the hydroxy-lower alkylcarbonyl in R^{A1} is not particularly limited. The number of hydroxy is, for example, 1 to 4, preferably 1 to 3, more preferably 1 or 2, further preferably 1.

The cycloalkyl in the cycloalkylcarbonyl in R^{A1} is preferably cycloalkyl having 3 to 10 carbon atoms, more preferably cycloalkyl having 3 to 8 carbon atoms, further preferably cycloalkyl having 3 to 6 carbon atoms, further preferably cyclopropyl.

The lower alkyl in R^{A2} and R^{A3} is preferably alkyl having 1 to 5 carbon atoms, more preferably alkyl having 1 to 3 carbon atoms, further preferably methyl or ethyl, further preferably methyl.

The lower alkyl in the hydroxy-lower alkyl in R^{A2} and R^{A3} is preferably alkyl having 1 to 5 carbon atoms, more preferably alkyl having 1 to 3 carbon atoms, further preferably methyl or ethyl, further preferably methyl.

The position or the number of hydroxy in the hydroxy-lower alkyl in R^{A2} and R^{A3} is not particularly limited. The number of hydroxy is, for example, 1 to 4, preferably 1 to 3, more preferably 1 or 2, further preferably 1.

When ring A or ring A' is a nitrogen-containing aliphatic heterocyclic group represented by the formula (AA), R^{A1} is preferably lower alkyl or lower alkylcarbonyl.

When ring A or ring A' is a nitrogen-containing aliphatic heterocyclic group represented by the formula (AA), R^{A2} and R^{A3} are the same or different and are each preferably a hydrogen atom, oxo, or lower alkyl.

When ring A or ring A' is a nitrogen-containing aliphatic heterocyclic group represented by the formula (AA), more preferably, R^{A1} is lower alkyl or lower alkylcarbonyl, and R^{A2} and R^{A3} are the same or different and are each a hydrogen atom, oxo or lower alkyl.

When ring A or ring A' is a nitrogen-containing aliphatic heterocyclic group represented by the formula (AA), further preferably, R^{A1} is lower alkyl, and R^{A2} and R^{A3} are the same or different and are each oxo or lower alkyl.

In a preferred embodiment, ring A or ring A' is a nitrogen-containing aliphatic heterocyclic group represented by the formula (AB), and in the formula (AB), R^{B1} represents lower alkyl or lower alkylcarbonyl, and R^{B2} and R^{B3} are the same or different and each represents a hydrogen atom or oxo.

The lower alkyl in R^{B1} is preferably alkyl having 1 to 5 carbon atoms, more preferably alkyl having 1 to 3 carbon atoms, further preferably methyl or ethyl, further preferably methyl.

The lower alkyl in the lower alkylcarbonyl in R^{B1} is preferably alkyl having 1 to 5 carbon atoms, more preferably alkyl having 1 to 3 carbon atoms, further preferably methyl or ethyl, further preferably methyl.

When ring A or ring A' is a nitrogen-containing aliphatic heterocyclic group represented by the formula (AB), R^{B1} is preferably lower alkyl.

In a preferred embodiment, ring A or ring A' is a nitrogen-containing aliphatic heterocyclic group represented by the formula (AC), and in the formula (AC), R^{C1} and R^{C2} are the same or different and each represents a hydrogen atom or oxo.

In a preferred embodiment, ring A or ring A' is a nitrogen-containing aliphatic heterocyclic group represented by the formula (AD), and in the formula (AD), R^{D1} represents lower alkylcarbonyl.

The lower alkyl in the lower alkylcarbonyl in R^{D1} is preferably alkyl having 1 to 5 carbon atoms, more preferably alkyl having 1 to 3 carbon atoms, further preferably methyl or ethyl, further preferably methyl.

In a preferred embodiment, ring A is an aromatic heterocyclic group represented by the formula (AE), and in the formula (AE), R^{E1} represents lower alkyl.

The lower alkyl in R^{E1} is preferably alkyl having 1 to 5 carbon atoms, more preferably alkyl having 1 to 3 carbon atoms, further preferably methyl or ethyl, further preferably methyl.

The number of R^{E1} is, for example, 0 to 2, preferably 1 or 2, more preferably 2.

In a preferred embodiment, ring A is an aromatic heterocyclic group represented by the formula (AF), and in the formula (AF), R^{F1} represents lower alkyl.

The lower alkyl in R^{F1} is preferably alkyl having 1 to 5 carbon atoms, more preferably alkyl having 1 to 3 carbon atoms, further preferably methyl or ethyl, further preferably methyl.

The number of R^{F1} is, for example, 0 to 2, preferably 1 or 2, more preferably 1.

In a preferred embodiment, ring A is aryl represented by the formula (AG), and in the formula (AG), R^{G1} represents lower alkoxycarbonyl or lower alkylcarbamoyl.

The lower alkoxy in the lower alkoxycarbonyl in R^{G1} is preferably alkoxy having 1 to 5 carbon atoms, more preferably alkoxy having 1 to 3 carbon atoms, further preferably methoxy or ethoxy, further preferably methoxy.

The lower alkyl in the lower alkylcarbamoyl in R^{G1} is preferably alkyl having 1 to 5 carbon atoms, more preferably alkyl having 1 to 3 carbon atoms, further preferably methyl or ethyl, further preferably methyl. In this context, when the lower alkylcarbamoyl has two or more lower alkyl, the lower alkyl may be the same as or different from each other. Thus, the lower alkylcarbamoyl in R^{G1} is preferably N-methylcarbamoyl or N,N-dimethylcarbamoyl.

The number of R^{G1} is, for example, 0 to 2, preferably 1 or 2, more preferably 1.

In a preferred embodiment, ring A is (i) the nitrogen-containing aliphatic heterocyclic group, (ii) the aromatic heterocyclic group, or (iii) the aryl, more preferably the formula (AA), the formula (AB), the formula (AC), the formula (AD), the formula (AE), the formula (AF), or the formula (AG), wherein R¹ is a hydrogen atom, R² is methyl, R³ is ethyl, R⁴ is absent, R⁵ is absent, R⁶ is absent, and Y is -O-CH₂-CH₂-, -CH₂-CH₂-CH₂-, - CH=CH-CH₂-, or -NH-C(=O)-CH₂-. In a preferred embodiment, ring A is (i) the nitrogen-containing aliphatic heterocyclic group, more preferably the formula (AA), wherein R¹ is a hydrogen atom, R² is methyl, R³ is ethyl, R⁴ is a fluorine atom, R⁵ is absent, R⁶ is absent, and Y is -O-CH₂-CH₂-. In a preferred alternative embodiment, ring A is (i)the nitrogen-containing aliphatic heterocyclic group, more preferably the formula (AA), wherein R¹ is a hydrogen atom, R² is methyl, R³ is ethyl, R⁴ is absent, R⁵ is a chlorine atom, R⁶ is absent, and Y is -O-CH₂-CH₂-. In a preferred alternative embodiment, ring A is (i) the nitrogen-containing aliphatic heterocyclic group, more preferably the formula (AA), (AB), or (AC), wherein R¹ is a hydrogen atom, R² is methyl, R³ is ethyl, R⁴ is absent, R⁵ is absent, R⁶ is a fluorine atom, and Y is -O-CH₂-CH₂- or -CH₂-CH₂-CH₂-. In a preferred alternative embodiment, ring A is (i) the nitrogen-containing aliphatic heterocyclic group, more preferably the formula (AA), wherein R¹ is a hydrogen atom, R² is methyl, R³ is ethyl, R⁴ is absent, R⁵ is absent, R⁶ is methoxy, and Y is -O-CH₂-CH₂-.

In a preferred embodiment, the compound (I) or the pharmaceutically acceptable salt thereof is a compound represented by the following formula or a pharmaceutically acceptable salt thereof:

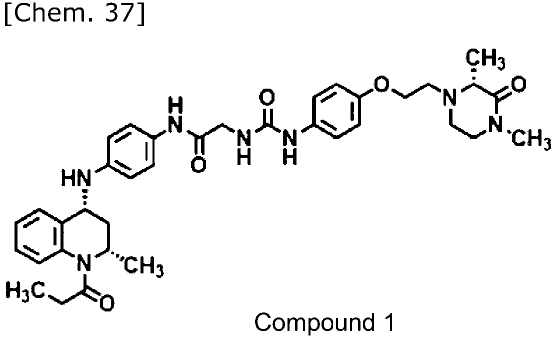

In a preferred embodiment, the compound (I) or the pharmaceutically acceptable salt thereof is a compound represented by the following formula or a pharmaceutically acceptable salt thereof:

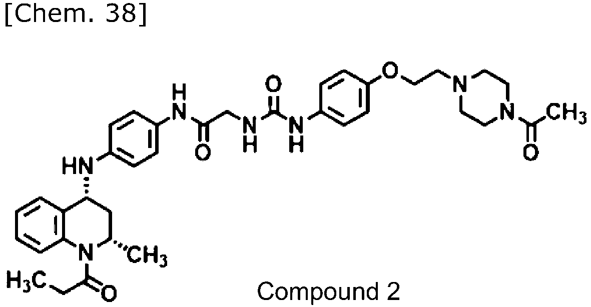

In a preferred embodiment, the compound (I) or the pharmaceutically acceptable salt thereof is a compound represented by the following formula or a pharmaceutically acceptable salt thereof:

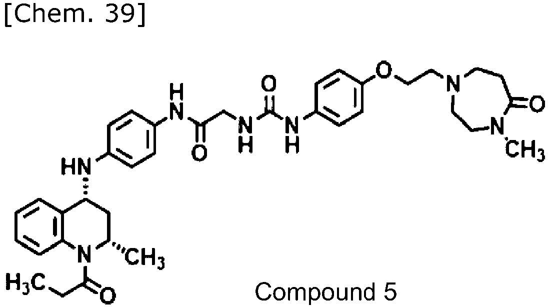

In a preferred embodiment, the compound (I) or the pharmaceutically acceptable salt thereof is a compound represented by the following formula or a pharmaceutically acceptable salt thereof:

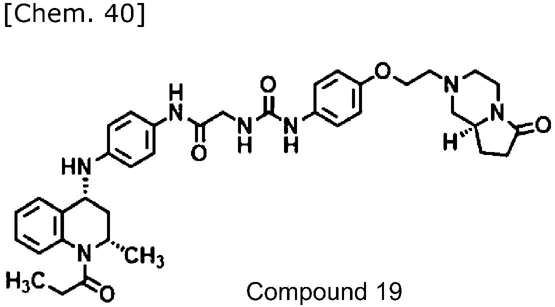

In a preferred embodiment, the compound (I) or the pharmaceutically acceptable salt thereof is a compound represented by the following formula or a pharmaceutically acceptable salt thereof:

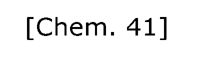

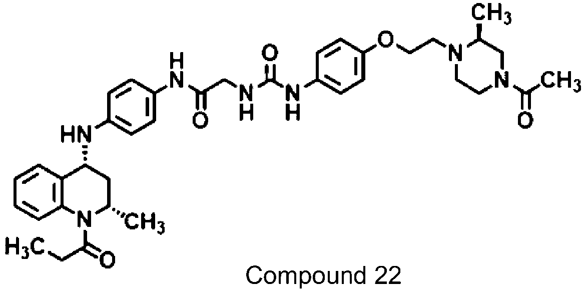

### <<Second aspect>>

A second aspect of the present invention provides a targeting molecule-drug conjugate containing a targeting molecule and a drug, wherein the drug is the compound (I) or the pharmaceutically acceptable salt thereof. The targeting molecule-drug conjugate according to the second aspect can avoid the safety hazard, i.e., peripheral neurotoxicity, while maintaining high antitumor activity. The drug (divalent BET degrader) is conjugated with a targeting molecule so that the drug can be efficiently delivered to target cells. Thus, the drug (divalent BET degrader) conjugated with the targeting molecule can exhibit an antitumor effect *in vivo* in a smaller amount of the drug (in terms of the divalent BET degrader) as compared with the drug alone.

The targeting molecule is, for example, a molecule capable of binding to a molecule, such as a protein or a peptide, present on the surface of target cells. The targeting molecule can bind to a molecule, such as a protein or a peptide, present on the surface of target cells and thereby deliver the drug to the target cells. The targeting molecule is preferably capable of specific binding to a molecule, such as a protein or a peptide, present on the surface of target cells. The term "specific" means that the targeting molecule exhibits no significant binding activity against other substances.

The target cells are preferably cancer cells. Examples of the molecule, such as a protein or a peptide, present on the surface of cancer cells include receptors, cancer antigens, MHC antigens, and differentiation antigens. The protein or the peptide may be bound with an MHC class I molecule or an MHC class II molecule and presented on the surface of cancer cells.

The cancer is preferably blood cancer, more preferably lymphoma, further preferably B cell lymphoma. Thus, the molecule, such as a protein or a peptide, present on the surface of cancer cells is a molecule, such as a protein or a peptide, expressed on lymphocytes (preferably B cells) of an organism affected by preferably blood cancer, more preferably lymphoma, further preferably B cell lymphoma.

Examples of the molecule, such as a protein or a peptide, present on the surface of cancer cells include folate receptor α (FoLRα), somatostatin receptor (SSTR2), fibroblast activation protein (FAP), erythropoietin-producing hepatocellular receptor A2 (EphA2), CD79b, CD19, CD22, HER2, Nectin4, TROP2, DLL3, PSMA, B7H3, B7H4, CD30, CD33, CD25, CD38, CD74, GM2, CADM1, CLL1, BCMA, HER3, and CDH6. Among them, somatostatin receptor, fibroblast activation protein, erythropoietin-producing hepatocellular receptor A2, CD79b, CD19, CD22, HER2, Nectin4, TROP2, DLL3, CD30, CD33, CD25, CD38, CD74, GM2, CADM1, BCMA, and CLL1 are preferred, CD79b, CD19, CD22, CD30, CD33, CD25, CD38, BCMA, and CD74 are more preferred, and CD79b, CD19, CD22, CD30, and BCMA are further preferred.

The targeting molecule is, for example, an antibody or an antigen binding fragment thereof, a small-molecule compound, or a peptide.

Examples of the small-molecule compound include folate, oncoFAP, AAZ+, and 2-[3-(1,3-dicarboxypropyl)ureido]-pentanedioic acid (DUPA).

Folate can bind to folate receptor. Thus, the targeting molecule-drug conjugate containing folate as the targeting molecule can target cells having the folate receptor on the surface (C.P. Leamon et al., Cancer Res. 2008, 68, 9839-9844).

A synthetic small-molecule compound called oncoFAP can bind to fibroblast activation protein. Thus, the targeting molecule-drug conjugate containing octreotide as the targeting molecule can target cells having the fibroblast activation protein on the surface (J. Millu et al., Proc Natl Acad Sci USA. 2021, 118, e2101852118).

A synthetic small-molecule compound called AAZ+ can bind to carbonic anhydrase 9. Thus, the targeting molecule-drug conjugate containing AAZ+ as the targeting molecule can target cells having the carbonic anhydrase 9 on the surface (S. Cazzamalli et al., J Am Chem Soc, 2018, 140, 1617-1621.

DUPA can bind to prostate-specific membrane antigen. Thus, the targeting molecule-drug conjugate containing DUPA as the targeting molecule can target cells having the prostate-specific membrane antigen on the surface (J. Roy et al., J. Med. Chem. 2015, 58, 3094-3103).

Examples of the peptide include BCY6099, octreotide, RGD peptide, exendin-4, bombesin-like peptide, and BCY8234.

A bicyclic peptide called BCY6099 can bind to erythropoietin-producing hepatocellular receptor A2. Thus, the targeting molecule-drug conjugate containing BCY6099 as the targeting molecule can target cells having the erythropoietin-producing hepatocellular receptor A2 on the surface (G. Bennett et al., Mol Cancer Ther 2020, 19, 1385-1394).

Octreotide can bind to somatostatin receptor, particularly, somatostatin receptor subtype 2. Thus, the targeting molecule-drug conjugate containing octreotide as the targeting molecule can target cells having the somatostatin receptor subtype 2 on the surface (B.H. White et al., J. Med. Chem. 2019, 62, 2708-2719).

RGD peptide can bind to integrin receptor, particularly, αvβ3 integrin receptor. Thus, the targeting molecule-drug conjugate containing RGD peptide as the targeting molecule can target cells having the αvβ3 integrin receptor on the surface (M. Lelle et al., J. Med. Chem. 2005, 48, 1098-1106).

Exendin-4 can bind to glucagon-like peptide-1 receptor. Thus, the targeting molecule-drug conjugate containing exendin-4 as the targeting molecule can target cells having the glucagon-like peptide-1 receptor on the surface (S. Son et al., The International Journal of Biochemistry & Cell Biology. 105, 13-19).

Bombesin-like peptide can bind to bombesin-like peptide receptor. Thus, the targeting molecule-drug conjugate containing bombesin-like peptide as the targeting molecule can target cells having the bombesin-like peptide receptor on the surface (A. Safavy et al., Bioconjugate Chem. 2006, 17, 565-570).

BCY8234 can bind to nectin-4. Thus, the targeting molecule-drug conjugate containing BCY8234 as the targeting molecule can target cells having the nectin-4 on the surface (M. Rigby et al., Mol Cancer Ther MCT-21-0875).

In a preferred embodiment, the targeting molecule is an antibody or an antigen binding fragment thereof.

In a more preferred embodiment, the targeting molecule is an antibody.

In a further preferred embodiment, the antibody is an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody.

Hereinafter, an embodiment in which the antibody is an anti-CD79b antibody will be described.

The anti-CD79b antibody is an antibody that binds to CD79b. The anti-CD79b antibody is preferably an antibody that specifically binds to CD79b. The anti-CD79b antibody may be a polyclonal antibody or a monoclonal antibody and is preferably a monoclonal antibody. The anti-CD79b antibody may be a human-type chimeric antibody, a humanized antibody, or a human antibody and is preferably a humanized antibody.

In one embodiment, the anti-CD79b antibody is an antibody having a heavy chain variable region (VH) and a light chain variable region (VL) of polatuzumab.

In one embodiment, the anti-CD79b antibody contains a heavy chain variable region (VH) containing CDR1 consisting of the amino acid sequence set forth in SEQ ID NO: 11, CDR2 consisting of the amino acid sequence set forth in SEQ ID NO: 12, and CDR3 consisting of the amino acid sequence set forth in SEQ ID NO: 13, and a light chain variable region (VL) containing CDR1 consisting of the amino acid sequence set forth in SEQ ID NO: 14, CDR2 consisting of the amino acid sequence set forth in SEQ ID NO: 15, and CDR3 consisting of the amino acid sequence set forth in SEQ ID NO: 16. CDR1, CDR2, and CDR3 are CDR1, CDR2 and, CDR3, respectively, based on the Kabat numbering.

In an alternative embodiment, the amino acid sequence of VH of the anti-CD79b antibody contains the amino acid sequence set forth in SEQ ID NO: 5, and the amino acid sequence of VL of the anti-CD79b antibody contains the amino acid sequence set forth in SEQ ID NO: 6. The amino acid sequence of VH of the anti-CD79b antibody may consist of the amino acid sequence set forth in SEQ ID NO: 5, and the amino acid sequence of VL of the anti-CD79b antibody may consist of the amino acid sequence set forth in SEQ ID NO: 6.

As shown in the table below, VH containing the amino acid sequence set forth in SEQ ID NO: 5 contains CDR1 consisting of the amino acid sequence set forth in SEQ ID NO: 11 (underlined in the table below), CDR2 consisting of the amino acid sequence set forth in SEQ ID NO: 12 (underlined in the table below), and CDR3 consisting of the amino acid sequence set forth in SEQ ID NO: 13 (underlined in the table below), and VL containing the amino acid sequence set forth in SEQ ID NO: 6 contains CDR1 consisting of the amino acid sequence set forth in SEQ ID NO: 14 (underlined in the table below), CDR2 consisting of the amino acid sequence set forth in SEQ ID NO: 15 (underlined in the table below), and CDR3 consisting of the amino acid sequence set forth in SEQ ID NO: 16 (underlined in the table below).

**[Table 1]**

| |
|---|
| SEQ ID NO: 5 |
| |
| SEQ ID NO: 6 |
| |

In a further alternative embodiment, the amino acid sequence of VH of the anti-CD79b antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 5, and the amino acid sequence of VL of the anti-CD79b antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 6. The amino acid sequence of VH of the anti-CD79b antibody may consist of an amino acid sequence having 90% or higher sequence identity to the amino acid sequence set forth in SEQ ID NO: 5, and the amino acid sequence of VL of the anti-CD79b antibody may consist of an amino acid sequence having 90% or higher sequence identity to the amino acid sequence set forth in SEQ ID NO: 6. The amino acid sequence having 90% or higher sequence identity to the amino acid sequence set forth in SEQ ID NO: 5 may be the amino acid sequence set forth in SEQ ID NO: 5, and the amino acid sequence having 90% or higher sequence identity to the amino acid sequence set forth in SEQ ID NO: 6 may be the amino acid sequence set forth in SEQ ID NO: 6.

VH containing the amino acid sequence having the sequence identity to the amino acid sequence set forth in SEQ ID NO: 5 preferably contains CDR1 consisting of the amino acid sequence set forth in SEQ ID NO: 11, CDR2 consisting of the amino acid sequence set forth in SEQ ID NO: 12, and CDR3 consisting of the amino acid sequence set forth in SEQ ID NO: 13.

VL containing the amino acid sequence having the sequence identity to the amino acid sequence set forth in SEQ ID NO: 6 preferably contains CDR1 consisting of the amino acid sequence set forth in SEQ ID NO: 14, CDR2 consisting of the amino acid sequence set forth in SEQ ID NO: 15, and CDR3 consisting of the amino acid sequence set forth in SEQ ID NO: 16.

VH containing the amino acid sequence having the sequence identity to the amino acid sequence set forth in SEQ ID NO: 5 has the activity of VH of the anti-CD79b antibody, i.e., binding activity against CD79b. Examples of the amino acid sequence having the sequence identity to the amino acid sequence set forth in SEQ ID NO: 5 include amino acid sequences derived from the amino acid sequence set forth in SEQ ID NO: 5 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 6 has the activity of VL of the anti-CD79b antibody, i.e., binding activity against CD79b. Examples of the amino acid sequence having the sequence identity to the amino acid sequence set forth in SEQ ID NO: 6 include amino acid sequences derived from the amino acid sequence set forth in SEQ ID NO: 6 by the deletion, substitution, insertion, or addition of, for example, 1 to 20, preferably 1 to 10, more preferably 1 to 5 amino acids.

Hereinafter, an embodiment in which the antibody is an anti-CD19 antibody will be described.

The anti-CD19 antibody is an antibody that binds to CD19. The anti-CD19 antibody is preferably an antibody that specifically binds to CD19. The anti-CD19 antibody may be a polyclonal antibody or a monoclonal antibody and is preferably a monoclonal antibody. The anti-CD19 antibody may be a human-type chimeric antibody, a humanized antibody, or a human antibody and is preferably a humanized antibody.

In one embodiment, the anti-CD19 antibody is an antibody having a heavy chain variable region (VH) and a light chain variable region (VL) of loncastuximab.

In one embodiment, the anti-CD19 antibody contains a heavy chain variable region (VH) containing CDR1 consisting of the amino acid sequence set forth in SEQ ID NO: 17, CDR2 consisting of the amino acid sequence set forth in SEQ ID NO: 18, and CDR3 consisting of the amino acid sequence set forth in SEQ ID NO: 19, and a light chain variable region (VL) containing CDR1 consisting of the amino acid sequence set forth in SEQ ID NO: 20, CDR2 consisting of the amino acid sequence set forth in SEQ ID NO: 21, and CDR3 consisting of the amino acid sequence set forth in SEQ ID NO: 22. CDR1, CDR2, and CDR3 are CDR1, CDR2 and, CDR3, respectively, based on the Kabat numbering.

In an alternative embodiment, the amino acid sequence of VH of the anti-CD19 antibody contains the amino acid sequence set forth in SEQ ID NO: 7, and the amino acid sequence of VL of the anti-CD19 antibody contains the amino acid sequence set forth in SEQ ID NO: 8. The amino acid sequence of VH of the anti-CD19 antibody may consist of the amino acid sequence set forth in SEQ ID NO: 7, and the amino acid sequence of VL of the anti-CD19 antibody may consist of the amino acid sequence set forth in SEQ ID NO: 8.

As shown in the table below, VH containing the amino acid sequence set forth in SEQ ID NO: 7 contains CDR1 consisting of the amino acid sequence set forth in SEQ ID NO: 17 (underlined in the table below), CDR2 consisting of the amino acid sequence set forth in SEQ ID NO: 18 (underlined in the table below), and CDR3 consisting of the amino acid sequence set forth in SEQ ID NO: 19 (underlined in the table below), and VL containing the amino acid sequence set forth in SEQ ID NO: 8 contains CDR1 consisting of the amino acid sequence set forth in SEQ ID NO: 20 (underlined in the table below), CDR2 consisting of the amino acid sequence set forth in SEQ ID NO: 21 (underlined in the table below), and CDR3 consisting of the amino acid sequence set forth in SEQ ID NO: 22 (underlined in the table below).

**[Table 2]**

| |
|---|
| SEQ ID NO: 7 |
| QVQLVQPGAEVVKPGASVKLSCKTSGYTFTSNWMHWVKQAPGQGLEWIGEIDPSDSYTNYNQN FQGKAKLTVDKSTSTAYMEVSSLRSDDTAVYYCARGSNPYYYAMDYWGQGTSVTVSS |
| SEQ ID NO: 8 |
| EIVLTQSPAIMSASPGERVTMTCSASSGVNYMHWYQQKPGTSPRRWIYDTSKLASGVPARFSGS GSGTSYSLTISSMEPEDAATYYCHQRGSYTFGGGTKLEIK |

In a further alternative embodiment, the amino acid sequence of VH of the anti-CD19 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 7, and the amino acid sequence of VL of the anti-CD19 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 8. The amino acid sequence of VH of the anti-CD19 antibody may consist of an amino acid sequence having 90% or higher sequence identity to the amino acid sequence set forth in SEQ ID NO: 7, and the amino acid sequence of VL of the anti-CD19 antibody may consist of an amino acid sequence having 90% or higher sequence identity to the amino acid sequence set forth in SEQ ID NO: 8. The amino acid sequence having 90% or higher sequence identity to the amino acid sequence set forth in SEQ ID NO: 7 may be the amino acid sequence set forth in SEQ ID NO: 7, and the amino acid sequence having 90% or higher sequence identity to the amino acid sequence set forth in SEQ ID NO: 8 may be the amino acid sequence set forth in SEQ ID NO: 8.

VH containing the amino acid sequence having the sequence identity to the amino acid sequence set forth in SEQ ID NO: 7 preferably contains CDR1 consisting of the amino acid sequence set forth in SEQ ID NO: 17, CDR2 consisting of the amino acid sequence set forth in SEQ ID NO: 18, and CDR3 consisting of the amino acid sequence set forth in SEQ ID NO: 19.

VL containing the amino acid sequence having the sequence identity to the amino acid sequence set forth in SEQ ID NO: 8 preferably contains CDR1 consisting of the amino acid sequence set forth in SEQ ID NO: 20, CDR2 consisting of the amino acid sequence set forth in SEQ ID NO: 21, and CDR3 consisting of the amino acid sequence set forth in SEQ ID NO: 22.

VH containing the amino acid sequence having the sequence identity to the amino acid sequence set forth in SEQ ID NO: 7 has the activity of VH of the anti-CD19 antibody, i.e., binding activity against CD19. Examples of the amino acid sequence having the sequence identity to the amino acid sequence set forth in SEQ ID NO: 7 include amino acid sequences derived from the amino acid sequence set forth in SEQ ID NO: 7 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 8 has the activity of VL of the anti-CD19 antibody, i.e., binding activity against CD19. Examples of the amino acid sequence having the sequence identity to the amino acid sequence set forth in SEQ ID NO: 8 include amino acid sequences derived from the amino acid sequence set forth in SEQ ID NO: 8 by the deletion, substitution, insertion, or addition of, for example, 1 to 20, preferably 1 to 10, more preferably 1 to 5 amino acids.

Hereinafter, an embodiment in which the antibody is an anti-CD22 antibody will be described.

The anti-CD22 antibody is an antibody that binds to CD22. The anti-CD22 antibody is preferably an antibody that specifically binds to CD22. The anti-CD22 antibody may be a polyclonal antibody or a monoclonal antibody and is preferably a monoclonal antibody. The anti-CD22 antibody may be a human-type chimeric antibody, a humanized antibody, or a human antibody and is preferably a humanized antibody.

In one embodiment, the anti-CD22 antibody is an antibody having a heavy chain variable region (VH) and a light chain variable region (VL) of inotuzumab.

In one embodiment, the anti-CD22 antibody contains a heavy chain variable region (VH) containing CDR1 consisting of the amino acid sequence set forth in SEQ ID NO: 23, CDR2 consisting of the amino acid sequence set forth in SEQ ID NO: 24, and CDR3 consisting of the amino acid sequence set forth in SEQ ID NO: 25, and a light chain variable region (VL) containing CDR1 consisting of the amino acid sequence set forth in SEQ ID NO: 26, CDR2 consisting of the amino acid sequence set forth in SEQ ID NO: 27, and CDR3 consisting of the amino acid sequence set forth in SEQ ID NO: 28. CDR1, CDR2, and CDR3 are CDR1, CDR2 and, CDR3, respectively, based on the Kabat numbering.

In an alternative embodiment, the amino acid sequence of VH of the anti-CD22 antibody contains the amino acid sequence set forth in SEQ ID NO: 9, and the amino acid sequence of VL of the anti-CD22 antibody contains the amino acid sequence set forth in SEQ ID NO: 10. The amino acid sequence of VH of the anti-CD22 antibody may consist of the amino acid sequence set forth in SEQ ID NO: 9, and the amino acid sequence of VL of the anti-CD22 antibody may consist of the amino acid sequence set forth in SEQ ID NO: 10.

As shown in the table below, VH containing the amino acid sequence set forth in SEQ ID NO: 9 contains CDR1 consisting of the amino acid sequence set forth in SEQ ID NO: 23 (underlined in the table below), CDR2 consisting of the amino acid sequence set forth in SEQ ID NO: 24 (underlined in the table below), and CDR3 consisting of the amino acid sequence set forth in SEQ ID NO: 25 (underlined in the table below), and VL containing the amino acid sequence set forth in SEQ ID NO: 10 contains CDR1 consisting of the amino acid sequence set forth in SEQ ID NO: 26 (underlined in the table below), CDR2 consisting of the amino acid sequence set forth in SEQ ID NO: 27 (underlined in the table below), and CDR3 consisting of the amino acid sequence set forth in SEQ ID NO: 28 (underlined in the table below).

**[Table 3]**

| |
|---|
| SEQ ID NO: 9 |
| EVQLVQSGAEVKKPGASVKVSCKASGYRFTNYWIHWVRQAPGQGLEWIGGINPGNNYATYRRKF QGRVTMTADTSTSTVYMELSSLRSEDTAVYYCTREGYGNYGAWFAYWGQGTLVTVSS |
| SEQ ID NO: 10 |
| DVQVTQSPSSLSASVGDRVTITCRSSQSLANSYGNTFLSWYLHKPGKAPQLLIYGISNRFSGVPD RFSGSGSGTDFTLTISSLQPEDFATYYCLQGTHQPYTFGQGTKVEIK |

In a further alternative embodiment, the amino acid sequence of VH of the anti-CD22 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 9, and the amino acid sequence of VL of the anti-CD22 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 10. The amino acid sequence of VH of the anti-CD22 antibody may consist of an amino acid sequence having 90% or higher sequence identity to the amino acid sequence set forth in SEQ ID NO: 9, and the amino acid sequence of VL of the anti-CD22 antibody may consist of an amino acid sequence having 90% or higher sequence identity to the amino acid sequence set forth in SEQ ID NO: 10. The amino acid sequence having 90% or higher sequence identity to the amino acid sequence set forth in SEQ ID NO: 9 may be the amino acid sequence set forth in SEQ ID NO: 9, and the amino acid sequence having 90% or higher sequence identity to the amino acid sequence set forth in SEQ ID NO: 10 may be the amino acid sequence set forth in SEQ ID NO: 10.

VH containing the amino acid sequence having the sequence identity to the amino acid sequence set forth in SEQ ID NO: 9 preferably contains CDR1 consisting of the amino acid sequence set forth in SEQ ID NO: 23, CDR2 consisting of the amino acid sequence set forth in SEQ ID NO: 24, and CDR3 consisting of the amino acid sequence set forth in SEQ ID NO: 25.

VL containing the amino acid sequence having the sequence identity to the amino acid sequence set forth in SEQ ID NO: 10 preferably contains CDR1 consisting of the amino acid sequence set forth in SEQ ID NO: 26, CDR2 consisting of the amino acid sequence set forth in SEQ ID NO: 27, and CDR3 consisting of the amino acid sequence set forth in SEQ ID NO: 28.

VH containing the amino acid sequence having the sequence identity to the amino acid sequence set forth in SEQ ID NO: 9 has the activity of VH of the anti-CD22 antibody, i.e., binding activity against CD22. Examples of the amino acid sequence having the sequence identity to the amino acid sequence set forth in SEQ ID NO: 9 include amino acid sequences derived from the amino acid sequence set forth in SEQ ID NO: 9 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 10 has the activity of VL of the anti-CD22 antibody, i.e., binding activity against CD22. Examples of the amino acid sequence having the sequence identity to the amino acid sequence set forth in SEQ ID NO: 10 include amino acid sequences derived from the amino acid sequence set forth in SEQ ID NO: 10 by the deletion, substitution, insertion, or addition of, for example, 1 to 20, preferably 1 to 10, more preferably 1 to 5 amino acids.

Hereinafter, an embodiment in which the antibody is an anti-HER2 antibody will be described.

The anti-HER2 antibody is an antibody that binds to HER2. The anti-HER2 antibody is preferably an antibody that specifically binds to HER2. The anti-HER2 antibody may be a polyclonal antibody or a monoclonal antibody and is preferably a monoclonal antibody. The anti-HER2 antibody may be a human-type chimeric antibody, a humanized antibody, or a human antibody and is preferably a humanized antibody.

In one embodiment, the anti-HER2 antibody is an antibody having a heavy chain variable region (VH) and a light chain variable region (VL) of trastuzumab.

In one embodiment, the anti-HER2 antibody contains a heavy chain variable region (VH) containing CDR1 consisting of the amino acid sequence set forth in SEQ ID NO: 67, CDR2 consisting of the amino acid sequence set forth in SEQ ID NO: 68, and CDR3 consisting of the amino acid sequence set forth in SEQ ID NO: 69, and a light chain variable region (VL) containing CDR1 consisting of the amino acid sequence set forth in SEQ ID NO: 70, CDR2 consisting of the amino acid sequence set forth in SEQ ID NO: 71, and CDR3 consisting of the amino acid sequence set forth in SEQ ID NO: 72. CDR1, CDR2, and CDR3 are CDR1, CDR2 and, CDR3, respectively, based on the Kabat numbering.

In an alternative embodiment, the amino acid sequence of VH of the anti-HER2 antibody contains the amino acid sequence set forth in SEQ ID NO: 29, and the amino acid sequence of VL of the anti-HER2 antibody contains the amino acid sequence set forth in SEQ ID NO: 30. The amino acid sequence of VH of the anti-HER2 antibody may consist of the amino acid sequence set forth in SEQ ID NO: 29, and the amino acid sequence of VL of the anti-HER2 antibody may consist of the amino acid sequence set forth in SEQ ID NO: 30.

As shown in the table below, VH containing the amino acid sequence set forth in SEQ ID NO: 29 contains CDR1 consisting of the amino acid sequence set forth in SEQ ID NO: 67 (underlined in the table below), CDR2 consisting of the amino acid sequence set forth in SEQ ID NO: 68 (underlined in the table below), and CDR3 consisting of the amino acid sequence set forth in SEQ ID NO: 69 (underlined in the table below), and VL containing the amino acid sequence set forth in SEQ ID NO: 30 contains CDR1 consisting of the amino acid sequence set forth in SEQ ID NO: 70 (underlined in the table below), CDR2 consisting of the amino acid sequence set forth in SEQ ID NO: 71 (underlined in the table below), and CDR3 consisting of the amino acid sequence set forth in SEQ ID NO: 72 (underlined in the table below).

**[Table 4]**

| |
|---|
| SEQ ID NO: 29 |
| |
| SEQ ID NO: 30 |
| |

In a further alternative embodiment, the amino acid sequence of VH of the anti-HER2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 29, and the amino acid sequence of VL of the anti-HER2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 30. The amino acid sequence of VH of the anti-HER2 antibody may consist of an amino acid sequence having 90% or higher sequence identity to the amino acid sequence set forth in SEQ ID NO: 29, and the amino acid sequence of VL of the anti-HER2 antibody may consist of an amino acid sequence having 90% or higher sequence identity to the amino acid sequence set forth in SEQ ID NO: 30. The amino acid sequence having 90% or higher sequence identity to the amino acid sequence set forth in SEQ ID NO: 29 may be the amino acid sequence set forth in SEQ ID NO: 29, and the amino acid sequence having 90% or higher sequence identity to the amino acid sequence set forth in SEQ ID NO: 30 may be the amino acid sequence set forth in SEQ ID NO: 30.

VH containing the amino acid sequence having the sequence identity to the amino acid sequence set forth in SEQ ID NO: 29 preferably contains CDR1 consisting of the amino acid sequence set forth in SEQ ID NO: 67, CDR2 consisting of the amino acid sequence set forth in SEQ ID NO: 68, and CDR3 consisting of the amino acid sequence set forth in SEQ ID NO: 69.

VL containing the amino acid sequence having the sequence identity to the amino acid sequence set forth in SEQ ID NO: 30 preferably contains CDR1 consisting of the amino acid sequence set forth in SEQ ID NO: 70, CDR2 consisting of the amino acid sequence set forth in SEQ ID NO: 71, and CDR3 consisting of the amino acid sequence set forth in SEQ ID NO: 72.

VH containing the amino acid sequence having the sequence identity to the amino acid sequence set forth in SEQ ID NO: 29 has the activity of VH of the anti-HER2 antibody, i.e., binding activity against HER2. Examples of the amino acid sequence having the sequence identity to the amino acid sequence set forth in SEQ ID NO: 29 include amino acid sequences derived from the amino acid sequence set forth in SEQ ID NO: 29 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 30 has the activity of VL of the anti-HER2 antibody, i.e., binding activity against HER2. Examples of the amino acid sequence having the sequence identity to the amino acid sequence set forth in SEQ ID NO: 30 include amino acid sequences derived from the amino acid sequence set forth in SEQ ID NO: 6 by the deletion, substitution, insertion, or addition of, for example, 1 to 20, preferably 1 to 10, more preferably 1 to 5 amino acids.

Hereinafter, an embodiment in which the antibody is an anti-DLL3 antibody will be described.

The anti-DLL3 antibody is an antibody that binds to DLL3. The anti-DLL3 antibody is preferably an antibody that specifically binds to DLL3. The anti-DLL3 antibody may be a polyclonal antibody or a monoclonal antibody and is preferably a monoclonal antibody. The anti-DLL3 antibody may be a human-type chimeric antibody, a humanized antibody, or a human antibody and is preferably a humanized antibody.

In one embodiment, the anti-DLL3 antibody is an antibody having a heavy chain variable region (VH) and a light chain variable region (VL) of rovalpituzumab.

In one embodiment, the anti-DLL3 antibody contains a heavy chain variable region (VH) containing CDR1 consisting of the amino acid sequence set forth in SEQ ID NO: 73, CDR2 consisting of the amino acid sequence set forth in SEQ ID NO: 74, and CDR3 consisting of the amino acid sequence set forth in SEQ ID NO: 75, and a light chain variable region (VL) containing CDR1 consisting of the amino acid sequence set forth in SEQ ID NO: 76, CDR2 consisting of the amino acid sequence set forth in SEQ ID NO: 77, and CDR3 consisting of the amino acid sequence set forth in SEQ ID NO: 78. CDR1, CDR2, and CDR3 are CDR1, CDR2 and, CDR3, respectively, based on the Kabat numbering.

In an alternative embodiment, the amino acid sequence of VH of the anti-DLL3 antibody contains the amino acid sequence set forth in SEQ ID NO: 31, and the amino acid sequence of VL of the anti-DLL3 antibody contains the amino acid sequence set forth in SEQ ID NO: 32. The amino acid sequence of VH of the anti-DLL3 antibody may consist of the amino acid sequence set forth in SEQ ID NO: 31, and the amino acid sequence of VL of the anti-DLL3 antibody may consist of the amino acid sequence set forth in SEQ ID NO: 32.

As shown in the table below, VH containing the amino acid sequence set forth in SEQ ID NO: 31 contains CDR1 consisting of the amino acid sequence set forth in SEQ ID NO: 73 (underlined in the table below), CDR2 consisting of the amino acid sequence set forth in SEQ ID NO: 74 (underlined in the table below), and CDR3 consisting of the amino acid sequence set forth in SEQ ID NO: 75 (underlined in the table below), and VL containing the amino acid sequence set forth in SEQ ID NO: 32 contains CDR1 consisting of the amino acid sequence set forth in SEQ ID NO: 76 (underlined in the table below), CDR2 consisting of the amino acid sequence set forth in SEQ ID NO: 77 (underlined in the table below), and CDR3 consisting of the amino acid sequence set forth in SEQ ID NO: 78 (underlined in the table below).

**[Table 5]**

| |
|---|
| SEQ ID NO: 31 |
| QVQLVQSGAEVKKPGASVKVSCKASGYTFTNYGMNWVRQAPGQGLEWMGWINTYTGEPTYAD DFKGRVTMTTDTSTSTAYMELRSLRSDDTAVYYCARIGDSSPSDYWGQGTLVTVSS |
| SEQ ID NO: 32 |
| EIVMTQSPATLSVSPGERATLSCKASQSVSNDVVWYQQKPGQAPRLLIYYASNRYTGIPARFSGS GSGTEFTLTISSLQSEDFAVYYCQQDYTSPWTFGQGTKLEIK |

In a further alternative embodiment, the amino acid sequence of VH of the anti-DLL3 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 31, and the amino acid sequence of VL of the anti-DLL3 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 32. The amino acid sequence of VH of the anti-DLL3 antibody may consist of an amino acid sequence having 90% or higher sequence identity to the amino acid sequence set forth in SEQ ID NO: 31, and the amino acid sequence of VL of the anti-DLL3 antibody may consist of an amino acid sequence having 90% or higher sequence identity to the amino acid sequence set forth in SEQ ID NO: 32. The amino acid sequence having 90% or higher sequence identity to the amino acid sequence set forth in SEQ ID NO: 31 may be the amino acid sequence set forth in SEQ ID NO: 31, and the amino acid sequence having 90% or higher sequence identity to the amino acid sequence set forth in SEQ ID NO: 32 may be the amino acid sequence set forth in SEQ ID NO: 32.

VH containing the amino acid sequence having the sequence identity to the amino acid sequence set forth in SEQ ID NO: 31 preferably contains CDR1 consisting of the amino acid sequence set forth in SEQ ID NO: 73, CDR2 consisting of the amino acid sequence set forth in SEQ ID NO: 74, and CDR3 consisting of the amino acid sequence set forth in SEQ ID NO: 75.

VL containing the amino acid sequence having the sequence identity to the amino acid sequence set forth in SEQ ID NO: 32 preferably contains CDR1 consisting of the amino acid sequence set forth in SEQ ID NO: 76, CDR2 consisting of the amino acid sequence set forth in SEQ ID NO: 77, and CDR3 consisting of the amino acid sequence set forth in SEQ ID NO: 78.

VH containing the amino acid sequence having the sequence identity to the amino acid sequence set forth in SEQ ID NO: 31 has the activity of VH of the anti-DLL3 antibody, i.e., binding activity against DLL3. Examples of the amino acid sequence having the sequence identity to the amino acid sequence set forth in SEQ ID NO: 31 include amino acid sequences derived from the amino acid sequence set forth in SEQ ID NO: 31 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 32 has the activity of VL of the anti-DLL3 antibody, i.e., binding activity against DLL3. Examples of the amino acid sequence having the sequence identity to the amino acid sequence set forth in SEQ ID NO: 32 include amino acid sequences derived from the amino acid sequence set forth in SEQ ID NO: 32 by the deletion, substitution, insertion, or addition of, for example, 1 to 20, preferably 1 to 10, more preferably 1 to 5 amino acids.

Hereinafter, an embodiment in which the antibody is an anti-GM2 antibody will be described.

The anti-GM2 antibody is an antibody that binds to GM2. The anti-GM2 antibody is preferably an antibody that specifically binds to GM2. The anti-GM2 antibody may be a polyclonal antibody or a monoclonal antibody and is preferably a monoclonal antibody. The anti-GM2 antibody may be a human-type chimeric antibody, a humanized antibody, or a human antibody and is preferably a humanized antibody.

In one embodiment, the anti-GM2 antibody is an antibody having a heavy chain variable region (VH) and a light chain variable region (VL) of KM8969 (WO 2001/023431).

In one embodiment, the anti-GM2 antibody contains a heavy chain variable region (VH) containing CDR1 consisting of the amino acid sequence set forth in SEQ ID NO: 79, CDR2 consisting of the amino acid sequence set forth in SEQ ID NO: 80, and CDR3 consisting of the amino acid sequence set forth in SEQ ID NO: 81, and a light chain variable region (VL) containing CDR1 consisting of the amino acid sequence set forth in SEQ ID NO: 82, CDR2 consisting of the amino acid sequence set forth in SEQ ID NO: 83, and CDR3 consisting of the amino acid sequence set forth in SEQ ID NO: 84. CDR1, CDR2, and CDR3 are CDR1, CDR2 and, CDR3, respectively, based on the Kabat numbering.

In an alternative embodiment, the amino acid sequence of VH of the anti-GM2 antibody contains the amino acid sequence set forth in SEQ ID NO: 33, and the amino acid sequence of VL of the anti-GM2 antibody contains the amino acid sequence set forth in SEQ ID NO: 34. The amino acid sequence of VH of the anti-GM2 antibody may consist of the amino acid sequence set forth in SEQ ID NO: 33, and the amino acid sequence of VL of the anti-GM2 antibody may consist of the amino acid sequence set forth in SEQ ID NO: 34.

As shown in the table below, VH containing the amino acid sequence set forth in SEQ ID NO: 33 contains CDR1 consisting of the amino acid sequence set forth in SEQ ID NO: 79 (underlined in the table below), CDR2 consisting of the amino acid sequence set forth in SEQ ID NO: 80 (underlined in the table below), and CDR3 consisting of the amino acid sequence set forth in SEQ ID NO: 81 (underlined in the table below), and VL containing the amino acid sequence set forth in SEQ ID NO: 34 contains CDR1 consisting of the amino acid sequence set forth in SEQ ID NO: 82 (underlined in the table below), CDR2 consisting of the amino acid sequence set forth in SEQ ID NO: 83 (underlined in the table below), and CDR3 consisting of the amino acid sequence set forth in SEQ ID NO: 84 (underlined in the table below).

**[Table 6]**

| |
|---|
| SEQ ID NO: 33 |
| EVQLVQSGAEVKKPGASVKVSCKASGYTFTDYNMDWVKQSPGQGLEWMGYIYPNNGGTGYNQ KFKSKVTITVDTSTSTAYMELHSLRSEDTAVYYCATYGHYYGYMFAYWGQGTLVTVSS |
| SEQ ID NO: 34 |
| DIQLTQSPSSLSASPGDRVTITCSASSSVSYMHWFQQKPGKAPKLWIYSTSNLASGVPARFSGS GSGTSYSLTISRLQPEDIATYYCQQRSSYPYTFGGGTKVEIK |

In a further alternative embodiment, the amino acid sequence of VH of the anti-GM2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 33, and the amino acid sequence of VL of the anti-GM2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 34. The amino acid sequence of VH of the anti-GM2 antibody may consist of an amino acid sequence having 90% or higher sequence identity to the amino acid sequence set forth in SEQ ID NO: 33, and the amino acid sequence of VL of the anti-GM2 antibody may consist of an amino acid sequence having 90% or higher sequence identity to the amino acid sequence set forth in SEQ ID NO: 34. The amino acid sequence having 90% or higher sequence identity to the amino acid sequence set forth in SEQ ID NO: 33 may be the amino acid sequence set forth in SEQ ID NO: 33, and the amino acid sequence having 90% or higher sequence identity to the amino acid sequence set forth in SEQ ID NO: 34 may be the amino acid sequence set forth in SEQ ID NO: 34.

VH containing the amino acid sequence having the sequence identity to the amino acid sequence set forth in SEQ ID NO: 33 preferably contains CDR1 consisting of the amino acid sequence set forth in SEQ ID NO: 79, CDR2 consisting of the amino acid sequence set forth in SEQ ID NO: 80, and CDR3 consisting of the amino acid sequence set forth in SEQ ID NO: 81.

VL containing the amino acid sequence having the sequence identity to the amino acid sequence set forth in SEQ ID NO: 34 preferably contains CDR1 consisting of the amino acid sequence set forth in SEQ ID NO: 82, CDR2 consisting of the amino acid sequence set forth in SEQ ID NO: 83, and CDR3 consisting of the amino acid sequence set forth in SEQ ID NO: 84.

VH containing the amino acid sequence having the sequence identity to the amino acid sequence set forth in SEQ ID NO: 33 has the activity of VH of the anti-GM2 antibody, i.e., binding activity against GM2. Examples of the amino acid sequence having the sequence identity to the amino acid sequence set forth in SEQ ID NO: 33 include amino acid sequences derived from the amino acid sequence set forth in SEQ ID NO: 33 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 34 has the activity of VL of the anti-GM2 antibody, i.e., binding activity against GM2. Examples of the amino acid sequence having the sequence identity to the amino acid sequence set forth in SEQ ID NO: 34 include amino acid sequences derived from the amino acid sequence set forth in SEQ ID NO: 34 by the deletion, substitution, insertion, or addition of, for example, 1 to 20, preferably 1 to 10, more preferably 1 to 5 amino acids.

Hereinafter, an embodiment in which the antibody is an anti-EphA2 antibody will be described.

The anti-EphA2 antibody is an antibody that binds to EphA2. The anti-EphA2 antibody is preferably an antibody that specifically binds to EphA2. The anti-EphA2 antibody may be a polyclonal antibody or a monoclonal antibody and is preferably a monoclonal antibody. The anti-EphA2 antibody may be a human-type chimeric antibody, a humanized antibody, or a human antibody and is preferably a humanized antibody.

In one embodiment, the anti-EphA2 antibody is an antibody having a heavy chain variable region (VH) and a light chain variable region (VL) of 1C1 (U.S. Patent Application Publication No. 2009/0304721).

In one embodiment, the anti-EphA2 antibody contains a heavy chain variable region (VH) containing CDR1 consisting of the amino acid sequence set forth in SEQ ID NO: 85, CDR2 consisting of the amino acid sequence set forth in SEQ ID NO: 86, and CDR3 consisting of the amino acid sequence set forth in SEQ ID NO: 87, and a light chain variable region (VL) containing CDR1 consisting of the amino acid sequence set forth in SEQ ID NO: 88, CDR2 consisting of the amino acid sequence set forth in SEQ ID NO: 89, and CDR3 consisting of the amino acid sequence set forth in SEQ ID NO: 90. CDR1, CDR2, and CDR3 are CDR1, CDR2 and, CDR3, respectively, based on the Kabat numbering.

In an alternative embodiment, the amino acid sequence of VH of the anti-EphA2 antibody contains the amino acid sequence set forth in SEQ ID NO: 35, and the amino acid sequence of VL of the anti-EphA2 antibody contains the amino acid sequence set forth in SEQ ID NO: 36. The amino acid sequence of VH of the anti-EphA2 antibody may consist of the amino acid sequence set forth in SEQ ID NO: 35, and the amino acid sequence of VL of the anti-EphA2 antibody may consist of the amino acid sequence set forth in SEQ ID NO: 36.

As shown in the table below, VH containing the amino acid sequence set forth in SEQ ID NO: 35 contains CDR1 consisting of the amino acid sequence set forth in SEQ ID NO: 85 (underlined in the table below), CDR2 consisting of the amino acid sequence set forth in SEQ ID NO: 86 (underlined in the table below), and CDR3 consisting of the amino acid sequence set forth in SEQ ID NO: 87 (underlined in the table below), and VL containing the amino acid sequence set forth in SEQ ID NO: 36 contains CDR1 consisting of the amino acid sequence set forth in SEQ ID NO: 88 (underlined in the table below), CDR2 consisting of the amino acid sequence set forth in SEQ ID NO: 89 (underlined in the table below), and CDR3 consisting of the amino acid sequence set forth in SEQ ID NO: 90 (underlined in the table below).

**[Table 7]**

| |
|---|
| SEQ ID NO: 35 |
| EVQLLESGGGLVQPGGSLRLSCAASGFTFSHYMMAWVRQAPGKGLEWVSRIGPSGGPTHYADS VKGRFTISRDNSKNTLYLQMNSLRAEDTAVYYCAGYDSGYDYVAVAGPAEYFQHWGQGTLVTV SS |
| SEQ ID NO: 36 |
| DIQMTQSPSSLSASVGDRVTITCRASQSISTWLAWYQQKPGKAPKLLIYKASNLHTGVPSRFSGS GSGTEFSLTISGLQPDDFATYYCQQYNSYSRTFGQGTKVEIK |

In a further alternative embodiment, the amino acid sequence of VH of the anti-EphA2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 35, and the amino acid sequence of VL of the anti-EphA2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 36. The amino acid sequence of VH of the anti-EphA2 antibody may consist of an amino acid sequence having 90% or higher sequence identity to the amino acid sequence set forth in SEQ ID NO: 35, and the amino acid sequence of VL of the anti-EphA2 antibody may consist of an amino acid sequence having 90% or higher sequence identity to the amino acid sequence set forth in SEQ ID NO: 36. The amino acid sequence having 90% or higher sequence identity to the amino acid sequence set forth in SEQ ID NO: 35 may be the amino acid sequence set forth in SEQ ID NO: 35, and the amino acid sequence having 90% or higher sequence identity to the amino acid sequence set forth in SEQ ID NO: 36 may be the amino acid sequence set forth in SEQ ID NO: 36.

VH containing the amino acid sequence having the sequence identity to the amino acid sequence set forth in SEQ ID NO: 35 preferably contains CDR1 consisting of the amino acid sequence set forth in SEQ ID NO: 85, CDR2 consisting of the amino acid sequence set forth in SEQ ID NO: 86, and CDR3 consisting of the amino acid sequence set forth in SEQ ID NO: 87.

VL containing the amino acid sequence having the sequence identity to the amino acid sequence set forth in SEQ ID NO: 36 preferably contains CDR1 consisting of the amino acid sequence set forth in SEQ ID NO: 88, CDR2 consisting of the amino acid sequence set forth in SEQ ID NO: 89, and CDR3 consisting of the amino acid sequence set forth in SEQ ID NO: 90.

VH containing the amino acid sequence having the sequence identity to the amino acid sequence set forth in SEQ ID NO: 35 has the activity of VH of the anti-EphA2 antibody, i.e., binding activity against EphA2. Examples of the amino acid sequence having the sequence identity to the amino acid sequence set forth in SEQ ID NO: 35 include amino acid sequences derived from the amino acid sequence set forth in SEQ ID NO: 35 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 36 has the activity of VL of the anti-EphA2 antibody, i.e., binding activity against EphA2. Examples of the amino acid sequence having the sequence identity to the amino acid sequence set forth in SEQ ID NO: 36 include amino acid sequences derived from the amino acid sequence set forth in SEQ ID NO: 36 by the deletion, substitution, insertion, or addition of, for example, 1 to 20, preferably 1 to 10, more preferably 1 to 5 amino acids.

Hereinafter, an embodiment in which the antibody is an anti-FAP antibody will be described.

The anti-FAP antibody is an antibody that binds to FAP. The anti-FAP antibody is preferably an antibody that specifically binds to FAP. The anti-FAP antibody may be a polyclonal antibody or a monoclonal antibody and is preferably a monoclonal antibody. The anti-FAP antibody may be a human-type chimeric antibody, a humanized antibody, or a human antibody and is preferably a humanized antibody.

In one embodiment, the anti-FAP antibody is an antibody having a heavy chain variable region (VH) and a light chain variable region (VL) of hu36 (U.S. Patent No. 10864278).

In one embodiment, the anti-FAP antibody contains a heavy chain variable region (VH) containing CDR1 consisting of the amino acid sequence set forth in SEQ ID NO: 91, CDR2 consisting of the amino acid sequence set forth in SEQ ID NO: 92, and CDR3 consisting of the amino acid sequence set forth in SEQ ID NO: 93, and a light chain variable region (VL) containing CDR1 consisting of the amino acid sequence set forth in SEQ ID NO: 94, CDR2 consisting of the amino acid sequence set forth in SEQ ID NO: 95, and CDR3 consisting of the amino acid sequence set forth in SEQ ID NO: 96. CDR1, CDR2, and CDR3 are CDR1, CDR2 and, CDR3, respectively, based on the Kabat numbering.

In an alternative embodiment, the amino acid sequence of VH of the anti-FAP antibody contains the amino acid sequence set forth in SEQ ID NO: 37, and the amino acid sequence of VL of the anti-FAP antibody contains the amino acid sequence set forth in SEQ ID NO: 38. The amino acid sequence of VH of the anti-FAP antibody may consist of the amino acid sequence set forth in SEQ ID NO: 37, and the amino acid sequence of VL of the anti-FAP antibody may consist of the amino acid sequence set forth in SEQ ID NO: 38.

As shown in the table below, VH containing the amino acid sequence set forth in SEQ ID NO: 37 contains CDR1 consisting of the amino acid sequence set forth in SEQ ID NO: 91 (underlined in the table below), CDR2 consisting of the amino acid sequence set forth in SEQ ID NO: 92 (underlined in the table below), and CDR3 consisting of the amino acid sequence set forth in SEQ ID NO: 93 (underlined in the table below), and VL containing the amino acid sequence set forth in SEQ ID NO: 38 contains CDR1 consisting of the amino acid sequence set forth in SEQ ID NO: 94 (underlined in the table below), CDR2 consisting of the amino acid sequence set forth in SEQ ID NO: 95 (underlined in the table below), and CDR3 consisting of the amino acid sequence set forth in SEQ ID NO: 96 (underlined in the table below).

**[Table 8]**

| |
|---|
| SEQ ID NO: 37 |
| QVQLKQSGAELVKPGASVKLSCKTSGYTFTENIIHWVKQRSGQGLEWIGWFHPGSGSIKYNEKKD KATLTADKSSSTVYMELSRLTSEDSAVYFCARHGGTGRGAMDYWGQGTSVTVSS |
| SEQ ID NO: 38 |
| DILMTQSPASSVVSLSGQRATISCRASKSVSTSAYSYMHWYQQKPGQPPKLLIYLASNLESGVPP RFSGSGSGTDFTLNIHPVEEEDAATYYCQHSRELPYTFGGGTKLEIK |

In a further alternative embodiment, the amino acid sequence of VH of the anti-FAP antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 37, and the amino acid sequence of VL of the anti-FAP antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 38. The amino acid sequence of VH of the anti-FAP antibody may consist of an amino acid sequence having 90% or higher sequence identity to the amino acid sequence set forth in SEQ ID NO: 37, and the amino acid sequence of VL of the anti-FAP antibody may consist of an amino acid sequence having 90% or higher sequence identity to the amino acid sequence set forth in SEQ ID NO: 38. The amino acid sequence having 90% or higher sequence identity to the amino acid sequence set forth in SEQ ID NO: 37 may be the amino acid sequence set forth in SEQ ID NO: 37, and the amino acid sequence having 90% or higher sequence identity to the amino acid sequence set forth in SEQ ID NO: 38 may be the amino acid sequence set forth in SEQ ID NO: 38.

VH containing the amino acid sequence having the sequence identity to the amino acid sequence set forth in SEQ ID NO: 37 preferably contains CDR1 consisting of the amino acid sequence set forth in SEQ ID NO: 91, CDR2 consisting of the amino acid sequence set forth in SEQ ID NO: 92, and CDR3 consisting of the amino acid sequence set forth in SEQ ID NO: 93.

VL containing the amino acid sequence having the sequence identity to the amino acid sequence set forth in SEQ ID NO: 38 preferably contains CDR1 consisting of the amino acid sequence set forth in SEQ ID NO: 94, CDR2 consisting of the amino acid sequence set forth in SEQ ID NO: 95, and CDR3 consisting of the amino acid sequence set forth in SEQ ID NO: 96.

VH containing the amino acid sequence having the sequence identity to the amino acid sequence set forth in SEQ ID NO: 37 has the activity of VH of the anti-FAP antibody, i.e., binding activity against FAP. Examples of the amino acid sequence having the sequence identity to the amino acid sequence set forth in SEQ ID NO: 37 include amino acid sequences derived from the amino acid sequence set forth in SEQ ID NO: 37 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 38 has the activity of VL of the anti-FAP antibody, i.e., binding activity against FAP. Examples of the amino acid sequence having the sequence identity to the amino acid sequence set forth in SEQ ID NO: 38 include amino acid sequences derived from the amino acid sequence set forth in SEQ ID NO: 38 by the deletion, substitution, insertion, or addition of, for example, 1 to 20, preferably 1 to 10, more preferably 1 to 5 amino acids.

Hereinafter, an embodiment in which the antibody is an anti-SSTR2 antibody will be described.

The anti-SSTR2 antibody is an antibody that binds to SSTR2. The anti-SSTR2 antibody is preferably an antibody that specifically binds to SSTR2. The anti-SSTR2 antibody may be a polyclonal antibody or a monoclonal antibody and is preferably a monoclonal antibody. The anti-SSTR2 antibody may be a human-type chimeric antibody, a humanized antibody, or a human antibody and is preferably a humanized antibody.

In one embodiment, the anti-SSTR2 antibody is an antibody having a heavy chain variable region (VH) and a light chain variable region (VL) of anti-SSTR2 mAb (U.S. Patent Application Publication No. 2021/0340264).

In one embodiment, the anti-SSTR2 antibody contains a heavy chain variable region (VH) containing CDR1 consisting of the amino acid sequence set forth in SEQ ID NO: 97, CDR2 consisting of the amino acid sequence set forth in SEQ ID NO: 98, and CDR3 consisting of the amino acid sequence set forth in SEQ ID NO: 99, and a light chain variable region (VL) containing CDR1 consisting of the amino acid sequence set forth in SEQ ID NO: 100, CDR2 consisting of the amino acid sequence set forth in SEQ ID NO: 101, and CDR3 consisting of the amino acid sequence set forth in SEQ ID NO: 102. CDR1, CDR2, and CDR3 are CDR1, CDR2 and, CDR3, respectively, based on the Kabat numbering.

In an alternative embodiment, the amino acid sequence of VH of the anti-SSTR2 antibody contains the amino acid sequence set forth in SEQ ID NO: 39, and the amino acid sequence of VL of the anti-SSTR2 antibody contains the amino acid sequence set forth in SEQ ID NO: 40. The amino acid sequence of VH of the anti-SSTR2 antibody may consist of the amino acid sequence set forth in SEQ ID NO: 39, and the amino acid sequence of VL of the anti-SSTR2 antibody may consist of the amino acid sequence set forth in SEQ ID NO: 40.

As shown in the table below, VH containing the amino acid sequence set forth in SEQ ID NO: 39 contains CDR1 consisting of the amino acid sequence set forth in SEQ ID NO: 97 (underlined in the table below), CDR2 consisting of the amino acid sequence set forth in SEQ ID NO: 98 (underlined in the table below), and CDR3 consisting of the amino acid sequence set forth in SEQ ID NO: 99 (underlined in the table below), and VL containing the amino acid sequence set forth in SEQ ID NO: 40 contains CDR1 consisting of the amino acid sequence set forth in SEQ ID NO: 100 (underlined in the table below), CDR2 consisting of the amino acid sequence set forth in SEQ ID NO: 101 (underlined in the table below), and CDR3 consisting of the amino acid sequence set forth in SEQ ID NO: 102 (underlined in the table below).

**[Table 9]**

| |
|---|
| SEQ ID NO: 39 |
| EVKLVESGGGLVQPGGSLRLSCATSGFTFTDYHLNWVRQPPGKALEWLALIRNKRYGYRTEYSA SVKGRFTISRDNSQSILYLQMNTLRAEDSATYYCARDFYDPFAYWGQGTLVTVSA |
| SEQ ID NO: 40 |
| DVVMTQTPLSLPVSLGDQASISCRSSQSLVHSNGNTYLHWYLQRPGQSPKLLIYKVSNRFSGVP DRFSGSGSGTDFTLKISRVEAEDLGVYFCSQSTHVPFTFGSGTKLEIK |

In a further alternative embodiment, the amino acid sequence of VH of the anti-SSTR2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 39, and the amino acid sequence of VL of the anti-SSTR2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 40. The amino acid sequence of VH of the anti-SSTR2 antibody may consist of an amino acid sequence having 90% or higher sequence identity to the amino acid sequence set forth in SEQ ID NO: 39, and the amino acid sequence of VL of the anti-SSTR2 antibody may consist of an amino acid sequence having 90% or higher sequence identity to the amino acid sequence set forth in SEQ ID NO: 40. The amino acid sequence having 90% or higher sequence identity to the amino acid sequence set forth in SEQ ID NO: 39 may be the amino acid sequence set forth in SEQ ID NO: 39, and the amino acid sequence having 90% or higher sequence identity to the amino acid sequence set forth in SEQ ID NO: 40 may be the amino acid sequence set forth in SEQ ID NO: 40.

VH containing the amino acid sequence having the sequence identity to the amino acid sequence set forth in SEQ ID NO: 39 preferably contains CDR1 consisting of the amino acid sequence set forth in SEQ ID NO: 97, CDR2 consisting of the amino acid sequence set forth in SEQ ID NO: 98, and CDR3 consisting of the amino acid sequence set forth in SEQ ID NO: 99.

VL containing the amino acid sequence having the sequence identity to the amino acid sequence set forth in SEQ ID NO: 40 preferably contains CDR1 consisting of the amino acid sequence set forth in SEQ ID NO: 100, CDR2 consisting of the amino acid sequence set forth in SEQ ID NO: 101, and CDR3 consisting of the amino acid sequence set forth in SEQ ID NO: 102.

VH containing the amino acid sequence having the sequence identity to the amino acid sequence set forth in SEQ ID NO: 39 has the activity of VH of the anti-SSTR2 antibody, i.e., binding activity against SSTR2. Examples of the amino acid sequence having the sequence identity to the amino acid sequence set forth in SEQ ID NO: 39 include amino acid sequences derived from the amino acid sequence set forth in SEQ ID NO: 39 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 40 has the activity of VL of the anti-SSTR2 antibody, i.e., binding activity against SSTR2. Examples of the amino acid sequence having the sequence identity to the amino acid sequence set forth in SEQ ID NO: 40 include amino acid sequences derived from the amino acid sequence set forth in SEQ ID NO: 40 by the deletion, substitution, insertion, or addition of, for example, 1 to 20, preferably 1 to 10, more preferably 1 to 5 amino acids.

Hereinafter, an embodiment in which the antibody is an anti-CD30 antibody will be described.

The anti-CD30 antibody is an antibody that binds to CD30. The anti-CD30 antibody is preferably an antibody that specifically binds to CD30. The anti-CD30 antibody may be a polyclonal antibody or a monoclonal antibody and is preferably a monoclonal antibody. The anti-CD30 antibody may be a human-type chimeric antibody, a humanized antibody, or a human antibody and is preferably a humanized antibody.

In one embodiment, the anti-CD30 antibody is an antibody having a heavy chain variable region (VH) and a light chain variable region (VL) of brentuximab.

In one embodiment, the anti-CD30 antibody contains a heavy chain variable region (VH) containing CDR1 consisting of the amino acid sequence set forth in SEQ ID NO: 103, CDR2 consisting of the amino acid sequence set forth in SEQ ID NO: 104, and CDR3 consisting of the amino acid sequence set forth in SEQ ID NO: 105, and a light chain variable region (VL) containing CDR1 consisting of the amino acid sequence set forth in SEQ ID NO: 106, CDR2 consisting of the amino acid sequence set forth in SEQ ID NO: 107, and CDR3 consisting of the amino acid sequence set forth in SEQ ID NO: 108. CDR1, CDR2, and CDR3 are CDR1, CDR2 and, CDR3, respectively, based on the Kabat numbering.

In an alternative embodiment, the amino acid sequence of VH of the anti-CD30 antibody contains the amino acid sequence set forth in SEQ ID NO: 41, and the amino acid sequence of VL of the anti-CD30 antibody contains the amino acid sequence set forth in SEQ ID NO: 42. The amino acid sequence of VH of the anti-CD30 antibody may consist of the amino acid sequence set forth in SEQ ID NO: 41, and the amino acid sequence of VL of the anti-CD30 antibody may consist of the amino acid sequence set forth in SEQ ID NO: 42.

As shown in the table below, VH containing the amino acid sequence set forth in SEQ ID NO: 41 contains CDR1 consisting of the amino acid sequence set forth in SEQ ID NO: 103 (underlined in the table below), CDR2 consisting of the amino acid sequence set forth in SEQ ID NO: 104 (underlined in the table below), and CDR3 consisting of the amino acid sequence set forth in SEQ ID NO: 105 (underlined in the table below), and VL containing the amino acid sequence set forth in SEQ ID NO: 42 contains CDR1 consisting of the amino acid sequence set forth in SEQ ID NO: 106 (underlined in the table below), CDR2 consisting of the amino acid sequence set forth in SEQ ID NO: 107 (underlined in the table below), and CDR3 consisting of the amino acid sequence set forth in SEQ ID NO: 108 (underlined in the table below).

**[Table 10]**

| |
|---|
| SEQ ID NO: 41 |
| QIQLQQSGPEVVKPGASVKISCKASGYTFTDYYITWVKQKPGQGLEWIGWIYPGSGNTKYNEKFK GKATLTVDTSSSTAFMQLSSLTSEDTAVYFCANYGNYWFAYWGQGTQVTVSA |
| SEQ ID NO: 42 |
| DIVLTQSPASLAVSLGQRATISCKASQSVDFDGDSYMNWYQQKPGQPPKVLIYAASNLESGIPAR FSGSGSGTDFTLNIHPVEEEDAATYYCQQSNEDPWTFGGGTKLEIK |

In a further alternative embodiment, the amino acid sequence of VH of the anti-CD30 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 41, and the amino acid sequence of VL of the anti-CD30 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 42. The amino acid sequence of VH of the anti-CD30 antibody may consist of an amino acid sequence having 90% or higher sequence identity to the amino acid sequence set forth in SEQ ID NO: 41, and the amino acid sequence of VL of the anti-CD30 antibody may consist of an amino acid sequence having 90% or higher sequence identity to the amino acid sequence set forth in SEQ ID NO: 42. The amino acid sequence having 90% or higher sequence identity to the amino acid sequence set forth in SEQ ID NO: 41 may be the amino acid sequence set forth in SEQ ID NO: 41, and the amino acid sequence having 90% or higher sequence identity to the amino acid sequence set forth in SEQ ID NO: 42 may be the amino acid sequence set forth in SEQ ID NO: 42.

VH containing the amino acid sequence having the sequence identity to the amino acid sequence set forth in SEQ ID NO: 41 preferably contains CDR1 consisting of the amino acid sequence set forth in SEQ ID NO: 103, CDR2 consisting of the amino acid sequence set forth in SEQ ID NO: 104, and CDR3 consisting of the amino acid sequence set forth in SEQ ID NO: 105.

VL containing the amino acid sequence having the sequence identity to the amino acid sequence set forth in SEQ ID NO: 42 preferably contains CDR1 consisting of the amino acid sequence set forth in SEQ ID NO: 106, CDR2 consisting of the amino acid sequence set forth in SEQ ID NO: 107, and CDR3 consisting of the amino acid sequence set forth in SEQ ID NO: 108.

VH containing the amino acid sequence having the sequence identity to the amino acid sequence set forth in SEQ ID NO: 41 has the activity of VH of the anti-CD30 antibody, i.e., binding activity against CD30. Examples of the amino acid sequence having the sequence identity to the amino acid sequence set forth in SEQ ID NO: 41 include amino acid sequences derived from the amino acid sequence set forth in SEQ ID NO: 41 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 42 has the activity of VL of the anti-CD30 antibody, i.e., binding activity against CD30. Examples of the amino acid sequence having the sequence identity to the amino acid sequence set forth in SEQ ID NO: 42 include amino acid sequences derived from the amino acid sequence set forth in SEQ ID NO: 42 by the deletion, substitution, insertion, or addition of, for example, 1 to 20, preferably 1 to 10, more preferably 1 to 5 amino acids.

Hereinafter, an embodiment in which the antibody is an anti-BCMA antibody will be described.

The anti-BCMA antibody is an antibody that binds to BCMA. The anti-BCMA antibody is preferably an antibody that specifically binds to BCMA. The anti-BCMA antibody may be a polyclonal antibody or a monoclonal antibody and is preferably a monoclonal antibody. The anti-BCMA antibody may be a human-type chimeric antibody, a humanized antibody, or a human antibody and is preferably a humanized antibody.

In one embodiment, the anti-BCMA antibody is an antibody having a heavy chain variable region (VH) and a light chain variable region (VL) of belantamab.

In one embodiment, the anti-BCMA antibody contains a heavy chain variable region (VH) containing CDR1 consisting of the amino acid sequence set forth in SEQ ID NO: 109, CDR2 consisting of the amino acid sequence set forth in SEQ ID NO: 110, and CDR3 consisting of the amino acid sequence set forth in SEQ ID NO: 111, and a light chain variable region (VL) containing CDR1 consisting of the amino acid sequence set forth in SEQ ID NO: 112, CDR2 consisting of the amino acid sequence set forth in SEQ ID NO: 113, and CDR3 consisting of the amino acid sequence set forth in SEQ ID NO: 114. CDR1, CDR2, and CDR3 are CDR1, CDR2 and, CDR3, respectively, based on the Kabat numbering.

In an alternative embodiment, the amino acid sequence of VH of the anti-BCMA antibody contains the amino acid sequence set forth in SEQ ID NO: 43, and the amino acid sequence of VL of the anti-BCMA antibody contains the amino acid sequence set forth in SEQ ID NO: 44. The amino acid sequence of VH of the anti-BCMA antibody may consist of the amino acid sequence set forth in SEQ ID NO: 43, and the amino acid sequence of VL of the anti-BCMA antibody may consist of the amino acid sequence set forth in SEQ ID NO: 44.

As shown in the table below, VH containing the amino acid sequence set forth in SEQ ID NO: 43 contains CDR1 consisting of the amino acid sequence set forth in SEQ ID NO: 109 (underlined in the table below), CDR2 consisting of the amino acid sequence set forth in SEQ ID NO: 110 (underlined in the table below), and CDR3 consisting of the amino acid sequence set forth in SEQ ID NO: 111 (underlined in the table below), and VL containing the amino acid sequence set forth in SEQ ID NO: 44 contains CDR1 consisting of the amino acid sequence set forth in SEQ ID NO: 112 (underlined in the table below), CDR2 consisting of the amino acid sequence set forth in SEQ ID NO: 113 (underlined in the table below), and CDR3 consisting of the amino acid sequence set forth in SEQ ID NO: 114 (underlined in the table below).

**[Table 11]**

| |
|---|
| SEQ ID NO: 43 |
| QVQLVQSGAEVKKPGSSVKVSCKASGGTFSNYWMHWVRQAPGQGLEWMGATYRGHSDTYYN QKFKGRVTITADKSTSTAYMELSSLRSEDTAVYYCARGAIYDGYDVLDNWGQGTLVTVSS |
| SEQ ID NO: 44 |
| DIQMTQSPSSLSASVGDRVTITCSASQDISNYLNWYQQKPGKAPKLLIYYTSNLHSGVPSRFSGS GSGTDFTLTISSLQPEDFATYYCQQYRKLPWTFGQGTKLEIK |

In a further alternative embodiment, the amino acid sequence of VH of the anti-BCMA antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 43, and the amino acid sequence of VL of the anti-BCMA antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 44. The amino acid sequence of VH of the anti-BCMA antibody may consist of an amino acid sequence having 90% or higher sequence identity to the amino acid sequence set forth in SEQ ID NO: 43, and the amino acid sequence of VL of the anti-BCMA antibody may consist of an amino acid sequence having 90% or higher sequence identity to the amino acid sequence set forth in SEQ ID NO: 44. The amino acid sequence having 90% or higher sequence identity to the amino acid sequence set forth in SEQ ID NO: 43 may be the amino acid sequence set forth in SEQ ID NO: 43, and the amino acid sequence having 90% or higher sequence identity to the amino acid sequence set forth in SEQ ID NO: 44 may be the amino acid sequence set forth in SEQ ID NO: 44.

VH containing the amino acid sequence having the sequence identity to the amino acid sequence set forth in SEQ ID NO: 43 preferably contains CDR1 consisting of the amino acid sequence set forth in SEQ ID NO: 109, CDR2 consisting of the amino acid sequence set forth in SEQ ID NO: 110, and CDR3 consisting of the amino acid sequence set forth in SEQ ID NO: 111.

VL containing the amino acid sequence having the sequence identity to the amino acid sequence set forth in SEQ ID NO: 44 preferably contains CDR1 consisting of the amino acid sequence set forth in SEQ ID NO: 112, CDR2 consisting of the amino acid sequence set forth in SEQ ID NO: 113, and CDR3 consisting of the amino acid sequence set forth in SEQ ID NO: 114.

VH containing the amino acid sequence having the sequence identity to the amino acid sequence set forth in SEQ ID NO: 43 has the activity of VH of the anti-BCMA antibody, i.e., binding activity against BCMA. Examples of the amino acid sequence having the sequence identity to the amino acid sequence set forth in SEQ ID NO: 43 include amino acid sequences derived from the amino acid sequence set forth in SEQ ID NO: 43 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 44 has the activity of VL of the anti-BCMA antibody, i.e., binding activity against BCMA. Examples of the amino acid sequence having the sequence identity to the amino acid sequence set forth in SEQ ID NO: 44 include amino acid sequences derived from the amino acid sequence set forth in SEQ ID NO: 44 by the deletion, substitution, insertion, or addition of, for example, 1 to 20, preferably 1 to 10, more preferably 1 to 5 amino acids.

Hereinafter, an embodiment in which the antibody is an anti-CD33 antibody will be described.

The anti-CD33 antibody is an antibody that binds to CD33. The anti-CD33 antibody is preferably an antibody that specifically binds to CD33. The anti-CD33 antibody may be a polyclonal antibody or a monoclonal antibody and is preferably a monoclonal antibody. The anti-CD33 antibody may be a human-type chimeric antibody, a humanized antibody, or a human antibody and is preferably a humanized antibody.

In one embodiment, the anti-CD33 antibody is an antibody having a heavy chain variable region (VH) and a light chain variable region (VL) of gemtuzumab.

In one embodiment, the anti-CD33 antibody contains a heavy chain variable region (VH) containing CDR1 consisting of the amino acid sequence set forth in SEQ ID NO: 115, CDR2 consisting of the amino acid sequence set forth in SEQ ID NO: 116, and CDR3 consisting of the amino acid sequence set forth in SEQ ID NO: 117, and a light chain variable region (VL) containing CDR1 consisting of the amino acid sequence set forth in SEQ ID NO: 118, CDR2 consisting of the amino acid sequence set forth in SEQ ID NO: 119, and CDR3 consisting of the amino acid sequence set forth in SEQ ID NO: 120. CDR1, CDR2, and CDR3 are CDR1, CDR2 and, CDR3, respectively, based on the Kabat numbering.

In an alternative embodiment, the amino acid sequence of VH of the anti-CD33 antibody contains the amino acid sequence set forth in SEQ ID NO: 45, and the amino acid sequence of VL of the anti-CD33 antibody contains the amino acid sequence set forth in SEQ ID NO: 46. The amino acid sequence of VH of the anti-CD33 antibody may consist of the amino acid sequence set forth in SEQ ID NO: 45, and the amino acid sequence of VL of the anti-CD33 antibody may consist of the amino acid sequence set forth in SEQ ID NO: 46.

As shown in the table below, VH containing the amino acid sequence set forth in SEQ ID NO: 45 contains CDR1 consisting of the amino acid sequence set forth in SEQ ID NO: 115 (underlined in the table below), CDR2 consisting of the amino acid sequence set forth in SEQ ID NO: 116 (underlined in the table below), and CDR3 consisting of the amino acid sequence set forth in SEQ ID NO: 117 (underlined in the table below), and VL containing the amino acid sequence set forth in SEQ ID NO: 46 contains CDR1 consisting of the amino acid sequence set forth in SEQ ID NO: 118 (underlined in the table below), CDR2 consisting of the amino acid sequence set forth in SEQ ID NO: 119 (underlined in the table below), and CDR3 consisting of the amino acid sequence set forth in SEQ ID NO: 120 (underlined in the table below).

**[Table 12]**

| |
|---|
| SEQ ID NO: 45 |
| QVQLVQSGAEVKKPGASVKVSCKASGYTFTNYDINWVRQAPGQGLEWIGWIYPGDGSTKYNEKF KAKATLTADTSTSTAYMELRSLRSDDTAVYYCASGYEDAMDYWGQGTTVTVSS |
| SEQ ID NO: 46 |
| DIQMTQSPSSLSASVGDRVTINCKASQDINSYLSWFQQKPGKAPKTLIYRANRLVDGVPSRFSGS GSGQDYTLTISSLQPEDFATYYCLQYDEFPLTFGGGTKVEIK |

In a further alternative embodiment, the amino acid sequence of VH of the anti-CD33 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 45, and the amino acid sequence of VL of the anti-CD33 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 46. The amino acid sequence of VH of the anti-CD33 antibody may consist of an amino acid sequence having 90% or higher sequence identity to the amino acid sequence set forth in SEQ ID NO: 45, and the amino acid sequence of VL of the anti-CD33 antibody may consist of an amino acid sequence having 90% or higher sequence identity to the amino acid sequence set forth in SEQ ID NO: 46. The amino acid sequence having 90% or higher sequence identity to the amino acid sequence set forth in SEQ ID NO: 45 may be the amino acid sequence set forth in SEQ ID NO: 45, and the amino acid sequence having 90% or higher sequence identity to the amino acid sequence set forth in SEQ ID NO: 46 may be the amino acid sequence set forth in SEQ ID NO: 46.

VH containing the amino acid sequence having the sequence identity to the amino acid sequence set forth in SEQ ID NO: 45 preferably contains CDR1 consisting of the amino acid sequence set forth in SEQ ID NO: 115, CDR2 consisting of the amino acid sequence set forth in SEQ ID NO: 116, and CDR3 consisting of the amino acid sequence set forth in SEQ ID NO: 117.

VL containing the amino acid sequence having the sequence identity to the amino acid sequence set forth in SEQ ID NO: 46 preferably contains CDR1 consisting of the amino acid sequence set forth in SEQ ID NO: 118, CDR2 consisting of the amino acid sequence set forth in SEQ ID NO: 119, and CDR3 consisting of the amino acid sequence set forth in SEQ ID NO: 120.

VH containing the amino acid sequence having the sequence identity to the amino acid sequence set forth in SEQ ID NO: 45 has the activity of VH of the anti-CD33 antibody, i.e., binding activity against CD33. Examples of the amino acid sequence having the sequence identity to the amino acid sequence set forth in SEQ ID NO: 45 include amino acid sequences derived from the amino acid sequence set forth in SEQ ID NO: 45 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 46 has the activity of VL of the anti-CD33 antibody, i.e., binding activity against CD33. Examples of the amino acid sequence having the sequence identity to the amino acid sequence set forth in SEQ ID NO: 46 include amino acid sequences derived from the amino acid sequence set forth in SEQ ID NO: 46 by the deletion, substitution, insertion, or addition of, for example, 1 to 20, preferably 1 to 10, more preferably 1 to 5 amino acids.

Hereinafter, an embodiment in which the antibody is an anti-FoLRα antibody will be described.

The anti-FoLRα antibody is an antibody that binds to FoLRα. The anti-FoLRα antibody is preferably an antibody that specifically binds to FoLRα. The anti-FoLRα antibody may be a polyclonal antibody or a monoclonal antibody and is preferably a monoclonal antibody. The anti-FoLRα antibody may be a human-type chimeric antibody, a humanized antibody, or a human antibody and is preferably a humanized antibody.

In one embodiment, the anti-FoLRα antibody is an antibody having a heavy chain variable region (VH) and a light chain variable region (VL) of mirvetuximab.

In one embodiment, the anti-FoLRα antibody contains a heavy chain variable region (VH) containing CDR1 consisting of the amino acid sequence set forth in SEQ ID NO: 121, CDR2 consisting of the amino acid sequence set forth in SEQ ID NO: 122, and CDR3 consisting of the amino acid sequence set forth in SEQ ID NO: 123, and a light chain variable region (VL) containing CDR1 consisting of the amino acid sequence set forth in SEQ ID NO: 124, CDR2 consisting of the amino acid sequence set forth in SEQ ID NO: 125, and CDR3 consisting of the amino acid sequence set forth in SEQ ID NO: 126. CDR1, CDR2, and CDR3 are CDR1, CDR2 and, CDR3, respectively, based on the Kabat numbering.

In an alternative embodiment, the amino acid sequence of VH of the anti-FoLRα antibody contains the amino acid sequence set forth in SEQ ID NO: 47, and the amino acid sequence of VL of the anti-FoLRα antibody contains the amino acid sequence set forth in SEQ ID NO: 48. The amino acid sequence of VH of the anti-FoLRα antibody may consist of the amino acid sequence set forth in SEQ ID NO: 47, and the amino acid sequence of VL of the anti-FoLRα antibody may consist of the amino acid sequence set forth in SEQ ID NO: 48.

As shown in the table below, VH containing the amino acid sequence set forth in SEQ ID NO: 47 contains CDR1 consisting of the amino acid sequence set forth in SEQ ID NO: 121 (underlined in the table below), CDR2 consisting of the amino acid sequence set forth in SEQ ID NO: 122 (underlined in the table below), and CDR3 consisting of the amino acid sequence set forth in SEQ ID NO: 123 (underlined in the table below), and VL containing the amino acid sequence set forth in SEQ ID NO: 48 contains CDR1 consisting of the amino acid sequence set forth in SEQ ID NO: 124 (underlined in the table below), CDR2 consisting of the amino acid sequence set forth in SEQ ID NO: 125 (underlined in the table below), and CDR3 consisting of the amino acid sequence set forth in SEQ ID NO: 126 (underlined in the table below).

**[Table 13]**

| |
|---|
| SEQ ID NO: 47 |
| EVQLVESGGGLVQPGGSLRLSCAASGFTFTDFYMNWVRQPPGKAPEWLGFIRNKANGYTTEFN PSVKGRFTISRDNSKNSLYLQMNSLKTEDTATYYCARTLYGYAYYYVMDAWGQGTLVTVSS |
| SEQ ID NO: 48 |
| DIQMTQSPSSLSASLGDRVTITCRTSEDIFRNLAWYQQKPGKAPKLLIYDTNRLADGVPSRFSGS GSGTDYTLTISSLQPEDFATYFCQQYDNYPLTFGQGTKLEIK |

In a further alternative embodiment, the amino acid sequence of VH of the anti-FoLRo antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 47, and the amino acid sequence of VL of the anti-FoLRo antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 48. The amino acid sequence of VH of the anti-FoLRα antibody may consist of an amino acid sequence having 90% or higher sequence identity to the amino acid sequence set forth in SEQ ID NO: 47, and the amino acid sequence of VL of the anti-FoLRα antibody may consist of an amino acid sequence having 90% or higher sequence identity to the amino acid sequence set forth in SEQ ID NO: 48. The amino acid sequence having 90% or higher sequence identity to the amino acid sequence set forth in SEQ ID NO: 47 may be the amino acid sequence set forth in SEQ ID NO: 47, and the amino acid sequence having 90% or higher sequence identity to the amino acid sequence set forth in SEQ ID NO: 48 may be the amino acid sequence set forth in SEQ ID NO: 48.

VH containing the amino acid sequence having the sequence identity to the amino acid sequence set forth in SEQ ID NO: 47 preferably contains CDR1 consisting of the amino acid sequence set forth in SEQ ID NO: 121, CDR2 consisting of the amino acid sequence set forth in SEQ ID NO: 122, and CDR3 consisting of the amino acid sequence set forth in SEQ ID NO: 123.

VL containing the amino acid sequence having the sequence identity to the amino acid sequence set forth in SEQ ID NO: 48 preferably contains CDR1 consisting of the amino acid sequence set forth in SEQ ID NO: 124, CDR2 consisting of the amino acid sequence set forth in SEQ ID NO: 125, and CDR3 consisting of the amino acid sequence set forth in SEQ ID NO: 126.

VH containing the amino acid sequence having the sequence identity to the amino acid sequence set forth in SEQ ID NO: 47 has the activity of VH of the anti-FoLRα antibody, i.e., binding activity against FoLRα. Examples of the amino acid sequence having the sequence identity to the amino acid sequence set forth in SEQ ID NO: 47 include amino acid sequences derived from the amino acid sequence set forth in SEQ ID NO: 47 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 48 has the activity of VL of the anti-FoLRα antibody, i.e., binding activity against FoLRα. Examples of the amino acid sequence having the sequence identity to the amino acid sequence set forth in SEQ ID NO: 48 include amino acid sequences derived from the amino acid sequence set forth in SEQ ID NO: 48 by the deletion, substitution, insertion, or addition of, for example, 1 to 20, preferably 1 to 10, more preferably 1 to 5 amino acids.

Hereinafter, an embodiment in which the antibody is an anti-CD25 antibody will be described.

The anti-CD25 antibody is an antibody that binds to CD25. The anti-CD25 antibody is preferably an antibody that specifically binds to CD25. The anti-CD25 antibody may be a polyclonal antibody or a monoclonal antibody and is preferably a monoclonal antibody. The anti-CD25 antibody may be a human-type chimeric antibody, a humanized antibody, or a human antibody and is preferably a humanized antibody.

In one embodiment, the anti-CD25 antibody is an antibody having a heavy chain variable region (VH) and a light chain variable region (VL) of camidanlumab.

In one embodiment, the anti-CD25 antibody contains a heavy chain variable region (VH) containing CDR1 consisting of the amino acid sequence set forth in SEQ ID NO: 127, CDR2 consisting of the amino acid sequence set forth in SEQ ID NO: 128, and CDR3 consisting of the amino acid sequence set forth in SEQ ID NO: 129, and a light chain variable region (VL) containing CDR1 consisting of the amino acid sequence set forth in SEQ ID NO: 130, CDR2 consisting of the amino acid sequence set forth in SEQ ID NO: 131, and CDR3 consisting of the amino acid sequence set forth in SEQ ID NO: 132. CDR1, CDR2, and CDR3 are CDR1, CDR2 and, CDR3, respectively, based on the Kabat numbering.

In an alternative embodiment, the amino acid sequence of VH of the anti-CD25 antibody contains the amino acid sequence set forth in SEQ ID NO: 49, and the amino acid sequence of VL of the anti-CD25 antibody contains the amino acid sequence set forth in SEQ ID NO: 50. The amino acid sequence of VH of the anti-CD25 antibody may consist of the amino acid sequence set forth in SEQ ID NO: 49, and the amino acid sequence of VL of the anti-CD25 antibody may consist of the amino acid sequence set forth in SEQ ID NO: 50.

As shown in the table below, VH containing the amino acid sequence set forth in SEQ ID NO: 49 contains CDR1 consisting of the amino acid sequence set forth in SEQ ID NO: 127 (underlined in the table below), CDR2 consisting of the amino acid sequence set forth in SEQ ID NO: 128 (underlined in the table below), and CDR3 consisting of the amino acid sequence set forth in SEQ ID NO: 129 (underlined in the table below), and VL containing the amino acid sequence set forth in SEQ ID NO: 50 contains CDR1 consisting of the amino acid sequence set forth in SEQ ID NO: 130 (underlined in the table below), CDR2 consisting of the amino acid sequence set forth in SEQ ID NO: 131 (underlined in the table below), and CDR3 consisting of the amino acid sequence set forth in SEQ ID NO: 132 (underlined in the table below).

**[Table 14]**

| |
|---|
| SEQ ID NO: 49 |
| QVQLVQSGAEVKKPGSSVKVSCKASGGTFSRYIINWVRQAPGQGLEWMGRIIPILGVENYAQKFQ GRVTITADKSTSTAYMELSSLRSEDTAVYYCARKDWFDYWGQGTLVTVSS |
| SEQ ID NO: 50 |
| EIVLTQSPGTLSLSPGERATLSCRASQSVSSYLAWYQQKPGQAPRLLIYGASSRATGIPDRFSGS GSGTDFTLTISRLEPEDFAVYYCQQYGSSPLTFGGGTKVEIK |

In a further alternative embodiment, the amino acid sequence of VH of the anti-CD25 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 49, and the amino acid sequence of VL of the anti-CD25 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 50. The amino acid sequence of VH of the anti-CD25 antibody may consist of an amino acid sequence having 90% or higher sequence identity to the amino acid sequence set forth in SEQ ID NO: 49, and the amino acid sequence of VL of the anti-CD25 antibody may consist of an amino acid sequence having 90% or higher sequence identity to the amino acid sequence set forth in SEQ ID NO: 50. The amino acid sequence having 90% or higher sequence identity to the amino acid sequence set forth in SEQ ID NO: 49 may be the amino acid sequence set forth in SEQ ID NO: 49, and the amino acid sequence having 90% or higher sequence identity to the amino acid sequence set forth in SEQ ID NO: 50 may be the amino acid sequence set forth in SEQ ID NO: 50.

VH containing the amino acid sequence having the sequence identity to the amino acid sequence set forth in SEQ ID NO: 49 preferably contains CDR1 consisting of the amino acid sequence set forth in SEQ ID NO: 127, CDR2 consisting of the amino acid sequence set forth in SEQ ID NO: 128, and CDR3 consisting of the amino acid sequence set forth in SEQ ID NO: 129.

VL containing the amino acid sequence having the sequence identity to the amino acid sequence set forth in SEQ ID NO: 50 preferably contains CDR1 consisting of the amino acid sequence set forth in SEQ ID NO: 130, CDR2 consisting of the amino acid sequence set forth in SEQ ID NO: 131, and CDR3 consisting of the amino acid sequence set forth in SEQ ID NO: 132.

VH containing the amino acid sequence having the sequence identity to the amino acid sequence set forth in SEQ ID NO: 49 has the activity of VH of the anti-CD25 antibody, i.e., binding activity against CD25. Examples of the amino acid sequence having the sequence identity to the amino acid sequence set forth in SEQ ID NO: 49 include amino acid sequences derived from the amino acid sequence set forth in SEQ ID NO: 49 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 50 has the activity of VL of the anti-CD25 antibody, i.e., binding activity against CD25. Examples of the amino acid sequence having the sequence identity to the amino acid sequence set forth in SEQ ID NO: 50 include amino acid sequences derived from the amino acid sequence set forth in SEQ ID NO: 50 by the deletion, substitution, insertion, or addition of, for example, 1 to 20, preferably 1 to 10, more preferably 1 to 5 amino acids.

Hereinafter, an embodiment in which the antibody is an anti-CADM1 antibody will be described.

The anti-CADM1 antibody is an antibody that binds to CADM1. The anti-CADM1 antibody is preferably an antibody that specifically binds to CADM1. The anti-CADM1 antibody may be a polyclonal antibody or a monoclonal antibody and is preferably a monoclonal antibody. The anti-CADM1 antibody may be a human-type chimeric antibody, a humanized antibody, or a human antibody and is preferably a humanized antibody.

In one embodiment, the anti-CADM1 antibody is an antibody having a heavy chain variable region (VH) and a light chain variable region (VL) of PTA021_A3 (U.S. Patent No. 8420084).

In one embodiment, the anti-CADM1 antibody contains a heavy chain variable region (VH) containing CDR1 consisting of the amino acid sequence set forth in SEQ ID NO: 133, CDR2 consisting of the amino acid sequence set forth in SEQ ID NO: 134, and CDR3 consisting of the amino acid sequence set forth in SEQ ID NO: 135, and a light chain variable region (VL) containing CDR1 consisting of the amino acid sequence set forth in SEQ ID NO: 136, CDR2 consisting of the amino acid sequence set forth in SEQ ID NO: 137, and CDR3 consisting of the amino acid sequence set forth in SEQ ID NO: 138. CDR1, CDR2, and CDR3 are CDR1, CDR2 and, CDR3, respectively, based on the Kabat numbering.

In an alternative embodiment, the amino acid sequence of VH of the anti-CADM1 antibody contains the amino acid sequence set forth in SEQ ID NO: 51, and the amino acid sequence of VL of the anti-CADM1 antibody contains the amino acid sequence set forth in SEQ ID NO: 52. The amino acid sequence of VH of the anti-CADM1 antibody may consist of the amino acid sequence set forth in SEQ ID NO: 51, and the amino acid sequence of VL of the anti-CADM1 antibody may consist of the amino acid sequence set forth in SEQ ID NO: 52.

As shown in the table below, VH containing the amino acid sequence set forth in SEQ ID NO: 51 contains CDR1 consisting of the amino acid sequence set forth in SEQ ID NO: 133 (underlined in the table below), CDR2 consisting of the amino acid sequence set forth in SEQ ID NO: 134 (underlined in the table below), and CDR3 consisting of the amino acid sequence set forth in SEQ ID NO: 135 (underlined in the table below), and VL containing the amino acid sequence set forth in SEQ ID NO: 52 contains CDR1 consisting of the amino acid sequence set forth in SEQ ID NO: 136 (underlined in the table below), CDR2 consisting of the amino acid sequence set forth in SEQ ID NO: 137 (underlined in the table below), and CDR3 consisting of the amino acid sequence set forth in SEQ ID NO: 138 (underlined in the table below).

**[Table 15]**

| |
|---|
| SEQ ID NO: 51 |
| QITLKESGPTLVKPTQTLTLTCTFSGFSLSTSGVGVGWIRQPPGKALEWLALIYWDDDKRYSPSL KSRLTITKDTSKNQVVLTMTNMDPVDTATYYCAHRRVEWVTLAGNWFDPWGQGTLVTVSS |
| SEQ ID NO: 52 |
| DIQMTQSPSSLSASVGDRVTITCRASQGISSWLAWYQQKPEKAPKSLIYAASSLQSGVPSRFSGS GSGTDFTLTISNLQPEDFATYYCQQYNSYPLTFGGGTKVEIK |

In a further alternative embodiment, the amino acid sequence of VH of the anti-CADM1 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 51, and the amino acid sequence of VL of the anti-CADM1 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 52. The amino acid sequence of VH of the anti-CADM1 antibody may consist of an amino acid sequence having 90% or higher sequence identity to the amino acid sequence set forth in SEQ ID NO: 51, and the amino acid sequence of VL of the anti-CADM1 antibody may consist of an amino acid sequence having 90% or higher sequence identity to the amino acid sequence set forth in SEQ ID NO: 52. The amino acid sequence having 90% or higher sequence identity to the amino acid sequence set forth in SEQ ID NO: 51 may be the amino acid sequence set forth in SEQ ID NO: 51, and the amino acid sequence having 90% or higher sequence identity to the amino acid sequence set forth in SEQ ID NO: 52 may be the amino acid sequence set forth in SEQ ID NO: 52.

VH containing the amino acid sequence having the sequence identity to the amino acid sequence set forth in SEQ ID NO: 51 preferably contains CDR1 consisting of the amino acid sequence set forth in SEQ ID NO: 133, CDR2 consisting of the amino acid sequence set forth in SEQ ID NO: 134, and CDR3 consisting of the amino acid sequence set forth in SEQ ID NO: 135.

VL containing the amino acid sequence having the sequence identity to the amino acid sequence set forth in SEQ ID NO: 52 preferably contains CDR1 consisting of the amino acid sequence set forth in SEQ ID NO: 136, CDR2 consisting of the amino acid sequence set forth in SEQ ID NO: 137, and CDR3 consisting of the amino acid sequence set forth in SEQ ID NO: 138.

VH containing the amino acid sequence having the sequence identity to the amino acid sequence set forth in SEQ ID NO: 51 has the activity of VH of the anti-CADM1 antibody, i.e., binding activity against CADM1. Examples of the amino acid sequence having the sequence identity to the amino acid sequence set forth in SEQ ID NO: 51 include amino acid sequences derived from the amino acid sequence set forth in SEQ ID NO: 51 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 52 has the activity of VL of the anti-CADM1 antibody, i.e., binding activity against CADM1. Examples of the amino acid sequence having the sequence identity to the amino acid sequence set forth in SEQ ID NO: 52 include amino acid sequences derived from the amino acid sequence set forth in SEQ ID NO: 52 by the deletion, substitution, insertion, or addition of, for example, 1 to 20, preferably 1 to 10, more preferably 1 to 5 amino acids.

Hereinafter, an embodiment in which the antibody is an anti-CLL1 antibody will be described.

The anti-CLL1 antibody is an antibody that binds to CLL1. The anti-CLL1 antibody is preferably an antibody that specifically binds to CLL1. The anti-CLL1 antibody may be a polyclonal antibody or a monoclonal antibody and is preferably a monoclonal antibody. The anti-CLL1 antibody may be a human-type chimeric antibody, a humanized antibody, or a human antibody and is preferably a humanized antibody.

In one embodiment, the anti-CLL1 antibody is an antibody having a heavy chain variable region (VH) and a light chain variable region (VL) of 6E7LAHle (WO 2016/205200).

In one embodiment, the anti-CLL1 antibody contains a heavy chain variable region (VH) containing CDR1 consisting of the amino acid sequence set forth in SEQ ID NO: 139, CDR2 consisting of the amino acid sequence set forth in SEQ ID NO: 140, and CDR3 consisting of the amino acid sequence set forth in SEQ ID NO: 141, and a light chain variable region (VL) containing CDR1 consisting of the amino acid sequence set forth in SEQ ID NO: 142, CDR2 consisting of the amino acid sequence set forth in SEQ ID NO: 143, and CDR3 consisting of the amino acid sequence set forth in SEQ ID NO: 144. CDR1, CDR2, and CDR3 are CDR1, CDR2 and, CDR3, respectively, based on the Kabat numbering.

In an alternative embodiment, the amino acid sequence of VH of the anti-CLL1 antibody contains the amino acid sequence set forth in SEQ ID NO: 53, and the amino acid sequence of VL of the anti-CLL1 antibody contains the amino acid sequence set forth in SEQ ID NO: 54. The amino acid sequence of VH of the anti-CLL1 antibody may consist of the amino acid sequence set forth in SEQ ID NO: 53, and the amino acid sequence of VL of the anti-CLL1 antibody may consist of the amino acid sequence set forth in SEQ ID NO: 54.

As shown in the table below, VH containing the amino acid sequence set forth in SEQ ID NO: 53 contains CDR1 consisting of the amino acid sequence set forth in SEQ ID NO: 139 (underlined in the table below), CDR2 consisting of the amino acid sequence set forth in SEQ ID NO: 140 (underlined in the table below), and CDR3 consisting of the amino acid sequence set forth in SEQ ID NO: 141 (underlined in the table below), and VL containing the amino acid sequence set forth in SEQ ID NO: 54 contains CDR1 consisting of the amino acid sequence set forth in SEQ ID NO: 142 (underlined in the table below), CDR2 consisting of the amino acid sequence set forth in SEQ ID NO: 143 (underlined in the table below), and CDR3 consisting of the amino acid sequence set forth in SEQ ID NO: 144 (underlined in the table below).

**[Table 16]**

| |
|---|
| SEQ ID NO: 53 |
| EVQLVQSGAEVKKPGASVKVSCKASGYSFTDYYMHWVRQAPGQGLEWIGRINPYAGAAFYSQN FKDRVTLTVDTSTSTAYLELSSLRSEDTAVYYCAIERGADLEGYAMDYWGQGTLVTVSS |
| SEQ ID NO: 54 |
| DIQMTQSPSSLSASVGDRVTITCRASQSVSTSSYNYMHWYQQKPGKPPKLLIKYASNLESGVPS RFSGSGSGTDFTLTISSLQPEDFATYYCQHSWEIPLTFGQGTKVEIK |

In a further alternative embodiment, the amino acid sequence of VH of the anti-CLL1 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 53, and the amino acid sequence of VL of the anti-CLL1 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 54. The amino acid sequence of VH of the anti-CLL1 antibody may consist of an amino acid sequence having 90% or higher sequence identity to the amino acid sequence set forth in SEQ ID NO: 53, and the amino acid sequence of VL of the anti-CLL1 antibody may consist of an amino acid sequence having 90% or higher sequence identity to the amino acid sequence set forth in SEQ ID NO: 54. The amino acid sequence having 90% or higher sequence identity to the amino acid sequence set forth in SEQ ID NO: 53 may be the amino acid sequence set forth in SEQ ID NO: 53, and the amino acid sequence having 90% or higher sequence identity to the amino acid sequence set forth in SEQ ID NO: 54 may be the amino acid sequence set forth in SEQ ID NO: 54.

VH containing the amino acid sequence having the sequence identity to the amino acid sequence set forth in SEQ ID NO: 53 preferably contains CDR1 consisting of the amino acid sequence set forth in SEQ ID NO: 139, CDR2 consisting of the amino acid sequence set forth in SEQ ID NO: 140, and CDR3 consisting of the amino acid sequence set forth in SEQ ID NO: 141.

VL containing the amino acid sequence having the sequence identity to the amino acid sequence set forth in SEQ ID NO: 54 preferably contains CDR1 consisting of the amino acid sequence set forth in SEQ ID NO: 142, CDR2 consisting of the amino acid sequence set forth in SEQ ID NO: 143, and CDR3 consisting of the amino acid sequence set forth in SEQ ID NO: 144.

VH containing the amino acid sequence having the sequence identity to the amino acid sequence set forth in SEQ ID NO: 53 has the activity of VH of the anti-CLL1 antibody, i.e., binding activity against CLL1. Examples of the amino acid sequence having the sequence identity to the amino acid sequence set forth in SEQ ID NO: 53 include amino acid sequences derived from the amino acid sequence set forth in SEQ ID NO: 53 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 54 has the activity of VL of the anti-CLL1 antibody, i.e., binding activity against CLL1. Examples of the amino acid sequence having the sequence identity to the amino acid sequence set forth in SEQ ID NO: 54 include amino acid sequences derived from the amino acid sequence set forth in SEQ ID NO: 54 by the deletion, substitution, insertion, or addition of, for example, 1 to 20, preferably 1 to 10, more preferably 1 to 5 amino acids.

Hereinafter, an embodiment in which the antibody is an anti-CD38 antibody will be described.

The anti-CD38 antibody is an antibody that binds to CD38. The anti-CD38 antibody is preferably an antibody that specifically binds to CD38. The anti-CD38 antibody may be a polyclonal antibody or a monoclonal antibody and is preferably a monoclonal antibody. The anti-CD38 antibody may be a human-type chimeric antibody, a humanized antibody, or a human antibody and is preferably a humanized antibody.

In one embodiment, the anti-CD38 antibody is an antibody having a heavy chain variable region (VH) and a light chain variable region (VL) of daratumumab.

In one embodiment, the anti-CD38 antibody contains a heavy chain variable region (VH) containing CDR1 consisting of the amino acid sequence set forth in SEQ ID NO: 145, CDR2 consisting of the amino acid sequence set forth in SEQ ID NO: 146, and CDR3 consisting of the amino acid sequence set forth in SEQ ID NO: 147, and a light chain variable region (VL) containing CDR1 consisting of the amino acid sequence set forth in SEQ ID NO: 148, CDR2 consisting of the amino acid sequence set forth in SEQ ID NO: 149, and CDR3 consisting of the amino acid sequence set forth in SEQ ID NO: 150. CDR1, CDR2, and CDR3 are CDR1, CDR2 and, CDR3, respectively, based on the Kabat numbering.

In an alternative embodiment, the amino acid sequence of VH of the anti-CD38 antibody contains the amino acid sequence set forth in SEQ ID NO: 55, and the amino acid sequence of VL of the anti-CD38 antibody contains the amino acid sequence set forth in SEQ ID NO: 56. The amino acid sequence of VH of the anti-CD38 antibody may consist of the amino acid sequence set forth in SEQ ID NO: 55, and the amino acid sequence of VL of the anti-CD38 antibody may consist of the amino acid sequence set forth in SEQ ID NO: 56.

As shown in the table below, VH containing the amino acid sequence set forth in SEQ ID NO: 55 contains CDR1 consisting of the amino acid sequence set forth in SEQ ID NO: 145 (underlined in the table below), CDR2 consisting of the amino acid sequence set forth in SEQ ID NO: 146 (underlined in the table below), and CDR3 consisting of the amino acid sequence set forth in SEQ ID NO: 147 (underlined in the table below), and VL containing the amino acid sequence set forth in SEQ ID NO: 56 contains CDR1 consisting of the amino acid sequence set forth in SEQ ID NO: 148 (underlined in the table below), CDR2 consisting of the amino acid sequence set forth in SEQ ID NO: 149 (underlined in the table below), and CDR3 consisting of the amino acid sequence set forth in SEQ ID NO: 150 (underlined in the table below).

**[Table 17]**

| |
|---|
| SEQ ID NO: 55 |
| |
| SEQ ID NO: 56 |
| |

In a further alternative embodiment, the amino acid sequence of VH of the anti-CD38 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 55, and the amino acid sequence of VL of the anti-CD38 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 56. The amino acid sequence of VH of the anti-CD38 antibody may consist of an amino acid sequence having 90% or higher sequence identity to the amino acid sequence set forth in SEQ ID NO: 55, and the amino acid sequence of VL of the anti-CD38 antibody may consist of an amino acid sequence having 90% or higher sequence identity to the amino acid sequence set forth in SEQ ID NO: 56. The amino acid sequence having 90% or higher sequence identity to the amino acid sequence set forth in SEQ ID NO: 55 may be the amino acid sequence set forth in SEQ ID NO: 55, and the amino acid sequence having 90% or higher sequence identity to the amino acid sequence set forth in SEQ ID NO: 56 may be the amino acid sequence set forth in SEQ ID NO: 56.

VH containing the amino acid sequence having the sequence identity to the amino acid sequence set forth in SEQ ID NO: 55 preferably contains CDR1 consisting of the amino acid sequence set forth in SEQ ID NO: 145, CDR2 consisting of the amino acid sequence set forth in SEQ ID NO: 146, and CDR3 consisting of the amino acid sequence set forth in SEQ ID NO: 147.

VL containing the amino acid sequence having the sequence identity to the amino acid sequence set forth in SEQ ID NO: 56 preferably contains CDR1 consisting of the amino acid sequence set forth in SEQ ID NO: 148, CDR2 consisting of the amino acid sequence set forth in SEQ ID NO: 149, and CDR3 consisting of the amino acid sequence set forth in SEQ ID NO: 150.

VH containing the amino acid sequence having the sequence identity to the amino acid sequence set forth in SEQ ID NO: 55 has the activity of VH of the anti-CD38 antibody, i.e., binding activity against CD38. Examples of the amino acid sequence having the sequence identity to the amino acid sequence set forth in SEQ ID NO: 55 include amino acid sequences derived from the amino acid sequence set forth in SEQ ID NO: 55 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 56 has the activity of VL of the anti-CD38 antibody, i.e., binding activity against CD38. Examples of the amino acid sequence having the sequence identity to the amino acid sequence set forth in SEQ ID NO: 56 include amino acid sequences derived from the amino acid sequence set forth in SEQ ID NO: 56 by the deletion, substitution, insertion, or addition of, for example, 1 to 20, preferably 1 to 10, more preferably 1 to 5 amino acids.

Hereinafter, an embodiment in which the antibody is an anti-CD74 antibody will be described.

The anti-CD74 antibody is an antibody that binds to CD74. The anti-CD74 antibody is preferably an antibody that specifically binds to CD74. The anti-CD74 antibody may be a polyclonal antibody or a monoclonal antibody and is preferably a monoclonal antibody. The anti-CD74 antibody may be a human-type chimeric antibody, a humanized antibody, or a human antibody and is preferably a humanized antibody.

In one embodiment, the anti-CD74 antibody is an antibody having a heavy chain variable region (VH) and a light chain variable region (VL) of milatuzumab.

In one embodiment, the anti-CD74 antibody contains a heavy chain variable region (VH) containing CDR1 consisting of the amino acid sequence set forth in SEQ ID NO: 151, CDR2 consisting of the amino acid sequence set forth in SEQ ID NO: 152, and CDR3 consisting of the amino acid sequence set forth in SEQ ID NO: 153, and a light chain variable region (VL) containing CDR1 consisting of the amino acid sequence set forth in SEQ ID NO: 154, CDR2 consisting of the amino acid sequence set forth in SEQ ID NO: 155, and CDR3 consisting of the amino acid sequence set forth in SEQ ID NO: 156. CDR1, CDR2, and CDR3 are CDR1, CDR2 and, CDR3, respectively, based on the Kabat numbering.

In an alternative embodiment, the amino acid sequence of VH of the anti-CD74 antibody contains the amino acid sequence set forth in SEQ ID NO: 57, and the amino acid sequence of VL of the anti-CD74 antibody contains the amino acid sequence set forth in SEQ ID NO: 58. The amino acid sequence of VH of the anti-CD74 antibody may consist of the amino acid sequence set forth in SEQ ID NO: 57, and the amino acid sequence of VL of the anti-CD74 antibody may consist of the amino acid sequence set forth in SEQ ID NO: 58.

As shown in the table below, VH containing the amino acid sequence set forth in SEQ ID NO: 57 contains CDR1 consisting of the amino acid sequence set forth in SEQ ID NO: 151 (underlined in the table below), CDR2 consisting of the amino acid sequence set forth in SEQ ID NO: 152 (underlined in the table below), and CDR3 consisting of the amino acid sequence set forth in SEQ ID NO: 153 (underlined in the table below), and VL containing the amino acid sequence set forth in SEQ ID NO: 58 contains CDR1 consisting of the amino acid sequence set forth in SEQ ID NO: 154 (underlined in the table below), CDR2 consisting of the amino acid sequence set forth in SEQ ID NO: 155 (underlined in the table below), and CDR3 consisting of the amino acid sequence set forth in SEQ ID NO: 156 (underlined in the table below).

**[Table 18]**

| |
|---|
| SEQ ID NO: 57 |
| QVQLQQSGSELKKPGASVKVSCKASGYTFTNYGVNWIKQAPGQGLQWMGWINPNTGEPTFDDD FKGRFAFSLDTSVSTAYLQISSLKADDTAVYFCSRSRGKNEAWFAYWGQGTLVTVSS |
| SEQ ID NO: 58 |
| DIQLTQSPLSLPVTLGQPASISCRSSQSLVHRNGNTYLHWFQQRPGQSPRLLIYTVSNRFSGVPD RFSGSGSGTDFTLKISRVEAEDVGVYFCSQSSHVPPTFGAGTRLEIK |

In a further alternative embodiment, the amino acid sequence of VH of the anti-CD74 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 57, and the amino acid sequence of VL of the anti-CD74 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 58. The amino acid sequence of VH of the anti-CD74 antibody may consist of an amino acid sequence having 90% or higher sequence identity to the amino acid sequence set forth in SEQ ID NO: 57, and the amino acid sequence of VL of the anti-CD74 antibody may consist of an amino acid sequence having 90% or higher sequence identity to the amino acid sequence set forth in SEQ ID NO: 58. The amino acid sequence having 90% or higher sequence identity to the amino acid sequence set forth in SEQ ID NO: 57 may be the amino acid sequence set forth in SEQ ID NO: 57, and the amino acid sequence having 90% or higher sequence identity to the amino acid sequence set forth in SEQ ID NO: 58 may be the amino acid sequence set forth in SEQ ID NO: 58.

VH containing the amino acid sequence having the sequence identity to the amino acid sequence set forth in SEQ ID NO: 57 preferably contains CDR1 consisting of the amino acid sequence set forth in SEQ ID NO: 151, CDR2 consisting of the amino acid sequence set forth in SEQ ID NO: 152, and CDR3 consisting of the amino acid sequence set forth in SEQ ID NO: 153.

VL containing the amino acid sequence having the sequence identity to the amino acid sequence set forth in SEQ ID NO: 58 preferably contains CDR1 consisting of the amino acid sequence set forth in SEQ ID NO: 154, CDR2 consisting of the amino acid sequence set forth in SEQ ID NO: 155, and CDR3 consisting of the amino acid sequence set forth in SEQ ID NO: 156.

VH containing the amino acid sequence having the sequence identity to the amino acid sequence set forth in SEQ ID NO: 57 has the activity of VH of the anti-CD74 antibody, i.e., binding activity against CD74. Examples of the amino acid sequence having the sequence identity to the amino acid sequence set forth in SEQ ID NO: 57 include amino acid sequences derived from the amino acid sequence set forth in SEQ ID NO: 57 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 58 has the activity of VL of the anti-CD74 antibody, i.e., binding activity against CD74. Examples of the amino acid sequence having the sequence identity to the amino acid sequence set forth in SEQ ID NO: 58 include amino acid sequences derived from the amino acid sequence set forth in SEQ ID NO: 58 by the deletion, substitution, insertion, or addition of, for example, 1 to 20, preferably 1 to 10, more preferably 1 to 5 amino acids.

Hereinafter, an embodiment in which the antibody is an anti-NECTIN4 antibody will be described.

The anti-NECTIN4 antibody is an antibody that binds to NECTIN4. The anti-NECTIN4 antibody is preferably an antibody that specifically binds to NECTIN4. The anti-NECTIN4 antibody may be a polyclonal antibody or a monoclonal antibody and is preferably a monoclonal antibody. The anti-NECTIN4 antibody may be a human-type chimeric antibody, a humanized antibody, or a human antibody and is preferably a humanized antibody.

In one embodiment, the anti-NECTIN4 antibody is an antibody having a heavy chain variable region (VH) and a light chain variable region (VL) of enfortumab.

In one embodiment, the anti-NECTIN4 antibody contains a heavy chain variable region (VH) containing CDR1 consisting of the amino acid sequence set forth in SEQ ID NO: 187, CDR2 consisting of the amino acid sequence set forth in SEQ ID NO: 188, and CDR3 consisting of the amino acid sequence set forth in SEQ ID NO: 189, and a light chain variable region (VL) containing CDR1 consisting of the amino acid sequence set forth in SEQ ID NO: 190, CDR2 consisting of the amino acid sequence set forth in SEQ ID NO: 191, and CDR3 consisting of the amino acid sequence set forth in SEQ ID NO: 192. CDR1, CDR2, and CDR3 are CDR1, CDR2 and, CDR3, respectively, based on the Kabat numbering.

In an alternative embodiment, the amino acid sequence of VH of the anti-NECTIN4 antibody contains the amino acid sequence set forth in SEQ ID NO: 183, and the amino acid sequence of VL of the anti-NECTIN4 antibody contains the amino acid sequence set forth in SEQ ID NO: 184. The amino acid sequence of VH of the anti-NECTIN4 antibody may consist of the amino acid sequence set forth in SEQ ID NO: 183, and the amino acid sequence of VL of the anti-NECTIN4 antibody may consist of the amino acid sequence set forth in SEQ ID NO: 184.

As shown in the table below, VH containing the amino acid sequence set forth in SEQ ID NO: 183 contains CDR1 consisting of the amino acid sequence set forth in SEQ ID NO: 187 (underlined in the table below), CDR2 consisting of the amino acid sequence set forth in SEQ ID NO: 188 (underlined in the table below), and CDR3 consisting of the amino acid sequence set forth in SEQ ID NO: 189 (underlined in the table below), and VL containing the amino acid sequence set forth in SEQ ID NO: 184 contains CDR1 consisting of the amino acid sequence set forth in SEQ ID NO: 190 (underlined in the table below), CDR2 consisting of the amino acid sequence set forth in SEQ ID NO: 191 (underlined in the table below), and CDR3 consisting of the amino acid sequence set forth in SEQ ID NO: 192 (underlined in the table below).

**[Table 19]**

| |
|---|
| SEQ ID NO: 183 |
| EVQLVESGGGLVQPGGSLRLSCAASGFTFSSYNMNWVRQAPGKGLEWVSYISSSSSTIYYADSV KGRFTISRDNAKNSLSLQMNSLRDEDTAVYYCARAYYYGMDVWGQGTTVTVSS |
| SEQ ID NO: 184 |
| DIQMTQSPSSVSASVGDRVTITCRASQGISGWLAWYQQKPGKAPKFLIYAASTLQSGVPSRFSG SGSGTDFTLTISSLQPEDFATYYCQQANSFPPTFGGGTKVEIK |

In a further alternative embodiment, the amino acid sequence of VH of the anti-NECTIN4 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 183, and the amino acid sequence of VL of the anti-NECTIN4 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 184. The amino acid sequence of VH of the anti-NECTIN4 antibody may consist of an amino acid sequence having 90% or higher sequence identity to the amino acid sequence set forth in SEQ ID NO: 183, and the amino acid sequence of VL of the anti-NECTIN4 antibody may consist of an amino acid sequence having 90% or higher sequence identity to the amino acid sequence set forth in SEQ ID NO: 184. The amino acid sequence having 90% or higher sequence identity to the amino acid sequence set forth in SEQ ID NO: 183 may be the amino acid sequence set forth in SEQ ID NO: 183, and the amino acid sequence having 90% or higher sequence identity to the amino acid sequence set forth in SEQ ID NO: 184 may be the amino acid sequence set forth in SEQ ID NO: 184.

VH containing the amino acid sequence having the sequence identity to the amino acid sequence set forth in SEQ ID NO: 183 preferably contains CDR1 consisting of the amino acid sequence set forth in SEQ ID NO: 187, CDR2 consisting of the amino acid sequence set forth in SEQ ID NO: 188, and CDR3 consisting of the amino acid sequence set forth in SEQ ID NO: 189.

VL containing the amino acid sequence having the sequence identity to the amino acid sequence set forth in SEQ ID NO: 184 preferably contains CDR1 consisting of the amino acid sequence set forth in SEQ ID NO: 190, CDR2 consisting of the amino acid sequence set forth in SEQ ID NO: 191, and CDR3 consisting of the amino acid sequence set forth in SEQ ID NO: 192.

VH containing the amino acid sequence having the sequence identity to the amino acid sequence set forth in SEQ ID NO: 183 has the activity of VH of the anti-NECTIN4 antibody, i.e., binding activity against NECTIN4. Examples of the amino acid sequence having the sequence identity to the amino acid sequence set forth in SEQ ID NO: 183 include amino acid sequences derived from the amino acid sequence set forth in SEQ ID NO: 183 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 184 has the activity of VL of the anti-NECTIN4 antibody, i.e., binding activity against NECTIN4. Examples of the amino acid sequence having the sequence identity to the amino acid sequence set forth in SEQ ID NO: 184 include amino acid sequences derived from the amino acid sequence set forth in SEQ ID NO: 184 by the deletion, substitution, insertion, or addition of, for example, 1 to 20, preferably 1 to 10, more preferably 1 to 5 amino acids.

Hereinafter, an embodiment in which the antibody is an anti-TROP2 antibody will be described.

The anti-TROP2 antibody is an antibody that binds to TROP2. The anti-TROP2 antibody is preferably an antibody that specifically binds to TROP2. The anti-TROP2 antibody may be a polyclonal antibody or a monoclonal antibody and is preferably a monoclonal antibody. The anti-TROP2 antibody may be a human-type chimeric antibody, a humanized antibody, or a human antibody and is preferably a humanized antibody.

In one embodiment, the anti-TROP2 antibody is an antibody having a heavy chain variable region (VH) and a light chain variable region (VL) of sacituzumab.

In one embodiment, the anti-TROP2 antibody contains a heavy chain variable region (VH) containing CDR1 consisting of the amino acid sequence set forth in SEQ ID NO: 193, CDR2 consisting of the amino acid sequence set forth in SEQ ID NO: 194, and CDR3 consisting of the amino acid sequence set forth in SEQ ID NO: 195, and a light chain variable region (VL) containing CDR1 consisting of the amino acid sequence set forth in SEQ ID NO: 196, CDR2 consisting of the amino acid sequence set forth in SEQ ID NO: 197, and CDR3 consisting of the amino acid sequence set forth in SEQ ID NO: 198. CDR1, CDR2, and CDR3 are CDR1, CDR2 and, CDR3, respectively, based on the Kabat numbering.

In an alternative embodiment, the amino acid sequence of VH of the anti-TROP2 antibody contains the amino acid sequence set forth in SEQ ID NO: 185, and the amino acid sequence of VL of the anti-TROP2 antibody contains the amino acid sequence set forth in SEQ ID NO: 186. The amino acid sequence of VH of the anti-TROP2 antibody may consist of the amino acid sequence set forth in SEQ ID NO: 185, and the amino acid sequence of VL of the anti-TROP2 antibody may consist of the amino acid sequence set forth in SEQ ID NO: 186.

As shown in the table below, VH containing the amino acid sequence set forth in SEQ ID NO: 185 contains CDR1 consisting of the amino acid sequence set forth in SEQ ID NO: 193 (underlined in the table below), CDR2 consisting of the amino acid sequence set forth in SEQ ID NO: 194 (underlined in the table below), and CDR3 consisting of the amino acid sequence set forth in SEQ ID NO: 195 (underlined in the table below), and VL containing the amino acid sequence set forth in SEQ ID NO: 186 contains CDR1 consisting of the amino acid sequence set forth in SEQ ID NO: 196 (underlined in the table below), CDR2 consisting of the amino acid sequence set forth in SEQ ID NO: 197 (underlined in the table below), and CDR3 consisting of the amino acid sequence set forth in SEQ ID NO: 198 (underlined in the table below).

**[Table 20]**

| |
|---|
| SEQ ID NO: 185 |
| QVQLQQSGSELKKPGASVKVSCKASGYTFTNYGMNWVKQAPGQGLKWMGWINTYTGEPTYTD DFKGRFAFSLDTSVSTAYLQISSLKADDTAVYFCARGGFGSSYWYFDVWGQGSLVTVSS |
| SEQ ID NO: 186 |
| DIQLTQSPSSLSASVGDRVSITCKASQDVSIAVAWYQQKPGKAPKLLIYSASYRYTGVPDRFSGS GSGTDFTLTISSLQPEDFAVYYCQQHYITPLTFGAGTKVEIK |

In a further alternative embodiment, the amino acid sequence of VH of the anti-TROP2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 185, and the amino acid sequence of VL of the anti-TROP2 antibody contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 186. The amino acid sequence of VH of the anti-TROP2 antibody may consist of an amino acid sequence having 90% or higher sequence identity to the amino acid sequence set forth in SEQ ID NO: 185, and the amino acid sequence of VL of the anti-TROP2 antibody may consist of an amino acid sequence having 90% or higher sequence identity to the amino acid sequence set forth in SEQ ID NO: 186. The amino acid sequence having 90% or higher sequence identity to the amino acid sequence set forth in SEQ ID NO: 185 may be the amino acid sequence set forth in SEQ ID NO: 185, and the amino acid sequence having 90% or higher sequence identity to the amino acid sequence set forth in SEQ ID NO: 186 may be the amino acid sequence set forth in SEQ ID NO: 186.

VH containing the amino acid sequence having the sequence identity to the amino acid sequence set forth in SEQ ID NO: 185 preferably contains CDR1 consisting of the amino acid sequence set forth in SEQ ID NO: 193, CDR2 consisting of the amino acid sequence set forth in SEQ ID NO: 194, and CDR3 consisting of the amino acid sequence set forth in SEQ ID NO: 195.

VL containing the amino acid sequence having the sequence identity to the amino acid sequence set forth in SEQ ID NO: 186 preferably contains CDR1 consisting of the amino acid sequence set forth in SEQ ID NO: 196, CDR2 consisting of the amino acid sequence set forth in SEQ ID NO: 197, and CDR3 consisting of the amino acid sequence set forth in SEQ ID NO: 198.

VH containing the amino acid sequence having the sequence identity to the amino acid sequence set forth in SEQ ID NO: 185 has the activity of VH of the anti-TROP2 antibody, i.e., binding activity against TROP2. Examples of the amino acid sequence having the sequence identity to the amino acid sequence set forth in SEQ ID NO: 185 include amino acid sequences derived from the amino acid sequence set forth in SEQ ID NO: 185 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 186 has the activity of VL of the anti-TROP2 antibody, i.e., binding activity against TROP2. Examples of the amino acid sequence having the sequence identity to the amino acid sequence set forth in SEQ ID NO: 186 include amino acid sequences derived from the amino acid sequence set forth in SEQ ID NO: 186 by the deletion, substitution, insertion, or addition of, for example, 1 to 20, preferably 1 to 10, more preferably 1 to 5 amino acids.

Hereinafer, an embodiment in which the antibody is an anti-CD79b antibody and is a human antibody G4055 (human antibody G4055) will be described (hereinafter, also referred to as the anti-CD79b antibody (human antibody G4055) or the anti-CD79b_G4055 antibody).

The anti-CD79b antibody (human antibody G4055) is an antibody that binds to CD79b. The anti-CD79b antibody (human antibody G4055) is preferably an antibody that specifically binds to CD79b. The anti-CD79b antibody (human antibody G4055) may be a polyclonal antibody or a monoclonal antibody and is preferably a monoclonal antibody. The anti-CD79b antibody (human antibody G4055) is a human antibody.

In one embodiment, the anti-CD79b antibody (human antibody G4055) contains a heavy chain variable region (VH) containing CDR1 consisting of the amino acid sequence set forth in SEQ ID NO: 157, CDR2 consisting of the amino acid sequence set forth in SEQ ID NO: 158, and CDR3 consisting of the amino acid sequence set forth in SEQ ID NO: 159, and a light chain variable region (VL) containing CDR1 consisting of the amino acid sequence set forth in SEQ ID NO: 160, CDR2 consisting of the amino acid sequence set forth in SEQ ID NO: 161, and CDR3 consisting of the amino acid sequence set forth in SEQ ID NO: 162. CDR1, CDR2, and CDR3 are CDR1, CDR2 and, CDR3, respectively, based on the Kabat numbering.

In an alternative embodiment, the amino acid sequence of VH of the anti-CD79b antibody (human antibody G4055) contains the amino acid sequence set forth in SEQ ID NO: 59, and the amino acid sequence of VL of the anti-CD79b antibody (human antibody G4055) contains the amino acid sequence set forth in SEQ ID NO: 60. The amino acid sequence of VH of the anti-CD79b antibody (human antibody G4055) may consist of the amino acid sequence set forth in SEQ ID NO: 59, and the amino acid sequence of VL of the anti-CD79b antibody (human antibody G4055) may consist of the amino acid sequence set forth in SEQ ID NO: 60.

As shown in the table below, VH containing the amino acid sequence set forth in SEQ ID NO: 59 contains CDR1 consisting of the amino acid sequence set forth in SEQ ID NO: 157 (underlined in the table below), CDR2 consisting of the amino acid sequence set forth in SEQ ID NO: 158 (underlined in the table below), and CDR3 consisting of the amino acid sequence set forth in SEQ ID NO: 159 (underlined in the table below), and VL containing the amino acid sequence set forth in SEQ ID NO: 60 contains CDR1 consisting of the amino acid sequence set forth in SEQ ID NO: 160 (underlined in the table below), CDR2 consisting of the amino acid sequence set forth in SEQ ID NO: 161 (underlined in the table below), and CDR3 consisting of the amino acid sequence set forth in SEQ ID NO: 162 (underlined in the table below).

**[Table 21]**

| |
|---|
| SEQ ID NO: 59 |
| QVQLVESGGGVVQSGRSLRLSCATSGFTFSIYGMHWVRQAPGKGLEWVALIWYDGSNKYYADS VKGRFTISRDNSKNTLYLQMNSLRAEDTAVYYCAGGDYDDHYGMDVWGQGTTVTVSS |
| SEQ ID NO: 60 |
| AIQLTQSPSSLSASVGDRVTITCRASQGISSALAWYQQKPGKAPKLLIYDASSLESGVPSRFSGS GSGTDFTLTISSLQPEDFATYYCQQFNSFPLTFGGGTKVEIK |

In a further alternative embodiment, the amino acid sequence of VH of the anti-CD79b antibody (human antibody G4055) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 59, and the amino acid sequence of VL of the anti-CD79b antibody (human antibody G4055) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 60. The amino acid sequence of VH of the anti-CD79b antibody (human antibody G4055) may consist of an amino acid sequence having 90% or higher sequence identity to the amino acid sequence set forth in SEQ ID NO: 59, and the amino acid sequence of VL of the anti-CD79b antibody (human antibody G4055) may consist of an amino acid sequence having 90% or higher sequence identity to the amino acid sequence set forth in SEQ ID NO: 60. The amino acid sequence having 90% or higher sequence identity to the amino acid sequence set forth in SEQ ID NO: 59 may be the amino acid sequence set forth in SEQ ID NO: 59, and the amino acid sequence having 90% or higher sequence identity to the amino acid sequence set forth in SEQ ID NO: 60 may be the amino acid sequence set forth in SEQ ID NO: 60.

VH containing the amino acid sequence having the sequence identity to the amino acid sequence set forth in SEQ ID NO: 59 preferably contains CDR1 consisting of the amino acid sequence set forth in SEQ ID NO: 157, CDR2 consisting of the amino acid sequence set forth in SEQ ID NO: 158, and CDR3 consisting of the amino acid sequence set forth in SEQ ID NO: 159.

VL containing the amino acid sequence having the sequence identity to the amino acid sequence set forth in SEQ ID NO: 60 preferably contains CDR1 consisting of the amino acid sequence set forth in SEQ ID NO: 160, CDR2 consisting of the amino acid sequence set forth in SEQ ID NO: 161, and CDR3 consisting of the amino acid sequence set forth in SEQ ID NO: 162.

VH containing the amino acid sequence having the sequence identity to the amino acid sequence set forth in SEQ ID NO: 59 has the activity of VH of the anti-CD79b antibody (human antibody G4055), i.e., binding activity against CD79b. Examples of the amino acid sequence having the sequence identity to the amino acid sequence set forth in SEQ ID NO: 59 include amino acid sequences derived from the amino acid sequence set forth in SEQ ID NO: 59 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 60 has the activity of VL of the anti-CD79b antibody (human antibody G4055), i.e., binding activity against CD79b. Examples of the amino acid sequence having the sequence identity to the amino acid sequence set forth in SEQ ID NO: 60 include amino acid sequences derived from the amino acid sequence set forth in SEQ ID NO: 60 by the deletion, substitution, insertion, or addition of, for example, 1 to 20, preferably 1 to 10, more preferably 1 to 5 amino acids.

Hereinafter, an embodiment in which the antibody is an anti-CD79b antibody and is a human antibody G4022 (human antibody G4022) will be described (hereinafter, also referred to as the anti-CD79b antibody (human antibody G4022) or the anti-CD79b_G4022 antibody).

The anti-CD79b antibody (human antibody G4022) is an antibody that binds to CD79b. The anti-CD79b antibody (human antibody G4022) is preferably an antibody that specifically binds to CD79b. The anti-CD79b antibody (human antibody G4022) may be a polyclonal antibody or a monoclonal antibody and is preferably a monoclonal antibody. The anti-CD79b antibody (human antibody G4022) is a human antibody.

In one embodiment, the anti-CD79b antibody (human antibody G4022) contains a heavy chain variable region (VH) containing CDR1 consisting of the amino acid sequence set forth in SEQ ID NO: 163, CDR2 consisting of the amino acid sequence set forth in SEQ ID NO: 164, and CDR3 consisting of the amino acid sequence set forth in SEQ ID NO: 165, and a light chain variable region (VL) containing CDR1 consisting of the amino acid sequence set forth in SEQ ID NO: 166, CDR2 consisting of the amino acid sequence set forth in SEQ ID NO: 167, and CDR3 consisting of the amino acid sequence set forth in SEQ ID NO: 168. CDR1, CDR2, and CDR3 are CDR1, CDR2 and, CDR3, respectively, based on the Kabat numbering.

In an alternative embodiment, the amino acid sequence of VH of the anti-CD79b antibody (human antibody G4022) contains the amino acid sequence set forth in SEQ ID NO: 61, and the amino acid sequence of VL of the anti-CD79b antibody (human antibody G4022) contains the amino acid sequence set forth in SEQ ID NO: 62. The amino acid sequence of VH of the anti-CD79b antibody (human antibody G4022) may consist of the amino acid sequence set forth in SEQ ID NO: 61, and the amino acid sequence of VL of the anti-CD79b antibody (human antibody G4022) may consist of the amino acid sequence set forth in SEQ ID NO: 62.

As shown in the table below, VH containing the amino acid sequence set forth in SEQ ID NO: 61 contains CDR1 consisting of the amino acid sequence set forth in SEQ ID NO: 163 (underlined in the table below), CDR2 consisting of the amino acid sequence set forth in SEQ ID NO: 164 (underlined in the table below), and CDR3 consisting of the amino acid sequence set forth in SEQ ID NO: 165 (underlined in the table below), and VL containing the amino acid sequence set forth in SEQ ID NO: 62 contains CDR1 consisting of the amino acid sequence set forth in SEQ ID NO: 166 (underlined in the table below), CDR2 consisting of the amino acid sequence set forth in SEQ ID NO: 167 (underlined in the table below), and CDR3 consisting of the amino acid sequence set forth in SEQ ID NO: 168 (underlined in the table below).

**[Table 22]**

| |
|---|
| SEQ ID NO: 61 |
| EVQLVESGGGLVQPGRSLRLSCAASGFTFDGYAMHWVRQAPGKGLEWVSGISWDGRHIGYADS VKGRFTISRDNAKNSLYLQMNSLRAEDTALYYCSKDKGTVTSYYYYYGTDVWGQGTTVTVSS |
| SEQ ID NO: 62 |
| AIQLTQSPSSLSASVGDRVTITCRASQGISSALAWYQQKPGKAPKLLIYDASSLESGVPSRFSGS GSGTDFTLTISSLQPEDFATYYCQQFNSYPFTFGPGTKVDIK |

In a further alternative embodiment, the amino acid sequence of VH of the anti-CD79b antibody (human antibody G4022) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 61, and the amino acid sequence of VL of the anti-CD79b antibody (human antibody G4022) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 62. The amino acid sequence of VH of the anti-CD79b antibody (human antibody G4022) may consist of an amino acid sequence having 90% or higher sequence identity to the amino acid sequence set forth in SEQ ID NO: 61, and the amino acid sequence of VL of the anti-CD79b antibody (human antibody G4022) may consist of an amino acid sequence having 90% or higher sequence identity to the amino acid sequence set forth in SEQ ID NO: 62. The amino acid sequence having 90% or higher sequence identity to the amino acid sequence set forth in SEQ ID NO: 61 may be the amino acid sequence set forth in SEQ ID NO: 61, and the amino acid sequence having 90% or higher sequence identity to the amino acid sequence set forth in SEQ ID NO: 62 may be the amino acid sequence set forth in SEQ ID NO: 62.

VH containing the amino acid sequence having the sequence identity to the amino acid sequence set forth in SEQ ID NO: 61 preferably contains CDR1 consisting of the amino acid sequence set forth in SEQ ID NO: 163, CDR2 consisting of the amino acid sequence set forth in SEQ ID NO: 164, and CDR3 consisting of the amino acid sequence set forth in SEQ ID NO: 165.

VL containing the amino acid sequence having the sequence identity to the amino acid sequence set forth in SEQ ID NO: 62 preferably contains CDR1 consisting of the amino acid sequence set forth in SEQ ID NO: 166, CDR2 consisting of the amino acid sequence set forth in SEQ ID NO: 167, and CDR3 consisting of the amino acid sequence set forth in SEQ ID NO: 168.

VH containing the amino acid sequence having the sequence identity to the amino acid sequence set forth in SEQ ID NO: 61 has the activity of VH of the anti-CD79b antibody (human antibody G4022), i.e., binding activity against CD79b. Examples of the amino acid sequence having the sequence identity to the amino acid sequence set forth in SEQ ID NO: 61 include amino acid sequences derived from the amino acid sequence set forth in SEQ ID NO: 61 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 62 has the activity of VL of the anti-CD79b antibody (human antibody G4022), i.e., binding activity against CD79b. Examples of the amino acid sequence having the sequence identity to the amino acid sequence set forth in SEQ ID NO: 62 include amino acid sequences derived from the amino acid sequence set forth in SEQ ID NO: 62 by the deletion, substitution, insertion, or addition of, for example, 1 to 20, preferably 1 to 10, more preferably 1 to 5 amino acids.

Hereinafter, an embodiment in which the antibody is an anti-CD79b antibody and is a human antibody G4046 (human antibody G4046) will be described (hereinafter, also referred to as the anti-CD79b antibody (human antibody G4046) or the anti-CD79b_G4046 antibody).

The anti-CD79b antibody (human antibody G4046) is an antibody that binds to CD79b. The anti-CD79b antibody (human antibody G4046) is preferably an antibody that specifically binds to CD79b. The anti-CD79b antibody (human antibody G4046) may be a polyclonal antibody or a monoclonal antibody and is preferably a monoclonal antibody. The anti-CD79b antibody (human antibody G4046) is a human antibody.

In one embodiment, the anti-CD79b antibody (human antibody G4046) contains a heavy chain variable region (VH) containing CDR1 consisting of the amino acid sequence set forth in SEQ ID NO: 169, CDR2 consisting of the amino acid sequence set forth in SEQ ID NO: 170, and CDR3 consisting of the amino acid sequence set forth in SEQ ID NO: 171, and a light chain variable region (VL) containing CDR1 consisting of the amino acid sequence set forth in SEQ ID NO: 172, CDR2 consisting of the amino acid sequence set forth in SEQ ID NO: 173, and CDR3 consisting of the amino acid sequence set forth in SEQ ID NO: 174. CDR1, CDR2, and CDR3 are CDR1, CDR2 and, CDR3, respectively, based on the Kabat numbering.

In an alternative embodiment, the amino acid sequence of VH of the anti-CD79b antibody (human antibody G4046) contains the amino acid sequence set forth in SEQ ID NO: 63, and the amino acid sequence of VL of the anti-CD79b antibody (human antibody G4066) contains the amino acid sequence set forth in SEQ ID NO: 64. The amino acid sequence of VH of the anti-CD79b antibody (human antibody G4066) may consist of the amino acid sequence set forth in SEQ ID NO: 63, and the amino acid sequence of VL of the anti-CD79b antibody (human antibody G4066) may consist of the amino acid sequence set forth in SEQ ID NO: 64.

As shown in the table below, VH containing the amino acid sequence set forth in SEQ ID NO: 63 contains CDR1 consisting of the amino acid sequence set forth in SEQ ID NO: 169 (underlined in the table below), CDR2 consisting of the amino acid sequence set forth in SEQ ID NO: 170 (underlined in the table below), and CDR3 consisting of the amino acid sequence set forth in SEQ ID NO: 171 (underlined in the table below), and VL containing the amino acid sequence set forth in SEQ ID NO: 64 contains CDR1 consisting of the amino acid sequence set forth in SEQ ID NO: 172 (underlined in the table below), CDR2 consisting of the amino acid sequence set forth in SEQ ID NO: 173 (underlined in the table below), and CDR3 consisting of the amino acid sequence set forth in SEQ ID NO: 174 (underlined in the table below).

**[Table 23]**

| |
|---|
| SEQ ID NO: 63 |
| QVQLVESGGGVVQPGRSLRLSCAASGFTFSIYGMHWVRQAPGKGLEWVALIWYDGSNKYYADS VKGRFTISRDNSKNTLYLQMNSLRAEDTAVYYCAGGDYDYHYGMDVWGQGTTVTVSS |
| SEQ ID NO: 64 |
| AIQLTQSPSSLSASVGDRVTITCRASQGISSALAWYQQKPGKPPKLLIYDASSLESGVPSRFSGS GSGTDFTLTISSLQPEDFATYYCQQFNSFPLTFGGGTKVEIK |

In a further alternative embodiment, the amino acid sequence of VH of the anti-CD79b antibody (human antibody G4046) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 63, and the amino acid sequence of VL of the anti-CD79b antibody (human antibody G4046) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 64. The amino acid sequence of VH of the anti-CD79b antibody (human antibody G4046) may consist of an amino acid sequence having 90% or higher sequence identity to the amino acid sequence set forth in SEQ ID NO: 63, and the amino acid sequence of VL of the anti-CD79b antibody (human antibody G4046) may consist of an amino acid sequence having 90% or higher sequence identity to the amino acid sequence set forth in SEQ ID NO: 64. The amino acid sequence having 90% or higher sequence identity to the amino acid sequence set forth in SEQ ID NO: 63 may be the amino acid sequence set forth in SEQ ID NO: 63, and the amino acid sequence having 90% or higher sequence identity to the amino acid sequence set forth in SEQ ID NO: 64 may be the amino acid sequence set forth in SEQ ID NO: 64.

VH containing the amino acid sequence having the sequence identity to the amino acid sequence set forth in SEQ ID NO: 63 preferably contains CDR1 consisting of the amino acid sequence set forth in SEQ ID NO: 169, CDR2 consisting of the amino acid sequence set forth in SEQ ID NO: 170, and CDR3 consisting of the amino acid sequence set forth in SEQ ID NO: 171.

VL containing the amino acid sequence having the sequence identity to the amino acid sequence set forth in SEQ ID NO: 64 preferably contains CDR1 consisting of the amino acid sequence set forth in SEQ ID NO: 172, CDR2 consisting of the amino acid sequence set forth in SEQ ID NO: 173, and CDR3 consisting of the amino acid sequence set forth in SEQ ID NO: 174.

VH containing the amino acid sequence having the sequence identity to the amino acid sequence set forth in SEQ ID NO: 63 has the activity of VH of the anti-CD79b antibody (human antibody G4046), i.e., binding activity against CD79b. Examples of the amino acid sequence having the sequence identity to the amino acid sequence set forth in SEQ ID NO: 63 include amino acid sequences derived from the amino acid sequence set forth in SEQ ID NO: 63 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 64 has the activity of VL of the anti-CD79b antibody (human antibody G4046), i.e., binding activity against CD79b. Examples of the amino acid sequence having the sequence identity to the amino acid sequence set forth in SEQ ID NO: 64 include amino acid sequences derived from the amino acid sequence set forth in SEQ ID NO: 64 by the deletion, substitution, insertion, or addition of, for example, 1 to 20, preferably 1 to 10, more preferably 1 to 5 amino acids.

Hereinafter, an embodiment in which the antibody is an anti-CD79b antibody and is a human antibody G4047 (human antibody G4047) will be described (hereinafter, also referred to as the anti-CD79b antibody (human antibody G4047) or the anti-CD79b_G4047 antibody).

The anti-CD79b antibody (human antibody G4047) is an antibody that binds to CD79b. The anti-CD79b antibody (human antibody G4047) is preferably an antibody that specifically binds to CD79b. The anti-CD79b antibody (human antibody G4047) may be a polyclonal antibody or a monoclonal antibody and is preferably a monoclonal antibody. The anti-CD79b antibody (human antibody G4047) is a human antibody.

In one embodiment, the anti-CD79b antibody (human antibody G4047) contains a heavy chain variable region (VH) containing CDR1 consisting of the amino acid sequence set forth in SEQ ID NO: 175, CDR2 consisting of the amino acid sequence set forth in SEQ ID NO: 176, and CDR3 consisting of the amino acid sequence set forth in SEQ ID NO: 177, and a light chain variable region (VL) containing CDR1 consisting of the amino acid sequence set forth in SEQ ID NO: 178, CDR2 consisting of the amino acid sequence set forth in SEQ ID NO: 179, and CDR3 consisting of the amino acid sequence set forth in SEQ ID NO: 180. CDR1, CDR2, and CDR3 are CDR1, CDR2 and, CDR3, respectively, based on the Kabat numbering.

In an alternative embodiment, the amino acid sequence of VH of the anti-CD79b antibody (human antibody G4047) contains the amino acid sequence set forth in SEQ ID NO: 65, and the amino acid sequence of VL of the anti-CD79b antibody (human antibody G4047) contains the amino acid sequence set forth in SEQ ID NO: 66. The amino acid sequence of VH of the anti-CD79b antibody (human antibody G4047) may consist of the amino acid sequence set forth in SEQ ID NO: 65, and the amino acid sequence of VL of the anti-CD79b antibody (human antibody G4047) may consist of the amino acid sequence set forth in SEQ ID NO: 66.

As shown in the table below, VH containing the amino acid sequence set forth in SEQ ID NO: 65 contains CDR1 consisting of the amino acid sequence set forth in SEQ ID NO: 175 (underlined in the table below), CDR2 consisting of the amino acid sequence set forth in SEQ ID NO: 176 (underlined in the table below), and CDR3 consisting of the amino acid sequence set forth in SEQ ID NO: 177 (underlined in the table below), and VL containing the amino acid sequence set forth in SEQ ID NO: 66 contains CDR1 consisting of the amino acid sequence set forth in SEQ ID NO: 178 (underlined in the table below), CDR2 consisting of the amino acid sequence set forth in SEQ ID NO: 179 (underlined in the table below), and CDR3 consisting of the amino acid sequence set forth in SEQ ID NO: 180 (underlined in the table below).

**[Table 24]**

| |
|---|
| SEQ ID NO: 65 |
| QVQLVESGGGVVQPGRSLRLSCAASGFTFSIYGMHWVRQAPGKGLEWVALIWYDGSNKYYADS VKGRFTISRDNSKNTLYLQMNSLRAEDTAVYYCAGGDYYDSGSSYYHYGMDVWGQGTTVTVSS |
| SEQ ID NO: 66 |
| AIQLTQSPSSLSASVGDRVTITCRASQGVSSALAWYQQKPGKPPKLLIYDASSLESGVPSRFSGS GSGTDFTLTISSLQPEDFATYYCQQFNSFPLTFGGGTKVEIK |

In a further alternative embodiment, the amino acid sequence of VH of the anti-CD79b antibody (human antibody G4047) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 65, and the amino acid sequence of VL of the anti-CD79b antibody (human antibody G4047) contains an amino acid sequence having 90% or higher, preferably 95% or higher, more preferably 96% or higher, further preferably 97% or higher, further preferably 98% or higher, further preferably 99% or higher sequence identity to the amino acid sequence set forth in SEQ ID NO: 66. The amino acid sequence of VH of the anti-CD79b antibody (human antibody G4047) may consist of an amino acid sequence having 90% or higher sequence identity to the amino acid sequence set forth in SEQ ID NO: 65, and the amino acid sequence of VL of the anti-CD79b antibody (human antibody G4047) may consist of an amino acid sequence having 90% or higher sequence identity to the amino acid sequence set forth in SEQ ID NO: 66. The amino acid sequence having 90% or higher sequence identity to the amino acid sequence set forth in SEQ ID NO: 65 may be the amino acid sequence set forth in SEQ ID NO: 65, and the amino acid sequence having 90% or higher sequence identity to the amino acid sequence set forth in SEQ ID NO: 66 may be the amino acid sequence set forth in SEQ ID NO: 66.

VH containing the amino acid sequence having the sequence identity to the amino acid sequence set forth in SEQ ID NO: 65 preferably contains CDR1 consisting of the amino acid sequence set forth in SEQ ID NO: 175, CDR2 consisting of the amino acid sequence set forth in SEQ ID NO: 176, and CDR3 consisting of the amino acid sequence set forth in SEQ ID NO: 177.

VL containing the amino acid sequence having the sequence identity to the amino acid sequence set forth in SEQ ID NO: 66 preferably contains CDR1 consisting of the amino acid sequence set forth in SEQ ID NO: 178, CDR2 consisting of the amino acid sequence set forth in SEQ ID NO: 179, and CDR3 consisting of the amino acid sequence set forth in SEQ ID NO: 180.

VH containing the amino acid sequence having the sequence identity to the amino acid sequence set forth in SEQ ID NO: 65 has the activity of VH of the anti-CD79b antibody (human antibody G4047), i.e., binding activity against CD79b. Examples of the amino acid sequence having the sequence identity to the amino acid sequence set forth in SEQ ID NO: 65 include amino acid sequences derived from the amino acid sequence set forth in SEQ ID NO: 65 by the deletion, substitution, insertion, or addition of, for example, 1 to 20, preferably 1 to 10, more preferably 1 to 5 amino acids.

VL containing the amino acid sequence having the sequence identity to the amino acid sequence set forth in SEQ ID NO: 66 has the activity of VL of the anti-CD79b antibody (human antibody G4047), i.e., binding activity against CD79b. Examples of the amino acid sequence having the sequence identity to the amino acid sequence set forth in SEQ ID NO: 66 include amino acid sequences derived from the amino acid sequence set forth in SEQ ID NO: 66 by the deletion, substitution, insertion, or addition of, for example, 1 to 20, preferably 1 to 10, more preferably 1 to 5 amino acids.

Hereinafter, the heavy chain constant region (CH) of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-97 antibody, an anti-SSTR2 antibody, an anti-CD30 antibody, an anti-BCMA antibody, an anti-CD33 antibody, an anti-FoLRα antibody, an anti- antibody, an anti-CADM1 antibody, an anti-CLL1 antibody, an anti-CD38 antibody, an anti-CD74 antibody, an anti-NECTIN4 antibody, or an anti-TROP2 antibody) will be described.

CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may be any CH that belongs to a human immunoglobulin (hereinafter, also referred to as "hIg"). For example, CH of a hIgG class, specifically, CH of a hIgG subclass such as hIgG1, hIgG2, hIgG3, or hIgG4, or a variant thereof can be used.

CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) is, for example, hIgG1 CH or a variant thereof, or hIgG4 CH or a variant thereof.

In one embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (a) the amino acid sequence set forth in SEQ ID NO: 1, or (b) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 1. The amino acid sequence set forth in SEQ ID NO: 1 is the amino acid sequence of CH of hIgG1. The amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (a) or (b). Examples of the amino acid sequence (b) include amino acid sequences derived from the amino acid sequence set forth in SEQ ID NO: 1 by the deletion, substitution, insertion, or addition of, for example, 1 to 20, preferably 1 to 10, more preferably 1 to 5 amino acids. Examples of the amino acid sequence (b) include amino acid sequences derived from the amino acid sequence set forth in SEQ ID NO: 1 by the substitution of at least one amino acid by cysteine. The amine acid to be substituted by cysteine is an amino acid other than cysteine.

In an alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (c) the amino acid sequence set forth in SEQ ID NO: 2, or (d) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 2. The amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (c) or (d). Examples of the amino acid sequence (d) include amino acid sequences derived from the amino acid sequence set forth in SEQ ID NO: 2 by the deletion, substitution, insertion, or addition of, for example, 1 to 20, preferably 1 to 10, more preferably 1 to 5 amino acids. Examples of the amino acid sequence (d) include amino acid sequences derived from the amino acid sequence set forth in SEQ ID NO: 2 by the substitution of at least one amino acid by cysteine. The amine acid to be substituted by cysteine is an amino acid other than cysteine.

In an alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (i) the amino acid sequence set forth in SEQ ID NO: 181, or (j) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 181. The amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (i) or (j). Examples of the amino acid sequence (j) include amino acid sequences derived from the amino acid sequence set forth in SEQ ID NO: 181 by the deletion, substitution, insertion, or addition of, for example, 1 to 20, preferably 1 to 10, more preferably 1 to 5 amino acids. Examples of the amino acid sequence (j) include amino acid sequences derived from the amino acid sequence set forth in SEQ ID NO: 181 by the substitution of at least two amino acids by cysteine and lysine. The amine acid to be substituted by cysteine is an amino acid other than cysteine, and the amino acid to be substituted by lysine is an amino acid other than lysine.

In an alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (k) the amino acid sequence set forth in SEQ ID NO: 182, or (l) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 182. The amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (k) or (l). Examples of the amino acid sequence (l) include amino acid sequences derived from the amino acid sequence set forth in SEQ ID NO: 182 by the deletion, substitution, insertion, or addition of, for example, 1 to 20, preferably 1 to 10, more preferably 1 to 5 amino acids. Examples of the amino acid sequence (l) include amino acid sequences derived from the amino acid sequence set forth in SEQ ID NO: 182 by the substitution of at least two amino acids by cysteine and phenylalanine. The amine acid to be substituted by cysteine is an amino acid other than cysteine, and the amino acid to be substituted by phenylalanine is an amino acid other than phenylalanine.

As shown in the table below, the amino acid sequence set forth in SEQ ID NO: 2 is an amino acid sequence derived from the amino acid sequence set forth in SEQ ID NO: 1 by the substitution of serine (underlined in the table below) at position 239 of the heavy chain designated by the EU numbering of Kabat by cysteine (underlined in the table below). Hereinafter, the substitution of serine at position 239 of the heavy chain by cysteine is also referred to as a "S239C mutation". The linker in the drug-linker conjugate can be connected to CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) via a sulfur atom of cysteine introduced by the S239C mutation.

**[Table 25]**

| |
|---|
| SEQ ID NO: 1 |
| |
| SEQ ID NO: 2 |
| ASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPCVFLFPP KPKDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVMHEALHNHYT QKSLSLSPGK |

The amino acid sequence (d) preferably retains the S239C mutation.

As shown in the table below, the amino acid sequence set forth in SEQ ID NO: 181 is an amino acid sequence derived from the human IgG4 heavy chain constant region (CH) by the substitution of serine (underlined in the table below) at position 239 of the heavy chain designated by the EU numbering of Kabat by cysteine (underlined in the table below) and the substitution of arginine at position 409 of the heavy chain by lysine (underlined in the table below). An IgG4 variant containing or consisting of the sequence as the heavy chain constant region can be used as a null body form with attenuated binding to Fcγ receptor (FcgR). Hereinafter, the substitution of serine at position 239 of the heavy chain by cysteine is also referred to as a "S239C mutation", and the substitution of arginine at position 409 of the heavy chain by lysine is also referred to as a "R409K mutation". The linker in the drug-linker conjugate can be connected to CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRa antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) via a sulfur atom of cysteine introduced by the S239C mutation.

**[Table 26]**

| |
|---|
| SEQ ID NO: 181 |
| ASTKGPSVFPLAPCSRSTSESTAALGCLVKDYFPEPVTVSWNSGALTSGVHTFPAVLQSSGLYS LSSVVTVPSSSLGTKTYTCNVDHKPSNTKVDKRVESKYGPPCPPCPAPEFEGGPCVFLFPPKPK DTLMISRTPEVTCVVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSLTCLVKGFYPSDI AVEWESNGQPENNYKTTPPVLDSDGSFFLYSKTVDKSRWQEGNVFSCSVMHEALHNHYTQKSL SLSLGK |

The amino acid sequence (j) preferably retains the S239C mutation and the R409K mutation.

As shown in the table below, the amino acid sequence set forth in SEQ ID NO: 182 is an amino acid sequence derived from the human IgG4 heavy chain constant region (CH) by the substitution of serine at position 239 of the heavy chain designated by the EU numbering of Kabat by cysteine (underlined in the table below) and the substitution of histidine at position 435 of the heavy chain by phenylalanine (underlined in the table below). An IgG4 variant containing or consisting of the sequence as the heavy chain constant region can be used as a short-half-life form with attenuated binding to neonatal Fc receptor (FcRn). Hereinafter, the substitution of serine at position 239 of the heavy chain by cysteine is also referred to as a "S239C mutation", and the substitution of histidine at position 435 of the heavy chain by phenylalanine is also referred to as a "H435F mutation". The linker in the drug-linker conjugate can be connected to CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRa antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) via a sulfur atom of cysteine introduced by the S239C mutation.

**[Table 27]**

| |
|---|
| SEQ ID NO: 182 |
| ASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPCVFLFPP KPKDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVMHEALHNFYTQ KSLSLSPGK |

The amino acid sequence (l) preferably retains the S239C mutation and the H435F mutation.

The antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may have one or two or more glycans linked to CH. The glycan of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) can be selected from, for example, an N-linked glycan and an O-linked glycan and is preferably an N-linked glycan. The N-linked glycan is, for example, an N-linked glycan that is linked to an asparagine residue of CH, and the O-linked glycan is, for example, an O-linked glycan that is linked to a serine residue or a threonine residue of CH. Examples of the N-linked glycan include complex glycans, high-mannose glycans, and hybrid glycans. Examples of the O-linked glycan include core 1 to core 8.

Hereinafter, the light chain constant region (CL) of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) will be described.

CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may be any CL that belongs to hIg. For example, CL of a human κ chain or a human λ chain or a variant thereof can be used.

CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) is, for example, CL of a human κ chain or a variant thereof.

In one embodiment, the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (e) the amino acid sequence set forth in SEQ ID NO: 3, or (f) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 3. The amino acid sequence set forth in SEQ ID NO: 3 is the amino acid sequence of CH of a human κ chain. The amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (e) or (f). Examples of the amino acid sequence (f) include amino acid sequences derived from the amino acid sequence set forth in SEQ ID NO: 3 by the deletion, substitution, insertion, or addition of, for example, 1 to 20, preferably 1 to 10, more preferably 1 to 5 amino acids. Examples of the amino acid sequence (f) include amino acid sequences derived from the amino acid sequence set forth in SEQ ID NO: 3 by the substitution of at least one amino acid by cysteine. The amine acid to be substituted by cysteine is an amino acid other than cysteine.

In an alternative embodiment, the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains (g) the amino acid sequence set forth in SEQ ID NO: 4, or (h) an amino acid sequence having 80% or higher, preferably 85% or higher, more preferably 90% or higher, further preferably 95% or higher, further preferably 96% or higher, further preferably 97% or higher, further preferably 98% or higher, further preferably 99% or higher sequence identity to the amino acid sequence set forth in SEQ ID NO: 4. The amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) may consist of the amino acid sequence (g) or (h). Examples of the amino acid sequence (h) include amino acid sequences derived from the amino acid sequence set forth in SEQ ID NO: 4 by the deletion, substitution, insertion, or addition of, for example, 1 to 20, preferably 1 to 10, more preferably 1 to 5 amino acids. Examples of the amino acid sequence (h) include amino acid sequences derived from the amino acid sequence set forth in SEQ ID NO: 4 by the substitution of at least one amino acid by cysteine. The amine acid to be substituted by cysteine is an amino acid other than cysteine.

As shown in the table below, the amino acid sequence set forth in SEQ ID NO: 4 is an amino acid sequence derived from the amino acid sequence set forth in SEQ ID NO: 3 by the substitution of glutamine (underlined in the table below) at position 124 of the light chain designated by the EU numbering of Kabat by cysteine (underlined in the table below). Hereinafter, the substitution of glutamine at position 124 of the light chain by cysteine is also referred to as a "Q124C mutation". The linker in the drug-linker conjugate can be connected to CL of the antibody (e.g., an anti-CD79b antibody, an anti-CD19 antibody, or an anti-CD22 antibody) via a sulfur atom of cysteine introduced by the Q124C mutation.

**[Table 28]**

| |
|---|
| SEQ ID NO: 3 |
| |
| SEQ ID NO: 4 |
| RTVAAPSVFIFPPSDECLKSGTASVVCLLNNFYPREAKVQWKVDNALQSGNSQESVTEQDSKDS TYSLSSTLTLSKADYEKHKVYACEVTHQGLSSPVTKSFNRGEC |

The amino acid sequence (h) preferably retains the Q124C mutation.

Hereinafter, the combination of CH and CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRa antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) will be described.

In one embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (c) or (d), preferably the amino acid sequence (c), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (e) or (f), preferably the amino acid sequence (e).

In an alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (a) or (b), preferably the amino acid sequence (a), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (g) or (h), preferably the amino acid sequence (g).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (c) or (d), preferably the amino acid sequence (c), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (g) or (h), preferably the amino acid sequence (g).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (a) or (b), preferably the amino acid sequence (a), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (e) or (f), preferably the amino acid sequence (e).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (i) or (j), preferably the amino acid sequence (i), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (e) or (f), preferably the amino acid sequence (e).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (k) or (l), preferably the amino acid sequence (k), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (e) or (f), preferably the amino acid sequence (e).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (i) or (j), preferably the amino acid sequence (i), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (g) or (h), preferably the amino acid sequence (g).

In a further alternative embodiment, the amino acid sequence of CH of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (k) or (l), preferably the amino acid sequence (k), and the amino acid sequence of CL of the antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) contains the amino acid sequence (g) or (h), preferably the amino acid sequence (g).

In the targeting molecule-drug conjugate, the drug (compound (I) or pharmaceutically acceptable salt thereof) is preferably linked to the targeting molecule via a linker.

The linker may have any structure as long as the structure has a function of connecting the targeting molecule and the drug. The linker may have a structure having a special function of, for example, being cleaved near or inside target cells or tissues, or a branched structure that can connect a plurality of drugs. For example, a known linker (e.g., linkers described in S.J. Walsh et al., Chem. Soc. Rev. 2021, 50, 1305-1353; L.N. Tumey, (2020). Antibody-Drug Conjugates-Methods and Protocols: New York, Springer; and L. Ducry, (2013). Antibody-Drug Conjugate: New York, Springer) can be connected to the compound (I) or the pharmaceutically acceptable salt thereof with reference to a known production method (e.g., M. Frigerio & A.F. Kyle, Curr. Top. Med. Chem. 2017, 17, 3393-3424). Specific examples thereof include a linker consisting of any one member selected from the group consisting of a peptide, an oligosaccharide, -(CH₂)-, an oxygen atom, a sulfur atom, -NH-, -(CH₂CH₂O)-, -CO-, -PO-, an amino acid, paraaminobenzyl (PAB), cyclic alkyl having 3 to 10 carbon atoms, and a structure represented by the following formula, and a linker containing a structure where two or more units selected from the above group are linked.

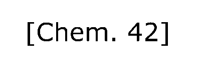

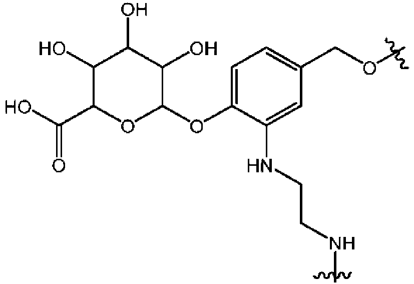

Examples of the amino acid constituting the linker include valine (Val), citrulline (Cit), phenylalanine (Phe), lysine (Lys), D-valine (D-Val), leucine (Leu), isoleucine (Ile), glycine (Gly), alanine (Ala), asparagine (Asn), glutamic acid (Glu), cysteic acid (Cya), arginine (Arg), glutamine (Gln), serine (Ser), proline (Pro), threonine (Thr), tyrosine (Tyr), and combinations thereof. Alternatively, the amino acid may be, for example, a peptide mimetic represented by the following formula (IV):

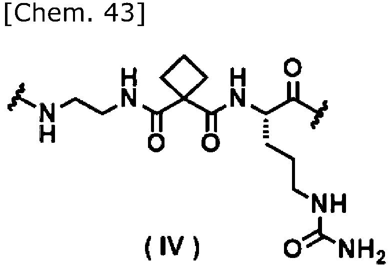

The targeting molecule (also including a glycan linked to an antibody when the targeting molecule is the antibody) can be linked to the linker by reacting a functional group, such as an α,β-unsaturated carbonyl group, an α,β-unsaturated sulfinyl group, an α,β-unsaturated sulfonyl group, a sulfhydryl group, a hydroxy group, an amino group, an amide group, a formyl group, a carboxyl group, an azide group, an alkynyl group, an alkenyl group, a haloalkyl group, or a carbonyl group, introduced in the targeting molecule with a functional group contained in the linker under proper conditions. For example, a bond can be formed through the nucleophilic reaction of a nucleophilic functional group such as a sulfhydryl group in the antibody with a Michael acceptor such as an α,β-unsaturated carbonyl group contained in the linker. For example, a bond can also be formed through the cyclization reaction of an azide group in the antibody with an alkynyl group contained in the linker in the presence or absence of a catalyst.

When the targeting molecule is an antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody), the linker is preferably connected to an amino acid residue of CH and/or CL of the antibody or connected to an N-linked glycan linked to an amino acid residue of CH of the antibody.

When the targeting molecule is an antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) having a S239C mutation introduced in CH, the antibody and the linker are preferably connected via -S-formed from an SH group of cysteine at position 239 and a functional group of the linker (i.e., via a sulfur atom of cysteine at position 239).

When the targeting molecule is an antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) having a Q124C mutation introduced in CL, the antibody and the linker are preferably connected via -S-formed from an SH group of cysteine at position 124 and a functional group of the linker (i.e., via a sulfur atom of cysteine at position 124).

When the targeting molecule is an antibody (e.g., an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-NECTIN4 antibody, or an anti-TROP2 antibody) having an azide group-containing glycan introduced in CH, the antibody and the linker are preferably connected via triazole formed from the azide group of the glycan and a functional group of the linker.

The targeting molecule-drug conjugate is preferably represented by the following formula (II):

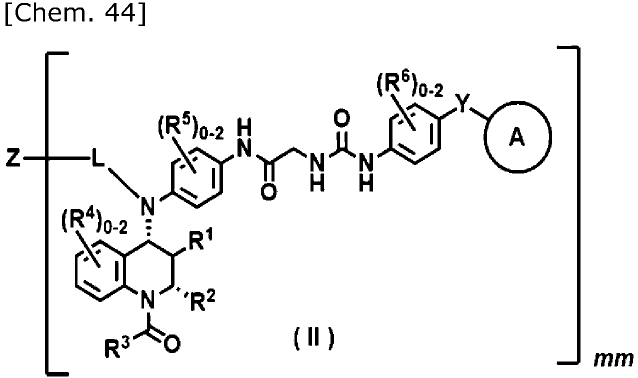

In the formula (II),
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-,
ring A represents
   (i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
   (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
   (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
L represents a linker,
mm represents an integer of 1 to 8, and
Z represents a targeting molecule.

The description about R¹, R², R³, R⁴, R⁵, R⁶, Y, and ring A in the formula (I) is also applied to R¹, R², R³, R⁴, R⁵, R⁶, Y, and ring A in the formula (II).

In the formula (II), mm is preferably an integer of 1 to 20, more preferably an integer of 1 to 8, further preferably an integer of 1 to 4, most preferably 1 or 2.

In a preferred embodiment, the aliphatic heterocyclic group in ring A in the formula (II) is a nitrogen-containing aliphatic heterocyclic group.

In a more preferred embodiment, ring A represented by (i) the nitrogen-containing aliphatic heterocyclic group in the formula (II) is the following ring A':

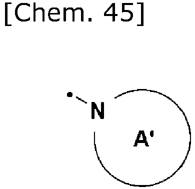

When ring A' has a substituent, the substituent of ring A' is as defined for the substituent of ring A which is (i) the aliphatic heterocyclic group.

In a more preferred embodiment, the targeting molecule-drug conjugate of the formula (II) is a targeting molecule-drug conjugate represented by the following formula (II'):

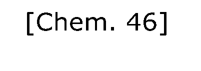

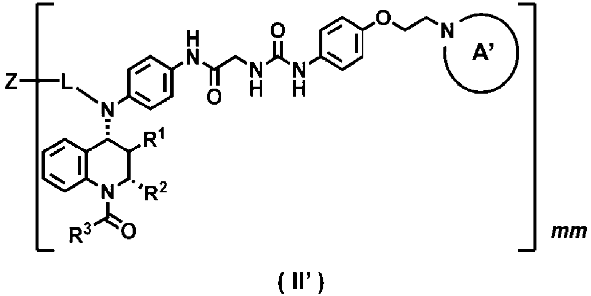

The description about R¹, R², and R³ in the formula (I) or (I') is also applied to R¹, R², and R³ in the formula (II'). The description about ring A' in the formula (I) or (I') is also applied to ring A' in the formula (II'). The description about L, mm, and Z in the (II) is also applied to L, mm, and Z in the (II').

In an embodiment in which the targeting molecule is an antibody, the average drug-antibody ratio (DAR) of a mixture of two or more targeting molecule-drug conjugates is preferably 1 to 8, more preferably 1 to 4, further preferably 1 to 2. DAR can be calculated from a peak area ratio determined by hydrophobic interaction chromatography (HIC) analysis or by reverse-phase HPLC analysis after the reduction of the antibody-drug conjugate, or from the ratio of the ion intensity of a linker payload-connected heavy chain and light chain to that of a linker payload-unconnected heavy chain and light chain determined by the pretreatment (glycan removal using PNGaseF and subsequent separation between the heavy chain and the light chain by reduction operation) of the antibody-drug conjugate followed by LC/MS analysis, according to a method described in, for example, Antibody-Drug Conjugates (Laurent Ducry ed., 2013, Humana Press) or Antibody-Drug Conjugates Methods and Protocols (L. Nathan Tumey ed., 2020, Humana Press).

When the targeting molecule is an antibody (e.g., an anti-CD79b antibody, an anti-CD19 antibody, or an anti-CD22 antibody), linker L is preferably connected to an amino acid residue of CH and/or CL of the antibody or connected to an N-linked glycan linked to an amino acid residue of CH of the antibody.

When the targeting molecule is an antibody (e.g., an anti-CD79b antibody, an anti-CD19 antibody, or an anti-CD22 antibody) having an S239C mutation introduced in CH, the antibody and linker L are preferably connected via -S- formed from an SH group of cysteine at position 239 and a functional group of linker L (i.e., via a sulfur atom of cysteine at position 239).

When the targeting molecule is an antibody (e.g., an anti-CD79b antibody, an anti-CD19 antibody, or an anti-CD22 antibody) having a Q124C mutation introduced in CL, the antibody and linker L are preferably connected via -S- formed from an SH group of cysteine at position 124 and a functional group of linker L (i.e., via a sulfur atom of cysteine at position 124).

When the targeting molecule is an antibody (e.g., an anti-CD79b antibody, an anti-CD19 antibody, or an anti-CD22 antibody) having an azide group-containing glycan introduced in CH, the antibody and the linker are preferably connected via triazole formed from the azide group of the glycan and a functional group of the linker.

When the targeting molecule-drug conjugate is represented by the formula (II) or (II'), L is preferably represented by the following formula (III):

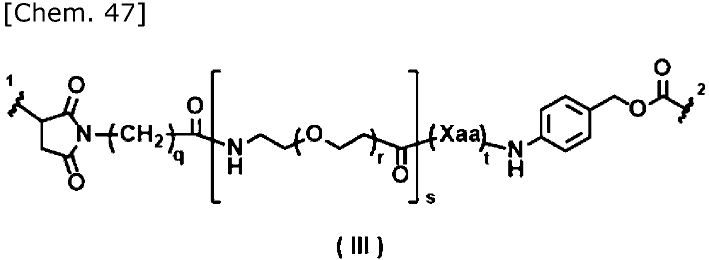

In the formula (III),
wavy line 1 represents a bonding site to Z,
wavy line 2 represents a bonding site to the N atom, and
each Xaa independently represents
   a residue of an amino acid selected from valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents
   a peptide mimetic represented by the following formula (IV): 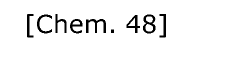 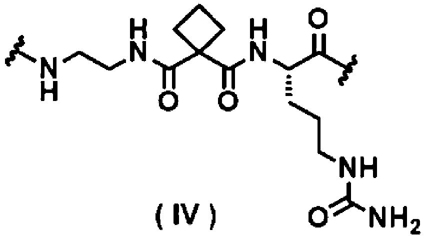 wherein
      q represents an integer of 1 to 5,
      r represents an integer of 2 to 24,
      s represents 0 or 1, and
      t represents 1, 2, 3, or 4.

In the formula (III), when t represents 2, 3, or 4, t Xaa may be the same or different.

In the formula (III), each Xaa is independently preferably a residue of at least one amino acid selected from the group consisting of valine, alanine, citrulline, glycine, phenylalanine, glutamic acid, and proline, or a peptide mimetic represented by the formula (IV), more preferably a residue of an amino acid selected from valine and alanine.

In the formula (III), the bond on the left side of each Xaa is formed from an amino group of each Xaa, and the bond on the right side of each Xaa is formed from a carboxyl group of each Xaa. Specifically, in the formula (III), each Xaa is represented by -NH-Xaa'-CO- [wherein Xaa' represents a moiety that excludes the amino group and the carboxyl group from Xaa].

In the formula (III), q is preferably 2 or 5, more preferably 2.

In the formula (III), r is preferably 6 or 12, more preferably 12.

In the formula (III), s is preferably 1.

In the formula (III), t is preferably 2.

In the (III), preferably, t is 2, and two Xaa are preferably valine and alanine. Specifically, -(Xaa)ₜ- is preferably a dipeptide residue represented by the formula: -valine residue-alanine residue-. In the dipeptide residue represented by the formula: - valine residue-alanine residue-, the alanine residue is preferably bonded to the N atom.

In an alternative embodiment of the present invention, an N atom in the compound represented by the formula (I) or the formula (I') in the targeting molecule-drug conjugate may be converted to a quaternary ammonium salt and bonded to the linker (L). Ring A in the compound represented by the formula (I) preferably contains a nitrogen atom as a heteroatom in (i) the aliphatic heterocyclic group or (ii) the aromatic heterocyclic group.

### <<Third aspect>>

A third aspect of the present invention provides a therapeutic agent for cancer, containing the compound (I) or the pharmaceutically acceptable salt thereof according to the first aspect or the targeting molecule-drug conjugate according to the second aspect as an active ingredient.

In one embodiment, the cancer is preferably blood cancer, more preferably lymphoma, further preferably B cell lymphoma.

Examples of the B cell lymphoma include diffuse large B cell lymphoma (DLBCL), chronic lymphocytic leukemia/small lymphocytic lymphoma, lymphoplasmacytic lymphoma, splenic marginal zone lymphoma, MALT lymphoma, nodal marginal zone lymphoma, follicular lymphoma, mantle cell lymphoma, and Burkitt's lymphoma. Among them, DLBCL is preferred.

In an alternative embodiment, when the targeting molecule of the targeting molecule-drug conjugate according to the second aspect is an anti-CD79b antibody, an anti-CD22 antibody, an anti-CD19 antibody, or an anti-CD74 antibody (specifically, in the case of ADC using an anti-CD79b antibody, ADC using an anti-CD22 antibody, ADC using an anti-CD19 antibody, or ADC using an anti-CD74 antibody), the cancer is preferably B cell lymphoma, more preferably CD79b-positive B cell lymphoma, CD22-positive B cell lymphoma, CD19-positive B cell lymphoma, or CD74-positive B cell lymphoma, respectively. When the targeting molecule is an anti-HER2 antibody (specifically, in the case of ADC using an anti-HER2 antibody), the cancer is preferably breast cancer, stomach cancer, colorectal cancer, or urinary bladder cancer, more preferably HER2-positive breast cancer, HER2-positive stomach cancer, HER2-positive colorectal cancer, or HER2-positive urinary bladder cancer. When the targeting molecule is an anti-DLL3 antibody or an anti-GM2 antibody (specifically, in the case of ADC using an anti-DLL3 antibody or ADC using an anti-GM2 antibody), the cancer is preferably small-cell lung cancer, more preferably DLL3-positive small-cell lung cancer or GM2-positive small-cell lung cancer, respectively. When the targeting molecule is an anti-EphA2 antibody (specifically, in the case of ADC using an anti-EphA2 antibody), the cancer is preferably bile duct adenocarcinoma, glioblastoma, nasopharyngeal cancer, stomach cancer, Ewing's sarcoma, or ovary cancer, more preferably EphA2-positive bile duct adenocarcinoma, EphA2-positive glioblastoma, EphA2-positive nasopharyngeal cancer, EphA2-positive stomach cancer, EphA2-positive Ewing's sarcoma, or EphA2-positive ovary cancer. When the targeting molecule is an anti-FAP antibody (specifically, in the case of ADC using an anti-FAP antibody), the cancer is preferably thyroid gland cancer or acute myeloid leukemia (e.g., acute monocytic leukemia), more preferably FAP-positive thyroid gland cancer or FAP-positive acute myeloid leukemia (e.g., FAP-positive acute monocytic leukemia). When the targeting molecule is an anti-SSTR2 antibody (specifically, in the case of ADC using an anti-SSTR2 antibody), the cancer is preferably pancreatic cancer, more preferably SSTR2-positive pancreatic cancer. When the targeting molecule is an anti-CD30 antibody (specifically, in the case of ADC using an anti-CD30 antibody), the cancer is preferably T cell lymphoma, more preferably CD30-positive T cell lymphoma. When the targeting molecule is an anti-BCMA antibody, an anti-CD38 antibody, or an anti-CLL1 antibody (specifically, in the case of ADC using an anti-BCMA antibody or ADC using an anti-CD38 antibody or in the case of an anti-CLL1 antibody), the cancer is preferably multiple myeloma, more preferably BCMA-positive multiple myeloma, CD38-positive multiple myeloma, or CLL1-positive multiple myeloma, respectively. When the targeting molecule is an anti-CD33 antibody (specifically, in the case of ADC using an anti-CD33 antibody), the cancer is preferably acute myeloid leukemia (e.g., acute monocytic leukemia), more preferably CD33-positive acute myeloid leukemia (e.g., CD33-positive acute monocytic leukemia). When the targeting molecule is an anti-FoLRα antibody (specifically, in the case of ADC using an anti-FoLRα antibody), the cancer is preferably ovary cancer, uterine corpus cancer, breast cancer, or non-small cell lung cancer, more preferably FoLRα-positive ovary cancer, FoLRα-positive uterine corpus cancer, FoLRα-positive breast cancer, or FoLRα-positive non-small cell lung cancer. When the targeting molecule is an anti-CD25 antibody (specifically, in the case of ADC using an anti-CD25 antibody), the cancer is preferably B cell lymphoma or acute myeloid leukemia (e.g., acute monocytic leukemia), more preferably CD25-positive B cell lymphoma or CD25-positive acute myeloid leukemia (e.g., CD25-positive acute monocytic leukemia). When the targeting molecule is an anti-CADM1 antibody (specifically, in the case of ADC using an anti-CADM1 antibody), the cancer is preferably T cell lymphoma or small-cell lung cancer, more preferably CADM1-positive T cell lymphoma or CADM1-positive small-cell lung cancer. When the targeting molecule is an anti-NECTIN4 antibody (specifically, in the case of ADC using an anti-NECTIN4 antibody), the cancer is preferably urinary bladder cancer (e.g., urothelial cancer), more preferably NECTIN4-positive urinary bladder cancer (e.g., NECTIN4-positive urothelial cancer). When the targeting molecule is an anti-TROP2 antibody (specifically, in the case of ADC using an anti-TROP2 antibody), the cancer is preferably lung cancer (e.g., non-small cell lung cancer) or urinary bladder cancer, more preferably TROP2-positive lung cancer (e.g., TROP2-positive non-small cell lung cancer) or TROP2-positive urinary bladder cancer.

The therapeutic agent for cancer is administered to, for example, an animal or a human in need of the treatment of cancer, preferably a human in need of the treatment of cancer.

The compound (I) or the pharmaceutically acceptable salt thereof according to the first aspect or the targeting molecule-drug conjugate according to the second aspect may be administered alone and usually, is desirably provided as various pharmaceutical preparations.

The pharmaceutical preparation can contain, as an active ingredient, the compound (I) or the pharmaceutically acceptable salt thereof according to the first aspect or the targeting molecule-drug conjugate according to the second aspect alone or as a mixture with any of other active ingredients for treatment. The pharmaceutical preparation is produced according to a freely-selected method well known in the technical field of pharmaceutics by mixing the active ingredient with one or more pharmaceutically acceptable carriers (e.g., an attenuant, a solvent, or a diluent).

In one embodiment, the pharmaceutical preparation is a pharmaceutical composition containing the compound (I) or the pharmaceutically acceptable salt thereof according to the first aspect and one or two or more carriers.

In an alternative embodiment, the pharmaceutical preparation is a pharmaceutical composition containing the targeting molecule-drug conjugate according to the second aspect and one or two or more carriers.

The most effective administration route is desirably used for the treatment. Examples thereof can include parental routes such as intravenous routes.

Examples of the administration form include injections.

A formulation appropriate for the parenteral administration, for example, an injection, can be produced using, for example, an attenuant such as a salt solution, a glucose solution, or a mixed solution of saline and a glucose solution, or a solvent.

The dose and frequency of administration of the compound (I) or the pharmaceutically acceptable salt thereof according to the first aspect or the targeting molecule-drug conjugate according to the second aspect differ depending on the administration form, the age or body weight of the patient, the nature or severity of the symptoms to be treated, or the like. For oral administration, the compound (I) or the pharmaceutically acceptable salt thereof or the targeting molecule-drug conjugate is usually administered in the range of 0.01 to 1000 mg, preferably 0.05 to 100 mg, per adult once daily or several times a day. For parenteral administrations such as intravenous administration, the compound (I) or the pharmaceutically acceptable salt thereof or the targeting molecule-drug conjugate is administered at 0.001 to 1000 mg, preferably 0.01 to 100 mg, per adult once daily or several times a day. However, such a dose and frequency of administration vary depending on various conditions mentioned above.

The amount of the compound (I) or the pharmaceutically acceptable salt thereof according to the first aspect blended can be appropriately adjusted depending on the dosage form of the therapeutic agent for cancer, or the like.

The amount of the targeting molecule-drug conjugate according to the second aspect blended can be appropriately adjusted depending on the dosage form of the therapeutic agent for cancer, or the like.

### <<Fourth aspect>>

A fourth aspect of the present invention provides a method for treating cancer, including administering the compound (I) or the pharmaceutically acceptable salt thereof according to the first aspect or the targeting molecule-drug conjugate according to the second aspect to a subject.

The subject is a subject in need of the treatment of cancer. The subject also encompasses a nonhuman animal and is preferably a human.

In one embodiment, the cancer is preferably blood cancer, more preferably lymphoma, further preferably B cell lymphoma.

Examples of the B cell lymphoma include diffuse large B cell lymphoma (DLBCL), chronic lymphocytic leukemia/small lymphocytic lymphoma, lymphoplasmacytic lymphoma, splenic marginal zone lymphoma, MALT lymphoma, nodal marginal zone lymphoma, follicular lymphoma, mantle cell lymphoma, and Burkitt's lymphoma. Among them, DLBCL is preferred.

In an alternative embodiment, when the targeting molecule of the targeting molecule-drug conjugate according to the second aspect is an anti-CD79b antibody, an anti-CD22 antibody, an anti-CD19 antibody, or an anti-CD74 antibody (specifically, in the case of ADC using an anti-CD79b antibody, ADC using an anti-CD22 antibody, ADC using an anti-CD19 antibody, or ADC using an anti-CD74 antibody), the cancer is preferably B cell lymphoma, more preferably CD79b-positive B cell lymphoma, CD22-positive B cell lymphoma, CD19-positive B cell lymphoma, or CD74-positive B cell lymphoma, respectively. When the targeting molecule is an anti-HER2 antibody (specifically, in the case of ADC using an anti-HER2 antibody), the cancer is preferably breast cancer, stomach cancer, colorectal cancer, or urinary bladder cancer, more preferably HER2-positive breast cancer, HER2-positive stomach cancer, HER2-positive colorectal cancer, or HER2-positive urinary bladder cancer. When the targeting molecule is an anti-DLL3 antibody or an anti-GM2 antibody (specifically, in the case of ADC using an anti-DLL3 antibody or ADC using an anti-GM2 antibody), the cancer is preferably small-cell lung cancer, more preferably DLL3-positive small-cell lung cancer or GM2-positive small-cell lung cancer, respectively. When the targeting molecule is an anti-EphA2 antibody (specifically, in the case of ADC using an anti-EphA2 antibody), the cancer is preferably bile duct adenocarcinoma, glioblastoma, nasopharyngeal cancer, stomach cancer, Ewing's sarcoma, or ovary cancer, more preferably EphA2-positive bile duct adenocarcinoma, EphA2-positive glioblastoma, EphA2-positive nasopharyngeal cancer, EphA2-positive stomach cancer, EphA2-positive Ewing's sarcoma, or EphA2-positive ovary cancer. When the targeting molecule is an anti-FAP antibody (specifically, in the case of ADC using an anti-FAP antibody), the cancer is preferably thyroid gland cancer or acute myeloid leukemia (e.g., acute monocytic leukemia), more preferably FAP-positive thyroid gland cancer or FAP-positive acute myeloid leukemia (e.g., FAP-positive acute monocytic leukemia). When the targeting molecule is an anti-SSTR2 antibody (specifically, in the case of ADC using an anti-SSTR2 antibody), the cancer is preferably pancreatic cancer, more preferably SSTR2-positive pancreatic cancer. When the targeting molecule is an anti-CD30 antibody (specifically, in the case of ADC using an anti-CD30 antibody), the cancer is preferably T cell lymphoma, more preferably CD30-positive T cell lymphoma. When the targeting molecule is an anti-BCMA antibody, an anti-CD38 antibody, or an anti-CLL1 antibody (specifically, in the case of ADC using an anti-BCMA antibody or ADC using an anti-CD38 antibody or in the case of an anti-CLL1 antibody), the cancer is preferably multiple myeloma, more preferably BCMA-positive multiple myeloma, CD38-positive multiple myeloma, or CLL1-positive multiple myeloma, respectively. When the targeting molecule is an anti-CD33 antibody (specifically, in the case of ADC using an anti-CD33 antibody), the cancer is preferably acute myeloid leukemia (e.g., acute monocytic leukemia), more preferably CD33-positive acute myeloid leukemia (e.g., CD33-positive acute monocytic leukemia). When the targeting molecule is an anti-FoLRα antibody (specifically, in the case of ADC using an anti-FoLRα antibody), the cancer is preferably ovary cancer, uterine corpus cancer, breast cancer, or non-small cell lung cancer, more preferably FoLRα-positive ovary cancer, FoLRα-positive uterine corpus cancer, FoLRα-positive breast cancer, or FoLRα-positive non-small cell lung cancer. When the targeting molecule is an anti-CD25 antibody (specifically, in the case of ADC using an anti-CD25 antibody), the cancer is preferably B cell lymphoma or acute myeloid leukemia (e.g., acute monocytic leukemia), more preferably CD25-positive B cell lymphoma or CD25-positive acute myeloid leukemia (e.g., CD25-positive acute monocytic leukemia). When the targeting molecule is an anti-CADM1 antibody (specifically, in the case of ADC using an anti-CADM1 antibody), the cancer is preferably T cell lymphoma or small-cell lung cancer, more preferably CADM1-positive T cell lymphoma or CADM1-positive small-cell lung cancer. When the targeting molecule is an anti-NECTIN4 antibody (specifically, in the case of ADC using an anti-NECTIN4 antibody), the cancer is preferably urinary bladder cancer (e.g., urothelial cancer), more preferably NECTIN4-positive urinary bladder cancer (e.g., NECTIN4-positive urothelial cancer). When the targeting molecule is an anti-TROP2 antibody (specifically, in the case of ADC using an anti-TROP2 antibody), the cancer is preferably lung cancer (e.g., non-small cell lung cancer) or urinary bladder cancer, more preferably TROP2-positive lung cancer (e.g., TROP2-positive non-small cell lung cancer) or TROP2-positive urinary bladder cancer.

The description about the third aspect is also applied to the fourth aspect.

### <<Fifth aspect>>

A fifth aspect of the present invention provides the compound (I) or the pharmaceutically acceptable salt thereof according to the first aspect or the targeting molecule-drug conjugate according to the second aspect for use in the treatment of cancer.

In one embodiment, the cancer is preferably blood cancer, more preferably lymphoma, further preferably B cell lymphoma.

Examples of the B cell lymphoma include diffuse large B cell lymphoma (DLBCL), chronic lymphocytic leukemia/small lymphocytic lymphoma, lymphoplasmacytic lymphoma, splenic marginal zone lymphoma, MALT lymphoma, nodal marginal zone lymphoma, follicular lymphoma, mantle cell lymphoma, and Burkitt's lymphoma. Among them, DLBCL is preferred.

In an alternative embodiment, when the targeting molecule of the targeting molecule-drug conjugate according to the second aspect is an anti-CD79b antibody, an anti-CD22 antibody, an anti-CD19 antibody, or an anti-CD74 antibody (specifically, in the case of ADC using an anti-CD79b antibody, ADC using an anti-CD22 antibody, ADC using an anti-CD19 antibody, or ADC using an anti-CD74 antibody), the cancer is preferably B cell lymphoma, more preferably CD79b-positive B cell lymphoma, CD22-positive B cell lymphoma, CD19-positive B cell lymphoma, or CD74-positive B cell lymphoma, respectively. When the targeting molecule is an anti-HER2 antibody (specifically, in the case of ADC using an anti-HER2 antibody), the cancer is preferably breast cancer, stomach cancer, colorectal cancer, or urinary bladder cancer, more preferably HER2-positive breast cancer, HER2-positive stomach cancer, HER2-positive colorectal cancer, or HER2-positive urinary bladder cancer. When the targeting molecule is an anti-DLL3 antibody or an anti-GM2 antibody (specifically, in the case of ADC using an anti-DLL3 antibody or ADC using an anti-GM2 antibody), the cancer is preferably small-cell lung cancer, more preferably DLL3-positive small-cell lung cancer or GM2-positive small-cell lung cancer, respectively. When the targeting molecule is an anti-EphA2 antibody (specifically, in the case of ADC using an anti-EphA2 antibody), the cancer is preferably bile duct adenocarcinoma, glioblastoma, nasopharyngeal cancer, stomach cancer, Ewing's sarcoma, or ovary cancer, more preferably EphA2-positive bile duct adenocarcinoma, EphA2-positive glioblastoma, EphA2-positive nasopharyngeal cancer, EphA2-positive stomach cancer, EphA2-positive Ewing's sarcoma, or EphA2-positive ovary cancer. When the targeting molecule is an anti-FAP antibody (specifically, in the case of ADC using an anti-FAP antibody), the cancer is preferably thyroid gland cancer or acute myeloid leukemia (e.g., acute monocytic leukemia), more preferably FAP-positive thyroid gland cancer or FAP-positive acute myeloid leukemia (e.g., FAP-positive acute monocytic leukemia). When the targeting molecule is an anti-SSTR2 antibody (specifically, in the case of ADC using an anti-SSTR2 antibody), the cancer is preferably pancreatic cancer, more preferably SSTR2-positive pancreatic cancer. When the targeting molecule is an anti-CD30 antibody (specifically, in the case of ADC using an anti-CD30 antibody), the cancer is preferably T cell lymphoma, more preferably CD30-positive T cell lymphoma. When the targeting molecule is an anti-BCMA antibody, an anti-CD38 antibody, or an anti-CLL1 antibody (specifically, in the case of ADC using an anti-BCMA antibody or ADC using an anti-CD38 antibody or in the case of an anti-CLL1 antibody), the cancer is preferably multiple myeloma, more preferably BCMA-positive multiple myeloma, CD38-positive multiple myeloma, or CLL1-positive multiple myeloma, respectively. When the targeting molecule is an anti-CD33 antibody (specifically, in the case of ADC using an anti-CD33 antibody), the cancer is preferably acute myeloid leukemia (e.g., acute monocytic leukemia), more preferably CD33-positive acute myeloid leukemia (e.g., CD33-positive acute monocytic leukemia). When the targeting molecule is an anti-FoLRα antibody (specifically, in the case of ADC using an anti-FoLRα antibody), the cancer is preferably ovary cancer, uterine corpus cancer, breast cancer, or non-small cell lung cancer, more preferably FoLRα-positive ovary cancer, FoLRα-positive uterine corpus cancer, FoLRα-positive breast cancer, or FoLRα-positive non-small cell lung cancer. When the targeting molecule is an anti-CD25 antibody (specifically, in the case of ADC using an anti-CD25 antibody), the cancer is preferably B cell lymphoma or acute myeloid leukemia (e.g., acute monocytic leukemia), more preferably CD25-positive B cell lymphoma or CD25-positive acute myeloid leukemia (e.g., CD25-positive acute monocytic leukemia). When the targeting molecule is an anti-CADM1 antibody (specifically, in the case of ADC using an anti-CADM1 antibody), the cancer is preferably T cell lymphoma or small-cell lung cancer, more preferably CADM1-positive T cell lymphoma or CADM1-positive small-cell lung cancer. When the targeting molecule is an anti-NECTIN4 antibody (specifically, in the case of ADC using an anti-NECTIN4 antibody), the cancer is preferably urinary bladder cancer (e.g., urothelial cancer), more preferably NECTIN4-positive urinary bladder cancer (e.g., NECTIN4-positive urothelial cancer). When the targeting molecule is an anti-TROP2 antibody (specifically, in the case of ADC using an anti-TROP2 antibody), the cancer is preferably lung cancer (e.g., non-small cell lung cancer) or urinary bladder cancer, more preferably TROP2-positive lung cancer (e.g., TROP2-positive non-small cell lung cancer) or TROP2-positive urinary bladder cancer.

The description about the third aspect is also applied to the fifth aspect.

### <<Sixth aspect>>

A sixth aspect of the present invention provides use of the compound (I) or the pharmaceutically acceptable salt thereof according to the first aspect or the targeting molecule-drug conjugate according to the second aspect for the production of a drug for the treatment of cancer.

In one embodiment, the cancer is preferably blood cancer, more preferably lymphoma, further preferably B cell lymphoma.

Examples of the B cell lymphoma include diffuse large B cell lymphoma (DLBCL), chronic lymphocytic leukemia/small lymphocytic lymphoma, lymphoplasmacytic lymphoma, splenic marginal zone lymphoma, MALT lymphoma, nodal marginal zone lymphoma, follicular lymphoma, mantle cell lymphoma, and Burkitt's lymphoma. Among them, DLBCL is preferred.

In an alternative embodiment, when the targeting molecule of the targeting molecule-drug conjugate according to the second aspect is an anti-CD79b antibody, an anti-CD22 antibody, an anti-CD19 antibody, or an anti-CD74 antibody (specifically, in the case of ADC using an anti-CD79b antibody, ADC using an anti-CD22 antibody, ADC using an anti-CD19 antibody, or ADC using an anti-CD74 antibody), the cancer is preferably B cell lymphoma, more preferably CD79b-positive B cell lymphoma, CD22-positive B cell lymphoma, CD19-positive B cell lymphoma, or CD74-positive B cell lymphoma, respectively. When the targeting molecule is an anti-HER2 antibody (specifically, in the case of ADC using an anti-HER2 antibody), the cancer is preferably breast cancer, stomach cancer, colorectal cancer, or urinary bladder cancer, more preferably HER2-positive breast cancer, HER2-positive stomach cancer, HER2-positive colorectal cancer, or HER2-positive urinary bladder cancer. When the targeting molecule is an anti-DLL3 antibody or an anti-GM2 antibody (specifically, in the case of ADC using an anti-DLL3 antibody or ADC using an anti-GM2 antibody), the cancer is preferably small-cell lung cancer, more preferably DLL3-positive small-cell lung cancer or GM2-positive small-cell lung cancer, respectively. When the targeting molecule is an anti-EphA2 antibody (specifically, in the case of ADC using an anti-EphA2 antibody), the cancer is preferably bile duct adenocarcinoma, glioblastoma, nasopharyngeal cancer, stomach cancer, Ewing's sarcoma, or ovary cancer, more preferably EphA2-positive bile duct adenocarcinoma, EphA2-positive glioblastoma, EphA2-positive nasopharyngeal cancer, EphA2-positive stomach cancer, EphA2-positive Ewing's sarcoma, or EphA2-positive ovary cancer. When the targeting molecule is an anti-FAP antibody (specifically, in the case of ADC using an anti-FAP antibody), the cancer is preferably thyroid gland cancer or acute myeloid leukemia (e.g., acute monocytic leukemia), more preferably FAP-positive thyroid gland cancer or FAP-positive acute myeloid leukemia (e.g., FAP-positive acute monocytic leukemia). When the targeting molecule is an anti-SSTR2 antibody (specifically, in the case of ADC using an anti-SSTR2 antibody), the cancer is preferably pancreatic cancer, more preferably SSTR2-positive pancreatic cancer. When the targeting molecule is an anti-CD30 antibody (specifically, in the case of ADC using an anti-CD30 antibody), the cancer is preferably T cell lymphoma, more preferably CD30-positive T cell lymphoma. When the targeting molecule is an anti-BCMA antibody, an anti-CD38 antibody, or an anti-CLL1 antibody (specifically, in the case of ADC using an anti-BCMA antibody or ADC using an anti-CD38 antibody or in the case of an anti-CLL1 antibody), the cancer is preferably multiple myeloma, more preferably BCMA-positive multiple myeloma, CD38-positive multiple myeloma, or CLL1-positive multiple myeloma, respectively. When the targeting molecule is an anti-CD33 antibody (specifically, in the case of ADC using an anti-CD33 antibody), the cancer is preferably acute myeloid leukemia (e.g., acute monocytic leukemia), more preferably CD33-positive acute myeloid leukemia (e.g., CD33-positive acute monocytic leukemia). When the targeting molecule is an anti-FoLRα antibody (specifically, in the case of ADC using an anti-FoLRα antibody), the cancer is preferably ovary cancer, uterine corpus cancer, breast cancer, or non-small cell lung cancer, more preferably FoLRα-positive ovary cancer, FoLRα-positive uterine corpus cancer, FoLRα-positive breast cancer, or FoLRα-positive non-small cell lung cancer. When the targeting molecule is an anti-CD25 antibody (specifically, in the case of ADC using an anti-CD25 antibody), the cancer is preferably B cell lymphoma or acute myeloid leukemia (e.g., acute monocytic leukemia), more preferably CD25-positive B cell lymphoma or CD25-positive acute myeloid leukemia (e.g., CD25-positive acute monocytic leukemia). When the targeting molecule is an anti-CADM1 antibody (specifically, in the case of ADC using an anti-CADM1 antibody), the cancer is preferably T cell lymphoma or small-cell lung cancer, more preferably CADM1-positive T cell lymphoma or CADM1-positive small-cell lung cancer. When the targeting molecule is an anti-NECTIN4 antibody (specifically, in the case of ADC using an anti-NECTIN4 antibody), the cancer is preferably urinary bladder cancer (e.g., urothelial cancer), more preferably NECTIN4-positive urinary bladder cancer (e.g., NECTIN4-positive urothelial cancer). When the targeting molecule is an anti-TROP2 antibody (specifically, in the case of ADC using an anti-TROP2 antibody), the cancer is preferably lung cancer (e.g., non-small cell lung cancer) or urinary bladder cancer, more preferably TROP2-positive lung cancer (e.g., TROP2-positive non-small cell lung cancer) or TROP2-positive urinary bladder cancer.

The description about the third aspect is also applied to the sixth aspect.

### <<Seventh aspect>>

A seventh aspect of the present invention provides a drug-linker conjugate containing a drug and a linker linked to the drug, wherein the drug is the compound (I) or the pharmaceutically acceptable salt thereof. In the present specification, the drug-linker conjugate is also referred to as a "linker payload".

In a preferred embodiment, the drug-linker conjugate is represented by the following formula (V):

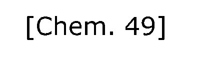

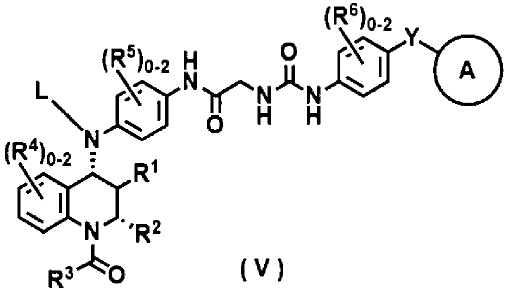

In the formula (V),
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-,
ring A represents
   (i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
   (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
   (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, and
L represents a linker.

The description about R¹, R², R³, R⁴, R⁵, R⁶, Y, and ring A in the formula (I) is also applied to R¹, R², R³, R⁴, R⁵, R⁶, Y, and ring A in the formula (V). The description about L in the (II) is also applied to L in the (V).

In a preferred embodiment, (i) the aliphatic heterocyclic group in ring A in the formula (V) is a nitrogen-containing aliphatic heterocyclic group.

In a more preferred embodiment, ring A represented by (i) the nitrogen-containing aliphatic heterocyclic group in the formula (V) is the following ring A':

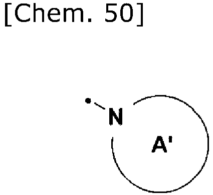

When ring A' has a substituent, the substituent of ring A' is as defined for the substituent of ring A which is (i) the aliphatic heterocyclic group.

In a more preferred embodiment, the drug-linker conjugate of the formula (V) is a drug-linker conjugate represented by the following formula (V'):

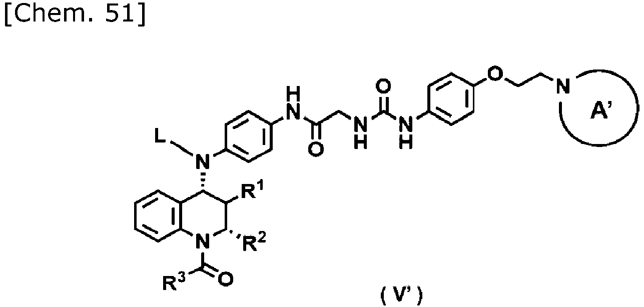

The description about R¹, R², and R³ in the formula (I) or (I') is also applied to R¹, R², and R³ in the formula (V'). The description about ring A' in the formula (I) or (I') is also applied to ring A' in the formula (V'). The description about L in the (II) is also applied to L in the (V').

In a more preferred embodiment, the drug-linker conjugate is represented by the following formula (Va):

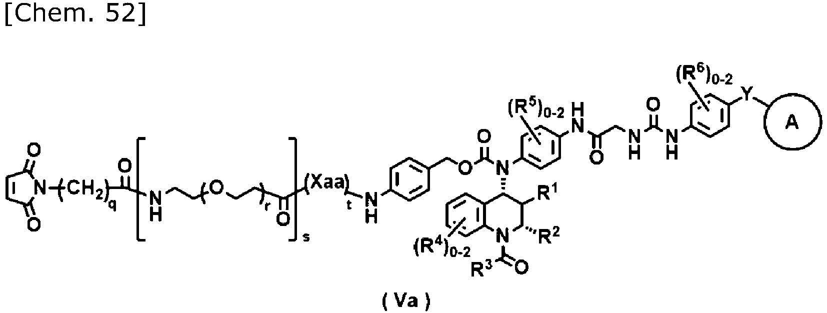

In the formula (Va),
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-,
ring A represents
   (i) a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
   (ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
   (iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
each Xaa is independently at least one amino acid residue selected from the group consisting of valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents a peptide mimetic represented by the following formula (IV): 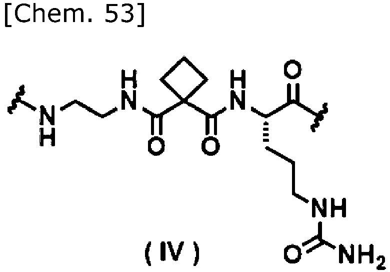 wherein,
   q represents an integer of 1 to 5,
   r represents an integer of 2 to 24,
   s represents 0 or 1, and
   t represents 1, 2, 3, or 4.

The description about R¹, R², R³, R⁴, R⁵, R⁶, and ring A in the formula (I) is also applied to R¹, R², R³, R⁴, R⁵, R⁶, and ring A in the formula (Va).

The description about Xaa, q, r, s, and t in the formula (III) is also applied to Xaa, q, r, s, and t in the formula (Va).

In a preferred embodiment, (i) the aliphatic heterocyclic group in ring A in the formula (Va) is a nitrogen-containing aliphatic heterocyclic group.

In a more preferred embodiment, ring A represented by (i) the nitrogen-containing aliphatic heterocyclic group in the formula (Va) is the following ring A':

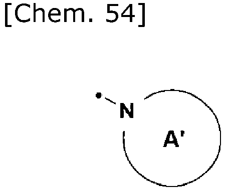

When ring A' has a substituent, the substituent of ring A' is as defined for the substituent of ring A which is (i) the aliphatic heterocyclic group.

In a more preferred embodiment, the drug-linker conjugate of the formula (Va) is a drug-linker conjugate represented by the following formula (Va'):

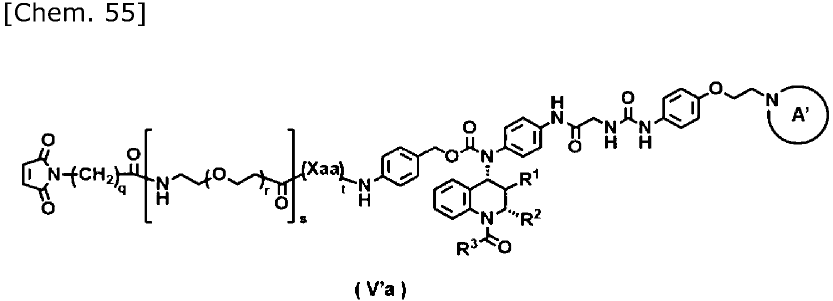

The description about R¹, R², and R³ in the formula (I) or (I') is also applied to R¹, R², and R³ in the formula (V'a). The description about ring A' in the formula (I) or (I') is also applied to ring A' in the formula (V'a). The description about Xaa, q, r, s, and t in the formula (III) is also applied to Xaa, q, r, s, and t in the formula (V'a).

The drug-linker conjugate according to the seventh aspect can be used in the production of the targeting molecule-drug conjugate according to the second aspect.

### <<Production method>>

Hereinafter, each of general methods for producing the compound (I), the drug-linker conjugate, and the targeting molecule-drug conjugate will be described.

In each production method described below, when the defined group changes under the conditions of the production method or is not proper for carrying out the production method, the compound of interest can be produced by using a method of introducing and removing a protecting group commonly used in organic synthetic chemistry [e.g., Protective Groups in Organic Synthesis 53rd edition (a method described in Greene's Protective Groups in Organic Synthesis, fifth Edition), Authored by Greene (T.W. Greene), John Wiley & Sons Inc. (2014)]. The order of reaction steps such as introduction of substituents may be changed, if necessary. In each production method described below, when the obtained product is a mixture of optical isomers, the single optical isomer of interest can be obtained by purification operation such as optical resolution commonly used in organic synthetic chemistry in a freely-selected step.

### [Production method 1-1]

Among compounds represented by the formula (I), a compound (I)-1 in which Y is -Q-CH₂-CH₂- can be produced according to the following steps.

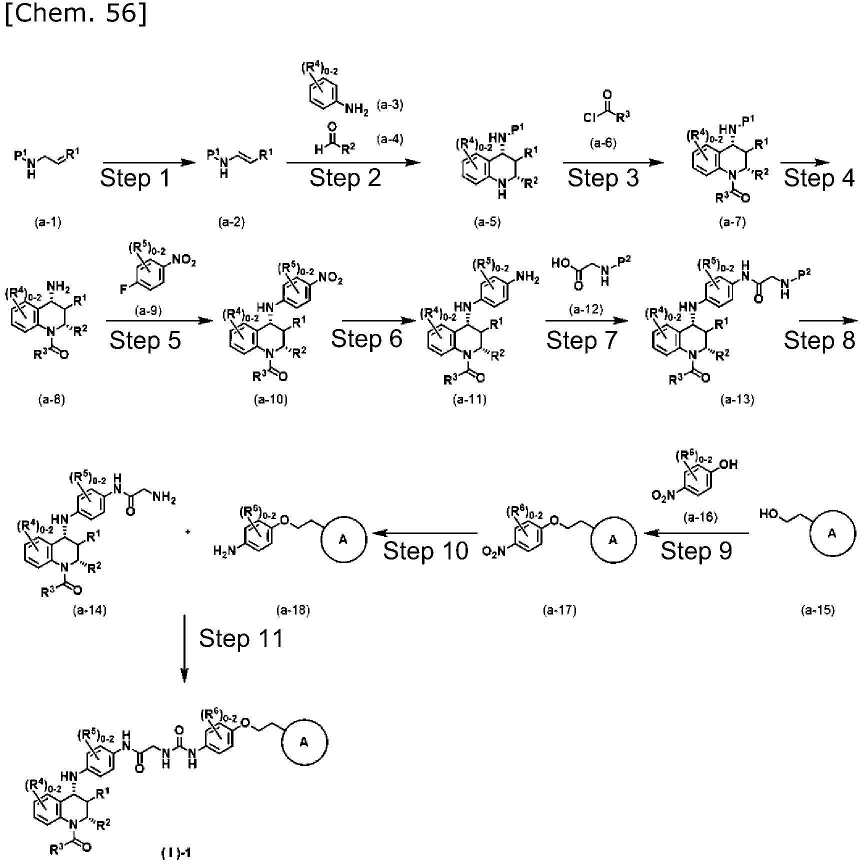

In the steps, R¹, R², R³, R⁴, R⁵, R⁶, and ring A are each as defined above, P¹ represents, for example, a protecting group for amine, such as formyl, tert-butoxycarbonyl (Boc), or p-methoxybenzyl (PMB), and P² represents, for example, a protecting group for amine, such as 2-(trimethylsilyl)ethoxycarbonyl (Teoc), tert-butoxycarbonyl (Boc), 9-fluorenylmethyloxycarbonyl (Fmoc), or allyloxycarbonyl (Alloc).

### (Step 1)

A compound (a-2) can be produced by reacting a compound (a-1) and 0.001 equivalents to 0.5 equivalents of a rhodium catalyst in a solvent at a temperature between -20°C and the boiling point of the solvent used for 5 minutes to 120 hours.

Examples of the rhodium catalyst include rhodium tris(triphenylphosphine)carbonyl hydride.

Examples of the solvent include tetrahydrofuran (THF) and acetonitrile, which can be used alone or as a mixture.

The compound (a-1) can be obtained as a commercially available product, or can be obtained by known methods [e.g., Experimental Chemistry Lecture, 5th Edition, Volume 13, p. 118, Maruzen Co., Ltd. (2004)] or methods based thereon.

### (Step 2)

A compound (a-5) can be produced by reacting a compound (a-3), 1 equivalent to 5 equivalents of the compound (a-2), and 1 equivalent to 5 equivalents of a compound (a-4) in the presence of a catalytic amount of bismuth(III) chloride in a solvent at a temperature between -20°C and the boiling point of the solvent used for 5 minutes to 120 hours.

Examples of the solvent include THF and acetonitrile, which can be used alone or as a mixture.

The compound (a-3) can be obtained as a commercially available product, or can be obtained by known methods [e.g., Experimental Chemistry Lecture, 5th Edition, Volume 14, p. 351, Maruzen Co., Ltd. (2004)] or methods based thereon.

The compound (a-4) can be obtained as a commercially available product, or can be obtained by known methods [e.g., Experimental Chemistry Lecture, 5th Edition, Volume 15, p. 1, Maruzen Co., Ltd. (2004)] or methods based thereon.

The compound (a-5) can be obtained as a single optical isomer by purification operation such as optical resolution commonly used in organic synthetic chemistry.

### (Step 3)

A compound (a-7) can be produced by reacting the compound (a-5) and 1 equivalent to 5 equivalents of a compound (a-6) in the presence of 1 equivalent to a large excess of a base in a solvent at a temperature between -20°C and the boiling point of the solvent used for 5 minutes to 120 hours.

Examples of the base include sodium hydride, potassium hydride, lithium diisopropylamide (LDA), lithium bistrimethylsilylamide, sodium bistrimethylsilylamide, sodium methoxide, potassium ethoxide, potassium tert-butoxide, potassium carbonate, sodium hydroxide, 1,8-diazabicyclo[5.4.0]-7-undecene (DBU), triethylamine, N,N-diisopropylethylamine, pyridine, and 4-dimethylaminopyridine.

Examples of the solvent include chloroform, dichloromethane, dimethylformamide (DMF), dimethylacetamide (DMA), N-methylpyrrolidone (NMP), dimethyl sulfoxide (DMSO), THF, and acetonitrile, which can be used alone or as a mixture.

The compound (a-6) can be obtained as a commercially available product, or can be obtained by known methods [e.g., Experimental Chemistry Lecture, 5th Edition, Volume 16, p. 101, Maruzen Co., Ltd. (2004)] or methods based thereon.

### (Step 4)

A compound (a-8) can be produced by reacting the compound (a-7) in the presence of 1 equivalent to a large excess of an acid in a solvent at a temperature between -20°C and the boiling point of the solvent used for 5 minutes to 120 hours, when P¹ in the compound (a-7) is, for example, formyl or Boc.

Examples of the acid include hydrochloric acid, sulfuric acid, trifluoroacetic acid, trifluoromethanesulfonic acid, and bis(trifluoromethanesulfonyl)imide.

Examples of the solvent include chloroform, dichloromethane, toluene, THF, and acetonitrile, which can be used alone or as a mixture.

In an alternative approach, the compound (a-8) can be produced by reacting the compound (a-7) in the presence of 1 equivalent to 5 equivalents of an oxidizing agent in a solvent at a temperature between -20°C and the boiling point of the solvent used for 5 minutes to 120 hours, when P¹ in the compound (a-7) is, for example, PMB. The compound can also be produced by the same method as in the case where P¹ is Boc.

Examples of the oxidizing agent include 2,3-dichloro-5,6-dicyano-p-benzoquinone (DDQ) and cerium(IV) ammonium nitrate (CAN).

Examples of the solvent include chloroform, dichloromethane, dichloroethane, acetonitrile, and water, which can be used alone or as a mixture.

### (Step 5)

A compound (a-10) can be produced by reacting the compound (a-8) and 1 equivalent to 5 equivalents of a compound (a-9) in the presence of 1 equivalent to a large excess of a base in a solvent at a temperature between -20°C and the boiling point of the solvent used for 5 minutes to 120 hours.

Examples of the base include sodium hydride, potassium hydride, LDA, lithium bistrimethylsilylamide, sodium bistrimethylsilylamide, sodium methoxide, potassium ethoxide, potassium tert-butoxide, potassium carbonate, sodium hydroxide, DBU, triethylamine, and N,N-diisopropylethylamine.

Examples of the solvent include DMF, DMA, NMP, DMSO, THF, and acetonitrile, which can be used alone or as a mixture.

The compound (a-9) can be obtained as a commercially available product, or can be obtained by known methods [e.g., Experimental Chemistry Lecture, 5th Edition, Volume 13, p. 341, Maruzen Co., Ltd. (2004)] or methods based thereon.

### (Step 6)

A compound (a-11) can be produced by reacting the compound (a-10) in the presence of 1 equivalent to a large excess of iron and 1 equivalent to a large excess of an acid in a solvent at a temperature between -20°C and the boiling point of the solvent used for 5 minutes to 120 hours.

Examples of the acid include ammonium chloride, acetic acid, and hydrochloric acid.

Examples of the solvent include methanol, ethanol, and water, which can be used alone or as a mixture.

### (Step 7)

A compound (a-13) can be produced by reacting the compound (a-11) and 1 equivalent to 5 equivalents of a compound (a-12) in the presence of 1 equivalent to a large excess of a condensing agent, 1 equivalent to a large excess of a base, and optionally 0.01 equivalents to a large excess of an additive in a solvent at a temperature between -20°C and the boiling point of the solvent used for 5 minutes to 120 hours.

Examples of the condensing agent include dicyclohexylcarbodiimide (DCC), N-(3-dimethylaminopropyl)-N'-ethylcarbodiimide hydrochloride (EDC), O-(7-azabenzotriazol-1-yl)-N,N,N',N'-tetramethyluronium hexafluorophosphate (HATU), (1-cyano-2-ethoxy-2-oxoethylideneaminooxy)dimethylamino-morpholino-carbenium hexafluorophosphate (COMU), and chloro-N,N,N',N'-tetramethylformamidinium hexafluorophosphate (TCFH).

Examples of the base include triethylamine, N,N-diisopropylethylamine, and 1-methylimidazole (NMI).

Examples of the additive include 1-hydroxybenzotriazole monohydrate (HOBt), 1-hydroxy-7-azabenzotriazole (HOAt), and ethyl cyano(hydroxyimino)acetate.

Examples of the solvent include chloroform, dichloromethane, DMF, DMA, THF, and acetonitrile, which can be used alone or as a mixture.

The compound (a-12) can be obtained as a commercially available product, or can be obtained by known methods [e.g., Experimental Chemistry Lecture, 5th Edition, Volume 16, p. 175, Maruzen Co., Ltd. (2004)] or methods based thereon.

### (Step 8)

A compound (a-14) can be produced by reacting the compound (a-13) in the presence of 1 equivalent to 10 equivalents of a fluorine compound in a solvent at a temperature between - 20°C and the boiling point of the solvent used for 5 minutes to 120 hours, when P² in the compound (a-13) is, for example, Teoc.

Examples of the fluorine compound include cesium fluoride and tetrabutylammonium fluoride (TBAF).

Examples of the solvent include THF, DMF, and NMP, which can be used alone or as a mixture.

In an alternative approach, the compound (a-14) can be produced by reacting the compound (a-13) in the presence of 1 equivalent to a large excess of an acid in a solvent at a temperature between -20°C and the boiling point of the solvent used for 5 minutes to 120 hours, when P² in the compound (a-13) is, for example, Boc.

Examples of the acid include hydrogen chloride, sulfuric acid, trifluoroacetic acid, trifluoromethanesulfonic acid, and bis(trifluoromethanesulfonyl)imide.

Examples of the solvent include chloroform, dichloromethane, toluene, THF, and acetonitrile, which can be used alone or as a mixture.

In an alternative approach, the compound (a-14) can be produced by reacting the compound (a-13) in the presence of 1 equivalent to a large excess of a base in a solvent at a temperature between -20°C and the boiling point of the solvent used for 5 minutes to 120 hours, when P² in the compound (a-13) is, for example, Fmoc.

Examples of the base include pyrrolidine, piperidine, and morpholine.

Examples of the solvent include methanol, ethanol, DMF, DMA, NMP, DMSO, THF, and acetonitrile, which can be used alone or as a mixture.

In an alternative approach, the compound (a-14) can be produced by reacting the compound (a-13) in the presence of 0.001 to 0.5 equivalents of a palladium catalyst and 1 equivalent to a large excess of an additive in a solvent at a temperature between -20°C and the boiling point of the solvent used for 5 minutes to 120 hours, when P² in the compound (a-13) is, for example, Alloc.

Examples of the palladium catalyst include tetrakis(triphenylphosphine)palladium (Pd(PPh₃)₄) and tris(dibenzylideneacetone)dipalladium (Pd₂(dba)₃).

Examples of the additive include piperidine, pyrrolidine, morpholine, N,N-dimethylbarbituric acid, and phenylsilane.

Examples of the solvent include THF, DMF, and NMP, which can be used alone or as a mixture.

### (Step 9)

A compound (a-17) can be produced by reacting a compound (a-15) and 1 to 10 equivalents of a compound (a-16) in the presence of 1 to 10 equivalents of a phosphine compound and 1 to 10 equivalents of an azo compound in a solvent at a temperature between -78°C and the boiling point of the solvent used for 5 minutes to 120 hours.

Examples of the phosphine compound include triphenylphosphine and tributylphosphine.

Examples of the azo compound include diethyl azodicarboxylate (DEAD), di-tert-butyl azadicarboxylate, diisopropyl azadicarboxylate (DIAD), N,N,N',N'-tetramethylazadicarboxamide, 1,1'-(azadicarbonyl)dipiperazine, and N,N,N',N'-tetraisopropylazadicarboxamide.

The combination of the phosphine compound and the azo compound used is preferably, for example, a combination of triphenylphosphine and DIAD.

Examples of the solvent include dichloromethane, chloroform, 1,2-dichloroethane, toluene, ethyl acetate, acetonitrile, diethyl ether, THF, DME, 1, 4-dioxane, DMF, DMA, and NMP, which are used alone or as a mixture.

The compound (a-15) can be obtained as a commercially available product, or can be obtained by known methods [e.g., Experimental Chemistry Lecture, 5th Edition, Volume 14, p. 1, Maruzen Co., Ltd. (2005)] or methods based thereon.

The compound (a-16) can be obtained as a commercially available product, or can be obtained by known methods [e.g., Experimental Chemistry Lecture, 5th Edition, Volume 14, p. 171, Maruzen Co., Ltd. (2005)] or methods based thereon.

### (Step 10)

A compound (a-18) can be produced by reacting the compound (a-17) in the presence of 0.001 equivalents to 0.5 equivalents of a palladium catalyst in a solvent at a temperature between -20°C and the boiling point of the solvent used for 5 minutes to 120 hours under a hydrogen atmosphere.

Examples of the palladium catalyst include palladium-carbon and palladium hydroxide.

Examples of the solvent include methanol, ethanol, ethyl acetate, THF, and 1,4-dioxane, which can be used alone or as a mixture.

### (Step 11)

The compound (I)-1 can be produced by reacting the compound (a-14), 1 equivalent to 10 equivalents of the compound (a-18), and 0.4 equivalents to 10 equivalents of a carbonyl reagent in the presence of 1 equivalent to a large excess of a base in a solvent at a temperature between -20°C and the boiling point of the solvent used for 5 minutes to 120 hours.

Examples of the carbonyl reagent include phosgene, triphosgene, and 1,1-carbonyldiimidazole (CDI).

Examples of the base include triethylamine, N,N-diisopropylethylamine, pyridine, and 4-dimethylaminopyridine.

Examples of the solvent include dichloromethane, chloroform, 1,2-dichloroethane, toluene, ethyl acetate, acetonitrile, diethyl ether, THF, DMF, NMP, and pyridine, which can be used alone or as a mixture.

### [Production method 1-2]

Among compounds represented by the formula (I), a compound (I)-2 in which Y is -CH₂-CH₂-CH₂- and a compound (I)-3 in which Y is -CH=CH-CH₂- can be produced according to the following steps.

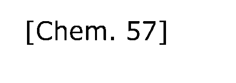

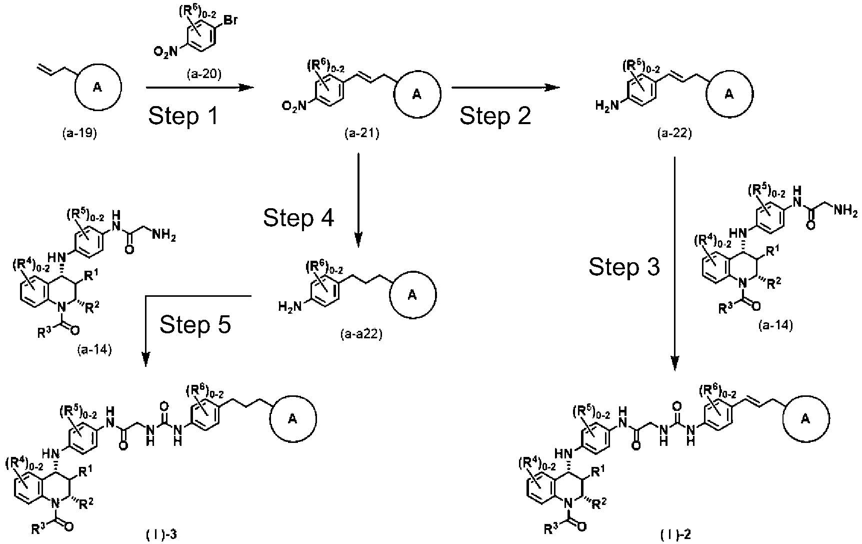

In the steps, R¹, R², R³, R⁴, R⁵, R⁶, and ring A are each as defined above.

### (Step 1)

A compound (a-21) can be produced by reacting a compound (a-19) in the presence of 1 to 10 equivalents of a compound (a-20), 0.1 to 10 equivalents of a base, and 0.001 to 1 equivalents of a palladium catalyst in a solvent at a temperature between -20°C and the boiling point of the solvent used for 5 minutes to 120 hours

Examples of the base include potassium acetate, sodium bicarbonate, potassium carbonate, potassium hydroxide, sodium hydroxide, sodium methoxide, potassium tert-butoxide, triethylamine, diisopropylethylamine, N-methylmorpholine, and DBU.

Examples of the palladium catalyst include palladium acetate, tris(dibenzylideneacetone)dipalladium, tetrakis(triphenylphosphine)palladium, 1:1 adducts of 1,1'-bis(diphenylphosphino)ferrocenedichloropalladium and dichloromethane.

Examples of the solvent include dichloromethane, chloroform, 1,2-dichloroethane, toluene, ethyl acetate, acetonitrile, diethyl ether, THF, DME, 1,4-dioxane, DMF, DMA, and NMP, which are used alone or as a mixture.

The compound (a-19) can be obtained as a commercially available product, or can be obtained by known methods [e.g., Experimental Chemistry Lecture, 5th Edition, Volume 13, p. 118, Maruzen Co., Ltd. (2004)] or methods based thereon.

The compound (a-20) can be obtained as a commercially available product, or can be obtained by known methods [e.g., Experimental Chemistry Lecture, 5th Edition, Volume 13, p. 341, Maruzen Co., Ltd. (2004)] or methods based thereon.

### (Step 2)

A compound (a-22) can be produced in the same manner as in step 6 of production method 1-1 using the compound (a-21).

### (Step 3)

The compound (I)-2 can be produced in the same manner as in step 11 of production method 1-1 using the compound (a-14) and 1 equivalent to 10 equivalents of the compound (a-22).

### (Step 4)

A compound (a-a22) can be produced in the same manner as in step 10 of production method 1-1 using the compound (a-21).

### (Step 5)

The compound (I)-3 can be produced in the same manner as in step 11 of production method 1-1 using the compound (a-14) and 1 equivalent to 10 equivalents of the compound (a-a22).

### [Production method 1-3]

Among compounds represented by the formula (I), a compound (I)-4 in which Y is -NH-C(=O)-CH₂- can be produced according to the following steps.

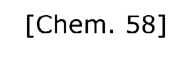

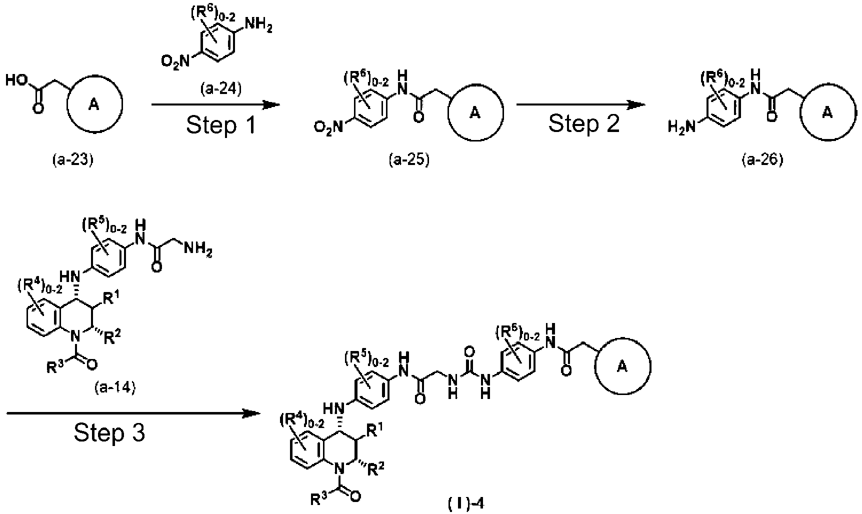

In the steps, R¹, R², R³, R⁴, R⁵, R⁶, and ring A are each as defined above.

### (Step 1)

A compound (a-25) can be produced in the same manner as in step 7 of production method 1-1 using a compound (a-23) and 1 equivalent to 10 equivalents of a compound (a-24).

The compound (a-23) can be obtained as a commercially available product, or can be obtained by known methods [e.g., Experimental Chemistry Lecture, 5th Edition, Volume 16, p. 351, Maruzen Co., Ltd. (2005)] or methods based thereon.

The compound (a-24) can be obtained as a commercially available product, or can be obtained by known methods [e.g., Experimental Chemistry Lecture, 5th Edition, Volume 14, p. 351, Maruzen Co., Ltd. (2004)] or methods based thereon.

### (Step 2)

A compound (a-26) can be produced in the same manner as in step 6 of production method 1-1 using the compound (a-25).

### (Step 3)

The compound (I)-4 can be produced in the same manner as in step 11 of production method 1-1 using the compound (a-14) and 1 equivalent to 10 equivalents of the compound (a-26).

### [Production method 1-4]

Among compounds represented by the formula (I), a compound represented by the formula (I') can be produced according to the following steps.

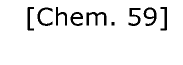

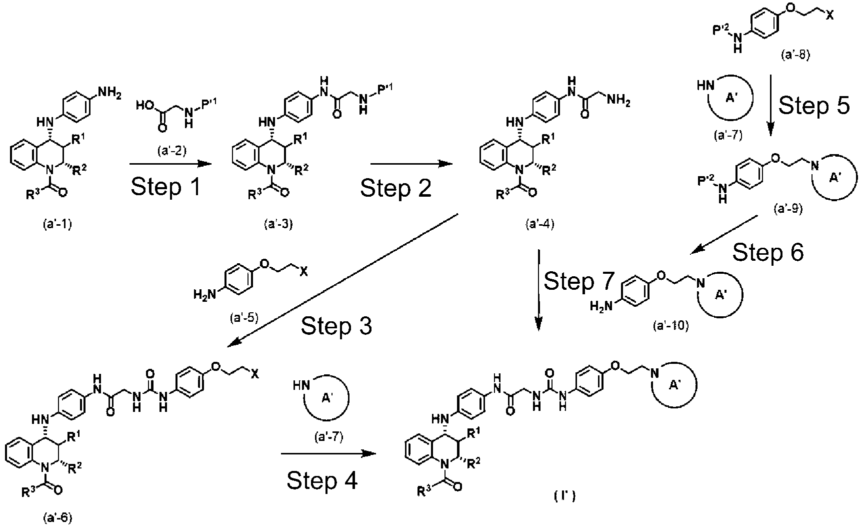

In the steps, R¹, R², R³, and ring A' are each as defined above, X represents a leaving group such as halogen, P'¹ and P'² are the same or different and each represents, for example, a protecting group for amine, such as 2-(trimethylsilyl)ethoxycarbonyl (Teoc), tert-butoxycarbonyl (Boc), 9-fluorenylmethyloxycarbonyl (Fmoc), or allyloxycarbonyl (Alloc).

### (Step 1)

A compound (a'-3) can be produced by reacting a compound (a'-1) and 1 equivalent to 5 equivalents of a compound (a'-2) in the presence of 1 equivalent to a large excess of a condensing agent, 1 equivalent to a large excess of a base, and optionally 0.01 equivalents to a large excess of an additive in a solvent at a temperature between -20°C and the boiling point of the solvent used for 5 minutes to 120 hours.

Examples of the condensing agent include dicyclohexylcarbodiimide (DCC), N-(3-dimethylaminopropyl)-N'-ethylcarbodiimide hydrochloride (EDC), O-(7-azabenzotriazol-1-yl)-N,N,N',N'-tetramethyluronium hexafluorophosphate (HATU), and (1-cyano-2-ethoxy-2-oxoethylideneaminooxy)dimethylamino-morpholino-carbenium hexafluorophosphate (COMU).

Examples of the base include triethylamine, and N,N-diisopropylethylamine.

Examples of the additive include 1-hydroxybenzotriazole monohydrate (HOBt), 1-hydroxy-7-azabenzotriazole (HOAt), and ethyl cyano(hydroxyimino)acetate.

Examples of the solvent include chloroform, dichloromethane, dimethylformamide (DMF), dimethylacetamide (DMA), tetrahydrofuran (THF), and acetonitrile, which can be used alone or as a mixture.

The compound (a'-1) can be obtained by, for example, a method described in Reference Example 7 of WO2021/065980.

The compound (a'-2) can be obtained as a commercially available product, or can be obtained by known methods [e.g., Experimental Chemistry Lecture, 5th Edition, Volume 14, p. 351, Maruzen Co., Ltd. (2004)] or methods based thereon.

### (Step 2)

A compound (a'-4) can be produced by reacting the compound (a'-3) in the presence of 1 equivalent to 10 equivalents of a fluoride ion in a solvent at a temperature between -20°C and the boiling point of the solvent used for 5 minutes to 120 hours, when P¹ in the compound (a'-3) is, for example, Teoc.

Examples of the fluoride ion include cesium fluoride and tetrabutylammonium fluoride (TBAF).

Examples of the solvent include THF, DMF, and N-methylpyrrolidone (NMP), which can be used alone or as a mixture.

In an alternative approach, the compound (a'-4) can be produced by reacting the compound (a'-3) in the presence of 1 equivalent to a large excess of an acid in a solvent at a temperature between -20°C and the boiling point of the solvent used for 5 minutes to 120 hours, when P¹ in the compound (a'-3) is, for example, Boc.

Examples of the acid include hydrogen chloride, sulfuric acid, trifluoroacetic acid, trifluoromethanesulfonic acid, and bis(trifluoromethanesulfonyl)imide.

Examples of the solvent include chloroform, dichloromethane, toluene, THF, and acetonitrile, which can be used alone or as a mixture.

In an alternative approach, the compound (a'-4) can be produced by reacting the compound (a'-3) in the presence of 1 equivalent to a large excess of a base in a solvent at a temperature between -20°C and the boiling point of the solvent used for 5 minutes to 120 hours, when P¹ in the compound (a'-3) is, for example, Fmoc.

Examples of the base include pyrrolidine, piperidine, and morpholine.

Examples of the solvent include methanol, ethanol, DMF, DMA, NMP, dimethyl sulfoxide (DMSO), THF, and acetonitrile, which can be used alone or as a mixture.

In an alternative approach, the compound (a'-4) can be produced by reacting the compound (a'-3) in the presence of 0.001 to 0.5 equivalents of a palladium catalyst and 1 equivalent to a large excess of an additive in a solvent at a temperature between - 20°C and the boiling point of the solvent used for 5 minutes to 120 hours, when P¹ in the compound (a'-3) is, for example, Alloc.

Examples of the palladium catalyst include tetrakis(triphenylphosphine)palladium (Pd(PPh₃)₄) and tris(dibenzylideneacetone)dipalladium (Pd₂(dba)₃).

Examples of the additive include piperidine, pyrrolidine, morpholine, N,N-dimethylbarbituric acid, and phenylsilane.

Examples of the solvent include THF, DMF, and NMP, which can be used alone or as a mixture.

### (Step 3)

A compound (a'-6) can be produced by reacting the compound (a'-4), 1 equivalent to 10 equivalents of a compound (a'-5), and 0.4 equivalents to 10 equivalents of a carbonyl reagent in the presence of 1 equivalent to a large excess of a base in a solvent at a temperature between -20°C and the boiling point of the solvent used for 5 minutes to 120 hours.

Examples of the carbonyl reagent include phosgene, triphosgene, and 1,1-carbonyldiimidazole (CDI).

Examples of the base include triethylamine, N,N-diisopropylethylamine, pyridine, and 4-dimethylaminopyridine.

Examples of the solvent include dichloromethane, chloroform, 1,2-dichloroethane, toluene, ethyl acetate, acetonitrile, diethyl ether, THF, DMF, NMP, and pyridine, which can be used alone or as a mixture.

The compound (a'-5) can be obtained as a commercially available product, or can be obtained by known methods [e.g., Experimental Chemistry Lecture, 5th Edition, Volume 15, p. 1, Maruzen Co., Ltd. (2004)] or methods based thereon.

### (Step 4)

The compound (I) can be produced by reacting the compound (a'-6) and 1 equivalent to 10 equivalents of a compound (a'-7) in the presence of 1 equivalent to a large excess of a base in a solvent at a temperature between -20°C and the boiling point of the solvent used for 5 minutes to 120 hours.

Examples of the base include sodium hydride, potassium hydride, lithium diisopropylamide (LDA), lithium bistrimethylsilylamide, sodium bistrimethylsilylamide, sodium methoxide, potassium ethoxide, potassium tert-butoxide, potassium carbonate, sodium hydroxide, triethylamine, N,N-diisopropylethylamine, pyridine, and 4-dimethylaminopyridine.

Examples of the solvent include DMF, DMA, NMP, DMSO, THF, and acetonitrile, which can be used alone or as a mixture.

The compound (a'-7) can be obtained as a commercially available product, or can be obtained by known methods [e.g., Experimental Chemistry Lecture, 5th Edition, Volume 14, p. 351, Maruzen Co., Ltd. (2004)] or methods based thereon.

### (Step 5)

A compound (a'-9) can be produced in the same manner as in step 4 of production method 1 using a compound (a'-8) and the compound (a'-7).

### (Step 6)

A compound (a'-10) can be produced in the same manner as in step 2 of production method 1 using the compound (a'-9).

### (Step 7)

The compound (I) can be produced in the same manner as in step 3 of production method 1 using the compound (a'-4) and 1 equivalent to 10 equivalents of the compound (a'-10).

### [Production method 2]

Next, a method for producing the drug-linker conjugate containing the compound represented by the formula (I) as a drug to be conjugated with a targeting molecule in the targeting molecule-drug conjugate will be described.

The linker contained in the drug-linker conjugate may have any structure as long as the structure has a function of connecting the targeting molecule and the drug. The linker may have a structure having a special function of, for example, being cleaved near or inside target cells or tissues, or a branched structure that can connect a plurality of drugs. In the targeting molecule-drug conjugate of the present invention, for example, a known linker (e.g., linkers described in S.J. Walsh et al., Chem. Soc. Rev. 2021, 50, 1305-1353; L.N. Tumey, (2020). Antibody-Drug Conjugates-Methods and Protocols: New York, Springer; and L. Ducry, (2013). Antibody-Drug Conjugate: New York, Springer) can be connected, for use, to the drug of the present invention (compound represented by the formula (I)) with reference to a known production method (e.g., M. Frigerio & A.F. Kyle, Curr. Top. Med. Chem. 2017, 17, 3393-3424). Specific examples thereof include a linker consisting of any one member selected from the group consisting of a peptide, an oligosaccharide, -(CH₂)-, an oxygen atom, a sulfur atom, -NH-, -(CH₂CH₂O)-, -CO-, -PO-, an amino acid, paraaminobenzyl (PAB), cyclic alkyl having 3 to 10 carbon atoms, and a structure represented by the following formula, and a linker containing a structure where two or more units selected from the above group are linked.

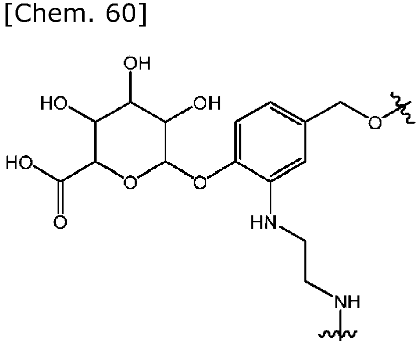

Examples of the amino acid constituting the linker include valine (Val), citrulline (Cit), phenylalanine (Phe), lysine (Lys), D-valine (D-Val), leucine (Leu), glycine (Gly), alanine (Ala), isoleucine (Ile), asparagine (Asn), glutamic acid (Glu), cysteic acid (Cya), arginine (Arg), glutamine (Gln), serine (Ser), proline (Pro), threonine (Thr), tyrosine (Tyr), and combinations thereof. Alternatively, the amino acid may be, for example, a peptide mimetic represented by the following formula (IV):

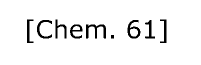

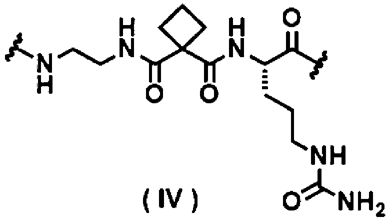

More specifically, a general method for producing, for example, a drug-linker conjugate represented by the formula (V) (e.g., the formula (Va)) as one form of the drug-linker conjugate will be described.

The drug-linker conjugate represented by the formula (Va) can be produced according to the following steps.

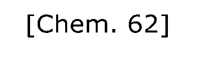

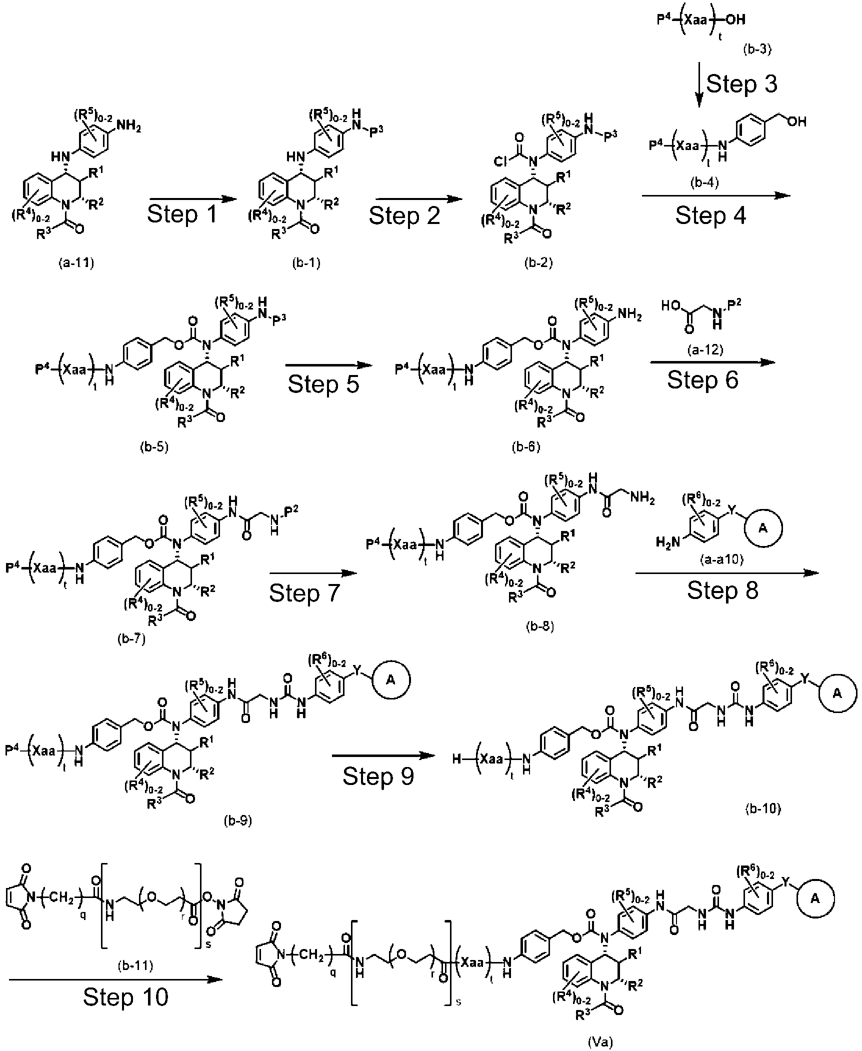

In the steps, R¹, R², R³, R⁴, R⁵, R⁶, Y, ring A, Xaa, q, r, s, t, and P² are each as defined above, and P³ and P⁴ are the same or different and each represents, for example, a protecting group for amine, such as 2-(trimethylsilyl)ethoxycarbonyl (Teoc), tert-butoxycarbonyl (Boc), 9-fluorenylmethyloxycarbonyl (Fmoc), or allyloxycarbonyl (Alloc). In one embodiment, examples of the drug-linker conjugate represented by the formula (Va) which is produced by the above steps and these steps include a drug-linker conjugate represented by the formula (Va) wherein R¹, R², R³, Xaa, q, r, s, t, and P² are each as defined above, P³ and P⁴ are the same or different and each represents, for example, a protecting group for amine, such as 2-(trimethylsilyl)ethoxycarbonyl (Teoc), tert-butoxycarbonyl (Boc), 9-fluorenylmethyloxycarbonyl (Fmoc), or allyloxycarbonyl (Alloc), R⁴, R⁵, and R⁶ are absent, Y is -O-CH₂-CH₂-, and ring A is ring A', and steps therefor.

### (Step 1)

A compound (b-1) can be produced by reacting a compound (a-11) in the presence of 1 equivalent to a large excess of a carbonyl reagent and optionally 1 equivalent to a large excess of a base in a solvent at a temperature between -20°C and the boiling point of the solvent used for 5 minutes to 120 hours.

Examples of the carbonyl reagent include N-(2-(trimethylsilyl)ethoxycarbonyloxy)succinimide (Teoc-OSu) and 4-(2-(trimethylsilyl)ethoxycarbonyloxy)nitrobenzene (Teoc-ONp), when P³ in the compound (b-1) is, for example, Teoc.

Examples of the carbonyl reagent include di-tert-butyl dicarbonate, when P³ in the compound (b-1) is, for example, Boc.

Examples of the carbonyl reagent include 9-fluorenylmethyl chloroformate (Fmoc-Cl) and N-((9H-fluoren-9-ylmethoxy)carbonyloxy)succinimide (Fmoc-OSu), when P³ in the compound (b-1) is, for example, Fmoc.

Examples of the carbonyl reagent include allyl chloroformate (Alloc-Cl), when P³ in the compound (b-1) is, for example, Alloc.

Examples of the base include triethylamine, N,N-diisopropylethylamine, pyridine, and 4-dimethylaminopyridine.

Examples of the solvent include dichloromethane, chloroform, 1,2-dichloroethane, toluene, ethyl acetate, acetonitrile, diethyl ether, THF, DMF, NMP, and pyridine, which can be used alone or as a mixture.

### (Step 2)

A compound (b-2) can be produced by reacting the compound (b-1) in the presence of 0.4 equivalents to 10 equivalents of triphosgene and 1 equivalent to a large excess of a base in a solvent at a temperature between 0°C and the boiling point of the solvent used for 5 minutes to 120 hours.

Examples of the base include triethylamine and N,N-diisopropylethylamine.

Examples of the solvent include THF and acetonitrile, which can be used alone or as a mixture.

### (Step 3)

A compound (b-4) can be produced in the same manner as in step 7 of production method 1-1 using a compound (b-3) and 1 equivalent to 10 equivalents of 4-hydroxymethylaniline.

The compound (b-3) can be obtained as a commercially available product, or can be obtained by known methods [e.g., Experimental Chemistry Lecture, 5th Edition, Volume 14, p. 351, Maruzen Co., Ltd. (2004)] or methods based thereon.

Necessary protection and deprotection steps may be involved depending on the type of a functional group contained in AA.

### (Step 4)

A compound (b-5) can be produced by reacting the compound (b-2) in the presence of 1 equivalent to 10 equivalents of a compound (b-4) and 1 equivalent to a large excess of a base in a solvent at a temperature between -20°C and the boiling point of the solvent used for 5 minutes to 120 hours.

Examples of the base include sodium hydride, potassium hydride, LDA, lithium bistrimethylsilylamide, sodium bistrimethylsilylamide, sodium methoxide, potassium ethoxide, and potassium tert-butoxide.

Examples of the solvent include chloroform, dichloromethane, DMF, DMA, NMP, DMSO, THF, and acetonitrile, which can be used alone or as a mixture.

### (Step 5)

A compound (b-6) can be produced in the same manner as in step 8 of production method 1-1 using the compound (b-5).

### (Step 6)

A compound (b-7) can be produced in the same manner as in step 7 of production method 1-1 using the compound (b-6) and the compound (a-12).

### (Step 7)

A compound (b-8) can be produced in the same manner as in step 8 of production method 1-1 using the compound (b-7).

### (Step 8)

A compound (b-9) can be produced in the same manner as in step 11 of production method 1-1 using the compound (b-8) and 1 equivalent to 10 equivalents of a compound (a-a10).

Among compounds (a-a10), a compound in which Y is -O-CH₂-CH₂- can be obtained as a compound (a-18).

Among compounds (a-a10), a compound in which Y is - CH=CH-CH₂- can be obtained as a compound (a-22).

Among compounds (a-a10), a compound in which Y is -NH-C(=O)-CH₂- can be obtained as a compound (a-26).

Among compounds (a-a10), a compound in which Y is -CH₂-CH₂-CH₂- can be obtained as a compound (a-a22).

### (Step 9)

A compound (b-10) can be produced in the same manner as in step 8 of production method 1-1 using the compound (b-9).

### (Step 10)

The compound (Va) can be produced by reacting the compound (b-10) in the presence of 1 equivalent to 10 equivalents of a compound (b-11) and optionally 1 equivalent to a large excess of a base in a solvent at a temperature between -20°C and the boiling point of the solvent used for 5 minutes to 120 hours.

Examples of the base include triethylamine, N,N-diisopropylethylamine, pyridine, and 4-dimethylaminopyridine.

Examples of the solvent include dichloromethane, chloroform, 1,2-dichloroethane, toluene, ethyl acetate, acetonitrile, diethyl ether, THF, DMF, NMP, and pyridine, which can be used alone or as a mixture.

The compound (b-11) can be obtained as a commercially available product, or can be obtained by known methods [e.g., Experimental Chemistry Lecture, 5th Edition, Volume 16, p. 258, Maruzen Co., Ltd. (2004)] or methods based thereon.

### [Production method 3]

Next, a method for producing the targeting molecule-drug conjugate of the present invention by conjugating the drug-linker conjugate obtained in production method 2 with the targeting molecule will be described.

Although the following detailed description about the conjugation of the targeting molecule in the present specification is directed mainly to the case where the targeting molecule is, but not limited to, an antibody, a small-molecule compound, or a peptide molecule for convenience and briefness, those skilled in the art can understand that the targeting molecule other than those listed herein can be conjugated by changing or modifying points to be changed or modified.

When the targeting molecule is an antibody, the targeting molecule-drug conjugate can be produced by conjugating the antibody and the drug-linker conjugate according to known methods (e.g., methods described in L.N. Tumey, (2020). Antibody-Drug Conjugates-Methods and Protocols: New York, Springer; and L. Ducry, (2013). Antibody-Drug Conjugate: New York, Springer).

Examples thereof include a method of reacting a functional group, such as an α,β-unsaturated carbonyl group, an α,β-unsaturated sulfinyl group, an α,β-unsaturated sulfonyl group, a sulfhydryl group, a hydroxy group, an amino group, an amide group, a formyl group, a carboxyl group, an azide group, an alkynyl group, an alkenyl group, a haloalkyl group, or a carbonyl group, introduced in the antibody molecule (including a glycan linked to the antibody) with a functional group contained in the drug-linker conjugate under proper conditions. The combination of the functional group contained in the antibody and the functional group contained in the drug-linker conjugate can be properly selected on the basis of common knowledge. For example, a bond can also be formed through the nucleophilic reaction of a nucleophilic functional group such as a sulfhydryl group in the antibody molecule with a Michael acceptor such as an α,β-unsaturated carbonyl group contained in the drug-linker conjugate. For example, a bond can also be formed through the cyclization reaction of an azide group in the antibody molecule with an alkynyl group contained in the drug-linker conjugate in the presence or absence of a catalyst.

More specifically, examples of one form of the antibody-drug conjugate include an antibody-drug conjugate represented by the formula (VI).

The antibody-drug conjugate represented by the formula (VI) can be produced according to the following steps.

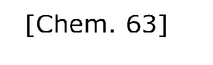

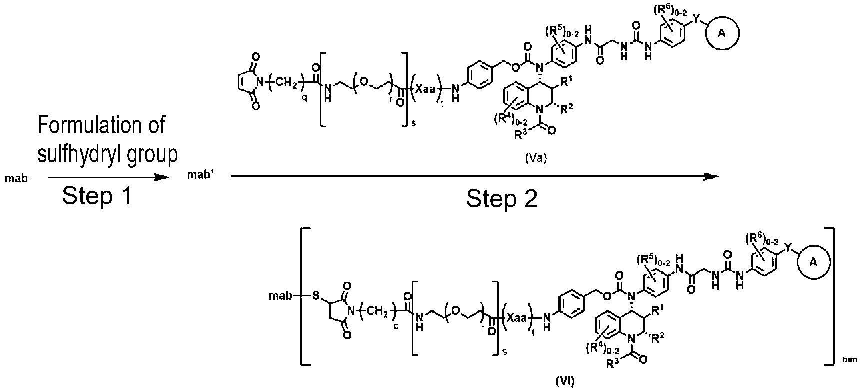

In the steps, R¹, R², R³, R⁴, R⁵, R⁶, Y, ring A, Xaa, q, r, s, and t are each as defined above, mab represents an antibody, mab' represents an antibody having a sulfhydryl group, and mm represents 1 to 8. In one embodiment, examples of the antibody-drug conjugate represented by the formula (VI) which is produced by the above steps and these steps include an antibody-drug conjugate represented by the formula (VI) wherein R¹, R², R³, Xaa, q, r, s, and t are each as defined above, mab represents an antibody, mab' represents an antibody having a sulfhydryl group, mm represents 1 to 8, R⁴, R⁵, and R⁶ are absent, Y is -O-CH₂-CH₂-, and ring A is ring A', and steps therefor.

### (Step 1) Formation of sulfhydryl group

mab' can be obtained by methods described in, for example, L.N. Tumey, (2020). Antibody-Drug Conjugates-Methods and Protocols: New York, Springer; and L. Ducry, (2013). Antibody-Drug Conjugate: New York, Springer using mab.

Specifically, mab' can be produced, for example, by reacting mab in the presence of 1 equivalent to a large excess of a reducing agent in a solvent at a temperature between 0°C and the boiling point of the solvent used for 5 minutes to 24 hours.

Examples of the reducing agent include dithiothreitol, 2-mercaptoethanol, and tris(2-carboxyethyl)phosphine hydrochloride (TCEP).

Examples of the solvent include tris(hydroxymethyl)aminomethane hydrochloride buffer solutions (Tris-HCl), phosphate-buffered saline (PBS), N-(2-hydroxyethyl)piperazine-N'-2-ethanesulfonic acid buffer solutions (HEPES), acetate-based buffer solutions, borate-based buffer solutions, phosphate-based buffer solutions, carbonate-based buffer solutions, and citrate-based buffer solutions. pH at the time of reaction is usually 2 to 12.

In an alternative aspect, mab' can be produced by reacting mab having a cysteine mutation at a freely-selected position in the presence of 10 equivalents to a large excess of a reducing agent in a solvent at a temperature between 0°C and the boiling point of the solvent used for 5 minutes to 24 hours, then removing, if necessary, the reducing agent, and reacting the resultant in the presence of 10 equivalents to a large excess of a oxidizing agent and optionally an additive in a solvent at a temperature between 0°C and the boiling point of the solvent used for 5 minutes to 24 hours.

Examples of the reducing agent include dithiothreitol, 2-mercaptoethanol, and TCEP.

Examples of the oxidizing agent include dehydroascorbic acid (DHAA).

Examples of the additive include ethylenediaminetetraacetic acid.

Examples of the solvent include Tris-HCl, PBS, HEPES, and acetate-based, borate-based, phosphate-based, carbonate-based, and citrate-based buffer solutions. pH at the time of reaction is usually 2 to 12.

### (Step 2)

The antibody-drug conjugate (VI) can be produced by reacting mab' obtained in step 1 in a solvent in the presence of 1 equivalent to a large excess of the compound (Va) obtained in step 10 of production method 2 and an additive optionally used at a volume ratio of 1% to 50% to the solvent at a temperature between 0°C and the boiling point of the solvent used for 5 minutes to 24 hours.

Examples of the additive include DMF, DMA, DMSO, propylene glycol, and acetonitrile.

Examples of the solvent include Tris-HCl, PBS, HEPES, and acetate-based, borate-based, phosphate-based, carbonate-based, and citrate-based buffer solutions. pH at the time of reaction is usually 2 to 12.

Those skilled in the art can understand that the conditions and the method described above are given for illustrative purposes and do not limit the present invention and other methods for conjugation are also known in the art and can be used in the present invention.

Operation, such as preparation, purification, or analysis, of the antibody-drug conjugate (ADC) described above may be performed by use of known methods described in, for example, Antibody-Drug Conjugates (Laurent Ducry ed., 2013, Humana Press) or Antibody-Drug Conjugates Methods and Protocols (L. Nathan Tumey ed., 2020, Humana Press).

When the targeting molecule is a small-molecule compound or a peptide molecule, the targeting molecule-drug conjugate can be produced by conjugating the small-molecule compound or the peptide molecule and the drug-linker conjugate through chemical reaction commonly used in organic chemical synthesis (e.g., methods described in Kelly Bugatti. ChemBioChem. 2023, 24, e202300254).

Examples thereof include a method of reacting a functional group, such as an α,β-unsaturated carbonyl group, an α,β-unsaturated sulfinyl group, an α,β-unsaturated sulfonyl group, a sulfhydryl group, a hydroxy group, an amino group, an amide group, a formyl group, a carboxyl group, an azide group, an alkynyl group, an alkenyl group, a haloalkyl group, or a carbonyl group, contained in the small-molecule compound or the peptide molecule with a functional group contained in the drug-linker conjugate under proper conditions. The combination of the functional group contained in the small-molecule compound or the peptide molecule and the functional group contained in the drug-linker conjugate can be properly selected on the basis of common knowledge. For example, a bond can also be formed through the nucleophilic reaction of a nucleophilic functional group such as a sulfhydryl group in the small-molecule compound or the peptide molecule with a Michael acceptor such as an α,β-unsaturated carbonyl group contained in the drug-linker conjugate. For example, a bond can also be formed through the condensation reaction of a carboxyl group in the small-molecule compound or the peptide molecule with an amino group contained in the drug-linker conjugate in the presence of a condensing agent.

More specifically, examples of one form of the small-molecule compound-drug conjugate or the peptide molecule-drug conjugate include a small-molecule compound-drug conjugate or a peptide molecule-drug conjugate represented by the formula (VIIa).

The small-molecule compound-drug conjugate or the peptide molecule-drug conjugate represented by the formula (VIIa) can be produced according to the following steps.

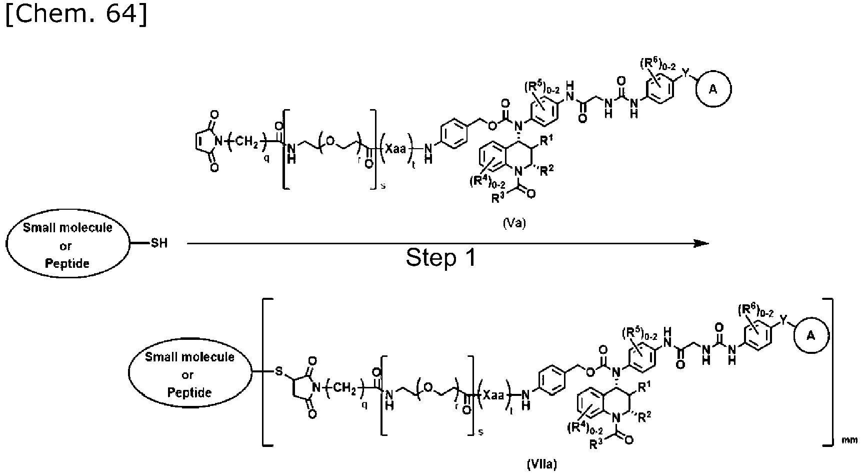

In the steps, R¹, R², R³, R⁴, R⁵, R⁶, Y, ring A, Xaa, q, r, s, and t are each as defined above, Small molecule represents a small-molecule compound, Peptide represents a peptide molecule, and mm represents 1 to 8.

The small-molecule compound-drug conjugate or the peptide molecule-drug conjugate represented by the formula (VIIb) can be produced according to the following steps.

### (Step 1)

The small-molecule compound-drug conjugate or the peptide molecule-drug conjugate (VIIa) can be produced by reacting a small-molecule compound or a peptide molecule in the presence of 1 equivalent to a large excess of the compound (Va) obtained in step 10 of production method 2 and optionally 1 equivalent to a large excess of a base in a solvent at a temperature between 0°C and the boiling point of the solvent used for 5 minutes to 24 hours.

Examples of the base include triethylamine, N,N-diisopropylethylamine, pyridine, and 4-dimethylaminopyridine.

Examples of the solvent include methanol, ethanol, DMF, DMA, NMP, DMSO, THF, and acetonitrile.

The small-molecule compound can be obtained as a commercially available product, or can be obtained by known methods [e.g., Experimental Chemistry Lecture, 5th Edition, Volume 16, p. 1, Maruzen Co., Ltd. (2005)] or methods based thereon.

The peptide molecule can be obtained as a commercially available product, or can be obtained by known methods [e.g., Experimental Chemistry Lecture, 5th Edition, Volume 16, p. 257, Maruzen Co., Ltd. (2005)] or methods based thereon.

A small-molecule compound-drug conjugate or a peptide molecule-drug conjugate represented by the formula (VIIb) can be produced according to the following steps.

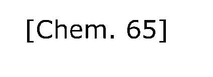

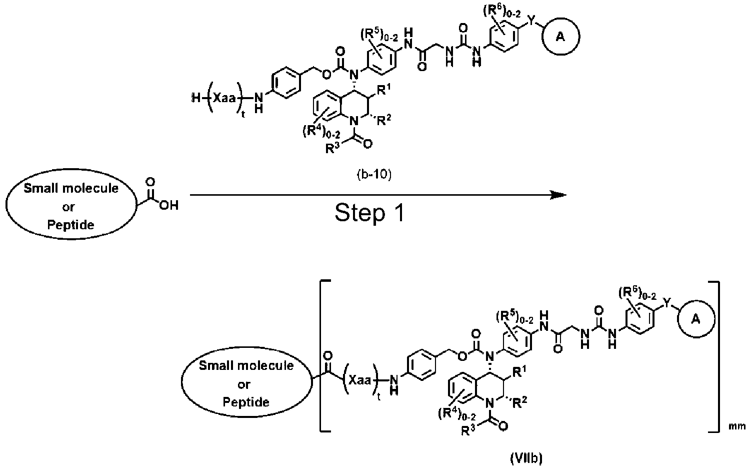

In the steps, R¹, R², R³, R⁴, R⁵, R⁶, Y, ring A, Xaa, and t are each as defined above, Small molecule represents a small-molecule compound, Peptide represents a peptide molecule, and mm represents 1 to 8.

The small-molecule compound-drug conjugate or the peptide molecule-drug conjugate represented by the formula (VIIb) can be produced according to the following steps.

### (Step 1)

The small-molecule compound-drug conjugate or the peptide molecule-drug conjugate (VIIb) can be produced in the same manner as in step 7 of production method 1-1 using a small-molecule compound or a peptide molecule and 1 equivalent to 5 equivalents of the compound (b-10).

The small-molecule compound can be obtained as a commercially available product, or can be obtained by known methods [e.g., Experimental Chemistry Lecture, 5th Edition, Volume 16, p. 1, Maruzen Co., Ltd. (2005)] or methods based thereon.

The peptide molecule can be obtained as a commercially available product, or can be obtained by known methods [e.g., Experimental Chemistry Lecture, 5th Edition, Volume 16, p. 257, Maruzen Co., Ltd. (2005)] or methods based thereon.

### <<Preparation of gene recombinant antibody>>

Hereinafter, a method for preparing a humanized antibody will be described as a preparation example of a gene recombinant antibody. For example, a gene recombinant mouse antibody, rat antibody, and rabbit antibody can also be prepared by similar methods.

### (1) Construction of vector for gene recombinant antibody expression

A vector for gene recombinant antibody expression is an expression vector for an animal cell with incorporated DNAs encoding human antibody CH and CL, and can be constructed by respectively cloning DNAs encoding human antibody CH and CL into expression vectors for animal cells.

CH and CL of a freely-selected human antibody can be used as human antibody C regions. For example, human antibody CH of a γ1 subclass and CL of a κ class can be used. cDNAs can be used as the DNAs encoding human antibody CH and CL, or chromosomal DNAs made up of exons and introns may be used. A codon encoding an amino acid residue is added, inserted, or substituted into the DNAs encoding human antibody CH or CL so that the amino acid residue can be added, inserted, or substituted at a corresponding position. Any expression vector for an animal cell can be used as long as a gene encoding a human antibody C region can be incorporated and expressed. For example, pAGE107 [Cytotechnol., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981)], pSG1bd2-4 [Cytotechnol., 4, 173 (1990)], pSE1UK1Sed1-3 [Cytotechnol., 13, 79 (1993)], or pCI (Promega Corp.) can be used. Examples of the promoter and the enhancer in the expression vector for an animal cell include SV40 early promoter [J. Biochem., 101, 1307 (1987)], Moloney murine leukemia virus LTR [Biochem. Biophys. Res. Commun., 149, 960 (1987)], and immunoglobulin H chain promoter [Cell, 41, 479 (1985)] and enhancer [Cell, 33, 717 (1983)].

The vector for gene recombinant antibody expression can be used as a vector for gene recombinant antibody expression of type in which the antibody H chain and L chain are encoded on one vector (tandem type) [J. Immunol. Methods, 167, 271 (1994)]or type in which the antibody H chain and L chain are encoded on separate vectors. For example, pKANTEX93 (WO 97/10354) or pEE18 [Hybridoma, 17, 559 (1998)] can be used as the vector for gene recombinant antibody expression of tandem type. For example, pCI-hK and pCI-hG1, or pCI-hG4 (vectors obtained by inserting human L chain (κ) C region and human H chain (G1 or G4) C region DNAs, respectively, to pCI vectors of Promega Corp.) can be used as the vectors for gene recombinant antibody expressions of type in which the antibody H chain and L chain are encoded on separate vectors.

### (2) Construction of humanized antibody expression vector

cDNA encoding VH or VL of the gene recombinant antibody of interest can be cloned upstream of each gene encoding human antibody CH or CL in the vector for gene recombinant antibody expression obtained in (1) to construct a humanized antibody expression vector.

Any cell can be used as a host cell to which the gene recombinant antibody expression vector is to be transferred as long as the host cell can be allowed to express the gene recombinant antibody. For example, Expi293 cells (Thermo Fisher Scientific Inc.), Expi293F cells (Thermo Fisher Scientific Inc.), CHO-K1 (ATCC CCL-61), DUKXB11 (ATCC CCL-9096), Pro-5 (ATCC CCL-1781), CHO-S (Life Technologies, Cat# 11619), rat myeloma cells YB2/3HL.P2.G11.16Ag.20 (ATCC No: CRL1662, or also called YB2/0), mouse myeloma cells NS0, mouse myeloma cells SP2/0-Ag14 (ATCC No: CRL1581), mouse P3X63-Ag8.653 cells (ATCC No: CRL1580), or dihydrofolate reductase gene (Dihydrofolate Reductase ; hereinafter, also referred to as dhfr)-deficient CHO cells (CHO/DG44 cells) [Proc. Natl. Acad. Sci. USA, 77, 4216 (1980)] can be used.

The expression vector can be transferred to the host cell by use of lipofection [Proc. Natl. Acad. Sci. USA, 84, 7413 (1987)] or the like.

Alternatively, host cells with reduced or deleted activity of a protein such as an enzyme involved in the synthesis of an intracellular glyconucleotide GDP-fucose, a protein such as an enzyme involved in the glycan modification of an α bond of position 1 of fucose to position 6 of N-acetylglucosamine at the reducing end of an N-glycoside-linked complex glycan, or a protein involved in the transport of an intracellular glyconucleotide GDP-fucose to the Golgi body, for example, α1,6-fucosyltransferase gene-deficient CHO cells (WO 2005/035586 and WO 02/31140) or Lec13 with acquired lectin resistance [Somatic Cell and Molecular genetics, 12, 55 (1986)], may be used.

For example, RPMI1640 medium (manufactured by Thermo Fisher Scientific Inc.), GIT medium (manufactured by Nihon Pharmaceutical Co., Ltd.), EX-CELL301 medium (manufactured by SAFC Biosciences), IMDM medium (manufactured by Thermo Fisher Scientific Inc.), or Hybridoma SFM medium (manufactured by Thermo Fisher Scientific Inc.), or any of these media supplemented with various additives such as FBS is used as a medium for animal cell culture. The obtained transformant is cultured in the medium and thereby allowed to express and accumulate the gene recombinant antibody in a culture supernatant. The expression level and antigen binding activity of the gene recombinant antibody in the culture supernatant can be measured by ELISA or the like. The expression level of the gene recombinant antibody produced by the transformant can be improved by exploiting a dhfr gene amplification system (Japanese Unexamined Patent Application Publication No. H2-257891) or the like.

The gene recombinant antibody can be purified from the culture supernatant of the transformant using a protein A column [Monoclonal Antibodies-Principles and practice, Third edition, Academic Press (1996), Antibodies-A Laboratory Manual, Cold Spring Harbor Laboratory (1988)]. Methods for use in protein purification, such as gel filtration, ion exchange chromatography, and ultrafiltration may be combined.

The molecule weight of the H chain or the L chain of the purified gene recombinant antibody, or the whole antibody molecule can be measured by use of, for example, polyacrylamide gel electrophoresis [Nature, 227, 680 (1970)] or Western blotting [Monoclonal Antibodies-Principles and practice, Third edition, Academic Press (1996), Antibodies-A Laboratory Manual, Cold Spring Harbor Laboratory (1988)].

### (3) Activity evaluation of purified monoclonal antibody or antigen binding fragment of the antibody

The purified monoclonal antibody of the present invention or an antigen binding fragment of the antibody can be evaluated for its activity as follows.

The binding activity of the purified antibody against the antigen can be measured by reacting the antibody with antigen-positive cells, thoroughly washing the cells thus reacted with PBS containing 1 to 10% bovine serum albumin (BSA) (hereinafter, referred to as BSA-PBS) or the like, and then reacting the cells with an anti-immunoglobulin antibody labeled with a fluorescent reagent or the like as a secondary antibody, followed by use of flow cytometry. Alternatively, a fluorescent antibody technique [Cancer Immunol. Immunother., 36, 373 (1993)] or the like may be used in the measurement. Any cell may be used as long as the cell expresses the antigen of interest. Examples thereof include antigen-positive human cells, human cell lines, and cell lines forced to express the antigen.

### [Examples]

The proton nuclear magnetic resonance spectra (¹H NMR) used in the following reference examples and examples were measured at 300 MHz or 400 MHz, and exchangeable protons could not be clearly observed depending on the compound and the measurement conditions. Note that the multiplicity of a signal was indicated with common term, while br indicated that the signal appears broad. In addition, ChemDraw Professional version 20.1.1 was used as necessary for naming each compound synthesized.

### [Example 1] 2-(3-(4-(2-((R)-2,4-Dimethyl-3-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 1)

### (Step 1)

1-((2S,4R)-4-((4-Aminophenyl)amino)-2-methyl-3,4-dihydroquinolin-1(2H)-yl)propan-1-one (3.0 g, 9.7 mmol) synthesized according to the method described in step 3 of Reference Example 7 of WO2021/065980 was dissolved in DMF (50 mL), then HATU (4.8 g, 13 mmol), (((9H-fluoren-9-yl)methoxy)carbonyl)glycine (3.5 g, 12 mmol), and N,N-diisopropylethylamine (3.8 g, 29 mmol) were added to the solution, and the mixture was stirred at room temperature for 3 hours. The reaction mixture was subjected to extraction with ethyl acetate. The organic layer was dried over anhydrous sodium sulfate and concentrated under reduced pressure to give a crude product of (9H-fluoren-9-yl)methyl (2-((4-(((2S,4R)-2-methyl-1-propionyl-1,2,3,4-tetrahydroquinolin-4-yl)amino)phenyl)amino)-2-oxoethyl)carbamate (6.5 g).

ESIMS, (M+H)⁺, m/z: 589.

### (Step 2)

The crude product of (9H-fluoren-9-yl)methyl (2-((4-(((2S,4R)-2-methyl-1-propionyl-1,2,3,4-tetrahydroquinolin-4-yl)amino)phenyl)amino)-2-oxoethyl) carbamate (6.5 g) obtained in step 1 was dissolved in DMF (65 mL), then piperidine (4.7 g, 55 mmol) was added to the solution, and the mixture was stirred overnight at room temperature. The reaction mixture was purified by reverse-phase HPLC (0.05% solution of trifluoroacetic acid (TFA) in acetonitrile/0.05% aqueous TFA solution = 5/95 to 100/0) to give 2-amino-N-(4-(((2S,4R)-2-methyl-1-propionyl-1,2,3,4-tetrahydroquinolin-4-yl)amino)phenyl)acetamide (2.8 g, total yield of two steps: 69%).

ESIMS, (M+H)⁺, m/z: 367.

### (Step 3)

(R)-4-(2-(4-Aminophenoxy)ethyl)-1,3-dimethylpiperazin-2-one (160 mg, 0.60 mmol) synthesized according to the method described in step 3 of Reference Example 40 of WO2021/065980 was dissolved in acetonitrile (10 mL), then triphosgene (89 mg, 0.30 mmol) was added to the solution at 0°C, and the mixture was stirred at 0°C for 2 hours. 2-Amino-N-(4-(((2S,4R)-2-methyl-1-propionyl-1,2,3,4-tetrahydroquinolin-4-yl)amino)phenyl)acetamide (220 mg, 0.60 mmol) obtained in step 2 and triethylamine (300 mg, 3.0 mmol) were added to the reaction mixture at 0°C, and the mixture was stirred at room temperature for 4 hours. The reaction mixture was concentrated under reduced pressure, and the obtained residue was purified by silica gel column chromatography (dichloromethane/methanol = 10/1) to give compound 1 (100 mg, yield: 26%).

ESIMS, (M+H)⁺, m/z: 656. ¹H NMR (DMSO-d₆, δ): 9.66 (s, 1H), 8.60 (s, 1H), 7.35-7.20 (m, 6H), 7.17 (s, 2H), 6.82 (d, J = 8.8 Hz, 2H), 6.61 (d, J = 8.8 Hz, 2H), 6.29 (t, J = 5.6 Hz, 1H), 5.91 (d, J = 7.8 Hz, 1H), 4.80-4.65 (m, 1H), 4.15-4.05 (m, 1H), 4.01 (t, J = 8.0 Hz, 2H), 3.86 (d, J = 5.6 Hz, 2H), 3.30-3.20 (m, 2H), 3.20-3.05 (m, 2H), 2.98-2.90 (m, 1H), 2.81 (s, 3H), 2.79-2.63 (m, 2H), 2.63-2.54 (m, 2H), 2.30-2.10 (m, 1H), 1.23 (d, J = 6.8 Hz, 3H), 1.23-1.10 (m, 1H), 1.07-0.97 (m, 6H).

### [Example 2] 2-(3-(4-(2-(4-Acetylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 2)

### (Step 1)

tert-Butyl (4-(2-(4-acetylpiperazin-1-yl)ethoxy)phenyl)carbamate (660 mg, yield: quantitative) was obtained in the same manner as in step 2 of Example 5 using commercially available tert-butyl (4-(2-chloroethoxy)phenyl)carbamate (300 mg, 1.10 mmol) and commercially available 1-(piperazin-1-yl)ethan-1-one (170 mg, 1.33 mmol).

ESIMS, (M+H)⁺, m/z: 364.

### (Step 2)

tert-Butyl (4-(2-(4-acetylpiperazin-1-yl)ethoxy)phenyl)carbamate (400 mg, 1.1 mmol) obtained in step 1 was dissolved in dichloromethane (1.7 mL), then TFA (1.7 mL) was added to the solution, and the mixture was stirred overnight at room temperature. The reaction mixture was concentrated under reduced pressure to give a crude product of 1-(4-(2-(4-aminophenoxy)ethyl)piperazin-1-yl)ethan-1-one TFA salt (290 mg).

ESIMS, (M+H)⁺, m/z: 264.

### (Step 3)

The crude product of 1-(4-(2-(4-aminophenoxy)ethyl)piperazin-1-yl)ethan-1-one TFA salt (55 mg) obtained in step 2 was dissolved in DMF (2 mL), then triethylamine (0.20 mL, 1.5 mmol) and CDI (41 mg, 0.25 mmol) were added to the solution, and the mixture was stirred at room temperature for 1 hour. 2-Amino-N-(4-(((2S,4R)-2-methyl-1-propionyl-1,2,3,4-tetrahydroquinolin-4-yl)amino)phenyl)acetamide hydrochloride (100 mg, 0.23 mmol) synthesized according to the method described in Example 5i of WO2021/065980 and 4-dimethylaminopyridine (DMAP) (2.6 mg, 0.02 mmol) were added to the reaction mixture, and the mixture was stirred overnight at room temperature. The reaction mixture was diluted with water, followed by extraction with chloroform. The organic layer was concentrated under reduced pressure, and the obtained residue was purified by reverse-phase HPLC (acetonitrile/10 mM aqueous ammonium bicarbonate solution = 5/95 to 90/10) to give compound 2 (45 mg, total yield of two steps: 30%). ESIMS, (M+H)⁺, m/z: 656. ¹H NMR (CDCl₃, δ): 8.65 (s, 1H), 7.40 (s, 1H), 7.33-7.21 (m, 4H), 7.15 (t, J = 7.9 Hz, 4H), 6.76 (d, J = 8.6 Hz, 2H), 6.52 (d, J = 8.6 Hz, 2H), 6.30 (s, 1H), 4.90 (s, 1H), 4.05-4.04 (m, 5H), 3.92 (d, J = 7.2 Hz, 1H), 3.66-3.63 (m, 2H), 3.50 (s, 2H), 2.83 (d, J = 5.0 Hz, 2H), 2.61-2.56 (m, 6H), 2.37-2.33 (m, 1H), 2.09 (s, 3H), 1.28-1.23 (m, 3H), 1.14-1.13 (m, 3H), 0.87-0.84 (m, 1H).

### [Example 3] 2-(3-(4-(2-((3R,5S)-4-Acetyl-3,5-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 3)

### (Step 1)

tert-Butyl (4-(2-((3R,5S)-4-acetyl-3,5-dimethylpiperazin-1-yl)ethoxy)phenyl)carbamate (550 mg, yield: quantitative) was obtained in the same manner as in step 2 of Example 5 using commercially available tert-butyl (4-(2-chloroethoxy)phenyl)carbamate (300 mg, 1.1 mmol) and commercially available 1-((2S,6R)-2,6-dimethylpiperazin-1-yl)ethan-1-one (210 mg, 1.3 mmol).

ESIMS, (M+H)⁺, m/z: 392.

### (Step 2)

A crude product of1-((2R,6S)-4-(2-(4-aminophenoxy)ethyl)-2,6-dimethylpiperazin-1-yl)ethan-1-one trifluoroacetate (500 mg) was obtained in the same manner as in step 2 of Example 2 using tert-butyl (4-(2-((3R,5S)-4-acetyl-3,5-dimethylpiperazin-1-yl)ethoxy)phenyl)carbamate (430 mg, 1.1 mmol) obtained in step 1.

ESIMS, (M+H)⁺, m/z: 292.

### (Step 3)

Compound 3 (48 mg, total yield of two steps: 35%) was obtained in the same manner as in step 3 of Example 2 using the crude product of 1-((2R,6S)-4-(2-(4-aminophenoxy)ethyl)-2,6-dimethylpiperazin-1-yl)ethan-1-one TFA salt (95 mg) obtained in step 2 and 2-amino-N-(4-(((2S,4R)-2-methyl-1-propionyl-1,2,3,4-tetrahydroquinolin-4-yl)amino)phenyl)acetamide hydrochloride (95 mg, 0.22 mmol) synthesized according to the method described in Example 5i of WO2021/065980.

ESIMS, (M+H)⁺, m/z: 684. ¹H NMR (CDCl₃, δ):8.66 (br s, 1H), 7.42 (br s, 1H), 7.28-7.25 (m, 4H), 7.16-7.13 (m, 4H), 6.78 (d, J = 9.1 Hz, 2H), 6.52 (t, J = 8.6 Hz, 2H), 6.35 (br s, 1H), 4.89 (s, 1H), 4.07 (t, J = 6.6 Hz, 5H), 3.90 (d, J = 7.2 Hz, 1H), 2.78-2.75 (m, 3H), 2.62-2.55 (m, 2H), 2.40-2.21 (m, 2H), 2.12-2.08 (m, 2H), 1.44-1.21 (m, 8H), 1.16-1.13 (m, 9H), 0.87-0.85 (m, 1H).

### [Example 4] 2-(3-(4-(2-((2S,6R)-4-Acetyl-2,6-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 4)

### (Step 1)

tert-Butyl (4-(2-((2S,6R)-4-acetyl-2,6-dimethylpiperazin-1-yl)ethoxy)phenyl)carbamate(550 mg, yield: quantitative) was obtained in the same manner as in step 2 of Example 5 using commercially available tert-butyl (4-(2-chloroethoxy)phenyl)carbamate (300 mg, 1.1 mmol) and commercially available 1-((3R,5S)-3,5-dimethylpiperazin-1-yl)ethan-1-one hydrochloride (320 mg, 1.7 mmol).

ESIMS, (M+H)⁺, m/z: 392.

### (Step 2)

A crude product of 1-((3S,5R)-4-(2-(4-aminophenoxy)ethyl)-3,5-dimethylpiperazin-1-yl)ethan-1-one TFA salt (240 mg) was obtained in the same manner as in step 2 of Example 2 using tert-butyl (4-(2-((2S,6R)-4-acetyl-2,6-dimethylpiperazin-1-yl)ethoxy)phenyl)carbamate (240 mg, 0.62 mmol) obtained in step 1.

ESIMS, (M+H)⁺, m/z: 292.

### (Step 3)

Compound 4 (18 mg, total yield of two steps: 13%) was obtained in the same manner as in step 3 of Example 2 using the crude product of 1-((3S,5R)-4-(2-(4-aminophenoxy)ethyl)-3,5-dimethylpiperazin-1-yl)ethan-1-one TFA salt (58 mg) obtained in step 2 and 2-amino-N-(4-(((2S,4R)-2-methyl-1-propionyl-1,2,3,4-tetrahydroquinolin-4-yl)amino)phenyl)acetamide hydrochloride (96 mg, 0.22 mmol) synthesized according to the method described in Example 5i of WO2021/065980.

ESIMS, (M+H)⁺, m/z: 684. ¹H NMR (CDCl₃, δ): 8.52 (br s, 1H), 7.30-7.23 (m, 4H), 7.16 (t, J = 8.4 Hz, 5H), 6.77 (d, J = 8.6 Hz, 2H), 6.54 (d, J = 8.6 Hz, 2H), 6.14 (br s, 1H), 4.90 (br s, 1H), 4.41-4.35 (m, 1H), 4.11-4.08 (m, 1H), 4.04-4.02 (m, 2H), 3.93-3.89 (m, 3H), 3.62-3.58 (m, 1H), 3.13-3.11 (m, 2H), 2.92-2.90 (m, 1H), 2.65-2.52 (m, 3H), 2.37-2.33 (m, 1H), 2.06 (s, 3H), 1.42-1.02 (m, 13H), 0.95-0.73 (m, 2H).

### [Example 5] N-(4-(((2S,4R)-2-Methyl-1-propionyl-1,2,3,4-tetrahydroquinolin-4-yl)amino)phenyl)-2-(3-(4-(2-(4-methyl-5-oxo-1,4-diazepan-1-yl)ethoxy)phenyl)ureido)acetamide (compound 5)

### (Step 1)

2-(3-(4-(2-Bromoethoxy)phenyl)ureido)-N-(4-(((2S,4R)-2-methyl-1-propionyl-1,2,3,4-tetrahydroquinolin-4-yl)amino)phenyl)acetamide (50 mg, yield: 18%) was obtained in the same manner as in step 3 of Example 1 using commercially available 4-(2-bromoethoxy)aniline (100 mg, 0.46 mmol), and 2-amino-N-(4-(((2S,4R)-2-methyl-1-propionyl-1,2,3,4-tetrahydroquinolin-4-yl)amino)phenyl)acetamide (170 mg, 0.46 mmol) obtained in step 2 of Example 1.

ESIMS, (M+H)⁺, m/z: 609.

### (Step 2)

2-(3-(4-(2-Bromoethoxy)phenyl)ureido)-N-(4-(((2S,4R)-2-methyl-1-propionyl-1,2,3,4-tetrahydroquinolin-4-yl)amino)phenyl)acetamide (45 mg, 0.07 mmol) obtained in step 1, and commercially available 4-methyl-1,4-diazepan-5-one (9 mg, 0.07 mmol) were dissolved in DMA(2 mL), then potassium iodide (12 mg, 0.07 mmol) and N,N-diisopropylethylamine (19 mg, 0.14 mmol) were added to the solution, and the mixture was stirred overnight at 100°C. The reaction mixture was purified by reverse-phase HPLC (acetonitrile/5 mM aqueous ammonium bicarbonate solution = 10/90 to 95/5) to give compound 5 (22 mg, yield: 45%).

ESIMS, (M+H)⁺, m/z: 656. ¹H NMR (DMSO-d₆, δ): 9.67 (s, 1H), 8.60 (s, 1H), 7.34-7.22 (m, 6H), 7.21-7.14 (m, 2H), 6.83 (d, J = 8.8 Hz, 2H), 6.62 (d, J = 8.8 Hz, 2H), 6.29 (t, J = 5.6 Hz, 1H), 5.92 (d, J = 8.0 Hz, 1H), 4.85-4.70 (m, 1H), 4.18-4.08 (m, 1H), 4.00 (t, J = 5.6 Hz, 2H), 3.86 (d, J = 7.2 Hz, 2H), 3.50-3.40 (m, 3H), 2.84 (s, 3H), 2.78 (t, J = 5.6 Hz, 2H), 2.73-2.50 (m, 7H), 2.35-2.20 (m, 1H), 1.25-1.15 (m, 1H), 1.07-0.96 (m, 6H).

### [Example 6] N-(4-(((2S,4R)-2-Methyl-1-propionyl-1,2,3,4-tetrahydroquinolin-4-yl)amino)phenyl)-2-(3-(4-(2-(4-methyl-3-oxopiperazin-1-yl)ethoxy)phenyl)ureido)acetamide (compound 6)

### (Step 1)

1-Methyl-4-(2-(4-nitrophenoxy)ethyl)piperazin-2-one (160 mg, yield: 65%) was obtained in the same manner as in step 2 of Example 5 using commercially available 1-(2-bromoethoxy)-4-nitrobenzene (220 mg, 0.88 mmol) and commercially available 1-methylpiperazin-2-one (100 mg, 0.88 mmol).

ESIMS, (M+H)⁺, m/z: 280.

### (Step 2)

1-Methyl-4-(2-(4-nitrophenoxy)ethyl)piperazin-2-one (160 mg, 0.57 mmol) obtained in step 1 was dissolved in methanol (7 mL), then 10% palladium/carbon (40 mg) was added to the solution, and the mixture was stirred at room temperature for 2 hours under a hydrogen atmosphere. The reaction mixture was filtered through celite, and the filtrate was concentrated under reduced pressure. The obtained residue was purified by thin-layer chromatography (dichloromethane/methanol=40/1) to give 4-(2-(4-aminophenoxy)ethyl)-1-methylpiperazin-2-one (140 mg, yield: 93%).

ESIMS, (M+H)⁺, m/z: 250.

### (Step 3)

Compound 6 (15 mg, yield: 23%) was obtained in the same manner as in step 3 of Example 1 using 4-(2-(4-aminophenoxy)ethyl)-1-methylpiperazin-2-one (25 mg, 0.10 mmol) obtained in step 2, and 2-amino-N-(4-(((2S,4R)-2-methyl-1-propionyl-1,2,3,4-tetrahydroquinolin-4-yl)amino)phenyl)acetamide (29 mg, 0.08 mmol) obtained in step 2 of Example 1.

ESIMS, (M+H)⁺, m/z: 642. ¹H NMR (DMSO-d₆, δ): 9.67 (s, 1H), 8.60 (s, 1H), 7.33-7.23 (m, 6H), 7.23-7.15 (s, 2H), 6.83 (d, J = 8.8 Hz, 2H), 6.61 (d, J = 8.8 Hz, 2H), 6.30 (d, J = 5.6 Hz, 1H), 5.92 (d, J = 8.0 Hz, 1H), 4.80-4.65 (m, 1H), 4.21-4.10 (m, 1H), 4.03 (t, J = 5.6 Hz, 2H), 3.86 (d, J = 5.6 Hz, 2H), 3.35-3.25 (m, 2H), 3.09 (s, 2H), 2.81 (s, 3H), 2.80-2.70 (m, 4H), 2.64-2.56 (m, 2H), 2.29-2.21 (m, 1H), 1.30-1.10 (m, 1H), 1.07-0.96 (m, 6H).

### [Example 7] 2-(3-(4-(2-((S)-3,4-Dimethyl-5-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 7)

Compound 7 (23 mg, yield: 35%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and commercially available (S)-1,6-dimethylpiperazin-2-one hydrochloride (49 mg, 0.30 mmol).

ESIMS, (M+H)⁺, m/z: 656. ¹H NMR (DMSO-d₆, δ): 9.67 (s, 1H), 8.61 (s, 1H), 7.34-7.22 (m, 6H), 7.21-7.14 (m, 2H), 6.84 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.18-4.08 (m, 1H), 4.03 (t, J = 5.6 Hz, 2H), 3.86 (d, J = 7.2 Hz, 2H), 3.50-3.40 (m, 1H), 3.25-3.15 (m, 1H), 3.05-2.95 (m, 1H), 2.75 (s, 3H), 2.73-2.67 (m, 2H), 2.67-2.54 (m, 4H), 2.35-2.20 (m, 1H), 1.25-1.15 (m, 1H), 1.19 (d, J = 6.8 Hz, 3H), 1.07-0.96 (m, 6H).

### [Example 8] 2-(3-(4-(2-((S)-4-Acetyl-3-methylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 8)

### (Step 1)

Commercially available tert-butyl (S)-3-methylpiperazine-1-carboxylate (500 mg, 2.5 mmol) was dissolved in dichloromethane (10 mL), then triethylamine (760 mg, 7.5 mmol) and acetyl chloride (290 mg, 3.7 mmol) were added to the solution at 0°C, and the mixture was stirred at 0°C for 2 hours. A saturated aqueous sodium chloride solution was added to the reaction mixture, and an organic layer was extracted with ethyl acetate. The organic layer was dried over anhydrous sodium sulfate and concentrated under reduced pressure to give a crude product of tert-butyl (S)-4-acetyl-3-methylpiperazine-1-carboxylate (520 mg).

ESIMS, (M+H)⁺, m/z: 243.

### (Step 2)

The crude product of tert-butyl (S)-4-acetyl-3-methylpiperazine-1-carboxylate (520 mg) obtained in step 2 was dissolved in dichloromethane (4 mL), then a 4 mol/L solution of hydrogen chloride in 1,4-dioxane (4.0 mL, 16 mmol) was added to the solution, and the mixture was stirred at room temperature for 1 hour. The reaction mixture was concentrated under reduced pressure to give a crude product of (S)-1-(2-methylpiperazin-1-yl)ethan-1-one hydrochloride (380 mg).

ESIMS, (M+H)⁺, m/z: 143.

### (Step 3)

Compound 8 (27 mg, yield: 41%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and the crude product of (S)-1-(2-methylpiperazin-1-yl)ethan-1-one hydrochloride (53 mg) obtained in step 2.

ESIMS, (M+H)⁺, m/z: 670. ¹H NMR (DMSO-d₆, δ): 9.67 (s, 1H), 8.60 (s, 1H), 7.33-7.22 (m, 6H), 7.22-7.14 (m, 2H), 6.88 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.25-4.14 (m, 1H), 4.02 (t, J = 5.6 Hz, 2H), 3.86 (d, J = 7.2 Hz, 2H), 3.65-3.55 (m, 1H), 3.00-2.90 (m, 1H), 2.85-2.75 (m, 1H), 2.68-2.62 (m, 4H), 2.30-2.18 (m, 2H), 2.35-2.20 (m, 1H), 2.05-1.95 (m, 5H), 1.26-1.08 (m, 4H), 1.07-0.96 (m, 6H).

### [Example 9] 2-(3-(4-(2-((R)-4-Acetyl-3-methylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 9)

### (Step 1)

A crude product of tert-butyl (R)-4-acetyl-3-methylpiperazine-1-carboxylate (520 mg) was obtained in the same manner as in step 1 of Example 8 using commercially available tert-butyl (R)-3-methylpiperazine-1-carboxylate (500 mg, 2.5 mmol).

ESIMS, (M+H)⁺, m/z: 243.

### (Step 2)

A crude product of (R)-1-(2-methylpiperazin-1-yl)ethan-1-one hydrochloride (380 mg) was obtained in the same manner as in step 2 of Example 8 using the crude product of tert-butyl (R)-4-acetyl-3-methylpiperazine-1-carboxylate (520 mg) obtained in step 1.

ESIMS, (M+H)⁺, m/z: 143.

### (Step 3)

(R)-1-(2-Methyl-4-(2-(4-nitrophenoxy)ethyl)piperazin-1-yl)ethan-1-one (160 mg, yield: 98%) was obtained in the same manner as in step 2 of Example 5 using the crude product of (R)-1-(2-methylpiperazin-1-yl)ethan-1-one hydrochloride (100 mg) obtained in step 2 and commercially available 1-(2-bromoethoxy)-4-nitrobenzene (140 mg, 0.56 mmol).

ESIMS, (M+H)⁺, m/z: 308.

### (Step 4)

(R)-1-(4-(2-(4-Aminophenoxy)ethyl)-2-methylpiperazin-1-yl)ethan-1-one (160 mg, yield: 97%) was obtained in the same manner as in step 2 of Example 6 using (R)-1-(2-methyl-4-(2-(4-nitrophenoxy)ethyl)piperazin-1-yl)ethan-1-one (160 mg, 0.52 mmol) obtained in step 3.

### (Step 5)

Compound 9 (24 mg, yield: 40%) was obtained in the same manner as in step 3 of Example 1 using (R)-1-(4-(2-(4-aminophenoxy)ethyl)-2-methylpiperazin-1-yl)ethan-1-one (25 mg, 0.09 mmol) obtained in step 4 and 2-amino-N-(4-(((2S,4R)-2-methyl-1-propionyl-1,2,3,4-tetrahydroquinolin-4-yl)amino)phenyl)acetamide (30 mg, 0.08 mmol) obtained in step 2 of Example 1.

ESIMS, (M+H)⁺, m/z: 670. ¹H NMR (DMSO-d₆, δ): 9.67 (s, 1H), 8.60 (s, 1H), 7.33-7.22 (m, 6H), 7.22-7.14 (m, 2H), 6.88 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.25-4.14 (m, 1H), 4.02 (t, J = 5.6 Hz, 2H), 3.86 (d, J = 7.2 Hz, 2H), 3.65-3.55 (m, 1H), 3.00-2.90 (m, 1H), 2.85-2.75 (m, 1H), 2.68-2.62 (m, 4H), 2.30-2.18 (m, 2H), 2.35-2.20 (m, 1H), 2.05-1.95 (m, 5H), 1.26-1.08 (m, 4H), 1.07-0.96 (m, 6H).

### [Example 10] 2-(3-(4-(2-(4-Ethyl-3-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 10)

Compound 10 (18 mg, yield: 40%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (40 mg, 0.07 mmol) obtained in step 1 of Example 5 and commercially available 1-ethylpiperazin-2-one (11 mg, 0.09 mmol).

ESIMS, (M+H)⁺, m/z: 656. ¹H NMR (DMSO-d₆, δ): 9.67 (s, 1H), 8.61 (s, 1H), 7.35-7.20 (m, 6H), 7.20-7.14 (m, 2H), 6.84 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.25-4.10 (m, 1H), 4.03 (t, J = 5.6 Hz, 2H), 3.86 (d, J = 5.4 Hz, 2H), 3.33-3.23 (m, 4H), 3.08 (s, 2H), 2.85-2.70 (m, 4H), 2.60-2.45 (m, 2H), 2.35-2.20 (m, 1H), 1.22-1.09 (m, 1H), 1.07-0.96 (m, 9H).

### [Example 11] 2-(3-(4-(2-((S)-2,4-Dimethyl-5-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 11)

Compound 11 (12 mg, yield: 15%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (70 mg, 0.12 mmol) obtained in step 1 of Example 5 and commercially available (S)-1,5-dimethylpiperazin-2-one hydrochloride (11 mg, 0.09 mmol).

ESIMS, (M+H)⁺, m/z: 656. ¹H NMR (DMSO-d₆, δ): 9.67 (s, 1H), 8.61 (s, 1H), 7.34-7.22 (m, 6H), 7.20-7.10 (m, 2H), 6.83 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.19-4.08 (m, 1H), 4.00 (t, J = 5.6 Hz, 2H), 3.86 (d, J = 5.6 Hz, 2H), 3.31-3.24 (m, 3H), 3.15-3.10 (m, 1H), 3.10-3.00 (m, 1H), 3.00-2.90 (m, 2H), 2.80 (s, 3H), 2.65-2.50 (m, 2H), 2.30-2.15 (m, 1H), 1.25-1.10 (m, 1H), 1.09-0.96 (m, 9H).

### [Example 12] 2-(3-(4-(2-((R)-3,4-Dimethyl-5-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 12)

Compound 12 (13 mg, yield: 20%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and commercially available (R)-1,6-dimethylpiperazin-2-one hydrochloride (49 mg, 0.30 mmol).

ESIMS, (M+H)⁺, m/z: 656. ¹H NMR (DMSO-d₆, δ): 9.67 (s, 1H), 8.61 (s, 1H), 7.34-7.22 (m, 6H), 7.21-7.14 (m, 2H), 6.84 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.18-4.08 (m, 1H), 4.03 (t, J = 5.6 Hz, 2H), 3.86 (d, J = 7.2 Hz, 2H), 3.50-3.40 (m, 1H), 3.25-3.15 (m, 1H), 3.05-2.95 (m, 1H), 2.75 (s, 3H), 2.73-2.67 (m, 2H), 2.67-2.54 (m, 4H), 2.35-2.20 (m, 1H), 1.25-1.15 (m, 1H), 1.19 (d, J = 6.8 Hz, 3H), 1.07-0.96 (m, 6H).

### [Example 13] 2-(3-(4-(2-(4-(Cyclopropanecarbonyl)piperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 13)

Compound 13 (30 mg, yield: 67%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (40 mg, 0.07 mmol) obtained in step 1 of Example 5 and commercially available cyclopropyl(piperazin-1-yl)methanone (13 mg, 0.09 mmol).

ESIMS, (M+H)⁺, m/z: 682. ¹H NMR (DMSO-d₆, δ): 9.67 (s, 1H), 8.60 (s, 1H), 7.33-7.21 (m, 6H), 7.21-7.12 (m, 2H), 6.83 (d, J = 8.8 Hz, 2H), 6.60 (d, J = 8.8 Hz, 2H), 6.29 (t, J = 5.6 Hz, 1H), 5.92 (d, J = 8.0 Hz, 1H), 4.85-4.70 (m, 1H), 4.25-4.10 (m, 1H), 4.03 (t, J = 5.6 Hz, 2H), 3.86 (d, J = 7.2 Hz, 2H), 3.75-3.66 (m, 2H), 3.55-3.45 (m, 2H), 2.70 (t, J = 5.6 Hz, 2H), 2.60-2.50 (m, 4H), 2.50-2.40 (m, 2H), 2.35-2.20 (m, 1H), 2.02-1.91 (m, 1H), 1.25-1.15 (m, 1H), 1.15-2.00 (m, 6H), 0.85-0.70 (m, 4H).

### [Example 14] 2-(3-(4-(2-((1R,4R)-5-Acetyl-2,5-diazabicyclo[2.2.1]heptan-2-yl)ethoxy)phenyl)ureido)-N-(4-(((2S,4R)-2-methyl-1-propionyl-1,2,3,4-tetrahydroquinolin-4-yl)amino)phenyl)acetamide (compound 14)

Compound 14 (17 mg, yield: 38%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (40 mg, 0.07 mmol) obtained in step 1 of Example 5 and commercially available 1-((1R,4R)-2,5-diazabicyclo[2.2.1]heptan-2-yl)ethan-1-one (12 mg, 0.09 mmol).

ESIMS, (M+H)⁺, m/z: 668. ¹H NMR (DMSO-d₆, δ): 9.67 (s, 1H), 8.60 (s, 1H), 7.33-7.23 (m, 6H), 7.23-7.16 (m, 2H), 6.87 (d, J = 8.8 Hz, 2H), 6.60 (d, J = 8.8 Hz, 2H), 6.29 (t, J = 5.6 Hz, 1H), 5.92 (d, J = 8.0 Hz, 1H), 4.85-4.70 (m, 1H), 4.17-4.11 (m, 1H), 3.94 (t, J = 5.6 Hz, 2H), 3.85 (d, J = 5.2 Hz, 2H), 3.79-3.71 (m, 2H), 3.60-3.50 (m, 1H), 3.35-3.25 (m, 2H), 3.00-2.90 (m, 2H), 2.64-2.56 (m, 3H), 2.29-2.16 (m, 1H), 2.01 (s, 3H), 1.85-1.65 (m, 2H), 1.25-1.10 (m, 1H), 1.07-0.96 (m, 6H).

### [Example 15] 2-(3-(4-(2-(4-Acetyl-1,4-diazepan-1-yl)ethoxy)phenyl)ureido)-N-(4-(((2S,4R)-2-methyl-1-propionyl-1,2,3,4-tetrahydroquinolin-4-yl)amino)phenyl)acetamide (compound 15)

Compound 15 (25 mg, yield: 58%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (40 mg, 0.07 mmol) obtained in step 1 of Example 5 and commercially available 1-(1,4-diazepan-1-yl)ethan-1-one (12 mg, 0.09 mmol). ESIMS, (M+H)⁺, m/z: 670. ¹H NMR (DMSO-d₆, δ): 9.67 (s, 1H), 8.61 (s, 1H), 7.33-7.21 (m, 6H), 7.21-7.16 (m, 2H), 6.82 (d, J = 8.8 Hz, 2H), 6.60 (d, J = 8.8 Hz, 2H), 6.30 (t, J = 5.6 Hz, 1H), 5.92 (d, J = 8.0 Hz, 1H), 4.85-4.70 (m, 1H), 4.17-4.11 (m, 1H), 4.05-3.95 (m, 2H), 3.85 (d, J = 5.2 Hz, 2H), 3.51-3.40 (m, 4H), 2.87-2.75 (m, 3H), 2.72-2.60 (m, 3H), 2.60-2.50 (m, 2H), 2.35-2.20 (m, 1H), 1.98 (s, 3H), 1.85-1.66 (m, 2H), 1.21-1.09 (m, 1H), 1.07-0.96 (m, 6H).

### [Example 16] 2-(3-(4-(2-((R)-2,4-Dimethyl-5-oxopiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 16)

Compound 16 (13 mg, yield: 17%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (70 mg, 0.12 mmol) obtained in step 1 of Example 5 and commercially available (R)-1,5-dimethylpiperazin-2-one hydrochloride (44 mg, 0.35 mmol).

ESIMS, (M+H)⁺, m/z: 656. ¹H NMR (DMSO-d₆, δ): 9.67 (s, 1H), 8.61 (s, 1H), 7.34-7.22 (m, 6H), 7.20-7.10 (m, 2H), 6.83 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.19-4.08 (m, 1H), 4.00 (t, J = 5.6 Hz, 2H), 3.86 (d, J = 5.6 Hz, 2H), 3.31-3.24 (m, 3H), 3.15-3.10 (m, 1H), 3.10-3.00 (m, 1H), 3.00-2.90 (m, 2H), 2.80 (s, 3H), 2.65-2.50 (m, 2H), 2.30-2.15 (m, 1H), 1.25-1.10 (m, 1H), 1.09-0.96 (m, 9H).

### [Example 17] N-(4-(((2S,4R)-2-Methyl-1-propionyl-1,2,3,4-tetrahydroquinolin-4-yl)amino)phenyl)-2-(3-(4-(2-(4-methyl-3-oxo-1,4-diazepan-1-yl)ethoxy)phenyl)ureido)acetamide (compound 17)

Compound 17 (6.0 mg, yield: 8%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (70 mg, 0.12 mmol) obtained in step 1 of Example 5 and commercially available 1-methyl-1,4-diazepan-2-one hydrochloride (44 mg, 0.35 mmol).

ESIMS, (M+H)⁺, m/z: 656. ¹H NMR (DMSO-d₆, δ): 9.67 (s, 1H), 8.61 (s, 1H), 7.34-7.21 (m, 6H), 7.20-7.14 (m, 2H), 6.82 (d, J = 8.8 Hz, 2H), 6.61 (d, J = 8.8 Hz, 2H), 6.30 (t, J = 5.6 Hz, 1H), 5.92 (d, J = 8.0 Hz, 1H), 4.85-4.70 (m, 1H), 4.17-4.11 (m, 1H), 3.97 (t, J = 5.6 Hz, 2H), 3.86 (d, J = 7.2 Hz, 2H), 3.48-3.38 (m, 3H), 3.00-2.90 (m, 2H), 2.85 (s, 3H), 2.81 (t, J = 5.6 Hz, 2H), 2.70-2.58 (s, 3H), 2.35-2.20 (m, 1H), 1.70-1.58 (m, 2H), 1.22-1.09 (m, 1H), 1.07-0.96 (m, 6H).

### [Example 18] N-(4-(((2S,4R)-2-Methyl-1-propionyl-1,2,3,4-tetrahydroquinolin-4-yl)amino)phenyl)-2-(3-(4-(2-(4-propionylpiperazin-1-yl)ethoxy)phenyl)ureido)acetamide (compound 18)

Compound 18 (20 mg, yield: 30%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and commercially available 1-(piperazin-1-yl)propan-1-one hydrochloride (53 mg, 0.30 mmol).

ESIMS, (M+H)⁺, m/z: 670. ¹H NMR (DMSO-d₆, δ): 9.67 (s, 1H), 8.60 (s, 1H), 7.35-7.20 (m, 6H), 7.20-7.14 (m, 2H), 6.83 (d, J = 8.8 Hz, 2H), 6.61 (d, J = 8.8 Hz, 2H), 6.29 (t, J = 5.6 Hz, 1H), 5.92 (d, J = 7.2 Hz, 1H), 4.85-4.70 (m, 1H), 4.17-4.11 (m, 1H), 4.02 (t, J = 5.6 Hz, 2H), 3.86 (d, J = 7.2 Hz, 2H), 3.49-3.41 (m, 3H), 2.80-2.70 (m, 2H), 2.59-2.68 (m, 4H), 2.51-2.49 (m, 2H), 2.38-2.18 (m, 4H), 1.22-1.09 (m, 1H), 1.07-0.93 (m, 9H).

### [Example 19] N-(4-(((2S,4R)-2-Methyl-1-propionyl-1,2,3,4-tetrahydroquinolin-4-yl)amino)phenyl)-2-(3-(4-(2-((S)-6-oxohexahydropyrrolo[1,2-a]pyrazin-2(1H)-yl)ethoxy)phenyl)ureido)acetamide (compound 19)

Compound 19 (20 mg, yield: 30%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and commercially available (S)-hexahydropyrrolo[1,2-a]pyrazin-6(2H)-one hydrochloride (52 mg, 0.30 mmol).

ESIMS, (M+H)⁺, m/z: 668. ¹H NMR (DMSO-d₆, δ): 9.67 (s, 1H), 8.60 (s, 1H), 7.33-7.23 (m, 6H), 7.25-7.16 (m, 2H), 6.82 (d, J = 8.8 Hz, 2H), 6.60 (d, J = 8.8 Hz, 2H), 6.29 (t, J = 5.6 Hz, 1H), 5.92 (d, J = 8.0 Hz, 1H), 4.85-4.70 (m, 1H), 4.17-4.11 (m, 1H), 4.02 (t, J = 5.6 Hz, 2H), 3.85 (d, J = 7.2 Hz, 2H), 3.79-3.71 (m, 1H), 3.60-3.50 (m, 1H), 3.15-3.06 (m, 1H), 3.00-2.90 (m, 1H), 2.80-2.73 (m, 3H), 2.64-2.56 (m, 2H), 2.29-2.16 (m, 3H), 2.13-2.01 (m, 1H), 2.00-1.90 (m, 1H), 1.85-1.75 (m, 1H), 1.57-1.45 (m, 1H), 1.21-1.09 (m, 1H), 1.07-0.96 (m, 6H).

### [Example 20] 2-(3-(4-(2-(4-Acetyl-3,3-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 20)

### (Step 1)

A crude product of tert-butyl 4-acetyl-3,3-dimethylpiperazine-1-carboxylate (510 mg) was obtained in the same manner as in step 1 of Example 8 using commercially available tert-butyl 3,3-dimethylpiperazine-1-carboxylate (500 mg, 2.5 mmol).

ESIMS, (M+H)⁺, m/z: 257.

### (Step 2)

A crude product of 1-(2,2-dimethylpiperazin-1-yl)ethan-1-one hydrochloride (360 mg) was obtained in the same manner as in step 2 of Example 8 using the crude product of tert-butyl 4-acetyl-3,3-dimethylpiperazine-1-carboxylate (510 mg) obtained in step 1. ESIMS, (M+H)⁺, m/z: 157.

### (Step 3)

Compound 20 (30 mg, yield: 40%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (65 mg, 0.11 mmol) obtained in step 1 of Example 5 and the crude product of 1-(2,2-dimethylpiperazin-1-yl)ethan-1-one hydrochloride (62 mg) obtained in step 2.

ESIMS, (M+H)⁺, m/z: 684. ¹H NMR (DMSO-d₆, δ): 9.66 (s, 1H), 8.60 (s, 1H), 7.35-7.20 (m, 6H), 7.22-7.14 (m, 2H), 6.86 (d, J = 8.8 Hz, 2H), 6.59 (d, J = 8.8 Hz, 2H), 6.29 (t, J = 5.6 Hz, 1H), 5.91 (d, J = 8.0 Hz, 1H), 4.79-4.71 (m, 1H), 4.20-4.10 (m, 1H), 4.03 (t, J = 5.6 Hz, 2H), 3.86 (d, J = 5.2 Hz, 2H), 3.35-3.25 (m, 2H), 2.69-2.54 (m, 6H), 2.26 (s, 3H), 1.95 (s, 3H), 1.35 (s, 6H), 1.22-1.09 (m, 1H), 1.07-0.97 (m, 6H).

### [Example 21] 2-(3-(4-(2-((R)-4-Acetyl-2-methylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 21)

### (Step 1)

A crude product of tert-butyl (R)-4-acetyl-2-methylpiperazine-1-carboxylate (510 mg) was obtained in the same manner as in step 1 of Example 8 using commercially available tert-butyl (R)-2-methylpiperazine-1-carboxylate (500 mg, 2.5 mmol).

ESIMS, (M+H)⁺, m/z: 243.

### (Step 2)

A crude product of (R)-1-(3-methylpiperazin-1-yl)ethan-1-one hydrochloride (380 mg) was obtained in the same manner as in step 2 of Example 8 using the crude product of tert-butyl (R)-4-acetyl-2-methylpiperazine-1-carboxylate (510 mg) obtained in step 1.

ESIMS, (M+H)⁺, m/z: 143.

### (Step 3)

Compound 21 (1.7 mg, yield: 3%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and the crude product of (R)-1-(3-methylpiperazin-1-yl)ethan-1-one hydrochloride (53 mg) obtained in step 2.

ESIMS, (M+H)⁺, m/z: 670. ¹H NMR (CDCl₃, δ): 9.67 (s, 1H), 8.29 (s, 1H), 7.35-7.25 (m, 4H), 7.25-7.15 (m, 4H), 6.84 (d, J = 8.8 Hz, 3H), 6.59 (d, J = 8.8 Hz, 2H), 5.90-5.80 (m, 1H), 5.05-4.90 (m, 1H), 4.25-4.05 (m, 6H), 3.3.95-3.80 (m, 1H), 3.75-365 (m, 1H), 3.50-3.40 (m, 1H), 3.30-3.10 (m, 2H), 3.00-2.85 (m, 2H), 2.80-2.52 (m, 3H), 2.45-2.30 (m, 1H), 2.11 (s, 3H), 1.35-1.20 (m, 1H), 1.20-1.12 (m, 9H).

### [Example 22] 2-(3-(4-(2-((S)-4-Acetyl-2-methylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 22)

Compound 22 (6.5 mg, yield: 10%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (60 mg, 0.10 mmol) obtained in step 1 of Example 5 and commercially available (S)-1-(3-methylpiperazin-1-yl)ethan-1-one hydrochloride (53 mg, 0.30 mmol).

ESIMS, (M+H)⁺, m/z: 670. ¹H NMR (CDCl₃, δ): 9.67 (s, 1H), 8.29 (s, 1H), 7.35-7.25 (m, 3H), 7.25-7.15 (m, 4H), 6.84 (d, J = 8.8 Hz, 3H), 6.59 (d, J = 8.8 Hz, 2H), 5.90-5.80 (m, 1H), 5.05-4.90 (m, 1H), 4.25-4.05 (m, 6H), 3.3.95-3.80 (m, 1H), 3.75-365 (m, 1H), 3.50-3.40 (m, 1H), 3.30-3.10 (m, 2H), 3.00-2.85 (m, 2H), 2.80-2.52 (m, 4H), 2.45-2.30 (m, 1H), 2.11 (s, 3H), 1.35-1.20 (m, 1H), 1.20-1.12 (m, 9H).

### [Example 23] 2-(3-(4-(2-(4-Acetyl-2,2-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 23)

### (Step 1)

A crude product of tert-butyl 4-acetyl-2,2-dimethylpiperazine-1-carboxylate (510 mg) was obtained in the same manner as in step 1 of Example 8 using commercially available tert-butyl 2,2-dimethylpiperazine-1-carboxylate (500 mg, 2.3 mmol).

ESIMS, (M+H)⁺, m/z: 257.

### (Step 2)

A crude product of 1-(3,3-dimethylpiperazin-1-yl)ethan-1-one hydrochloride (360 mg) was obtained in the same manner as in step 2 of Example 8 using the crude product of tert-butyl 4-acetyl-2,2-dimethylpiperazine-1-carboxylate (510 mg) obtained in step 1. ESIMS, (M+H)⁺, m/z: 157.

### (Step 3)

Compound 23 (6.9 mg, yield: 10%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (65 mg, 0.11 mmol) obtained in step 1 of Example 5 and the crude product of 1-(3,3-dimethylpiperazin-1-yl)ethan-1-one hydrochloride (62 mg) obtained in step 2.

ESIMS, (M+H)⁺, m/z: 684. ¹H NMR (CDCl₃, δ): 9.57 (s, 1H), 8.34 (s, 1H), 7.45-7.20 (m, 4H), 7.23-7.17 (m, 4H), 6.84 (d, J = 8.8 Hz, 3H), 6.58 (d, J = 8.8 Hz, 2H), 5.90-5.80 (m, 1H), 4.96-4.90 (m, 1H), 4.25-4.14 (m, 1H), 4.15-4.00 (m, 4H), 3.95-3.86 (m, 1H), 3.75-3.65 (m, 1H), 3.60-3.50 (m, 1H), 3.50-3.41 (m, 1H), 3.30-3.22 (m, 1H), 2.90-2.70 (m, 2H), 2.70-2.52 (m, 3H), 2.45-2.30 (m, 1H), 2.13 (s, 3H), 1.31-1.22 (m, 1H), 1.20-1.12 (m, 6H), 1.10-1.00 (m, 6H).

### [Example 24] 2-(3-(4-(2-((1S,4S)-5-Acetyl-2,5-diazabicyclo[2.2.1]heptan-2-yl)ethoxy)phenyl)ureido)-N-(4-(((2S,4R)-2-methyl-1-propionyl-1,2,3,4-tetrahydroquinolin-4-yl)amino)phenyl)acetamide (compound 24)

Compound 24 (6.5 mg, yield: 9%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (65 mg, 0.11 mmol) obtained in step 1 of Example 5 and commercially available 1-((1S,4S)-2,5-diazabicyclo[2.2.1]heptan-2-yl)ethan-1-one hydrochloride (57 mg, 0.32 mmol).

ESIMS, (M+H)⁺, m/z: 668. ¹H NMR (CDCl₃, δ): 9.67 (s, 1H), 8.34 (s, 1H), 7.36-7.25 (m, 3H), 7.25-7.15 (m, 4H), 7.13-6.89 (m, 1H), 6.81 (t, J = 8.8 Hz, 2H), 6.58 (d, J = 8.8 Hz, 2H), 5.87 (s, 1H), 5.05-4.90 (m, 1H), 4.27-4.15 (m, 2H), 4.15-4.00 (m, 4H), 4.00-3.73 (m, 3H), 3.45-3.30 (m, 2H), 3.32-3.08 (m, 2H), 2.96-2.71 (m, 2H), 2.45-2.30 (m, 1H), 2.20-2.00 (m, 1H), 2.11 (s, 3H), 1.97-1.78 (m, 2H), 1.35-1.25 (m, 1H), 1.25-1.12 (m, 6H).

### [Example 25] 2-(3-(4-(2-((2R,6R)-4-Acetyl-2,6-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 25)

### (Step 1)

A crude product of tert-butyl (2S,6S)-4-acetyl-2,6-dimethylpiperazine-1-carboxylate (330 mg) was obtained in the same manner as in step 1 of Example 8 using commercially available tert-butyl (2S,6S)-2,6-dimethylpiperazine-1-carboxylate (250 mg, 1.2 mmol).

ESIMS, (M+H)⁺, m/z: 257.

### (Step 2)

A crude product of 1-((3S,5S)-3,5-dimethylpiperazin-1-yl)ethan-1-one hydrochloride (260 mg) was obtained in the same manner as in step 2 of Example 8 using the crude product of tert-butyl (2S,6S)-4-acetyl-2,6-dimethylpiperazine-1-carboxylate (330 mg) obtained in step 1.

ESIMS, (M+H)⁺, m/z: 157.

### (Step 3)

Compound 25 (6.6 mg, yield: 9%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (65 mg, 0.11 mmol) obtained in step 1 of Example 5 and the crude product of 1-((3S,5S)-3,5-dimethylpiperazin-1-yl)ethan-1-one hydrochloride (68 mg) obtained in step 2.

ESIMS, (M+H)⁺, m/z: 684. ¹H NMR (CDCl₃, δ): 9.67 (s, 1H), 8.33 (s, 1H), 7.35-7.25 (m, 3H), 7.23-7.14 (m, 4H), 6.84 (d, J = 8.8 Hz, 3H), 6.58 (d, J = 8.8 Hz, 2H), 5.88 (s, 1H), 5.05-4.90 (m, 1H), 4.20-3.98 (m, 5H), 3.95-3.80 (m, 2H), 3.60-3.50 (m, 1H), 3.30-3.20 (m, 2H), 3.20-2.99 (m, 3H), 2.90-2.80 (m, 1H), 2.71-2.52 (m, 2H), 2.45-2.30 (m, 1H), 2.10 (s, 3H), 1.31-1.22 (m, 1H), 1.20-1.05 (m, 12H).

### [Example 26] 2-(3-(4-(2-((3S,5S)-4-Acetyl-3,5-dimethylpiperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 26)

### (Step 1)

A crude product of tert-butyl (3S,5S)-4-acetyl-3,5-dimethylpiperazine-1-carboxylate (360 mg) was obtained in the same manner as in step 1 of Example 8 using commercially available tert-butyl (3S,5S)-3,5-dimethylpiperazine-1-carboxylate (260 mg, 1.2 mmol).

ESIMS, (M+H)⁺, m/z: 257.

### (Step 2)

A crude product of 1-((2S,6S)-2,6-dimethylpiperazin-1-yl) ethan-1-one hydrochloride (300 mg) was obtained in the same manner as in step 2 of Example 8 using the crude product of tert-butyl (3S,5S)-4-acetyl-3,5-dimethylpiperazine-1-carboxylate (360 mg) obtained in step 1.

ESIMS, (M+H)⁺, m/z: 157.

### (Step 3)

Compound 26 (6.0 mg, yield: 8%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (65 mg, 0.11 mmol) obtained in step 1 of Example 5 and the crude product of 1-((2S,6S)-2,6-dimethylpiperazin-1-yl)ethan-1-one hydrochloride (68 mg) obtained in step 2.

ESIMS, (M+H)⁺, m/z: 684. ¹H NMR (CDCl₃, δ): 9.67 (s, 1H), 8.33 (s, 1H), 7.35-7.25 (m, 3H), 7.23-7.14 (m, 4H), 6.84 (d, J = 8.8 Hz, 3H), 6.58 (d, J = 8.8 Hz, 2H), 5.88 (s, 1H), 5.05-4.90 (m, 1H), 4.20-4.05 (m, 5H), 4.05-3.95 (m, 2H), 3.95-3.85 (m, 1H), 3.10-2.90 (m, 4H), 2.75-2.52 (m, 4H), 2.45-2.30 (m, 1H), 2.13 (s, 3H), 1.40 (d, J = 6.4 Hz, 6H), 1.31-1.22 (m, 1H), 1.20-1.05 (m, 6H).

### [Example 27] 2-(3-(4-(2-((S)-4-Acetyl-3-(hydroxymethyl)piperazin-1-yl)ethoxy)phenyl)ureido)-N-(4-(((2S,4R)-2-methyl-1-propionyl-1,2,3,4-tetrahydroquinolin-4-yl)amino)phenyl)acetamide (compound 27)

### (Step 1)

Commercially available tert-butyl (S)-3-(hydroxymethyl)piperazine-1-carboxylate (800 mg, 3.7 mmol) was dissolved in dichloromethane (10 mL), then triethylamine (1.1 g, 11 mmol) and acetic anhydride (380 mg, 3.7 mmol) were added to the solution, and the mixture was stirred at room temperature for 1 hour. The reaction mixture was washed with water, and the obtained organic layer was concentrated under reduced pressure to give a crude product of tert-butyl (S)-4-acetyl-3-(hydroxymethyl)piperazine-1-carboxylate (800 mg).

ESIMS, (M+H)⁺, m/z: 259.

### (Step 2)

A crude product of (S)-1-(2-(hydroxymethyl)piperazin-1-yl)ethan-1-one (400 mg) was obtained in the same manner as in step 2 of Example 2 using the crude product of tert-butyl (S)-4-acetyl-3-(hydroxymethyl)piperazine-1-carboxylate (400 mg) obtained in step 1.

ESIMS, (M+H)⁺, m/z: 159.

### (Step 3)

Compound 27 (20 mg, yield: 9%) was obtained in the same manner as in step 2 of Example 5 using 2-(3-(4-(2-bromoethoxy)phenyl)ureido)-N-(4-(((2S,4R)-2-methyl-1-propionyl-1,2,3,4-tetrahydroquinolin-4-yl)amino)phenyl)acetamide (200 mg, 0.33 mmol) obtained in step 1 of Example 5 and the crude product of (S)-1-(2-(hydroxymethyl)piperazin-1-yl) ethan-1-one (450 mg) obtained in step 2.

ESIMS, (M+H)⁺, m/z: 686. ¹H NMR (DMSO-d₆, δ): 9.67 (s, 1H), 8.61 (s, 1H), 7.34-7.22 (m, 6H), 7.16 (d, J = 4.0 Hz, 2H), 6.83 (d, J = 8.8 Hz, 2H), 6.61 (d, J = 8.8 Hz, 2H), 6.29 (d, J = 5.6 Hz, 1H), 5.92 (d, J = 8.0 Hz, 1H), 4.82-4.65 (m, 2H), 4.33-4.10 (m, 2H), 4.01 (t, J = 6.0 Hz, 2H), 3.86 (d, J = 5.6 Hz, 2H), 3.81-3.55 (m, 3H), 3.21-2.87 (m, 3H), 2.81-2.58 (m, 4H), 2.32-2.12 (m, 2H), 2.12-1.82 (m, 1H), 2.08 (s, 3H), 1.27-1.10 (m, 1H), 1.07-0.97 (m, 6H)

### [Example 28]

### 2-((R)-2,4-Dimethyl-3-oxopiperazin-1-yl)-N-(4-(3-(2-((4-(((2S,4R)-2-methyl-1-propionyl-1,2,3,4-tetrahydroquinolin-4-yl)amino)phenyl)amino)-2-oxoethyl)ureido)phenyl)acetamide (compound 28)

### (Step 1)

Commercially available 4-nitroaniline (500 mg, 3.6 mmol) was dissolved in acetonitrile (10 mL), then 2-bromoacetyl bromide (1.0 g, 5.1 mmol) and sodium bicarbonate (910 mg, 11 mmol) were added to the solution at 0°C, and the mixture was stirred at 0°C for 2 hours. The reaction mixture was subjected to extraction with ethyl acetate, and the obtained organic layer was washed with a saturated aqueous sodium chloride solution. The obtained organic layer was dried over anhydrous sodium sulfate and concentrated under reduced pressure. The obtained residue was purified by silica gel column chromatography (chloroform/methanol = 10/1) to give 2-bromo-N-(4-nitrophenyl)acetamide (650 mg, yield: 65%).

ESIMS, (M+H)⁺, m/z: 259.

### (Step 2)

2-Bromo-N-(4-nitrophenyl)acetamide (300 mg, 1.2 mmol) obtained in step 1 was dissolved in acetonitrile (7 mL), then potassium carbonate (320 mg, 2.3 mmol) and commercially available (R)-1,3-dimethylpiperazin-2-one (220 mg, 1.7 mmol) were added to the solution, and the mixture was stirred at 70°C for 2 hours. The reaction mixture was diluted with ethyl acetate and washed with water and a saturated aqueous sodium chloride solution. The obtained organic layer was dried over anhydrous sodium sulfate and concentrated under reduced pressure. The obtained residue was purified by silica gel column chromatography (chloroform/methanol = 10/1) to give (R)-2-(2,4-dimethyl-3-oxopiperazin-1-yl)-N-(4-nitrophenyl)acetamide (350 mg, yield: 93%).

ESIMS, (M+H)⁺, m/z: 307.

### (Step 3)

(R)-N-(4-Aminophenyl)-2-(2,4-dimethyl-3-oxopiperazin-1-yl)acetamide(250 mg, yield: 76%) was obtained in the same manner as in step 2 of Example 6 using (R)-2-(2,4-dimethyl-3-oxopiperazin-1-yl)-N-(4-nitrophenyl)acetamide (350 mg, 1.1 mmol) obtained in step 2.

ESIMS, (M+H)⁺, m/z: 277.

### (Step 4)

Compound 28 (11 mg, yield: 7.6%) was obtained in the same manner as in step 3 of Example 2 using (R)-N-(4-aminophenyl)-2-(2,4-dimethyl-3-oxopiperazin-1-yl)acetamide (61 mg, 0.22 mmol) obtained in step 3, and 2-amino-N-(4-(((2S,4R)-2-methyl-1-propionyl-1,2,3,4-tetrahydroquinolin-4-yl)amino)phenyl)acetamide (80 mg, 0.22 mmol) obtained in step 2 of Example 1.

ESIMS, (M+H)⁺, m/z: 669. ¹H NMR (DMSO-d₆, δ): 9.68 (s, 1H), 9.58 (s, 1H), 8.74 (s, 1H), 7.53-7.45 (m, 2H), 7.34-7.23 (m, 6H), 7.26-7.13 (m, 2H), 6.61 (d, J = 8.8 Hz, 2H), 6.35 (t, J = 5.4 Hz, 1H), 5.92 (d, J = 8.0 Hz, 1H), 4.80-4.70 (m, 1H), 4.18-4.07 (m, 1H), 3.87 (d, J = 5.2 Hz, 2H), 3.39-3.18 (m, 5H), 3.10-3.00 (m, 1H), 2.84 (s, 3H), 2.85-2.70 (m, 1H), 2.65-2.50 (m, 2H), 2.35-2.20 (m, 1H), 1.26 (d, J = 6.9 Hz, 3H), 1.22-1.09 (m, 1H), 1.07-0.97 (m, 6H).

### [Example 29]

### 2-(3-(4-(3-((R)-2,4-Dimethyl-3-oxopiperazin-1-yl)propyl)phenyl)ureido)-N-(4-(((2S,4R)-2-methyl-1-propionyl-1,2,3,4-tetrahydroquinolin-4-yl)amino)phenyl)acetamide (compound 29)

### (Step 1)

(R)-1,3-Dimethyl-4-(3-(4-nitrophenyl)propyl)piperazin-2-one (100 mg, yield: 84%) was obtained in the same manner as in step 2 of Example 5 using commercially available 1-(3-bromopropyl)-4-nitrobenzene (100 mg, 0.41 mmol), and commercially available (R)-1,3-dimethylpiperazin-2-one (63 mg, 0.49 mmol).

ESIMS, (M+H)⁺, m/z: 292.

### (Step 2)

(R)-4-(3-(4-Aminophenyl)propyl)-1,3-dimethylpiperazin-2-one (83 mg, yield: 93%) was obtained in the same manner as in step 2 of Example 6 using (R)-1,3-dimethyl-4-(3-(4-nitrophenyl)propyl)piperazin-2-one (100 mg, 0.34 mmol) obtained in step 1.

ESIMS, (M+H)⁺, m/z: 262.

### (Step 3)

Compound 29 (100 mg, yield: 51%) was obtained in the same manner as in step 3 of Example 1 using (R)-4-(3-(4-aminophenyl)propyl)-1,3-dimethylpiperazin-2-one (79 mg, 0.30 mmol) obtained in step 2 and 2-amino-N-(4-(((2S,4R)-2-methyl-1-propionyl-1,2,3,4-tetrahydroquinolin-4-yl)amino)phenyl)acetamide (110 mg, 0.30 mmol) obtained in step 2 of Example 1.

ESIMS, (M+H)⁺, m/z: 654. ¹H NMR (DMSO-d₆, δ): 9.67 (s, 1H), 8.70 (s, 1H), 7.26-7.36 (m, 6H), 7.17 (d, J=4.0 Hz, 2H), 7.06 (d, J=8.8 Hz, 2H), 6.61 (d, J= 8.8 Hz, 2H), 6.40-6.30 (m, 1H), 5.91 (d, J = 8.0 Hz, 1H), 4.85-4.70 (m, 1H), 4.20-4.10 (m, 1H), 3.87 (d, J=5.6 Hz, 2H), 3.30-3.20 (m, 2H), 3.05-2.90 (m, 2H), 2.80 (s, 3H), 2.70-2.50 (m, 4H), 2.50-2.40 (m, 2H), 2.35-2.20 (m, 2H), 1.80-1.65 (m, 2H), 1.1.25-1.10 (m, 4H), 1.15-1.10 (m, 6H).

### [Example 30]

### 2-(3-(4-(2-((R)-2,4-Dimethyl-3-oxopiperazin-1-yl)ethoxy)-2-methoxyphenyl)ureido)-N-(4-(((2S,4R)-2-methyl-1-propionyl-1,2,3,4-tetrahydroquinolin-4-yl)amino)phenyl)acetamide (compound 30)

### (Step 1)

Commercially available 3-methoxy-4-nitrophenol (300 mg, 1.8 mmol) was dissolved in THF (15 mL), then commercially available 2-bromoethan-1-ol (330 mg, 2.7 mmol), triphenylphosphine (PPh₃) (560 mg, 2.1 mmol), and DIAD (460 mg, 2.1 mmol) were added to the solution, and the mixture was stirred at room temperature for 4 hours. The reaction mixture was concentrated under reduced pressure, and the obtained residue was purified by silica gel column chromatography (petroleum ether/ethyl acetate=95/5 to 30/70) to give 4-(2-bromoethoxy)-2-methoxy-1-nitrobenzene (500 mg, yield: 92%).

ESIMS, (M+H)⁺, m/z: 276.

### (Step 2)

(R)-4-(2-(3-Methoxy-4-nitrophenoxy)ethyl)-1,3-dimethylpiperazin-2-one (300 mg, yield: 80%) was obtained in the same manner as in step 2 of Example 5 using 4-(2-bromoethoxy)-2-methoxy-1-nitrobenzene (300 mg, 1.1 mmol) obtained in step 1 and (R)-1,3-dimethylpiperazin-2-one (210 mg, 1.6 mmol). ESIMS, (M+H)⁺, m/z: 324.

### (Step 3)

(R)-4-(2-(4-Amino-3-methoxyphenoxy)ethyl)-1,3-dimethylpiperazin-2-one (250 mg, yield: 86%) was obtained in the same manner as in step 2 of Example 6 using (R)-4-(2-(3-methoxy-4-nitrophenoxy)ethyl)-1,3-dimethylpiperazin-2-one (300 mg, 0.93 mmol) obtained in step 2.

ESIMS, (M+H)⁺, m/z: 294.

### (Step 4)

Compound 30 (53 mg, yield: 35%) was obtained in the same manner as in step 3 of Example 2 using (R)-4-(2-(4-amino-3-methoxyphenoxy)ethyl)-1,3-dimethylpiperazin-2-one (64 mg, 0.22 mmol) obtained in step 3 and 2-amino-N-(4-(((2S,4R)-2-methyl-1-propionyl-1,2,3,4-tetrahydroquinolin-4-yl)amino)phenyl)acetamide (80 mg, 0.22 mmol) obtained in step 2 of Example 1.

ESIMS, (M+H)⁺, m/z: 686. ¹H NMR (DMSO-d₆, δ): 9.64 (s, 1H), 7.99 (s, 1H), 7.85 (d, J = 8.8 Hz, 1H), 7.34-7.21 (m, 4H), 7.20-7.14 (m, 2H), 7.01 (t, J = 5.6 Hz, 1H), 6.64-6.55 (m, 3H), 6.44 (d, J = 8.8 Hz, 1H), 5.90 (d, J = 8.0 Hz, 1H), 4.85-4.72 (m, 1H), 4.18-4.07 (m, 1H), 4.03 (t, J = 5.6 Hz, 2H), 3.88-3.80 (m, 5H), 3.32-3.22 (m, 2H), 3.20-3.10 (m, 1H), 3.10-3.00 (m, 1H), 3.00-2.90 (m, 1H), 2.81 (s, 3H), 2.80-2.61 (m, 2H), 2.60-2.50 (m, 2H), 2.35-2.20 (m, 1H), 1.24 (d, J = 6.8 Hz, 3H), 1.22-1.09 (m, 1H), 1.07-0.97 (m, 6H).

### [Example 31]

### Methyl 3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzoate (compound 31)

### (Step 1)

Commercially available methyl 3-(2-hydroxyethyl)benzoate (300 mg, 1.7 mmol) was dissolved in THF (15 mL), then sodium hydride (60% w/w (mineral oil mixture), 270 mg, 6.7 mmol) was added to the solution, and the mixture was stirred at 0°C for 20 minutes. Commercially available 1-fluoro-4-nitrobenzene (0.20 mL, 1.8 mmol) was added to the reaction mixture, and the mixture was stirred at room temperature for 4 hours. Water was added to the reaction mixture, and the aqueous layer was subjected to extraction with ethyl acetate. The organic layer was washed with a saturated aqueous sodium chloride solution. The obtained organic layer was dried over anhydrous sodium sulfate and concentrated under reduced pressure. The obtained residue was purified by reverse-phase silica gel column chromatography (acetonitrile/water = 10/100 to 100/0) to give methyl 3-(2-(4-nitrophenoxy)ethyl)benzoate (150 mg, yield: 30%).

ESIMS, (M+H)⁺, m/z: 302.

### (Step 2)

Methyl 3-(2-(4-aminophenoxy)ethyl)benzoate (110 mg, yield: 84%) was obtained in the same manner as in step 2 of Example 6 using methyl 3-(2-(4-nitrophenoxy)ethyl)benzoate (150 mg, 0.49 mmol) obtained in step 1.

ESIMS, (M+H)⁺, m/z: 272.

### (Step 3)

Compound 31 (33 mg, yield: 19%) was obtained in the same manner as in step 3 of Example 1 using methyl 3-(2-(4-aminophenoxy)ethyl)benzoate (74 mg, 0.27 mmol) obtained in step 2 and 2-amino-N-(4-(((2S,4R)-2-methyl-1-propionyl-1,2,3,4-tetrahydroquinolin-4-yl)amino)phenyl)acetamide (100 mg, 0.27 mmol) obtained in step 2 of Example 1.

ESIMS, (M+H)⁺, m/z: 662. ¹H NMR (DMSO-d₆, δ): 9.67 (s, 1H), 8.60 (s, 1H), 7.92 (s, 1H), 7.83 (d, J = 7.6 Hz, 1H), 7.62 (d, J = 7.6 Hz, 1H), 7.47 (t, J = 7.6 Hz, 1H), 7.35-7.20 (m, 6H), 7.16 (s, 2H), 6.81 (d, J = 8.8 Hz, 2H), 6.61 (d, J = 8.8 Hz, 2H), 6.29 (t, J = 5.6 Hz, 1H), 5.92 (d, J = 8.0 Hz, 1H), 4.85-4.70 (m, 1H), 4.18-4.06 (m, 3H), 3.86 (s, 5H), 3.09 (t, J = 6.8 Hz, 2H), 2.65-2.50 (m, 2H), 2.26-2.18 (m, 1H), 1.19-1.07 (m, 1H), 1.07-0.86 (m, 6H).

### [Example 32]

### 2-(3-(4-(2-(4-Acetylpiperazin-1-yl)ethoxy)-3-fluorophenyl)ureido)-N-(4-(((2S,4R)-2-methyl-1-propionyl-1,2,3,4-tetrahydroquinolin-4-yl)amino)phenyl)acetamide (compound 32)

### (Step 1)

Commercially available 2-fluoro-4-nitrophenol (500 mg, 3.2 mmol) was dissolved in THF (10 mL), then 2-bromoethan-1-ol (480 mg, 3.8 mmol), PPh₃ (1.0 g, 3.8 mmol), and DIAD (770 mg, 3.8 mmol) were added to the solution, and the mixture was stirred at room temperature for 2 hours. The reaction mixture was concentrated under reduced pressure, and the obtained residue was purified by silica gel column chromatography (petroleum ether/ethyl acetate = 2/1) to give 1-(2-bromoethoxy)-2-fluoro-4-nitrobenzene (720 mg, 86%).

ESIMS, (M+H)⁺, m/z: 264.

### (Step 2)

1-(4-(2-(2-Fluoro-4-nitrophenoxy)ethyl)piperazin-1-yl)ethan-1-one (120 mg, yield: 78%) was obtained in the same manner as in step 2 of Example 5 using 1-(2-bromoethoxy)-2-fluoro-4-nitrobenzene (130 mg, 0.49 mmol) obtained in step 1 and 1-(piperazin-1-yl)ethan-1-one (130 mg, 0.98 mmol).

ESIMS, (M+H)⁺, m/z: 312.

### (Step 3)

1-(4-(2-(4-Amino-2-fluorophenoxy)ethyl)piperazin-1-yl)ethan-1-one (95 mg, yield: 88%) was obtained in the same manner as in step 2 of Example 6 using 1-(4-(2-(2-fluoro-4-nitrophenoxy)ethyl)piperazin-1-yl)ethan-1-one (120 mg, 0.38 mmol) obtained in step 2.

ESIMS, (M+H)⁺, m/z: 282.

### (Step 4)

Compound 32 (6.5 mg, yield: 2.9%) was obtained in the same manner as in step 3 of Example 1 using 1-(4-(2-(4-amino-2-fluorophenoxy)ethyl)piperazin-1-yl)ethan-1-one (95 mg, 0.33 mmol) obtained in step 3 and 2-amino-N-(4-(((2S,4R)-2-methyl-1-propionyl-1,2,3,4-tetrahydroquinolin-4-yl)amino)phenyl)acetamide (120 mg, 0.33 mmol) obtained in step 2 of Example 1.

ESIMS, (M+H)⁺, m/z: 674. ¹H NMR (DMSO-d₆, δ): 9.69 (s, 1H), 8.93 (s, 1H), 8.28 (s, 1H), 7.45 (dd, J = 14.0, 2.4 Hz, 1H), 7.34-7.21 (m, 4H), 7.21-7.14 (m, 2H), 7.7.14-7.05 (m, 1H), 7.01-6.93 (m, 1H), 6.61 (d, J = 8.8 Hz, 2H), 6.48 (t, J = 5.6 Hz, 1H), 5.92 (d, J = 8.0 Hz, 1H), 4.85-4.70 (m, 1H), 4.20-4.10 (m, 3H), 3.86 (d, J = 5.2 Hz, 2H), 3.50-3.40 (m, 4H), 2.71 (t, J = 5.6 Hz, 2H), 2.66-2.52 (m, 2H), 2.51-2.38 (m, 3H), 2.35-2.20 (m, 1H), 1.98 (s, 3H), 1.25-1.10 (m, 1H), 1.07-0.96 (m, 6H).

### [Example 33]

### 2-(3-(3-Fluoro-4-(2-(4-methyl-5-oxo-1,4-diazepan-1-yl)ethoxy)phenyl)ureido)-N-(4-(((2S,4R)-2-methyl-1-propionyl-1,2,3,4-tetrahydroquinolin-4-yl)amino)phenyl)acetamide (compound 33)

### (Step 1)

A crude product of 1-(2-(2-fluoro-4-nitrophenoxy)ethyl)-4-methyl-1,4-diazepan-5-one (120 mg) was obtained in the same manner as in step 2 of Example 5 using 1-(2-bromoethoxy)-2-fluoro-4-nitrobenzene (130 mg, 0.49 mmol) obtained in step 1 of Example 32 and commercially available 4-methyl-1,4-diazepan-5-one (82 mg, 0.64 mmol).

ESIMS, (M+H)⁺, m/z: 312.

### (Step 2)

1-(2-(4-Amino-2-fluorophenoxy)ethyl)-4-methyl-1,4-diazepan-5-one (100 mg, total yield of two steps: 72%) was obtained in the same manner as in step 2 of Example 6 using the crude product of 1-(2-(2-fluoro-4-nitrophenoxy)ethyl)-4-methyl-1,4-diazepan-5-one (120 mg) obtained in step 1.

ESIMS, (M+H)⁺, m/z: 282.

### (Step 3)

Compound 33 (38 mg, yield: 40%) was obtained in the same manner as in step 3 of Example 1 using 1-(2-(4-amino-2-fluorophenoxy)ethyl)-4-methyl-1,4-diazepan-5-one (39 mg, 0.14 mmol) obtained in step 2 and 2-amino-N-(4-(((2S,4R)-2-methyl-1-propionyl-1,2,3,4-tetrahydroquinolin-4-yl)amino)phenyl)acetamide (51 mg, 0.14 mmol) obtained in step 2 of Example 1.

ESIMS, (M+H)⁺, m/z: 674. ¹H NMR (DMSO-d₆, δ): 9.68 (s, 1H), 8.82 (s, 1H), 7.44 (dd, J = 14.0, 2.4 Hz, 1H), 7.35-7.20 (m, 4H), 7.20-7.13 (m, 2H), 7.13-7.03 (m, 1H), 7.03-6.95 (m, 1H), 6.61 (d, J = 8.8 Hz, 2H), 6.37 (t, J = 5.6 Hz, 1H), 5.92 (d, J = 8.0 Hz, 1H), 4.85-4.70 (m, 1H), 4.18-4.03 (m, 3H), 3.86 (d, J = 5.6 Hz, 2H), 3.41 (d, J = 4.8 Hz, 2H), 2.90-2.80 (m, 2H), 2.83 (s, 3H), 2.69-2.50 (m, 8H), 2.35-2.20 (m, 1H), 1.30-1.15 (m, 1H), 1.07-0.97 (m, 6H).

### [Example 34]

### N-(4-(((2S,4R)-2-Methyl-1-propionyl-1,2,3,4-tetrahydroquinolin-4-yl)amino)phenyl)-2-(3-(4-(3-(4-methyl-5-oxo-1,4-diazepan-1-yl)propyl)phenyl)ureido)acetamide (compound 34)

### (Step 1)

A crude product of 4-methyl-1-(3-(4-nitrophenyl)propyl)-1,4-diazepan-5-one (110 mg) was obtained in the same manner as in step 2 of Example 5 using commercially available 1-(3-bromopropyl)-4-nitrobenzene (130 mg, 0.53 mmol) and commercially available 4-methyl-1,4-diazepan-5-one (89 mg, 0.69 mmol).

ESIMS, (M+H)⁺, m/z: 292.

### (Step 2)

1-(3-(4-Aminophenyl)propyl)-4-methyl-1,4-diazepan-5-one (95 mg, total yield of two steps: 69%) was obtained in the same manner as in step 2 of Example 6 using the crude product of 4-methyl-1-(3-(4-nitrophenyl)propyl)-1,4-diazepan-5-one (110 mg) obtained in step 1.

ESIMS, (M+H)⁺, m/z: 262.

### (Step 3)

Compound 34 (34 mg, yield: 35%) was obtained in the same manner as in step 3 of Example 1 using 1-(3-(4-aminophenyl)propyl)-4-methyl-1,4-diazepan-5-one (36 mg, 0.14 mmol) obtained in step 2 and 2-amino-N-(4-(((2S,4R)-2-methyl-1-propionyl-1,2,3,4-tetrahydroquinolin-4-yl)amino)phenyl)acetamide (50 mg, 0.14 mmol) obtained in step 2 of Example 1.

ESIMS, (M+H)⁺, m/z: 654. ¹H NMR (DMSO-d₆, δ): 9.67 (s, 1H), 8.69 (s, 1H), 7.35-7.25 (m, 6H), 7.22-7.13 (m, 2H), 7.09-7.02 (m, 2H), 6.61 (d, J = 8.8 Hz, 2H), 6.35 (t, J = 5.6 Hz, 1H), 5.91 (d, J = 8.0 Hz, 1H), 4.81-4.72 (m, 2H), 4.25-4.10 (m, 1H), 3.87 (d, J = 5.6 Hz, 2H), 3.43-3.37 (m, 2H), 2.83 (s, 3H), 2.70-2.50 (m, 4H), 2.50-2.40 (m, 5H), 2.34 (t, J = 7.2 Hz, 2H), 2.34-2.20 (m, 1H), 1.70-1.60 (m, 2H), 1.25-1.10 (m, 1H), 1.07-0.97 (m, 6H).

### [Example 35]

### 2-(3-(4-(2-((S)-4-Acetyl-2-methylpiperazin-1-yl)ethoxy)-3-fluorophenyl)ureido)-N-(4-(((2S,4R)-2-methyl-1-propionyl-1,2,3,4-tetrahydroquinolin-4-yl)amino)phenyl)acetamide (compound 35)

### (Step 1)

A crude product of (S)-1-(4-(2-(2-fluoro-4-nitrophenoxy)ethyl)-3-methylpiperazin-1-yl)ethan-1-one (90 mg) was obtained in the same manner as in step 2 of Example 5 using 1-(2-bromoethoxy)-2-fluoro-4-nitrobenzene (130 mg, 0.49 mmol) obtained in step 2 of Example 32 and commercially available (S)-1-(3-methylpiperazin-1-yl)ethan-1-one hydrochloride (130 mg, 0.74 mmol).

ESIMS, (M+H)⁺, m/z: 326.

### (Step 2)

(S)-1-(4-(2-(4-Amino-2-fluorophenoxy)ethyl)-3-methylpiperazin-1-yl)ethan-1-one (80 mg, total yield of two steps: 55%) was obtained in the same manner as in step 2 of Example 6 using the crude product of (S)-1-(4-(2-(2-fluoro-4-nitrophenoxy)ethyl)-3-methylpiperazin-1-yl)ethan-1-one (90 mg) obtained in step 1.

ESIMS, (M+H)⁺, m/z: 296.

### (Step 3)

Compound 35 (22 mg, yield: 24%) was obtained in the same manner as in step 3 of Example 1 using (S)-1-(4-(2-(4-amino-2-fluorophenoxy)ethyl)-3-methylpiperazin-1-yl)ethan-1-one (40 mg, 0.14 mmol) obtained in step 2 and 2-amino-N-(4-(((2S,4R)-2-methyl-1-propionyl-1,2,3,4-tetrahydroquinolin-4-yl)amino)phenyl)acetamide (50 mg, 0.14 mmol) obtained in step 2 of Example 1.

ESIMS, (M+H)⁺, m/z: 688. ¹H NMR (DMSO-d₆, δ): 9.68 (s, 1H), 8.82 (s, 1H), 7.44 (dd, J = 14.0, 2.4 Hz, 1H), 7.33-7.23 (m, 4H), 7.26-7.13 (m, 2H), 7.13-7.05 (m, 1H), 7.05-6.95 (m, 1H), 6.59 (d, J = 8.8 Hz, 2H), 6.37 (t, J = 5.6 Hz, 1H), 5.92 (d, J = 8.0 Hz, 1H), 4.81-4.69 (m, 1H), 4.18-4.02 (m, 3H), 3.86 (d, J = 5.6 Hz, 3H), 3.61-3.52 (m, 1H), 3.30-3.20 (m, 1H), 3.04-2.75 (m, 3H), 2.74-2.53 (m, 3H), 2.50-2.40 (m, 1H), 2.34-2.17 (m, 2H), 1.98 (s, 3H), 1.26-1.09 (m, 1H), 1.07-0.96 (m, 9H).

### [Example 36]

### 2-((R)-2,4-Dimethyl-3-oxopiperazin-1-yl)-N-(3-fluoro-4-(3-(2-((4-(((2S,4R)-2-methyl-1-propionyl-1,2,3,4-tetrahydroquinolin-4-yl)amino)phenyl)amino)-2-oxoethyl)ureido)phenyl)acetamide (compound 36)

### (Step 1)

Commercially available 3-fluoro-4-nitroaniline (620 mg, 4.0 mmol) was dissolved in acetonitrile (10 mL), then commercially available 2-bromoacetyl bromide (890 mg, 4.4 mmol) and N,N-diisopropylethylamine (1.5 g, 12 mmol) were added to the solution, and the mixture was stirred at room temperature for 1 hour. The reaction mixture was concentrated under reduced pressure to give a crude product of 2-bromo-N-(3-fluoro-4-nitrophenyl)acetamide (890 mg).

ESIMS, (M+H)⁺, m/z: 277.

### (Step 2)

The crude product of 2-bromo-N-(3-fluoro-4-nitrophenyl)acetamide (890 mg) obtained in step 1 was dissolved in acetonitrile (20 mL), then (R)-1,3-dimethylpiperazin-2-one (820 mg, 6.4 mmol) and N,N-diisopropylethylamine (1.3 g, 9.6 mmol) were added to the solution, and the mixture was stirred at room temperature for 2 hours. The reaction mixture was concentrated under reduced pressure, and the obtained residue was purified by reverse-phase silica gel column chromatography (acetonitrile/water =0/100 to 100/0) to give (R)-2-(2,4-dimethyl-3-oxopiperazin-1-yl)-N-(3-fluoro-4-nitrophenyl)acetamide (550 mg, total yield of two steps: 42%).

ESIMS, (M+H)⁺, m/z: 325.

### (Step 3)

(R)-N-(4-Amino-3-fluorophenyl)-2-(2,4-dimethyl-3-oxopiperazin-1-yl)acetamide(330 mg, yield: 66%) was obtained in the same manner as in step 2 of Example 6 using (R)-2-(2,4-dimethyl-3-oxopiperazin-1-yl)-N-(3-fluoro-4-nitrophenyl)acetamide (550 mg, 1.7 mmol) obtained in step 2.

ESIMS, (M+H)⁺, m/z: 295.

### (Step 4)

Compound 36 (29 mg, yield: 24%) was obtained in the same manner as in step 3 of Example 1 using (R)-N-(4-amino-3-fluorophenyl)-2-(2,4-dimethyl-3-oxopiperazin-1-yl)acetamide (50 mg, 0.17 mmol) obtained in step 3 and 2-amino-N-(4-(((2S,4R)-2-methyl-1-propionyl-1,2,3,4-tetrahydroquinolin-4-yl)amino)phenyl)acetamide (62 mg, 0.17 mmol) obtained in step 2 of Example 1.

ESIMS, (M+H)⁺, m/z: 687. ¹H NMR (DMSO-d₆, δ): 9.78 (s, 1H), 9.69 (s, 1H), 8.53 (s, 1H), 7.97 (t, J = 8.8 Hz, 1H), 7.67 (dd, J = 13.6, 2.0 Hz, 1H), 7.34-7.22 (m, 5H), 7.22-7.14 (m, 2H), 6.89 (t, J = 5.6 Hz, 1H), 6.61 (d, J = 8.8 Hz, 2H), 5.91 (d, J = 8.0 Hz, 1H), 4.85-4.70 (m, 1H), 4.20-4.10 (m, 1H), 3.89 (d, J = 5.2 Hz, 2H), 3.50-3.25 (m, 5H), 3.10-3.00 (m, 1H), 2.84 (s, 3H), 2.84-2.76 (m, 1H), 2.66-2.54 (m, 2H), 2.35-2.20 (m, 1H), 1.26 (d, J = 6.8 Hz, 3H), 1.25-1.10 (m, 1H), 1.07-0.97 (m, 6H).

### [Example 37]

### 2-(3-(3-Fluoro-4-(2-((S)-6-oxohexahydropyrrolo[1,2-a]pyrazin-2(1H)-yl)ethoxy)phenyl)ureido)-N-(4-(((2S,4R)-2-methyl-1-propionyl-1,2,3,4-tetrahydroquinolin-4-yl)amino)phenyl)acetamide (compound 37)

### (Step 1)

A crude product of (S)-2-(2-(2-fluoro-4-nitrophenoxy)ethyl)hexahydropyrrolo[1,2-a]pyrazin-6(2H)-one (100 mg) was obtained in the same manner as in step 2 of Example 5 using 1-(2-bromoethoxy)-2-fluoro-4-nitrobenzene (130 mg, 0.49 mmol) obtained in step 1 of Example 32 and commercially available (S)-hexahydropyrrolo[1,2-a]pyrazin-6(2H)-one hydrochloride (130 mg, 0.74 mmol).

ESIMS, (M+H)⁺, m/z: 324.

### (Step 2)

(S)-2-(2-(4-Amino-2-fluorophenoxy)ethyl)hexahydropyrrolo[1,2-a]pyrazin-6(2H)-one (80 mg, total yield of two steps: 55%) was obtained in the same manner as in step 2 of Example 6 using the crude product of (S)-2-(2-(2-fluoro-4-nitrophenoxy)ethyl)hexahydropyrrolo[1,2-a]pyrazin-6(2H)-one (100 mg) obtained in step 1.

ESIMS, (M+H)⁺, m/z: 294.

### (Step 3)

Compound 37 (36 mg, yield: 37%) was obtained in the same manner as in step 3 of Example 1 using (S)-2-(2-(4-amino-2-fluorophenoxy)ethyl)hexahydropyrrolo [1,2-a]pyrazin-6(2H)-one (40 mg, 0.14 mmol) obtained in step 2 and 2-amino-N-(4-(((2S,4R)-2-methyl-1-propionyl-1,2,3,4-tetrahydroquinolin-4-yl)amino)phenyl)acetamide (50 mg, 0.14 mmol) obtained in step 2 of Example 1.

ESIMS, (M+H)⁺, m/z: 686. ¹H NMR (DMSO-d₆, δ): 9.68 (s, 1H), 8.83 (s, 1H), 7.44 (dd, J = 14.0, 2.4 Hz, 1H), 7.34-7.23 (m, 4H), 7.26-7.13 (m, 2H), 7.13-7.05 (m, 1H), 7.01-6.93 (m, 1H), 6.61 (d, J = 8.8 Hz, 2H), 6.37 (t, J = 5.6 Hz, 1H), 5.92 (d, J = 8.0 Hz, 1H), 4.79-4.69 (m, 1H), 4.20-4.10 (m, 3H), 3.86 (d, J = 5.6 Hz, 2H), 3.80-3.71 (m, 1H), 3.65-3.50 (m, 1H), 3.15-3.05 (m, 1H), 3.00-2.90 (m, 1H), 2.85-2.70 (m, 3H), 2.65-2.50 (m, 2H), 2.31-2.13 (m, 3H), 2.13-2.05 (m, 1H), 2.05-1.95 (m, 1H), 1.80 (t, J = 10.8 Hz, 1H), 1.57-1.43 (m, 1H), 1.26-1.09 (m, 1H), 1.07-0.97 (m, 6H).

### [Example 38]

### N-(4-(((2S,4R)-2-Methyl-1-propionyl-1,2,3,4-tetrahydroquinolin-4-yl)amino)phenyl)-2-(3-(4-(2-(4-methyl-5-oxo-1,4-diazepan-1-yl)acetamido)phenyl)ureido)acetamide (compound 38)

### (Step 1)

A crude product of 2-(4-methyl-5-oxo-1,4-diazepan-1-yl)-N-(4-nitrophenyl)acetamide (130 mg) was obtained in the same manner as in step 1 of Example 36 using 2-bromo-N-(4-nitrophenyl)acetamide (130 mg, 0.50 mmol) obtained in step 1 of Example 28 and commercially available 4-methyl-1,4-diazepan-5-one (84 mg, 0.65 mmol).

ESIMS, (M+H)⁺, m/z: 307.

### (Step 2)

N-(4-Aminophenyl)-2-(4-methyl-5-oxo-1,4-diazepan-1-yl)acetamide (100 mg, total yield of two steps: 72%) was obtained in the same manner as in step 2 of Example 6 using the crude product of 2-(4-methyl-5-oxo-1,4-diazepan-1-yl)-N-(4-nitrophenyl)acetamide (130 mg) obtained in step 1.

ESIMS, (M+H)⁺, m/z: 277.

### (Step 3)

Compound 38 (56 mg, yield: 61%) was obtained in the same manner as in step 3 of Example 1 using N-(4-aminophenyl)-2-(4-methyl-5-oxo-1,4-diazepan-1-yl)acetamide (38 mg, 0.14 mmol) obtained in step 2, and 2-amino-N-(4-(((2S,4R)-2-methyl-1-propionyl-1,2,3,4-tetrahydroquinolin-4-yl)amino)phenyl)acetamide (50 mg, 0.14 mmol) obtained in step 2 of Example 1.

ESIMS, (M+H)⁺, m/z: 669. ¹H NMR (DMSO-d₆, δ): 9.69 (s, 1H), 9.60 (s, 1H), 8.75 (s, 1H), 7.49 (d, J = 8.7 Hz, 2H), 7.37-7.22 (m, 6H), 7.22-7.14 (m, 2H), 6.61 (d, J = 8.8 Hz, 2H), 6.36 (t, J = 5.6 Hz, 1H), 5.93 (d, J = 8.0 Hz, 1H), 4.85-4.70 (m, 1H), 4.20-4.10 (m, 1H), 3.87 (d, J = 5.2 Hz, 2H), 3.54-3.47 (m, 2H), 3.21 (s, 2H), 2.86 (s, 3H), 2.75-2.60 (m, 8H), 2.35-2.20 (m, 1H), 1.25-1.10 (m, 1H), 1.08-0.97 (m, 6H).

### [Example 39]

N-(4-(((2S,4R)-2-Methyl-1-propionyl-1,2,3,4-tetrahydroquinolin-4-yl)amino)phenyl)-2-(3-(4-(2-((S)-6-oxohexahydropyrrolo[1,2-a]pyrazin-2(1H)-yl)acetamido)phenyl)ureido)acetamide (compound 39)

### (Step 1)

A crude product of (S)-N-(4-nitrophenyl)-2-(6-oxohexahydropyrrolo[1,2-a]pyrazin-2(1H)-yl)acetamide (120 mg) was obtained in the same manner as in step 2 of Example 5 using 2-bromo-N-(4-nitrophenyl)acetamide (130 mg, 0.48 mmol) obtained in step 1 of Example 28 and commercially available (S)-hexahydropyrrolo[1,2-a]pyrazin-6(2H)-one hydrochloride (130 mg, 0.72 mmol).

ESIMS, (M+H)⁺, m/z: 319.

### (Step 2)

(S)-N-(4-Aminophenyl)-2-(6-oxohexahydropyrrolo[1,2-a]pyrazin-2(1H)-yl)acetamide (90 mg, total yield of two steps: 65%) was obtained in the same manner as in step 2 of Example 6 using the crude product of (S)-N-(4-nitrophenyl)-2-(6-oxohexahydropyrrolo[1,2-a]pyrazin-2(1H)-yl)acetamide (120 mg) obtained in step 1.

ESIMS, (M+H)⁺, m/z: 289.

### (Step 3)

Compound 39 (5.6 mg, yield: 5.7%) was obtained in the same manner as in step 3 of Example 1 using (S)-N-(4-aminophenyl)-2-(6-oxohexahydropyrrolo[1,2-a] pyrazin-2(1H)-yl)acetamide (40 mg, 0.14 mmol) obtained in step 2 and 2-amino-N-(4-(((2S,4R)-2-methyl-1-propionyl-1,2,3,4-tetrahydroquinolin-4-yl)amino)phenyl)acetamide (51 mg, 0.14 mmol) obtained in step 2 of Example 1.

ESIMS, (M+H)⁺, m/z: 681. ¹H NMR (DMSO-d₆, δ): 9.69 (s, 1H), 9.61 (s, 1H), 8.75 (s, 1H), 7.52-7.45 (m, 2H), 7.33-7.22 (m, 6H), 7.20-7.14 (m, 2H), 6.61 (d, J = 8.8 Hz, 2H), 6.40-6.33 (m, 1H), 5.93 (d, J = 8.0 Hz, 1H), 4.76-4.70 (m, 1H), 4.18-4.09 (m, 1H), 3.87 (d, J = 5.2 Hz, 2H), 3.83-3.75 (m, 1H), 3.72-3.62 (m, 1H), 3.23-3.11 (m, 2H), 3.03-2.96 (m, 1H), 2.95-2.80 (m, 2H), 2.65-2.55 (m, 2H), 2.31-2.18 (m, 3H), 2.15-2.05 (m, 2H), 1.94 (t, J = 10.8 Hz, 1H), 1.59-1.47 (m, 1H), 1.25-1.10 (m, 1H), 1.08-0.97 (m, 6H).

### [Example 40]

### 2-(3-(4-(2-((R)-2,4-Dimethyl-3-oxopiperazin-1-yl)ethoxy)phenyl)ureido)-N-(4-(((2S^{*},4R^{*})-6-fluoro-2-methyl-1-propionyl-1,2,3,4-tetrahydroquinolin-4-yl)amino)phenyl)acetamide (compound 40)

### (Step 1)

Commercially available 4-fluoroaniline (9 g, 80 mmol) was dissolved in acetonitrile (150 mL), then bismuth(III) chloride (2.6 g, 8.1 mmol) and N-vinylformamide (12 g, 170 mmol) were added to the solution, and the mixture was stirred at 80°C for 4 hours. The reaction mixture was filtered through celite, and the filtrate was concentrated under reduced pressure. The obtained residue was purified by reverse-phase silica gel column chromatography (acetonitrile/water = 0/100 to 100/0) to give N-((2S*,4R*)-6-fluoro-2-methyl-1,2,3,4-tetrahydroquinolin-4-yl)formamide (4.2 g, yield: 25%).

ESIMS, (M+H)⁺, m/z: 209.

### (Step 2)

N-((2S^{*},4R^{*})-6-Fluoro-2-methyl-1,2,3,4-tetrahydroquinolin-4-yl)formamide (4.2 g, 20 mmol) obtained in step 1 was dissolved in pyridine (25 mL), then propionyl chloride (5.6 g, 61 mmol) was added to the solution, and the mixture was stirred at room temperature for 2 hours. The reaction mixture was concentrated under reduced pressure, and the obtained residue was purified by reverse-phase silica gel column chromatography (acetonitrile/water = 0/100 to 100/0) to give N-((2S*,4R*)-6-fluoro-2-methyl-1-propionyl-1,2,3,4-tetrahydroquinolin-4-yl)formamide (2.8 g, yield: 53%).

ESIMS, (M+H)⁺, m/z: 265.

### (Step 3)

N-((2S^{*},4R^{*})-6-Fluoro-2-methyl-1-propionyl-1,2,3,4-tetrahydroquinolin-4-yl)formamide (2.0 g, 7.6 mmol) obtained in step 2 was dissolved in methanol (25 mL), then hydrochloric acid (6 mol/L, 10 mL) was added to the solution, and the mixture was stirred at 80°C for 2 hours. The reaction mixture was concentrated under reduced pressure, and the obtained residue was purified by reverse-phase silica gel column chromatography (acetonitrile/water = 0/100 to 100/0) to give 1-((2S*,4R*)-4-amino-6-fluoro-2-methyl-3,4-dihydroquinolin-1(2H)-yl)propan-1-one (1.2 g, yield: 67%).

ESIMS, (M+H)⁺, m/z: 237.

### (Step 4)

1-((2S^{*},4R^{*})-4-Amino-6-fluoro-2-methyl-3,4-dihydroquinolin-1(2H)-yl)propan-1-one (2 g, 9 mmol) obtained in step 3 was dissolved in NMP (20 mL), then commercially available 1-fluoro-4-nitrobenzene (2.4 g, 17 mmol) and N,N-diisopropylethylamine (3.3 g, 25 mmol) were added to the solution, and the mixture was stirred at 120°C for 2 days. The reaction mixture was purified by reverse-phase silica gel column chromatography (acetonitrile/water = 0/100 to 100/0) to give 1-((2S*,4R*)-6-fluoro-2-methyl-4-((4-nitrophenyl)amino)-3,4-dihydroquinolin-1(2H)-yl)propan-1-one (510 mg, yield: 17%).

ESIMS, (M+H)⁺, m/z: 358.

### (Step 5)

1-((2S^{*},4R^{*})-4-((4-Aminophenyl)amino)-6-fluoro-2-methyl-3,4-dihydroquinolin-1(2H)-yl)propan-1-one (350 mg, yield: 75%) was obtained in the same manner as in step 2 of Example 6 using 1-((2S^{*},4R^{*})-6-fluoro-2-methyl-4-((4-nitrophenyl)amino)-3,4-dihydroquinolin-1(2H)-yl)propan-1-one (510 mg, 1.4 mmol) obtained in step 4.

ESIMS, (M+H)⁺, m/z: 328.

### (Step 6)

(9H-Fluoren-9-yl)methyl(2-((4-(((2S^{*},4R^{*})-6-fluoro-2-methyl-1-propionyl-1,2,3,4-tetrahydroquinolin-4-yl)amino)phenyl)amino)-2-oxoethyl)carbamate (210 mg, yield: 38%) was obtained in the same manner as in step 1 of Example 1 using
1-((2S^{*},4R^{*})-4-((4-aminophenyl)amino)-6-fluoro-2-methyl-3,4-dihydroquinolin-1(2H)-yl)propan-1-one (300 mg, 0.91 mmol) obtained in step 5 and commercially available N-Fmoc-glycine (300 mg, 1.0 mmol).

ESIMS, (M+H)⁺, m/z: 607.

### (Step 7)

2-Amino-N-(4-(((2S^{*},4R^{*})-6-fluoro-2-methyl-1-propionyl-1,2,3,4-tetrahydroquinolin-4-yl)amino)phenyl)acetamide (110 mg, yield: 69%) was obtained in the same manner as in step 2 of Example 1 using (9H-fluoren-9-yl)methyl(2-((4-(((2S*,4R*)-6-fluoro-2-methyl-1-propionyl-1,2,3,4-tetrahydroquinolin-4-yl)amino)phenyl)amino)-2-oxoethyl)carbamate (250 mg, 0.41 mmol) obtained in step 6.

ESIMS, (M+H)⁺, m/z: 385.

### (Step 8)

Compound 40 (63 mg, yield: 18%) was obtained in the same manner as in step 3 of Example 1 using 2-amino-N-(4-(((2S*,4R*)-6-fluoro-2-methyl-1-propionyl-1,2,3,4-tetrahydroquinolin-4-yl)amino)phenyl)acetamide (200 mg, 0.52 mmol) obtained in step 7 and (R)-4-(2-(4-aminophenoxy)ethyl)-1,3-dimethylpiperazin-2-one (140 mg, 0.52 mmol) synthesized according to the method described in step 3 of Reference Example 40 of WO2021/065980. ESIMS, (M+H)⁺, m/z: 674. ¹H NMR (DMSO-d₆, δ): 9.68 (s, 1H), 8.60 (s, 1H), 7.35-7.24 (m, 5H), 7.12-7.05 (m, 1H), 6.88 (dd, J = 8.0, 2.8 Hz, 1H), 6.82 (d, J = 8.8 Hz, 2H), 6.62 (d, J = 8.8 Hz, 2H), 6.29 (t, J = 5.6 Hz, 1H), 5.93 (d, J = 8.0 Hz, 1H), 4.75-4.69 (m, 1H), 4.25-4.10 (m, 1H), 4.01 (t, J = 5.6 Hz, 2H), 3.86 (d, J = 5.2 Hz, 2H), 3.32-3.24 (m, 2H), 3.20-3.10 (m, 1H), 3.10-3.00 (m, 1H), 3.00-2.91 (m, 1H), 2.80 (s, 3H), 2.78-2.68 (m, 1H), 2.68-2.54 (m, 3H), 2.35-2.20 (m, 1H), 1.23 (d, J = 6.8 Hz, 3H), 1.22-1.10 (m, 1H), 1.06-0.96 (m, 6H).

### [Example 41]

### 2-(3-(2-Fluoro-4-(2-(4-methyl-5-oxo-1,4-diazepan-1-yl)ethoxy)phenyl)ureido)-N-(4-(((2S,4R)-2-methyl-1-propionyl-1,2,3,4-tetrahydroquinolin-4-yl)amino)phenyl)acetamide (compound 41)

### (Step 1)

4-(2-Bromoethoxy)-2-fluoro-1-nitrobenzene(1.0 g, yield: 70%) was obtained in the same manner as in step 1 of Example 30 using commercially available 3-fluoro-4-nitrophenol (800 mg, 5.1 mmol), and 2-bromoethan-1-ol (1.3 g, 10 mmol).

ESIMS, (M+H)⁺, m/z: 264.

### (Step 2)

4-(2-Bromoethoxy)-2-fluoroaniline (230 mg, yield: 91%) was obtained in the same manner as in step 2 of Example 6 using 4-(2-bromoethoxy)-2-fluoro-1-nitrobenzene (270 mg, 1.1 mmol) obtained in step 1.

ESIMS, (M+H)⁺, m/z: 234.

### (Step 3)

2-(3-(4-(2-Bromoethoxy)-2-fluorophenyl)ureido)-N-(4-(((2S,4R)-2-methyl-1-propionyl-1,2,3,4-tetrahydroquinolin-4-yl)amino)phenyl)acetamide (500 mg, yield: 76%) was obtained in the same manner as in step 3 of Example 1 using 4-(2-bromoethoxy)-2-fluoroaniline (230 mg, 0.98 mmol) obtained in step 2 and 2-amino-N-(4-(((2S,4R)-2-methyl-1-propionyl-1,2,3,4-tetrahydroquinolin-4-yl)amino)phenyl)acetamide (360 mg, 0.98 mmol) obtained in step 2 of Example 1.

ESIMS, (M+H)⁺, m/z: 626.

### (Step 4)

Compound 41 (4.2 mg, yield: 6.4%) was obtained in the same manner as in step 2 of Example 5 using 2-(3-(4-(2-bromoethoxy)-2-fluorophenyl)ureido)-N-(4-(((2S,4R)-2-methyl-1-propionyl-1,2,3,4-tetrahydroquinolin-4-yl)amino)phenyl)acetamide (60 mg, 0.096 mmol) obtained in step 3 and commercially available 4-methyl-1,4-diazepan-5-one (18 mg, 0.14 mmol). ESIMS, (M+H)⁺, m/z: 674. ¹H NMR (DMSO-d₆, δ): 9.69 (s, 1H), 8.37 (s, 1H), 7.84 (t, J = 9.2 Hz, 1H), 7.34-7.22 (m, 4H), 7.19-7.14 (m, 2H), 6.87 (dd, J = 13.2, 2.8 Hz, 1H), 6.77 (t, J = 5.6 Hz, 1H), 6.73-6.67 (m, 1H), 6.61 (d, J = 8.8 Hz, 2H), 5.93 (d, J = 8.0 Hz, 1H), 4.80-4.70 (m, 1H), 4.20-4.10 (m, 1H), 4.03 (t, J = 5.6 Hz, 2H), 3.88 (d, J = 5.6 Hz, 2H), 3.50-3.40 (m, 3H), 2.84 (s, 3H), 2.79 (t, J = 5.6 Hz, 2H), 2.68-2.54 (m, 7H), 2.30-2.20 (m, 1H), 1.22-1.09 (m, 1H), 1.07-0.97 (m, 6H).

### [Example 42]

### 2-(3-(2-Fluoro-4-(2-((S)-6-oxohexahydropyrrolo[1,2-a]pyrazin-2(1H)-yl)ethoxy)phenyl)ureido)-N-(4-(((2S,4R)-2-methyl-1-propionyl-1,2,3,4-tetrahydroquinolin-4-yl)amino)phenyl)acetamide (compound 42)

Compound 42 (19 mg, yield: 17%) was obtained in the same manner as in step 2 of Example 5 using 2-(3-(4-(2-bromoethoxy)-2-fluorophenyl)ureido)-N-(4-(((2S,4R)-2-methyl-1-propionyl-1,2,3,4-tetrahydroquinolin-4-yl)amino)phenyl)acetamide (100 mg, 0.16 mmol) obtained in step 3 of Example 41 and commercially available (S)-hexahydropyrrolo[1,2-a]pyrazin-6(2H)-one hydrochloride (85 mg, 0.48 mmol).

ESIMS, (M+H)⁺, m/z: 686. ¹H NMR (DMSO-d₆, δ): 9.69 (s, 1H), 8.37 (s, 1H), 7.85 (t, J = 9.2 Hz, 1H), 7.34-7.22 (m, 4H), 7.17 (d, J = 5.6 Hz, 2H), 6.88 (dd, J = 13.2, 2.8 Hz, 1H), 6.77 (t, J = 5.6 Hz, 1H), 6.74-6.68 (m, 1H), 6.61 (d, J = 8.8 Hz, 2H), 5.93 (d, J = 8.0 Hz, 1H), 4.80-4.70 (m, 1H), 4.18-4.08 (m, 1H), 4.06 (t, J = 5.6 Hz, 2H), 3.88 (d, J = 5.6 Hz, 2H), 3.80-3.70 (m, 1H), 3.60-3.50 (m, 1H), 3.09-3.02 (m, 1H), 3.00-2.91 (m, 1H), 2.82-2.70 (m, 3H), 2.65-2.54 (m, 2H), 2.25-2.12 (m, 3H), 210-2.00 (m, 1H), 2.00-1.90 (m, 1H), 1.90-1.75 (m, 1H), 1.60-1.50 (m, 1H), 1.25-1.10 (m, 1H), 1.07-0.97 (m, 6H).

### [Example 43]

### 2-(3-(4-(2-(4-Acetylpiperazin-1-yl)ethoxy)-2-fluorophenyl)ureido)-N-(4-(((2S,4R)-2-methyl-1-propionyl-1,2,3,4-tetrahydroquinolin-4-yl)amino)phenyl)acetamide (compound 43)

Compound 43 (5.7 mg, yield: 8.8%) was obtained in the same manner as in step 2 of Example 5 using 2-(3-(4-(2-bromoethoxy)-2-fluorophenyl)ureido)-N-(4-(((2S,4R)-2-methyl-1-propionyl-1,2,3,4-tetrahydroquinolin-4-yl)amino)phenyl)acetamide (60 mg, 0.096 mmol) obtained in step 3 of Example 41 and commercially available 1-(piperazin-1-yl)ethan-1-one (18 mg, 0.14 mmol).

ESIMS, (M+H)⁺, m/z: 674. ¹H NMR (DMSO-d₆, δ): 9.69 (s, 1H), 8.38 (s, 1H), 7.84 (t, J = 9.2 Hz, 1H), 7.34-7.22 (m, 4H), 7.22-7.14 (m, 2H), 6.87 (dd, J = 12.8, 2.8 Hz, 1H), 6.80 (t, J = 5.6 Hz, 1H), 6.74-6.67 (m, 1H), 6.61 (d, J = 8.8 Hz, 2H), 5.92 (d, J = 8.0 Hz, 1H), 4.80-4.69 (m, 1H), 4.18-4.08 (m, 1H), 4.05 (t, J = 5.6 Hz, 2H), 3.88 (d, J = 5.6 Hz, 2H), 3.50-3.40 (m, 4H), 2.70 (t, J = 5.6 Hz, 2H), 2.60-2.50 (m, 2H), 2.50-2.38 (m, 4H), 2.35-2.20 (m, 1H), 1.98 (s, 3H), 1.26-1.09 (m, 1H), 1.07-0.97 (m, 6H).

### [Example 44]

### 2-(3-(4-(2-((S)-4-Acetyl-2-methylpiperazin-1-yl)ethoxy)-2-fluorophenyl)ureido)-N-(4-(((2S,4R)-2-methyl-1-propionyl-1,2,3,4-tetrahydroquinolin-4-yl)amino)phenyl)acetamide (compound 44)

Compound 44 (7.4 mg, yield: 2.7%) was obtained in the same manner as in step 2 of Example 5 using 2-(3-(4-(2-bromoethoxy)-2-fluorophenyl)ureido)-N-(4-(((2S,4R)-2-methyl-1-propionyl-1,2,3,4-tetrahydroquinolin-4-yl)amino)phenyl)acetamide (250 mg, 0.40 mmol) obtained in step 3 of Example 41 and commercially available (S)-1-(3-methylpiperazin-1-yl)ethan-1-one hydrochloride (210 mg, 1.2 mmol).

ESIMS, (M+H)⁺, m/z: 688. ¹H NMR (DMSO-d₆, δ): 9.70 (s, 1H), 8.41 (s, 1H), 7.85 (t, J = 9.2 Hz, 1H), 7.34-7.22 (m, 4H), 7.22-7.14 (m, 2H), 6.87 (dd, J = 13.2, 2.8 Hz, 1H), 6.80 (t, J = 5.4 Hz, 1H), 6.74-6.67 (m, 1H), 6.61 (d, J = 8.8 Hz, 2H), 5.93 (d, J = 8.0 Hz, 1H), 4.85-4.70 (m, 1H), 4.18-4.10 (m, 1H), 4.03 (t, J = 5.6 Hz, 2H), 3.90-3.80 (m, 1H), 3.87 (d, J = 5.6 Hz, 2H), 3.60-3.50 (m, 1H), 3.40-3.30 (m, 1H), 3.03-2.77 (m, 3H), 2.70-2.50 (m, 3H), 2.40-2.19 (m, 3H), 1.99 (s, 3H), 1.25-1.10 (m, 1H), 1.08-0.97 (m, 9H).

### [Example 45]

### 2-(3-(4-(3-((R)-2,4-Dimethyl-3-oxopiperazin-1-yl)propyl)-2-fluorophenyl)ureido)-N-(4-(((2S,4R)-2-methyl-1-propionyl-1,2,3,4-tetrahydroquinolin-4-yl)amino)phenyl)acetamide (compound 45)

### (Step 1)

Commercially available 4-bromo-2-fluoro-1-nitrobenzene (440 mg, 2.0 mmol) was dissolved in a mixed solvent of 1,4-dioxane and water (10 mL, 4:1), then commercially available (E)-tert-butyldimethyl((3-(4,4,5,5-tetramethyl-1,3,2-dioxaborolan-2-yl)allyl)oxy)silane (600 mg, 2.0 mmol), a dichloromethane adduct of [1,1'-bis(diphenylphosphino)ferrocene]palladium(II) dichloride (330 mg, 0.40 mmol), and potassium carbonate (550 mg, 4.0 mmol) were added to the solution, and the mixture was stirred at 90°C for 1 hour. The reaction mixture was concentrated under reduced pressure, and the obtained residue was purified by silica gel column chromatography (petroleum ether/ethyl acetate = 10/1) to give (E)-tert-butyl((3-(3-fluoro-4-nitrophenyl)allyl)oxy)dimethylsilane (400 mg, yield: 64%). ESIMS, (M+H)⁺, m/z: 312.

### (Step 2)

(E)-tert-Butyl((3-(3-fluoro-4-nitrophenyl)allyl)oxy)dimethylsilane (400 mg, 1.3 mmol) obtained in step 1 was dissolved in THF (10 mL), then TBAF (500 mg, 1.9 mmol) was added to the solution, and the mixture was stirred at room temperature for 1 hour. The reaction mixture was diluted with water, followed by extraction with ethyl acetate. The obtained organic layer was washed with a saturated aqueous sodium chloride solution, dried over anhydrous sodium sulfate, and concentrated under reduced pressure. The obtained residue was purified by thin-layer chromatography (petroleum ether/ethyl acetate=1/1) to give (E)-3-(3-fluoro-4-nitrophenyl)prop-2-en-1-ol (170 mg, yield: 67%).

ESIMS, (M+H)⁺, m/z: 198.

### (Step 3)

(E)-3-(3-Fluoro-4-nitrophenyl)prop-2-en-1-ol (170 mg, 0.86 mmol) obtained in step 2 was dissolved in dichloromethane (10 mL), then triethylamine (260 mg, 2.6 mmol) and methanesulfonyl chloride (150 mg, 1.3 mmol) were added to the solution, and the mixture was stirred at room temperature for 1 hour. The reaction mixture was diluted with water, followed by extraction with dichloromethane. The obtained organic layer was washed with a saturated aqueous sodium chloride solution, dried over anhydrous sodium sulfate, and concentrated under reduced pressure to give a crude product of (E)-3-(3-fluoro-4-nitrophenyl)allyl methanesulfonate (210 mg).

ESIMS, (M+H)⁺, m/z: 276.

### (Step 4)

(E)-3-(3-Fluoro-4-nitrophenyl)allyl methanesulfonate (210 mg, 0.76 mmol) obtained in step 3 was dissolved in NMP (5 mL), then commercially available (R)-1,3-dimethylpiperazin-2-one (200 mg, 1.5 mmol), potassium iodide (130 mg, 0.76 mmol), and N,N-diisopropylethylamine (300 mg, 2.3 mmol) were added to the solution, and the mixture was stirred at 70°C for 2 hours. The reaction mixture was diluted with water, followed by extraction with ethyl acetate. The obtained organic layer was washed with a saturated aqueous sodium chloride solution, dried over anhydrous sodium sulfate, and concentrated under reduced pressure. The obtained residue was purified by reverse-phase silica gel column chromatography (acetonitrile/water = 0/100 to 60/40) to give (R,E)-4-(3-(3-fluoro-4-nitrophenyl)allyl)-1,3-dimethylpiperazin-2-one (110 mg, total yield of two steps: 42%).

ESIMS, (M+H)⁺, m/z: 308.

### (Step 5)

(R)-4-(3-(4-Amino-3-fluorophenyl)propyl)-1,3-dimethylpiperazin-2-one (25 mg, yield: 55%) was obtained in the same manner as in step 2 of Example 6 using (R,E)-4-(3-(3-fluoro-4-nitrophenyl)allyl)-1,3-dimethylpiperazin-2-one (50 mg, 0.16 mmol) obtained in step 4.

ESIMS, (M+H)⁺, m/z: 280.

### (Step 6)

Compound 45 (10 mg, yield: 17%) was obtained in the same manner as in step 3 of Example 1 using (R)-4-(3-(4-amino-3-fluorophenyl)propyl)-1,3-dimethylpiperazin-2-one (25 mg, 0.080 mmol) obtained in step 5 and 2-amino-N-(4-(((2S,4R)-2-methyl-1-propionyl-1,2,3,4-tetrahydroquinolin-4-yl)amino)phenyl)acetamide (32 mg, 0.08 mmol) obtained in step 2 of Example 1.

ESIMS, (M+H)⁺, m/z: 672. ¹H NMR (DMSO-d₆, δ): 9.69 (s, 1H), 8.52 (s, 1H), 8.46 (s, 1H), 7.96 (t, J = 8.4 Hz, 1H), 7.34-7.22 (m, 4H), 7.22-7.14 (m, 2H), 7.05 (dd, J = 12.6, 2.0 Hz, 1H), 6.96-6.87 (m, 2H), 6.61 (d, J = 8.8 Hz, 2H), 5.91 (d, J = 8.0 Hz, 1H), 4.85-4.62 (m, 1H), 4.20-4.10 (m, 1H), 3.89 (d, J = 5.6 Hz, 2H), 3.35-3.25 (m, 3H), 3.05-2.96 (m, 2H), 2.80 (s, 3H), 2.64-2.54 (m, 3H), 2.33-2.19 (m, 3H), 1.85-1.70 (m, 2H), 1.22-1.09 (m, 4H), 1.07-0.97 (m, 6H).

### [Example 46]

### 2-(3-(4-((E)-3-((R)-2,4-Dimethyl-3-oxopiperazin-1-yl)prop-1-en-1-yl)phenyl)ureido)-N-(4-(((2S,4R)-2-methyl-1-propionyl-1,2,3,4-tetrahydroquinolin-4-yl)amino)phenyl)acetamide (compound 46)

### (Step 1)

(E)-tert-Butyldimethyl((3-(4-nitrophenyl)allyl)oxy)silane (580 mg, yield: 89%) was obtained in the same manner as in step 1 of Example 45 using commercially available 1-bromo-4-nitrobenzene (450 mg, 2.2 mmol) and commercially available (E)-tert-butyldimethyl((3-(4,4,5,5-tetramethyl-1,3,2-dioxaborolan-2-yl)allyl)oxy)silane (660 mg, 2.2 mmol).

ESIMS, (M+H)⁺, m/z: 294.

### (Step 2)

(E)-3-(4-Nitrophenyl)prop-2-en-1-ol (200 mg, yield: 57%) was obtained in the same manner as in step 2 of Example 45 using (E)-tert-butyldimethyl((3-(4-nitrophenyl)allyl)oxy)silane (580 mg, 2.0 mmol) obtained in step 1.

ESIMS, (M+H)⁺, m/z: 180.

### (Step 3)

(E)-3-(4-Nitrophenyl)allyl methanesulfonate (97 mg, yield: 34%) was obtained in the same manner as in step 3 of Example 45 using (E)-3-(4-nitrophenyl)prop-2-en-1-ol (200 mg, 1.1 mmol) obtained in step 2.

ESIMS, (M+H)⁺, m/z: 258.

### (Step 4)

(R,E)-1,3-Dimethyl-4-(3-(4-nitrophenyl)allyl)piperazin-2-one (100 mg, yield: 92%) was obtained in the same manner as in step 4 of Example 45 using (E)-3-(4-nitrophenyl)allyl methanesulfonate (97 mg, 0.37 mmol) obtained in step 3 and commercially available (R)-1,3-dimethylpiperazin-2-one (190 mg, 1.5 mmol).

ESIMS, (M+H)⁺, m/z: 290.

### (Step 5)

(R,E)-1,3-Dimethyl-4-(3-(4-nitrophenyl)allyl)piperazin-2-one (100 mg, 0.34 mmol) obtained in step 4 was dissolved in a mixed solvent of ethanol and water (3 mL, 2:1), then iron (190 mg, 3.5 mmol) and ammonium chloride (180 mg, 3.5 mmol) were added to the solution, and the mixture was stirred at 80°C for 1 hour. The reaction mixture was filtered through celite, and the filtrate was concentrated under reduced pressure. The obtained residue was purified by thin-layer chromatography (dichloromethane/methanol = 5/1) to give (R,E)-4-(3-(4-aminophenyl)allyl)-1,3-dimethylpiperazin-2-one (60 mg, yield: 67%).

ESIMS, (M+H)⁺, m/z: 260.

### (Step 6)

Compound 46 (22 mg, yield: 15%) was obtained in the same manner as in step 3 of Example 1 using (R,E)-4-(3-(4-aminophenyl)allyl)-1,3-dimethylpiperazin-2-one (60 mg, 0.23 mmol) obtained in step 5 and 2-amino-N-(4-(((2S,4R)-2-methyl-1-propionyl-1,2,3,4-tetrahydroquinolin-4-yl)amino)phenyl)acetamide (84 mg, 0.23 mmol) obtained in step 2 of Example 1.

ESIMS, (M+H)⁺, m/z: 652. ¹H NMR (DMSO-d₆, δ): 9.68 (s, 1H), 8.87 (s, 1H), 7.40-7.21 (m, 8H), 7.21-7.13 (m, 2H), 6.61(d, J = 8.8 Hz, 2H), 6.50-6.40 (m, 2H), 6.20-6.10 (m, 1H), 5.91 (d, J = 8.0 Hz, 1H), 4.76-4.67 (m, 1H), 4.25-4.10 (m, 1H), 3.87 (d, J = 5.2 Hz, 2H), 3.45-3.35 (m, 2H), 3.29-3.18 (m, 2H), 3.15-3.05 (m, 2H), 3.05-2.95 (m, 1H), 2.81 (s, 3H), 2.66-2.53 (m, 2H), 2.35-2.20 (m, 1H), 1.25 (d, J = 6.8 Hz, 3H), 1.21-1.09 (m, 1H), 1.07-0.96 (m, 6H).

### [Example 47]

### 2-(3-(4-(2-((R)-2,4-Dimethyl-3-oxopiperazin-1-yl)ethoxy)phenyl)ureido)-N-(4-(((2S^{*},4R^{*})-7-fluoro-2-methyl-1-propionyl-1,2,3,4-tetrahydroquinolin-4-yl)amino)phenyl)acetamide (compound 47)

### (Step 1)

N-((2S^{*},4R^{*})-7-Fluoro-2-methyl-1,2,3,4-tetrahydroquinolin-4-yl)formamide (28 g, yield: 70%) was obtained in the same manner as in step 1 of Example 40 using commercially available 3-fluoroaniline (20 g, 180 mmol) and commercially available N-vinylformamide (26 g, 370 mmol).

ESIMS, (M+H)⁺, m/z: 209.

### (Step 2)

N-((2S^{*},4R^{*})-7-Fluoro-2-methyl-1-propionyl-1,2,3,4-tetrahydroquinolin-4-yl)formamide (24 g, yield: 56%) was obtained in the same manner as in step 2 of Example 40 using N-((2S^{*},4R^{*})-7-fluoro-2-methyl-1,2,3,4-tetrahydroquinolin-4-yl)formamide (34 g, 160 mmol) obtained in step 1.

ESIMS, (M+H)⁺, m/z: 265.

### (Step 3)

1-((2S^{*},4R^{*})-4-Amino-7-fluoro-2-methyl-3,4-dihydroquinolin-1(2H)-yl)propan-1-one (13 g, yield: 59%) was obtained in the same manner as in step 3 of Example 40 using N-((2S*,4R*)-7-fluoro-2-methyl-1-propionyl-1,2,3,4-tetrahydroquinolin-4-yl)formamide (24 g, 92 mmol) obtained in step 2.

ESIMS, (M+H)⁺, m/z: 237.

### (Step 4)

1-((2S^{*},4R^{*})-4-Amino-7-fluoro-2-methyl-3,4-dihydroquinolin-1(2H)-yl)propan-1-one (1.3 g, 5.5 mmol) obtained in step 3 was dissolved in DMF (10 mL), then commercially available 1-fluoro-4-nitrobenzene (2.3 g, 17 mmol) and potassium carbonate (2.3 g, 17 mmol) were added to the solution, and the mixture was stirred at 120°C for 16 hours. The reaction mixture was diluted with ethyl acetate, and the obtained organic layer was washed with water saturated by sodium chloride, dried over anhydrous sodium sulfate, and concentrated under reduced pressure. The obtained residue was purified by silica gel column chromatography (diethyl ether/ethyl acetate=12/1 to 1/2) to give 1-((2S*,4R*)-7-fluoro-2-methyl-4-((4-nitrophenyl)amino)-3,4-dihydroquinolin-1(2H)-yl)propan-1-one (340 mg, yield: 16%).

ESIMS, (M+H)⁺, m/z: 358.

### (Step 5)

1-((2S^{*},4R^{*})-4-((4-Aminophenyl)amino)-7-fluoro-2-methyl-3,4-dihydroquinolin-1(2H)-yl)propan-1-one (280 mg, yield: 90%) was obtained in the same manner as in step 2 of Example 6 using 1-((2S^{*},4R^{*})-7-fluoro-2-methyl-4-((4-nitrophenyl)amino)-3,4-dihydroquinolin-1(2H)-yl)propan-1-one (320 mg, 0.90 mmol) obtained in step 4.

ESIMS, (M+H)⁺, m/z: 328.

### (Step 6)

(9H-Fluoren-9-yl)methyl(2-((4-(((2S^{*},4R^{*})-7-fluoro-2-methyl-1-propionyl-1,2,3,4-tetrahydroquinolin-4-yl)amino)phenyl)amino)-2-oxoethyl)carbamate (500 mg, yield: 89%) was obtained in the same manner as in step 1 of Example 1 using 1-((2S*,4R*)-4-((4-aminophenyl)amino)-7-fluoro-2-methyl-3,4-dihydroquinolin-1(2H)-yl)propan-1-one (280 mg, 0.86 mmol) obtained in step 5 and N-Fmoc-glycine (280 mg, 0.94 mmol). ESIMS, (M+H)⁺, m/z: 607.

### (Step 7)

2-Amino-N-(4-(((2S^{*},4R^{*})-7-fluoro-2-methyl-1-propionyl-1,2,3,4-tetrahydroquinolin-4-yl)amino)phenyl)acetamide (180 mg, 45%) was obtained in the same manner as in step 2 of Example 1 using (9H-fluoren-9-yl)methyl(2-((4-(((2S*,4R*)-7-fluoro-2-methyl-1-propionyl-1,2,3,4-tetrahydroquinolin-4-yl)amino)phenyl)amino)-2-oxoethyl)carbamate (500 mg, 0.82 mmol) obtained in step 6.

ESIMS, (M+H)⁺, m/z: 385.

### (Step 8)

Compound 47 (49 mg, yield: 27%) was obtained in the same manner as in step 3 of Example 1 using 2-amino-N-(4-(((2S*,4R*)-7-fluoro-2-methyl-1-propionyl-1,2,3,4-tetrahydroquinolin-4-yl)amino)phenyl)acetamide (100 mg, 0.27 mmol) obtained in step 7 and (R)-4-(2-(4-aminophenoxy)ethyl)-1,3-dimethylpiperazin-2-one (70 mg, 0.27 mmol) synthesized according to the method described in step 3 of Reference Example 40 of WO2021/065980. ESIMS, (M+H)⁺, m/z: 674. ¹H NMR (DMSO-d₆, δ): 9.68 (s, 1H), 8.63 (s, 1H), 7.34-7.25 (m, 5H), 7.20-7.10 (m, 1H), 7.10-7.00 (m, 1H), 6.83 (d, J = 8.8 Hz, 2H), 6.61 (d, J = 8.8 Hz, 2H), 6.32 (t, J = 5.2 Hz, 1H), 5.95 (d, J = 8.0 Hz, 1H), 4.85-4.70 (m, 1H), 4.20-4.10 (m, 1H), 4.02 (t, J = 5.6 Hz, 2H), 3.86 (d, J = 5.6 Hz, 2H), 3.35-3.25 (m, 2H), 3.20-3.00 (m, 2H), 3.00-2.90 (m, 1H), 2.81 (s, 3H), 2.79-2.59 (m, 3H), 2.35-2.20 (m, 2H), 1.35-1.20 (m, 1H), 1.24 (d, J = 6.8 Hz, 3H), 1.15-1.00 (m, 6H).

### [Example 48]

N-(4-(((2S,4R)-2-Methyl-1-propionyl-1,2,3,4-tetrahydroquinolin-4-yl)amino)phenyl)-2-(3-(4-(2-(6-methylpyridin-3-yl)ethoxy)phenyl)ureido)acetamide (compound 48)

### (Step 1)

2-Methyl-5-(2-(4-nitrophenoxy)ethyl)pyridine (180 mg, yield: 69%) was obtained in the same manner as in step 1 of Example 30 using commercially available 4-nitrophenol (170 mg, 1.0 mmol) and commercially available 2-(6-methylpyridin-3-yl)ethan-1-ol (150 mg, 1.1 mmol).

ESIMS, (M+H)⁺, m/z: 259.

### (Step 2)

4-(2-(6-Methylpyridin-3-yl)ethoxy)aniline (120 mg, yield: 75%) was obtained in the same manner as in step 2 of Example 6 using 2-methyl-5-(2-(4-nitrophenoxy)ethyl)pyridine (180 mg, 0.69 mmol) obtained in step 1.

ESIMS, (M+H)⁺, m/z: 229.

### (Step 3)

Compound 48 (18 mg, yield: 23%) was obtained in the same manner as in step 3 of Example 1 using 4-(2-(6-methylpyridin-3-yl)ethoxy)aniline (30 mg, 0.13 mmol) obtained in step 2 and 2-amino-N-(4-(((2S,4R)-2-methyl-1-propionyl-1,2,3,4-tetrahydroquinolin-4-yl)amino)phenyl)acetamide (48 mg, 0.13 mmol) obtained in step 2 of Example 1.

ESIMS, (M+H)⁺, m/z: 621. ¹H NMR (DMSO-d₆, δ): 9.66 (s, 1H), 8.62 (s, 1H), 8.37 (s, 1H), 7.62 (dd, J = 8.0, 1.6 Hz, 1H), 7.35-7.20 (m, 6H), 7.25-7.13 (m, 3H), 6.81 (d, J = 8.8 Hz, 2H), 6.60 (d, J = 8.8 Hz, 2H), 6.31 (t, J = 5.6 Hz, 1H), 5.91 (d, J = 8.0 Hz, 1H), 4.85-4.72 (m, 1H), 4.10 (t, J = 6.4 Hz, 3H), 3.85 (d, J = 5.2 Hz, 2H), 2.97 (t, J = 6.4 Hz, 2H), 2.70-2.50 (m, 2H), 2.42 (s, 3H), 2.35-2.20 (m, 1H), 1.21-1.08 (m, 1H), 1.07-0.96 (m, 6H).

### [Example 49]

### N-(4-(((2S,4R)-2-Methyl-1-propionyl-1,2,3,4-tetrahydroquinolin-4-yl)amino)phenyl)-2-(3-(4-(2-(5-methylpyridin-3-yl)ethoxy)phenyl)ureido)acetamide (compound 49)

### (Step 1)

Commercially available 3-bromo-5-methylpyridine (1.0 g, 5.8 mmol) was dissolved in a mixed solvent of 1,4-dioxane and water (16 mL, 7:1), then commercially available (E)-2-(2-ethoxyvinyl)-4,4,5,5-tetramethyl-1,3,2-dioxaborolane (1.4 g, 7.0 mmol), a dichloromethane adduct of [1,1'-bis(diphenylphosphino)ferrocene]palladium(II) dichloride (0.47 g, 0.58 mmol), and potassium carbonate (2.4 g, 17 mmol,) were added to the solution, and the mixture was stirred overnight at 80°C. Water was added to the reaction mixture, followed by extraction with ethyl acetate. The obtained organic layer was washed with a saturated aqueous sodium chloride solution, dried over anhydrous sodium sulfate, and concentrated under reduced pressure. The obtained residue was purified by silica gel column chromatography (petroleum ether/ethyl acetate = 1/1) to give (E)-3-(2-ethoxyvinyl)-5-methylpyridine (900 mg, yield: 88%). ESIMS, (M+H)⁺, m/z: 164.

### (Step 2)

(E)-3-(2-Ethoxyvinyl)-5-methylpyridine (900 mg, 5.5 mmol) obtained in step 1 was dissolved in THF (6 mL), then 2 mol/L hydrochloric acid (6 mL) was added to the solution, and the mixture was stirred at 80°C for 2 hours. The reaction mixture was neutralized into pH 7 with a saturated aqueous sodium bicarbonate solution, followed by extraction with a mixed solvent of dichloromethane and methanol (10:1). The obtained organic layer was washed with a saturated aqueous sodium chloride solution, dried over anhydrous sodium sulfate, and concentrated under reduced pressure to give a crude product of 2-(5-methylpyridin-3-yl)acetaldehyde (650 mg).

ESIMS, (M+H)⁺, m/z: 136.

### (Step 3)

The crude product of 2-(5-methylpyridin-3-yl)acetaldehyde (650 mg) obtained in step 2 was dissolved in methanol (10 mL), then sodium borohydride (360 mg, 9.6 mmol) was added to the solution, and the mixture was stirred at room temperature for 1 hour. Water was added to the reaction mixture, followed by extraction with a mixed solvent of dichloromethane and methanol (10:1). The obtained organic layer was dried over anhydrous sodium sulfate and concentrated under reduced pressure to give a crude product of 2-(5-methylpyridin-3-yl)ethan-1-ol (500 mg). ESIMS, (M+H)⁺, m/z: 138.

### (Step 4)

3-Methyl-5-(2-(4-nitrophenoxy)ethyl)pyridine (150 mg, total yield of three steps: 11%) was obtained in the same manner as in step 1 of Example 30 using the crude product of 2-(5-methylpyridin-3-yl)ethan-1-ol (650 mg) obtained in step 3 and commercially available 4-nitrophenol (1.3 g, 9.5 mmol).

ESIMS, (M+H)⁺, m/z: 259.

### (Step 5)

4-(2-(5-Methylpyridin-3-yl)ethoxy)aniline (100 mg, yield: 69%) was obtained in the same manner as in step 5 of Example 46 using 3-methyl-5-(2-(4-nitrophenoxy)ethyl)pyridine (15 mg, 0.58 mmol) of step 4.

ESIMS, (M+H)⁺, m/z: 229.

### (Step 6)

Compound 49 (30 mg, yield: 57%) was obtained in the same manner as in step 3 of Example 1 using 4-(2-(5-methylpyridin-3-yl)ethoxy)aniline (19 mg, 0.082 mmol) obtained in step 5 and 2-amino-N-(4-(((2S,4R)-2-methyl-1-propionyl-1,2,3,4-tetrahydroquinolin-4-yl)amino)phenyl)acetamide (30 mg, 0.082 mmol) obtained in step 2 of Example 1.

ESIMS, (M+H)⁺, m/z: 621. ¹H NMR (DMSO-d₆, δ): 9.66 (s, 1H), 8.63 (s, 1H), 8.32 (d, J = 2.1 Hz, 1H), 8.27 (d, J = 2.1 Hz, 1H), 7.56 (s, 1H), 7.33-7.21 (m, 6H), 7.16 (t, J = 2.8 Hz, 2H), 6.81 (d, J = 8.8 Hz, 2H), 6.60 (d, J = 8.8 Hz, 2H), 6.33 (t, J = 5.6 Hz, 1H), 5.90 (d, J = 8.0 Hz, 1H), 4.78-4.70 (m, 1H), 4.12 (q, J = 6.4 Hz, 3H), 3.85 (d, J = 5.4 Hz, 2H), 2.98 (t, J = 6.8 Hz, 2H), 2.66-2.51 (m, 2H), 2.28 (s, 3H), 2.30-2.20 (m, 1H), 1.26-1.09 (m, 1H), 1.07-0.96 (m, 6H).

### [Example 50]

N,N-Dimethyl-3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzamide (compound 50)

### (Step 1)

Compound 31 (140 mg, 0.21 mmol) obtained from step 3 of Example 31 was dissolved in methanol (2 mL), then a 4 mol/L aqueous sodium hydroxide solution (0.5 mL) was added to the solution, and the mixture was stirred at room temperature for 2 hours. The pH of the reaction mixture was adjusted to 4 using 2 mol/L hydrochloric acid, and the aqueous layer was subjected to extraction with dichloromethane. The organic layer was dried over anhydrous sodium sulfate and concentrated under reduced pressure. The obtained residue was purified by silica gel column chromatography (dichloromethane/methanol = 10/0 to 9/1) to give 3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzoate (120 mg, yield: 81%). ESIMS, (M+H)⁺, m/z: 650.

### (Step 2)

Compound 50 (41 mg, yield: 71%) was obtained in the same manner as in step 1 of Example 1 using 3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzoate (55 mg, 0.085 mmol) obtained in step 1 and a commercially available 30% dimethylamine ethanol solution (0.2 mL).

ESIMS, (M+H)⁺, m/z: 677. ¹H NMR (DMSO-d₆, δ): 9.66 (s, 1H), 8.64 (s, 1H), 8.57(s, 1H), 7.42-7.20 (m, 9H), 7.16 (d, J = 4.0 Hz, 2H), 6.81 (d, J = 8.8 Hz, 2H), 6.60 (d, J = 8.8 Hz, 2H), 6.34 (t, J = 5.6 Hz, 1H), 5.90 (d, J = 8.0 Hz, 1H), 4.85-4.70 (m, 1H), 4.13 (t, J = 6.8 Hz, 3H), 3.85 (d, J = 5.2 Hz, 2H), 3.04 (t, J = 6.8 Hz, 2H), 2.97 (s, 3H), 2.89 (s, 3H), 2.63-2.53 (m, 2H), 2.30-2.15 (m, 1H), 1.25-1.10-1.15 (m, 1H), 1.07-0.96 (m, 6H).

### [Example 51]

### N-Methyl-3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzamide (compound 51)

Compound 51 (20 mg, yield: 35%) was obtained in the same manner as in step 1 of Example 1 using 3-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)benzoate (55 mg, 0.085 mmol) obtained in step 1 of Example 50 and commercially available methylamine hydrochloride (12 mg, 0.17 mmol).

ESIMS, (M+H)⁺, m/z: 663. ¹H NMR (DMSO-d₆, δ): 9.65 (s, 1H), 8.59 (s, 1H), 8.38 (d, J = 4.6 Hz, 1H), 7.77 (s, 1H), 7.67 (d, J = 7.6 Hz, 1H), 7.46 (d, J = 7.6 Hz, 1H), 7.38 (t, J = 7.6 Hz, 1H), 7.35-7.20 (m, 6H), 7.16 (d, J = 4.2 Hz, 2H), 6.82 (d, J = 8.8 Hz, 2H), 6.60 (d, J = 8.8 Hz, 2H), 6.28 (t, J = 5.6 Hz, 1H), 5.90 (d, J = 8.0 Hz, 1H), 4.75-4.69 (m, 1H), 4.14 (t, J = 6.8 Hz, 3H), 3.85 (d, J = 5.4 Hz, 2H), 3.05 (t, J = 6.8 Hz, 2H), 2.78 (d, J = 4.5 Hz, 3H), 2.60-2.50 (m, 2H), 2.35-2.20 (m, 1H), 1.25-1.10 (m, 1H), 1.07-0.96 (m, 6H).

### [Example 52]

### N-(2-Chloro-4-(((2S^{*},4R^{*})-2-methyl-1-propionyl-1,2,3,4-tetrahydroquinolin-4-yl)amino)phenyl)-2-(3-(4-(2-((R)-2,4-dimethyl-3-oxopiperazin-1-yl)ethoxy)phenyl)ureido)acetamide (compound 52)

### (Step 1)

1-((2S^{*},4R^{*})-4-((3-Chloro-4-nitrophenyl)amino)-2-methyl-3,4-dihydroquinolin-1(2H)-yl)propan-1-one (3.0 g, yield: 89%) was obtained in the same manner as in step 4 of Example 47 using 1-[(2S^{*},4R^{*})-4-amino-2-methyl-3,4-dihydroquinolin-1(2H)-yl]propan-1-one (2 g, 9.2 mmol) synthesized according to the method described in step 3 of Reference Example 1 of WO2021/065980 and commercially available 2-chloro-4-fluoro-1-nitrobenzene (3.2 g, 18 mmol).

ESIMS, (M+H)⁺, m/z: 374.

### (Step 2)

1-((2S^{*},4R^{*})-4-((4-Amino-3-chlorophenyl)amino)-2-methyl-3,4-dihydroquinolin-1(2H)-yl)propan-1-one (1.0 g, yield: 37%) was obtained in the same manner as in step 5 of Example 46 using 1-((2S^{*},4R^{*})-4-((3-chloro-4-nitrophenyl)amino)-2-methyl-3,4-dihydroquinolin-1(2H)-yl)propan-1-one (3.0 g, 8.0 mmol) obtained in step 1.

ESIMS, (M+H)⁺, m/z: 344.

### (Step 3)

(9H-Fluoren-9-yl)methyl(2-((2-chloro-4-(((2S^{*},4R^{*})-2-methyl-1-propionyl-1,2,3,4-tetrahydroquinolin-4-yl)amino)phenyl)amino)-2-oxoethyl)carbamate (190 mg, yield: 11%) was obtained in the same manner as in step 1 of Example 1 using 1-((2S*,4R*)-4-((4-amino-3-chlorophenyl)amino)-2-methyl-3,4-dihydroquinolin-1(2H)-yl)propan-1-one (1.0 g, 2.9 mmol) obtained in step 2 and N-Fmoc-glycine (1.0 g, 3.5 mmol).

ESIMS, (M+H)⁺, m/z: 623.

### (Step 4)

2-Amino-N-(2-chloro-4-(((2S^{*},4R^{*})-2-methyl-1-propionyl-1,2,3,4-tetrahydroquinolin-4-yl)amino)phenyl)acetamide (80 mg, yield: 96%) was obtained in the same manner as in step 2 of Example 1 using (9H-fluoren-9-yl)methyl(2-((2-chloro-4-(((2S^{*},4R^{*})-2-methyl-1-propionyl-1,2,3,4-tetrahydroquinolin-4-yl)amino)phenyl)amino)-2-oxoethyl)carbamate (130 mg, 0.21 mmol) obtained in step 3.

ESIMS, (M+H)⁺, m/z: 401.

### (Step 5)

Compound 52 (8.9 mg, yield: 6.4%) was obtained in the same manner as in step 3 of Example 1 using 2-amino-N-(2-chloro-4-(((2S^{*},4R^{*})-2-methyl-1-propionyl-1,2,3,4-tetrahydroquinolin-4-yl)amino)phenyl)acetamide (80 mg, 0.20 mmol) obtained in step 4 and (R)-4-(2-(4-aminophenoxy)ethyl)-1,3-dimethylpiperazin-2-one (53 mg, 0.20 mmol) synthesized according to the method described in step 3 of Reference Example 40 of WO2021/065980.

ESIMS, (M+H)⁺, m/z: 690. ¹H NMR (DMSO-d₆, δ): 9.30 (s, 1H), 8.62 (s, 1H), 7.35-7.22 (m, 5H), 7.22-7.15 (m, 2H), 6.83 (d, J = 8.8 Hz, 2H), 6.75 (d, J = 2.4 Hz, 1H), 6.65-6.58 (m, 1H), 6.38-6.29 (m, 2H), 4.85-4.72 (m, 1H), 4.35-4.21 (m, 1H), 4.01 (t, J = 5.8 Hz, 2H), 3.90 (d, J = 5.4 Hz, 2H), 3.30-3.20 (m, 2H), 3.13-3.00 (m, 2H), 3.00-2.91 (m, 1H), 2.80 (s, 3H), 2.79-2.68 (m, 2H), 2.68-2.50 (m, 2H), 2.35-2.23 (m, 1H), 1.23 (d, J = 6.8 Hz, 3H), 1.23-1.10 (m, 1H), 1.10-1.00 (m, 6H).

### [Example 53]

### N-(4-(((2S^{*},4R^{*})-6-Chloro-2-methyl-1-propionyl-1,2,3,4-tetrahydroquinolin-4-yl)amino)phenyl)-2-(3-(4-(2-((R)-2,4-dimethyl-3-oxopiperazin-1-yl)ethoxy)phenyl)ureido)acetamide (compound 53)

### (Step 1)

N-((2S^{*},4R^{*})-7-Chloro-2-methyl-1,2,3,4-tetrahydroquinolin-4-yl)formamide (15 g, yield: 43%) was obtained in the same manner as in step 1 of Example 40 using commercially available 3-chloroaniline (10 g, 78 mmol) and N-vinylformamide (12 g, 160 mmol).

ESIMS, (M+H)⁺, m/z: 225.

### (Step 2)

N-((2S^{*},4R^{*})-7-Chloro-2-methyl-1-propionyl-1,2,3,4-tetrahydroquinolin-4-yl)formamide (4.3 g, yield: 25%) was obtained in the same manner as in step 2 of Example 40 using N-((2S^{*},4R^{*})-7-chloro-2-methyl-1,2,3,4-tetrahydroquinolin-4-yl)formamide (11 g, 49 mmol) obtained in step 1.

ESIMS, (M+H)⁺, m/z: 281.

### (Step 3)

1-((2S^{*},4R^{*})-4-Amino-7-chloro-2-methyl-3,4-dihydroquinolin-1(2H)-yl) propan-1-one (2.1 g, yield: 52%) was obtained in the same manner as in step 3 of Example 40 using N-((2S*,4R*)-7-chloro-2-methyl-1-propionyl-1,2,3,4-tetrahydroquinolin-4-yl)formamide (4.3 g, 15 mmol) obtained in step 2.

ESIMS, (M+H)⁺, m/z: 253.

### (Step 4)

1-((2S^{*},4R^{*})-6-Chloro-2-methyl-4-((4-nitrophenyl)amino)-3,4-dihydroquinolin-1(2H)-yl)propan-1-one (300 mg, yield: 24%) was obtained in the same manner as in step 4 of Example 47 using 1-((2S^{*},4R^{*})-4-amino-7-chloro-2-methyl-3,4-dihydroquinolin-1(2H)-yl)propan-1-one (800 mg, 3.2 mmol) obtained in step 3 and commercially available 1-fluoro-4-nitrobenzene (2.2 g, 16 mmol). ESIMS, (M+H)⁺, m/z: 374.

### (Step 5)

1-((2S^{*},4R^{*})-4-((4-Aminophenyl)amino)-6-chloro-2-methyl-3,4-dihydroquinolin-1(2H)-yl)propan-1-one (100 mg, yield: 51%) was obtained in the same manner as in step 5 of Example 46 using 1-((2S^{*},4R^{*})-6-chloro-2-methyl-4-((4-nitrophenyl)amino)-3,4-dihydroquinolin-1(2H)-yl)propan-1-one (200 mg, 0.53 mmol) obtained in step 4.

ESIMS, (M+H)⁺, m/z: 344.

### (Step 6)

(9H-Fluoren-9-yl)methyl(2-((4-(((2S^{*},4R^{*})-6-chloro-2-methyl-1-propionyl-1,2,3,4-tetrahydroquinolin-4-yl)amino)phenyl)amino)-2-oxoethyl)carbamate (120 mg, yield: 65%) was obtained in the same manner as in step 1 of Example 1 using 1-((2S*,4R*)-4-((4-aminophenyl)amino)-6-chloro-2-methyl-3,4-dihydroquinolin-1(2H)-yl)propan-1-one (100 mg, 0.26 mmol) obtained in step 5 and N-Fmoc-glycine (77 mg, 0.26 mmol).

ESIMS, (M+H)⁺, m/z: 623.

### (Step 7)

2-Amino-N-(4-(((2S^{*},4R^{*})-6-chloro-2-methyl-1-propionyl-1,2,3,4-tetrahydroquinolin-4-yl)amino)phenyl)acetamide (80 mg, yield: 95%) was obtained in the same manner as in step 2 of Example 1 using (9H-fluoren-9-yl)methyl(2-((4-(((2S*,4R*)-6-chloro-2-methyl-1-propionyl-1,2,3,4-tetrahydroquinolin-4-yl)amino)phenyl)amino)-2-oxoethyl)carbamate (120 mg, 0.18 mmol) obtained in step 6.

ESIMS, (M+H)⁺, m/z: 401.

### (Step 8)

Compound 53 (31 mg, yield: 46%) was obtained in the same manner as in step 3 of Example 1 using 2-amino-N-(4-(((2S*,4R*)-6-chloro-2-methyl-1-propionyl-1,2,3,4-tetrahydroquinolin-4-yl)amino)phenyl)acetamide (40 mg, 0.10 mmol) obtained in step 7 and R)-4-(2-(4-aminophenoxy)ethyl)-1,3-dimethylpiperazin-2-one (26 mg, 0.10 mmol) synthesized according to the method described in step 3 of Reference Example 40 of WO2021/065980. ESIMS, (M+H)⁺, m/z: 690. ¹H NMR (DMSO-d₆, δ): 9.69 (s, 1H), 8.60 (s, 1H), 7.39-7.24 (m, 6H), 7.10 (d, J = 2.4 Hz, 1H), 6.82 (d, J = 8.8 Hz, 2H), 6.62 (d, J = 8.8 Hz, 2H), 6.29 (t, J = 5.4 Hz, 1H), 5.97 (d, J = 8.0 Hz, 1H), 4.72-4.66 (m, 1H), 4.20-4.10 (m, 1H), 4.01 (t, J = 5.8 Hz, 2H), 3.86 (d, J = 5.4 Hz, 2H), 3.28-3.19 (m, 2H), 3.15-3.05 (m, 2H), 3.00-2.90 (m, 2H), 2.80 (s, 3H), 2.78-2.53 (m, 3H), 2.35-2.24 (m, 1H), 1.23 (d, J = 6.8 Hz, 3H), 1.19-1.09 (m, 1H), 1.07-0.96 (m, 6H).

### [Example 54]

### 2-(3-(4-(2-(2,4-Dimethyloxazol-5-yl)ethoxy)phenyl)ureido)-N-(4-(((2S,4R)-2-methyl-1-propionyl-1,2,3,4-tetrahydroquinolin-4-yl)amino)phenyl)acetamide (compound 54)

### (Step 1)

(E)-5-(2-Ethoxyvinyl)-2,4-dimethyloxazole(520 mg, yield: 55%) was obtained in the same manner as in step 1 of Example 49 using commercially available 5-bromo-2,4-dimethyloxazole (1.0 g, 5.7 mmol) and commercially available (E)-2-(2-ethoxyvinyl)-4,4,5,5-tetramethyl-1,3,2-dioxaborolane (1.1 g, 5.7 mmol). ESIMS, (M+H)⁺, m/z: 168.

### (Step 2)

2-(2,4-Dimethyloxazol-5-yl)acetaldehyde (230 mg, yield: 53%) was obtained in the same manner as in step 2 of Example 49 using (E)-5-(2-ethoxyvinyl)-2,4-dimethyloxazole (520 mg, 3.1 mmol) obtained in step 1.

ESIMS, (M+H)⁺, m/z: 140.

### (Step 3)

2-(2,4-Dimethyloxazol-5-yl)ethan-1-ol (40 mg, yield: 17%) was obtained in the same manner as in step 3 of Example 49 using 2-(2,4-dimethyloxazol-5-yl)acetaldehyde (230 mg, 1.7 mmol) obtained in step 2.

ESIMS, (M+H)⁺, m/z: 142.

### (Step 4)

2,4-Dimethyl-5-(2-(4-nitrophenoxy)ethyl)oxazole (35 mg, yield: 47%) was obtained in the same manner as in step 1 of Example 31 using 2-(2,4-dimethyloxazol-5-yl)ethan-1-ol (40 mg, 0.28 mmol) obtained in step 3 and 4-fluoronitrobenzene (40 mg, 0.28 mmol).

ESIMS, (M+H)⁺, m/z: 263.

### (Step 5)

4-(2-(2,4-Dimethyloxazol-5-yl)ethoxy)aniline (28 mg, yield: 90%) was obtained in the same manner as in step 5 of Example 46 using 2,4-dimethyl-5-(2-(4-nitrophenoxy)ethyl)-1,3-oxazole (35 mg, 0.13 mmol) obtained in step 4.

ESIMS, (M+H)⁺, m/z: 233.

### (Step 6)

Compound 54 (21 mg, yield: 28%) was obtained in the same manner as in step 3 of Example 1 using 4-(2-(2,4-dimethyloxazol-5-yl)ethoxy)aniline (28 mg, 0.12 mmol) obtained in step 5 and 2-amino-N-(4-(((2S,4R)-2-methyl-1-propionyl-1,2,3,4-tetrahydroquinolin-4-yl)amino)phenyl)acetamide (44 mg, 0.12 mmol) obtained in step 2 of Example 1.

ESIMS, (M+H)⁺, m/z: 625. ¹H NMR (DMSO-d₆, δ): 9.66 (s, 1H), 8.60 (s, 1H), 7.35-7.20 (m, 6H), 7.16 (d, J = 4.0 Hz, 2H), 6.80 (d, J = 8.8 Hz, 2H), 6.60 (d, J = 8.8 Hz, 2H), 6.29 (t, J = 5.4 Hz, 1H), 5.91 (d, J = 8.0 Hz, 1H), 4.85-4.70 (m, 1H), 4.17-4.02 (m, 3H), 3.85 (d, J = 5.4 Hz, 2H), 2.98 (t, J = 6.4 Hz, 2H), 2.66-2.52 (m, 2H), 2.35-2.20 (m, 1H), 2.29 (s, 3H), 1.98 (s, 3H), 1.26-1.08 (m, 1H), 1.07-0.96 (m, 6H).

### [Examples LP-1 to LP-29]

Compounds LP-1 to LP-29 shown in the tables below were produced.

**[Table 29-1]**

| No. | Structure | Exact Mass |
|---|---|---|
| LP-1 | 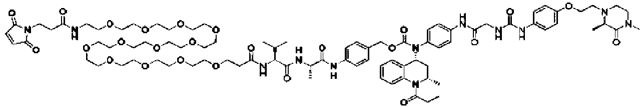 | 1725 |
| LP-2 | 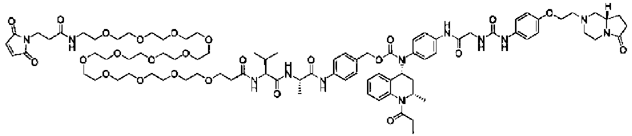 | 1737 |
| LP-3 | 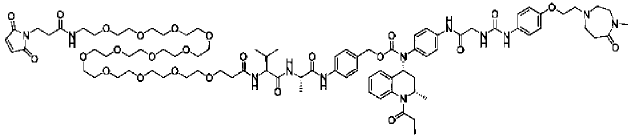 | 1725 |
| LP-4 | 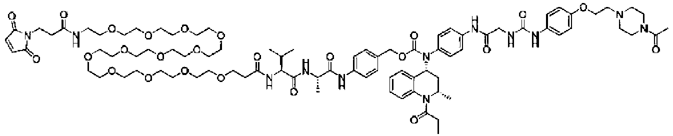 | 1725 |
| LP-5 | 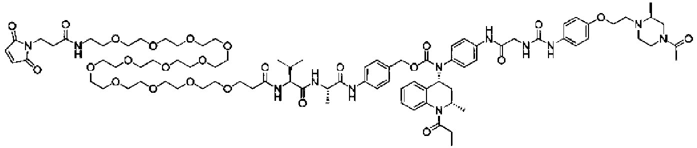 | 1739 |

**[Table 29-2]**

| No. | Structure | Exact Mass |
|---|---|---|
| LP-6 | 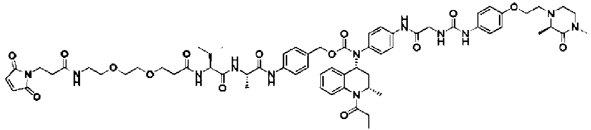 | 1285 |
| LP-7 | 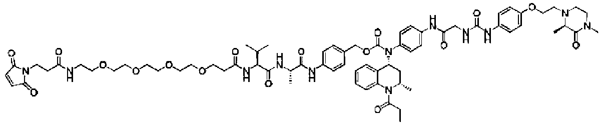 | 1373 |
| LP-8 | 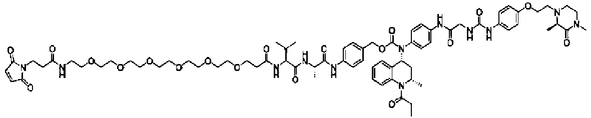 | 1461 |
| LP-9 | 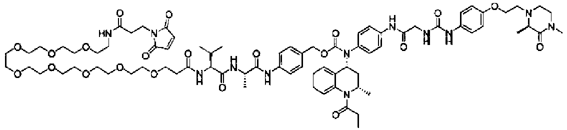 | 1549 |
| LP-10 | 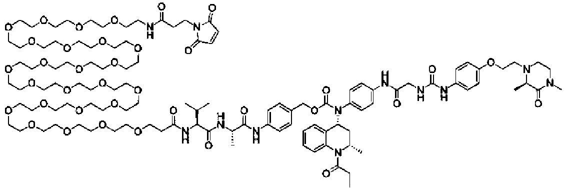 | 2253 |

**[Table 29-3]**

| No. | Structure | Exact Mass |
|---|---|---|
| LP-11 | 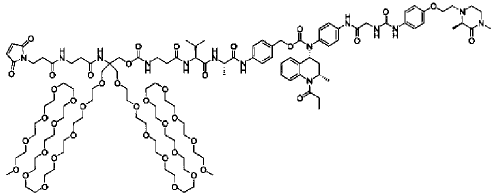 | 2499 |
| LP-12 | 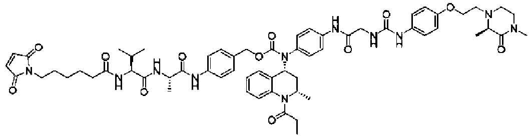 | 1168 |
| LP-13 | 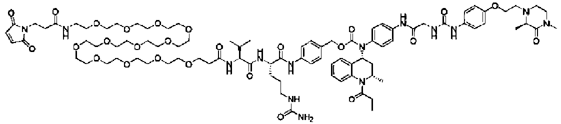 | 1811 |
| LP-14 | 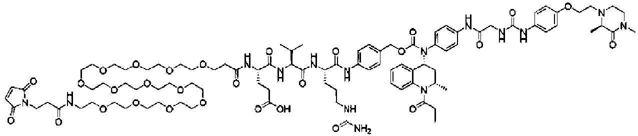 | 1940 |
| LP-15 | 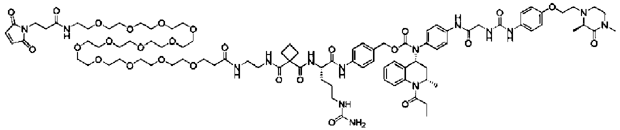 | 1880 |

**[Table 29-4]**

| No. | Structure | Exact Mass |
|---|---|---|
| LP-16 | 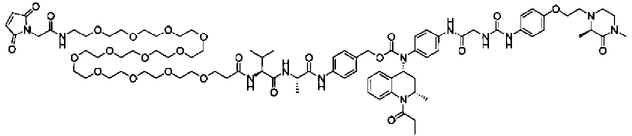 | 1711 |
| LP-17 | 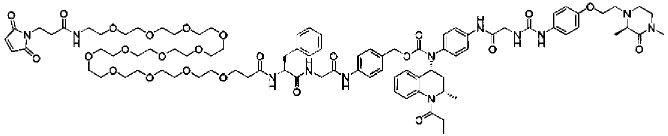 | 1759 |
| LP-18 | 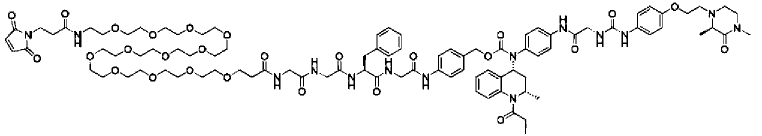 | 1873 |
| LP-19 | 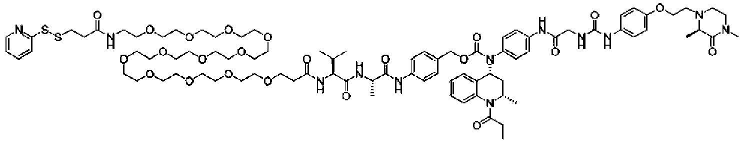 | 1771 |
| LP-20 | 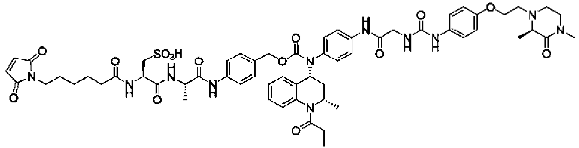 | 1220 |

**[Table 29-5]**

| No. | Structure | Exact Mass |
|---|---|---|
| LP-21 | 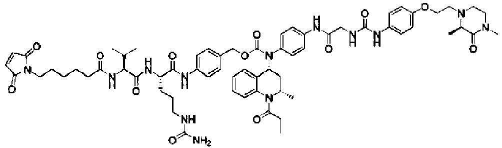 | 1254 |
| LP-22 | 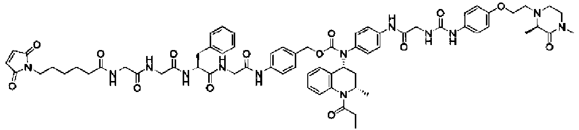 | 1316 |
| LP-23 | 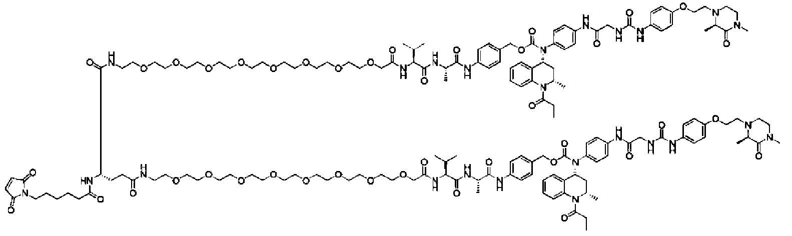 | 3072 |
| LP-24 | 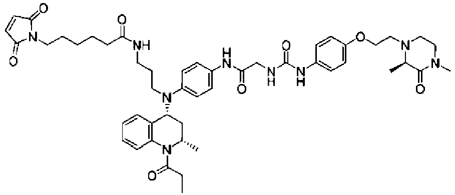 | 905 |

**[Table 29-2]**

| No. | Structure | Exact Mass |
|---|---|---|
| LP-25 | 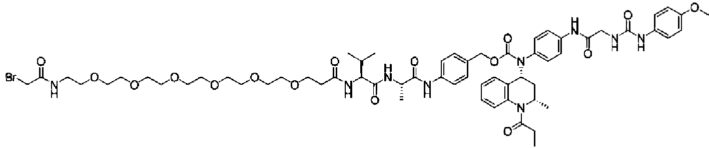 | 1430 |
| LP-26 | 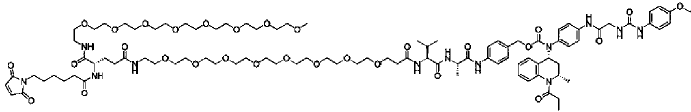 | 2085 |
| LP-27 | 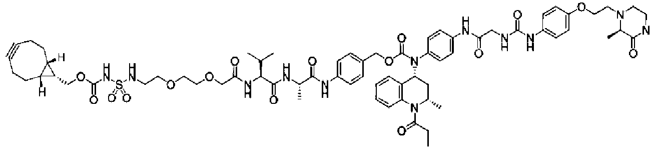 | 1375 |
| LP-28 | 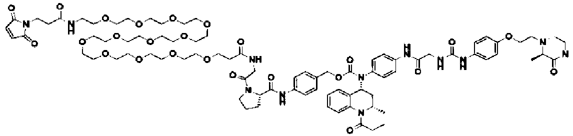 | 1709 |
| LP-29 | 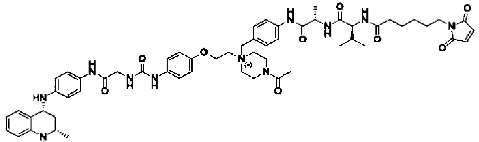 | 1125 |

### [Example LP-1] 4-((2S,5S)-49-(2,5-Dioxo-2,5-dihydro-1H-pyrrol- 1-yl)-5-isopropyl-2-methyl-4,7,47-trioxo- 10,12,16,19,22,25,28,31,34,37,40,43-dodecaoxa-3,6,46- triazanonatetracontanamido)bentyl (4-(2-(3-(4-(2-((R)-2,4- dimethyl-3-oxopiperazin-1- yl)ethoxy)phenyl)ureido)acetamido)phenyl)((2S,4R)-2-methyl-1- propionyl-1,2,3,4-tetrahydroquinolin-4-yl)carbamate (compound LP-1)

### (Step 1)

4-((S)-2-((S)-2-((((9H-Fluoren-9-yl)methoxy)carbonyl)amino)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (1.5 g, yield: 45%) was obtained in the same manner as in step 10 of Example LP-13 using 4-((S)-2-aminopropanamido)benzyl(4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (2.3 g, 2.6 mmol) obtained in step 8 of Example LP-20.

ESIMS, (M+H)⁺, m/z: 1198.

### (Step 2)

4-((S)-2-((S)-2-Amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (1.1 g, yield: 83%) was obtained in the same manner as in step 2 of Example 1 using 4-((S)-2-((S)-2-((((9H-fluoren-9-yl)methoxy)carbonyl)amino)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (1.5 g, 1.3 mmol) obtained in step 1.

ESIMS, (M+H)⁺, m/z: 975.

### (Step 3)

Compound LP-1 (21 mg, yield: 95%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (12 mg, 0.013 mmol) obtained in step 2 and commercially available 2,5-dioxopyrrolidin-1-yl1-(2,5-dioxo-2,5-dihydro-1H-pyrrol-1-yl)-3-oxo-7,10,13,16,19,22,25,28,31,34,37,40-dodecaoxa-4-azatritetracontan-43-oate (13 mg, 0.015 mmol).

ESIMS, (M+H)⁺, m/z: 1726. ¹H NMR (CDCl₃, δ): 9.20 (s, 1H), 8.96 (s, 1H), 7.90 (s, 1H), 7.60 (d, J = 8.2 Hz, 2H), 7.54-7.40 (m, 5H), 7.29-7.26 (m, 5H), 7.12-7.07 (m, 5H), 6.89 (br s, 1H), 6.77 (d, J = 8.6 Hz, 2H), 6.68 (s, 2H), 6.36 (br s, 1H), 5.13-5.10 (m, 1H), 5.02-4.99 (m, 1H), 4.77-4.75 (m, 1H), 4.65-4.63 (m, 1H), 4.28-4.23 (m, 2H), 4.05-4.02 (m, 4H), 3.83-3.76 (m, 4H), 3.65-3.47 (m, 42H), 3.37-3.30 (m, 5H), 3.15-3.14 (m, 1H), 3.03-2.75 (m, 6H), 2.53-2.45 (m, 6H), 2.30-2.21 (m, 4H), 1.75-1.73 (m, 1H), 1.61-1.59 (m, 1H), 1.42-1.40 (m, 4H), 1.27-1.24 (m, 2H), 1.01-0.91 (m, 12H).

### [Example LP-2] 4-((2S,5S)-49-(2,5-Dioxo-2,5-dihydro-1H-pyrrol-1-yl)-5-isopropyl-2-methyl-4,7,47-trioxo-10,13,16,19,22,25,28,31,34,37,40,43-dodecaoxa-3,6,46-triazanonatetracontanamido)benzyl ((2S,4R)-2-methyl-1-propionyl-1,2,3,4-tetrahydroquinolin-4-yl)(4-(2-(3-(4-(2-((S)-6-oxohexahydropyrrolo[1,2-a]pyrazin-2(1H)-yl)ethoxy)phenyl)ureido)acetamido)phenyl)carbamate (compound LP-2)

### (Step 1)

A crude product of 4-((S)-2-aminopropanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (670 mg) was obtained in the same manner as in step 2 of Example 2 using 4-((S)-2-((tert-butoxycarbonyl)amino)propanamido)benzyl(4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (750 mg, 0.83 mmol) obtained in step 5 of Example LP-20.

ESIMS, (M+H)⁺, m/z: 809.

### (Step 2)

4-((S)-2-((S)-2-((tert-Butoxycarbonyl)amino)-3-methylbutanamido)propanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (640 mg, total yield of two steps: 77%) was obtained in the same manner as in step 6 of Example LP-13 using the crude product of 4-((S)-2-aminopropanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (670 mg) obtained in step 1 and N-Boc-L-valine (270 mg, 1.2 mmol). ESIMS, (M+H)⁺, m/z: 1009.

### (Step 3)

4-((S)-2-((S)-2-((tert-Butoxycarbonyl)amino)-3-methylbutanamido)propanamido)benzyl (4-(2-aminoacetamido)phenyl)((2S,4R)-2-methyl-1-propionyl-1,2,3,4-tetrahydroquinolin-4-yl)carbamate (150 mg, yield: 31%) was obtained in the same manner as in step 2 of Example 1 using 4-((S)-2-((S)-2-((tert-butoxycarbonyl)amino)-3-methylbutanamido)propanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (630 mg, 0.63 mmol) obtained in step 2.

ESIMS, (M+H)⁺, m/z: 786.

### (Step 4)

4-((S)-2-((S)-2-((tert-Butoxycarbonyl)amino)-3-methylbutanamido)propanamido)benzyl (4-(2-(3-(4-(2-chloroethoxy)phenyl)ureido)acetamido)phenyl)((2S,4R)-2-methyl-1-propionyl-1,2,3,4-tetrahydroquinolin-4-yl)carbamate (74 mg, yield: 73%) was obtained in the same manner as in step 3 of Example 2 using 4-((S)-2-((S)-2-((tert-butoxycarbonyl)amino)-3-methylbutanamido)propanamido)benzyl (4-(2-aminoacetamido)phenyl)((2S,4R)-2-methyl-1-propionyl-1,2,3,4-tetrahydroquinolin-4-yl)carbamate (81 mg, 0.10 mmol) obtained in step 3 and commercially available 4-(2-chloroethoxy)aniline (27 mg, 0.16 mmol).

ESIMS, (M-Boc+H)⁺, m/z: 884.

### (Step 5)

4-((S)-2-((S)-2-((tert-Butoxycarbonyl)amino)-3-methylbutanamido)propanamido)benzyl ((2S,4R)-2-methyl-1-propionyl-1,2,3,4-tetrahydroquinolin-4-yl)(4-(2-(3-(4-(2-((S)-6-oxohexahydropyrrolo[1,2-a]pyrazin-2(1H)-yl)ethoxy)phenyl)ureido)acetamido)phenyl)carbamate (28 mg, yield: 70%) was obtained in the same manner as in step 2 of Example 5 using 4-((S)-2-((S)-2-((tert-butoxycarbonyl)amino)-3-methylbutanamido)propanamido)benzyl (4-(2-(3-(4-(2-chloroethoxy)phenyl)ureido)acetamido)phenyl)((2S,4R)-2-methyl-1-propionyl-1,2,3,4-tetrahydroquinolin-4-yl)carbamate (36 mg, 0.036 mmol) obtained in step 4 and commercially available (S)-hexahydropyrrolo[1,2-a]pyrazin-6(2H)-one hydrochloride (13 mg, 0.073 mmol).

ESIMS, (M+H)⁺, m/z: 1088.

### (Step 6)

4-((S)-2-((S)-2-Amino-3-methylbutanamido)propanamido)benzyl ((2S,4R)-2-methyl-1-propionyl-1,2,3,4-tetrahydroquinolin-4-yl)(4-(2-(3-(4-(2-((S)-6-oxohexahydropyrrolo[1,2-a]pyrazin-2(1H)-yl)ethoxy)phenyl)ureido)acetamido)phenyl)carbamate (15 mg, yield: 63%) was obtained in the same manner as in step 2 of Example 2 using 4-((S)-2-((S)-2-((tert-butoxycarbonyl)amino)-3-methylbutanamido)propanamido)benzyl ((2S,4R)-2-methyl-1-propionyl-1,2,3,4-tetrahydroquinolin-4-yl)(4-(2-(3-(4-(2-((S)-6-oxohexahydropyrrolo[1,2-a]pyrazin-2(1H)-yl)ethoxy)phenyl)ureido)acetamido)phenyl)carbamate (27 mg, 0.025 mmol) obtained in step 5.

ESIMS, (M+H)⁺, m/z: 988.

### (Step 7)

Compound LP-2 (12 mg, yield: 43%) was obtained in the same manner as in step 12 of Example LP-13 using 4-((S)-2-((S)-2-amino-3-methylbutanamido)propanamido)benzyl ((2S,4R)-2-methyl-1-propionyl-1,2,3,4-tetrahydroquinolin-4-yl)(4-(2-(3-(4-(2-((S)-6-oxohexahydropyrrolo[1,2-a]pyrazin-2(1H)-yl)ethoxy)phenyl)ureido)acetamido)phenyl)carbamate (15 mg, 0.016 mmol) obtained in step 6 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (16 mg, 0.019 mmol).

ESIMS, (M+H)⁺, m/z: 1738. ¹H NMR (CDCl₃, δ): 9.15 (s, 1H), 8.88 (s, 1H), 7.91-7.64 (m, 1H), 7.63-7.56 (m, 2H), 7.56-7.46 (m, 2H), 7.36-7.30 (m, 5H), 7.09 (d, J = 6.8 Hz, 4H), 6.78 (d, J = 8.6 Hz, 2H), 6.67 (s, 2H), 6.32 (br s, 1H), 5.12-5.01 (m, 2H), 4.77-4.74 (m, 1H), 4.64-4.61 (m, 1H), 4.30-4.22 (m, 2H), 4.05-4.02 (m, 4H), 3.82-3.80 (m, 4H), 3.61-3.51 (m, 43H), 3.38-3.37 (m, 2H), 3.22-3.19 (m, 1H), 3.05-3.02 (m, 2H), 2.90 (s, 2H), 2.63-2.60 (m, 5H), 2.53-2.39 (m, 6H), 2.28-2.20 (m, 5H), 2.00-1.98 (m, 1H), 1.75-1.73 (m, 1H), 1.61-1.59 (m, 2H), 1.44-1.42 (m, 3H), 1.07-0.95 (m, 14H), 0.88-0.84 (m, 2H).

### [Example LP-3] 4-((2S,5S)-49-(2,5-Dioxo-2,5-dihydro-1H-pyrrol-1-yl)-5-isopropyl-2-methyl-4,7,47-trioxo-10,13,16,19,22,25,28,31,34,37,40,43-dodecaoxa-3,6,46-triazanonatetracontanamido)benzyl ((2S,4R)-2-methyl-1-propionyl-1,2,3,4-tetrahydroquinolin-4-yl)(4-(2-(3-(4-(2-(4-methyl-5-oxo-1,4-diazepan-1-yl)ethoxy)phenyl)ureido)acetamido)phenyl)carbamate (compound LP-3)

### (Step 1)

4-((S)-2-((S)-2-((tert-Butoxycarbonyl)amino)-3-methylbutanamido)propanamido)benzyl ((2S,4R)-2-methyl-1-propionyl-1,2,3,4-tetrahydroquinolin-4-yl)(4-(2-(3-(4-(2-(4-methyl-5-oxo-1,4-diazepan-1-yl)ethoxy)phenyl)ureido)acetamido)phenyl)carbamate (28 mg, yield: 68%) was obtained in the same manner as in step 2 of Example 5 using 4-((S)-2-((S)-2-((tert-butoxycarbonyl)amino)-3-methylbutanamido)propanamido)benzyl (4-(2-(3-(4-(2-chloroethoxy)phenyl)ureido)acetamido)phenyl)((2S,4R)-2-methyl-1-propionyl-1,2,3,4-tetrahydroquinolin-4-yl)carbamate (37 mg, 0.038 mmol) obtained in step 4 of Example LP-2 and commercially available 4-methyl-1,4-diazepan-5-one (9.7 mg, 0.076 mmol). ESIMS, (M+H)⁺, m/z: 1076.

### (Step 2)

4-((S)-2-((S)-2-Amino-3-methylbutanamido)propanamido)benzyl ((2S,4R)-2-methyl-1-propionyl-1,2,3,4-tetrahydroquinolin-4-yl)(4-(2-(3-(4-(2-(4-methyl-5-oxo-1,4-diazepan-1-yl)ethoxy)phenyl)ureido)acetamido)phenyl)carbamate (9.4 mg, yield: 38%) was obtained in the same manner as in step 2 of Example 2 using 4-((S)-2-((S)-2-((tert-butoxycarbonyl)amino)-3-methylbutanamido)propanamido)benzyl ((2S,4R)-2-methyl-1-propionyl-1,2,3,4-tetrahydroquinolin-4-yl)(4-(2-(3-(4-(2-(4-methyl-5-oxo-1,4-diazepan-1-yl)ethoxy)phenyl)ureido)acetamido)phenyl)carbamate (28 mg, 0.026 mmol) obtained in step 1.

ESIMS, (M+H)⁺, m/z: 975.

### (Step 3)

Compound LP-3 (8.9 mg, yield: 54%) was obtained in the same manner as in step 12 of Example LP-13 using 4-((S)-2-((S)-2-amino-3-methylbutanamido)propanamido)benzyl ((2S,4R)-2-methyl-1-propionyl-1,2,3,4-tetrahydroquinolin-4-yl)(4-(2-(3-(4-(2-(4-methyl-5-oxo-1,4-diazepan-1-yl)ethoxy)phenyl)ureido)acetamido)phenyl)carbamate (9.4 mg, 0.0096 mmol) obtained in step 2 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (10 mg, 0.012 mmol).

ESIMS, (M+H)⁺, m/z: 1726. ¹H NMR (CDCl₃, δ): 9.16 (br s, 1H), 8.88 (br s, 1H), 7.86 (br s, 1H), 7.73-7.70 (m, 1H), 7.59 (d, J = 8.2 Hz, 2H), 7.51 (d, J = 8.6 Hz, 2H), 7.40-7.37 (m, 2H), 7.29-7.27 (m, 2H), 7.12-7.08 (m, 3H), 6.85 (s, 1H), 6.76 (d, J = 8.6 Hz, 2H), 6.68 (s, 2H), 6.34 (br s, 1H), 5.13-5.10 (m, 1H), 5.03-5.00 (m, 1H), 4.77-4.75 (m, 1H), 4.63 (t, J = 7.2 Hz, 1H), 4.34-4.26 (m, 1H), 4.25-4.18 (m, 1H), 4.05-4.04 (m, 4H), 3.82-3.77 (m, 4H), 3.62-3.53 (m, 47H), 3.39-3.36 (m, 2H), 2.98-2.96 (m, 5H), 2.86-2.84 (m, 3H), 2.76-2.74 (m, 2H), 2.66-2.60 (m, 5H), 2.51-2.46 (m, 4H), 2.28-2.22 (m, 2H), 1.75-1.73 (m, 1H), 1.43-1.42 (m, 4H), 1.09-0.95 (m, 12H), 0.89-0.85 (m, 2H).

### [Example LP-4] 4-((2S,5S)-49-(2,5-Dioxo-2,5-dihydro-1H-pyrrol-1-yl)-5-isopropyl-2-methyl-4,7,47-trioxo-10,13,16,19,22,25,28,31,34,37,40,43-dodecaoxa-3,6,46-triazanonatetracontanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-4)

### (Step 1)

4-((S)-2-((tert-Butoxycarbonyl)amino)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (600 mg, yield: 70%) was obtained in the same manner as in step 3 of Example 1 using 4-((S)-2-((tert-butoxycarbonyl)amino)propanamido)benzyl (4-(2-aminoacetamido)phenyl)((2S,4R)-2-methyl-1-propionyl-1,2,3,4-tetrahydroquinolin-4-yl)carbamate (600 mg, 0.87 mmol) obtained in step 6 of Example LP-20 and the crude product of 1-(4-(2-(4-aminophenoxy)ethyl)piperazin-1-yl)ethan-1-one TFA salt (280 mg) obtained in step 2 of Example 2.

ESIMS, (M+H)⁺, m/z: 976.

### (Step 2)

4-((S)-2-Aminopropanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (230 mg, yield: 42%) was obtained in the same manner as in step 2 of Example 2 using 4-((S)-2-((tert-butoxycarbonyl)amino)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (600 mg, 0.62 mmol) obtained in step 1.

ESIMS, (M+H)⁺, m/z: 876.

### (Step 3)

4-((S)-2-((S)-2-((((9H-Fluoren-9-yl)methoxy)carbonyl)amino)-3-methylbutanamido)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (41 mg, yield: 99%) was obtained in the same manner as in step 6 of Example LP-13 using 4-((S)-2-aminopropanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (30 mg, 0.034 mmol) obtained in step 2 and commercially available N-Fmoc-L-valine (23 mg, 0.068 mmol).

ESIMS, (M+H)⁺, m/z: 1198.

### (Step 4)

4-((S)-2-((S)-2-Amino-3-methylbutanamido)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (19 mg, yield: 57%) was obtained in the same manner as in step 2 of Example 1 using 4-((S)-2-((S)-2-((((9H-fluoren-9-yl)methoxy)carbonyl)amino)-3-methylbutanamido)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (41 mg, 0.034 mmol) obtained in step 3.

ESIMS, (M+H)⁺, m/z: 975.

### (Step 5)

Compound LP-4 (11 mg, yield: 65%) was obtained in the same manner as in step 12 of Example LP-13 using 4-((S)-2-((S)-2-amino-3-methylbutanamido)propanamido)benzyl (4-(2-(3-(4-(2-(4-acetylpiperazin-1-yl)ethoxy)phenyl)ureido)acetamido)phenyl)((2S,4R)-2-methyl-1-propionyl-1,2,3,4-tetrahydroquinolin-4-yl)carbamate (9.4 mg, 0.0096 mmol) obtained in step 4 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (10 mg, 0.012 mmol).

ESIMS, (M+H)⁺, m/z: 1727. ¹H NMR (CDCl₃, δ): 9.16 (br s, 1H), 8.87 (br s, 1H), 7.85 (br s, 1H), 7.71 (br s, 1H), 7.60 (d, J = 8.8 Hz, 2H), 7.51 (d, J = 8.8 Hz, 2H), 7.40-7.38 (m, 2H), 7.27-7.26 (m, 2H), 7.10-7.08 (m, 3H), 6.88 (s, 1H), 6.77 (d, J = 9.1 Hz, 2H), 6.68 (s, 2H), 6.33 (br s, 1H), 5.16-5.07 (m, 1H), 5.07-4.91 (m, 1H), 4.78-4.76 (m, 1H), 4.64-4.62 (m, 1H), 4.34-4.26 (m, 1H), 4.24-4.15 (m, 1H), 4.08-4.06 (m, 4H), 3.83-3.81 (m, 4H), 3.69-3.48 (m, 48H), 3.39-3.38 (m, 3H), 2.90-2.87 (m, 2H), 2.67-2.63 (m, 7H), 2.53-2.46 (m, 3H), 2.28-2.23 (m, 2H), 1.74 (s, 1H), 1.44-1.42 (m, 4H), 1.29-1.26 (m, 3H), 1.03-0.91 (m, 16H).

### [Example LP-5] 4-((2S,5S)-49-(2,5-Dioxo-2,5-dihydro-1H-pyrrol-1-yl)-5-isopropyl-2-methyl-4,7,47-trioxo-10,13,16,19,22,25,28,31,34,37,40,43-dodecaoxa-3,6,46-triazanonatetracontanamido)benzyl (4-(2-(3-(4-(2-((S)-4-acetyl-2-methylpiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-5)

### (Step 1)

4-((S)-2-((S)-2-((tert-Butoxycarbonyl)amino)-3-methylbutanamido)propanamido)benzyl (4-(2-(3-(4-(2-((S)-4-acetyl-2-methylpiperazin-1-yl)ethoxy)phenyl)ureido)acetamido)phenyl)((2S,4R)-2-methyl-1-propionyl-1,2,3,4-tetrahydroquinolin-4-yl)carbamate (16 mg, yield: 42%) was obtained in the same manner as in step 2 of Example 5 using 4-((S)-2-((S)-2-((tert-butoxycarbonyl)amino)-3-methylbutanamido)propanamido)benzyl (4-(2-(3-(4-(2-chloroethoxy)phenyl)ureido)acetamido)phenyl)((2S,4R)-2-methyl-1-propionyl-1,2,3,4-tetrahydroquinolin-4-yl)carbamate (35 mg, 0.036 mmol) obtained in step 4 of Example LP-2 and commercially available (S)-1-(3-methylpiperazin-1-yl)ethan-1-one hydrochloride (13 mg, 0.071 mmol).

ESIMS, (M+H)⁺, m/z: 1089.

### (Step 2)

4-((S)-2-((S)-2-Amino-3-methylbutanamido)propanamido)benzyl (4-(2-(3-(4-(2-((S)-4-acetyl-2-methylpiperazin-1-yl)ethoxy)phenyl)ureido)acetamido)phenyl)((2S,4R)-2-methyl-1-propionyl-1,2,3,4-tetrahydroquinolin-4-yl)carbamate (5.1 mg, yield: 34%) was obtained in the same manner as in step 2 of Example 2 using 4-((S)-2-((S)-2-((tert-butoxycarbonyl)amino)-3-methylbutanamido)propanamido)benzyl (4-(2-(3-(4-(2-((S)-4-acetyl-2-methylpiperazin-1-yl)ethoxy)phenyl)ureido)acetamido)phenyl)((2S,4R)-2-methyl-1-propionyl-1,2,3,4-tetrahydroquinolin-4-yl)carbamate (16 mg, 0.015 mmol) obtained in step 1.

ESIMS, (M+H)⁺, m/z: 989.

### (Step 3)

Compound LP-5 (6.6 mg, yield: 74%) was obtained in the same manner as in step 12 of Example LP-13 using 4-((S)-2-((S)-2-amino-3-methylbutanamido)propanamido)benzyl (4-(2-(3-(4-(2-((S)-4-acetyl-2-methylpiperazin-1-yl)ethoxy)phenyl)ureido)acetamido)phenyl)((2S,4R)-2-methyl-1-propionyl-1,2,3,4-tetrahydroquinolin-4-yl)carbamate (5.1 mg, 0.0052 mmol) obtained in step 2 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (5.4 mg, 0.0062 mmol).

ESIMS, (M+H)⁺, m/z: 1740. ¹H NMR (CDCl₃, δ): 9.10 (br s, 1H), 8.85 (br s, 1H), 7.73-7.71 (m, 2H), 7.61 (d, J = 8.2 Hz, 2H), 7.56-7.49 (m, 3H), 7.36-7.29 (m, 4H), 7.12-7.07 (m, 4H), 6.92-6.84 (m, 1H), 6.79 (d, J = 10.0 Hz, 2H), 6.67 (s, 2H), 6.25 (br s, 1H), 5.18-5.07 (m, 1H), 5.05-4.93 (m, 1H), 4.77-4.76 (m, 1H), 4.68-4.60 (m, 1H), 4.34-4.25 (m, 1H), 4.24-4.11 (m, 2H), 4.10-3.95 (m, 3H), 3.83-3.77 (m, 4H), 3.61-3.51 (m, 49H), 3.38-3.35 (m, 2H), 3.18-2.62 (m, 7H), 2.51-2.46 (m, 4H), 2.30-2.18 (m, 2H), 2.09 (s, 3H), 1.76-1.74 (m, 1H), 1.61-1.59 (m, 1H), 1.44-1.42 (m, 4H), 1.13-0.96 (m, 14H), 0.89-0.85 (m, 2H).

### [Example LP-6] 4-((2S,5S)-19-(2,5-Dioxo-2,5-dihydro-1H-pyrrol-1-yl)-5-isopropyl-2-methyl-4,7,17-trioxo-10,13-dioxa-3,6,16-triazanonadecanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-6)

Compound LP-6 (9.9 mg, yield: 75%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 3-(2-(2-(3-(2,5-dioxo-2,5-dihydro-1H-pyrrol-1-yl)propanamido)ethoxy)ethoxy)propanoate (5.2 mg, 0.012 mmol).

ESIMS, (M+H)⁺, m/z: 1286. ¹H NMR (CDCl₃, δ): 9.22 (br s, 1H), 8.96 (br s, 1H), 7.91-7.76 (m, 1H), 7.75-7.66 (m, 1H), 7.59-7.32 (m, 4H), 7.32-7.25 (m, 2H), 7.24-7.18 (m, 2H), 7.17-6.99 (m, 4H), 6.77 (d, J = 8.6 Hz, 2H), 6.64 (br s, 2H), 6.37 (s, 1H), 5.19-5.07 (m, 1H), 5.05-4.89 (m, 1H), 4.77-4.75 (m, 1H), 4.60-4.58 (m, 1H), 4.33-4.29 (m, 2H), 4.05-4.02 (m, 4H), 3.86-3.64 (m, 5H), 3.60-3.40 (m, 5H), 3.40-3.19 (m, 3H), 3.19-3.08 (m, 1H), 3.06-2.97 (m, 1H), 2.94 (s, 3H), 2.92-2.73 (m, 2H), 2.59-2.49 (m, 6H), 2.34-2.16 (m, 2H), 2.14-1.93 (m, 1H), 1.81-1.67 (m, 1H), 1.65-1.52 (m, 1H), 1.42-1.39 (m, 6H), 1.30-1.21 (m, 3H), 1.06-1.03 (m, 6H), 0.92-0.86 (m, 7H).

### [Example LP-7] 4-((2S,5S)-25-(2,5-Dioxo-2,5-dihydro-1H-pyrrol-1-yl)-5-isopropyl-2-methyl-4,7,23-trioxo-10,13,16,19-tetraoxa-3,6,22-triazapentacosanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-7)

Compound LP-7 (7.5 mg, yield: 53%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate (6.3 mg, 0.012 mmol).

ESIMS, (M+H)⁺, m/z: 1374. ¹H NMR (CDCl₃, δ): 9.17 (br s, 1H), 8.95 (br s, 1H), 7.53 (d, J = 8.2 Hz, 2H), 7.45-7.41 (m, 3H), 7.29-7.24 (m, 4H), 7.13-7.12 (m, 1H), 7.06 (d, J = 8.2 Hz, 4H), 6.77 (d, J = 8.6 Hz, 2H), 6.63 (s, 2H), 6.36 (br s, 1H), 5.14-5.11 (m, 1H), 5.01-4.97 (m, 1H), 4.77-4.76 (m, 1H), 4.62-4.60 (m, 1H), 4.29-4.25 (m, 3H), 4.04-4.02 (m, 4H), 3.80-3.75 (m, 3H), 3.64-3.56 (m, 12H), 3.49-3.47 (m, 2H), 3.33-3.28 (m, 5H), 3.16-3.10 (m, 1H), 3.04-2.97 (m, 1H), 2.94 (s, 3H), 2.89-2.83 (m, 1H), 2.81-2.75 (m, 1H), 2.59-2.44 (m, 5H), 2.27-2.20 (m, 2H), 1.92-1.73 (m, 4H), 1.59-1.55 (m, 1H), 1.42-1.39 (m, 6H), 1.30-1.21 (m, 3H), 1.13-1.03 (m, 4H), 0.96-0.93 (m, 4H), 0.91-0.84 (m, 2H).

### [Example LP-8] 4-((2S,5S)-31-(2,5-Dioxo-2,5-dihydro-1H-pyrrol-1-yl)-5-isopropyl-2-methyl-4,7,29-trioxo-10,13,16,19,22,25-hexaoxa-3,6,28-triazahentriacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-8)

Compound LP-8 (8.9 mg, yield: 59%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 1-(2,5-dioxo-2,5-dihydro-1H-pyrrol-1-yl)-3-oxo-7,10,13,16,19,22-hexaoxa-4-azapentacosan-25-oate (7.4 mg, 0.012 mmol).

ESIMS, (M+H)⁺, m/z: 1462. ¹H NMR (CDCl₃, δ): 9.14 (br s, 1H), 8.89 (br s, 1H), 7.80 (br s, 1H), 7.71 (br s, 1H), 7.57-7.53 (m, 2H), 7.47-7.42 (m, 3H), 7.29-7.25 (m, 2H), 7.13-7.12 (m, 1H), 7.07 (d, J = 8.2 Hz, 4H), 6.78 (d, J = 8.6 Hz, 2H), 6.61 (s, 2H), 6.32 (s, 1H), 5.14-5.11 (m, 1H), 5.01-4.98 (m, 1H), 4.77-4.76 (m, 1H), 4.64-4.62 (m, 1H), 4.30-4.21 (m, 2H), 4.05-4.03 (m, 3H), 3.81-3.74 (m, 3H), 3.64-3.46 (m, 20H), 3.36-3.28 (m, 4H), 3.17-3.12 (m, 1H), 3.02-2.99 (m, 1H), 2.94 (s, 3H), 2.88-2.80 (m, 2H), 2.65-2.62 (m, 3H), 2.52-2.44 (m, 4H), 2.30-2.18 (m, 2H), 1.75-1.72 (m, 1H), 1.57 (dd, J = 13.4, 6.6 Hz, 1H), 1.41 (t, J = 8.2 Hz, 6H), 1.33-1.18 (m, 3H), 1.08-1.04 (m, 5H), 0.97-0.94 (m, 6H), 0.89-0.85 (m, 5H).

### [Example LP-9] 4-((2S,5S)-37-(2,5-Dioxo-2,5-dihydro-1H-pyrrol-1-yl)-5-isopropyl-2-methyl-4,7,35-trioxo-10,13,16,19,22,25,28,31-octaoxa-3,6,34-triazaheptatriacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-9)

Compound LP-9 (10 mg, yield: 63%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 1-(2,5-dioxo-2,5-dihydro-1H-pyrrol-1-yl)-3-oxo-7,10,13,16,19,22,25,28-octaoxa-4-azahentriacontan-31-oate (8.5 mg, 0.012 mmol).

ESIMS, (M+H)⁺, m/z: 1550. ¹H NMR (CDCl₃, δ): 9.16 (br s, 1H), 8.88 (br s, 1H), 7.83 (br s, 1H), 7.72 (br s, 1H), 7.57 (d, J = 8.6 Hz, 2H), 7.55-7.46 (m, 2H), 7.40-7.35 (m, 2H), 7.30-7.24 (m, 2H), 7.12-7.08 (m, 4H), 6.95 (s, 1H), 6.78 (d, J = 8.6 Hz, 2H), 6.65 (s, 2H), 6.33 (br s, 1H), 5.13-5.10 (m, 1H), 5.02-4.99 (m, 1H), 4.77-4.75 (m, 1H), 4.64-4.62 (m, 1H), 4.31-4.21 (m, 2H), 4.05-4.03 (m, 4H), 3.80-3.74 (m, 4H), 3.64-3.46 (m, 26H), 3.35-3.32 (m, 5H), 3.14 (dt, J = 20.8, 7.2 Hz, 1H), 3.04-2.98 (m, 1H), 2.94 (s, 3H), 2.84 (tt, J = 18.8, 5.8 Hz, 2H), 2.64-2.61 (m, 6H), 2.52-2.44 (m, 4H), 2.27-2.20 (m, 2H), 1.75-1.72 (m, 1H), 1.42-1.39 (m, 6H), 1.27-1.24 (m, 2H), 1.08-1.04 (m, 6H), 0.99-0.93 (m, 6H), 0.91-0.84 (m, 1H).

### [Example LP-10] 4-((2S,5S)-85-(2,5-Dioxo-2,5-dihydro-1H-pyrrol-1-yl)-5-isopropyl-2-methyl-4,7,83-trioxo-10,13,16,19,22,25,28,31,34,37,40,43,46,49,52,55,58,61,64,67,70 ,73,76,79-tetracosaoxa-3,6,82-triazapentaoctacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-10)

Compound LP-10 (14 mg, yield: 58%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40,43,46,49,52,55,58,61,64,67, 70,73,76-tetracosaoxa-4-azanonaheptacontan-79-oate (17 mg, 0.012 mmol).

ESIMS, (M+H)⁺, m/z: 2255. ¹H NMR (CDCl₃, δ): 9.13 (br s, 1H), 8.86 (br s, 1H), 7.77-7.72 (m, 2H), 7.59 (d, J = 8.2 Hz, 2H), 7.52 (d, J = 8.2 Hz, 2H), 7.38-7.26 (m, 4H), 7.12-7.08 (m, 4H), 6.79 (d, J = 8.6 Hz, 2H), 6.69 (s, 2H), 6.28 (br s, 1H), 5.12-5.02 (m, 2H), 4.76-4.74 (m, 1H), 4.64-4.62 (m, 1H), 4.28-4.22 (m, 2H), 4.06-4.04 (m, 4H), 3.83-3.79 (m, 4H), 3.65-3.51 (m, 88H), 3.38-3.34 (m, 6H), 3.17-3.15 (m, 1H), 3.03-3.01 (m, 1H), 2.95 (s, 3H), 2.87-2.80 (m, 2H), 2.66-2.63 (m, 6H), 2.53-2.46 (m, 3H), 2.27-2.23 (m, 3H), 1.82-1.67 (m, 1H), 1.43-1.40 (m, 6H), 1.29-1.25 (m, 3H), 1.06-1.04 (m, 6H), 0.98-0.96 (m, 6H), 0.87-0.85 (m, 2H).

### [Example LP-11] 4-((49S,52S)-40-(3-(3-(2,5-Dioxo-2,5-dihydro-1H-pyrrol-1-yl)propanamido)propanamido)-49-isopropyl-52-methyl-40-(2,5,8,11,14,17,20,23,26,29,32,35,38-tridecaoxanonatriacontyl)-43,47,50-trioxo-2,5,8,11,14,17,20,23,26,29,32,35,38,42-tetradecaoxa-44,48,51-triazatripentacontan-53-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-11)

Compound LP-11 (13 mg, yield: 51%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 40-(3-(3-(2,5-dioxo-2,5-dihydro-1H-pyrrol-1-yl)propanamido)propanamido)-40-(2,5,8,11,14,17,20,23,26,29,32,35,38-tridecaoxanonatriacontyl)-43-oxo-2,5,8,11,14,17,20,23,26,29,32,35,38,42-tetradecaoxa-44-azaheptatetracontan-47-oate (20 mg, 0.012 mmol).

ESIMS, (M+H)⁺, m/z: 2500. ¹H NMR (CDCl₃, δ): 9.19 (br s, 1H), 9.08 (br s, 1H), 7.83 (br s, 1H), 7.72-7.70 (m, 1H), 7.54-7.44 (m, 6H), 7.30-7.28 (m, 4H), 7.16-7.09 (m, 5H), 6.86 (br s, 1H), 6.80 (d, J = 9.1 Hz, 2H), 6.70-6.66 (m, 2H), 6.27 (br s, 1H), 6.16 (br s, 1H), 5.16-5.13 (m, 1H), 5.02-4.99 (m, 1H), 4.77-4.75 (m, 1H), 4.65-4.62 (m, 1H), 4.44-4.42 (m, 1H), 4.30-4.29 (m, 1H), 4.20-4.18 (m, 1H), 4.06-4.04 (m, 4H), 3.79-3.72 (m, 5H), 3.65-3.53 (m, 92H), 3.38-3.31 (m, 12H), 3.16-3.15 (m, 1H), 3.03-3.01 (m, 1H), 2.95 (s, 3H), 2.89-2.81 (m, 2H), 2.67-2.64 (m, 4H), 2.52-2.48 (m, 6H), 2.30-2.28 (m, 4H), 1.75-1.72 (m, 1H), 1.61-1.59 (m, 1H), 1.44-1.40 (m, 6H), 1.29-1.25 (m, 3H), 1.03-0.92 (m, 12H).

### [Example LP-12] 4-((S)-2-((S)-2-(6-(2,5-Dioxo-2,5-dihydro-1H-pyrrol-1-yl)hexanamido)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-12)

Compound LP-12 (6.7 mg, yield: 56%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.010 mmol) obtained in step 2 of Example LP-1 and commercially available 2,5-dioxopyrrolidin-1-yl 6-(2,5-dioxo-2,5-dihydro-1H-pyrrol-1-yl)hexanoate (3.8 mg, 0.012 mmol).

ESIMS, (M+H)⁺, m/z: 1169. ¹H NMR (CDCl₃, δ): 9.34 (br s, 1H), 9.11 (br s, 1H), 7.91-7.88 (m, 2H), 7.41-7.39 (m, 5H), 7.28-7.25 (m, 2H), 7.14 (d, J = 8.6 Hz, 2H), 7.04-6.90 (m, 4H), 6.76-6.66 (m, 2H), 6.63 (s, 2H), 6.44 (br s, 1H), 5.10-4.97 (m, 2H), 4.75-4.73 (m, 1H), 4.64-4.61 (m, 1H), 4.41-4.37 (m, 1H), 4.08-4.04 (m, 4H), 3.47-3.35 (m, 5H), 3.24-3.22 (m, 1H), 3.10-3.08 (m, 1H), 2.95-2.92 (m, 4H), 2.50-2.46 (m, 2H), 2.34-2.01 (m, 8H), 1.64-1.25 (m, 13H), 1.06-1.01 (m, 6H), 0.88-0.84 (m, 6H).

### [Example LP-13] 4-((2S,5S)-49-(2,5-Dioxo-2,5-dihydro-1H-pyrrol-1-yl)-5-isopropyl-4,7,47-trioxo-2-(3-ureidopropyl)-10,13,16,19,22,25,28,31,34,37,40,43-dodecaoxa-3,6,46-triazanonatetracontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-13)

### (Step 1)

Commercially available (4-aminophenyl)methanol (5.0 g, 40.7 mmol) was dissolved in DMF (50 mL), then N-Boc-L-citrulline (12.3 g, 44.7 mmol), N,N-diisopropylethylamine (21.3 mL, 122.0 mmol), and HATU (23.2 g, 61.0 mmol) were added to the solution, and the mixture was stirred at room temperature for 16 hours. The reaction mixture was concentrated under reduced pressure, and the obtained residue was purified by silica gel column chromatography (dichloromethane/methanol = 90/10) to give tert-butyl (S)-(1-((4-(hydroxymethyl)phenyl-amino)-1-oxo-5-ureidopentan-2-yl)carbamate (6.0 g, yield: 39%).

ESIMS, (M+H)⁺, m/z: 382.

### (Step 2)

1-((2S,4R)-4-((4-Aminophenyl)amino)-2-methyl-3,4-dihydroquinolin-1(2H)-yl)propan-1-one (24 g, 77.6 mmol) synthesized according to the method described in Reference Example 7 of WO2021/065980, 4-nitrophenyl-2-(trimethylsilyl)ethyl carbonate (22 g, 77.6 mmol), and triethylamine (23.6 g, 232.7 mmol) were dissolved in N-methyl-2-pyrrolidone (250 mL), and the solution was stirred at 70°C for 6 hours. Water was added to the reaction mixture, and an organic layer was extracted with dichloromethane, dried over anhydrous sodium sulfate, and concentrated under reduced pressure. The obtained residue was purified by silica gel column chromatography to give 2-(trimethylsilyl)ethyl (4-(((2S,4R)-2-methyl-1-propionyl-1,2,3,4-tetrahydroquinolin-4-yl)amino)phenyl)carbamate (22 g, yield: 63%).

ESIMS, (M+H)⁺, m/z: 454.

### (Step 3)

2-(Trimethylsilyl)ethyl (4-(((2S,4R)-2-methyl-1-propionyl-1,2,3,4-tetrahydroquinolin-4-yl)amino)phenyl)carbamate (0.53 g, 1.18 mmol) obtained in step 2 was dissolved in acetonitrile, then triphosgene 0.70 g, 2.36 mmol) was added to the solution, then triethylamine (3.3 mL, 23.6 mmol) was added dropwise thereto, and the mixture was stirred at room temperature for 30 minutes. 1 mol/L hydrochloric acid was added to the reaction mixture, and an organic layer was extracted with chloroform, dried over anhydrous magnesium sulfate, and concentrated under reduced pressure to give a crude product of 2-(trimethylsilyl)ethyl (4-((chlorocarbonyl)((2S,4R)-2-methyl-1-propionyl-1,2,3,4-tetrahydroquinolin-4-yl)amino)phenyl)carbamate.

### (Step 4)

The whole amount of the crude product of 2-(trimethylsilyl)ethyl (4-((chlorocarbonyl)((2S,4R)-2-methyl-1-propionyl-1,2,3,4-tetrahydroquinolin-4-yl)amino)phenyl)carbamate obtained in step 3 was dissolved in THF (11.8 mL), and the solution was cooled to 0°C. tert-Butyl (S)-(1-((4-(hydroxymethyl)phenyl-amino)-1-oxo-5-ureidopentan-2-yl)carbamate (0.54 g, 1.42 mmol) obtained in step 1 and tert-butoxy potassium (0.27 g, 2.36 mmol) were added thereto, and the mixture was stirred at 0°C for 2 hours. A saturated aqueous sodium bicarbonate solution was added to the reaction mixture, and an organic layer was extracted with chloroform, washed with a saturated aqueous sodium chloride solution, dried over anhydrous magnesium sulfate, and concentrated under reduced pressure. The obtained residue was purified by silica gel column chromatography (chloroform/methanol = 99/1 to 90/10) to give 4-((S)-2-((tert-butoxycarbonyl)amino)-5-ureidopentanamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (0.52 g, total yield of two steps: 51%).

ESIMS, (M+H)⁺, m/z: 862.

### (Step 5)

4-((S)-2-((tert-Butoxycarbonyl)amino)-5-ureidopentanamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (0.70 g, 0.81 mmol) obtained in step 4 was dissolved in DMF (8.1 mL), then cesium fluoride (0.62 g, 4.07 mmol) was added to the solution, and the mixture was stirred at room temperature for 2 hours and stirred at 50°C for 4 hours. The reaction mixture was concentrated under reduced pressure, and the obtained residue was purified by silica gel column chromatography (chloroform/methanol = 99/1 to 85/15) to give 4-((S)-2-((tert-butoxycarbonyl)amino)-5-ureidopentanamido)benzyl (4-aminophenyl)((2S,4R)-2-methyl-1-propionyl-1,2,3,4-tetrahydroquinolin-4-yl)carbamate (0.42 g, yield: 72%).

ESIMS, (M+H)⁺, m/z: 717.

### (Step 6)

Commercially available N-Fmoc-glycine (0.26 g, 0.88 mmol) was dissolved in DMF (5.9 mL), then COMU (0.38 g, 0.88 mmol) and N,N-diisopropylethylamine (0.3 mL, 1.76 mmol) were added to the solution, and the mixture was stirred at room temperature for 15 minutes. 4-((S)-2-((tert-Butoxycarbonyl)amino)-5-ureidopentanamido)benzyl (4-aminophenyl)((2S,4R)-2-methyl-1-propionyl-1,2,3,4-tetrahydroquinolin-4-yl)carbamate (0.42 g, 0.59 mmol) obtained in step 5 was added to the reaction mixture, and the mixture was stirred at room temperature for 1 hour. The reaction mixture was concentrated under reduced pressure to give a crude product of 4-((S)-2-((tert-butoxycarbonyl)amino)-5-ureidopentanamido)benzyl (4-(2-((((9H-fluoren-9-yl)methoxy)carbonyl)aminoacetamido)phenyl)((2S,4R)-2-methyl-1-propionyl-1,2,3,4-tetrahydroquinolin-4-yl)carbamate.

ESIMS, (M+H)⁺, m/z: 995.

### (Step 7)

4-((S)-2-((tert-Butoxycarbonyl)amino)-5-ureidopentanamido)benzyl (4-(2-aminoacetamido)phenyl)((2S,4R)-2-methyl-1-propionyl-1,2,3,4-tetrahydroquinolin-4-yl)carbamate (0.38 g, total yield of two steps: 84%) was obtained in the same manner as in step 2 of Example 1 using the whole amount of the crude product of 4-((S)-2-((tert-butoxycarbonyl)amino)-5-ureidopentanamido)benzyl (4-(2-((((9H-fluoren-9-yl)methoxy)carbonyl)aminoacetamido)phenyl)((2S,4R)-2-methyl-1-propionyl-1,2,3,4-tetrahydroquinolin-4-yl)carbamate obtained in step 6.

ESIMS, (M+H)⁺, m/z: 774.

### (Step 8)

(R)-4-(2-(4-Aminophenoxy)ethyl)-1,3-dimethylpiperazin-2-one (0.14 g, 0.52 mmol) described in Reference Example 40 of WO2021/065980 was dissolved in DMF (10 mL), then triethylamine (0.18 mL, 1.29 mmol) and CDI (84.0 mg, 0.52 mmol) were added to the solution, and the mixture was stirred at room temperature for 4 hours. A solution of 4-((S)-2-((tert-butoxycarbonyl)amino)-5-ureidopentanamido)benzyl (4-(2-aminoacetamido)phenyl)((2S,4R)-2-methyl-1-propionyl-1,2,3,4-tetrahydroquinolin-4-yl)carbamate (0.20 g, 0.26 mmol) obtained in step 7 in DMF (1 mL) was added to the reaction mixture, and the mixture was stirred overnight at room temperature. The reaction mixture was concentrated under reduced pressure, and the obtained residue was purified by silica gel column chromatography (chloroform/methanol = 95/5 to 80/20) to give 4-((S)-2-((tert-butoxycarbonyl)amino)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.22 g, yield: 81%).

ESIMS, (M+H)⁺, m/z: 1063.

### (Step 9)

4-((S)-2-((tert-Butoxycarbonyl)amino)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.12 g, 0.52 mmol) obtained in step 8 was dissolved in chloroform (1.6 mL), then methanol(0.4 mL) and trifluoroacetic acid (2.0 mL, 26.0 mmol) were added to the solution, and the mixture was stirred at room temperature for 2 hours. The reaction mixture was concentrated under reduced pressure, and a 1 mol/L aqueous sodium hydroxide solution was added to the obtained residue, followed by extraction with a 1:1 mixed solution of THF and ethyl acetate. The organic layer was washed with a saturated aqueous sodium chloride solution and dried over magnesium sulfate to give 4-((S)-2-amino-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (69.7 mg, yield: 66%).

ESIMS, (M+H)⁺, m/z: 963.

### (Step 10)

4-((S)-2-Amino-5-ureidopentanamido)benzyl(4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.15 g, 0.16 mmol) obtained in step 9 was dissolved in DMF (1.6 mL), then N,N-diisopropylethylamine (0.14 mL, 0.79 mmol) and N-Fmoc-L-valine (0.13 g, 0.32 mmol) were added to the solution at room temperature, and the mixture was stirred at room temperature for 1 hour. The reaction mixture was concentrated under reduced pressure to give a crude product of 4-((S)-2-((S)-2-((((9H-fluoren-9-yl)methoxy)carbonyl)amino)-3-methoxybutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate.

ESIMS, (M+H)⁺, m/z: 1284.

### (Step 11)

4-((S)-2-((S)-2-Amino-3-methoxybutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (48.3 mg, total yield of two steps: 29%) was obtained in the same manner as in step 2 of Example 1 using the whole amount of the crude product of 4-((S)-2-((S)-2-((((9H-fluoren-9-yl)methoxy)carbonyl)amino)-3-methoxybutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate obtained in step 10.

ESIMS, (M+H)⁺, m/z: 1062.

### (Step 12)

4-((S)-2-((S)-2-Amino-3-methoxybutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (8.4 mg, 0.0079 mmol) obtained in step 11 was dissolved in DMF (0.5 mL), then triethylamine (0.003 mL, 0.02 mmol) and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (8.2 mg, 0.0095 mmol) were added to the solution, and the mixture was stirred at room temperature for 1 hour. The reaction mixture was purified by silica gel column chromatography (chloroform/methanol = 97/3 to 85/15) to give compound LP-13 (9.4 mg, yield: 66%).

ESIMS, (M+H)⁺, m/z: 1812. ¹H NMR (CD₃OD, δ): 7.64-7.51 (m, 4H), 7.41-7.23 (m, 6H), 7.23-7.15 (m, 4H), 6.88 (d, J = 9.4 Hz, 2H), 6.81 (s, 2H), 5.09-5.06 (m, 2H), 4.75-4.71 (m, 1H), 4.52-4.48 (m, 1H), 4.21 (t, J = 7.2 Hz, 1H), 4.14 (t, J = 5.2 Hz, 2H), 4.00 (s, 2H), 3.77-3.75 (m, 6H), 3.64-3.58 (m, 48H), 3.49 (t, J = 5.6 Hz, 2H), 3.44-3.41 (m, 3H), 3.13-3.10 (m, 1H), 3.03-2.99 (m, 1H), 2.94 (s, 3H), 2.56 (t, J = 5.8 Hz, 3H), 2.55-2.46 (m, 3H), 2.45 (t, J = 7.0 Hz, 3H), 2.36-2.23 (m, 2H), 2.17-2.08 (m, 1H), 1.94-1.88 (m, 1H), 1.82-1.70 (m, 1H), 1.64-1.54 (m, 2H), 1.42 (d, J = 6.7 Hz, 3H), 1.38-1.37 (m, 1H), 0.99 (d, J = 6.3 Hz, 6H), 0.98 (t, J = 6.5 Hz, 3H), 0.98 (d, J = 6.7 Hz, 3H), 0.92-0.85 (m, 1H).

### [Example LP-14] (S)-45-(((S)-1-(((S)-1-((4-((((4-(2-(3-(4-(2-((R)-2,4-Dimethyl-3-oxopiperazin-1-yl)ethoxy)phenyl)ureido)acetamido)phenyl)((2S,4R) -2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)carbamoyl)-1-(2,5-dioxo-2,5-dihydro-1H-pyrrol-1-yl)-3,43-dioxo-7,10,13,16,19,22,25,28,31,34,37,40-dodecaoxa-4,44-diazaoctatetracontan-48-oic acid (compound LP-14)

### (Step 1)

A crude product of tert-butyl (S)-4-((((9H-fluoren-9-yl)methoxy)carbonyl)amino)-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoate was obtained in the same manner as in step 6 of Example LP-13 using 4-((S)-2-((S)-2-amino-3-methoxybutanamido)-5-ureidopentanamido)benzyl(4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate(21.3 mg, 0.02 mmol) obtained in step 11 of Example LP-13 and commercially available tert-butyl (S)-4-((((9H-fluoren-9-yl)methoxy)carbonyl)amino)-5-oxopentanoate (17.1 mg, 0.04 mmol).

ESIMS, (M+H)⁺, m/z: 1469.

### (Step 2)

tert-Butyl (S)-4-((((9H-fluoren-9-yl)methoxy)carbonyl)amino)-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoate (28.0 mg, total yield of two steps: quantitative) was obtained in the same manner as in step 2 of Example 1 using the whole amount of the crude product of tert-butyl (S)-4-((((9H-fluoren-9-yl)methoxy)carbonyl)amino)-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoate obtained in step 1.

ESIMS, (M+H)⁺, m/z: 1247.

### (Step 3)

(S)-4-((((9H-Fluoren-9-yl)methoxy)carbonyl)amino)-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoate (22.0 mg, 0.02 mmol) obtained in step 2 was dissolved in chloroform (0.6 mL), then TFA (0.3 mL, 3.89 mmol) was added to the solution, and the mixture was stirred at room temperature for 5 hours. The reaction mixture was concentrated under reduced pressure, and the obtained residue was purified by reverse-phase HPLC (acetonitrile/10 mmol/L aqueous ammonium bicarbonate solution = 25/75 to 35/65) to give (S)-4-amino-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoic acid (9.2 mg, yield: 44%).

ESIMS, (M+H)⁺, m/z: 1191.

### (Step 4)

Compound LP-14 (13.4 mg, yield: 89%) was obtained in the same manner as in step 12 of Example LP-13 using (S)-4-amino-5-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxo-5-ureidopentan-2-yl)amino)-3-methyl-1-oxobutan-2-yl)amino)-5-oxopentanoic acid (9.2 mg, 0.0077 mmol) obtained in step 3.

ESIMS, (M+H)⁺, m/z: 1941. ¹H NMR (CD₃OD, δ): 7.58-7.56 (m, 4H), 7.34-7.33 (m, 3H), 7.28-7.25 (m, 3H), 7.19-7.17 (m, 4H), 6.87-6.85 (m, 2H), 6.81 (s, 2H), 5.12-5.03 (m, 2H), 4.76-4.70 (m, 1H), 4.47 (dd, J = 9.4, 4.9 Hz, 1H), 4.38 (dd, J = 8.3, 5.6 Hz, 1H), 4.19 (d, J = 6.7 Hz, 1H), 4.08 (t, J = 5.8 Hz, 2H), 4.00 (s, 2H), 3.78-3.68 (m, 7H), 3.64-3.57 (m, 48H), 3.50-3.48 (m, 2H), 3.38-3.35 (m, 2H), 3.18-3.12 (m, 1H), 3.05-3.00 (m, 1H), 2.93 (s, 3H), 2.90-2.89 (m, 1H), 2.82-2.79 (m, 1H), 2.52 (t, J = 6.3 Hz, 2H), 2.47-2.45 (m, 3H), 2.36-2.25 (m, 2H), 2.21-2.05 (m, 2H), 2.00-1.87 (m, 2H), 1.83-1.75 (m, 1H), 1.63-1.53 (m, 2H), 1.36 (d, J = 7.6 Hz, 3H), 1.30-1.27 (m, 1H), 0.99 (t, J = 8.5 Hz, 3H), 0.98 (t, J = 5.6 Hz, 3H), 0.97 (d, J = 6.7 Hz, 6H), 0.93-0.87 (m, 1H).

### [Example LP-15] 4-((S)-2-(1-((46-(2,5-Dioxo-2,5-dihydro-1H-pyrrol-1-yl)-4,44-dioxo-7,10,13,16,19,22,25,28,31,34,37,40-dodecaoxa-3,43-diazahexatetracontyl)carbamoyl)cyclobutane-1-carboxamido)-5-ureidopentanamido)benzyl (4-(2-(3-(4-(2-((R)-2,4-dimethyl-3-oxapiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-15)

### (Step 1)

Commercially available tert-butyl (2-aminoethyl)carbamate (0.25 g, 1.56 mmol) and commercially available 1-(ethoxycarbonyl)cyclobutane-1-carboxylic acid (0.32 g, 1.87 mmol) were dissolved in THF (15.6 mL), then N,N-diisopropylethylamine (1.36 mL, 7.80 mmol) and HATU (0.71 g, 1.87 mmol) were added to the solution at room temperature, and the mixture was stirred at room temperature for 3 days. The reaction mixture was concentrated under reduced pressure, and water was added to the residue, followed by extraction with chloroform. The obtained organic layer was sequentially washed with 1 mol/L hydrochloric acid and a 1 mol/L aqueous sodium hydroxide solution, dried over anhydrous magnesium sulfate, and concentrated under reduced pressure to give a crude product of ethyl 1-((2-((tert-butoxycarbonyl)amino)ethyl)carbamoyl)cyclobutane-1-carboxylate (0.78 g).

ESIMS, (M-H)⁻, m/z: 313.

### (Step 2)

The crude product of ethyl 1-((2-((tert-butoxycarbonyl)amino)ethyl)carbamoyl)cyclobutane-1-carboxylate (0.78 g) obtained in step 1 was dissolved in ethanol (15.6 mL), then a 4 mol/L aqueous sodium hydroxide solution (11.7 mL, 46.8 mmol) was added to the solution at room temperature, and the mixture was stirred at room temperature for 2 hours. The reaction mixture was concentrated under reduced pressure, then water was added to the residue, and the aqueous layer was washed with chloroform. The obtained aqueous layer was cooled to 0°C, then 6 mol/L hydrochloric acid(7.0 mL) was added thereto, and 1 mol/L hydrochloric acid was added thereto. The aqueous layer was subjected to extraction with chloroform, and the organic layer was dried over anhydrous magnesium sulfate and concentrated under reduced pressure to give 1-((2-((tert-butoxycarbonyl)amino)ethyl)carbamoyl)cyclobutane-1-carboxylic acid (0.43 g, total yield of two steps: 96%).

ESIMS, (M-H)⁻, m/z: 285.

### (Step 3)

4-((S)-2-(1-((2-((tert-Butoxycarbonyl)amino)ethyl)carbamoyl)cyclobutane-1-carboxamido)-5-ureidopentanamido)benzyl (4-(2-(3-(4-(2-((R)-2,4-dimethyl-3-oxapiperazin-1-yl)ethoxy)phenyl)ureido)acetamido)phenyl)((2S,4R)-2-methyl-1-propionyl-1,2,3,4-tetrahydroquinolin-4-yl)carbamate (33.5 mg, yield: 52%) was obtained in the same manner as in step 6 of Example LP-13 using 1-((2-((tert-butoxycarbonyl)amino)ethyl)carbamoyl)cyclobutane-1-carboxylic acid (30.0 mg, 0.10 mmol) obtained in step 2 and 4-((S)-2-amino-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (50.0 mg, 0.05 mmol) obtained in step 9 of Example LP-13.

ESIMS, (M+H)⁺, m/z: 1231.

### (Step 4)

4-((S)-2-(1-((2-Aminoethyl)carbamoyl)cyclobutane-1-carboxamido)-5-ureidopentanamido)benzyl (4-(2-(3-(4-(2-((R)-2,4-dimethyl-3-oxapiperazin-1-yl)ethoxy)phenyl)ureido)acetamido)phenyl)((2S,4R)-2-methyl-1-propionyl-1,2,3,4-tetrahydroquinolin-4-yl)carbamate (16.5 mg, yield: 54%) was obtained in the same manner as in step 3 of Example LP-14 using 4-((S)-2-(1-((2-((tert-butoxycarbonyl)amino)ethyl)carbamoyl)cyclobutane-1-carboxamido)-5-ureidopentanamido)benzyl (4-(2-(3-(4-(2-((R)-2,4-dimethyl-3-oxapiperazin-1-yl)ethoxy)phenyl)ureido)acetamido)phenyl)((2S,4R)-2-methyl-1-propionyl-1,2,3,4-tetrahydroquinolin-4-yl)carbamate (33.5 mg, 0.03 mmol) obtained in step 3.

ESIMS, (M+H)⁺, m/z: 1132.

### (Step 5)

Compound LP-15 (20.4 mg, yield: 74%) was obtained in the same manner as in step 12 of Example LP-13 using 4-((S)-2-(1-((2-aminoethyl)carbamoyl)cyclobutane-1-carboxamido)-5-ureidopentanamido)benzyl (4-(2-(3-(4-(2-((R)-2,4-dimethyl-3-oxapiperazin-1-yl)ethoxy)phenyl)ureido)acetamido)phenyl)((2S,4R)-2-methyl-1-propionyl-1,2,3,4-tetrahydroquinolin-4-yl)carbamate (16.5 mg, 0.02 mmol) obtained in step 4.

ESIMS, (M+H)⁺, m/z: 1882. ¹H NMR (CD₃OD, δ): 7.59-7.56 (m, 4H), 7.36-7.33 (m, 3H), 7.28-7.25 (m, 3H), 7.19-7.17 (m, 4H), 6.85 (d, J = 9.0 Hz, 2H), 6.81 (s, 2H), 5.12-5.06 (m, 2H), 4.72 (dd, J = 15.5, 9.2 Hz, 1H), 4.51 (dd, J = 9.6, 4.3 Hz, 1H), 4.08 (t, J = 5.6 Hz, 2H), 4.01 (s, 2H), 3.75-3.74 (m, 1H), 3.75 (t, J = 6.7 Hz, 2H), 3.54-3.53 (m, 2H), 3.48 (t, J = 5.6 Hz, 2H), 3.38-3.35 (m, 6H), 3.21-3.15 (m, 2H), 3.05-2.98 (m, 1H), 2.92 (s, 3H), 2.88 (dt, J = 13.6, 5.2 Hz, 2H), 2.82-2.76 (m, 2H), 2.67-2.60 (m, 2H), 2.55-2.22 (m, 9H), 2.45 (t, J = 7.0 Hz, 9H), 2.39 (t, J = 6.1 Hz, 9H), 1.95-1.92 (m, 3H), 1.81-1.78 (m, 1H), 1.69-1.54 (m, 2H), 1.37 (t, J = 6.3 Hz, 1H), 1.36 (d, J = 6.7 Hz, 3H), 1.00 (t, J = 6.7 Hz, 3H), 1.00 (d, J = 6.3 Hz, 3H).

### [Example LP-16] 4-((44S,47S)-1-(2,5-Dioxo-2,5-dihydro-1H-pyrrol-1-yl)-44-isopropyl-47-methyl-2,42,45-trioxo-6,9,12,15,18,21,24,27,30,33,36,39-dodecaoxa-3,43,46-triazaoctatetracontan-48-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-16)

### (Step 1)

A crude product of 4-((45S,48S)-1-(9H-fluoren-9-yl)-45-isopropyl-48-methyl-3,43,46-trioxo-2,7,10,13,16,19,22,25,28,31,34,37,40-tridecaoxa-4,44,47-triazanonatetracontan-49-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (130 mg) was obtained in the same manner as in step 6 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (70 mg, 0.072 mmol) obtained in step 2 of Example LP-1 and commercially available 1-(9H-fluoren-9-yl)-3-oxo-2,7,10,13,16,19,22,25,28,31,34,37,40-tridecaoxa-4-azatritetracontan-43-oic acid (72 mg, 0.086 mmol).

ESIMS, (M+H)⁺, m/z: 1798.

### (Step 2)

4-((41S,44S)-1-Amino-41-isopropyl-44-methyl-39,42-dioxo-3,6,9,12,15,18,21,24,27,30,33,36-dodecaoxa-40,43-diazapentatetracontan-45-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (18 mg, total yield of two steps: 16%) was obtained in the same manner as in step 2 of Example 1 using the crude product of 4-((45S,48S)-1-(9H-fluoren-9-yl)-45-isopropyl-48-methyl-3,43,46-trioxo-2,7,10,13,16,19,22,25,28,31,34,37,40-tridecaoxa-4,44,47-triazanonatetracontan-49-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (130 mg) obtained in step 1.

ESIMS, (M+H)⁺, m/z: 1574.

### (Step 3)

Compound LP-16 (6.9 mg, yield: 50%) was obtained in the same manner as in step 12 of Example LP-13 using 4-((41S,44S)-1-amino-41-isopropyl-44-methyl-39,42-dioxo-3,6,9,12,15,18,21,24,27,30,33,36-dodecaoxa-40,43-diazapentatetracontan-45-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (13 mg, 0.0081 mmol) obtained in step 2 and commercially available 2,5-dioxopyrrolidin-1-yl 2-(2,5-dioxo-2,5-dihydro-1H-pyrrol-1-yl)acetate (2.4 mg, 0.0097 mmol).

ESIMS, (M+H)⁺, m/z: 1712. ¹H NMR (CDCl₃, δ): 9.10 (br s, 1H), 8.83 (br s, 1H), 7.72-7.70 (m, 1H), 7.60 (d, J = 7.2 Hz, 2H), 7.51 (d, J = 8.6 Hz, 2H), 7.36-7.30 (m, 6H), 7.13-7.08 (m, 5H), 6.80 (d, J = 7.7 Hz, 2H), 6.72 (s, 2H), 6.22 (br s, 1H), 5.13-5.10 (m, 1H), 5.03-5.00 (m, 1H), 4.77-4.74 (m, 1H), 4.63-4.62 (m, 1H), 4.30-4.29 (m, 1H), 4.21 (s, 3H), 4.07-4.04 (m, 4H), 3.79-3.77 (m, 2H), 3.64-3.50 (m, 39H), 3.41-3.31 (m, 4H), 3.19-3.16 (m, 2H), 3.04-3.02 (m, 2H), 2.95 (s, 3H), 2.90-2.84 (m, 2H), 2.66-2.65 (m, 6H), 2.51-2.47 (m, 2H), 2.29-2.25 (m, 2H), 1.41 (t, J = 7.5 Hz, 6H), 1.27-1.19 (m, 4H), 1.09-1.04 (m, 6H), 0.97 (t, J = 8.2 Hz, 6H), 0.87-0.85 (m, 1H).

### [Example LP-17] 4-((S)-5-Benzyl-49-(2,5-dioxo-2,5-dihydro-1H-pyrrol-1-yl)-4,7,47-trioxo-10,13,16,19,22,25,28,31,34,37,40,43-dodecaoxa-3,6,46-triazanonatetracontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-17)

### (Step 1)

tert-Butyl (2-((4-(hydroxymethyl)phenyl-amino)-2-oxoethyl)carbamate (6.0 g, yield: 29%) was obtained in the same manner as in step 1 of Example LP-13 using commercially available N-Boc-glycine (15.7 g, 89.4 mmol).

ESIMS, (M+H)⁺, m/z: 281.

### (Step 2)

A crude product of 2-(trimethylsilyl)ethyl (4-((chlorocarbonyl)((2S,4R)-2-methyl-1-propionyl-1,2,3,4-tetrahydroquinolin-4-yl)amino)phenyl)carbamate (3.0 g) was obtained in the same manner as in step 3 of Example LP-13 using 2-(trimethylsilyl)ethyl (4-(((2S,4R)-2-methyl-1-propionyl-1,2,3,4-tetrahydroquinolin-4-yl)amino)phenyl)carbamate (4.0 g, 8.82 mmol) obtained in step 2 of Example LP-13.

ESIMS, (M+H)⁺, m/z: 516.

### (Step 3)

4-(2-((tert-Butoxycarbonyl)amino)acetamido)benzyl((2S,4R)-2-methyl-1-propionyl-1,2,3,4-tetrahydroquinolin-4-yl)(4-(((2-(trimethylsilyl)ethoxy)carbonyl)amino)phenyl)carbamate (5.16 g, total yield of two steps: 56%) was obtained in the same manner as in step 4 of Example LP-13 using the crude product of 2-(trimethylsilyl)ethyl (4-((chlorocarbonyl)((2S,4R)-2-methyl-1-propionyl-1,2,3,4-tetrahydroquinolin-4-yl)amino)phenyl)carbamate (3.0 g) obtained in step 2 and tert-butyl (2-((4-(hydroxymethyl)phenyl-amino)-2-oxoethyl)carbamate (3.26 g, 11.6 mmol) obtained in step 1.

ESIMS, (M+H)⁺, m/z: 760.

### (Step 4)

4-(2-((tert-Butoxycarbonyl) amino)acetamido)benzyl((2S,4R)-2-methyl-1-propionyl-1,2,3,4-tetrahydroquinolin-4-yl)(4-(((2-(trimethylsilyl)ethoxy)carbonyl)amino)phenyl)carbamate (1.2 g, 1.58 mmol) obtained in step 3 was dissolved in THF (20 mL), then tetrabutylammonium fluoride (0.83 g, 3.16 mmol) was added to the solution, and the mixture was stirred at room temperature for 4 hours. Water was added to the reaction mixture, and an organic layer was extracted with ethyl acetate, washed with a saturated aqueous sodium chloride solution, and concentrated under reduced pressure. The obtained residue was purified by silica gel column chromatography (petroleum ether/ethyl acetate = 10/1 to 0/100) to give 4-(2-((tert-butoxycarbonyl)amino)acetamido)benzyl(4-aminophenyl)((2S,4R)-2-methyl-1-propionyl-1,2,3,4-tetrahydroquinolin-4-yl)carbamate (0.70 g, yield: 72%).

ESIMS, (M+H)⁺, m/z: 616.

### (Step 5)

4-(2-((tert-Butoxycarbonyl)amino)acetamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (0.70 g, yield: 69%) was obtained in the same manner as in step 6 of Example LP-13 using 4-(2-((tert-butoxycarbonyl)amino)acetamido)benzyl (4-aminophenyl)((2S,4R)-2-methyl-1-propionyl-1,2,3,4-tetrahydroquinolin-4-yl)carbamate (0.70 g, 1.14 mmol) obtained in step 4.

ESIMS, (M+H)⁺, m/z: 895.

### (Step 6)

4-(2-((tert-Butoxycarbonyl)amino)acetamido)benzyl (4-(2-aminoacetamido)phenyl)((2S,4R)-2-methyl-1-propionyl-1,2,3,4-tetrahydroquinolin-4-yl)carbamate (0.32 g, yield: 61%) was obtained in the same manner as in step 2 of Example 1 using 4-(2-((tert-butoxycarbonyl)amino)acetamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (0.70 g, 0.78 mmol) obtained in step 5.

ESIMS, (M+H)⁺, m/z: 673.

### (Step 7)

4-(2-((tert-Butoxycarbonyl)amino)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.50 g, yield: 37%) was obtained in the same manner as in step 3 of Example 1 using 4-(2-((tert-butoxycarbonyl)amino)acetamido)benzyl (4-(2-aminoacetamido)phenyl)((2S,4R)-2-methyl-1-propionyl-1,2,3,4-tetrahydroquinolin-4-yl)carbamate (0.65 g, 0.97 mmol) obtained in step 6.

ESIMS m/z: [M + H]⁺ 963.

### (Step 8)

4-(2-Aminoacetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.24 g, yield: 84%) was obtained in the same manner as in step 3 of Example LP-14 using 4-(2-((tert-butoxycarbonyl)amino)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.32 g, 0.33 mmol) obtained in step 7.

ESIMS, (M+H)⁺, m/z: 862.

### (Step 9)

A crude product of 4-(2-((S)-2-((((9H-fluoren-9-yl)methoxy)carbonyl)amino)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate was obtained in the same manner as in step 6 of Example LP-13 using 4-((S)-2-amino-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (50.0 mg, 0.06 mmol) obtained in step 8 and N-Fmoc-L-phenylalanine (45.0 mg, 0.12 mmol).

ESIMS, (M+H)⁺, m/z: 1232.

### (Step 10)

4-(2-((S)-2-Amino)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (43.3 mg, total yield of two steps: 74%) was obtained in the same manner as in step 2 of Example 1 using the whole amount of the crude product of 4-(2-((S)-2-((((9H-fluoren-9-yl)methoxy)carbonyl)amino)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate obtained in step 9.

ESIMS, (M+H)⁺, m/z: 1010.

### (Step 11)

Compound LP-17 (11.0 mg, yield: 63%) was obtained in the same manner as in step 12 of Example LP-13 using 4-(2-((S)-2-amino)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10.0 mg, 0.0099 mmol) obtained in step 10.

ESIMS, (M+H)⁺, m/z:1761. ¹H NMR (CD₃OD, δ): 7.63-7.61 (m, 2H), 7.58-7.56 (m, 2H), 7.35-7.32 (m, 3H), 7.28-7.23 (m, 7H), 7.20-7.18 (m, 5H), 6.86 (dt, J = 9.7, 2.6 Hz, 2H), 6.80 (s, 2H), 5.14-5.03 (m, 2H), 4.73 (dd, J = 14.4, 8.1 Hz, 1H), 4.52 (dd, J = 9.0, 5.8 Hz, 1H), 4.08 (t, J = 6.1 Hz, 2H), 4.06 (d, J = 14.0 Hz, 1H), 4.00 (s, 2H), 3.86 (d, J = 16.6 Hz, 1H), 3.75 (t, J = 7.0 Hz, 2H), 3.68-3.56 (m, 48H), 3.52-3.46 (m, 6H), 3.37-3.35 (m, 2H), 3.21-3.19 (m, 3H), 3.03-2.98 (m, 2H), 2.93 (s, 3H), 2.88 (dt, J = 13.8, 5.3 Hz, 1H), 2.82-2.76 (m, 1H), 2.56-2.43 (m, 4H), 2.30-2.26 (m, 2H), 1.40-1.33 (m, 1H), 1.36 (d, J = 6.5 Hz, 3H), 1.33-1.25 (m, 1H), 1.30 (t, J = 7.4 Hz, 3H), 1.00 (d, J = 6.3 Hz, 3H).

### [Example LP-18] 4-((S)-5-Benzyl-55-(2,5-dioxo-2,5-dihydro-1H-pyrrol-1-yl)-4,7,10,13,53-pentaoxo-16,19,22,25,28,31,34,37,40,43,46,49-dodecaoxa-3,6,9,12,52-pentaazapentapentacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-18)

### (Step 1)

A crude product of 4-((S)-11-benzyl-1-(9H-fluoren-9-yl)-3,6,9,12-tetraoxo-2-oxa-4,7,10,13-tetraazapentadecan-15-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate was obtained in the same manner as in step 6 of Example LP-13 using 4-(2-aminoacetamido)benzyl(4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (50.0 mg, 0.06 mmol) obtained in step 8 of Example LP-17 and commercially available (((9H-fluoren-9-yl)methoxy)carbonyl)glycylglycyl-L-phenylalanine (58.0 mg, 0.12 mmol).

ESIMS, (M+H)⁺, m/z: 1347.

### (Step 2)

4-(2-((S)-2-(2-(2-Aminoacetamido)acetamido)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (26.7 mg, total yield of two steps: 41%) was obtained in the same manner as in step 2 of Example 1 using the whole amount of the crude product of 4-((S)-11-benzyl-1-(9H-fluoren-9-yl)-3,6,9,12-tetraoxo-2-oxa-4,7,10,13-tetraazapentadecan-15-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate obtained in step 1.

ESIMS, (M+H)⁺, m/z: 1123.

### (Step 3)

Compound LP-18 (17.4 mg, yield: 80%) was obtained in the same manner as in step 12 of Example LP-13 using 4-(2-((S)-2-(2-(2-aminoacetamido)acetamido)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (13.0 mg, 0.01 mmol) obtained in step 2.

ESIMS, (M+H)⁺, m/z:1873. ¹H NMR (CD₃OD, δ): 7.69 (d, J = 8.5 Hz, 2H), 7.60-7.57 (m, 4H), 7.43 (t, J = 7.2 Hz, 2H), 7.38-7.33 (m, 4H), 7.29-7.24 (m, 4H), 7.20-7.19 (m, 3H), 6.87-6.85 (m, 2H), 6.80 (s, 2H), 5.10 (dd, J = 20.6, 13.0 Hz, 2H), 4.77-4.70 (m, 1H), 4.09-4.08 (m, 6H), 4.00 (s, 2H), 3.88 (s, 2H), 3.75 (t, J = 7.0 Hz, 2H), 3.68-3.65 (m, 2H), 3.61-3.57 (m, 48H), 3.47 (t, J = 5.4 Hz, 2H), 3.36-3.34 (m, 2H), 3.22-3.20 (m, 4H), 3.02 (d, J = 4.5 Hz, 1H), 2.92 (s, 3H), 2.90-2.88 (m, 1H), 2.85-2.78 (m, 1H), 2.54-2.45 (m, 4H), 2.32-2.28 (m, 2H), 1.38-1.35 (m, 1H), 1.36 (d, J = 6.7 Hz, 3H), 1.31 (t, J = 7.4 Hz, 3H), 1.02-1.00 (m, 1H), 1.00 (d, J = 6.3 Hz, 3H).

### [Example LP-19] 4-((2S,5S)-5-Isopropyl-2-methyl-4,7,47-trioxo-49-(pyridin-2-yldisulfanyl)-10,13,16,19,22,25,28,31,34,37,40,43-dodecaoxa-3,6,46-triazanonatetracontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-19)

Compound LP-19 (4.0 mg, yield: 23%) was obtained in the same manner as in step 12 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (9.7 mg, 0.010 mmol) obtained in step 2 of Example LP-1 and commercially available 2,5-dioxopyrrolidin-1-yl 3-oxo-1-(pyridin-2-yldisulfanyl)-7,10,13,16,19,22,25,28,31,34,37,40-dodecaoxa-4-azatritetracontan-43-oate (11 mg, 0.012 mmol).

ESIMS, (M+H)⁺, m/z: 1772. ¹H NMR (CDCl₃, δ): 9.10 (br s, 1H), 8.81 (br s, 1H), 8.46 (d, J = 4.1 Hz, 1H), 7.99 (d, J = 8.6 Hz, 1H), 7.66-7.61 (m, 5H), 7.50-7.48 (m, 2H), 7.41-7.38 (m, 1H), 7.34-7.17 (m, 3H), 7.12-7.08 (m, 9H), 6.80 (d, J = 8.4 Hz, 2H), 6.21 (br s, 1H), 5.36-5.33 (m, 1H), 5.13-5.10 (m, 1H), 5.02-4.99 (m, 1H), 4.77-4.74 (m, 1H), 4.65-4.63 (m, 1H), 4.21-4.20 (m, 1H), 4.08-4.05 (m, 4H), 3.79-3.77 (m, 1H), 3.61-3.55 (m, 38H), 3.44-3.25 (m, 5H), 3.16-3.13 (m, 1H), 3.08-2.98 (m, 3H), 2.94 (s, 3H), 2.89-2.75 (m, 2H), 2.63-2.61 (m, 2H), 2.52-2.44 (m, 2H), 2.28-2.20 (m, 3H), 2.02-2.00 (m, 1H), 1.63-1.61 (m, 2H), 1.46-1.38 (m, 8H), 1.07-0.98 (m, 10H), 0.89-0.85 (m, 8H).

### [Example LP-20] (R)-3-(((S)-1-((4-((((4-(2-(3-(4-(2-((R)-2,4-Dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-2-(6-(2,5-dioxo-2,5-dihydro-1H-pyrrol-1-yl)hexanamido)-3-oxopropane-1-sulfonic acid (compound LP-20)

### (Step 1)

tert-Butyl (S)-(1-((4-(hydroxymethyl)phenyl)amino)-1-oxopropan-2-yl)carbamate (7.0 g, yield: 29%) was obtained in the same manner as in step 1 of Example LP-13 using commercially available N-Boc-L-alanine (15.4 g, 81.2 mmol).

ESIMS, (M+H)⁺, m/z: 295.

### (Step 2)

2-(Trimethylsilyl)ethyl (4-((chlorocarbonyl)((2S,4R)-2-methyl-1-propionyl-1,2,3,4-tetrahydroquinolin-4-yl)amino)phenyl)carbamate (3.5 g, yield: 94%) was obtained in the same manner as in step 3 of Example LP-13 using 2-(trimethylsilyl)ethyl (4-(((2S,4R)-2-methyl-1-propionyl-1,2,3,4-tetrahydroquinolin-4-yl)amino)phenyl)carbamate (3.5 g, 7.72 mmol) obtained in step 2 of Example LP-13.

ESIMS, (M+H)⁺, m/z: 516.

### (Step 3)

4-((S)-2-((tert-Butoxycarbonyl)amino)propanamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (5.06 g, yield: 73%) was obtained in the same manner as in step 4 of Example LP-13 using 2-(trimethylsilyl)ethyl (4-((chlorocarbonyl)((2S,4R)-2-methyl-1-propionyl-1,2,3,4-tetrahydroquinolin-4-yl)amino)phenyl)carbamate (3.85 g, 7.46 mmol) obtained in step 2 and tert-butyl (S)-(1-((4-(hydroxymethyl)phenyl)amino)-1-oxopropan-2-yl)carbamate (4.39 g, 14.9 mmol) obtained in step 1.

ESIMS, (M+H)⁺, m/z: 774.

### (Step 4)

A crude product of 4-((S)-2-((tert-butoxycarbonyl)amino)propanamido)benzyl (4-aminophenyl)((2S,4R)-2-methyl-1-propionyl-1,2,3,4-tetrahydroquinolin-4-yl)carbamate was obtained in the same manner as in step 4 of Example LP-17 using 4-((S)-2-((tert-butoxycarbonyl)amino)propanamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (1.2 g, 1.55 mmol) obtained in step 3.

ESIMS, (M+H)⁺, m/z: 630.

### (Step 5)

4-((S)-2-((tert-Butoxycarbonyl)amino)propanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (1.5 g, total yield of two steps: 94%) was obtained in the same manner as in step 6 of Example LP-13 using the whole amount of the crude product of 4-((S)-2-((tert-butoxycarbonyl)amino)propanamido)benzyl(4-aminophenyl)((2S,4R)-2-methyl-1-propionyl-1,2,3,4-tetrahydroquinolin-4-yl)carbamate obtained in step 4.

ESIMS, (M+H)⁺, m/z: 909.

### (Step 6)

4-((S)-2-((tert-Butoxycarbonyl)amino)propanamido)benzyl (4-(2-aminoacetamido)phenyl)((2S,4R)-2-methyl-1-propionyl-1,2,3,4-tetrahydroquinolin-4-yl)carbamate (1 g, yield: 88%) was obtained in the same manner as in step 2 of Example 1 using 4-((S)-2-((tert-butoxycarbonyl)amino)propanamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (1.5 g, 1.65 mmol) obtained in step 5.

ESIMS, (M+H)⁺, m/z: 687.

### (Step 7)

4-((S)-2-((tert-Butoxycarbonyl)amino)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.80 g, yield: 56%) was obtained in the same manner as in step 3 of Example 1 using 4-((S)-2-((tert-butoxycarbonyl)amino)propanamido)benzyl (4-(2-aminoacetamido)phenyl)((2S,4R)-2-methyl-1-propionyl-1,2,3,4-tetrahydroquinolin-4-yl)carbamate (1 g, 1.46 mmol) obtained in step 6.

ESIMS m/z: [M + H]⁺ 976.

### (Step 8)

4-((S)-2-Aminopropanamido)benzyl(4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.20 g, yield: 28%) was obtained in the same manner as in step 3 of Example LP-14 using 4-((S)-2-((tert-butoxycarbonyl)amino)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.80 g, 0.82 mmol) obtained in step 7.

ESIMS, (M+H)⁺, m/z: 876.

### (Step 9)

A crude product of (R)-2-((((9H-fluoren-9-yl)methoxy)carbonyl)amino)-3-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-oxopropane-1-sulfonic acid was obtained in the same manner as in step 6 of Example LP-13 using 4-((S)-2-amino-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (81.6 mg, 0.09 mmol) obtained in step 8 and commercially available N-Fmoc-D-cysteic acid (72.9 mg, 0.19 mmol).

ESIMS, (M+H)⁺, m/z: 1250.

### (Step 10)

(R)-2-Amino-3-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)-carbamoyl)oxy)methyl)phenyl)amino)-1-oxopropan-2-yl)amino)-3-oxopropane-1-sulfonic acid (74.9 mg, total yield of two steps: 78%) was obtained in the same manner as in step 2 of Example 1 using the whole amount of the crude product of (R)-2-((((9H-fluoren-9-yl)methoxy)carbonyl)amino)-3-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-oxopropane-1-sulfonic acid obtained in step 9.

ESIMS, (M+H)⁺, m/z: 1028.

### (Step 11)

Compound LP-20 (20.9 mg, yield: 70%) was obtained in the same manner as in step 12 of Example LP-13 using (R)-2-amino-3-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)-carbamoyl)oxy)methyl)phenyl)amino)-1-oxopropan-2-yl)amino)-3-oxopropane-1-sulfonic acid (25.0 mg, 0.02 mmol) obtained in step 10 and commercially available 2,5-dioxopyrrolidin-1-yl 6-(2,5-dioxo-2,5-dihydro-1H-pyrrol-1-yl)hexanoate (9.00 mg, 0.03 mmol).

ESIMS, (M+H)⁺, m/z:1221. ¹H NMR (CD₃OD, δ): 7.65 (d, J = 8.1 Hz, 2H), 7.56 (d, J = 9.0 Hz, 2H), 7.35-7.33 (m, 3H), 7.29-7.23 (m, 3H), 7.16-7.14 (m, 4H), 6.85 (dt, J = 10.0, 2.7 Hz, 2H), 6.76 (s, 2H), 5.10-5.05 (m, 2H), 4.78-4.70 (m, 2H), 4.47 (q, J = 7.0 Hz, 1H), 4.07 (t, J = 6.1 Hz, 2H), 3.99 (s, 2H), 3.45 (t, J = 7.2 Hz, 2H), 3.40-3.33 (m, 4H), 3.20-3.18 (m, 2H), 3.02 (dt, J = 13.8, 5.3 Hz, 1H), 2.92 (s, 3H), 2.87 (dt, J = 13.8, 5.4 Hz, 1H), 2.82-2.76 (m, 1H), 2.53-2.50 (m, 1H), 2.30-2.26 (m, 4H), 1.62-1.56 (m, 5H), 1.47-1.44 (m, 1H), 1.45 (d, J = 7.2 Hz, 3H), 1.36 (d, J = 7.2 Hz, 3H), 1.30-1.28 (m, 1H), 1.13 (t, J = 7.4 Hz, 1H), 1.01 (t, J = 7.9 Hz, 3H), 0.99 (d, J = 6.3 Hz, 3H).

### [Example LP-21] 4-((S)-2-((S)-2-(6-(2,5-Dioxo-2,5-dihydro-1H-pyrrol-1-yl)hexanamido)-3-methylbutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-21)

Compound LP-21 (18.1 mg, yield: 61%) was obtained in the same manner as in step 12 of Example LP-13 using 4-((S)-2-((S)-2-amino-3-methoxybutanamido)-5-ureidopentanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (25.0 mg, 0.02 mmol) obtained in step 11 of Example LP-13 and commercially available 2,5-dioxopyrrolidin-1-yl 6-(2,5-dioxo-2,5-dihydro-1H-pyrrol-1-yl)hexanoate (8.71 mg, 0.03 mmol).

ESIMS, (M+H)⁺, m/z: 1255. ¹H NMR (CD₃OD, δ):7.56 (d, J = 8.5 Hz, 2H), 7.52 (d, J = 8.1 Hz, 2H), 7.34-7.32 (m, 3H), 7.27-7.26 (m, 3H), 7.19-7.17 (m, 4H), 6.85 (td, J = 6.3, 3.9 Hz, 2H), 6.77 (s, 2H), 5.12-5.03 (m, 2H), 4.72 (dd, J = 15.0, 8.8 Hz, 1H), 4.49 (dd, J = 8.8, 5.2 Hz, 1H), 4.16 (d, J = 7.6 Hz, 1H), 4.08 (t, J = 5.8 Hz, 2H), 3.99 (s, 2H), 3.62-3.58 (m, 1H), 3.51-3.44 (m, 3H), 3.37-3.35 (m, 2H), 3.19 (dt, J = 12.6, 5.4 Hz, 2H), 3.13-2.99 (m, 2H), 2.93 (s, 3H), 2.87 (dt, J = 13.9, 5.4 Hz, 1H), 2.81-2.77 (m, 1H), 2.56-2.44 (m, 1H), 2.35-2.27 (m, 3H), 2.07 (td, J = 14.1, 7.2 Hz, 1H), 1.92-1.84 (m, 1H), 1.76-1.73 (m, 1H), 1.64-1.52 (m, 7H), 1.36 (d, J = 7.2 Hz, 3H), 1.32-1.30 (m, 2H), 0.98 (t, J = 8.0 Hz, 3H), 0.97 (d, J = 4.5 Hz, 6H), 0.95 (d, J = 4.0 Hz, 3H), 0.92-0.90 (m, 1H).

### [Example LP-22] 4-((S)-5-Benzyl-18-(2,5-dioxo-2,5-dihydro-1H-pyrrol-1-yl)-4,7,10,13-tetraoxo-3,6,9,12-tetraazaoctadecanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-22)

Compound LP-22 (5.1 mg, yield: 44%) was obtained in the same manner as in step 12 of Example LP-13 using 4-(2-((S)-2-(2-(2-aminoacetamido)acetamido)-3-phenylpropanamido)acetamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (10 mg, 0.0089 mmol) obtained in step 2 of Example LP-18 and commercially available 2,5-dioxopyrrolidin-1-yl 6-(2,5-dioxo-2,5-dihydro-1H-pyrrol-1-yl)hexanoate (3.0 mg, 0.0098 mmol). ESIMS, (M+H)⁺, m/z:1317. ¹H NMR (CD₃OD, δ): 7.72-7.70 (m, 1H), 7.62-7.55 (m, 4H), 7.38-7.35 (m, 1H), 7.33-7.32 (m, 2H), 7.26 (dd, J = 6.3, 2.3 Hz, 5H), 7.20-7.19 (m, 5H), 6.86 (d, J = 9.1 Hz, 2H), 6.74 (s, 2H), 5.12-5.07 (m, 2H), 5.01-4.82 (m, 2H), 4.73-4.72 (m, 1H), 4.54 (dd, J = 9.1, 5.9 Hz, 1H), 4.09-3.99 (m, 5H), 3.87-3.78 (m, 5H), 3.62-3.58 (m, 2H), 3.44-3.35 (m, 4H), 3.30-3.16 (m, 2H), 3.04-3.00 (m, 2H), 2.93 (s, 3H), 2.89-2.86 (m, 1H), 2.82-2.76 (m, 1H), 2.52-2.49 (m, 1H), 2.30-2.21 (m, 3H), 1.59-1.51 (m, 4H), 1.35-1.21 (m, 7H), 1.00-0.99 (m, 4H), 0.92-0.89 (m, 1H).

### [Example LP-23] 4-((2S,5S,35S,67S,70S)-71-((4-((((4-(2-(3-(4-(2-((R)-2,4-Dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-35-(6-(2,5-dioxo-2,5-dihydro-1H-pyrrol-1-yl)hexanamido)-5,67-diisopropyl-2,70-dimethyl-4,7,34,38,65,68,71-heptaoxo-9,12,15,18,21,24,27,30,42,45,48,51,54,57,60,63-hexadecaoxa-3,6,33,39,66,69-hexaazahenheptacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-23)

### (Step 1)

A crude product of 4-((32S,35S)-1-(9H-fluoren-9-yl)-32-isopropyl-35-methyl-3,30,33-trioxo-2,7,10,13,16,19,22,25,28-nonaoxa-4,31,34-triazahexatriacontan-36-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (95 mg) was obtained in the same manner as in step 6 of Example LP-13 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (57 mg, 0.059 mmol) obtained in step 2 of Example LP-1 and commercially available 1-(9H-fluoren-9-yl)-3-oxo-2,7,10,13,16,19,22,25,28-nonaoxa-4-azatriacontan-30-oic acid (57 mg, 0.088 mmol). ESIMS, (M+H)⁺, m/z: 1607.

### (Step 2)

4-((2S,5S)-32-Amino-5-isopropyl-2-methyl-4,7-dioxo-9,12,15,18,21,24,27,30-octaoxa-3,6-diazadotriacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (28 mg, total yield of two steps: 34%) was obtained in the same manner as in step 2 of Example 1 using the crude product of 4-((32S,35S)-1-(9H-fluoren-9-yl)-32-isopropyl-35-methyl-3,30,33-trioxo-2,7,10,13,16,19,22,25,28-nonaoxa-4,31,34-triazahexatriacontan-36-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (95 mg) obtained in step 1.

ESIMS, (M+H)⁺, m/z: 1386.

### (Step 3)

Commercially available N-Boc-L-glutamic acid (100 mg, 0.40 mmol) was dissolved in THF (4 mL), then triethylamine (0.28 mL, 2.0 mol) and perfluorophenyl 2,2,2-trifluoroacetate (0.21 mL, 1.2 mmol) were added to the solution, and the mixture was stirred at room temperature for 1 hour. Water was added to the reaction mixture, and the aqueous layer was subjected to extraction with chloroform. The obtained organic layer was concentrated under reduced pressure. The obtained residue was purified by silica gel column chromatography (heptane/ethyl acetate = 9/1 to 1/1) to give bis(perfluorophenyl) (tert-butoxycarbonyl)-L-glutamate (210 mg, yield: 90%).

ESIMS, (M+H)⁺, m/z: 580.

### (Step 4)

4-((2S,5S)-32-Amino-5-isopropyl-2-methyl-4,7-dioxo-9,12,15,18,21,24,27,30-octaoxa-3,6-diazadotriacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (26 mg, 0.019 mmol) obtained in step 2 was dissolved in DMF (0.4 mL), then bis(perfluorophenyl) (tert-butoxycarbonyl)-L-glutamate (5.4 mg, 0.0093 mmol) obtained in step 3 was added to the solution, and the mixture was stirred at room temperature for 23 hours. The reaction mixture was concentrated under reduced pressure to give a crude product of 4-((2S,5S,35S,67S,70S)-35-((tert-butoxycarbonyl)amino)-71-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-5,67-diisopropyl-2,70-dimethyl-4,7,34,38,65,68,71-heptaoxo-9,12,15,18,21,24,27,30,42,45,48,51,54,57,60,63-hexadecaoxa-3,6,33,39,66,69-hexaazahenheptacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (28 mg). ESIMS, (M+2H)²⁺, m/z: 1491.

### (Step 5)

4-((2S,5S,35S,67S,70S)-35-Amino-71-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-5,67-diisopropyl-2,70-dimethyl-4,7,34,38,65,68,71-heptaoxo-9,12,15,18,21,24,27,30,42,45,48,51,54,57,60,63-hexadecaoxa-3,6,33,39,66,69-hexaazahenheptacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (8.6 mg, total yield of two steps: 31%) was obtained in the same manner as in step 2 of Example 2 using the crude product of 4-((2S,5S,35S,67S,70S)-35-((tert-butoxycarbonyl)amino)-71-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-5,67-diisopropyl-2,70-dimethyl-4,7,34,38,65,68,71-heptaoxo-9,12,15,18,21,24,27,30,42,45,48,51,54,57,60,63-hexadecaoxa-3,6,33,39,66,69-hexaazahenheptacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (28 mg) obtained in step 4.

ESIMS, (M+2H)²⁺, m/z: 1441.

### (Step 6)

Compound LP-23 (8.9 mg, yield: 97%) was obtained in the same manner as in step 12 of Example LP-13 using 4-((2S,5S,35S,67S,70S)-35-amino-71-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-5,67-diisopropyl-2,70-dimethyl-4,7,34,38,65,68,71-heptaoxo-9,12,15,18,21,24,27,30,42,45,48,51,54,57,60,63-hexadecaoxa-3,6,33,39,66,69-hexaazahenheptacontanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (8.6 mg, 0.0030 mmol) obtained in step 5 and commercially available 2,5-dioxopyrrolidin-1-yl 6-(2,5-dioxo-2,5-dihydro-1H-pyrrol-1-yl)hexanoate (1.1 mg, 0.0036 mmol).

ESIMS, (M+2H)²⁺, m/z: 1538. ¹H NMR (CDCl₃, δ): 9.33 (br s, 2H), 9.22 (br s, 2H), 7.98 (br s, 2H), 7.72-7.70 (m, 2H), 7.61-7.59 (m, 2H), 7.49-7.47 (m, 14H), 7.40-7.38 (m, 2H), 7.26-7.24 (m, 5H), 7.14-7.11 (m, 2H), 7.06 (d, J = 8.2 Hz, 8H), 6.76 (d, J = 8.6 Hz, 4H), 6.67 (d, J = 3.2 Hz, 2H), 6.42 (br s, 2H), 5.14-5.11 (m, 2H), 5.02-4.99 (m, 2H), 4.77-4.74 (m, 2H), 4.65-4.63 (m, 2H), 4.44-4.42 (m, 1H), 4.35-4.30 (m, 4H), 4.05-4.02 (m, 13H), 3.67-3.50 (m, 63H), 3.33-3.31 (m, 8H), 3.13-3.11 (m, 2H), 2.95-2.82 (m, 14H), 2.63 (s, 4H), 2.51-2.47 (m, 2H), 2.24-2.10 (m, 8H), 1.75-1.73 (m, 2H), 1.58-1.38 (m, 16H), 1.27-1.24 (m, 4H), 1.07-1.04 (m, 8H), 0.95-0.90 (m, 12H).

### [Example LP-24] N-(3-((4-(2-(3-(4-(2-((R)-2,4-Dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)amino)propyl)-6-(2,5-dioxo-2,5-dihydro-1H-pyrrol-1-yl)hexanamide (compound LP-24)

### (Step 1)

2-(Trimethylsilyl)ethyl (4-(((2S,4R)-2-methyl-1-propionyl-1,2,3,4-tetrahydroquinolin-4-yl)amino)phenyl)carbamate (1.0 g, 2.2 mmol) obtained in step 2 of Example LP-13 was dissolved in acetic acid (10 mL), then commercially available tert-butyl (3-oxopropyl)carbamate (1.2 g, 6.6 mmol) was added to the solution, and the mixture was stirred at room temperature for 10 minutes. Sodium cyanoborohydride (0.83 g, 13 mmol) was added to the reaction mixture, and the mixture was stirred at room temperature for 4 hours. A saturated aqueous sodium bicarbonate solution was added to the reaction mixture, and the aqueous layer was subjected to extraction with ethyl acetate. The obtained organic layer was dried over anhydrous sodium sulfate and concentrated under reduced pressure. The obtained residue was purified by silica gel column chromatography (petroleum ether/ethyl acetate = 90/10 to 0/100) to give 2-(trimethylsilyl)ethyl (4-((3-((tert-butoxycarbonyl)amino)propyl)((2S,4R)-2-methyl-1-propionyl-1,2,3,4-tetrahydroquinolin-4-yl)amino)phenyl)carbamate (1.5 g, yield: 98%).

ESIMS, (M+H)⁺, m/z: 611.

### (Step 2)

tert-Butyl (3-((4-aminophenyl)((2S,4R)-2-methyl-1-propionyl-1,2,3,4-tetrahydroquinolin-4-yl)amino)propyl)carbamate (0.9 g, yield: 87%) was obtained in the same manner as in step 5 of Example LP-13 using 2-(trimethylsilyl)ethyl (4-((3-((tert-butoxycarbonyl)amino)propyl)((2S,4R)-2-methyl-1-propionyl-1,2,3,4-tetrahydroquinolin-4-yl)amino)phenyl)carbamate (1.3 g, 2.1 mmol) obtained in step 1.

ESIMS, (M+H)⁺, m/z: 467.

### (Step 3)

(9H-Fluoren-9-yl)methyl (2-((4-((3-((tert-butoxycarbonyl)amino)propyl)((2S,4R)-2-methyl-1-propionyl-1,2,3,4-tetrahydroquinolin-4-yl)amino)phenyl)amino)-2-oxoethyl)carbamate (850 mg, yield: 57%) was obtained in the same manner as in step 1 of Example 1 using tert-butyl (3-((4-aminophenyl)((2S,4R)-2-methyl-1-propionyl-1,2,3,4-tetrahydroquinolin-4-yl)amino)propyl)carbamate (900 mg, 1.9 mmol) obtained in step 2 and commercially available (((9H-fluoren-9-yl)methoxy)carbonyl)glycine (570 mg, 1.9 mmol).

ESIMS, (M+H)⁺, m/z: 746.

### (Step 4)

tert-Butyl (3-((4-(2-aminoacetamido)phenyl)((2S,4R)-2-methyl-1-propionyl-1,2,3,4-tetrahydroquinolin-4-yl)amino)propyl)carbamate (430 mg, yield: 71%) was obtained in the same manner as in step 2 of Example 1 using (9H-fluoren-9-yl)methyl (2-((4-((3-((tert-butoxycarbonyl)amino)propyl)((2S,4R)-2-methyl-1-propionyl-1,2,3,4-tetrahydroquinolin-4-yl)amino)phenyl)amino)-2-oxoethyl)carbamate (850 mg, 1.1 mmol) obtained in step 3.

ESIMS, (M+H)⁺, m/z: 524.

### (Step 5)

tert-Butyl (3-((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)amino)propyl)carbamate (550 mg, yield: 77%) was obtained in the same manner as in step 3 of Example 1 using tert-butyl (3-((4-(2-aminoacetamido)phenyl)((2S,4R)-2-methyl-1-propionyl-1,2,3,4-tetrahydroquinolin-4-yl)amino)propyl)carbamate (430 mg, 0.82 mmol) obtained in step 4.

ESIMS, (M+H)⁺, m/z: 813.

### (Step 6)

tert-Butyl (3-((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)amino)propyl)carbamate (500 mg, 0.62 mmol) obtained in step 5 was dissolved in dichloromethane (10 mL), then a boron trifluoride-diethyl ether complex (350 mg, 1.2 mmol) was added to the solution at 0°C, and the mixture was stirred at room temperature for 4 hours. Methanol was added to the reaction mixture, and the mixture was concentrated under reduced pressure. The obtained residue was purified by reverse-phase HPLC (acetonitrile/10 mmol/L aqueous ammonium bicarbonate solution = 10/90 to 40/60) to give N-(4-((3-aminopropyl)((2S,4R)-2-methyl-1-propionyl-1,2,3,4-tetrahydroquinolin-4-yl)amino)phenyl)-2-(3-(4-(2-((R)-2,4-dimethyl-3-oxopiperazin-1-yl)ethoxy)phenyl)ureido)acetamide (260 mg, yield: 56%).

ESIMS, (M+H)⁺, m/z: 713.

### (Step 7)

Compound LP-24 (18 mg, yield: 91%) was obtained in the same manner as in step 12 of Example LP-13 using N-(4-((3-aminopropyl)((2S,4R)-2-methyl-1-propionyl-1,2,3,4-tetrahydroquinolin-4-yl)amino)phenyl)-2-(3-(4-(2-((R)-2,4-dimethyl-3-oxopiperazin-1-yl)ethoxy)phenyl)ureido)acetamide (16 mg, 0.022 mmol) obtained in step 6 and commercially available 2,5-dioxopyrrolidin-1-yl 6-(2,5-dioxo-2,5-dihydro-1H-pyrrol-1-yl)hexanoate (10 mg, 0.033 mmol).

ESIMS, (M+H)⁺, m/z: 907. ¹H NMR (CDCl₃, δ): 8.79 (br s, 1H), 7.73 (br s, 1H), 7.28-7.21 (m, 3H), 7.16-7.13 (m, 3H), 7.08 (t, J = 7.5 Hz, 1H), 6.90-6.88 (m, 1H), 6.73 (d, J = 9.1 Hz, 2H), 6.64 (s, 2H), 6.58 (d, J = 9.1 Hz, 2H), 6.46 (br s, 1H), 6.38 (br s, 1H), 4.78-4.75 (m, 1H), 4.36-4.33 (m, 1H), 4.02-3.99 (m, 4H), 3.45 (t, J = 7.2 Hz, 2H), 3.33-3.27 (m, 6H), 3.14-3.10 (m, 1H), 2.98-2.94 (m, 4H), 2.88-2.85 (m, 1H), 2.79-2.76 (m, 1H), 2.57-2.50 (m, 2H), 2.33-2.29 (m, 1H), 2.16 (t, J = 7.9 Hz, 2H), 1.74 (s, 1H), 1.60-1.50 (m, 5H), 1.38 (d, J = 7.2 Hz, 4H), 1.30-1.26 (m, 3H), 1.18 (d, J = 6.3 Hz, 3H), 1.10 (t, J = 7.5 Hz, 3H).

### [Example LP-25] 4-((26S,29S)-1-Bromo-26-isopropyl-29-methyl-2,24,27-trioxo-6,9,12,15,18,21-hexaoxa-3,25,28-triazatriacontan-30-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-25)

### (Step 1)

Commercially available 1-(9H-fluoren-9-yl)-3-oxo-2,7,10,13,16,19,22-heptaoxa-4-azapentacosan-25-oic acid (43 mg, 0.044 mmol) was dissolved in DMF (1 mL), then COMU (0.38 g, 0.88 mmol) and N,N-diisopropylethylamine (0.3 mL, 1.8 mmol) were added to the solution, and the mixture was stirred at room temperature for 15 minutes. 4-((S)-2-((S)-2-Amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (43 mg, 0.044 mmol) obtained in step 2 of Example LP-1 was added to the reaction mixture, and the mixture was stirred at room temperature for 22 hours. Piperidine (0.043 mL, 0.44 mmol) was added to the reaction mixture, and the mixture was stirred at room temperature for 5 hours. The reaction mixture was concentrated under reduced pressure, and the residue was purified by reverse-phase HPLC (acetonitrile/10 mmol/L aqueous ammonium bicarbonate solution = 25/75 to 35/65) to give 4-((23S,26S)-1-amino-23-isopropyl-26-methyl-21,24-dioxo-3,6,9,12,15,18-hexaoxa-22,25-diazaheptacosan-27-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (29 mg, yield: 51%).

ESIMS, (M+H)⁺, m/z: 1311.

### (Step 2)

Compound LP-25 (8.5 mg, yield: 95%) was obtained in the same manner as in step 12 of Example LP-13 using 4-((23S,26S)-1-amino-23-isopropyl-26-methyl-21,24-dioxo-3,6,9,12,15,18-hexaoxa-22,25-diazaheptacosan-27-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (8.2 mg, 0.063 mmol) obtained in step 1 and N-(bromoacetoxy)succinimide (1.8 mg, 0.0075 mmol).

ESIMS, (M+H)⁺, m/z: 1432. ¹H NMR (CDCl₃, δ): 9.21 (br s, 1H), 8.94 (br s, 1H), 7.97-7.89 (m, 1H), 7.71 (br s, 1H), 7.59-7.39 (m, 8H), 7.25-7.23 (m, 3H), 7.11-7.08 (m, 5H), 6.75 (d, J = 8.6 Hz, 2H), 6.36 (br s, 1H), 5.13-5.10 (m, 1H), 5.01-4.98 (m, 1H), 4.75 (br s, 1H), 4.63-4.61 (m, 1H), 4.30-4.29 (m, 1H), 4.21-4.20 (m, 1H), 4.07-4.04 (m, 4H), 3.88 (s, 2H), 3.76-3.74 (m, 2H), 3.64-3.28 (m, 25H), 3.14-3.12 (m, 1H), 2.96-2.94 (m, 4H), 2.70-2.63 (m, 4H), 2.52-2.20 (m, 3H), 1.75-1.72 (m, 1H), 1.59-1.57 (m, 1H), 1.46-1.42 (m, 6H), 1.31-1.27 (m, 3H), 1.07-1.04 (m, 4H), 0.97-0.94 (m, 4H), 0.87-0.85 (m, 3H).

### [Example LP-26] 4-((28S,61S,64S)-28-(6-(2,5-Dioxo-2,5-dihydro-1H-pyrrol-1-yl)hexanamido)-61-isopropyl-64-methyl-27,31,59,62-tetraoxo-2,5,8,11,14,17,20,23,35,38,41,44,47,50,53,56-hexadecaoxa-26,32,60,63-tetraazapentahexacontan-65-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-26)

### (Step 1)

4-((29S,32S)-1-Amino-29-isopropyl-32-methyl-27,30-dioxo-3,6,9,12,15,18,21,24-octaoxa-28,31-diazatritriacontan-33-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (29 mg, yield: 67%) was obtained in the same manner as in step 1 of Example LP-25 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (30 mg, 0.031 mmol) obtained in step 2 of Example LP-1 and commercially available 1-(9H-fluoren-9-yl)-3-oxo-2,7,10,13,16,19,22,25,28-nonaoxa-4-azahentriacontan-31-oic acid (31 mg, 0.046 mmol). ESIMS, (M+H)⁺, m/z: 1399.

### (Step 2)

tert-Butyl (S)-28-((((9H-fluoren-9-yl)methoxy)carbonyl)amino)-27-oxo-2,5,8,11,14,17,20,23-octaoxa-26-azahentriacontan-31-oate (69 mg, yield: 100%) was obtained in the same manner as in step 6 of Example LP-13 using commercially available N-Fmoc-L-glutamic acid 5-tert-butyl ester (37 mg, 0.087 mmol) and commercially available 2,5,8,11,14,17,20,23-octaoxapentacosan-25-amine (50 mg, 0.13 mmol).

ESIMS, (M+H)⁺, m/z: 791.

### (Step 3)

A crude product of (S)-28-((((9H-fluoren-9-yl)methoxy)carbonyl)amino)-27-oxo-2,5,8,11,14,17,20,23-octaoxa-26-azahentriacontan-31-oic acid (64 mg) was obtained in the same manner as in step 2 of Example 2 using tert-butyl (S)-28-((((9H-fluoren-9-yl)methoxy)carbonyl)amino)-27-oxo-2,5,8,11,14,17,20,23-octaoxa-26-azahentriacontan-31-oate (69 mg, 0.087 mmol) obtained in step 2.

ESIMS, (M+H)⁺, m/z: 736.

### (Step 4)

Perfluorophenyl (S)-28-((((9H-fluoren-9-yl)methoxy)carbonyl)amino)-27-oxo-2,5,8,11,14,17,20,23-octaoxa-26-azahentriacontan-31-oate (52 mg, yield: 66%) was obtained in the same manner as in step 3 of Example LP-23 using the crude product of (S)-28-((((9H-fluoren-9-yl)methoxy)carbonyl)amino)-27-oxo-2,5,8,11,14,17,20,23-octaoxa-26-azahentriacontan-31-oic acid (64 mg) obtained in step 3.

ESIMS, (M+H)⁺, m/z: 902.

### (Step 5)

4-((29S,32S)-1-Amino-29-isopropyl-32-methyl-27,30-dioxo-3,6,9,12,15,18,21,24-octaoxa-28,31-diazatritriacontan-33-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (19 mg, 0.014 mmol) obtained in step 1 was dissolved in DMF (0.2 mL), then N,N-diisopropylethylamine (0.012 mL, 0.07 mmol) and perfluorophenyl (S)-28-((((9H-fluoren-9-yl)methoxy)carbonyl)amino)-27-oxo-2,5,8,11,14,17,20,23-octaoxa-26-azahentriacontan-31-oate (0.1 mol/L solution in DMF, 0.16 mL, 0.016 mmol) obtained in step 4 were added to the solution, and the mixture was stirred at room temperature for 4 hours. Piperidine (0.014 mL, 0.14 mmol) was added to the reaction mixture, and the mixture was stirred at room temperature for 2 hours. The reaction mixture was concentrated under reduced pressure, and the obtained residue was purified by reverse-phase HPLC (acetonitrile/10 mmol/L aqueous ammonium bicarbonate solution = 30/70 to 35/65) to give 4-((28S,61S,64S)-28-amino-61-isopropyl-64-methyl-27,31,59,62-tetraoxo-2,5,8,11,14,17,20,23,35,38,41,44,47,50,53,56-hexadecaoxa-26,32,60,63-tetraazapentahexacontan-65-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (12 mg, yield: 46%).

ESIMS, (M+H)⁺, m/z: 1895.

### (Step 6)

Compound LP-26 (13 mg, yield: quantitative) was obtained in the same manner as in step 12 of Example LP-13 using 4-((28S,61S,64S)-28-amino-61-isopropyl-64-methyl-27,31,59,62-tetraoxo-2,5,8,11,14,17,20,23,35,38,41,44,47,50,53,56-hexadecaoxa-26,32,60,63-tetraazapentahexacontan-65-amido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (12 mg, 0.0063 mmol) obtained in step 5 and commercially available 2,5-dioxopyrrolidin-1-yl 6-(2,5-dioxo-2,5-dihydro-1H-pyrrol-1-yl)hexanoate (2.3 mg, 0.0076 mmol).

ESIMS, (M+H)⁺, m/z: 2087. ¹H NMR (CDCl₃, δ): 9.25 (br s, 1H), 8.96 (br s, 1H), 7.87 (br s, 1H), 7.72-7.70 (m, 1H), 7.60 (d, J = 8.2 Hz, 2H), 7.50-7.43 (m, 7H), 7.29-7.28 (m, 4H), 7.13-7.09 (m, 5H), 6.80 (d, J = 9.1 Hz, 2H), 6.68 (s, 2H), 6.29 (br s, 1H), 5.14-5.11 (m, 1H), 5.03-5.00 (m, 1H), 4.77-4.74 (m, 1H), 4.65-4.63 (m, 1H), 4.41-4.39 (m, 1H), 4.31-4.28 (m, 1H), 4.23-4.22 (m, 1H), 4.08-4.05 (m, 4H), 3.79-3.77 (m, 2H), 3.64-3.45 (m, 60H), 3.39-3.30 (m, 11H), 3.18-3.14 (m, 1H), 3.03-3.01 (m, 1H), 2.95 (s, 3H), 2.87-2.82 (m, 2H), 2.67-2.64 (m, 4H), 2.51-2.47 (m, 2H), 2.28-2.20 (m, 4H), 2.03-1.95 (m, 2H), 1.75-1.72 (m, 1H), 1.60-1.51 (m, 4H), 1.43-1.40 (m, 7H), 1.09-1.04 (m, 6H), 0.98 (t, J = 7.5 Hz, 6H), 0.87-0.85 (m, 2H).

### [Example LP-27] 4-((2S,5S)-14-((N-((((1R,8S,9s)-Bicyclo[6.1.0]non-4-yn-9-yl)methoxy)carbonyl)sulfamoyl)amino)-5-isopropyl-2-methyl-4,7-dioxo-9,12-dioxa-3,6-diazatetradecanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-27)

### (Step 1)

4-((2S,5S)-14-Amino-5-isopropyl-2-methyl-4,7-dioxo-9,12-dioxa-3,6-diazatetradecanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (19 mg, yield: 64%) was obtained in the same manner as in step 1 of Example LP-25 using 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (25 mg, 0.026 mmol) obtained in step 2 of Example LP-1 and commercially available 1-(9H-fluoren-9-yl)-3-oxo-2,7,10-trioxa-4-azadodecan-12-oic acid (15 mg, 0.038 mmol).

ESIMS, (M+H)⁺, m/z: 1121.

### (Step 2)

Commercially available ((1R,8S,9s)-bicyclo[6.1.0]non-4-yn-9-yl)methanol (3.7 mg, 0.025 mmol) was dissolved in acetonitrile (0.4 mL), then chlorosulfonyl isocyanate (0.0025 mL, 0.028 mmol) was added to the solution, and the mixture was stirred at 0°C for 5 minutes. Triethylamine (0.014 mL, 0.10 mmol) was added to the reaction mixture, and the mixture was stirred at 0°C for 10 minutes. 4-((2S,5S)-14-Amino-5-isopropyl-2-methyl-4,7-dioxo-9,12-dioxa-3,6-diazatetradecanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (19 mg, 0.017 mmol) obtained in step 1 was added to the reaction mixture, and the mixture was stirred at room temperature for 3.5 hours. The reaction mixture was concentrated under reduced pressure and diluted with a saturated aqueous ammonium chloride solution, and the aqueous layer was subjected to extraction with chloroform. The organic layer was concentrated under reduced pressure. The obtained residue was purified by reverse-phase HPLC (acetonitrile/10 mmol/L aqueous ammonium bicarbonate solution = 30/70 to 40/60) to give compound LP-27(5.9 mg, yield: 26%). ESIMS, (M+H)⁺, m/z: 1376. ¹H NMR (CDCl₃, δ): 9.08 (br s, 1H), 8.80 (br s, 1H), 7.61-7.59 (m, 1H), 7.44-7.40 (m, 5H), 7.28-7.20 (m, 8H), 7.13 (br s, 1H), 7.06-7.03 (m, 4H), 6.79-6.76 (m, 2H), 6.32-6.01 (m, 1H), 5.36-5.33 (m, 1H), 5.14-5.12 (m, 1H), 5.00-4.98 (m, 1H), 4.78-4.76 (m, 2H), 4.44-4.42 (m, 1H), 4.09-3.99 (m, 6H), 3.66-3.62 (m, 4H), 3.33-3.31 (m, 4H), 3.14-3.11 (m, 1H), 2.98-2.76 (m, 6H), 2.51-2.47 (m, 1H), 2.25-2.14 (m, 8H), 1.38-1.27 (m, 16H), 1.08-1.04 (m, 6H), 0.92-0.83 (m, 8H).

### [Example LP-28] 4-((S)-1-(46-(2,5-Dioxo-2,5-dihydro-1H-pyrrol-1-yl)-4,44-dioxo-7,10,13,16,19,22,25,28,31,34,37,40-dodecaoxa-3,43-diazahexatetracontanoyl)pyrrolidine-2-carboxamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound LP-28)

### (Step 1)

A crude product of (9H-fluoren-9-yl)methyl(S)-2-((4-(((tertbutyldimethylsilyl)oxy)methyl)phenyl)carbamoyl)pyrrolidine-1-carboxylate (1.5 g) was obtained in the same manner as in step 1 of Example 1 using commercially available N-Fmoc-L-proline (1.0 g, 4.2 mmol) and commercially available 4-(((tertbutyldimethylsilyl)oxy)methyl)aniline (1.4 g, 4.2 mmol).

ESIMS, (M+H)⁺, m/z: 557.

### (Step 2)

(S)-N-(4-(((tertButyldimethylsilyl)oxy)methyl)phenyl)pyrrolidine-2-carboxamide (700 mg, total yield of two steps: 50%) was obtained in the same manner as in step 2 of Example 1 using the crude product of (9H-fluoren-9-yl)methyl(S)-2-((4-(((tertbutyldimethylsilyl)oxy)methyl)phenyl)carbamoyl)pyrrolidine-1-carboxylate (1.5 g) obtained in step 1.

ESIMS, (M+H)⁺, m/z: 335.

### (Step 3)

A crude product of tert-butyl (S)-(2-(2-((4-(((tertbutyldimethylsilyl)oxy)methyl)phenyl)carbamoyl)pyrrolidin-1-yl)-2-oxoethyl) carbamate (1.0 g) was obtained in the same manner as in step 1 of Example 1 using (S)-N-(4-(((tertbutyldimethylsilyl)oxy)methyl)phenyl)pyrrolidine-2-carboxamide (700 mg, 2.1 mmol) obtained in step 2 and N-Boc-glycine (370 mg, 2.1 mmol).

ESIMS, (M+H)⁺, m/z: 492.

### (Step 4)

tert-Butyl (S)-(2-(2-((4-(hydroxymethyl)phenyl)carbamoyl)pyrrolidin-1-yl)-2-oxoethyl)carbamate (640 mg, total yield of two steps: 81%) was obtained in the same manner as in step 2 of Example 45 using the crude product of tert-butyl (S)-(2-(2-((4-(((tertbutyldimethylsilyl)oxy)methyl)phenyl)carbamoyl)pyrrolidin-1-yl)-2-oxoethyl)carbamate (1.0 g) obtained in step 3.

ESIMS, (M+H)⁺, m/z: 378.

### (Step 5)

4-((S)-1-((tert-Butoxycarbonyl)glycyl)pyrrolidine-2-carboxamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (500 mg, yield: 71%) was obtained in the same manner as in step 4 of Example LP-13 using 2-(trimethylsilyl)ethyl (4-((chlorocarbonyl)((2S,4R)-2-methyl-1-propionyl-1,2,3,4-tetrahydroquinolin-4-yl)amino)phenyl)carbamate (400 mg, 0.78 mmol) obtained in step 3 of Example LP-13 and tert-butyl (S)-(2-(2-((4-(hydroxymethyl)phenyl)carbamoyl)pyrrolidin-1-yl)-2-oxoethyl)carbamate (440 mg, 1.2 mmol) obtained in step 4. ESIMS, (M+H)⁺, m/z: 857.

### (Step 6)

4-((S)-1-((tert-Butoxycarbonyl)glycyl)pyrrolidine-2-carboxamido)benzyl (4-aminophenyl)((2S,4R)-2-methyl-1-propionyl-1,2,3,4-tetrahydroquinolin-4-yl)carbamate (400 mg, yield: 90%) was obtained in the same manner as in step 2 of Example 45 using 4-((S)-1-((tert-butoxycarbonyl)glycyl)pyrrolidine-2-carboxamido)benzyl ((2S,4R)-2-methyl-1-propionyl-1,2,3,4-tetrahydroquinolin-4-yl)(4-(((2-(trimethylsilyl)ethoxy)carbonyl)amino)phenyl)carbamate (500 mg, 0.58 mmol) obtained in step 5.

ESIMS, (M+H)⁺, m/z: 713.

### (Step 7)

4-((S)-1-((tert-Butoxycarbonyl)glycyl)pyrrolidine-2-carboxamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (400 mg, yield: 61%) was obtained in the same manner as in step 1 of Example 1 using 4-((S)-1-((tert-butoxycarbonyl)glycyl)pyrrolidine-2-carboxamido)benzyl (4-aminophenyl)((2S,4R)-2-methyl-1-propionyl-1,2,3,4-tetrahydroquinolin-4-yl)carbamate(400 mg, 1.2 mmol) obtained in step 6 and N-Fmoc-glycine (210 mg, 1.2 mmol). ESIMS, (M+H)⁺, m/z: 992.

### (Step 8)

4-((S)-1-((tert-Butoxycarbonyl)glycyl)pyrrolidine-2-carboxamido)benzyl (4-(2-aminoacetamido)phenyl)((2S,4R)-2-methyl-1-propionyl-1,2,3,4-tetrahydroquinolin-4-yl)carbamate (200 mg, yield: 61%) was obtained in the same manner as in step 2 of Example 1 using 4-((S)-1-((tert-butoxycarbonyl)glycyl)pyrrolidine-2-carboxamido)benzyl (4-(2-((((9H-fluoren-9-yl)methoxy)carbonyl)amino)acetamido)phenyl)((2S,4R)-2-methyl-1-propionyl-1,2,3,4-tetrahydroquinolin-4-yl)carbamate (400 mg, 0.40 mmol) obtained in step 7.

ESIMS, (M+H)⁺, m/z: 770.

### (Step 9)

4-((S)-1-((tert-Butoxycarbonyl)glycyl)pyrrolidine-2-carboxamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (150 mg, yield: 53%) was obtained in the same manner as in step 3 of Example 2 using 4-((S)-1-((tert-butoxycarbonyl)glycyl)pyrrolidine-2-carboxamido)benzyl (4-(2-aminoacetamido)phenyl)((2S,4R)-2-methyl-1-propionyl-1,2,3,4-tetrahydroquinolin-4-yl)carbamate (190 mg, 0.25 mmol) obtained in step 8 and commercially available (R)-4-(2-(4-aminophenoxy)ethyl)-1,3-dimethylpiperazin-2-one (65 mg, 0.25 mmol).

ESIMS, (M+H)⁺, m/z: 1060.

### (Step 10)

4-((S)-1-Glycylpyrrolidine-2-carboxamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (160 mg, yield: 22%) was obtained in the same manner as in step 2 of Example 2 using 4-((S)-1-((tert-butoxycarbonyl)glycyl)pyrrolidine-2-carboxamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (780 mg, 0.74 mmol) obtained in step 9.

ESIMS, (M+H)⁺, m/z: 959.

### (Step 11)

Compound LP-28 (18 mg, yield: 68%) was obtained in the same manner as in step 12 of Example LP-13 using 4-((S)-1-glycylpyrrolidine-2-carboxamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (15 mg, 0.016 mmol) obtained in step 10 and commercially available 2,5-dioxopyrrolidin-1-yl 1-(2,5-dioxo-2,5-dihydro-1H-pyrrol-1-yl)-3-oxo-7,10,13,16,19,22,25,28,31,34,37,40-dodecaoxa-4-azatritetracontan-43-oate (16 mg, 0.019 mmol).

ESIMS, (M+H)⁺, m/z: 1710. ¹H NMR (DMSO-d₆, δ): 10.12 (br s, 1H), 9.95 (br s, 1H), 8.61 (br s, 1H), 8.32 (br s, 1H), 8.11-7.89 (m, 2H), 7.77-7.62 (m, 2H), 7.60-7.44 (m, 3H), 7.42-7.24 (m, 5H), 7.24-7.07 (m, 3H), 7.00 (s, 2H), 6.82 (d, J = 9.0 Hz, 2H), 6.43-6.23 (m, 1H), 5.40-5.25 (m, 1H), 5.02 (q, J = 12.7 Hz, 2H), 4.60 (t, J = 9.6 Hz, 1H), 4.46-4.35 (m, 1H), 4.28-4.10 (m, 2H), 4.06-3.96 (m, 4H), 3.94-3.83 (m, 4H), 3.63-3.55 (m, 4H), 3.54-3.44 (m, 42H), 3.29-3.20 (m, 2H), 3.18-3.09 (m, 3H), 3.08-2.99 (m, 1H), 2.96-2.85 (m, 1H), 2.80 (s, 3H), 2.78-2.63 (m, 2H), 2.39 (t, J = 6.7 Hz, 2H), 2.32 (t, J = 7.4 Hz, 2H), 2.20-2.05 (m, 2H), 2.03-1.91 (m, 2H), 1.70-1.54 (m, 1H), 1.52-1.41 (m, 1H), 1.37-1.14 (m, 9H), 1.00-0.75 (m, 4H).

### [Example LP-29] 4-Acetyl-1-(4-((S)-2-((S)-2-(6-(2,5-dioxo-2,5-dihydro-1H-pyrrol-1-yl)hexanamido)-3-methylbutanamido)propanamido)benzyl)-1-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)piperazin-1-ium (compound LP-29)

### (Step 1)

tert-Butyl (S)-(1-((4-(((tertbutyldimethylsilyl)oxy)methyl)phenyl)amino)-1-oxopropan-2-yl)carbamate (19 g, yield: 94%) was obtained in the same manner as in step 1 of Example 1 using commercially available N-Boc-L-alanine (11 g, 57 mmol) and commercially available 4-(((tertbutyldimethylsilyl)oxy)methyl)aniline (13 g, 56 mmol).

ESIMS, (M+H)⁺, m/z: 409.

### (Step 2)

(S)-2-Amino-N-(4-(hydroxymethyl)phenyl)propanamide (4.5 g, yield: 99%) was obtained in the same manner as in step 2 of Example 2 using tert-butyl (S)-(1-((4-(((tertbutyldimethylsilyl)oxy)methyl)phenyl)amino)-1-oxopropan-2-yl)carbamate (9.0 g, 22 mmol) obtained in step 1.

ESIMS, (M+H)⁺, m/z: 195.

### (Step 3)

Commercially available N-Boc-valine (3.0 g, 14 mmol) was dissolved in DMF (50 mL), then N,N-diisopropylethylamine (5.4 g, 41 mmol), and CDI (2.5 g, 15 mmol) were added to the solution, and the mixture was stirred at room temperature for 1 hour. (S)-2-Amino-N-(4-(hydroxymethyl)phenyl)propanamide (2.7 g, 14 mmol) obtained in step 2 was added to the reaction mixture, and the mixture was stirred at room temperature for 3 hours. The reaction mixture was diluted with ethyl acetate, and an organic layer washed with water was dried over anhydrous sodium sulfate and concentrated under reduced pressure. The obtained residue was purified by silica gel column chromatography (dichloromethane/methanol = 10/1) to give tert-butyl ((S)-1-(((S)-1-((4-(hydroxymethyl)phenyl)amino)-1-oxopropan-2-yl)amino)-3-methyl-1-oxobutan-2-yl)carbamate (1.4 g, yield: 25%).

ESIMS, (M+H)⁺, m/z: 394.

### (Step 4)

tert-Butyl ((S)-1-(((S)-1-((4-(hydroxymethyl)phenyl)amino)-1-oxopropan-2-yl)amino)-3-methyl-1-oxobutan-2-yl)carbamate (1.2 g, 3.1 mmol) obtained in step 3 was dissolved in THF (30 mL), then triethylamine (0.46 g, 4.6 mmol) and MsCl (0.8 g, 4.6 mmol) were added to the solution at 0°C, and the mixture was stirred at room temperature for 2 hours. Lithium bromide (0.79 g, 9.2 mmol) was added to the reaction mixture, and the mixture was stirred at room temperature for 1 hour. The reaction mixture was concentrated under reduced pressure, and the obtained residue was purified by silica gel column chromatography (petroleum ether/ethyl acetate = 90/10 to 40/60) to give tert-butyl ((S)-1-(((S)-1-((4-(bromomethyl)phenyl)amino)-1-oxopropan-2-yl)amino)-3-methyl-1-oxobutan-2-yl)carbamate (640 mg, yield: 29%).

ESIMS, (M+H)⁺, m/z: 456.

### (Step 5)

Compound 2 (59 mg, 0.090 mmol) obtained in step 3 of Example 2 was dissolved in DMF (0.9 mL), then N,N-diisopropylethylamine (47 mg, 0.14 mmol) and tert-butyl ((S)-1-(((S)-1-((4-(bromomethyl)phenyl)amino)-1-oxopropan-2-yl)amino)-3-methyl-1-oxobutan-2-yl)carbamate (74 mg, 0.16 mmol) obtained in step 4 were added to the solution, and the mixture was stirred at room temperature for 1 hour. The reaction mixture was concentrated under reduced pressure, and the obtained residue was purified by reverse-phase HPLC (acetonitrile/0.1% aqueous TFA solution = 28/72) to give 4-acetyl-1-(4-((S)-2-((S)-2-((tert-butoxycarbonyl)amino)-3-methylbutanamido)propanamido)benzyl)-1-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)piperazin-1-ium (44 mg, yield: 47%).

ESIMS, (M)⁺, m/z: 1032.

### (Step 6)

A crude product of 4-acetyl-1-(4-((S)-2-((S)-2-amino-3-methylbutanamido)propanamido)benzyl)-1-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)piperazin-1-ium (40 mg) was obtained in the same manner as in step 2 of Example 2 using 4-acetyl-1-(4-((S)-2-((S)-2-((tert-butoxycarbonyl)amino)-3-methylbutanamido)propanamido)benzyl)-1-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)piperazin-1-ium (44 mg, 0.043 mmol) obtained in step 5.

ESIMS, (M)⁺, m/z: 932.

### (Step 7)

Compound LP-29 (17 mg, total yield of two steps: 35%) was obtained in the same manner as in step 12 of Example LP-13 using the crude product of 4-acetyl-1-(4-((S)-2-((S)-2-amino-3-methylbutanamido)propanamido)benzyl)-1-(2-(4-(3-(2-((4-(((2S,4R)-2-methyl-1-propionyl-1,2,3,4-tetrahydroquinolin-4-yl)amino)phenyl)amino)-2-oxoethyl)ureido)phenoxy)ethyl)piperazin-1-ium (40 mg) obtained in step 6 and commercially available 2,5-dioxopyrrolidin-1-yl 6-(2,5-dioxo-2,5-dihydro-1H-pyrrol-1-yl)hexanoate (23 mg, 0.073 mmol).

ESIMS, (M)⁺, m/z: 1125. ¹H NMR (CD₃OD, δ): 9.95 (br s, 1H), 9.57 (br s, 1H), 8.35 (d, J = 6.7 Hz, 1H), 8.01 (d, J = 7.2 Hz, 1H), 7.81 (d, J = 7.6 Hz, 2H), 7.54 (d, J = 8.5 Hz, 2H), 7.40-7.16 (m, 9H), 6.97 (d, J = 9.0 Hz, 2H), 6.62 (d, J = 9.0 Hz, 2H), 4.80 (s, 2H), 4.63-4.53 (m, 2H), 4.51-4.32 (m, 2H), 4.18-4.06 (m, 3H), 4.02-3.93 (m, 2H), 3.90-3.83 (m, 2H), 3.77-3.55 (m, 4H), 3.53-3.40 (m, 4H), 3.33-3.28 (m, 4H), 2.74-2.53 (m, 2H), 2.47-2.34 (m, 1H), 2.33-2.23 (m, 2H), 2.22-2.13 (m, 3H), 2.12-1.97 (m, 2H), 1.70-1.50 (m, 2H), 1.45 (d, J = 7.2 Hz, 3H), 1.40-1.20 (m, 4H), 1.16-1.07 (m, 6H), 1.03-0.94 (m, 6H), 0.93-0.83 (m, 1H).

### [Reference Example 1] Preparation of antibody and antibody binding fragment

Antibodies were prepared on the basis of information on the amino acid sequences of variable regions of a known anti-CD79b antibody (humanized antibody huMA79b.v28, WO2009/012268A1), anti-CD19 antibody (humanized antibody RB4v1.2, WO2018/229222A1), anti-CD22 antibody (CDR-grafted humanized antibody of mouse monoclonal antibody 5/44, WO2006/042240A2), anti-Her2 antibody (humanized antibody HUV_{Hk}I and HUV_{Lk}I, WO1992/022653A1), anti-DLL3 antibody (humanized antibody hSC16.15 and hSC16.56, WO2014/130879A2), anti-GM2 antibody (humanized antibody KM8969, WO2001/023431A1), anti-EphA2 antibody (humanized antibody 1C1, US2009/0304721A1), anti-FAP antibody (humanized antibody hu36, US10864278B2), anti-FOLR1 antibody (humanized antibody HV7LV3, WO2014/186403A2), anti-SSTR2 antibody (humanized antibody anti-SSTR2 mAb, US2021/0340264A1), anti-CD30 antibody (humanized antibody AC10, WO2003/043583A2), anti-BCMA antibody (humanized antibody CA8 Humanised V_{H} J6 and CA8 Humanised V_{L} M0, WO2012/163805A1), anti-CD33 antibody (humanized antibody h2H12, US9587019B2), anti-CD25 antibody (humanized antibody HuMax-TAC, WO2014/057119A1), anti-CLL1 antibody (humanized antibody 6E7LAHle, WO2016/205200A1), anti-CADM1 antibody (humanized antibody PTA021_A3, US8420084B2), anti-CD38 antibody (humanized antibody antibody-005, WO2006/099875A1), anti-CD74 antibody (humanized antibody hLL1Vk, WO2003/074567A2), anti-Nectin4 antibody (humanized antibody VH3-48 and L5, WO2012/047724A1), and anti-TROP2 antibody (humanized antibody RS7, US8574575B2). Anti-CD79b antibodies (human antibodies G4055, G4022, G4046, and G4047) were newly obtained as monoclonal antibodies by administering a peptide of an extracellular N-terminal region of CD79b to a human antibody-producing transgenic mouse, and cloning an antibody gene of a single B cell. Antibodies were prepared on the basis of information on the amino acid sequences of variable regions of an identified antibody gene. Table 30 shows the amino acid sequences of the heavy chain variable region (VH), the light chain variable region (VL), the heavy chain constant region (CH), and the light chain constant region (CL) of each antibody. In the table, Ab-24 and Ab-28 each represent an anti-CD79b antibody (human antibody G4055), Ab-25 and Ab-29 each represent an anti-CD79b antibody (human antibody G4022), Ab-26 and Ab-30 each represent an anti-CD79b antibody (human antibody G4046), and Ab-27 and Ab-31 each represent an anti-CD79b antibody (human antibody G4047).

**[Table 30-1]**

| Antibody No. | Antigen | Mutation site | | Variable region | | | | | | | | Constant region | |
|---|---|---|---|---|---|---|---|---|---|---|---|---|---|
| | | | | VH | | | | VL | | | | CH | CL |
| | | | | Full length | CDR1 | CDR2 | CDR3 | Full length | CDR1 | CDR2 | CDR3 | | |
| | | | | SEQ ID NO | | | | SEQ ID NO | | | | SEQ ID NO | |
| Ab-1 | CD79b | CH | S239C | 5 | 11 | 12 | 13 | 6 | 14 | 15 | 16 | 2 | 3 |
| Ab-2 | CD79b | CL | Q124C | 5 | 11 | 12 | 13 | 6 | 14 | 15 | 16 | 1 | 4 |
| Ab-3 | CD79b | CH/CL | S239C/Q124C | 5 | 11 | 12 | 13 | 6 | 14 | 15 | 16 | 2 | 4 |
| Ab-4 | CD22 | CH | S239C | 9 | 23 | 24 | 25 | 10 | 26 | 27 | 28 | 2 | 3 |
| Ab-5 | CD19 | CH | S239C | 7 | 17 | 18 | 19 | 8 | 20 | 21 | 22 | 2 | 3 |
| Ab-6 | CD79b | - | - | 5 | 11 | 12 | 13 | 6 | 14 | 15 | 16 | 1 | 3 |
| Ab-7 | CD79b | CL | Q124C (Fab) | 5 | 11 | 12 | 13 | 6 | 14 | 15 | 16 | 199 | 4 |
| Ab-8 | HER2 | CH | S239C | 29 | 67 | 68 | 69 | 30 | 70 | 71 | 72 | 2 | 3 |
| Ab-9 | CD79b | CH | S239C, R409K | 5 | 11 | 12 | 13 | 6 | 14 | 15 | 16 | 182 | 3 |
| Ab-10 | DLL3 | CH | S239C | 31 | 73 | 74 | 75 | 32 | 76 | 77 | 78 | 2 | 3 |
| Ab-11 | GM2 | CH | S239C | 33 | 79 | 80 | 81 | 34 | 82 | 83 | 84 | 2 | 3 |
| Ab-12 | EphA2 | CH | S239C | 35 | 85 | 86 | 87 | 36 | 88 | 89 | 90 | 2 | 3 |
| Ab-13 | FAP | CH | S239C | 37 | 91 | 92 | 93 | 38 | 94 | 95 | 96 | 2 | 3 |
| Ab-14 | SSTR2 | CH | S239C | 39 | 97 | 98 | 99 | 40 | 100 | 101 | 102 | 2 | 3 |
| Ab-15 | CD30 | CH | S239C | 41 | 103 | 104 | 105 | 42 | 106 | 107 | 108 | 2 | 3 |
| Ab-16 | BCMA | CH | S239C | 43 | 109 | 110 | 111 | 44 | 112 | 113 | 114 | 2 | 3 |
| Ab-17 | CD33 | CH | S239C | 45 | 115 | 116 | 117 | 46 | 118 | 119 | 120 | 2 | 3 |
| Ab-18 | FoLRα | CH | S239C | 47 | 121 | 122 | 123 | 48 | 124 | 125 | 126 | 2 | 3 |
| Ab-19 | CD25 | CH | S239C | 49 | 127 | 128 | 129 | 50 | 130 | 131 | 132 | 2 | 3 |
| Ab-20 | CADM1 | CH | S239C | 51 | 133 | 134 | 135 | 52 | 136 | 137 | 138 | 2 | 3 |

**[Table 30-2]**

| Antibody No. | Antigen | Mutation site | | Variable region | | | | | | | | Constant region | |
|---|---|---|---|---|---|---|---|---|---|---|---|---|---|
| | | | | VH | | | | VL | | | | CH | CL |
| | | | | Full length | CDR1 | CDR2 | CDR3 | Full length | CDR1 | CDR2 | CDR3 | | |
| | | | | SEQ ID NO | | | | SEQ ID NO | | | | SEQ ID NO | |
| Ab-21 | CLL1 | CH | S239C | 53 | 139 | 140 | 141 | 54 | 142 | 143 | 144 | 2 | 3 |
| Ab-22 | CD38 | CH | S239C | 55 | 145 | 146 | 147 | 56 | 148 | 149 | 150 | 2 | 3 |
| Ab-23 | CD74 | CH | S239C | 57 | 151 | 152 | 153 | 58 | 154 | 155 | 156 | 2 | 3 |
| Ab-24 | CD79b | CH | S239C | 59 | 157 | 158 | 159 | 60 | 160 | 161 | 162 | 2 | 3 |
| Ab-25 | CD79b | CH | S239C | 61 | 163 | 164 | 165 | 62 | 166 | 167 | 168 | 2 | 3 |
| Ab-26 | CD79b | CH | S239C | 63 | 169 | 170 | 171 | 64 | 172 | 173 | 174 | 2 | 3 |
| Ab-27 | CD79b | CH | S239C | 65 | 175 | 176 | 177 | 66 | 178 | 179 | 180 | 2 | 3 |
| Ab-28 | CD79b | CL | Q124C | 59 | 157 | 158 | 159 | 60 | 160 | 161 | 162 | 1 | 4 |
| Ab-29 | CD79b | CL | Q124C | 61 | 163 | 164 | 165 | 62 | 166 | 167 | 168 | 1 | 4 |
| Ab-30 | CD79b | CL | Q124C | 63 | 169 | 170 | 171 | 64 | 172 | 173 | 174 | 1 | 4 |
| Ab-31 | CD79b | CL | Q124C | 65 | 175 | 176 | 177 | 66 | 178 | 179 | 180 | 1 | 4 |
| Ab-32 | HER2 | - | - | 29 | 67 | 68 | 69 | 30 | 70 | 71 | 72 | 1 | 3 |
| Ab-33 | CD79b | CH | S239C, H435F | 5 | 11 | 12 | 13 | 6 | 14 | 15 | 16 | 182 | 3 |
| Ab-34 | NECTIN4 | CH | S239C | 183 | 187 | 188 | 189 | 184 | 190 | 191 | 192 | 2 | 3 |
| Ab-35 | TROP2 | CH | S239C | 185 | 193 | 194 | 195 | 186 | 196 | 197 | 198 | 2 | 3 |

An antibody having CH of SEQ ID NO: 1 was prepared using pCI-OtCAG_hG1 plasmid vector containing a synthetic gene encoding a signal sequence and a synthetic gene encoding wild-type CH of a human IgG1 subclass (SEQ ID NO: 1). The pCI-OtCAG_hG1 plasmid vector is a vector prepared through net synthesis by using pCI vector manufactured by Promega Corp. as a backbone, and introducing thereto restriction enzyme sites BstZ17I and NheI necessary for expressing the human antibody heavy chain gene.

An antibody having CH of SEQ ID NO: 2 was prepared using a variant of pCI-OtCAG_hG1 plasmid vector containing a synthetic gene encoding mutant CH derived from wild-type CH of a human IgG1 subclass (SEQ ID NO: 1) by the substitution of serine at position 239 (EU numbering) of the heavy chain with cysteine. Hereinafter, the substitution of serine at position 239 (EU numbering) of the heavy chain with cysteine is referred to as an "S239C mutation".

An antibody having CH of SEQ ID NO: 181 was prepared using a variant of pCI-OtCAG_hG4PE plasmid vector containing a synthetic gene encoding mutant CH derived from wild-type CH of a human IgG4 subclass by the substitution of serine at position 239 (EU numbering) of the heavy chain with cysteine and arginine at position 409 (EU numbering) thereof with lysine. Hereinafter, the substitution of serine at position 239 (EU numbering) of the heavy chain with cysteine is referred to as an "S239C mutation", and the substitution of arginine at position 409 (EU numbering) of the heavy chain with lysine is also referred to as an "R409K mutation".

An antibody having CH of SEQ ID NO: 182 was prepared using a variant of pCI-OtCAG_hG1 plasmid vector containing a synthetic gene encoding mutant CH derived from wild-type CH of a human IgG1 subclass by the substitution of serine at position 239 (EU numbering) of the heavy chain with cysteine and histidine at position 435 (EU numbering) thereof with phenylalanine. Hereinafter, the substitution of serine at position 239 (EU numbering) of the heavy chain with cysteine is referred to as an "S239C mutation", and the substitution of histidine at position 435 (EU numbering) of the heavy chain with phenylalanine is also referred to as an "H435F mutation".

An antibody having CL of SEQ ID NO: 3 was prepared using pCI-OtCMV_hK plasmid vector containing a synthetic gene encoding a signal sequence and a synthetic gene encoding wild-type CL of a human κ chain. The pCI-OtCMV_hK plasmid vector is a vector prepared through net synthesis by using pCI vector manufactured by Promega Corp. as a backbone, and introducing thereto restriction enzyme sites XbaI and BsiWI necessary for expressing the human antibody light chain gene.

An antibody having CL of SEQ ID NO: 4 was prepared using a variant of pCI-OtCMV_hK plasmid vector containing a synthetic gene encoding mutant CL derived from wild-type CL of a human κ chain (SEQ ID NO: 3) by the substitution of glutamine at position 124 (EU numbering) of the light chain with cysteine. Hereinafter, the substitution of glutamine at position 124 (EU numbering) of the light chain with cysteine is referred to as a "Q124C mutation".

pCI-hG1 and pCI-hG4 plasmid vectors serving as antibody heavy chain expression vectors were constructed by inserting a synthetic gene of VH to the pCI-OtCAG_hG1 plasmid vector or the variant thereof cleaved with restriction enzymes BstZ17I and NheI, through In-Fusion reaction. pCI-hK plasmid vectors serving as antibody light chain expression vectors were constructed by inserting a synthetic gene of VL to the pCI-OtCMV_hK plasmid vector or the variant thereof cleaved with restriction enzymes XbaI and BsiWI, through In-Fusion reaction. Human antibodies were produced using these vectors and Expi293 Expression System (Thermo Fisher Scientific Inc.). Procedures were performed according to the attached manual as follows.

6 × 10⁸ Expi293F cells (Thermo Fisher Scientific Inc.) per reaction were added to 200 mL of Expi293 Expression Medium (Thermo Fisher Scientific Inc.). 120 µg of the pCI-hG1 or pCI-hG4 plasmid vector, 120 µg of the pCI-hK plasmid vector, and ExpiFectamin 293 Reagent (Thermo Fisher Scientific Inc.) were added to Opti-MEM (Thermo Fisher Scientific Inc.) and left standing for 20 minutes, and the plasmid solution was then added to the cell-containing solution described above. After further overnight culture, ExpiFectamin 293 Transfection Enhancer was added to the cell-containing solution (culture volume: 300 mL in total). The cell-containing solution was further cultured for 2 days, and the culture supernatant was then recovered.

Each antibody was purified using MabSelect SuRe (Cytiva). The recovered culture supernatant was centrifuged, and the obtained culture supernatant was filtered through a filter. A column was packed with 1 mL of a support and buffer-was replaced with Dulbecco's phosphate-buffered saline (DPBS). The culture supernatant was added to the column so that the antibody was adsorbed to the support, followed by the washing of the column twice with 10 mL of DPBS. The antibody was eluted by the addition of 2.5 mL of Arg-Antibody Elution Buffer (Nacalai Tesque, Inc.) or a citrate buffer to the column. The antibody solution was buffer-exchanged into DPBS using NAP column (Cytiva) and used in subsequent analysis.

The obtained antibodies were
an anti-CD79b antibody (Ab-1) in which VH and VL were identical to VH and VL (SEQ ID NOs: 5 and 6) of a known anti-CD79b antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b antibody (Ab-2) in which VH and VL were identical to VH and VL (SEQ ID NOs: 5 and 6) of a known anti-CD79b antibody, CH was wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-CD79b antibody (Ab-3) in which VH and VL were identical to VH and VL (SEQ ID NOs: 5 and 6) of a known anti-CD79b antibody, CH was mutant CH (SEQ ID NO: 2) derived from wild-type CH of a human IgG1 subclass (SEQ ID NO: 1) by the introduction of an S239C mutation, and CL was mutant CL (SEQ ID NO: 4) derived from wild-type CL of a human κ chain (SEQ ID NO: 3) by the introduction of a Q124C mutation,
an anti-CD22 antibody (Ab-4) in which VH and VL were identical to VH and VL (SEQ ID NOs: 9 and 10) of a known anti-CD22 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD19 antibody (Ab-5) in which VH and VL were identical to VH and VL (SEQ ID NOs: 7 and 8) of a known anti-CD19 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b antibody (wild-type) (Ab-6) in which VH and VL were identical to VH and VL (SEQ ID NOs: 5 and 6) of a known anti-CD79b antibody, CH was wild-type CH of a human IgG1 subclass (SEQ ID NO: 1), and CL was wild-type CL of a human κ chain (SEQ ID NO: 3),
anti-CD79b antibody Fab (Ab-7) in which VH and VL were identical to VH and VL (SEQ ID NOs: 5 and 6) of a known anti-CD79b antibody, CH was mutant CH (SEQ ID NO: 199) derived from a Fab region of wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-HER2 antibody (Ab-8) in which VH and VL were identical to VH and VL (SEQ ID NOs: 29 and 30) of a known anti-HER2 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b antibody (Ab-9) in which VH and VL were identical to VH and VL (SEQ ID NOs: 5 and 6) of a known anti-CD79b antibody, CH was mutant CH (SEQ ID NO: 182) derived from wild-type CH of a human IgG4 subclass by the introduction of S239C and R409K mutations, and CL was wild-type CL of a human κ chain (SEQ ID NO: 3),
an anti-DLL3 antibody (Ab-10) in which VH and VL were identical to VH and VL (SEQ ID NOs: 31 and 32) of a known anti-DLL3 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-GM2 antibody (Ab-11) in which VH and VL were identical to VH and VL (SEQ ID NOs: 33 and 34) of a known anti-GM2 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-EphA2 antibody (Ab-12) in which VH and VL were identical to VH and VL (SEQ ID NOs: 35 and 36) of a known anti-EphA2 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-FAP antibody (Ab-13) in which VH and VL were identical to VH and VL (SEQ ID NOs: 37 and 38) of a known an anti-FAP antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-SSTR2 antibody (Ab-14) in which VH and VL were identical to VH and VL (SEQ ID NOs: 39 and 40) of a known anti-SSTR2 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD30 antibody (Ab-15) in which VH and VL were identical to VH and VL (SEQ ID NOs: 41 and 42) of a known anti-CD30 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-BCMA antibody (Ab-16) in which VH and VL were identical to VH and VL (SEQ ID NOs: 43 and 44) of a known anti-BCMA antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD33 antibody (Ab-17) in which VH and VL were identical to VH and VL (SEQ ID NOs: 45 and 46) of a known anti-CD33 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-FoLRα antibody (Ab-18) in which VH and VL were identical to VH and VL (SEQ ID NOs: 47 and 48) of a known anti-FoLRα antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD25 antibody (Ab-19) in which VH and VL were identical to VH and VL (SEQ ID NOs: 49 and 50) of a known anti-CD25 antibody, CH was mutant CH (SEQ ID NO: 2) derived from wild-type CH of a human IgG1 subclass (SEQ ID NO: 1) by the introduction of a nS239C mutation, and CL was wild-type CL of a human κ chain (SEQ ID NO: 3),
an anti-CADM1 antibody (Ab-20) in which VH and VL were identical to VH and VL (SEQ ID NOs: 51 and 52) of a known anti-CADM1 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CLL1 antibody (Ab-21) in which VH and VL were identical to VH and VL (SEQ ID NOs: 53 and 54) of a known anti-CLL1 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD38 antibody (Ab-22) in which VH and VL were identical to VH and VL (SEQ ID NOs: 55 and 56) of a known anti-CD38 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD74 antibody (Ab-23) in which VH and VL were identical to VH and VL (SEQ ID NOs: 57 and 58) of a known anti-CD74 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b_G4055 antibody (Ab-24) in which VH and VL had the amino acid sequences of SEQ ID NOs: 59 and 60, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b_G4022 antibody (Ab-25) in which VH and VL had the amino acid sequences of SEQ ID NOs: 61 and 62, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b_G4046 antibody (Ab-26) in which VH and VL had the amino acid sequences of SEQ ID NOs: 63 and 64, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b_G4047 antibody (Ab-27) in which VH and VL had the amino acid sequences of SEQ ID NOs: 65 and 66, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3),
an anti-CD79b_G4055 antibody (Ab-28) in which VH and VL had the amino acid sequences of SEQ ID NOs: 59 and 60, CH was wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-CD79b_G4022 antibody (Ab-29) in which VH and VL had the amino acid sequences of SEQ ID NOs: 61 and 62, CH was wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-CD79b_G4046 antibody (Ab-30) in which VH and VL had the amino acid sequences of SEQ ID NOs: 63 and 64, CH was wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-CD79b_G4047 antibody (Ab-31) in which VH and VL had the amino acid sequences of SEQ ID NOs: 65 and 66, CH was wild-type CH of a human IgG1 subclass (SEQ ID NO: 1), and CL was mutant CL (SEQ ID NO: 4) derived from wild-type CL of a human κ chain (SEQ ID NO: 3) by the introduction of a Q124C mutation,
an anti-HER2 antibody (wild-type) (Ab-32) in which VH and VL were identical to VH and VL (SEQ ID NOs: 29 and 30) of a known anti-HER2 antibody, CH was wild-type CH of a human IgG1 subclass (SEQ ID NO: 1), and CL was wild-type CL of a human κ chain (SEQ ID NO: 3),
an anti-CD79b antibody (Ab-33) in which VH and VL were identical to VH and VL (SEQ ID NOs: 5 and 6) of a known anti-CD79b antibody, CH was mutant CH (SEQ ID NO: 182) derived from wild-type CH of a human IgG1 subclass (SEQ ID NO: 1) by the introduction of S239C and H435F mutations, and CL was wild-type CL of a human κ chain (SEQ ID NO: 3),
an anti-NECTIN4 antibody (Ab-34) in which VH and VL were identical to VH and VL (SEQ ID NOs: 183 and 184) of a known anti-NECTIN4 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3), and
an anti-TROP2 antibody (Ab-35) in which VH and VL were identical to VH and VL (SEQ ID NOs: 185 and 186) of a known anti-TROP2 antibody, CH was mutant CH (SEQ ID NO: 2) derived from wild-type CH of a human IgG1 subclass (SEQ ID NO: 1) by the introduction of an S239C mutation, and CL was wild-type CL of a human κ chain (SEQ ID NO: 3).

### [Common operation 1] Ultrafiltration

A solution of an antibody or an antibody-drug conjugate was buffer-exchanged into the desired buffer using Amicon Ultra (50K, Merck Millipore) according to a method described in manufacturer's catalog.

A solution of an antigen binding fragment of the antibody or an antigen binding fragment of the antibody-drug conjugate was buffer-exchanged into the desired buffer using Amicon Ultra (10K, Merck Millipore) according to a method described in manufacturer's catalog.

### [Common operation 2] Gel filtration

A solution of an antibody or an antibody-drug conjugate was buffer-exchanged into Dulbecco's phosphate-buffered saline (DPBS, Gibco) using NAP(Registered trademark) column (Cytiva) according to a method described in manufacturer's catalog.

### [Common operation 3] Cation exchange column chromatography purification

A solution of an antibody-drug conjugate or an antigen binding fragment of the antibody-drug conjugate in a MES buffer (50 mmol/L, aqueous 2-morpholinoethanesulfonic acid solution, pH = 5.5) was subjected to elution and fractionation with MES buffers having sequentially elevated NaCl concentrations using a cation exchange column (Vivapure S (Sartorius) or HiTrap Capto S, Cytiva) according to a method described in manufacturer's catalog to recover a fraction containing the antibody-drug conjugate.

### [Common operation 4] Size-exclusion chromatography (SEC) analysis

An antibody, an antibody-drug conjugate, an antigen binding fragment of the antibody, or an antigen binding fragment of the antibody -drug conjugate was analyzed by SEC under the conditions shown in SEC analysis conditions 1 to measure a monomer ratio and a monomer HPLC area.

The antigen binding fragment of the antibody or the antigen binding fragment of the antibody-drug conjugate was analyzed by SEC under the conditions shown in SEC analysis conditions 2 to measure a monomer ratio and a monomer HPLC area.

### <SEC analysis conditions 1>

Column; XBridge(Registered trademark) Protein BEH SEC 200 Å, 2.5 µm, 4.6 × 150 mm (Waters Corp.)
Guard column; XBridge(Registered trademark) Protein BEH SEC 200 Å, 2.5 µm, 4.6 × 30 mm (Waters Corp.)
Mobile phase: 0.5 mol/L sodium chloride, 0.05 mol/L phosphate buffer (pH 7):ethanol = 95:5
Flow rate: 0.3 mL/min, isocratic
Column temperature: 25°C
Detection wavelength; 227 nm

### <SEC analysis conditions 2>

Column; XBridge(Registered trademark) Protein BEH SEC, 125 Å, 2.5 µm, 4.6 × 150 mm (Waters Corp.)
Mobile phase: 0.5 mol/L sodium chloride, 0.05 mol/L phosphate buffer (pH 7):ethanol = 95:5
Flow rate: 0.3 mL/min, isocratic
Column temperature: 25°C
Detection wavelength; 227 nm

### [Common operation 5] Concentration measurement of antibody-drug conjugate or antigen binding fragment of antibody-drug conjugate

The concentration of an antibody-drug conjugate or an antigen binding fragment of the antibody-drug conjugate was measured by the calibration curve technique on the assumption that the antibody-drug conjugate or the antigen binding fragment of the antibody-drug conjugate had the same absorption coefficient at 227 nm as that of the antibody or the antigen binding fragment of the antibody used as a starting material.

The calibration curve at 227 nm of the antibody used as a starting material was created according to the method described in [Common operation 4] SEC analysis. The antibody-drug conjugate or the antigen binding fragment of the antibody-drug conjugate was injected to HPLC, and the concentration of the antibody-drug conjugate or the antigen binding fragment of the antibody-drug conjugate was calculated from the amount of the conjugate injected, a dilution ratio, an area value at 227 nm, and the calibration curve of the antibody used as a starting material.

### [Common operation 6] Measurement of average drug-antibody ratio of antibody-drug conjugate or antigen binding fragment of antibody-drug conjugate

An average drug-antibody ratio (DAR) was calculated from the ratio of the ion intensity of a linker payload-connected heavy chain and light chain to that of a linker payload-unconnected heavy chain and light chain determined by the pretreatment (glycan removal using PNGaseF and subsequent separation between the heavy chain and the light chain by reduction operation) of the antibody-drug conjugate or the antigen binding fragment of the antibody-drug conjugate followed by LC/MS analysis, according to the method described in, for example, Antibody-Drug Conjugates (Laurent Ducry ed., 2013, Humana Press) or Antibody-Drug Conjugates Methods and Protocols (L. Nathan Tumey ed., 2020, Humana Press). The average drug-antibody ratio of the antigen binding fragment of the antibody-drug conjugate was calculated from the ratio of the ion intensity of a linker payload-connected light chain to that of a linker payload-unconnected light chain determined by LC/MS analysis.

### [Example ADC-1]

### (Step 1) Reduction of antibody

A 4.4 mg/mL solution in PBS was prepared using Ab-1 obtained in Reference Example 1, and 6.0 mL thereof was weighed and concentration-adjusted to 1.5 mg/mL by the addition of DPBS (18 mL). A 500 mmol/L aqueous triscarboxyethylphosphine hydrochloride solution (TCEP, 0.018 mL, 0.1% in terms of a volume ratio to the volume of the solution) was added thereto, and the mixture was mixed and then reacted at 37°C for 1 hour. The buffer was replaced with EDTA/PBS (PBS containing 1.0 mmol/L ethylenediaminetetraacetic acid, pH 6.5) according to [Common operation 1] to give 4.5 mL (4.9 mg/mL) of a reduced antibody solution of Ab-1.

### (Step 2) Reoxidation of antibody

A 10 mmol/L solution of dehydroascorbic acid (DHAA) in DMSO (0.53 mL, 30 equivalents based on the antibody) was added to the reduced antibody solution of Ab-1 (4.5 mL) obtained in step 1, and the mixture was mixed and then reacted at room temperature for 2 hours. The buffer was replaced with EDTA/PBS (PBS containing 1.0 mmol/L ethylenediaminetetraacetic acid, pH 7.6) according to [Common operation 1] to give 2.2 mL (9.8 mg/mL) of a reoxidized antibody solution.

### (Step 3) Synthesis of ADC

DPBS (0.44 mL), a 10 mmol/L solution in DMSO (0.045 mL, 10 equivalents based on the antibody) prepared using LP-1 obtained in Example LP-1, and propylene glycol (1.1 mL) were sequentially added to the reoxidized antibody solution (0.69 mL) obtained in step 2, and the mixture was mixed and then reacted at room temperature for 1 hour. The reaction solution was diluted into 4.5 mL with DPBS and then subjected to the gel filtration described in [Common operation 2]. The obtained solution was buffer-exchanged into PBS containing 0.050 mg/mL polysorbate 80 (PS80/PBS) according to [Common operation 1] to give 1.5 mL of an ADC-1 solution. The monomer fraction was measured by the approach described in [Common operation 4] and was consequently 97%. The concentration was measured by the approach described in [Common operation 5] and was consequently 3.5 mg/mL. The average DAR was measured by the approach described in [Common operation 6] and was consequently 1.6.

### [Example ADC-2]

ADC-2 described in Table 31 below was obtained in the same manner as in Example ADC-1 using antibody Ab-1 obtained in Reference Example 1 and compound LP-3 obtained in Example LP-3.

### [Example ADC-3]

ADC-3 described in Table 31 below was obtained in the same manner as in Example ADC-1 using antibody Ab-1 obtained in Reference Example 1 and compound LP-2 obtained in Example LP-2.

### [Example ADC-4]

ADC-4 described in Table 31 below was obtained in the same manner as in Example ADC-1 using antibody Ab-1 obtained in Reference Example 1 and compound LP-4 obtained in Example LP-4.

### [Example ADC-5]

ADC-5 described in Table 31 below was obtained in the same manner as in Example ADC-1 using antibody Ab-1 obtained in Reference Example 1 and compound LP-5 obtained in Example LP-5.

### [Example ADC-6]

ADC-6 described in Table 31 below was obtained in the same manner as in Example ADC-1 using antibody Ab-1 obtained in Reference Example 1 and compound LP-8 obtained in Example LP-8 except that [Common operation 3] was performed instead of [Common operation 2] as a purification step in the operational procedures of Example ADC-1.

### [Example ADC-7]

ADC-7 described in Table 31 below was obtained in the same manner as in Example ADC-6 using antibody Ab-1 obtained in Reference Example 1 and compound LP-12 obtained in Example LP-12.

### [Example ADC-8]

ADC-8 described in Table 31 below was obtained in the same manner as in Example ADC-1 using antibody Ab-1 obtained in Reference Example 1 and compound LP-13 obtained in Example LP-13.

### [Example ADC-9]

ADC-9 described in Table 31 below was obtained in the same manner as in Example ADC-1 using antibody Ab-1 obtained in Reference Example 1 and compound LP-19 obtained in Example LP-19. In the average DAR measurement, analysis was conducted by excluding reduction treatment from the pretreatment of [Common operation 6], and the average drug-antibody ratio was calculated from the ratio of the ion intensity of a linker payload-connected antibody to that of a linker payload-unconnected antibody.

### [Example ADC-10]

ADC-10 described in Table 31 below was obtained in the same manner as in Example ADC-1 using antibody Ab-1 obtained in Reference Example 1 and compound LP-20 obtained in Example LP-20.

### [Example ADC-11]

ADC-11 described in Table 31 below was obtained in the same manner as in Example ADC-6 using antibody Ab-5 obtained in Reference Example 1 and compound LP-1 obtained in Example LP-1.

### [Example ADC-12]

ADC-12 described in Table 31 below was obtained in the same manner as in Example ADC-6 using antibody Ab-4 obtained in Reference Example 1 and compound LP-1 obtained in Example LP-1.

### [Example ADC-13]

ADC-13 described in Table 31 below was obtained in the same manner as in Example ADC-6 using antibody Ab-1 obtained in Reference Example 1 and compound LP-21 obtained in Example LP-21.

### [Example ADC-14]

ADC-14 described in Table 31 below was obtained in the same manner as in Example ADC-6 using antibody Ab-1 obtained in Reference Example 1 and compound LP-23 obtained in Example LP-23.

### [Example ADC-15]

ADC-15 described in Table 31 below was obtained in the same manner as in Example ADC-6 using antibody Ab-1 obtained in Reference Example 1 and compound LP-26 obtained in Example LP-26.

### [Example ADC-16]

A 3.9 mg/mL solution in PBS was prepared using antibody Ab-6 obtained in Reference Example 1, and 0.51 mL thereof was weighed. 0.92 mL of a solution of an antibody harboring an azide group was obtained using GlyCLICK(Registered trademark) Azide Activation (Genovis AB) according to a method described in manufacturer's catalog. 0.91 mL of the obtained antibody solution was weighed, then a 10 mmol/L solution in DMSO (0.012 mL, 10 equivalents based on the antibody) prepared using LP-27 obtained in Example LP-27, and propylene glycol (0.092 mL) were sequentially added thereto, and the mixture was mixed and then reacted at room temperature for 2 hours. The solvent in the antibody solution was replaced with an MES buffer (50 mmol/L aqueous 2-morpholinoethanesulfonic acid solution, pH 5.5) according to [Common operation 1], and the resulting solution was purified by cation exchange column chromatography as described in [Common operation 3]. The obtained solution was buffer-exchanged into PBS containing 0.050 mg/mL polysorbate 80 (PS80/PBS) according to [Common operation 1] to give 0.44 mL of an ADC-16 solution. The monomer fraction was measured by the approach described in [Common operation 4] and was consequently 97%. The concentration was measured by the approach described in [Common operation 5] and was consequently 2.7 mg/mL. The average DAR was measured by the analysis described in [Common operation 6] excluding PNGaseF treatment from the pretreatment and was consequently 2.0.

### [Example ADC-17]

ADC-17 described in Table 31 below was obtained in the same manner as in Example ADC-6 using antibody Ab-8 obtained in Reference Example 1 and compound LP-1 obtained in Example LP-1.

### [Example ADC-18]

ADC-18 described in Table 31 below was obtained in the same manner as in Example ADC-6 using antibody Ab-9 obtained in Reference Example 1 and compound LP-1 obtained in Example LP-1.

### [Example ADC-19]

ADC-19 described in Table 31 below was obtained using ADC Express Service (TM) from Merck KGaA (link on the application date: adc-express-flyer-fl4728en-mk.pdf (sigmaaldrich.com) and using antibody Ab-32 obtained in Reference Example 1 and compound LP-1 obtained in Example LP-1.

### [Example ADC-20]

ADC-20 described in Table 31 below was obtained using ADC Express Service (TM) from Merck KGaA (link on the application date: adc-express-flyer-fl4728en-mk.pdf (sigmaaldrich.com) and using antibody Ab-32 obtained in Reference Example 1 and compound LP-1 obtained in Example LP-1.

### [Example ADC-21]

ADC-21 described in Table 31 below was obtained in the same manner as in Example ADC-6 using antibody Ab-10 obtained in Reference Example 1 and compound LP-1 obtained in Example LP-1.

### [Example ADC-22]

ADC-22 described in Table 31 below was obtained in the same manner as in Example ADC-6 using antibody Ab-11 obtained in Reference Example 1 and compound LP-1 obtained in Example LP-1.

### [Example ADC-23]

ADC-23 described in Table 31 below was obtained in the same manner as in Example ADC-6 using antibody Ab-12 obtained in Reference Example 1 and compound LP-1 obtained in Example LP-1.

### [Example ADC-24]

ADC-24 described in Table 31 below was obtained in the same manner as in Example ADC-6 using antibody Ab-13 obtained in Reference Example 1 and compound LP-1 obtained in Example LP-1.

### [Example ADC-25]

ADC-25 described in Table 31 below was obtained in the same manner as in Example ADC-6 using antibody Ab-18 obtained in Reference Example 1 and compound LP-1 obtained in Example LP-1.

### [Example ADC-26]

ADC-26 described in Table 31 below was obtained in the same manner as in Example ADC-6 using antibody Ab-14 obtained in Reference Example 1 and compound LP-1 obtained in Example LP-1.

### [Example ADC-27]

ADC-27 described in Table 31 below was obtained in the same manner as in Example ADC-6 using antibody Ab-15 obtained in Reference Example 1 and compound LP-1 obtained in Example LP-1.

### [Example ADC-28]

ADC-28 described in Table 31 below was obtained in the same manner as in Example ADC-6 using antibody Ab-16 obtained in Reference Example 1 and compound LP-1 obtained in Example LP-1.

### [Example ADC-29]

ADC-29 described in Table 31 below was obtained in the same manner as in Example ADC-6 using antibody Ab-17 obtained in Reference Example 1 and compound LP-1 obtained in Example LP-1.

### [Example ADC-30]

ADC-30 described in Table 31 below was obtained in the same manner as in Example ADC-6 using antibody Ab-19 obtained in Reference Example 1 and compound LP-1 obtained in Example LP-1.

### [Example ADC-31]

ADC-31 described in Table 31 below was obtained in the same manner as in Example ADC-6 using antibody Ab-21 obtained in Reference Example 1 and compound LP-1 obtained in Example LP-1.

### [Example ADC-32]

ADC-32 described in Table 31 below was obtained in the same manner as in Example ADC-6 using antibody Ab-20 obtained in Reference Example 1 and compound LP-1 obtained in Example LP-1.

### [Example ADC-33]

ADC-33 described in Table 31 below was obtained in the same manner as in Example ADC-6 using antibody Ab-27 (anti-CD79b antibody (human antibody G4047)) obtained in Reference Example 1 and compound LP-1 obtained in Example LP-1.

### [Example ADC-34]

ADC-34 described in Table 31 below was obtained in the same manner as in Example ADC-6 using antibody Ab-24 (anti-CD79b antibody (human antibody G4055)) obtained in Reference Example 1 and compound LP-1 obtained in Example LP-1.

### [Example ADC-35]

ADC-35 described in Table 31 below was obtained in the same manner as in Example ADC-1 using antibody Ab-25 (anti-CD79b antibody (human antibody G4022)) obtained in Reference Example 1 and compound LP-1 obtained in Example LP-1.

### [Example ADC-36]

ADC-36 described in Table 31 below was obtained in the same manner as in Example ADC-6 using antibody Ab-26 (anti-CD79b antibody (human antibody G4046)) obtained in Reference Example 1 and compound LP-1 obtained in Example LP-1.

### [Example ADC-37]

ADC-37 described in Table 31 below was obtained in the same manner as in Example ADC-6 using antibody Ab-28 (anti-CD79b antibody (human antibody G4055)) obtained in Reference Example 1 and compound LP-1 obtained in Example LP-1.

### [Example ADC-38]

ADC-38 described in Table 31 below was obtained in the same manner as in Example ADC-6 using antibody Ab-29 (anti-CD79b antibody (human antibody G4022)) obtained in Reference Example 1 and compound LP-1 obtained in Example LP-1.

### [Example ADC-39]

ADC-39 described in Table 31 below was obtained in the same manner as in Example ADC-6 using antibody Ab-30 (anti-CD79b antibody (human antibody G4046)) obtained in Reference Example 1 and compound LP-1 obtained in Example LP-1.

### [Example ADC-40]

ADC-40 described in Table 31 below was obtained in the same manner as in Example ADC-6 using antibody Ab-31 (anti-CD79b antibody (human antibody G4047)) obtained in Reference Example 1 and compound LP-1 obtained in Example LP-1.

### [Example ADC-41]

ADC-41 described in Table 31 below was obtained in the same manner as in Example ADC-1 using antibody Ab-1 obtained in Reference Example 1 and compound LP-28 obtained in Example LP-28.

### [Example ADC-42]

ADC-42 described in Table 31 below was obtained in the same manner as in Example ADC-6 using antibody Ab-1 obtained in Reference Example 1 and compound LP-29 obtained in Example LP-29.

### [Example ADC-43]

ADC-43 described in Table 31 below was obtained in the same manner as in Example ADC-6 using antibody Ab-22 obtained in Reference Example 1 and compound LP-1 obtained in Example LP-1.

### [Example ADC-44]

ADC-44 described in Table 31 below was obtained in the same manner as in Example ADC-6 using antibody Ab-33 obtained in Reference Example 1 and compound LP-1 obtained in Example LP-1.

### [Example ADC-45]

ADC-45 described in Table 31 below was obtained in the same manner as in Example ADC-6 using antibody Ab-7 obtained in Reference Example 1 and compound LP-1 obtained in Example LP-1.

### [Example ADC-46]

ADC-46 described in Table 31 below was obtained in the same manner as in Example ADC-6 using antibody Ab-23 obtained in Reference Example 1 and compound LP-1 obtained in Example LP-1.

### [Example ADC-47]

ADC-47 described in Table 31 below was obtained in the same manner as in Example ADC-6 using antibody Ab-34 obtained in Reference Example 1 and compound LP-1 obtained in Example LP-1.

### [Example ADC-48]

ADC-47 described in Table 31 below was obtained in the same manner as in Example ADC-6 using antibody Ab-35 obtained in Reference Example 1 and compound LP-1 obtained in Example LP-1.

**[Table 31-1]**

| ADC No | Antibody | Antigen | Mutation | Linker payload | Average DAR | ADC concentration (mg/mL) | Monomer ratio (%) |
|---|---|---|---|---|---|---|---|
| ADC-1 | Ab-1 | CD79b | Heavy chain S239C | LP-1 | 1.6 | 3.5 | 97 |
| ADC-2 | Ab-1 | CD79b | Heavy chain S239C | LP-3 | 1.6 | 2.0 | 97 |
| ADC-3 | Ab-1 | CD79b | Heavy chain S239C | LP-2 | 1.6 | 2.1 | 98 |
| ADC-4 | Ab-1 | CD79b | Heavy chain S239C | LP-4 | 1.6 | 2.3 | 97 |
| ADC-5 | Ab-1 | CD79b | Heavy chain S239C | LP-5 | 1.6 | 2.2 | 97 |
| ADC-6 | Ab-1 | CD79b | Heavy chain S239C | LP-8 | 1.8 | 3.3 | 98 |
| ADC-7 | Ab-1 | CD79b | Heavy chain S239C | LP-12 | 2.1 | 4.3 | 98 |
| ADC-8 | Ab-1 | CD79b | Heavy chain S239C | LP-13 | 1.8 | 6.0 | 98 |
| ADC-9 | Ab-1 | CD79b | Heavy chain S239C | LP-19 | 1.8 | 2.3 | 98 |
| ADC-10 | Ab-1 | CD79b | Heavy chain S239C | LP-20 | 1.7 | 2.4 | 98 |
| ADC-11 | Ab-5 | CD19 | Heavy chain S239C | LP-1 | 1.8 | 2.3 | 99 |
| ADC-12 | Ab-4 | CD22 | Heavy chain S239C | LP-1 | 1.7 | 2.3 | 95 |
| ADC-13 | Ab-1 | CD79b | Heavy chain S239C | LP-21 | 1.8 | 2.4 | 97 |
| ADC-14 | Ab-1 | CD79b | Heavy chain S239C | LP-23 | 3.7 | 1.8 | 96 |
| ADC-15 | Ab-1 | CD79b | Heavy chain S239C | LP-26 | 1.8 | 2.6 | 95 |
| ADC-16 | Ab-6 | CD79b | - | LP-27 | 2.0 | 2.7 | 97 |
| ADC-17 | Ab-8 | HER2 | Heavy chain S239C | LP-1 | 1.8 | 5.7 | 99 |
| ADC-18 | Ab-9 | CD79b | Heavy chain S239C, R409K | LP-1 | 1.7 | 2.6 | 88 |
| ADC-19 | Ab-32 | HER2 | - | LP-1 | 3.2 | 2.0 | 98 |
| ADC-20 | Ab-32 | HER2 | - | LP-1 | 4.1 | 2.0 | 98 |
| ADC-21 | Ab-10 | DLL3 | Heavy chain S239C | LP-1 | 1.9 | 2.29 | 98 |
| ADC-22 | Ab-11 | GM2 | Heavy chain S239C | LP-1 | 1.8 | 1.6 | 99 |
| ADC-23 | Ab-12 | EphA2 | Heavy chain S239C | LP-1 | 1.7 | 1.7 | 98 |
| ADC-24 | Ab-13 | FAP | Heavy chain S239C | LP-1 | 1.7 | 1.5 | 99 |
| ADC-25 | Ab-18 | FoLRα | Heavy chain S239C | LP-1 | 1.7 | 1.7 | 99 |
| ADC-26 | Ab-14 | SSTR2 | Heavy chain S239C | LP-1 | 2 | 3.1 | 99 |
| ADC-27 | Ab-15 | CD30 | Heavy chain S239C | LP-1 | 1.8 | 2.9 | 96 |
| ADC-28 | Ab-16 | BCMA | Heavy chain S239C | LP-1 | 1.7 | 2.5 | 98 |

| [Table 31-2] | | | | | | | |
|---|---|---|---|---|---|---|---|
| ADC No | Antibody | Antigen | Mutation | Linker payload | Average DAR | ADC concentration (mg/mL) | Monomer ratio (%) |
| ADC-29 | Ab-17 | CD33 | Heavy chain S239C | LP-1 | 1.9 | 2.7 | 95 |
| ADC-30 | Ab-19 | CD25 | Heavy chain S239C | LP-1 | 1.7 | 2.8 | 97 |
| ADC-31 | Ab-21 | CLL1 | Heavy chain S239C | LP-1 | 1.9 | 3.3 | 92 |
| ADC-32 | Ab-20 | CADM1 | Heavy chain S239C | LP-1 | 1.8 | 3.5 | 97 |
| ADC-33 | Ab-27 | CD79b | Heavy chain S239C | LP-1 | 1.6 | 3.7 | 97 |
| ADC-34 | Ab-24 | CD79b | Heavy chain S239C | LP-1 | 1.8 | 2.7 | 97 |
| ADC-35 | Ab-25 | CD79b | Heavy chain S239C | LP-1 | 1.9 | 3.5 | 95 |
| ADC-36 | Ab-26 | CD79b | Heavy chain S239C | LP-1 | 1.8 | 3.2 | 98 |
| ADC-37 | Ab-28 | CD79b | Light chain Q124C | LP-1 | 1.6 | 3.2 | 98 |
| ADC-38 | Ab-29 | CD79b | Light chain Q124C | LP-1 | 2 | 3.1 | 98 |
| ADC-39 | Ab-30 | CD79b | Light chain Q124C | LP-1 | 1.6 | 3.6 | 98 |
| ADC-40 | Ab-31 | CD79b | Light chain Q124C | LP-1 | 1.6 | 3.4 | 98 |
| ADC-41 | Ab-1 | CD79b | Heavy chain S239C | LP-28 | 1.9 | 3.6 | 94 |
| ADC-42 | Ab-1 | CD79b | Heavy chain S239C | LP-29 | 2.2 | 2.5 | 95 |
| ADC-43 | Ab-22 | CD38 | Heavy chain S239C | LP-1 | 1.6 | 2.5 | 98 |
| ADC-44 | Ab-33 | CD79b | Heavy chain S239C, H435F | LP-1 | 2.0 | 3.4 | 99.5 |
| ADC-45 | Ab-7 | CD79b | Light chain Q124C | LP-1 | 1.1 | 2 | 99.5 |
| ADC-46 | Ab-23 | CD74 | Heavy chain S239C | LP-1 | 1.9 | 1.6 | 99 |
| ADC-47 | Ab-34 | Nectin4 | Heavy chain S239C | LP-1 | 2.0 | 2.7 | 98 |
| ADC-48 | Ab-35 | TROP2 | Heavy chain S239C | LP-1 | 2.1 | 1.5 | 99 |

### [Example SMDC-1, SMDC-2, PDC-1, PDC-2]

Compounds SMDC-1, SMDC-2, PDC-1, and PDC-2 shown in the table below were produced.

**[Table 32]**

| No. | Structure | Exact Mass |
|---|---|---|
| SMDC-1 | 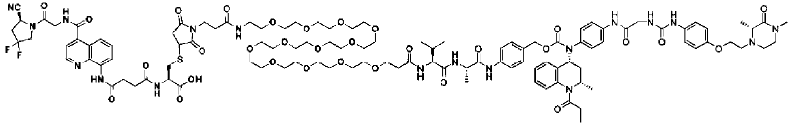 | 2287 |
| SMDC-2 | 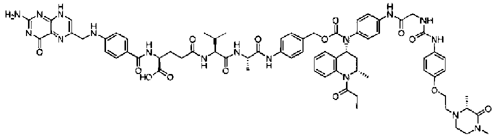 | 1398 |
| PDC-1 | 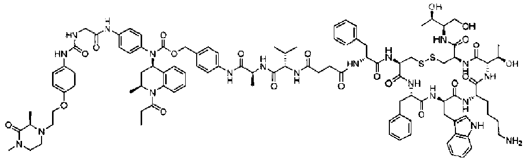 | 2075 |
| PDC-2 | 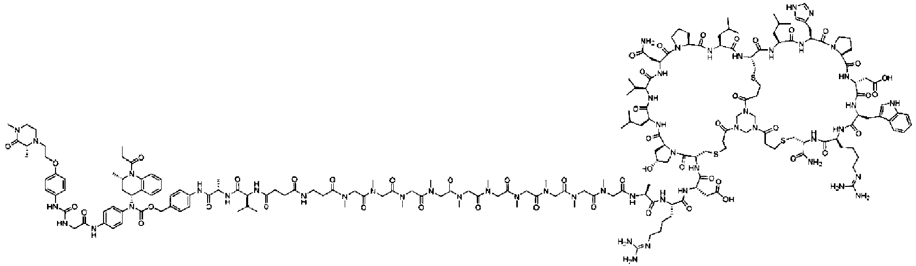 | 4237 |

### [Example SMDC-1] N-(4-((4-((2-((S)-2-Cyano-4,4-difluoropyrrolidin-1-yl)-2-oxoethyl)carbamoyl)quinolin-8-yl)amino)-4-oxobutanoyl)-S-(1-((S)-45-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)carbamoyl)-46-methyl-3,43-dioxo-7,10,13,16,19,22,25,28,31,34,37,40-dodecaoxa-4,44-diazaheptatetracontyl)-2,5-dioxopyrrolidin-3-yl)-L-cysteine (compound SMDC-1)

### (Step 1)

A crude product of tert-butyl N-(4-((4-((2-((S)-2-cyano-4,4-difluoropyrrolidin-1-yl)-2-oxoethyl)carbamoyl)quinolin-8-yl)amino)-4-oxobutanoyl)-S-trityl-L-cysteinate was obtained in the same manner as in step 1 of Example LP-13 using (S)-4-((4-((2-(2-cyano-4,4-difluoropyrrolidin-1-yl)-2-oxoethyl)carbamoyl)quinolin-8-yl)amino)-4-oxobutanoic acid (15 mg, 0.033 mmol) synthesized according to the method described in Example 2 of WO2021/160825 and commercially available tert-butyl S-trityl-L-cysteine (16.4 mg, 0.039 mmol).

ESIMS, (M+H)⁺, m/z: 861.

### (Step 2)

The whole amount of the crude product of tert-butyl N-(4-((4-((2-((S)-2-cyano-4,4-difluoropyrrolidin-1-yl)-2-oxoethyl)carbamoyl)quinolin-8-yl)amino)-4-oxobutanoyl)-S-trityl-L-cysteinate obtained in step 1 was dissolved in dichloromethane (0.60 mL), then triisopropylsilane (26 mg, 0.17 mmol) and trifluoroacetic acid (0.60 mL, 7.8 mmol) were added to the solution, and the mixture was stirred at room temperature for 3 hours. The reaction mixture was concentrated under reduced pressure, and the obtained residue was purified by reverse-phase HPLC (0.05% solution of TFA in acetonitrile/0.05% aqueous TFA solution = 15/85 to 25/75) to give a crude product of (4-((4-((2-((S)-2-cyano-4,4-difluoropyrrolidin-1-yl)-2-oxoethyl)carbamoyl)quinolin-8-yl)amino)-4-oxobutanoyl)-L-cysteine.

ESIMS, (M+H)⁺, m/z: 563.

### (Step 3)

The whole amount of the crude product of (4-((4-((2-((S)-2-cyano-4,4-difluoropyrrolidin-1-yl)-2-oxoethyl)carbamoyl)quinolin-8-yl)amino)-4-oxobutanoyl)-L-cysteine obtained in step 2 was dissolved in THF (0.60 mL), then N,N-diisopropylethylamine (0.020 mL, 0.12 mmol) and compound LP-1 (15 mg, 0.0087 mmol) obtained in step 3 of Example LP-1 were added to the solution at room temperature, and the mixture was stirred at room temperature for 3 hours. The reaction mixture was concentrated under reduced pressure, and the obtained residue was purified by silica gel column chromatography (chloroform/methanol = 90/10 to 60/40) to give compound SMDC-1 (8.8 mg, total yield of three steps: 12%).

ESIMS, (M+2H)²⁺, m/z: 1145.

### [Example SMDC-2] N²-(4-(((2-Amino-4-oxo-4,8-dihydropteridin-6-yl)methyl)amino)benzoyl)-N⁵-((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)-L-glutamine (compound SMDC-2)

### (Step 1)

Commercially available (S)-2-(4-(((2-amino-4-oxo-4,8-dihydropteridin-6-yl)methyl)amino)benzamido)-5-((2,5-dioxopyrrolidin-1-yl)oxy)-5-oxopentanoic acid (15 mg, 0.028 mmol) was dissolved in DMSO (1.0 mL), then 4-((S)-2-((S)-2-amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (27 mg, 0.028 mmol) obtained in step 2 of Example LP-1 and N,N-diisopropylethylamine (0.015 mL, 0.084 mmol) were added to the solution, and the mixture was stirred overnight at room temperature and concentrated under reduced pressure. The obtained residue was purified by reverse-phase HPLC (acetonitrile/5 mM aqueous ammonium bicarbonate solution = 15/85 to 25/75) to give compound SMDC-2 (16 mg, 41%). ESIMS, (M+H)⁺, m/z: 1399.

### [Example PDC-1] 4-((S)-2-((S)-2-(4-(((R)-1-(((4R,7S,10S,13R,16S,19R)-13-((1H-Indol-3-yl)methyl)-10-(4-aminobutyl)-16-benzyl-4-(((2R,3R)-1,3-dihydroxybutan-2-yl)carbamoyl)-7-((R)-1-hydroxyethyl)-6,9,12,15,18-pentaoxo-1,2-dithia-5,8,11,14,17-pentaazacycloicosan-19-yl)amino)-1-oxo-3-phenylpropan-2-yl)amino)-4-oxobutanamido)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (compound PDC-1)

### (Step 1)

4-((S)-2-((S)-2-Amino-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (0.22 g, 0.22 mmol) obtained in step 2 of Example LP-1 was dissolved in DMF (2.2 mL), then N,N-diisopropylethylamine (0.19 mL, 1.1 mmol) and succinic anhydride (27 mg, 0.27 mmol) were added to the solution, and the mixture was stirred at 50°C for 1 hour. The reaction mixture was concentrated under reduced pressure, and the obtained residue was purified by silica gel column chromatography (chloroform/methanol = 97/3 to 80/20) to give 4-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)amino)-3-methyl-1-oxobutan-2-yl)amino)-4-oxobutanoic acid (0.22 g, 95%).

ESIMS, (M+H)⁺, m/z: 1076.

### (Step 2)

4-(((S)-1-(((S)-1-((4-((((4-(2-(3-(4-(2-((R)-2,4-Dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)amino)-3-methyl-1-oxobutan-2-yl)amino)-4-oxobutanoic acid (0.22 g, 0.21 mmol) obtained in step 1 was dissolved in DMF (2.1 mL), then N,N-diisopropylethylamine (0.36 mL, 2.1 mmol) and pentafluorophenyl trifluoroacetate (0.29 g, 1.0 mmol) were added to the solution, and the mixture was stirred at room temperature for 1 hour. Water was added to the reaction mixture, and the precipitated white crystals were collected by filtration. The obtained crystals were dissolved in chloroform, and the solution was dried over anhydrous magnesium sulfate and concentrated under reduced pressure to give a crude product of pentafluorophenyl 4-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)amino)-3-methyl-1-oxobutan-2-yl)amino)-4-oxobutanoate.

ESIMS, (M+H)⁺, m/z: 1242.

### (Step 3)

Commercially available octreotide acetate (22 mg, 0.020 mmol) was dissolved in DMF (0.50 mL) and THF (0.50 mL), and the solution was cooled to -78°C. N,N-Diisopropylethylamine (0.018 mL, 0.10 mmol) and (9H-fluoren-9-yl)methyl (2,5-dioxopyrrolidin-1-yl) carbonate (7.6 mg, 0.022 mmol) were added thereto, and the mixture was stirred at -40°C for 1 hour and stirred overnight at - 20°C. Benzylamine was added to the reaction mixture, and the mixture was concentrated under reduced pressure. The obtained residue was purified by silica gel column chromatography (chloroform/methanol = 97/3 to 85/15) to give (9H-fluoren-9-yl)methyl (4-((4R,7S,10S,13R,16S,19R)-13-((1H-indol-3-yl)methyl)-19-((R)-2-amino-3-phenylpropanamido)-16-benzyl-4-(((2R,3R)-1,3-dihydroxybutan-2-yl)carbamoyl)-7-((R)-1-hydroxyethyl)-6,9,12,15,18-pentaoxo-1,2-dithia-5,8,11,14,17-pentaazacycloicosan-10-yl)butyl)carbamate (16 mg, 63%). ESIMS, (M+H)⁺, m/z: 1242.

### (Step 4)

(9H-Fluoren-9-yl)methyl (4-((4R,7S,10S,13R,16S,19R)-13-((1H-indol-3-yl)methyl)-19-((R)-2-amino-3-phenylpropanamido)-16-benzyl-4-(((2R,3R)-1,3-dihydroxybutan-2-yl)carbamoyl)-7-((R)-1-hydroxyethyl)-6,9,12,15,18-pentaoxo-1,2-dithia-5,8,11,14,17-pentaazacycloicosan-10-yl)butyl)carbamate (16 mg, 0.013 mmol) obtained in step 3 was dissolved in THF (0.13 mL), then N,N-diisopropylethylamine (0.011 mL, 0.064 mmol) and the crude product of pentafluorophenyl 4-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)amino)-3-methyl-1-oxobutan-2-yl)amino)-4-oxobutanoate (22 mg) obtained in step 2 were added to the solution, and the mixture was stirred at room temperature for 2 hours. The reaction mixture was concentrated under reduced pressure, and the obtained residue was purified by silica gel column chromatography (chloroform/methanol = 100/0 to 90/10) to give 4-((S)-2-((S)-2-(4-(((R)-1-(((4R,7S,10S,13R,16S,19R)-13-((1H-indol-3-yl)methyl)-10-(4-((((9H-fluoren-9-yl)methoxy)carbonyl)amino)butyl)-16-benzyl-4-(((2R,3R)-1,3-dihydroxybutan-2-yl)carbamoyl)-7-((R)-1-hydroxyethyl)-6,9,12,15,18-pentaoxo-1,2-dithia-5,8,11,14,17-pentaazacycloicosan-19-yl)amino)-1-oxo-3-phenylpropan-2-yl)amino)-4-oxobutanamido)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (18 mg, 62%).

ESIMS, (M+H)⁺, m/z: 2298.

### (Step 5)

Compound PDC-1 (12 mg, 69%) was obtained in the same manner as in step 2 of Example 1 using 4-((S)-2-((S)-2-(4-(((R)-1-(((4R,7S,10S,13R,16S,19R)-13-((1H-indol-3-yl)methyl)-10-(4-((((9H-fluoren-9-yl)methoxy)carbonyl)amino)butyl)-16-benzyl-4-(((2R,3R)-1,3-dihydroxybutan-2-yl)carbamoyl)-7-((R)-1-hydroxyethyl)-6,9,12,15,18-pentaoxo-1,2-dithia-5,8,11,14,17-pentaazacycloicosan-19-yl)amino)-1-oxo-3-phenylpropan-2-yl)amino)-4-oxobutanamido)-3-methylbutanamido)propanamido)benzyl (4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamate (18 mg, 0.0080 mmol) obtained in step 4.

ESIMS, (M+H)⁺, m/z: 2076.

### [Example PDC-2]

### BCY6099-compound 1 conjugate (compound PDC-2)

### (Step 1)

Compound PDC-2 (8.9 mg, 69%) was obtained in the same manner as in step 4 of Example PDC-1 using BCY6099 (10 mg, 0.0030 mmol) synthesized according to the method described in Compound 66 of WO2019/122863 and the crude product of pentafluorophenyl 4-(((S)-1-(((S)-1-((4-((((4-(2-(3-(4-(2-((R)-2,4-dimethyl-3-oxopiperazin-1-yl)ethoxy)phenyl)ureido)acetamido)phenyl)((2S,4R)-2-methyl-1-propionyl-1,2,3,4-tetrahydroquinolin-4-yl)carbamoyl)oxy)methyl)phenyl)amino)-1-oxopropan-2-yl)amino)-3-methyl-1-oxobutan-2-yl)amino)-3-methyl-1-oxobutan-2-yl)amino)-4-oxobutanoate (4.14 mg) obtained in step 2 of Example PDC-1.

ESIMS, (M+3H)³⁺, m/z: 1414.

### [Reference Example 2] Synthesis of comparative compound

N-(4-(((2S^{*},4R^{*})-2-Methyl-1-propionyl-1,2,3,4-tetrahydroquinolin-4-yl)amino)phenyl)-2-(3-(4-(((2S^{*},4R^{*})-2-methyl-1-propionyl-1,2,3,4-tetrahydroquinolin-4-yl)amino)phenyl)ureido)acetamide was synthesized according to the method described in Example 6j of WO2021/065980.

### [Test Example 1] Evaluation of in vitro cell survival inhibitory effect of compound using SU-DHL-4 cell line

SU-DHL-4 cells (Code No. ACC 495, Deutsche Sammlung von Mikroorga nismen und Zellkulturen GmbH; DSMZ) were cultured in RPMI1640 medium (Code No. 11875-093, Thermo Fisher Scientific Inc.) supplemented with heat-inactivated fetal bovine serum (FBS: Code No. 10099-148, Thermo Fisher Scientific Inc.) in an amount that attained a final concentration of 10 vol% and a penicillin/streptomycin solution (PS: Code No. 15140-122, Thermo Fisher Scientific Inc.) in an amount that attained a final concentration of 1 vol% at 37°C using a CO₂ incubator (95% air, 5% CO₂). The cells were passaged at intervals of twice per week so as to maintain a cell density of 1 × 10 ⁶ cells/mL or less in a volume of 20 to 40 mL.

The cultured SU-DHL-4 cells were seeded at 2000 cells/well to a transparent 384-well plate (Code No. 781182, Greiner bio-one). The cells thus seeded were precultured at 37°C for 18 to 25 hours in a CO₂ incubator. Subsequently, a 1, 0.1, or 0.01 mmol/L solution of a test compound in DMSO was prepared, and nine serial dilutions were prepared at a dilution factor of √10 so as to attain 200 times the final concentration using the RPMI1640 medium containing FBS and PS, and added in a volume of 1/200 to each well. The final concentration of DMSO was set to 0.1 vol% for this preparation. The cells thus supplemented with the test compound were cultured at 37°C for 72 hours in a CO₂ incubator. Subsequently, 0.020 mL of a solution of Cell Counting Kit-8 (Code No. CK04, Dojindo Laboratories) diluted 2-fold or 4-fold with phosphate-buffered saline (PBS) was added to each well. Color reaction was performed at 37°C for 2 to 3 hours in a CO₂ incubator, followed by absorbance measurement at a wavelength of 450 nm using SpectraMax 340PC (Molecular Devices, LLC). The rate of inhibition of cell survival was calculated according to the following formula, and the concentration (IC₅₀ value) of the test compound that exhibited a 50% rate of inhibition of cell survival was calculated. 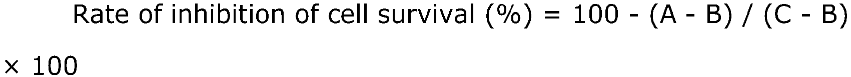 Rate of inhibition of cell survival (%) = 100 - (A - B) / (C - B) × 100
A: Absorbance of the well supplemented with the test compound
B: Absorbance of the well supplemented with no cell
C: Absorbance of the well supplemented with no test compound

The results were as shown in the column "SU-DHL-4 IC₅₀ (nmol/L)" of Table 33 below.

As mentioned above, the compound (I) of the present invention typified by the test compounds exhibited a cell survival inhibitory effect as strong as an IC₅₀ value that fell below 5 nmol/L on the SU-DHL-4 cells. Thus, the compound (I) of the present invention was found useful for treating cancer such as B cell lymphoma (in particular, diffuse large B cell lymphoma).

### [Test Example 2] Evaluation of in vitro cell survival inhibitory effect of compound using nerve cell (HIP-009 cell)

HIP-009 cells (Code No. 23002-009, Hippocampal Neural Stem Cells, Cosmo Bio Co., Ltd.) were cultured in Neural Stem Cell Growth Media (Code No. 21001-250, Cosmo Bio Co., Ltd.) at 37°C for 2 to 4 weeks using a CO₂ incubator (95% air, 5% CO₂) until confluency with half-volume media exchanges once or twice per week, then allowed to differentiate in Neuro Transition Media (Code No. 21003-250, Cosmo Bio Co., Ltd.), and seeded at 5000 to 10000 cells/0.04 mL/well to a 384-well plate (Code No. 781091, Greiner bio-one) using Neural Differentiation media (Code No. 210040250, Cosmo Bio Co., Ltd.). Neurites were elongated with half-volume media exchanges once or twice per week. Each medium was supplemented with supplements as described in the instruction and used after being allowed to pass through a 0.22 µm filter. The cells were used in evaluation 3 weeks or later after seeding to the 384-well plate by confirming the elongated neurites.

In order to detach the cells, Trypsin (Code No. 41004-100, Cosmo Bio Co., Ltd.) was added thereto, and the mixture was incubated at 37°C for 3 to 10 minutes using a CO₂ incubator (95% air, 5% CO₂). Cell detachment reaction was stopped by the addition of Trypsin Inhibitor (Code No. 41005-100, Cosmo Bio Co., Ltd.) in an equal amount.

The 384-well plate was subjected to the following operation before cell seeding. A poly(ethylenimine) solution (Code No. 03880-100ML, Sigma-Aldrich Co., LLC) was diluted with 1 × Borate Buffer (dilution of Pierce 20 × Borate Buffer (Code No. 28341, Thermo Fisher Scientific Inc.)) to prepare a 0.07% poly(ethylenimine) solution, 0.02 mL of which was added to each well and incubated at 37°C for 1 hour. Then, the plate was washed twice with 0.1 mL of PBS and once with 0.1 mL of Otsuka distilled water and left standing overnight for drying. 0.02 mL of Laminin from Engelbreth-Holm-Swarm murine sarcoma basement membrane (Code No. L2020-1MG, Sigma-Aldrich Co., LLC) diluted into 0.0033 mg/mL with PBS was added to each well and incubated at 37°C for 1 hour. Laminin was sucked out, then 0.1 mL of Otsuka distilled water was added to the plate until just before seeding, and the plate was left standing.

The cells of day 0 were photographed using Cell3iMager duos (SCREEN Holdings Co., Ltd.) before addition of a test compound, and the total area occupied by the cells was calculated. Nine serial dilutions of the test compound (10000 mmol/L solution in DMSO) were prepared at a dilution factor of √10 so as to attain 5 times the final concentration using the Neural Differentiation Media, and added in a volume of 1/5 to each well. The final concentration of DMSO was set to 0.1 vol% for this preparation. The cells thus supplemented with the test compound were cultured at 37°C for 7 days in a CO₂ incubator (95% air, 5% CO₂). The cells of day 7 were photographed using Cell3iMager duos, and the total area occupied by the cells was calculated. The rate of inhibition of cell survival was calculated according to the following formula, and the concentration (IC₅₀ value) of the test compound that exhibited a 50% rate of inhibition of cell survival was calculated. $Rate of inhibition of cell survival \left(%\right) = 100 - A / B \times 100$
A: Total area of the cells of day 7
B: Total area of the cells of day 0

The results were as shown in the column "HIP-009 IC₅₀ (nmol/L)" in Table 33 below. The selectivity of each compound for nerve cells was calculated according to the following formula using the results thus obtained and the results of Test Example 1. 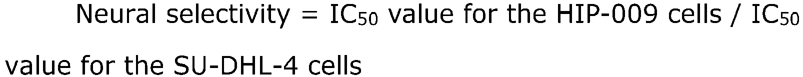 Neural selectivity = IC50 value for the HIP-009 cells / IC50 value for the SU-DHL-4 cells

The results of calculating the neural selectivity are described in the column "Neural selectivity" in Table 33 below.

The degree of improvement in the neural selectivity of each compound compared with the neural selectivity of a comparative compound was calculated according to the following formula using the neural selectivity value of each compound thus calculated.

Degree of improvement (vs. the comparative compound) = Neural selectivity of each compound / Neural selectivity of the comparative compound

The results of calculating the degree of improvement (vs. the comparative compound) are described in the column "Degree of improvement" in Table 33 below.

As mentioned above, the compound (I) of the present invention typified by the test compounds exhibited drastically improved selectivity for the nerve cells as compared with the known comparative compound. Thus, the compound (I) of the present invention was found to have desirable features as a medicament that avoids the safety hazard, i.e., peripheral neurotoxicity, to be avoided.

**[Table 33-1]**

| 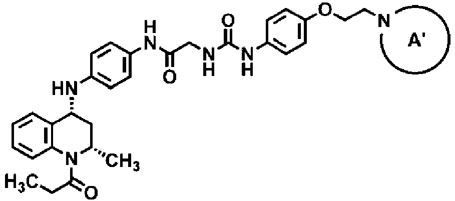 | | | | | |
|---|---|---|---|---|---|
| Compound No. | 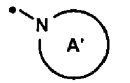 | **SU-DHL4 IC₅₀ (nmol/L)** | **HIP-009 IC₅₀ (nmol/L)** | Nerve selectivity | Degree of improvement (vs. comparative compound) |
| **1** | 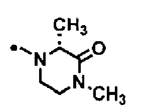 | **0.061** | **160** | **2600** | **20** |
| **2** | 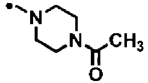 | **0.075** | **480** | **6300** | **48** |
| **3** | 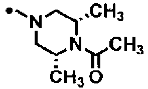 | **0.050** | **320** | **6300** | **48** |
| **4** | 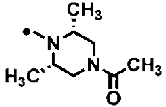 | **0.061** | **100** | **1700** | **13** |
| **5** | 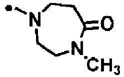 | **0.021** | **96** | **4500** | **35** |
| **6** | 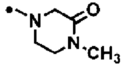 | **0.20** | **600** | **3000** | **23** |
| **7** | 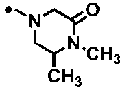 | **3.4** | **2300** | **660** | **5.1** |

**[Table 33-2]**

| 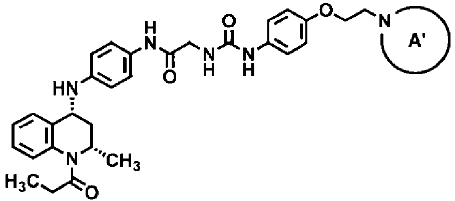 | | | | | |
|---|---|---|---|---|---|
| Compound No. | 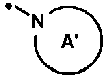 | **SU-DHL-4 IC₅₀ (nmol/L)** | **HIP-009 IC₅₀ (nmol/L)** | Nerve selectivity | Degree of improvement (vs. comparative compound) |
| **8** | 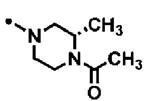 | **0.026** | **260** | **10000** | **77** |
| **9** | 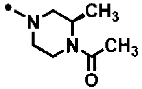 | **0.14** | **520** | **3900** | **30** |
| **10** | 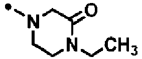 | **0.99** | **1600** | **1600** | **12** |
| **11** | 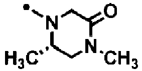 | **0.19** | **900** | **4900** | **38** |
| **12** | 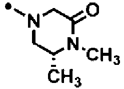 | **0.42** | **830** | **2000** | **15** |
| **13** | 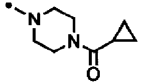 | **0.52** | **760** | **1500** | **12** |
| **14** | 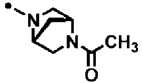 | **0.27** | **720** | **2600** | **20** |

**[Table 33-3]**

| 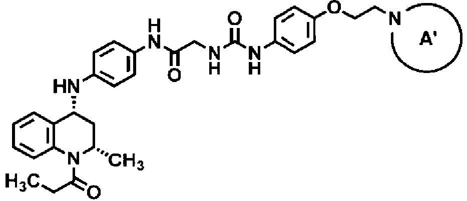 | | | | | |
|---|---|---|---|---|---|
| Compound No. | 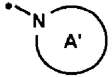 | **SU-DHL-4 IC₅₀ (nmol/L)** | **HIP-009 IC₅₀ (nmol/L)** | Nerve selectivity | Degree of improvement (vs. comparative compound) |
| **15** | 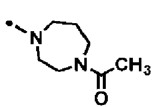 | **0.27** | **650** | **2400** | **18** |
| **16** | 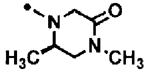 | **0.94** | **2100** | **2300** | **18** |
| **17** | 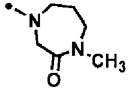 | **0.29** | **2500** | **8700** | **67** |
| **18** | 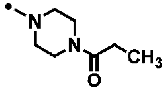 | **0.20** | **850** | **4300** | **33** |
| **19** | 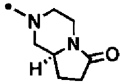 | **0.033** | **320** | **9600** | **74** |
| **20** | 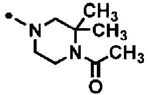 | **1.8** | **2500** | **1400** | **11** |
| **21** | 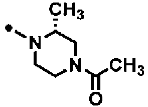 | **0.072** | **530** | **7400** | **57** |

**[Table 33-4]**

| 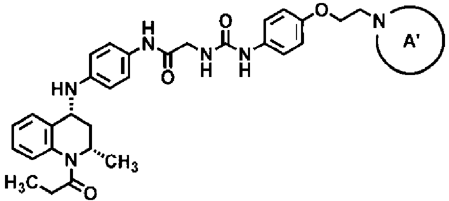 | | | | | |
|---|---|---|---|---|---|
| Compound No. | 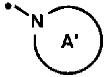 | **SU-DHL4 IC₅₀ (nmol/L)** | **HIP-009 IC₅₀ (nmol/L)** | Nerve selectivity | Degree of improvement (vs. comparative compound) |
| **22** | 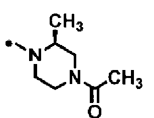 | **0.081** | **560** | **6900** | **53** |
| **23** | 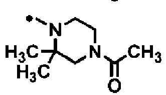 | **2.0** | **2100** | **1100** | **8.5** |
| **24** | 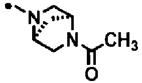 | **1.5** | **1400** | **930** | **7.2** |
| **25** | 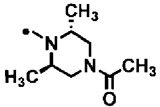 | **0.61** | **980** | **1600** | **12** |
| **26** | 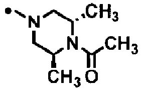 | **0.40** | **910** | **2300** | **18** |
| **27** | 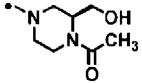 | **0.92** | **4200** | **4600** | **35** |
| Comparative compound | | **0.098** | **13** | **130** | - |

**[Table 33-5]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **28** | 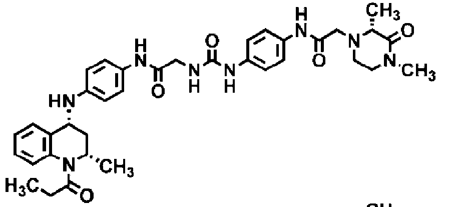 | ① **0.30** |
| | | ② **340** |
| | | ③ **1100** |
| | | ④ **26** |
| **29** | 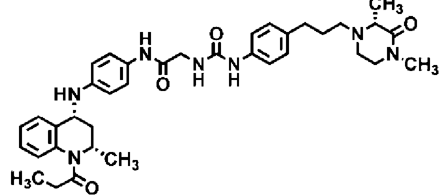 | ① **0.26** |
| | | ② **270** |
| | | ③ **1000** |
| | | ④ **24** |
| **30** | 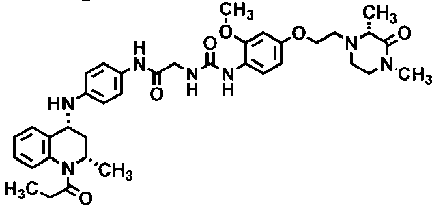 | ① **0.13** |
| | | ② **270** |
| | | ③ **2000** |
| | | ④ **46** |
| **31** | 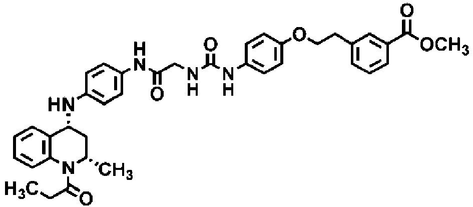 | ① **0.21** |
| | | ② **270** |
| | | ② **1300** |
| | | ④ **29** |
| **32** | 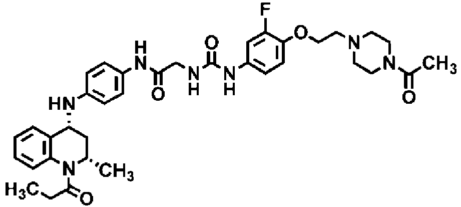 | ① **0.028** |
| | | ② **80** |
| | | ③ **2800** |
| | | ④ **65** |

**[Table 33-6]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **33** | 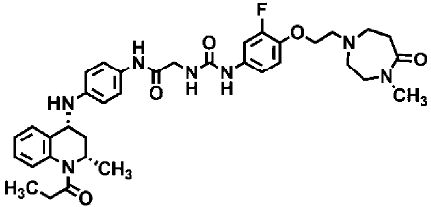 | ① **0.016** |
| | | ② **16** |
| | | ③ **1000** |
| | | ④ **23** |
| **34** | 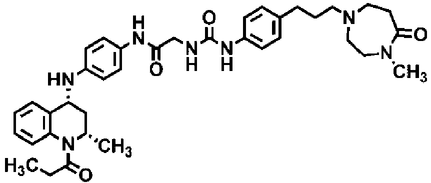 | ① **0.21** |
| | | ② **120** |
| | | ③ **600** |
| | | ④ **14** |
| **35** | 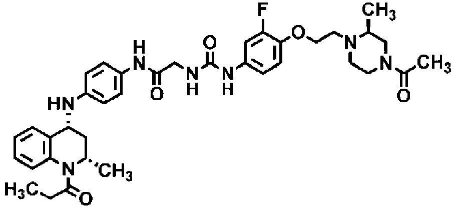 | ① **0.028** |
| | | ② **69** |
| | | ③ **2500** |
| | | ④ **57** |
| **36** | 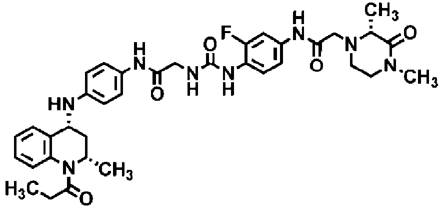 | ① **0.14** |
| | | ② **77** |
| | | ③ **540** |
| | | ④ **12** |
| **37** | 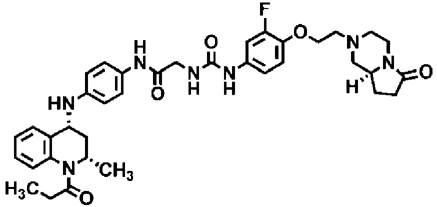 | ① **0.012** |
| | | ② **32** |
| | | ③ **2700** |
| | | ④ **63** |

**[Table 33-7]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **38** | 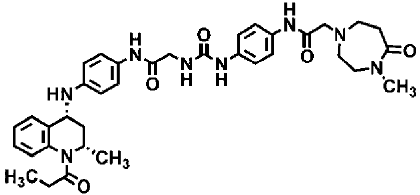 | ① **0.31** |
| | | ② **280** |
| | | ③ **910** |
| | | ④ **21** |
| **39** | 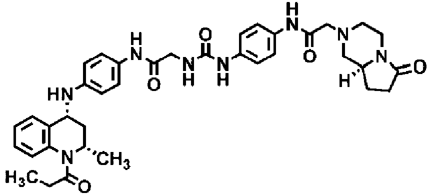 | ① **0.28** |
| | | ② **160** |
| | | ③ **560** |
| | | ④ **13** |
| **40** | 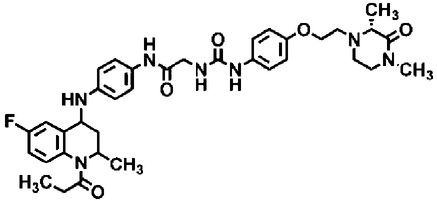 | ① **0.032** |
| | | ② **190** |
| | | ③ **5800** |
| | | ④ **130** |
| **41** | 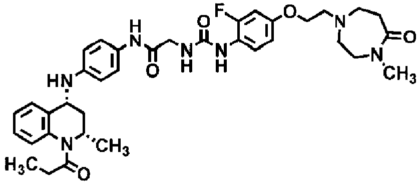 | ① **0.031** |
| | | ② **47** |
| | | ③ **1500** |
| | | ④ **35** |
| **42** | 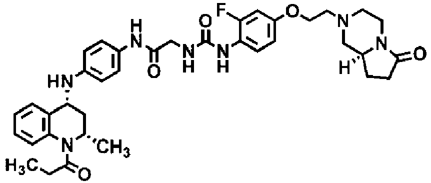 | ① **0.033** |
| | | ② **71** |
| | | ③ **2200** |
| | | ④ **50** |

**[Table 33-8]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **43** | 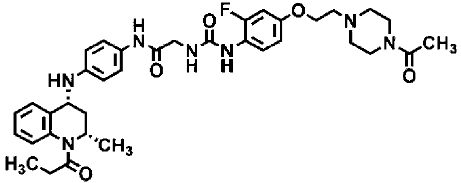 | ① **0.10** |
| | | ② **200** |
| | | ③ **2000** |
| | | ④ **45** |
| **44** | 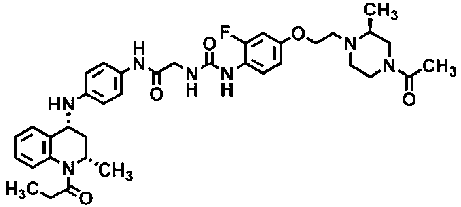 | ① **0.068** |
| | | ② **120** |
| | | ③ **1700** |
| | | ④ **40** |
| **45** | 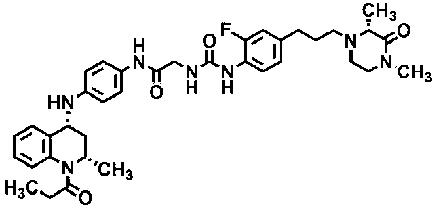 | ① **0.13** |
| | | ② **200** |
| | | ③ **1700** |
| | | ④ **38** |
| **46** | 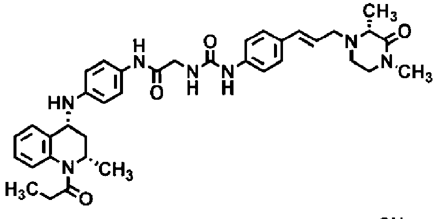 | ① **0.049** |
| | | ② **50** |
| | | ③ **1000** |
| | | ④ **24** |
| **47** | 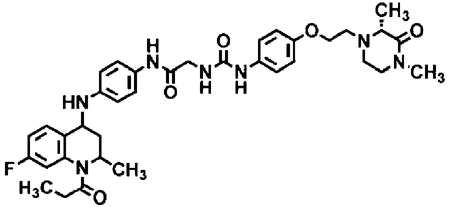 | ① **0.023** |
| | | ② **140** |
| | | ③ **6000** |
| | | ④ **140** |

**[Table 33-9]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **48** | 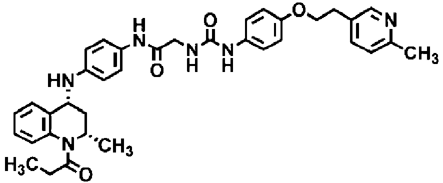 | ① **0.17** |
| | | ② **200** |
| | | ③ **1200** |
| | | ④ **28** |
| **49** | 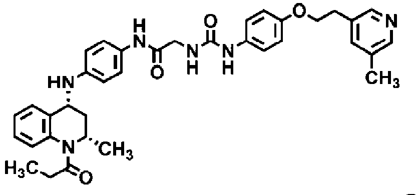 | ① **0.38** |
| | | ② **290** |
| | | ③ **760** |
| | | ④ **17** |
| **50** | 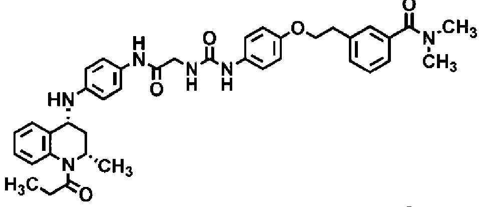 | ① **0.33** |
| | | ② **350** |
| | | ③ **1100** |
| | | ④ **25** |
| **51** | 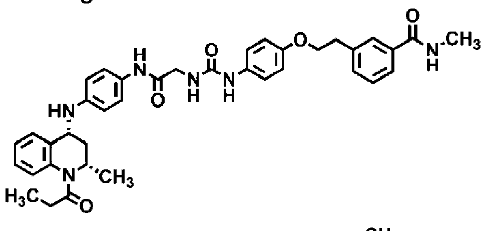 | ① **0.042** |
| | | ② **80** |
| | | ③ **1900** |
| | | ④ **44** |
| **52** | 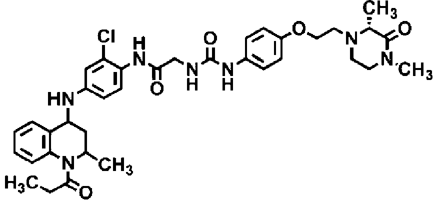 | ① **0.11** |
| | | ② **60** |
| | | ③ **550** |
| | | ④ **13** |

**[Table 33-10]**

| Compound No. | Structural formula | ① **SU-DHL-4 IC₅₀ (nmol/L)** |
|---|---|---|
| | | ② **HIP-009 IC₅₀ (nmol/L)** |
| | | ③ Nerve selectivity |
| | | ④ Degree of improvement (vs. comparative compound) |
| **53** | 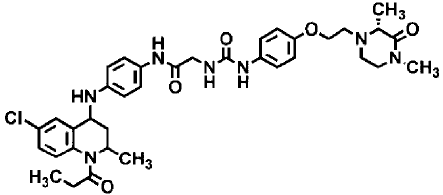 | ① **0.065** |
| | | ② **46** |
| | | ③ **710** |
| | | ④ **16** |
| **54** | 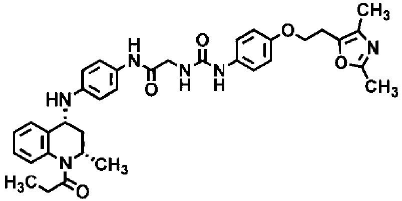 | ① **0.12** |
| | | ② **190** |
| | | ③ **1600** |
| | | ④ **37** |

In the table, the compound of compound No. 40 is a mixture of the following two types of diastereomers, and the active moiety is presumed to be the left compound from structureactivity relationship

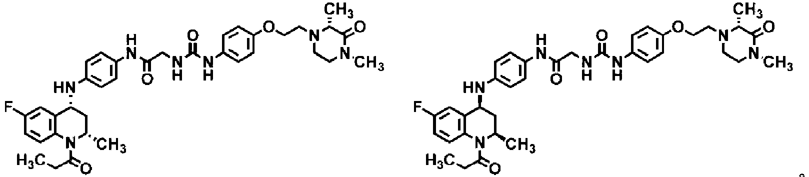

In the table, the compound of compound No. 47 is a mixture of the following two types of diastereomers, and the active moiety is presumed to be the left compound from structureactivity relationship

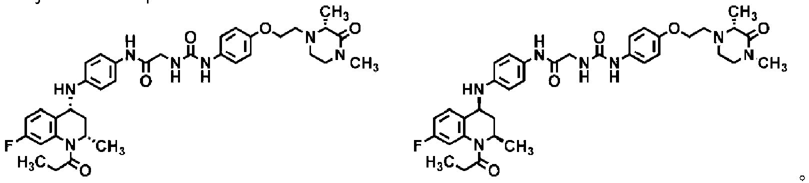

In the table, the compound of compound No. 52 is a mixture of the following two types of diastereomers, and the active moiety is presumed to be the left compound from structureactivity relationship

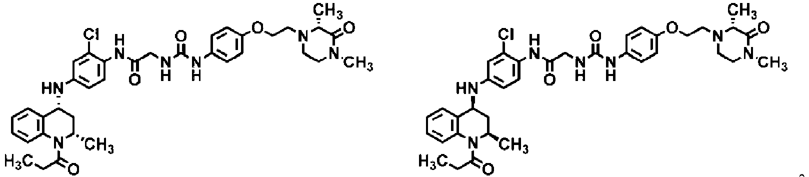

In the table, the compound of compound No. 53 is a mixture of the following two types of diastereomers, and the active moiety is presumed to be the left compound from structureactivity relationship

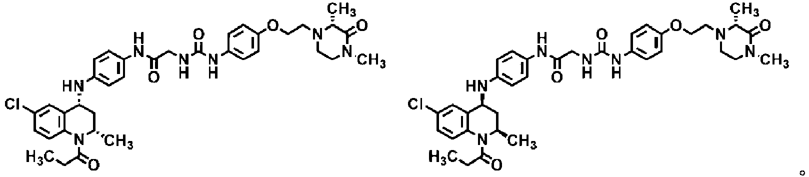

### [Reference Example 3] Method for establishing stably DLL3-expressing NCI-N417 cell

NCI-N417 cells were adjusted to 1.6 × 10⁶ cells/mL with RPMI medium and seeded at 125 µL (2 × 10⁵ cells/well) to a 24-well plate (Code No. 142475, Thermo Fisher Scientific Inc.). 0.4 µL (final concentration: 8 µg/mL) of 10 mg/mL Polybrene (Hexadimethrine bromide, Code No. H9268-5G, Sigma-Aldrich Co., LLC), 300 µL of RPMI1640 medium, and 200 µL of DLL3 (NM_016941) Human Tagged ORF Clone Lentiviral Particle (OriGene Technologies, Inc.) were mixed, and 250 µL of the mixture was added to the cells, followed by spin infection at 1000 × g at 37°C for 2 hours. The cells were recovered into a 1.5 mL tube, centrifuged at 300 × g at room temperature for 5 minutes, washed with RPMI medium supplemented with heat-inactivated FBS in an amount that attained a final concentration of 10 vol% and a PS solution in an amount that attained a final concentration of 1 vol%, then seeded in the whole amount to a 24-well plate, and cultured at 37°C for 72 hours in an incubator. Then, the cells were expansion-cultured while passaged every 3 days so as to attain 1.5 × 10⁵ cells/mL. The cells were adjusted to 5 mL at 1 × 10⁶ cells/mL, and a GFP-positive fraction was sorted (100 µL Chip, 488 nm laser) with Cell sorter MA900 (Sony Biotechnology Inc.) and seeded at approximately 1 cell per well to a 96-well plate (Code No. 163320, Thermo Fisher Scientific Inc.). Approximately 2 weeks later, proliferated cells in the wells were established as a stably expressing line.

### [Test Example 3] Evaluation of in vitro cell survival inhibitory effect of ADC using cell line

Various commercially available cells (SU-DHL-4 cells, EoL-1 cells, SUP-M2 cells, OPM-2 cells, MOLM-13 cells, NOMO-1 cells, MOLP-8 cells, NCI-N417 cells, NCI-H146 cells, OVCAR3 cells, U937 cells, RT4 cells, IGR-OV1 cells, UM-UC-3 cells, and MIA PaCa-2 cells) and stably DLL3-expressing NCI-N417 cells prepared by transferring DLL3 gene to commercially available NCI-N417 cells (hereinafter, also referred to as stably DLL3-expressing NCI-N417 cells) were each cultured in a medium supplemented with heat-inactivated FBS in an amount that attained a final concentration of 10 vol% and a PS solution in an amount that attained a final concentration of 1 vol% at 37°C in a CO₂ incubator (95% air, 5% CO₂). RPMI1640 medium was used for the culture of SU-DHL-4 cells, EoL-1 cells, SUP-M2 cells, OPM-2 cells, MOLM-13 cells, NOMO-1 cells, MOLP-8 cells, stably DLL3-expressing NCI-N417 cells, NCI-N417 cells, NCI-H146 cells, IGR-OV1 cells, or U937 cells. DMEM medium was used for the culture of OVCAR3 cells, RT4 cells, UM-UC-3 cells, or MIA PaCa-2 cells. The SU-DHL-4 cells, the EoL-1 cells, the SUP-M2 cells, the OPM-2 cells, the MOLM-13 cells, the NOMO-1 cells, the MOLP-8 cells, and the U937 cells were passaged at intervals of twice per week such that the number of cells seeded was 4×10 ⁶ cells/mL or less (e.g., 1 × 10 ⁶ cells/mL or less) in a volume of 20 mL in a T75 flask (Code No. 156800, Thermo Fisher Scientific Inc.). The OVCAR3 cells, the stably DLL3-expressing NCI-N417 cells, the NCI-N417 cells, the RT4 cells, the IGR-OV1 cells, the UM-UC-3 cells, and the MIA PaCa-2 cells were washed with 3 mL of phosphate-buffered saline (PBS) after removal of a culture supernatant by suction when passaged in a T75 flask (Code No. 156499, Thermo Fisher Scientific Inc.). Then, 2.0 mL of TrypLE(TM) Express Enzyme (1×), phenol red (Code No. 12605010, Thermo Fisher Scientific Inc.) was added to the cells, which were then left standing at 37°C for 2 to 5 minutes in an incubator and detached. Then, the cells were suspended in 8.0 mL of DMEM medium.

The cultured cells of each line were seeded to a black 384-well plate (Code No. 781086, Greiner bio-one). The number of cells seeded was set to 2000 cells/well each of the SU-DHL-4 cells, the OVCAR3 cells, and the U937 cells, 2600 cells/well of the EoL-1 cells, 1000 cells/well of the SUP-M2 cells, 3000 cells/well each of the OPM-2 cells, the MOLM-13 cells, and the IGR-OV1 cells, 4000 cells/well each of the NOMO-1 cells, the MOLP-8 cells, the stably DLL3-expressing NCI-N417 cells, and the NCI-N417 cells, 8000 cells/well of the NCI-H146 cells, and 500 cells/well each of the RT4 cells, the UM-UC-3 cells, and the MIA PaCa-2 cells. The cells thus seeded were precultured at 37°C for 18 to 25 hours in a CO₂ incubator. Subsequently, a test substance was prepared into 2500 nmol/L or 25 nmol/L, and six serial dilutions were prepared at a dilution factor of 10 so as to attain 5 times the final concentration using RPMI1640 or DMEM medium containing 10 vol% heat-inactivated FBS, 1 vol% PS, and 20 vol% heat-inactivated human serum (Code No. H4522-20ML, Sigma-Aldrich Co., LLC), and added in a volume of 1/5 to each well. The human serum was added in order to suppress the nonspecific binding of the antibody. The cells thus supplemented with the test compound were cultured at 50 µL/well at 37°C for 92 hours, 96 hours, or 144 hours in a CO₂ incubator. Subsequently, of a solution of □CellTiter-Glo 2.0 Cell Viability Assay (Code No. G924B, Promega Co)□ diluted 4-fold with PBS was added to each well. The cells were reacted at room temperature for approximately 10 minutes, followed by luminescent measurement using EnVision (PerkinElmer, Inc.). The rate of inhibition of cell survival was calculated according to the following formula, and the concentration (IC₅₀ value) of the test compound that exhibited a 50% rate of inhibition of cell survival was calculated. 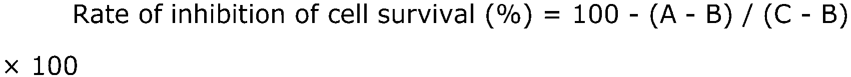 Rate of inhibition of cell survival (%) = 100 - (A - B) / (C - B) × 100
A: Absorbance of the well supplemented with the test compound
B: Absorbance of the well supplemented with no cell
C: Absorbance of the well supplemented with no test compound

The results were as shown in the column "IC₅₀ (nmol/L)" of Table 34 below.

**[Table 34-1]**

| ADC No. | Evaluation time (hr) | Antigen-positive cell | | Antigen-negative cell | |
|---|---|---|---|---|---|
| | | Cell name | IC₅₀ (nmol/L) | Cell name | IC₅₀ (nmol/L) |
| ADC-1 | 96 | SU-DHL-4 | 0.034 | EoL-1 | 79 |
| ADC-2 | 96 | SU-DHL-4 | 0.012 | EoL-1 | 16 |
| ADC-3 | 96 | SU-DHL-4 | 0.026 | EoL-1 | 18 |
| ADC-4 | 96 | SU-DHL-4 | 0.091 | EoL-1 | 38 |
| ADC-5 | 96 | SU-DHL-4 | 0.080 | EoL-1 | 16 |
| ADC-6 | 96 | SU-DHL-4 | 0.022 | EoL-1 | 33 |
| ADC-7 | 96 | SU-DHL-4 | 0.020 | EoL-1 | 120 |
| ADC-8 | 96 | SU-DHL-4 | 0.022 | EoL-1 | 130 |
| ADC-9 | 96 | SU-DHL-4 | 0.022 | EoL-1 | 84 |
| ADC-10 | 96 | SU-DHL-4 | 0.026 | EoL-1 | 160 |
| ADC-11 | 96 | SU-DHL-4 | 0.0072 | EoL-1 | 140 |
| ADC-12 | 96 | SU-DHL-4 | 0.015 | EoL-1 | 14 |
| ADC-13 | 96 | SU-DHL-4 | 0.012 | EoL-1 | 52 |
| ADC-14 | 96 | SU-DHL-4 | 0.010 | EoL-1 | >5.0 |
| ADC-15 | 96 | SU-DHL-4 | 0.039 | EoL-1 | 260 |
| ADC-16 | 96 | SU-DHL-4 | 0.023 | EoL-1 | 110 |
| ADC-17 | 144 | RT-4 | 0.12 | UM-UC-3 | 6.4 |
| ADC-18 | 96 | SU-DHL-4 | 0.013 | EoL-1 | 100 |
| ADC-21 | 72 | stably DLL3-expressing NCI-N417 | <0.001 | N/A | N/A |
| ADC-22 | 72 | NCI-N417 | 0.33 | NCl-H146 | 57 |
| ADC-23 | 96 | OVCAR3 | 13 | U937 | 210 |
| ADC-24 | 96 | U937 | 13 | OVCAR3 | 150 |
| ADC-25 | 96 | IGR-OV1 | 0.031 | SU-DHL-4 | 5 |
| ADC-27 | 96 | SUP-M2 | 0.035 | OPM-2 | 15 |
| ADC-28 | 96 | OPM-2 | 0.024 | EoL-1 | 19 |
| ADC-29 | 96 | MOLM-13 | 0.017 | SU-DHL-4 | 19 |
| ADC-30 | 96 | NOMO-1 | 1.3 | SU-DHL-4 | 19 |

**[Table 34-2]**

| ADC No. | Evaluation time (hr) | Antigen-positive cell | | Antigen-negative cell | |
|---|---|---|---|---|---|
| | | Cell name | IC₅₀ (nmol/L) | Cell name | IC₅₀ (nmol/L) |
| ADC-31 | 96 | MOLP-8 | 0.0041 | SU-DHL-4 | 32 |
| ADC-32 | 96 | SUP-M2 | 0.014 | SU-DHL-4 | 17 |
| ADC-33 | 96 | SU-DHL-4 | 0.019 | EoL-1 | 45 |
| ADC-34 | 96 | SU-DHL-4 | 0.0079 | EoL-1 | 30 |
| ADC-35 | 96 | SU-DHL-4 | 2.67 | EoL-1 | 34 |
| ADC-36 | 96 | SU-DHL-4 | 0.010 | EoL-1 | 68 |
| ADC-37 | 96 | SU-DHL-4 | 0.012 | EoL-1 | 24 |
| ADC-38 | 96 | SU-DHL-4 | 2.03 | EoL-1 | 24 |
| ADC-39 | 96 | SU-DHL-4 | 0.012 | EoL-1 | 23 |
| ADC-40 | 96 | SU-DHL-4 | 0.020 | EoL-1 | 24 |
| ADC-41 | 96 | SU-DHL-4 | 0.024 | EoL-1 | 4.9 |
| ADC-42 | 96 | SU-DHL-4 | 0.13 | EoL-1 | 210 |
| ADC-43 | 96 | OPM-2 | 0.019 | EoL-1 | 46 |
| ADC-44 | 96 | SU-DHL-4 | 0.010 | EoL-1 | 93 |
| ADC-45 | 96 | SU-DHL-4 | 0.23 | EoL-1 | 20 |
| ADC-46 | 96 | SU-DHL-4 | <0.005 | MIA PaCa-2 | 160 |
| ADC-47 | 144 | RT-4 | 0.10 | UM-UC-3 | 40 |
| ADC-48 | 144 | RT-4 | 0.0032 | UM-UC-3 | 47 |

As mentioned above, the ADC of the present invention typified by the test compounds exhibited an antigen-dependent cell survival inhibitory effect as strong as an IC₅₀ value that fell below 0.1 nmol/L on a plurality of cell lines including the SU-DHL-4 cells. The origins of cells used as antigen-positive cells are as shown in Table 35. Thus, the ADC of the present invention was found useful for treating blood cancer and solid cancer, such as B cell lymphoma (in particular, diffuse large B cell lymphoma) as well as T cell lymphoma, multiple myeloma, acute myeloid leukemia, acute monocytic leukemia, small-cell lung cancer, ovary cancer, and urinary bladder cancer. The ADC of the present invention exhibited such a strong survival inhibitory effect on the antigen-positive cell lines, whereas its survival inhibitory effect on antigen-negative cell lines was relatively weak. The survival inhibitory effect on the antigen-positive cell lines exhibited activity 10 or more times (mostly 100 or more times) stronger than that on the antigen-negative cell lines. Thus, the ADC of the present invention was found to have more desirable features as a medicament having a low safety risk of adverse reaction which is unlikely to damage non-target cells.

**[Table 35]**

| Cell | Origin |
|---|---|
| SU-DHL-4 | B cell lymphoma (diffuse large B cell lymphoma) |
| Stably DLL3-expressing NCI-N417 | Small-cell lung cancer |
| NCI-N417 | Small-cell lung cancer |
| OVCAR3 | Ovary cancer |
| U937 | Acute myeloid leukemia |
| SUP-M2 | T cell lymphoma |
| OPM-2 | Multiple myeloma |
| MOLM-13 | Acute myeloid leukemia |
| IGR-OV1 | Ovary cancer |
| MOLP-8 | Multiple myeloma |
| NOMO-1 | Acute monocytic leukemia |
| RT-4 | Urinary bladder cancer |
| UM-UC-3 | Urinary bladder cancer |
| MIA PaCa-2 | Pancreatic cancer |

### [Test Example 4] In vivo drug efficacy evaluation of compound and ADC

SU-DHL-4 cells suspended in phosphate-buffered saline (PBS) containing 50 vol% Matrigel (Corning Inc.) were ventrally subcutaneously transplanted at 1 × 10⁷ cells/0.1 mL/head to 5-week-old male SCID mice. Individuals having a tumor volume of 140 mm³ or more were selected and grouped on 15 days after transplantation.

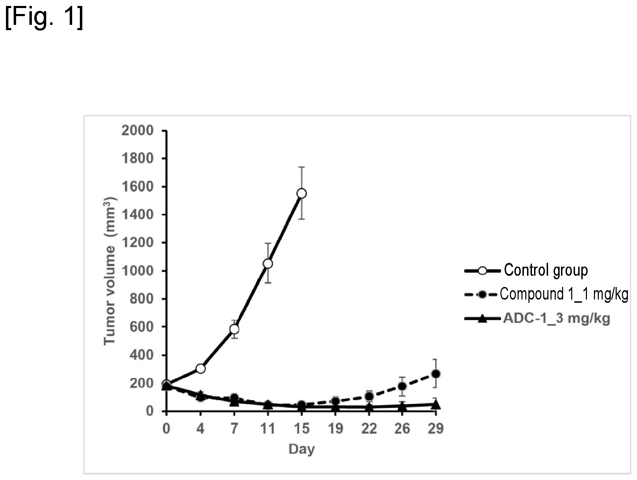

The day of grouping was defined as day 0, and diluted compound 1 was administered at 1 mg/kg body weight to the tail vein on day 1 and day 8. Diluted ADC-1 was administered at 3 mg/kg body weight to the tail vein on day 1. PBS containing 0.05 mg/mL polysorbate 80 was administered to a control group. Compound 1 was diluted with 4.29% Tween 80 (final proportions of DMSO and Tween 80: 30% and 3%, respectively). ADC-1 was diluted with PBS containing 0.05 mg/mL polysorbate 80. The tumor volumes and body weights of the mice were measured twice a week. The results are shown in Figure 1.

As shown in Figure 1, the compound (I) of the present invention typified by compound 1, and the ADC of the present invention typified by ADC-1 exhibited strong antitumor activity. Thus, the compound (I) of the present invention typified by compound 1, and the ADC of the present invention typified by ADC-1 were found useful for treating cancer such as B cell lymphoma (in particular, diffuse large B cell lymphoma).

### [Test Example 5] BET protein degradation evaluation by Western blotting (WB) using SU-DHL-4 cell line

SU-DHL-4 was subcultured using RPMI1640 medium containing 10 vol% FBS and PS. The conditions of culture involved 37°C and 5% CO₂.

The following antibodies were used in protein detection. Primary antibody:
Anti-BRD4 antibody: BRD4 (E2A7X) Rabbit mAb (Cell Signaling Technology, Inc., Cat No. #13440S)
Anti-BRD2 antibody: Brd2 (D89B4) Rabbit mAb (Cell Signaling Technology, Inc., Cat No. #5848S)
Anti-BRD3 antibody: Anti-BRD3 antibody (2088C3a) (Santa Cruz Biotechnology, Inc., Cat No. sc-81202)
Anti-beta actin antibody: Monoclonal Anti-β Actin antibody produced in mouse (SIGMA, Cat No. A5441)
Anti-GAPDH antibody: Monoclonal Anti-GAPDH antibody produced in mouse (SIGMA, Cat No. G8795)

### Secondary antibody:

Anti-Mouse IgG, HRP-Linked Whole Ab Sheep (Cytiva, Cat No. NA931-1ML)
Anti-Rabbit IgG, HRP-Linked Whole Ab Donkey (Cytiva, Cat No. NA934-1ML)

Changes in the amounts of BRD4, BRD2, and BRD3 proteins in the SU-DHL-4 cell line caused by a test compound (compound 1, 2, 5, 19, or 22) were detected by WB.

The cultured SU-DHL-4 cells were seeded at 6 × 10⁵ cells/well to a transparent 6-well plate (Code No. 140675, Thermo Fisher Scientific Inc.). The cells thus seeded were precultured at 37°C for 18 to 25 hours in a CO₂ incubator. Subsequently, a 0.1 mmol/L solution of each test compound in DMSO was prepared, then diluted so as to attain 200 times the final concentration using the RPMI1640 medium containing FBS and PS, and added in a volume of 1/200 to each well. The final concentration of DMSO was set to 0.1 vol% for this preparation. The cells thus supplemented with the test compound were cultured at 50 µL/well at 37°C for 24 hours in a CO₂ incubator.

After recovery of the cells, the supernatant was aspirated, and Lysis buffer 6 (R&D systems, Inc., Cat No. 895561) was added at 0.1 mL/sample to the pellets. The mixture was incubated on ice for 60 minutes, then vortexed, and centrifuged under conditions of approximately 530 × g, 10 minutes, and 4°C to recover a supernatant.

The protein concentrations of each sample were quantified using Pierce BCA Protein Assay Kit (Thermo Fisher Scientific Inc., Cat No. 23225) according to the manual attached to the kit. Then, the sample was prepared into 1 mg/mL by dilution with Lane Marker Reducing Sample Buffer (5x) (Thermo Fisher Scientific Inc., Cat No. 39000) and Lysis buffer 6. The dilution was incubated at 95°C for 5 minutes to prepare a WB sample. The WB sample was preserved at -30°C until electrophoresis.

The WB sample was thawed, vortexed, and then spun down for use. The gel for electrophoresis used was SuperSep Ace, 5-20 % 17 well (Wako Pure Chemical Corp., Cat No. 194-15021). The sample was added at 0.010 mL/well (amount of the protein: 0.01 mg/well), and proteins were separated by SDS-PAGE.

The proteins were transferred (100 mA/gel, 60 min) from the gel after electrophoresis to Immobilon-P Transfer membrane (Merck Millipore, Cat No. IPVH304F0) using a semi-dry transfer apparatus (ATTO Corp., Cat No. WSE-4020).

The transfer membrane was blocked by dipping in 1X TBS Tween(TM)-20 (TBS-T, Thermo Fisher Scientific Inc., Cat No. 28360) containing 5 w/v% skimmed milk (nacalai, Cat No. 31149-75) (hereinafter, referred to as 5% skimmed milk-TBS-T) for 1 hour.

For primary antibody reaction, the BRD4 antibody, the BRD2 antibody, and the GAPDH antibody were diluted 1000-fold with 5% skimmed milk-TBS-T and reacted overnight at 4°C. The beta actin antibody was diluted 2000-fold with 5% skimmed milk-TBS-T and reacted overnight at 4°C. The BRD3 antibody was diluted 200-fold with Can Get Signal Solution 1 (Toyobo Co., Ltd., Cat No. NKB-201) and reacted overnight at 4°C.

For secondary antibody reaction of the BRD4 antibody and the BRD2 antibody, the Anti-Rabbit IgG, HRP-Linked Whole Ab Donkey antibody was diluted 5000-fold with 5 % skimmed milk-TBS-T and reacted for 1 hour. For secondary antibody reaction of the BRD3 antibody, the beta actin antibody, and the GAPDH antibody, the Anti-Mouse IgG, HRP-Linked Whole Ab Sheep antibody was diluted 5000-fold with 5 % skimmed milk-TBS-T and reacted for 1 hour.

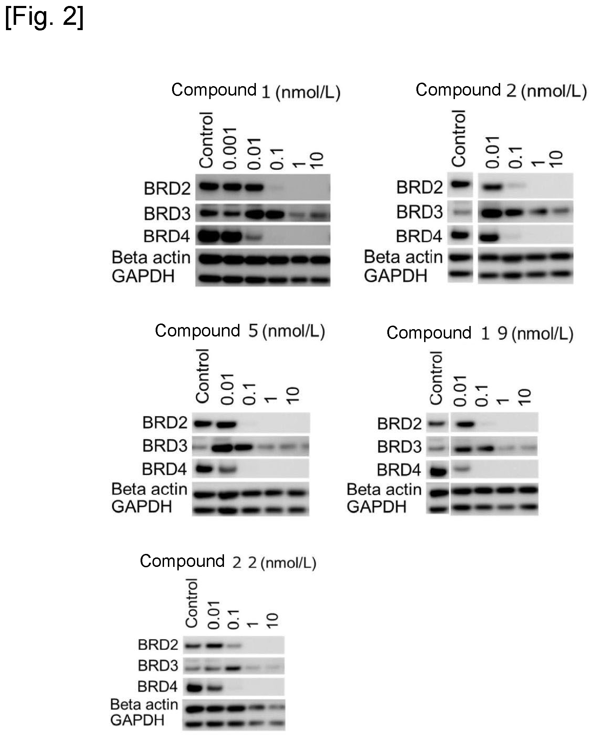

The luminescent reagents used were Supersignal West Femto Maximum Sensitivity Substrate (Thermo Fisher Scientific Inc., Cat No. #34096) for the detection of BRD4, BRD2, and BRD3, and SuperSignal West Pico Chemiluminescent Substrate (Thermo Fisher Scientific Inc., Cat No. #34080) for the detection of beta actin and GAPDH. The detection was performed using Amersham imager 600 imagers (AI600, Cytiva). The results are shown in Figure 2.

As shown in Figure 2, the band observed for the groups supplemented with compound 1, 2, 5, 19, or 22 was faint, as compared with Control, indicating that the compound of the present invention typified by the test compounds induces the degradation of BET proteins such as BRD2, BRD3, and BRD4.

### Free Text of Sequence Listing

SEQ ID NO: 2 - Description of artificial sequence: Amino acid sequence of a CH variant of hIgG1 containing an S239C mutation
SEQ ID NO: 4 - Description of artificial sequence: Amino acid sequence of a CL variant of a human κ chain containing a Q124C mutation
SEQ ID NO: 5 - Description of artificial sequence: Amino acid sequence of VH of an anti-CD79b antibody
SEQ ID NO: 6 - Description of artificial sequence: Amino acid sequence of VL of an anti-CD79b antibody
SEQ ID NO: 7 - Description of artificial sequence: Amino acid sequence of VH of an anti-CD19 antibody
SEQ ID NO: 8 - Description of artificial sequence: Amino acid sequence of VL of an anti-CD19 antibody
SEQ ID NO: 9 - Description of artificial sequence: Amino acid sequence of VH of an anti-CD22 antibody
SEQ ID NO: 10 - Description of artificial sequence: Amino acid sequence of VL of an anti-CD22 antibody
SEQ ID NO: 11 - Description of artificial sequence: Amino acid sequence of CDRH1 of an anti-CD79b antibody
SEQ ID NO: 12 - Description of artificial sequence: Amino acid sequence of CDRH2 of an anti-CD79b antibody
SEQ ID NO: 13 - Description of artificial sequence: Amino acid sequence of CDRH3 of an anti-CD79b antibody
SEQ ID NO: 14 - Description of artificial sequence: Amino acid sequence of CDRL1 of an anti-CD79b antibody
SEQ ID NO: 15 - Description of artificial sequence: Amino acid sequence of CDRL2 of an anti-CD79b antibody
SEQ ID NO: 16 - Description of artificial sequence: Amino acid sequence of CDRL3 of an anti-CD79b antibody
SEQ ID NO: 17 - Description of artificial sequence: Amino acid sequence of CDRH1 of an anti-CD19 antibody
SEQ ID NO: 18 - Description of artificial sequence: Amino acid sequence of CDRH2 of an anti-CD19 antibody
SEQ ID NO: 19 - Description of artificial sequence: Amino acid sequence of CDRH3 of an anti-CD19 antibody
SEQ ID NO: 20 - Description of artificial sequence: Amino acid sequence of CDRL1 of an anti-CD19 antibody
SEQ ID NO: 21 - Description of artificial sequence: Amino acid sequence of CDRL2 of an anti-CD19 antibody
SEQ ID NO: 22 - Description of artificial sequence: Amino acid sequence of CDRL3 of an anti-CD19 antibody
SEQ ID NO: 23 - Description of artificial sequence: Amino acid sequence of CDRH1 of an anti-CD22 antibody
SEQ ID NO: 24 - Description of artificial sequence: Amino acid sequence of CDRH2 of an anti-CD22 antibody
SEQ ID NO: 25 - Description of artificial sequence: Amino acid sequence of CDRH3 of an anti-CD22 antibody
SEQ ID NO: 26 - Description of artificial sequence: Amino acid sequence of CDRL1 of an anti-CD22 antibody
SEQ ID NO: 27 - Description of artificial sequence: Amino acid sequence of CDRL2 of an anti-CD22 antibody
SEQ ID NO: 28 - Description of artificial sequence: Amino acid sequence of CDRL3 of an anti-CD22 antibody
SEQ ID NO: 29 - Description of artificial sequence: Amino acid sequence of VH of an anti-HER2 antibody
SEQ ID NO: 30 - Description of artificial sequence: Amino acid sequence of VL of an anti-HER2 antibody
SEQ ID NO: 31 - Description of artificial sequence: Amino acid sequence of VH of an anti-DLL3 antibody
SEQ ID NO: 32 - Description of artificial sequence: Amino acid sequence of VL of an anti-DLL3 antibody
SEQ ID NO: 33 - Description of artificial sequence: Amino acid sequence of VH of an anti-GM2 antibody
SEQ ID NO: 34 - Description of artificial sequence: Amino acid sequence of VL of an anti-GM2 antibody
SEQ ID NO: 35 - Description of artificial sequence: Amino acid sequence of VH of an anti-EphA2 antibody
SEQ ID NO: 36 - Description of artificial sequence: Amino acid sequence of VL of an anti-EphA2 antibody
SEQ ID NO: 37 - Description of artificial sequence: Amino acid sequence of VH of an anti-FAP antibody
SEQ ID NO: 38 - Description of artificial sequence: Amino acid sequence of VL of an anti-FAP antibody
SEQ ID NO: 39 - Description of artificial sequence: Amino acid sequence of VH of an anti-SSTR2 antibody
SEQ ID NO: 40 - Description of artificial sequence: Amino acid sequence of VL of an anti-SSTR2 antibody
SEQ ID NO: 41 - Description of artificial sequence: Amino acid sequence of VH of an anti-CD30 antibody
SEQ ID NO: 42 - Description of artificial sequence: Amino acid sequence of VL of an anti-CD30 antibody
SEQ ID NO: 43 - Description of artificial sequence: Amino acid sequence of VH of an anti-BCMA antibody
SEQ ID NO: 44 - Description of artificial sequence: Amino acid sequence of VL of an anti-BCMA antibody
SEQ ID NO: 45 - Description of artificial sequence: Amino acid sequence of VH of an anti-CD33 antibody
SEQ ID NO: 46 - Description of artificial sequence: Amino acid sequence of VL of an anti-CD33 antibody
SEQ ID NO: 47 - Description of artificial sequence: Amino acid sequence of VH of an anti-FoLRα antibody
SEQ ID NO: 48 - Description of artificial sequence: Amino acid sequence of VL of an anti-FoLRα antibody
SEQ ID NO: 49 - Description of artificial sequence: Amino acid sequence of VH of an anti-CD25 antibody
SEQ ID NO: 50 - Description of artificial sequence: Amino acid sequence of VL of an anti-CD25 antibody
SEQ ID NO: 51 - Description of artificial sequence: Amino acid sequence of VH of an anti-CADM1 antibody
SEQ ID NO: 52 - Description of artificial sequence: Amino acid sequence of VL of an anti-CADM1 antibody
SEQ ID NO: 53 - Description of artificial sequence: Amino acid sequence of VH of an anti-CLL1 antibody
SEQ ID NO: 54 - Description of artificial sequence: Amino acid sequence of VL of an anti-CLL1 antibody
SEQ ID NO: 55 - Description of artificial sequence: Amino acid sequence of VH of an anti-CD38 antibody
SEQ ID NO: 56 - Description of artificial sequence: Amino acid sequence of VL of an anti-CD38 antibody
SEQ ID NO: 57 - Description of artificial sequence: Amino acid sequence of VH of an anti-CD74 antibody
SEQ ID NO: 58 - Description of artificial sequence: Amino acid sequence of VL of an anti-CD74 antibody
SEQ ID NO: 59 - Description of artificial sequence: Amino acid sequence of VH of an anti-CD79b(G4055) antibody
SEQ ID NO: 60 - Description of artificial sequence: Amino acid sequence of VL of an anti-CD79b(G4055) antibody
SEQ ID NO: 61 - Description of artificial sequence: Amino acid sequence of VH of an anti-CD79b(G4022) antibody
SEQ ID NO: 62 - Description of artificial sequence: Amino acid sequence of VL of an anti-CD79b(G4022) antibody
SEQ ID NO: 63 - Description of artificial sequence: Amino acid sequence of VH of an anti-CD79b(G4046) antibody
SEQ ID NO: 64 - Description of artificial sequence: Amino acid sequence of VL of an anti-CD79b(G4046) antibody
SEQ ID NO: 65 - Description of artificial sequence: Amino acid sequence of VH of an anti-CD79b(G4047) antibody
SEQ ID NO: 66 - Description of artificial sequence: Amino acid sequence of VL of an anti-CD79b(G4047) antibody
SEQ ID NO: 67 - Description of artificial sequence: Amino acid sequence of CDRH1 of an anti-HER2 antibody
SEQ ID NO: 68 - Description of artificial sequence: Amino acid sequence of CDRH2 of an anti-HER2 antibody
SEQ ID NO: 69 - Description of artificial sequence: Amino acid sequence of CDRH3 of an anti-HER2 antibody
SEQ ID NO: 70 - Description of artificial sequence: Amino acid sequence of CDRL1 of an anti-HER2 antibody
SEQ ID NO: 71 - Description of artificial sequence: Amino acid sequence of CDRL2 of an anti-HER2 antibody
SEQ ID NO: 72 - Description of artificial sequence: Amino acid sequence of CDRL3 of an anti-HER2 antibody
SEQ ID NO: 73 - Description of artificial sequence: Amino acid sequence of CDRH1 of an anti-DLL3 antibody
SEQ ID NO: 74 - Description of artificial sequence: Amino acid sequence of CDRH2 of an anti-DLL3 antibody
SEQ ID NO: 75 - Description of artificial sequence: Amino acid sequence of CDRH3 of an anti-DLL3 antibody
SEQ ID NO: 76 - Description of artificial sequence: Amino acid sequence of CDRL1 of an anti-DLL3 antibody
SEQ ID NO: 77 - Description of artificial sequence: Amino acid sequence of CDRL2 of an anti-DLL3 antibody
SEQ ID NO: 78 - Description of artificial sequence: Amino acid sequence of CDRL3 of an anti-DLL3 antibody
SEQ ID NO: 79 - Description of artificial sequence: Amino acid sequence of CDRH1 of an anti-GM2 antibody
SEQ ID NO: 80 - Description of artificial sequence: Amino acid sequence of CDRH2 of an anti-GM2 antibody
SEQ ID NO: 81 - Description of artificial sequence: Amino acid sequence of CDRH3 of an anti-GM2 antibody
SEQ ID NO: 82 - Description of artificial sequence: Amino acid sequence of CDRL1 of an anti-GM2 antibody
SEQ ID NO: 83 - Description of artificial sequence: Amino acid sequence of CDRL2 of an anti-GM2 antibody
SEQ ID NO: 84 - Description of artificial sequence: Amino acid sequence of CDRL3 of an anti-GM2 antibody
SEQ ID NO: 85 - Description of artificial sequence: Amino acid sequence of CDRH1 of an anti-EphA2 antibody
SEQ ID NO: 86 - Description of artificial sequence: Amino acid sequence of CDRH2 of an anti-EphA2 antibody
SEQ ID NO: 87 - Description of artificial sequence: Amino acid sequence of CDRH3 of an anti-EphA2 antibody
SEQ ID NO: 88 - Description of artificial sequence: Amino acid sequence of CDRL1 of an anti-EphA2 antibody
SEQ ID NO: 89 - Description of artificial sequence: Amino acid sequence of CDRL2 of an anti-EphA2 antibody
SEQ ID NO: 90 - Description of artificial sequence: Amino acid sequence of CDRL3 of an anti-EphA2 antibody
SEQ ID NO: 91 - Description of artificial sequence: Amino acid sequence of CDRH1 of an anti-FAP antibody
SEQ ID NO: 92 - Description of artificial sequence: Amino acid sequence of CDRH2 of an anti-FAP antibody
SEQ ID NO: 93 - Description of artificial sequence: Amino acid sequence of CDRH3 of an anti-FAP antibody
SEQ ID NO: 94 - Description of artificial sequence: Amino acid sequence of CDRL1 of an anti-FAP antibody
SEQ ID NO: 95 - Description of artificial sequence: Amino acid sequence of CDRL2 of an anti-FAP antibody
SEQ ID NO: 96 - Description of artificial sequence: Amino acid sequence of CDRL3 of an anti-FAP antibody
SEQ ID NO: 97 - Description of artificial sequence: Amino acid sequence of CDRH1 of an anti-SSTR2 antibody
SEQ ID NO: 98 - Description of artificial sequence: Amino acid sequence of CDRH2 of an anti-SSTR2 antibody
SEQ ID NO: 99 - Description of artificial sequence: Amino acid sequence of CDRH3 of an anti-SSTR2 antibody
SEQ ID NO: 100 - Description of artificial sequence: Amino acid sequence of CDRL1 of an anti-SSTR2 antibody
SEQ ID NO: 101 - Description of artificial sequence: Amino acid sequence of CDRL2 of an anti-SSTR2 antibody
SEQ ID NO: 102 - Description of artificial sequence: Amino acid sequence of CDRL3 of an anti-SSTR2 antibody
SEQ ID NO: 103 - Description of artificial sequence: Amino acid sequence of CDRH1 of an anti-CD30 antibody
SEQ ID NO: 104 - Description of artificial sequence: Amino acid sequence of CDRH2 of an anti-CD30 antibody
SEQ ID NO: 105 - Description of artificial sequence: Amino acid sequence of CDRH3 of an anti-CD30 antibody
SEQ ID NO: 106 - Description of artificial sequence: Amino acid sequence of CDRL1 of an anti-CD30 antibody
SEQ ID NO: 107 - Description of artificial sequence: Amino acid sequence of CDRL2 of an anti-CD30 antibody
SEQ ID NO: 108 - Description of artificial sequence: Amino acid sequence of CDRL3 of an anti-CD30 antibody
SEQ ID NO: 109 - Description of artificial sequence: Amino acid sequence of CDRH1 of an anti-BCMA antibody
SEQ ID NO: 110 - Description of artificial sequence: Amino acid sequence of CDRH2 of an anti-BCMA antibody
SEQ ID NO: 111 - Description of artificial sequence: Amino acid sequence of CDRH3 of an anti-BCMA antibody
SEQ ID NO: 112 - Description of artificial sequence: Amino acid sequence of CDRL1 of an anti-BCMA antibody
SEQ ID NO: 113 - Description of artificial sequence: Amino acid sequence of CDRL2 of an anti-BCMA antibody
SEQ ID NO: 114 - Description of artificial sequence: Amino acid sequence of CDRL3 of an anti-BCMA antibody
SEQ ID NO: 115 - Description of artificial sequence: Amino acid sequence of CDRH1 of an anti-CD33 antibody
SEQ ID NO: 116 - Description of artificial sequence: Amino acid sequence of CDRH2 of an anti-CD33 antibody
SEQ ID NO: 117 - Description of artificial sequence: Amino acid sequence of CDRH3 of an anti-CD33 antibody
SEQ ID NO: 118 - Description of artificial sequence: Amino acid sequence of CDRL1 of an anti-CD33 antibody
SEQ ID NO: 119 - Description of artificial sequence: Amino acid sequence of CDRL2 of an anti-CD33 antibody
SEQ ID NO: 120 - Description of artificial sequence: Amino acid sequence of CDRL3 of an anti-CD33 antibody
SEQ ID NO: 121 - Description of artificial sequence: Amino acid sequence of CDRH1 of an anti-FoLRα antibody
SEQ ID NO: 122 - Description of artificial sequence: Amino acid sequence of CDRH2 of an anti-FoLRα antibody
SEQ ID NO: 123 - Description of artificial sequence: Amino acid sequence of CDRH3 of an anti-FoLRα antibody
SEQ ID NO: 124 - Description of artificial sequence: Amino acid sequence of CDRL1 of an anti-FoLRα antibody
SEQ ID NO: 125 - Description of artificial sequence: Amino acid sequence of CDRL2 of an anti-FoLRα antibody
SEQ ID NO: 126 - Description of artificial sequence: Amino acid sequence of CDRL3 of an anti-FoLRα antibody
SEQ ID NO: 127 - Description of artificial sequence: Amino acid sequence of CDRH1 of an anti-CD25 antibody
SEQ ID NO: 128 - Description of artificial sequence: Amino acid sequence of CDRH2 of an anti-CD25 antibody
SEQ ID NO: 129 - Description of artificial sequence: Amino acid sequence of CDRH3 of an anti-CD25 antibody
SEQ ID NO: 130 - Description of artificial sequence: Amino acid sequence of CDRL1 of an anti-CD25 antibody
SEQ ID NO: 131 - Description of artificial sequence: Amino acid sequence of CDRL2 of an anti-CD25 antibody
SEQ ID NO: 132 - Description of artificial sequence: Amino acid sequence of CDRL3 of an anti-CD25 antibody
SEQ ID NO: 133 - Description of artificial sequence: Amino acid sequence of CDRH1 of an anti-CADM1 antibody
SEQ ID NO: 134 - Description of artificial sequence: Amino acid sequence of CDRH2 of an anti-CADM1 antibody
SEQ ID NO: 135 - Description of artificial sequence: Amino acid sequence of CDRH3 of an anti-CADM1 antibody
SEQ ID NO: 136 - Description of artificial sequence: Amino acid sequence of CDRL1 of an anti-CADM1 antibody
SEQ ID NO: 137 - Description of artificial sequence: Amino acid sequence of CDRL2 of an anti-CADM1 antibody
SEQ ID NO: 138 - Description of artificial sequence: Amino acid sequence of CDRL3 of an anti-CADM1 antibody
SEQ ID NO: 139 - Description of artificial sequence: Amino acid sequence of CDRH1 of an anti-CLL1 antibody
SEQ ID NO: 140 - Description of artificial sequence: Amino acid sequence of CDRH2 of an anti-CLL1 antibody
SEQ ID NO: 141 - Description of artificial sequence: Amino acid sequence of CDRH3 of an anti-CLL1 antibody
SEQ ID NO: 142 - Description of artificial sequence: Amino acid sequence of CDRL1 of an anti-CLL1 antibody
SEQ ID NO: 143 - Description of artificial sequence: Amino acid sequence of CDRL2 of an anti-CLL1 antibody
SEQ ID NO: 144 - Description of artificial sequence: Amino acid sequence of CDRL3 of an anti-CLL1 antibody
SEQ ID NO: 145 - Description of artificial sequence: Amino acid sequence of CDRH1 of an anti-CD38 antibody
SEQ ID NO: 146 - Description of artificial sequence: Amino acid sequence of CDRH2 of an anti-CD38 antibody
SEQ ID NO: 147 - Description of artificial sequence: Amino acid sequence of CDRH3 of an anti-CD38 antibody
SEQ ID NO: 148 - Description of artificial sequence: Amino acid sequence of CDRL1 of an anti-CD38 antibody
SEQ ID NO: 149 - Description of artificial sequence: Amino acid sequence of CDRL2 of an anti-CD38 antibody
SEQ ID NO: 150 - Description of artificial sequence: Amino acid sequence of CDRL3 of an anti-CD38 antibody
SEQ ID NO: 151 - Description of artificial sequence: Amino acid sequence of CDRH1 of an anti-CD74 antibody
SEQ ID NO: 152 - Description of artificial sequence: Amino acid sequence of CDRH2 of an anti-CD74 antibody
SEQ ID NO: 153 - Description of artificial sequence: Amino acid sequence of CDRH3 of an anti-CD74 antibody
SEQ ID NO: 154 - Description of artificial sequence: Amino acid sequence of CDRL1 of an anti-CD74 antibody
SEQ ID NO: 155 - Description of artificial sequence: Amino acid sequence of CDRL2 of an anti-CD74 antibody
SEQ ID NO: 156 - Description of artificial sequence: Amino acid sequence of CDRL3 of an anti-CD74 antibody
SEQ ID NO: 157 - Description of artificial sequence: Amino acid sequence of CDRH1 of an anti-CD79b(G4055) antibody
SEQ ID NO: 158 - Description of artificial sequence: Amino acid sequence of CDRH2 of an anti-CD79b(G4055) antibody
SEQ ID NO: 159 - Description of artificial sequence: Amino acid sequence of CDRH3 of an anti-CD79b(G4055) antibody
SEQ ID NO: 160 - Description of artificial sequence: Amino acid sequence of CDRL1 of an anti-CD79b(G4055) antibody
SEQ ID NO: 161 - Description of artificial sequence: Amino acid sequence of CDRL2 of an anti-CD79b(G4055) antibody
SEQ ID NO: 162 - Description of artificial sequence: Amino acid sequence of CDRL3 of an anti-CD79b(G4055) antibody
SEQ ID NO: 163 - Description of artificial sequence: Amino acid sequence of CDRH1 of an anti-CD79b(G4022) antibody
SEQ ID NO: 164 - Description of artificial sequence: Amino acid sequence of CDRH2 of an anti-CD79b(G4022) antibody
SEQ ID NO: 165 - Description of artificial sequence: Amino acid sequence of CDRH3 of an anti-CD79b(G4022) antibody
SEQ ID NO: 166 - Description of artificial sequence: Amino acid sequence of CDRL1 of an anti-CD79b(G4022) antibody
SEQ ID NO: 167 - Description of artificial sequence: Amino acid sequence of CDRL2 of an anti-CD79b(G4022) antibody
SEQ ID NO: 168 - Description of artificial sequence: Amino acid sequence of CDRL3 of an anti-CD79b(G4022) antibody
SEQ ID NO: 169 - Description of artificial sequence: Amino acid sequence of CDRH1 of an anti-CD79b(G4046) antibody
SEQ ID NO: 170 - Description of artificial sequence: Amino acid sequence of CDRH2 of an anti-CD79b(G4046) antibody
SEQ ID NO: 171 - Description of artificial sequence: Amino acid sequence of CDRH3 of an anti-CD79b(G4046) antibody
SEQ ID NO: 172 - Description of artificial sequence: Amino acid sequence of CDRL1 of an anti-CD79b(G4046) antibody
SEQ ID NO: 173 - Description of artificial sequence: Amino acid sequence of CDRL2 of an anti-CD79b(G4046) antibody
SEQ ID NO: 174 - Description of artificial sequence: Amino acid sequence of CDRL3 of an anti-CD79b(G4046) antibody
SEQ ID NO: 175 - Description of artificial sequence: Amino acid sequence of CDRH1 of an anti-CD79b(G4047) antibody
SEQ ID NO: 176 - Description of artificial sequence: Amino acid sequence of CDRH2 of an anti-CD79b(G4047) antibody
SEQ ID NO: 177 - Description of artificial sequence: Amino acid sequence of CDRH3 of an anti-CD79b(G4047) antibody
SEQ ID NO: 178 - Description of artificial sequence: Amino acid sequence of CDRL1 of an anti-CD79b(G4047) antibody
SEQ ID NO: 179 - Description of artificial sequence: Amino acid sequence of CDRL2 of an anti-CD79b(G4047) antibody
SEQ ID NO: 180 - Description of artificial sequence: Amino acid sequence of CDRL3 of an anti-CD79b(G4047) antibody
SEQ ID NO: 181 - Description of artificial sequence: Amino acid sequence of a CH variant of hIgG4 containing an R409K mutation and an S239C mutation
SEQ ID NO: 182 - Description of artificial sequence: Amino acid sequence of a CH variant of hIgG1 containing an S239C mutation and an H435F mutation
SEQ ID NO: 183 - Description of artificial sequence: Amino acid sequence of VH of an anti-NECTIN4 antibody
SEQ ID NO: 184 - Description of artificial sequence: Amino acid sequence of VL of an anti-NECTIN4 antibody
SEQ ID NO: 185 - Description of artificial sequence: Amino acid sequence of VH of an anti-TROP2 antibody
SEQ ID NO: 186 - Description of artificial sequence: Amino acid sequence of VL of an anti-TROP2 antibody
SEQ ID NO: 187 - Description of artificial sequence: Amino acid sequence of CDRH1 of an anti-NECTIN4 antibody
SEQ ID NO: 188 - Description of artificial sequence: Amino acid sequence of CDRH2 of an anti-NECTIN4 antibody
SEQ ID NO: 189 - Description of artificial sequence: Amino acid sequence of CDRH3 of an anti-NECTIN4 antibody
SEQ ID NO: 190 - Description of artificial sequence: Amino acid sequence of CDRL1 of an anti-NECTIN4 antibody
SEQ ID NO: 191 - Description of artificial sequence: Amino acid sequence of CDRL2 of an anti-NECTIN4 antibody
SEQ ID NO: 192 - Description of artificial sequence: Amino acid sequence of CDRL3 of an anti-NECTIN4 antibody
SEQ ID NO: 193 - Description of artificial sequence: Amino acid sequence of CDRH1 of an anti-TROP2 antibody
SEQ ID NO: 194 - Description of artificial sequence: Amino acid sequence of CDRH2 of an anti-TROP2 antibody
SEQ ID NO: 195 - Description of artificial sequence: Amino acid sequence of CDRH3 of an anti-TROP2 antibody
SEQ ID NO: 196 - Description of artificial sequence: Amino acid sequence of CDRL1 of an anti-TROP2 antibody
SEQ ID NO: 197 - Description of artificial sequence: Amino acid sequence of CDRL2 of an anti-TROP2 antibody
SEQ ID NO: 198 - Description of artificial sequence: Amino acid sequence of CDRL3 of an anti-TROP2 antibody
SEQ ID NO: 199 - Description of artificial sequence: Amino acid sequence of the heavy chain of an antigen binding fragment of an anti-CD79b antibody

## Claims

1. A compound represented by the following formula (I) or a pharmaceutically acceptable salt thereof: 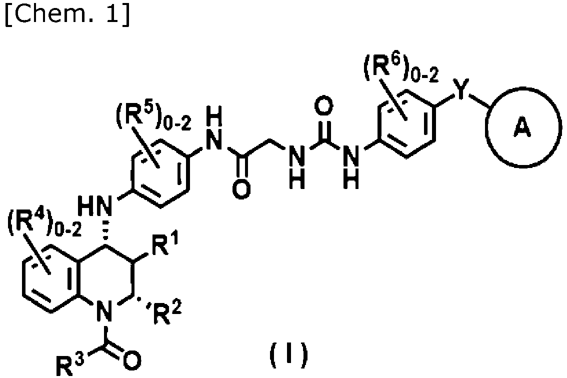 (wherein,
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-, and
ring A represents
(i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
(ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
(iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).

2. The compound or the pharmaceutically acceptable salt thereof according to claim 1, wherein (i) the aliphatic heterocyclic group in ring A is a nitrogen-containing aliphatic heterocyclic group.

3. The compound or the pharmaceutically acceptable salt thereof according to claim 2, wherein ring A represented by (i) the nitrogen-containing aliphatic heterocyclic group is the following ring A': 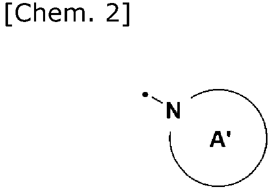 (wherein, when ring A' has a substituent, the substituent of ring A' is as defined for the substituent of ring A).

4. The compound or the pharmaceutically acceptable salt thereof according to claim 1, wherein the compound represented by the formula (I) is a compound represented by the following formula (I'): 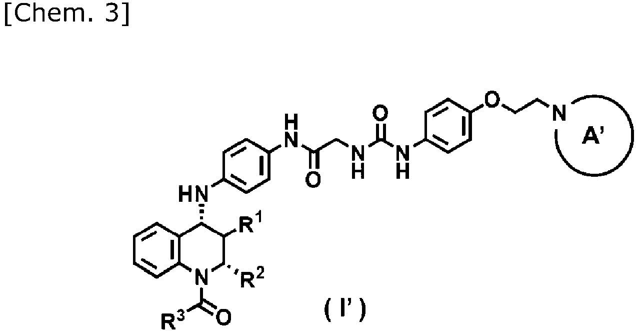 (wherein,
R¹, R², and R³ are as defined in the formula (I), and
ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).

5. The compound or the pharmaceutically acceptable salt thereof according to claim 1, wherein ring A is an aliphatic heterocyclic group represented by the following formula (AA), (AB), (AC), or (AD), an aromatic heterocyclic group represented by the following formula (AE) or (AF), or aryl represented by the following formula (AG): 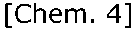 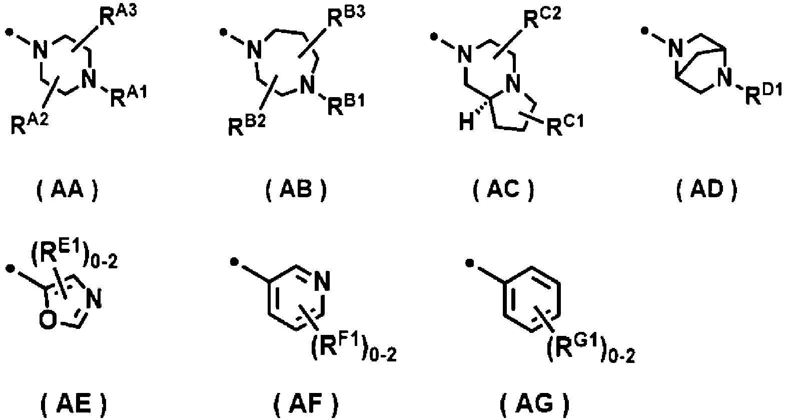 (wherein,
R^{A1}, R^{B1}, R^{D1}, R^{E1}, R^{F1}, and R^{G1} are the same or different and each represents lower alkyl, hydroxy-lower alkyl, lower alkylcarbonyl, hydroxy-lower alkylcarbonyl, lower alkoxycarbonyl, cycloalkylcarbonyl, or lower alkylcarbamoyl, and
R^{A2}, R^{A3}, R^{B2}, R^{B3}, R^{C1}, and R^{C2} are the same or different and each represents a hydrogen atom, oxo, lower alkyl, or hydroxy-lower alkyl).

6. The compound or the pharmaceutically acceptable salt thereof according to claim 1, wherein ring A is an aliphatic heterocyclic group represented by the following formula (AA): 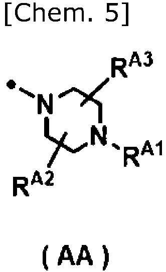 (wherein,
R^{A1} represents lower alkyl, lower alkylcarbonyl, or cycloalkylcarbonyl, and
R^{A2} and R^{A3} are the same or different and each represents a hydrogen atom, oxo, lower alkyl, or hydroxy-lower alkyl).

7. The compound or the pharmaceutically acceptable salt thereof according to claim 1, wherein ring A is an aliphatic heterocyclic group represented by the following formula (AB): 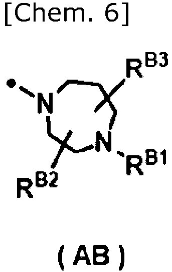 (wherein,
R^{B1} represents lower alkyl or lower alkylcarbonyl, and
R^{B2} and R^{B3} are the same or different and each represents a hydrogen atom or oxo).

8. The compound or the pharmaceutically acceptable salt thereof according to claim 1, wherein ring A is an aliphatic heterocyclic group represented by the following formula (AC): 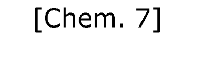 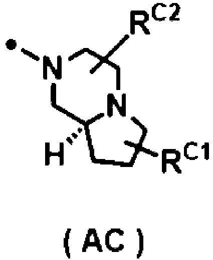 (wherein, R^{C1} and R^{C2} are the same or different and each represents a hydrogen atom or oxo).

9. The compound or the pharmaceutically acceptable salt thereof according to claim 1, wherein ring A is an aliphatic heterocyclic group represented by the following formula (AD): 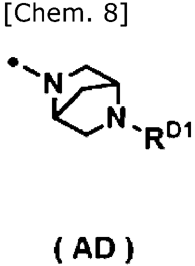 (wherein, R^{D1} represents lower alkylcarbonyl).

10. The compound or the pharmaceutically acceptable salt thereof according to claim 1, wherein ring A is an aromatic heterocyclic group represented by the following formula (AE): 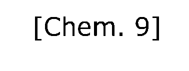 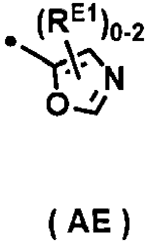 (wherein, R^{E1} represents lower alkyl).

11. The compound or the pharmaceutically acceptable salt thereof according to claim 1, wherein ring A is an aromatic heterocyclic group represented by the following formula (AF): 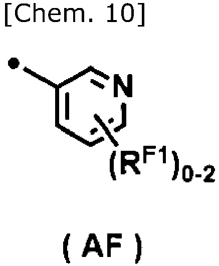 (wherein, R^{F1} represents lower alkyl).

12. The compound or the pharmaceutically acceptable salt thereof according to claim 1, wherein ring A is aryl represented by the following formula (AG): 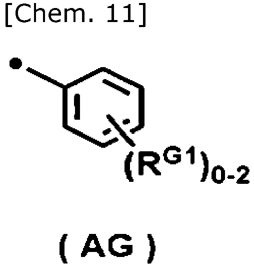 (wherein, R^{G1} represents lower alkoxycarbonyl or lower alkylcarbamoyl).

13. The compound or the pharmaceutically acceptable salt thereof according to claim 1, wherein Y is -O-CH₂-CH₂-.

14. A targeting molecule-drug conjugate comprising a targeting molecule and a drug,
wherein the drug is the compound or the pharmaceutically acceptable salt thereof according to any one of claims 1 to 13.

15. The targeting molecule-drug conjugate according to claim 14, wherein the drug is linked to the targeting molecule via a linker.

16. The targeting molecule-drug conjugate according to claim 15, wherein the targeting molecule-drug conjugate is represented by the following formula (II): 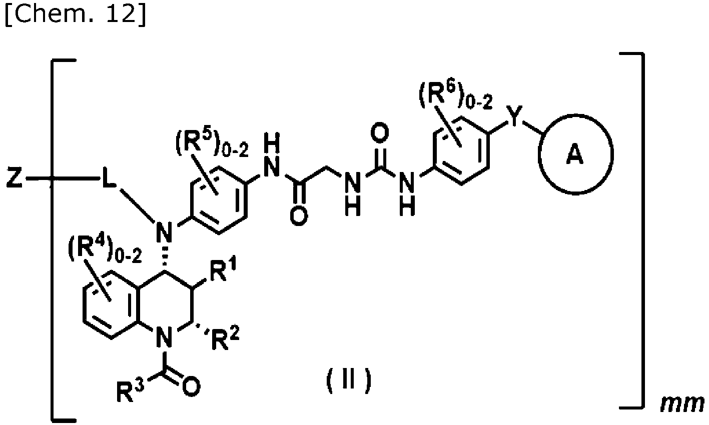 (wherein,
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-,
ring A represents
(i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
(ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
(iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
L represents a linker,
mm represents an integer of 1 to 8, and
Z represents a targeting molecule).

17. The targeting molecule-drug conjugate according to claim 16, wherein ring A represented by (i) the aliphatic heterocyclic group is the following ring A': 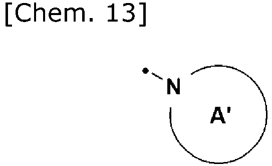 (wherein, when ring A' has a substituent, the substituent of ring A' is as defined for the substituent of ring A).

18. The targeting molecule-drug conjugate according to claim 16, wherein the targeting molecule-drug conjugate is represented by the following formula (II'): 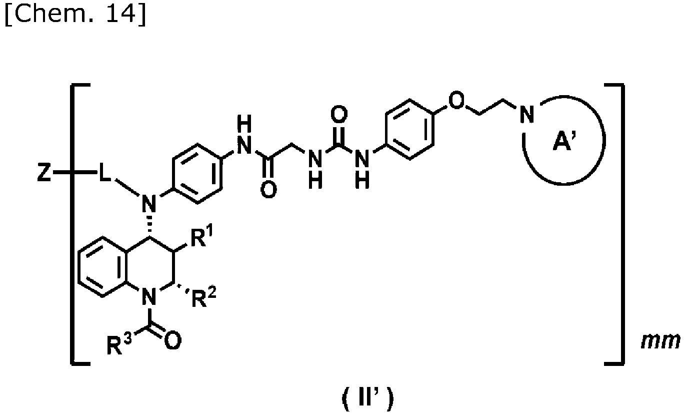 (wherein,
R¹, R², R³, L, mm, and Z are as defined in the formula (II), and
ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).

19. The targeting molecule-drug conjugate according to claim 16, wherein L is represented by the following formula (III): 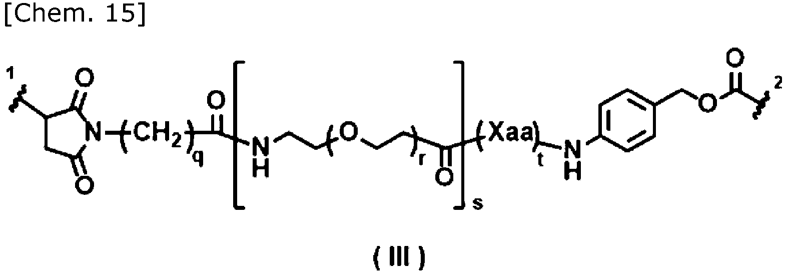 (wherein,
wavy line 1 represents a bonding site to Z,
wavy line 2 represents a bonding site to the N atom, and
each Xaa independently represents
(a) a residue of at least one amino acid selected from the group consisting of valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents
(b) a peptide mimetic represented by the following formula (IV): 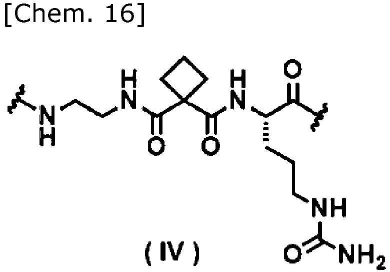 wherein,
q represents an integer of 1 to 5,
r represents an integer of 2 to 24,
s represents 0 or 1, and
t represents 1, 2, 3, or 4).

20. The targeting molecule-drug conjugate according to claim 14, wherein the targeting molecule is an antibody or an antigen binding fragment thereof, a small-molecule compound, or a peptide.

21. The targeting molecule-drug conjugate according to claim 14, wherein the targeting molecule is an antibody.

22. The targeting molecule-drug conjugate according to claim 21, wherein the antibody is an anti-CD79b antibody, an anti-CD19 antibody, an anti-CD22 antibody, an anti-HER2 antibody, an anti-DLL3 antibody, an anti-GM2 antibody, an anti-EphA2 antibody, an anti-FAP antibody, an anti-SSTR2 antibody, an anti-CD30 antibody, an anti-BCMA antibody, an anti-CD33 antibody, an anti-FoLRα antibody, an anti-CD25 antibody, an anti-CADM1 antibody, an anti-CLL1 antibody, an anti-CD38 antibody, an anti-CD74 antibody, an anti-Nectin4, or an anti-TROP2 antibody.

23. A drug-linker conjugate comprising a drug and a linker linked to the drug, wherein
the drug is the compound or the pharmaceutically acceptable salt thereof according to any one of claims 1 to 13.

24. The drug-linker conjugate according to claim 23, wherein the drug-linker conjugate is represented by the following formula (V): 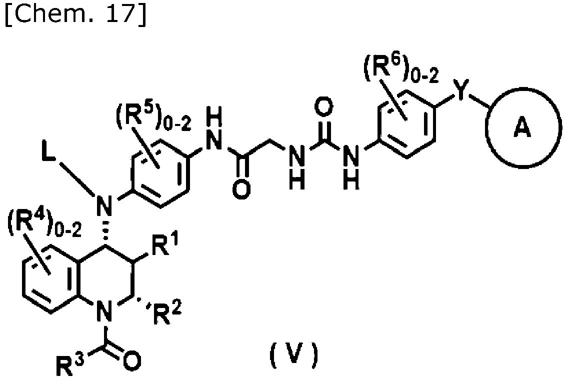 (wherein,
R¹ and R² are the same or different and each represents a hydrogen atom or lower alkyl,
R³ represents lower alkyl,
R⁴ represents halogen,
R⁵ represents halogen,
R⁶ represents halogen or lower alkoxy,
Y represents -O-CH₂-CH₂-, -CH₂-CH₂-CH₂-, -CH=CH-CH₂-, or - NH-C(=O)-CH₂-,
ring A represents
(i) an aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl,
(ii) an aromatic heterocyclic group optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, or
(iii) aryl optionally having 1 to 3 substituents selected from the group consisting of lower alkyl, hydroxy-lower alkyl, lower alkylcarbonyl, hydroxy-lower alkylcarbonyl, lower alkoxycarbonyl, cycloalkylcarbonyl, and lower alkylcarbamoyl, and
L represents a linker).

25. The drug-linker conjugate according to claim 24, wherein ring A represented by (i) the aliphatic heterocyclic group is the following ring A': 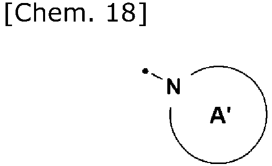 (wherein, when ring A' has a substituent, the substituent of ring A' is as defined for the substituent of ring A).

26. The drug-linker conjugate according to claim 24, wherein the drug-linker conjugate is represented by the following formula (V'): 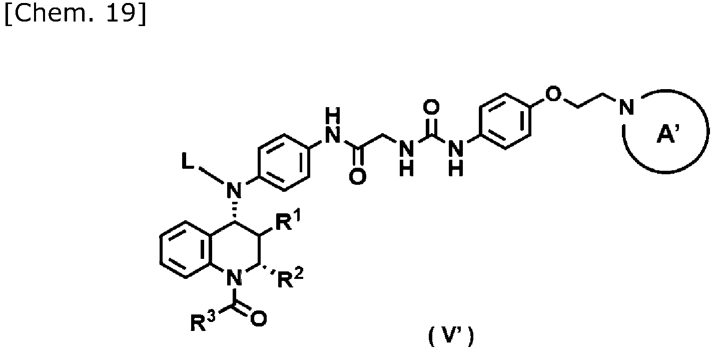 (wherein,
R¹, R², R³, and L are as defined in the formula (V), and
ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).

27. The drug-linker conjugate according to claim 24, wherein the drug-linker conjugate is represented by the following formula (Va): 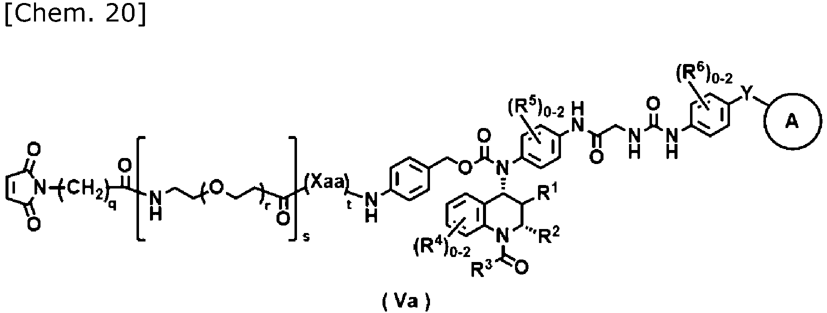 (wherein,
R¹, R², R³, R⁴, R⁵, R⁶, Y, and ring A are as defined above, and
each Xaa independently represents
(a) a residue of at least one amino acid selected from the group consisting of valine, alanine, citrulline, glycine, phenylalanine, glutamic acid, cysteic acid, arginine, asparagine, aspartic acid, glutamine, leucine, isoleucine, lysine, serine, proline, threonine, and tyrosine, or represents
(b) a peptide mimetic represented by the following formula (IV): 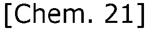 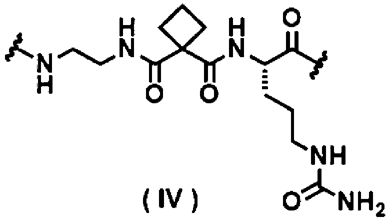 wherein,
q represents an integer of 1 to 5,
r represents an integer of 2 to 24,
s represents 0 or 1, and
t represents 1, 2, 3, or 4).

28. The drug-linker conjugate according to claim 27, wherein ring A represented by (i) the aliphatic heterocyclic group is the following ring A': 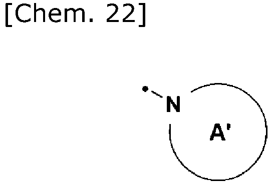 (wherein, when ring A' has a substituent, the substituent of ring A' is as defined for the substituent of ring A).

29. The drug-linker conjugate according to claim 27, wherein the drug-linker conjugate is represented by the following formula (V'a): 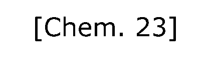 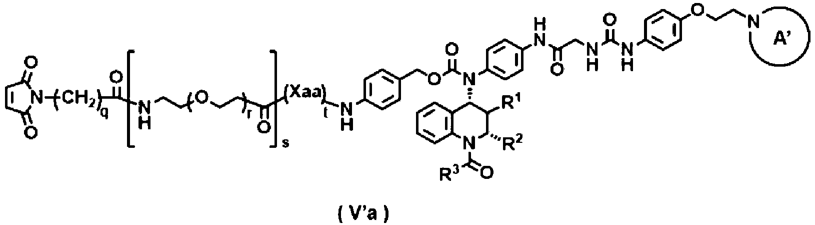 (wherein,
R¹, R², R³, Xaa, q, r, s, and t are as defined in the formula (Va), and
ring A' is a nitrogen-containing aliphatic heterocyclic group optionally having 1 to 3 substituents selected from the group consisting of oxo, lower alkyl, hydroxy-lower alkyl, lower alkylcarbonyl, hydroxy-lower alkylcarbonyl, lower alkoxycarbonyl, cycloalkylcarbonyl, and lower alkylcarbamoyl).
